# EUROPEAN PATENT APPLICATION

(11) **EP 4 840 445 A1**
(43) Date of publication of application: **18.11.2026**
(21) Application number: 25741659.4
(22) Date of filing: 20.01.2025
(51) Int. Cl.: A61K 9/51, A61K 9/72, A61K 47/18, A61K 47/22, A61K 47/26, A61K 47/24, A61K 47/28

(54) **PULMONARY DELIVERY SYSTEM CONTAINING IONIZABLE LIPID, AND PREPARATION METHOD THEREFOR AND USE THEREOF**

(30) Priority: 19.01.2024 CN 202410079919
(71) Applicant: Scindy Pharmaceutical Co., Ltd, Suzhou, Jiangsu 215124 (CN)
(72) Inventor: CHEN, Xiaobao, Suzhou, Jiangsu 215124 (CN)
(74) Representative: Elzaburu S.L.P.
(86) International application number: PCT/CN2025/073416
(87) International publication number: WO 2025/153098

(57) **Abstract**

A pulmonary delivery system comprising ionizable lipids, a preparation method and use thereof, which belongs to the technical field of biomedicine. The pulmonary delivery system comprising ionizable lipids comprises ionizable lipids, helper lipids and bioactive substances, and is free of polymer-conjugated lipids. The pulmonary delivery system comprising ionizable lipids is capable of delivering the bioactive substance to the lungs. The pulmonary delivery system has the characteristics of uniform particle size and high safety, and can efficiently deliver a therapeutic agent to the lungs. Before and after atomization, the particle properties such as the particle size, polydispersity index, potential, and encapsulation efficiency remain unchanged. The pulmonary delivery system provides a safe and effective solution for the treatment of pulmonary diseases, and has broad application prospects.

## Description

### CROSS-REFERENCE TO RELATED APPLICATIONS

This application claims priority to Chinese Patent Application No. 202410079919.8, filed on January 19, 2024, the entire contents of which are incorporated herein by reference.

### TECHNICAL FIELD

The present application relates to the technical field of biomedicine, and specifically belongs to a pulmonary delivery system comprising ionizable lipids, a preparation method and use thereof.

### BACKGROUND

Pulmonary administration, also referred to as inhalation delivery, primarily involves oral inhalation, passage through the pharynx into the respiratory tract, and ultimately reaching the deep respiratory tract or lungs, thereby exerting local or systemic therapeutic effects. Compared with other routes of administration, pulmonary administration offers advantages including a large absorption area, thin alveolar epithelial cell membranes, and high permeability. The absorption site is characterized by rich blood flow and relatively low enzymatic activity, which avoids hepatic first-pass metabolism, resulting in high bioavailability of pulmonary administration. For drugs susceptible to degradation in the gastrointestinal tract or exhibiting substantial hepatic first-pass effects, such as protein or peptide drugs, pulmonary administration can significantly improve bioavailability.

Respiratory epithelial cells line the respiratory tract, and their principal functions include moistening the respiratory tract, protecting the airways from potential pathogens, infections, and tissue damage, and facilitating gas exchange. Dysfunction of airway epithelial cells can lead to numerous conditions, including asthma, chronic obstructive pulmonary disease, and cystic fibrosis. Delivering payloads to respiratory epithelial cells can be used to induce immunity against an antigen of interest and regulate airway epithelial cell function, e.g., by restoring defective proteins or adjusting cellular activity. Taking cystic fibrosis as an example, statistics indicate that the incidence thereof in Caucasians at birth is 1 in 2,500 to 3,500. Currently, most existing therapies for cystic fibrosis merely provide symptomatic control via antibiotics, anti-inflammatory agents, bronchodilators, and nutritional supplementation, without achieving complete or reliable disease control.

Accordingly, there is an urgent need to develop novel therapies for the treatment of conditions associated with respiratory epithelial cell dysfunction or other intractable pulmonary diseases. Inhaled nebulized formulations can deliver drugs directly to the lungs, thereby increasing pulmonary drug concentrations and reducing systemic toxicity - this targeted delivery is an inherent advantage of respiratory administration. However, the development of inhaled formulations, particularly inhaled nucleic acid lipid nanoparticle (LNP) formulations, faces the challenge of poor nebulizability, namely the inability to withstand shear damage during nebulization, resulting in leakage of nucleic acid drugs, marked increases in particle size and dispersity, and significant reductions in transfection efficiency post-nebulization. Accordingly, the primary challenge in developing nebulized nucleic acid LNP formulations is to overcome the deleterious effects of nebulization on LNPs. Presently, there is a paucity of patents and literature regarding nebulized inhaled nucleic acid LNP formulations. Most reports screen formulations by modifying the ratios of the four LNP components to achieve minimal changes in particle size, PDI, and encapsulation efficiency following nebulization. Nevertheless, particle size generally increases markedly post-nebulization, and encapsulation efficiency decreases significantly.

CN116348147A discloses compositions for delivering payload molecules to airway epithelium, comprising nanoparticle cores formed from ionizable lipids, phospholipids, structural lipids, and PEG-lipids, encapsulating polynucleotides or polypeptides, with cationic agents on the core surface. The specification discloses Administration Procedure B (nasal-only aerosol exposure), wherein a vibrating mesh nebulizer generates aerosol at a defined inlet airflow rate for exposure to the nasal and oral cavities of animals. The aerodynamic particle size distribution of the aerosol is monitored at the administration port before and after each dose, with a respirable particle size target of 1-4 µm for rats. It is inferred that the particle size of the composition increases to the micrometer level post-nebulization.

CN115708818B discloses a universal formulation for nebulized inhaled lipid nanoparticles, said formulation comprising a lipid nanodispersion system and a surface tension reducing regulator. While said universal lipid nanoparticle formulation maintains particle size and encapsulation efficiency unchanged before and after nebulization, it necessitates the additional incorporation of organic solvents or surfactants as surface tension regulators to mitigate LNP damage during nebulization. Nevertheless, said organic solvents or surfactants may increase cytotoxicity and reduce cellular tolerance.

Although lipid nanoparticles have succeeded in delivering mRNA vaccines, inhaled mRNA therapies for pulmonary diseases remain challenging. LNPs are readily degraded by shear stress during nebulization, impairing effective mRNA delivery. Accordingly, improving LNP structural stability is urgently needed to meet the requirements of nebulized administration.

### SUMMARY

In view of the problems existing in the prior art, the present application provides a pulmonary delivery system comprising ionizable lipids, a preparation method and use thereof. Free of polymer-conjugated lipids, the system contains ionizable lipids, helper lipids and bioactive substances and is administered to the lungs via nebulization. The system features uniform particle size and superior safety, efficiently transports therapeutics to the lungs, and keeps particle size, polydispersity index, zeta potential and encapsulation efficiency stable pre- and post-nebulization, which serves as a safe and effective regimen for treating pulmonary diseases.

To achieve the above objectives, in a first aspect, the present application provides a pulmonary delivery system comprising ionizable lipids, which comprises ionizable lipids, helper lipids and bioactive substances, the pulmonary delivery system comprising ionizable lipids is free of polymer-conjugated lipids; the ionizable lipids are selected from at least one of a first ionizable lipid, a second ionizable lipid and a third ionizable lipid;

The first ionizable lipid is selected from a compound having general formula (1) or a pharmaceutically acceptable salt, stereoisomer, tautomer thereof; 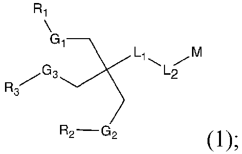
wherein R₁, R₂, and R₃ are each independently selected from H, C₅₋₄₀ linear or branched alkyl group, C₅₋₄₀ linear or branched alkenyl group, C₅₋₄₀ linear or branched alkynyl group, a 3-6 membered saturated or partially unsaturated cycloaliphatic group comprising 1-3 side chains, or a 6-10 membered aromatic group comprising 1-3 side chains; the side chains are each independently selected from C10-30 linear or branched alkyl, C10-30 linear or branched alkenyl, and C10-30 linear or branched alkynyl; provided that at most one of R₁, R₂ and R₃ is H;
M is selected from -NR₄R₅, a saturated or partially unsaturated 3-6 membered heterocyclic group containing at least one nitrogen atom, and a 6-10 membered heteroaryl group containing at least one nitrogen atom; the heterocyclic group and heteroaryl group are unsubstituted or substituted with one or more substituents selected from -OH, carboxyl, amino, oxo and halogen;
R₄ and R₅ are each independently selected from H, C₁₋₆ linear or branched alkyl, C₂₋₆ linear or branched alkenyl, or C₂₋₆ linear or branched alkynyl; the C₁₋₆linear or branched alkyl, C₂₋₆ linear or branched alkenyl or C₂₋₆ linear or branched alkynyl is unsubstituted or substituted with one or more substituents selected from -OH, carboxyl, aminoamido, amidino, guanidyl and halogen;
G₁, G₂ and G₃ are each independently selected from -O-, -S-, -NR₆-, -S-S-, -C(=O)-, -C(=S)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₆-, -NR₆-S(=O)₂-, -P(=O)(OR₆)O-, -OP(=O)(OR₆)- or -OP(=O)(OR₆)O-; wherein each of R₆ and R₁₃ is independently selected from H, hydroxyl, C₁₋₃₀ linear or branched alkyl or cycloalkyl, and C₂₋₃₀ linear or branched alkenyl;
L₁ is selected from -X₁- or -(CR₇R₈)ₘ₋X₁-; wherein each X₁ is independently selected from -O-, -S-, -NR₁₄-, -S-S-, -C(=O)-, -C(=S)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₄-, -NR₁₄-S(=O)₂-, -P(=O)(OR₁₄)O-, -OP(=O)(OR₁₄)- or -OP(=O)(OR₁₄)O-; wherein m is an integer of 2-6; R₇ and R₈ are each independently selected from H, hydroxyl, halogen, C₁₋₆ linear or branched alkyl or cycloalkyl, or C₂₋₆ linear or branched alkenyl; wherein each of R₁₄ and R₁₅ is independently selected from H, C₁₋₃₀ linear or branched alkyl or cycloalkyl, and C₂₋₃₀ linear or branched alkenyl;
L₂ is -(CR₉R₁₀)ₙ- or -(CR₉R₁₀)ₙ-X₂-(CR₁₁R₁₂)ₖ-; wherein X₂ is selected from -O-, -S-, -NR₁₆-, -S-S-, -C(=O)-, -C(=S)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₆-, NR₁₆-S(=O)₂-, -P(=O)(OR₁₆)O-, -OP(=O)(OR₁₆)- or -OP(=O)(OR₁₆)O-; n is an integer of 1-6; k is an integer of 1-6; R₉, R₁₀, R₁₁ and R₁₂ are each independently selected from H, hydroxyl, halogen, C₁₋₆ linear or branched alkyl or cycloalkyl, or C₂₋₆ linear or branched alkenyl; each of R₁₆ and R₁₇ is independently selected from H, C₁₋₃₀ linear or branched alkyl or cycloalkyl, and C₂₋₃₀ linear or branched alkenyl;
the alkyl, cycloalkyl and alkenyl groups defined in R₄ to R₁₇ are unsubstituted or substituted with one or more groups selected from hydroxyl, mercapto, amino, substituted amino, and halogen;
the salt does not include quaternary ammonium salt.

In a preferred embodiment, the bioactive substance comprises at least one selected from DNA, RNA, proteins, peptides and small-molecule compounds.

In a preferred embodiment, the dosage of the bioactive substances accounts for 1-50% (w/w), more preferably 2-30% (w/w), of the total mass of lipid raw materials and bioactive substances.

In a preferred embodiment, R₁, R₂ and R₃ are each independently selected from a group shown as follows: 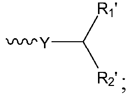 wherein Y is absent, or is C₁₋₃₀ linear or branched alkyl or cycloalkyl, C₂₋₂₀ linear or branched alkenyl, or C₂₋₂₀ linear or branched alkynyl; R₁'and R₂' are each independently selected from H, C₁₋₃₀ linear or branched alkyl, C₂₋₃₀ linear or branched alkenyl, or C₂₋₃₀ linear or branched alkynyl, and the total carbon chain length of Y, R₁'and R₂' is 8-40.

In a preferred embodiment, R₁, R₂ and R₃ are each independently selected froma group shown as follows: 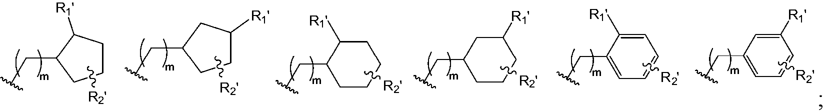 wherein R₁'and R₂' are each independently selected from H, C₁₋₃₀ linear or branched alkyl, C₂₋₃₀ linear or branched alkenyl, or C₂₋₃₀ linear or branched alkynyl, and the total carbon chain length of R₁'and R₂' is 8-30.

In a preferred embodiment, R₁, R₂ and R₃ are each independently selected from any one of the following groups:

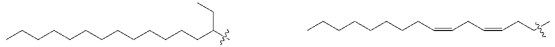

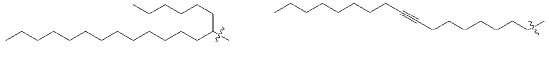

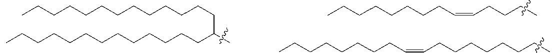

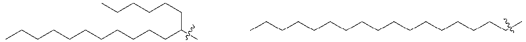

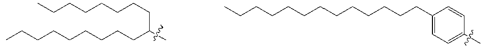

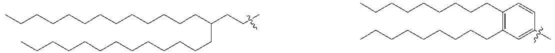

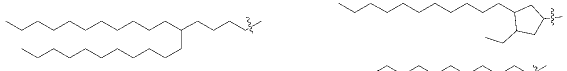

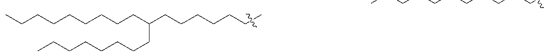

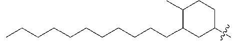

In a preferred embodiment, G₁, G₂ and G₃ are each independently selected from -O-, -S-, -NR₆-, -S-S-, -C(=O)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -P(=O)(OR₆)O-, -OP(=O)(OR₆)- or -OP(=O)(OR₆)O-.

In a preferred embodiment, L₁ is selected from -(CR₇R₈)ₘ-X₁-, and X₁ is selected from -O-, -S-, -NR₁₄-, -S-S-, -C(=O)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -P(=O)(OR₁₄)O-, -OP(=O)(OR₁₄)- or -OP(=O)(OR₁₄)O-.

In a preferred embodiment, L₂ is -(CR₉R₁₀)ₙ-X₂-(CR₁₁R₁₂)i-, and X₂ is selected from -O-, -S-, -NR₁₆-, -S-S-, -C(=O)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -P(=O)(OR₁₆)O-, -OP(=O)(OR₁₆)- or -OP(=O)(OR₁₆)O-.

In a preferred embodiment, M is selected from the following 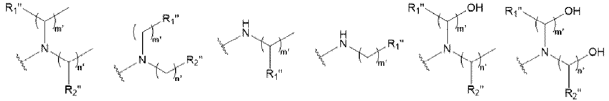 structures 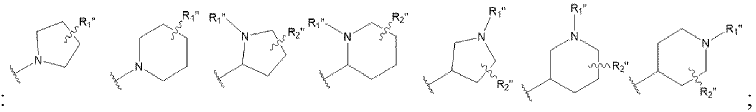 wherein m' and n' are each independently an integer of 0-6; R₁" and R₂" are each independently selected from H, C₁₋₆ alkyl, C₂₋₆ alkenyl, guanidyl, amidino, amido, aliphatic amino group, or a 3-10 membered nitrogen-containing heterocycle; the nitrogen-containing heterocycle is selected from pyrrole, imidazole, pyridine, pyrazole, triazole, oxazole, isoxazole, thiophene, isothiazole, pyridazine, pyrazine, piperazine, indole, benzimidazole, carbazole, quinoline, isoquinoline, purine, and pyrimidine, and tautomers thereof, which is unsubstituted or optionally substituted with one or more organic groups selected from hydroxyl, mercapto, amino, substituted amino, halogen, C₁-C₆ alkyl, C₂-C₆ alkenyl, C₂-C₆ alkynyl, and C₆-C₁₄ aryl.

In a preferred embodiment, the compound of Formula (1) is selected from compounds represented by Formula (1A):

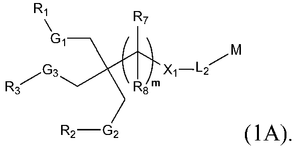

In a preferred embodiment, the compound of Formula (1) is selected from compounds represented by Formula (1B):

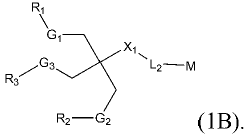

In a preferred embodiment, the compound of Formula (1) is selected from compounds represented by Formula (1C):

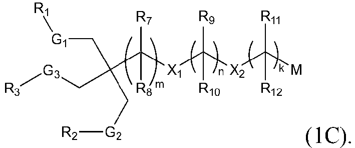

In a preferred embodiment, the compound of Formula (1) is selected from compounds represented by Formula (1D):

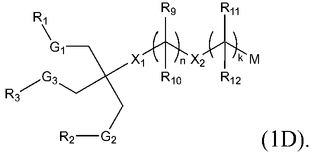

In a preferred embodiment, the compound of Formula (1) is selected from compounds represented by Formula (1E):

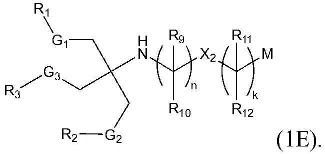

In a preferred embodiment, the compound of Formula (1) is selected from compounds represented by Formula (1F):

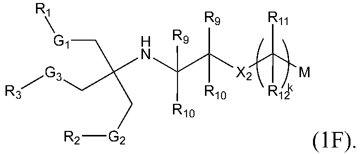

In a preferred embodiment, the compound of Formula (1) is selected from compounds represented byf Formula (1 G):

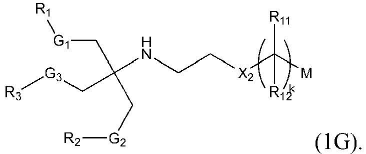

In a preferred embodiment, the compound of Formula (1) is selected from compounds represented by Formula (1H):

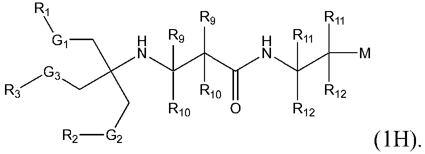

In a preferred embodiment, M is selected from any one of the following groups:

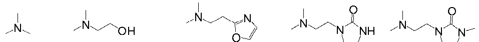

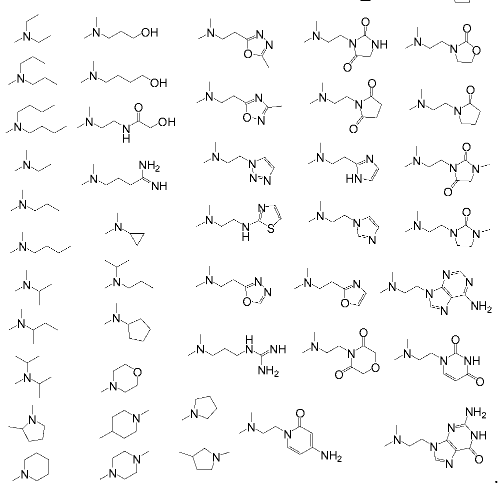

In a preferred embodiment, the compound of Formula (1) is selected from compounds represented by Formula (11):

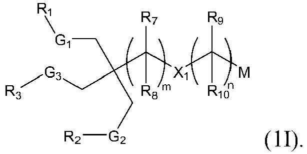

In a preferred embodiment, the compound of Formula (1) is selected from compounds represented by Formula (1J):

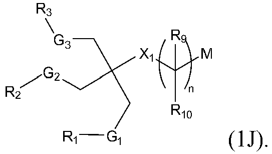

In a preferred embodiment, the compound of Formula (1) is selected from compounds represented by Formula (1K):

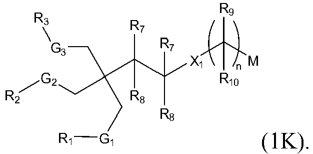

In a preferred embodiment, Y is absent, and the compound of Formula (1) is selected from compounds represented by Formula (1L): 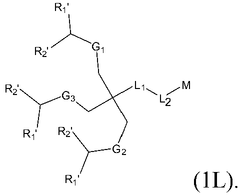 wherein R₁'and R₂' are each independently selected from H, C₁₋₃₀ linear or branched alkyl, C₂₋₃₀ linear or branched alkenyl, and C₂₋₃₀ linear or branched alkynyl, and the total carbon chain length of R₁'and R₂' is 8-40.

In a preferred embodiment, the compound of Formula (1) is selected from compounds represented by Formula (1M):

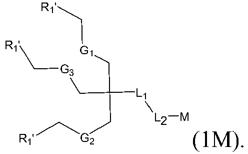

In a preferred embodiment, the compound of Formula (1) is selected from:

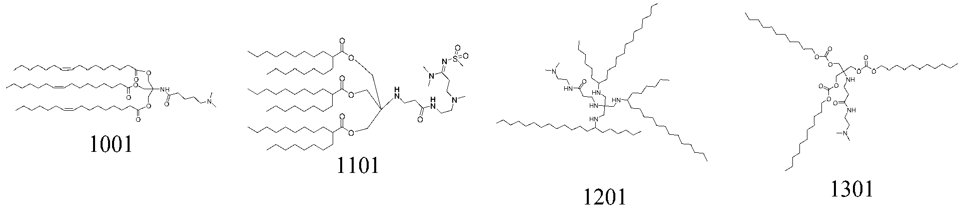

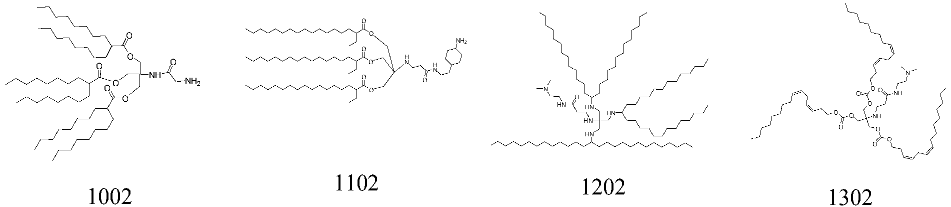

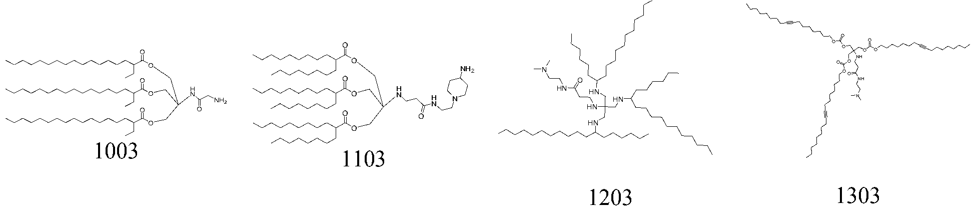

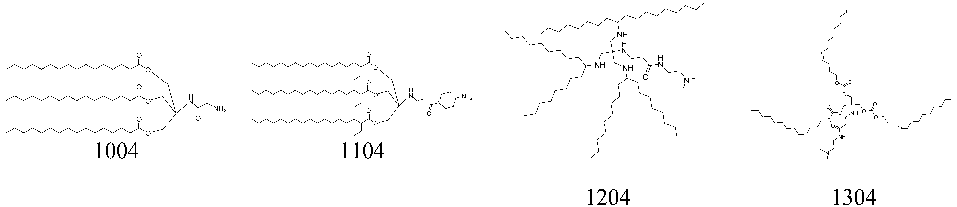

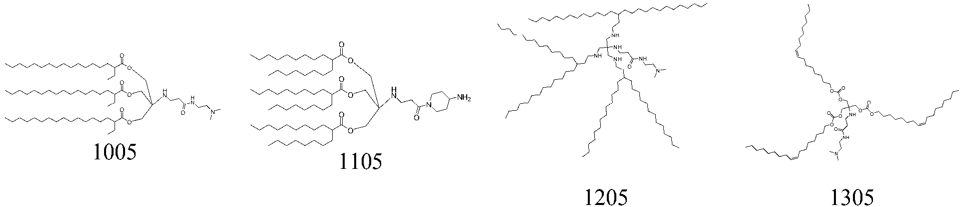

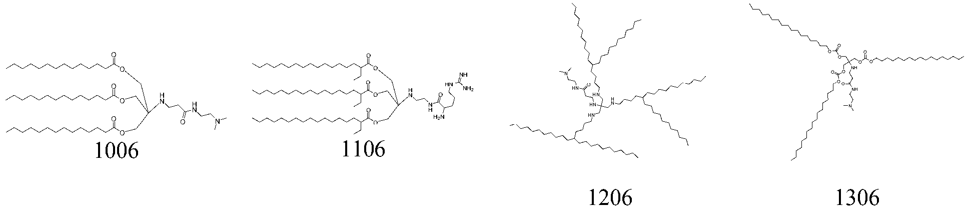

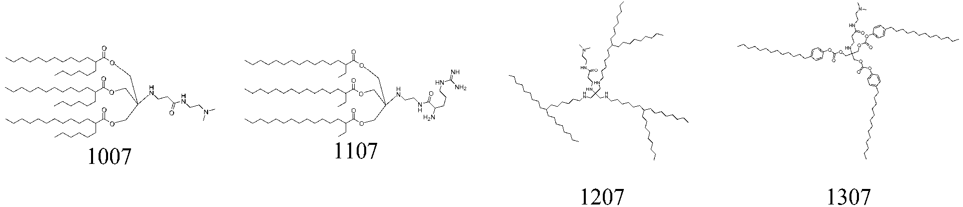

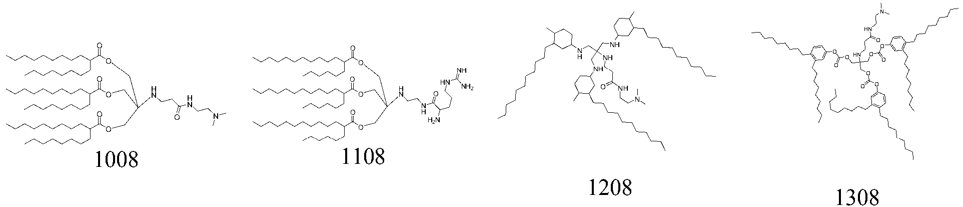

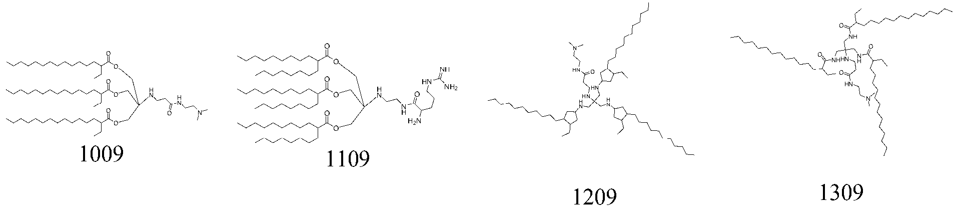

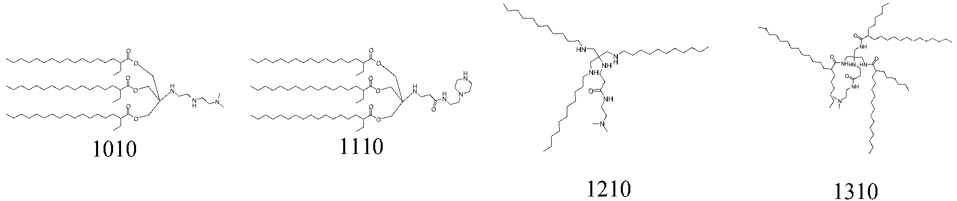

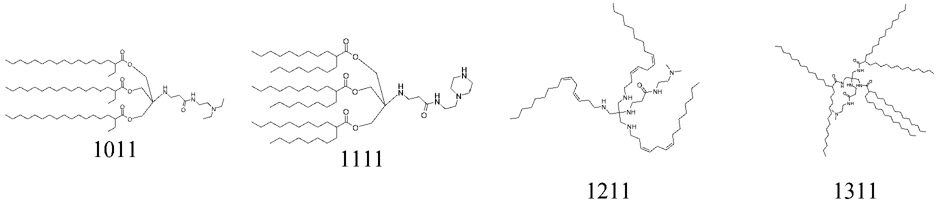

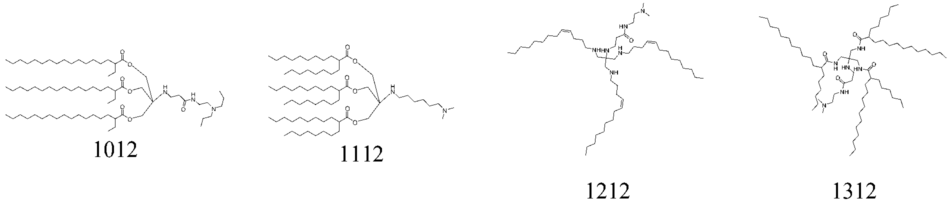

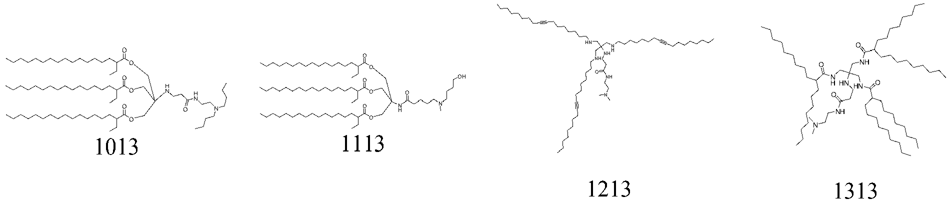

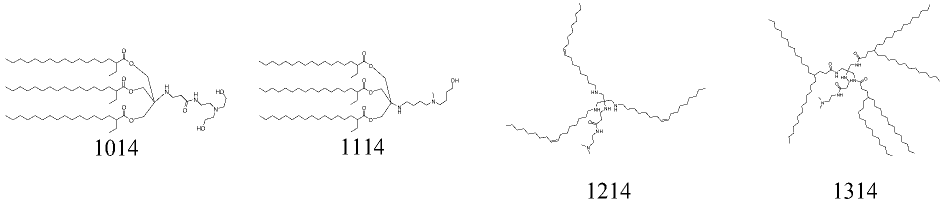

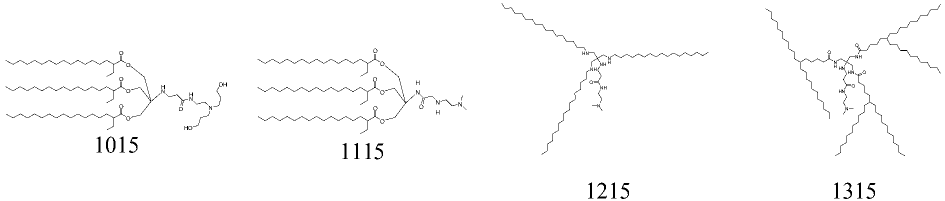

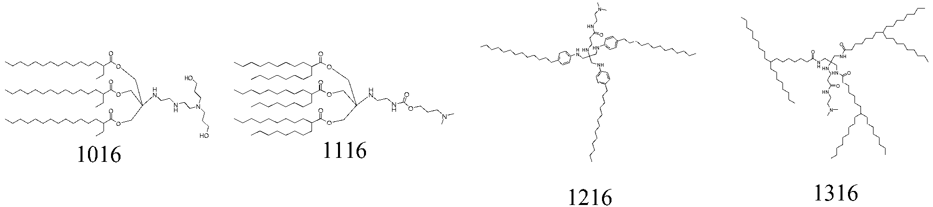

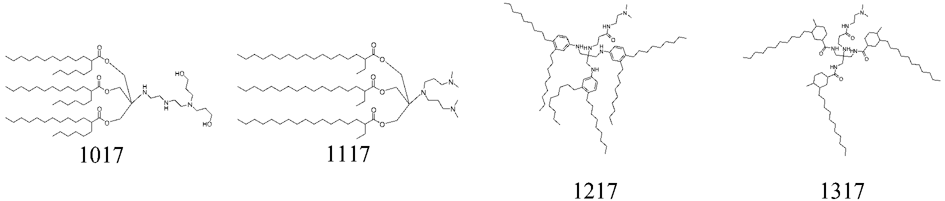

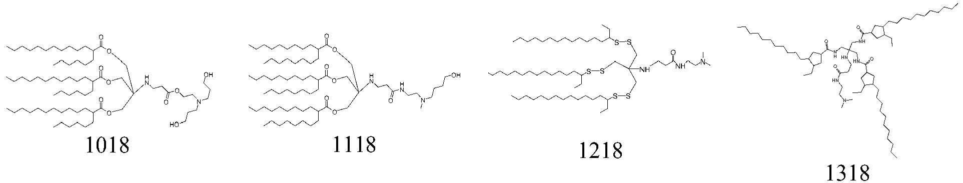

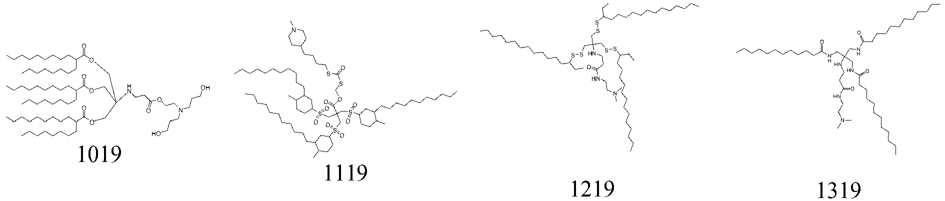

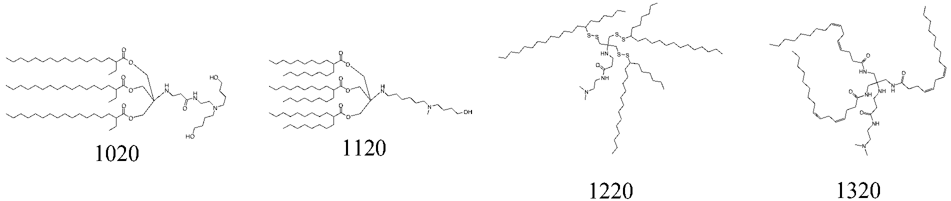

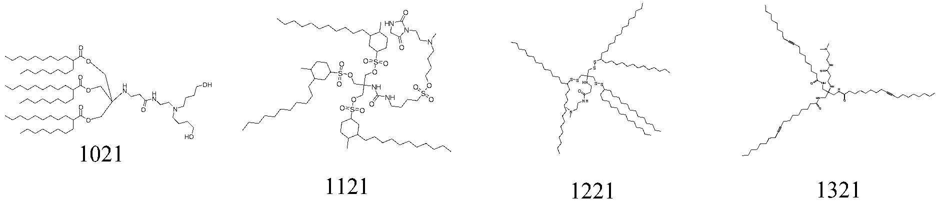

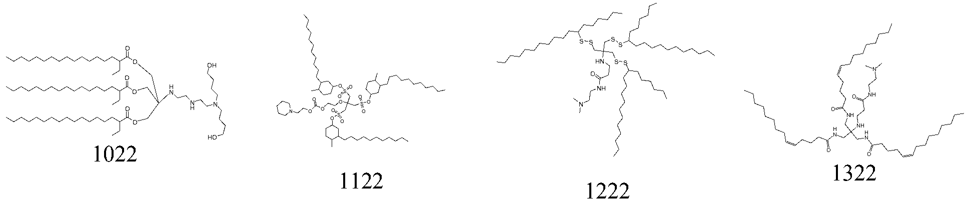

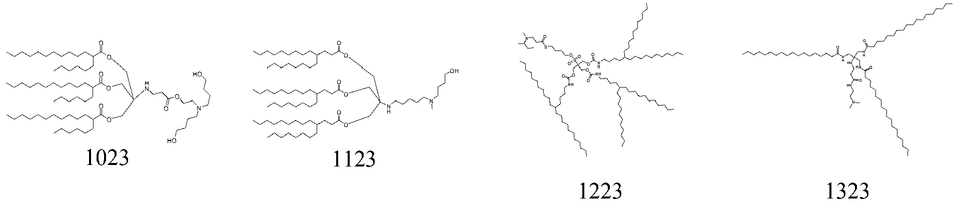

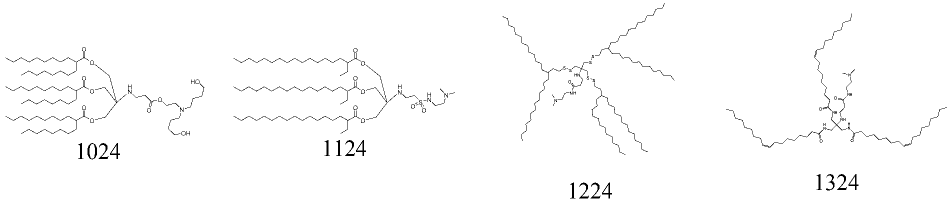

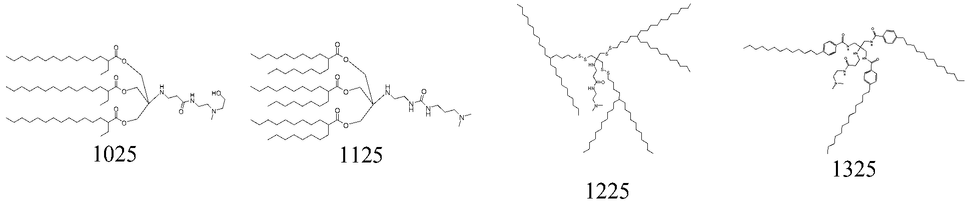

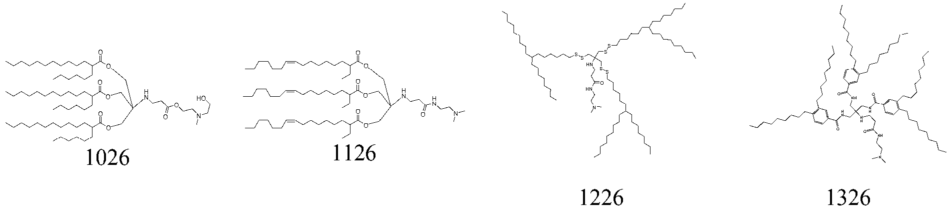

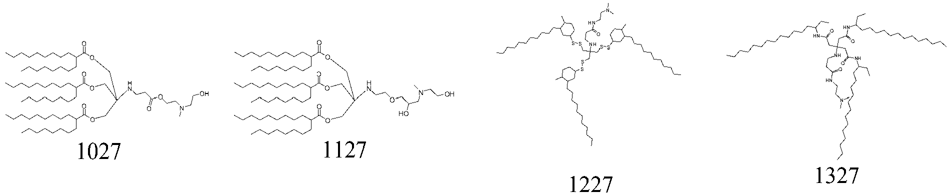

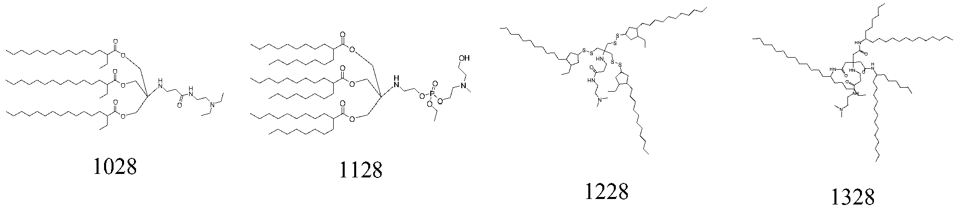

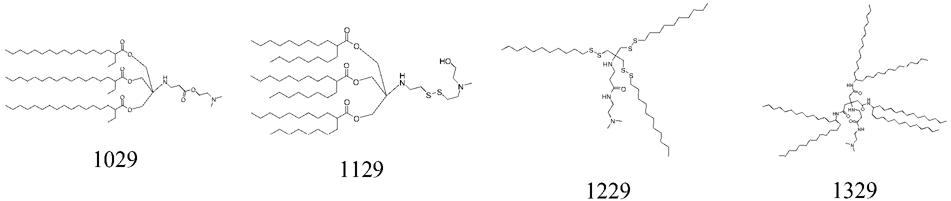

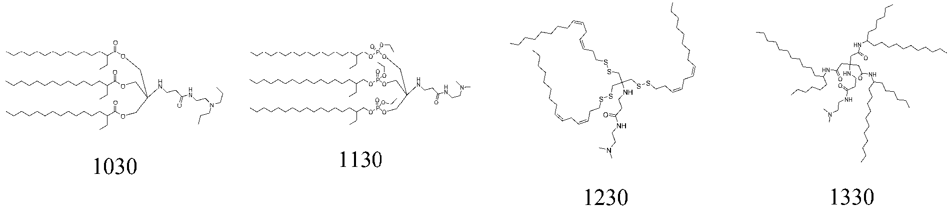

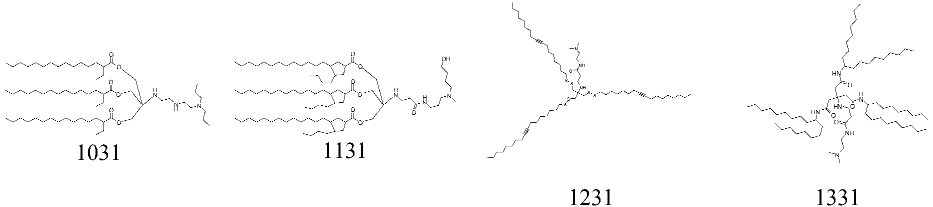

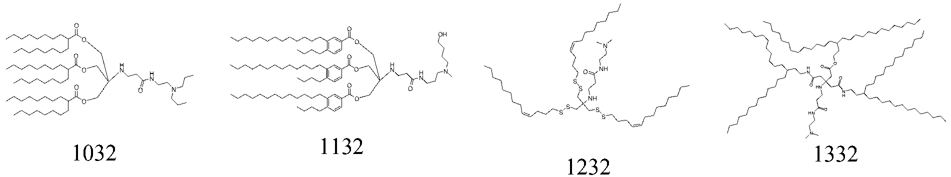

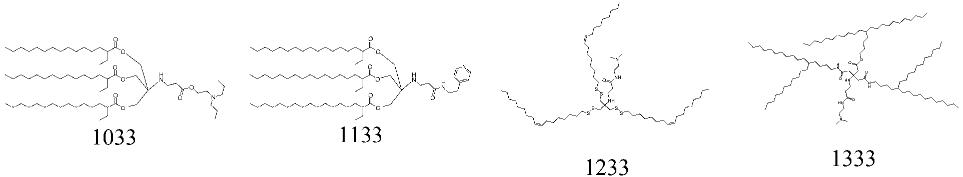

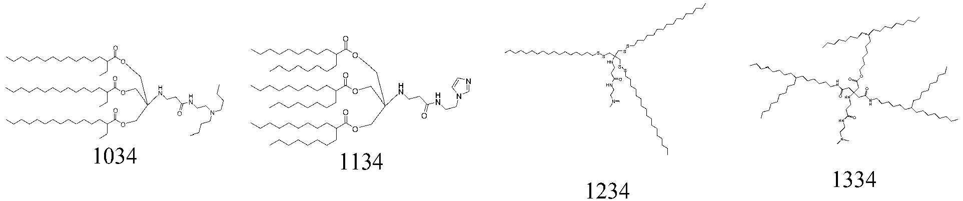

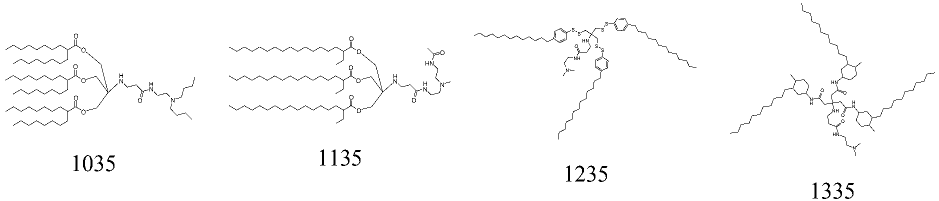

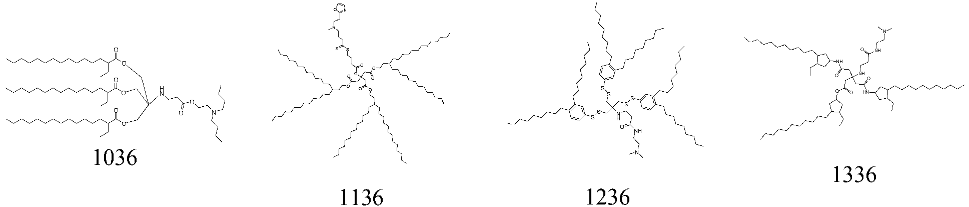

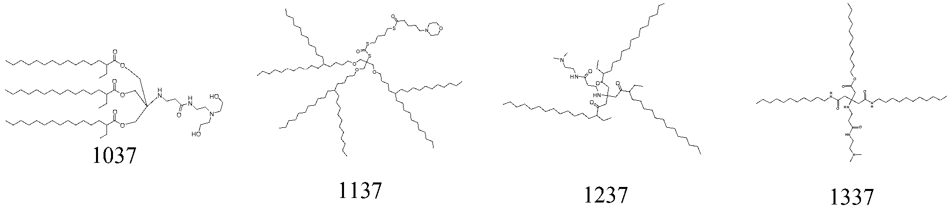

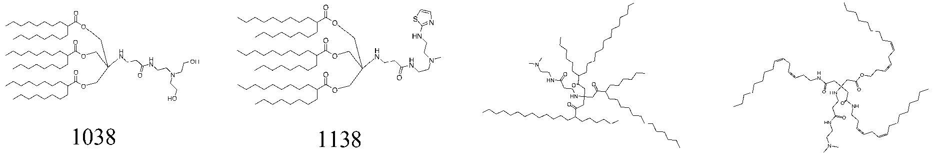

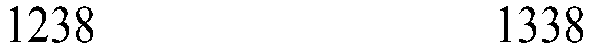

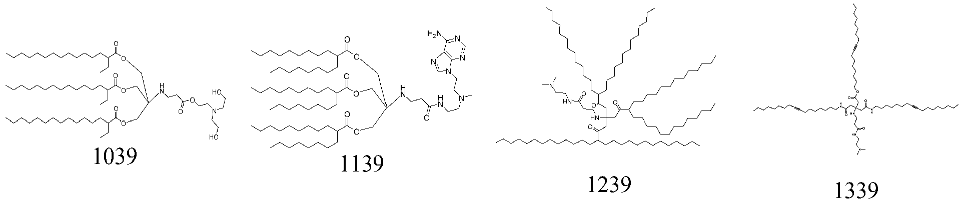

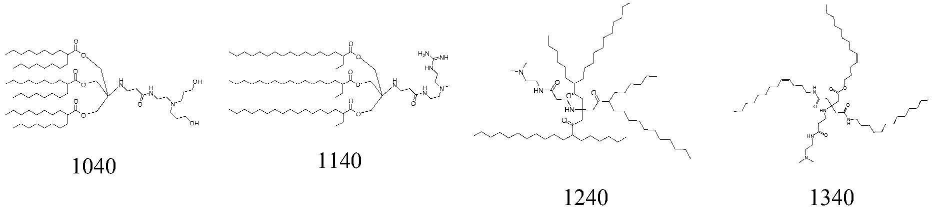

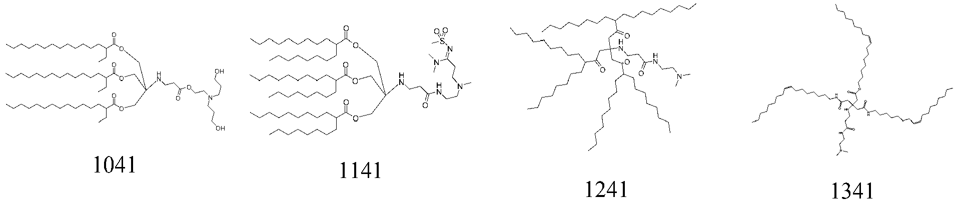

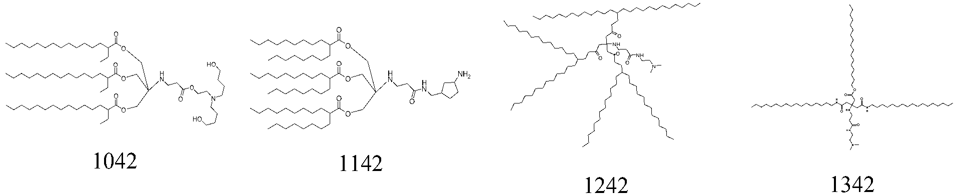

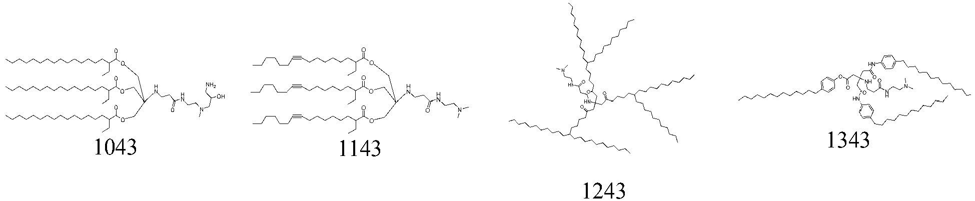

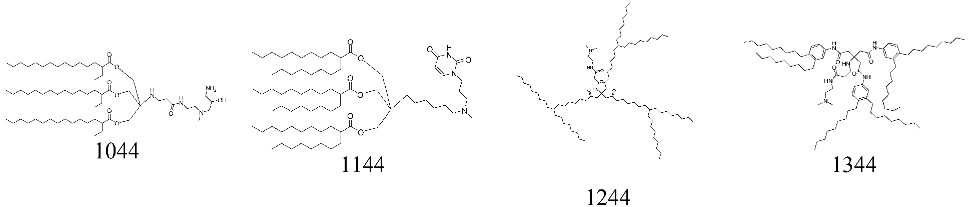

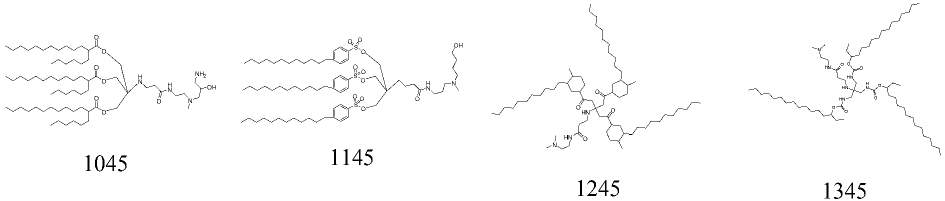

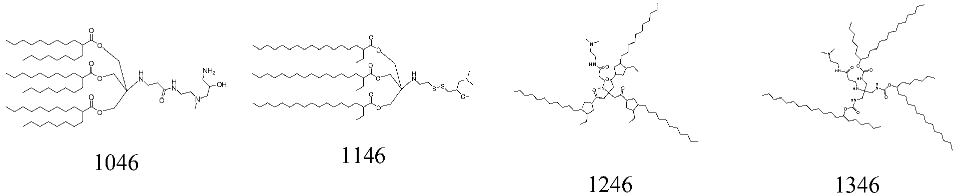

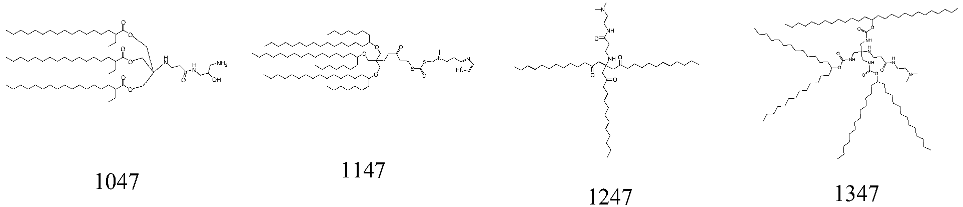

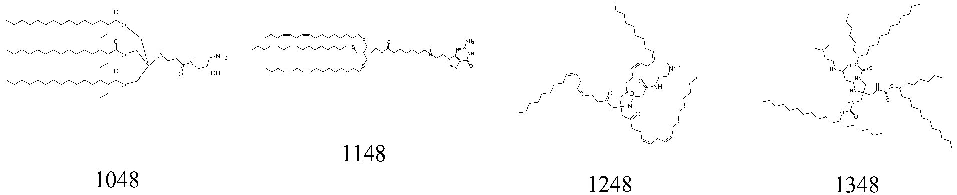

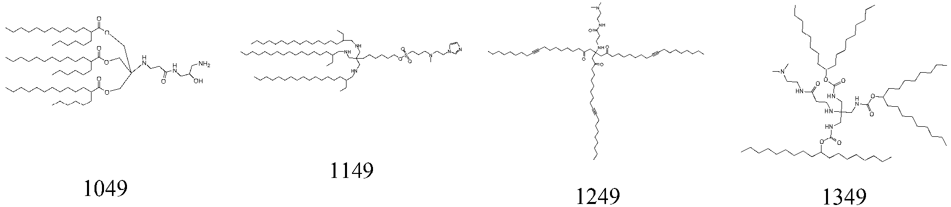

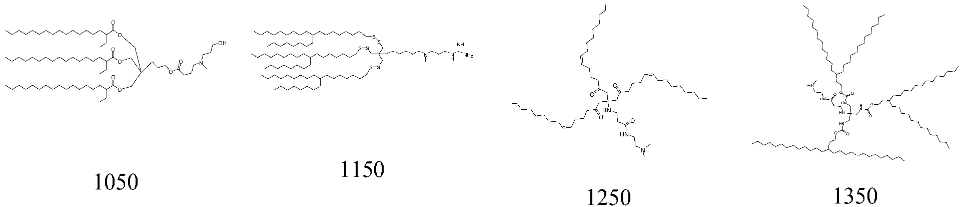

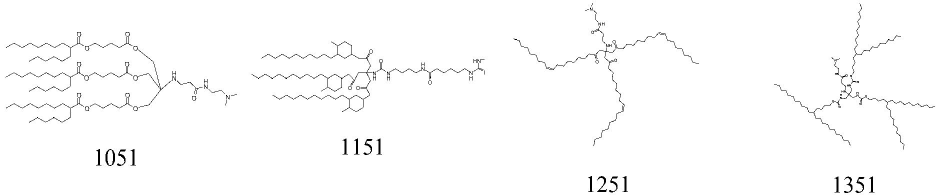

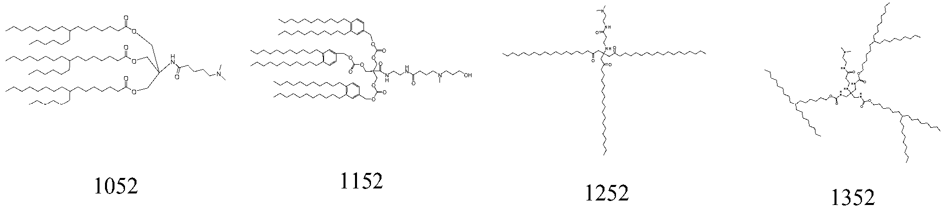

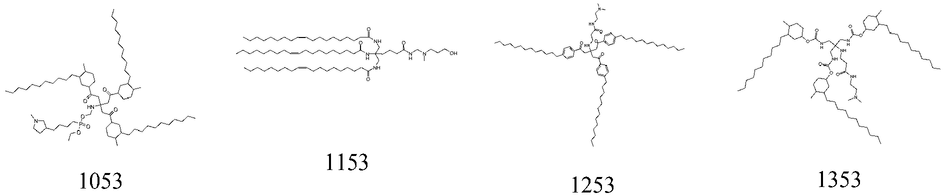

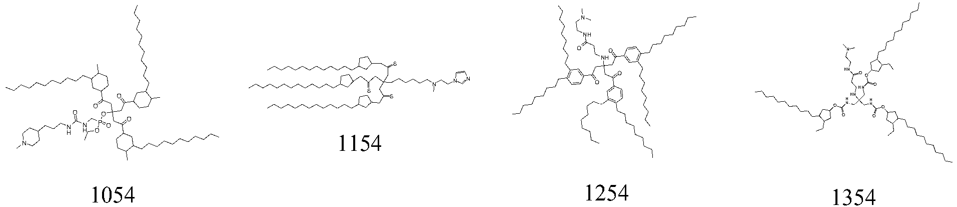

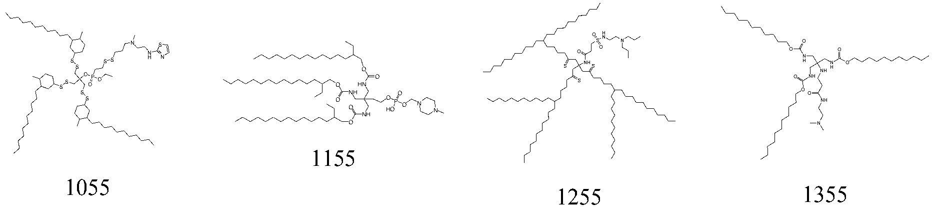

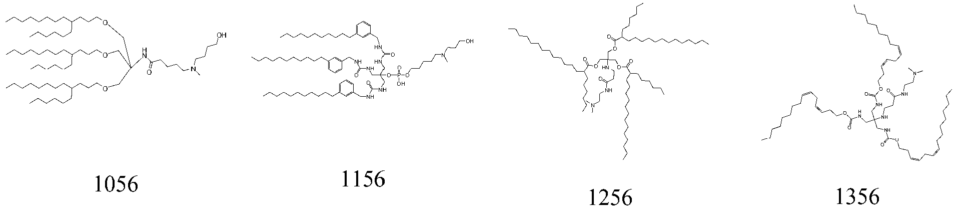

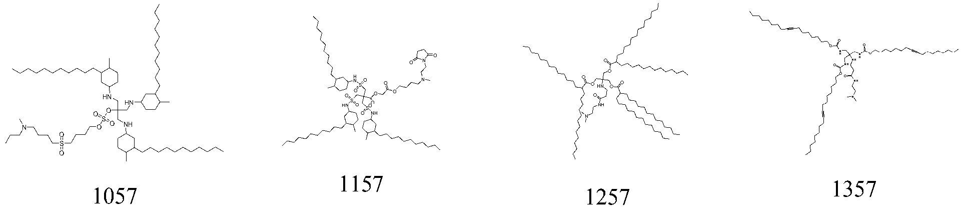

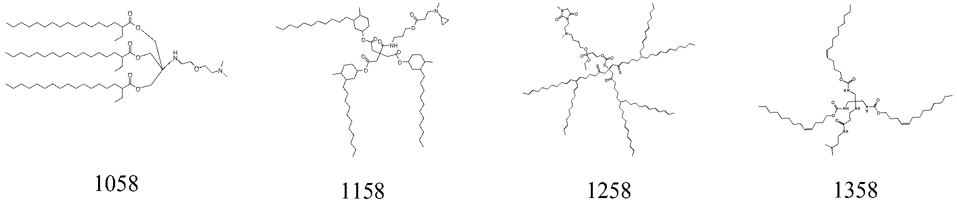

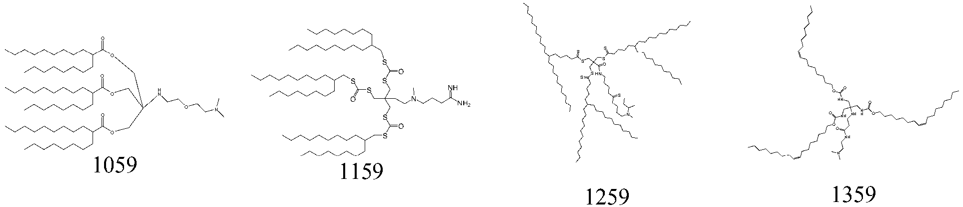

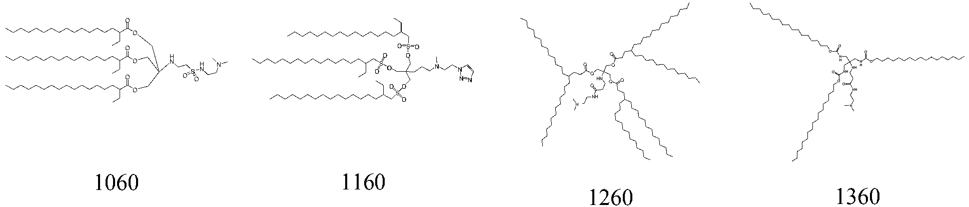

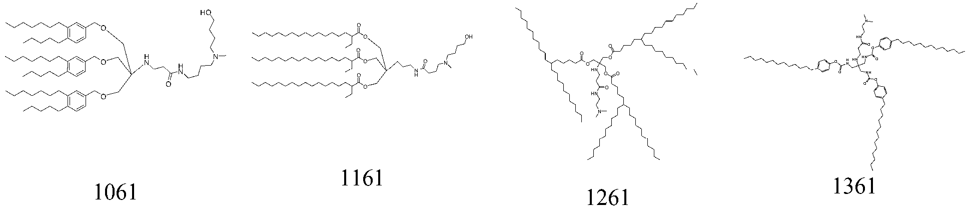

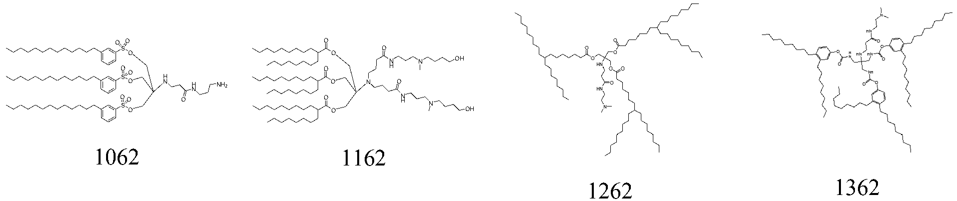

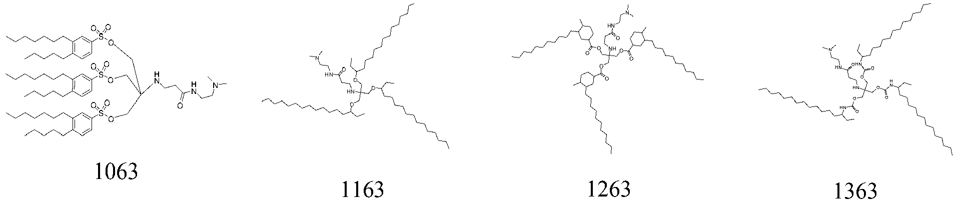

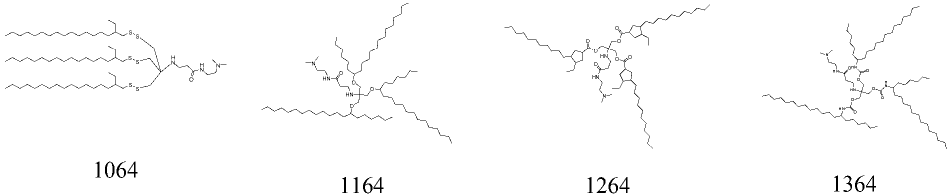

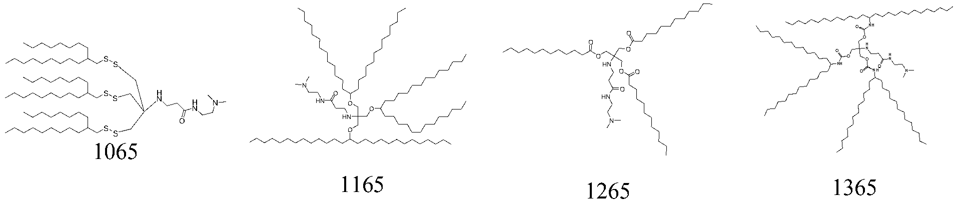

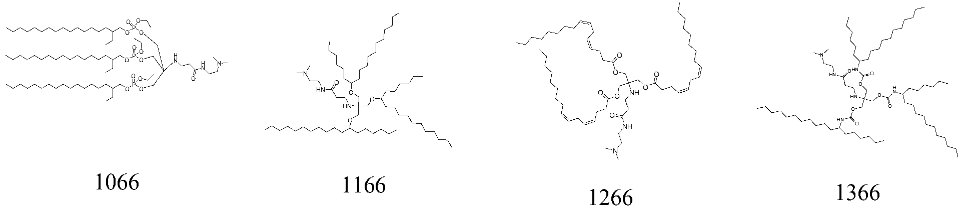

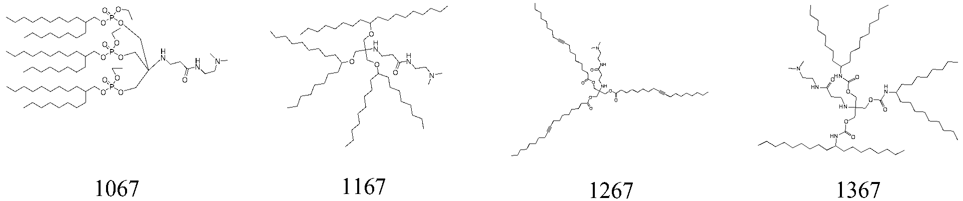

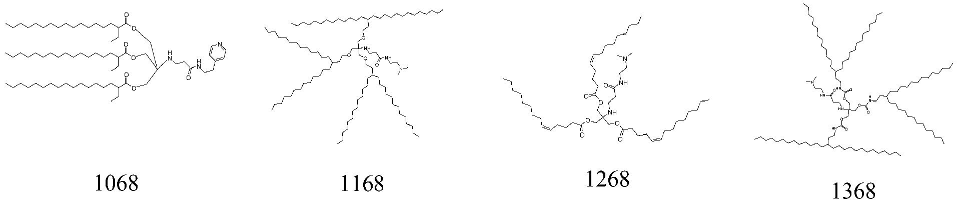

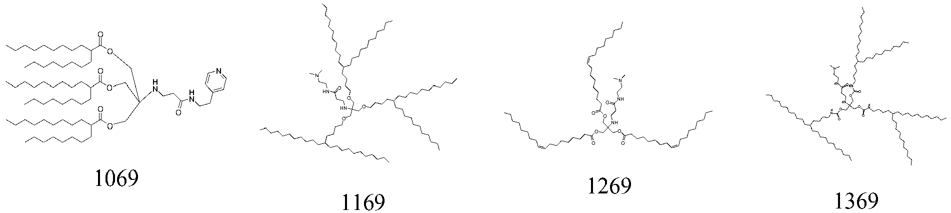

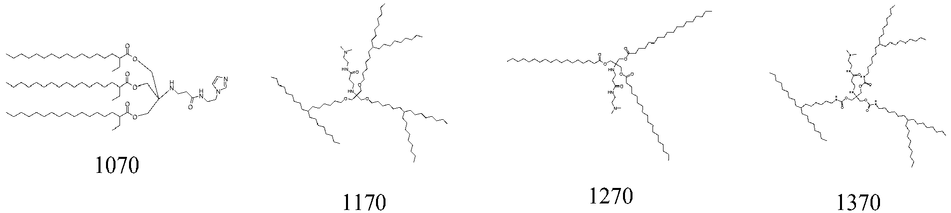

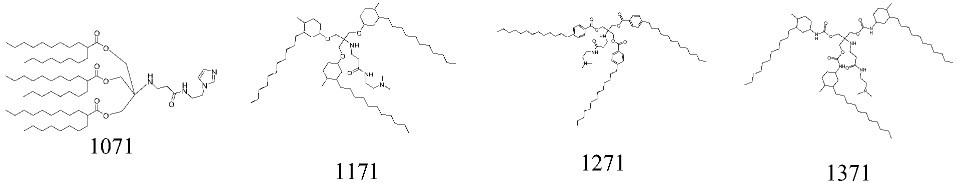

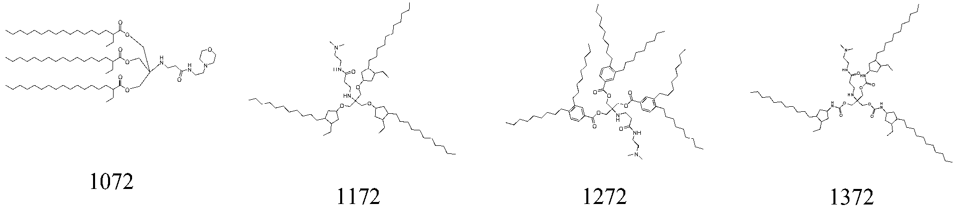

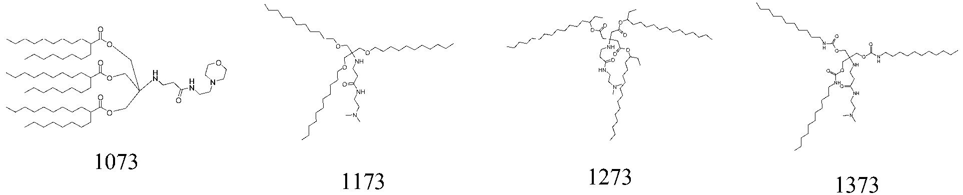

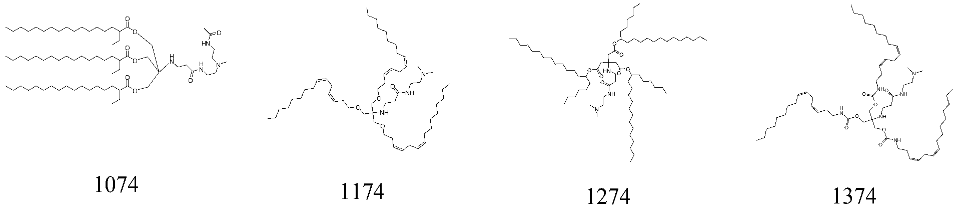

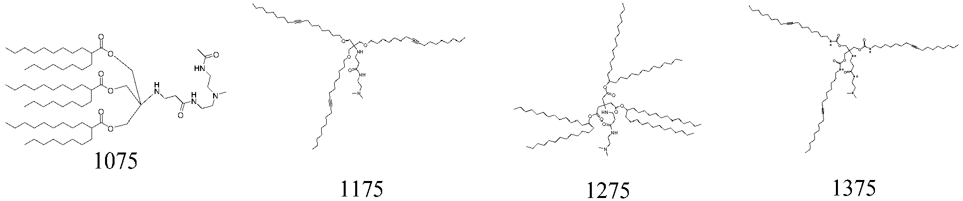

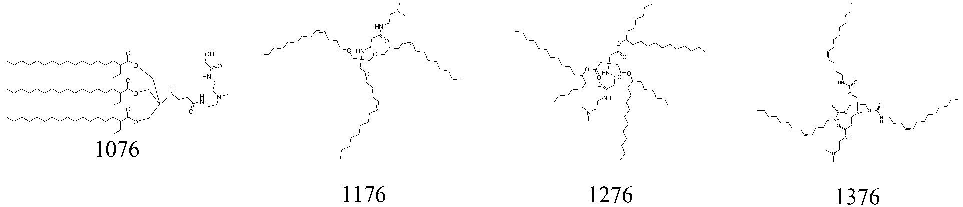

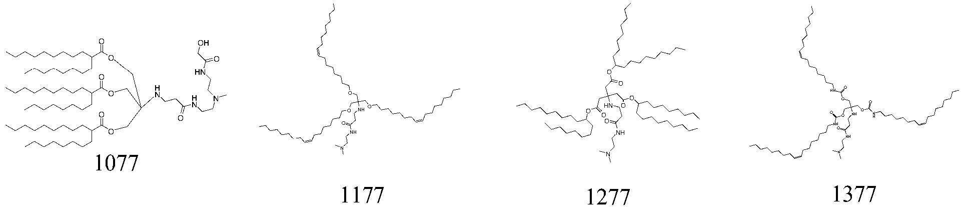

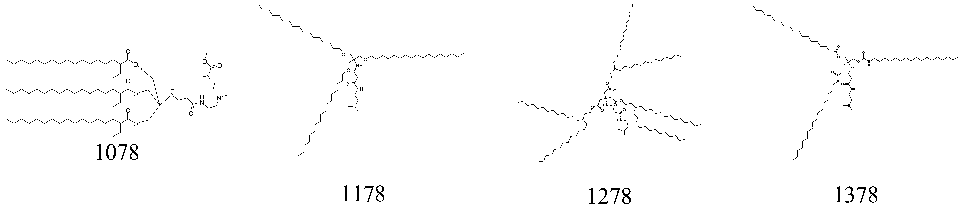

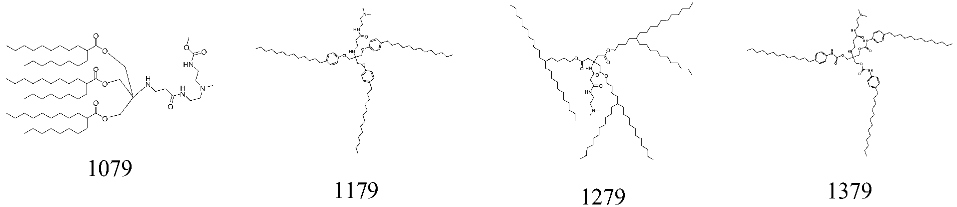

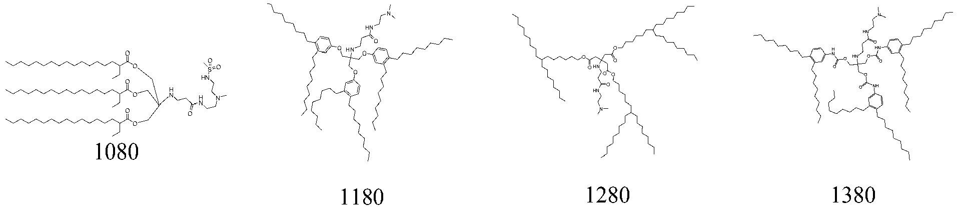

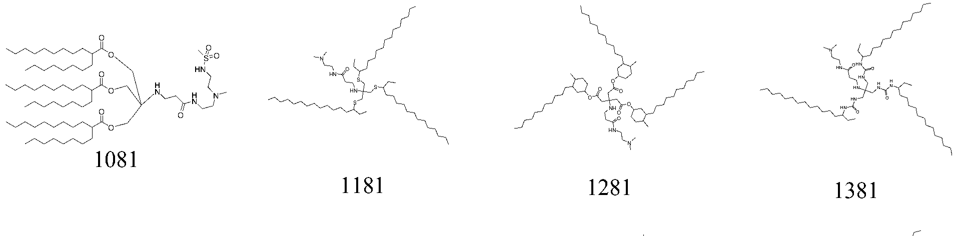

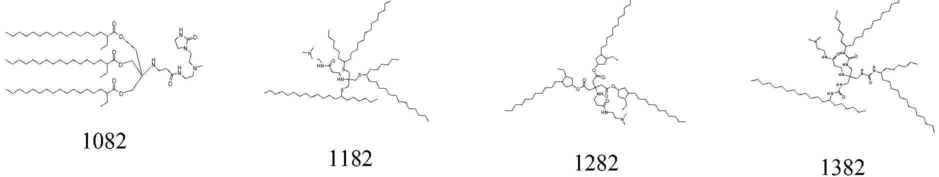

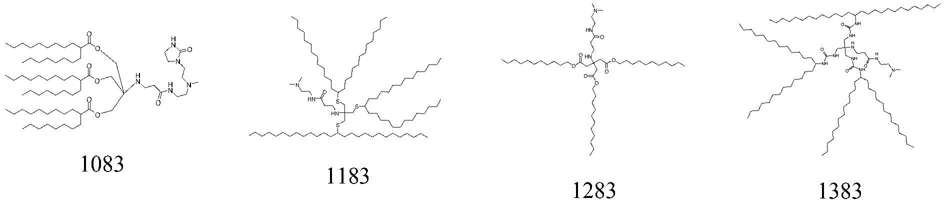

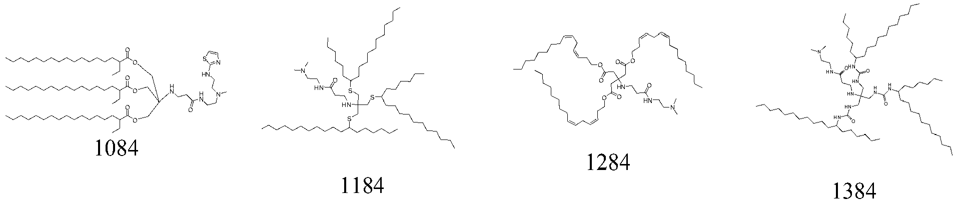

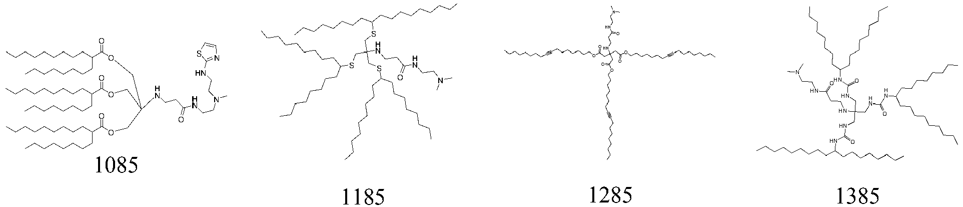

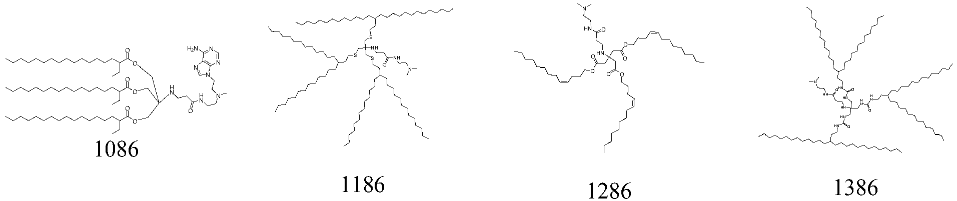

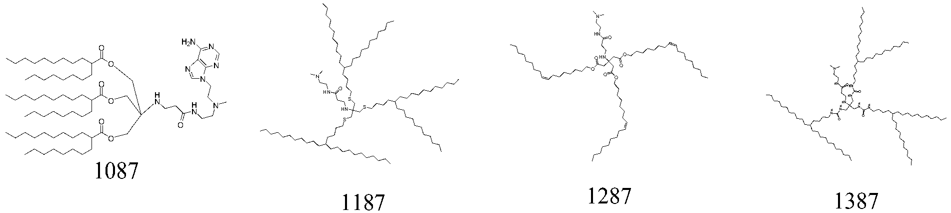

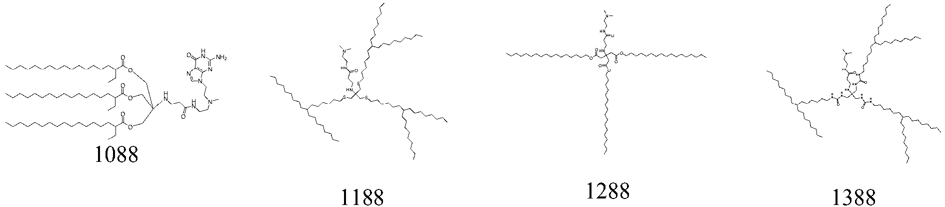

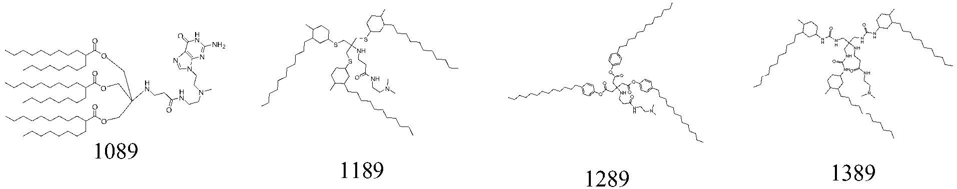

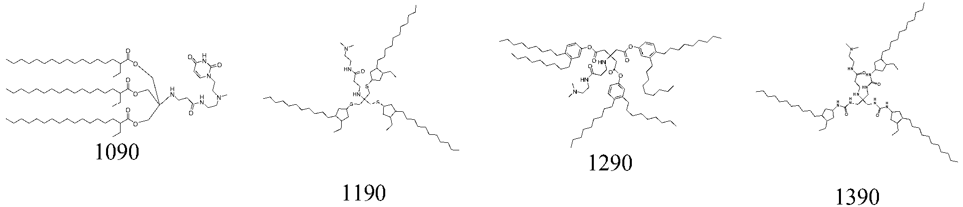

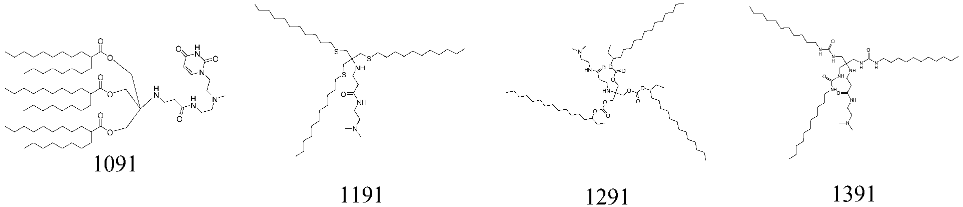

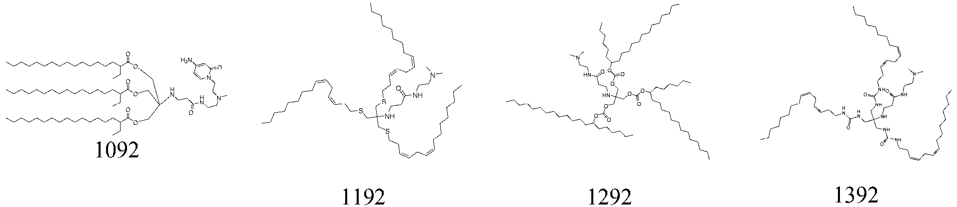

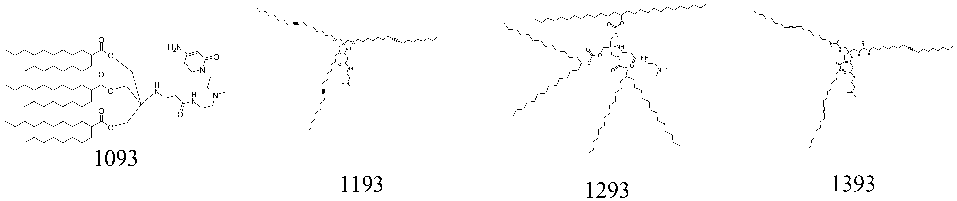

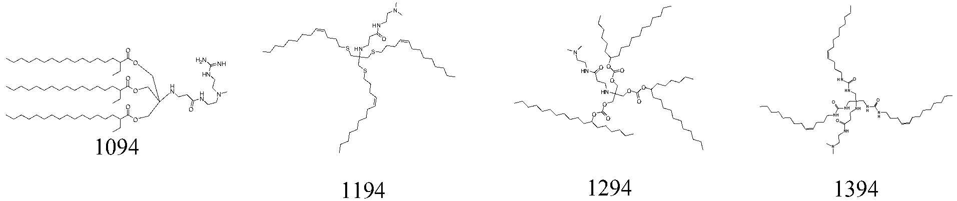

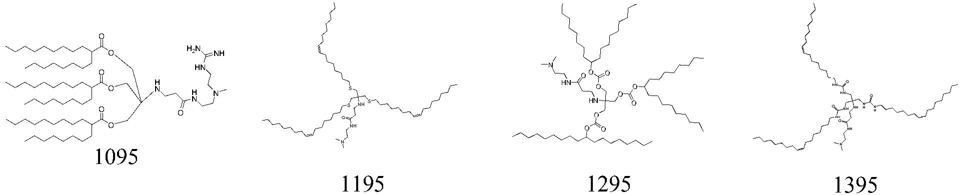

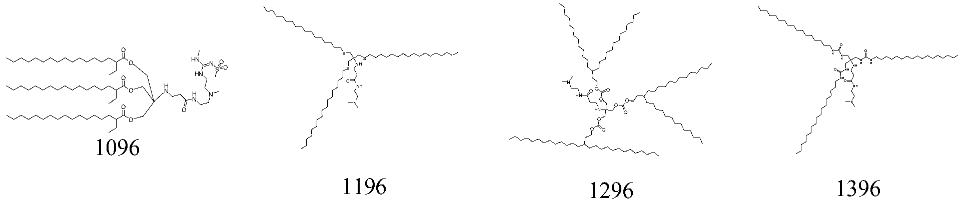

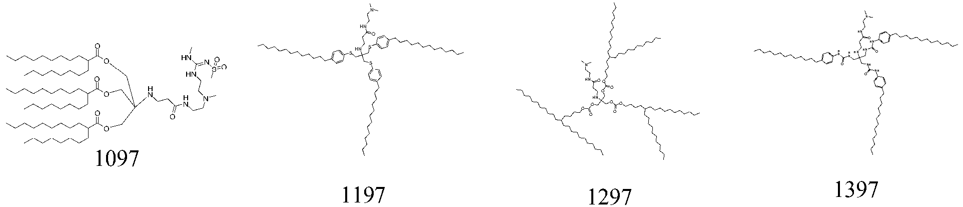

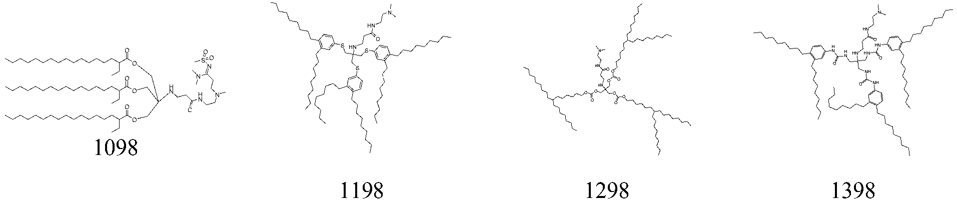

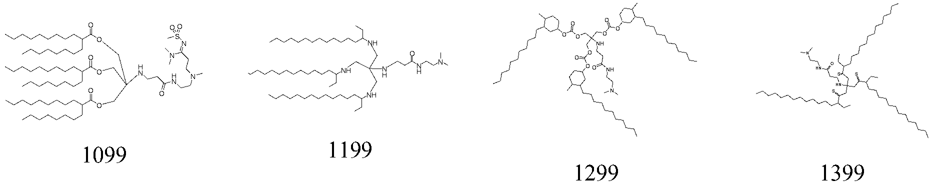

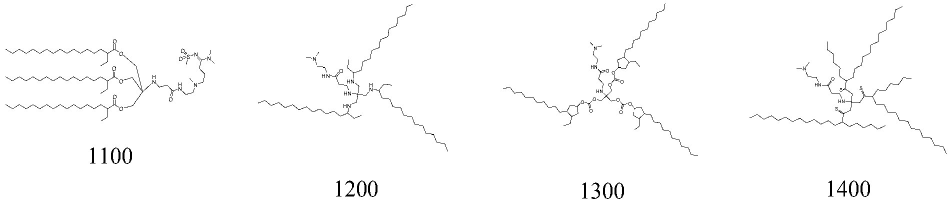

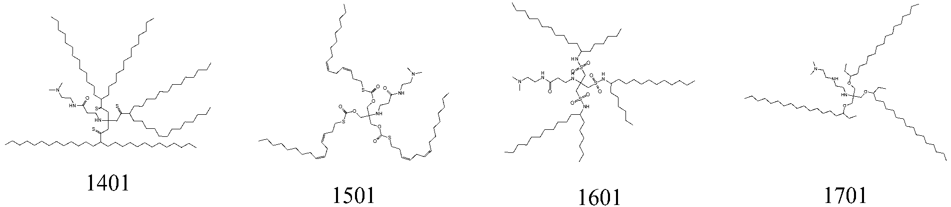

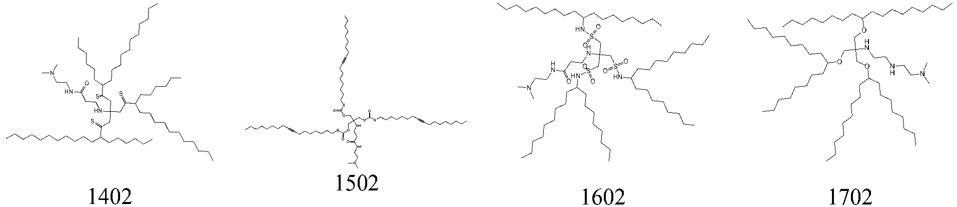

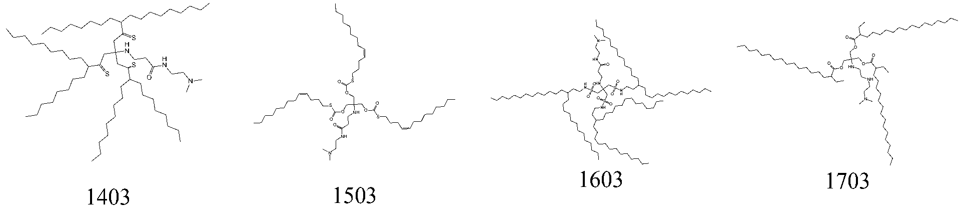

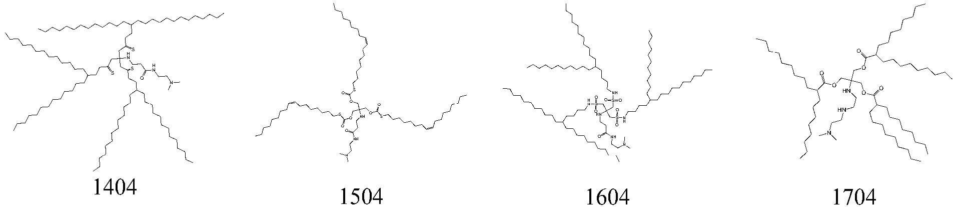

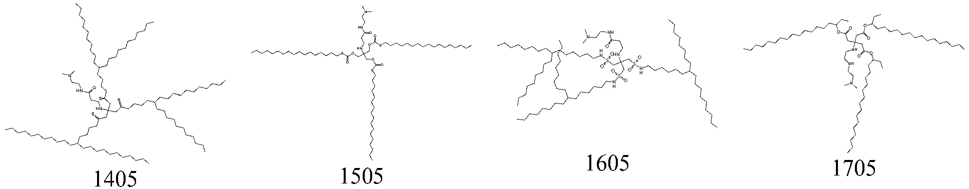

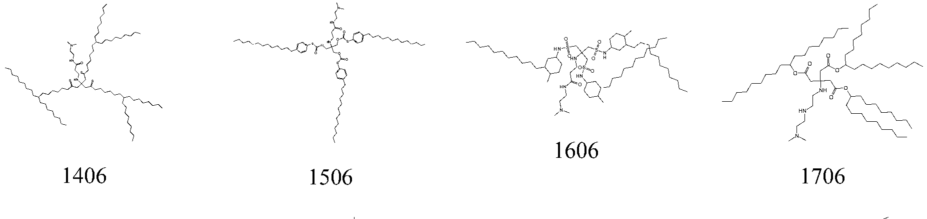

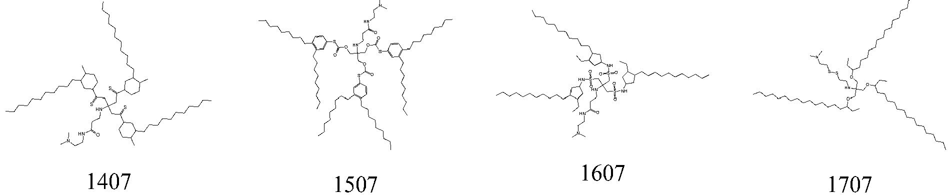

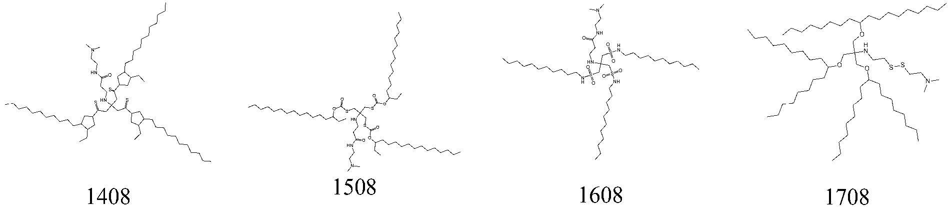

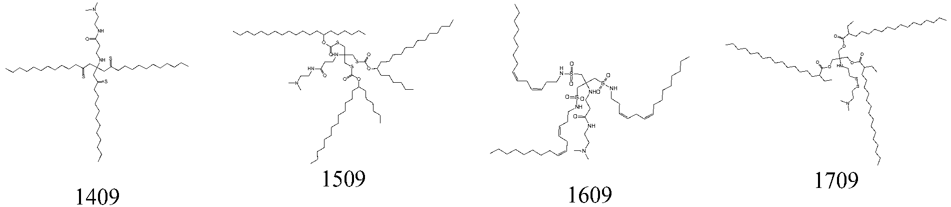

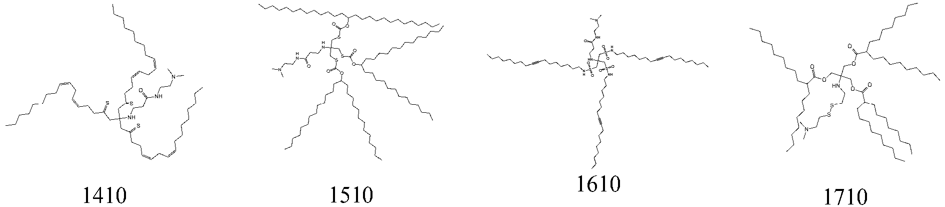

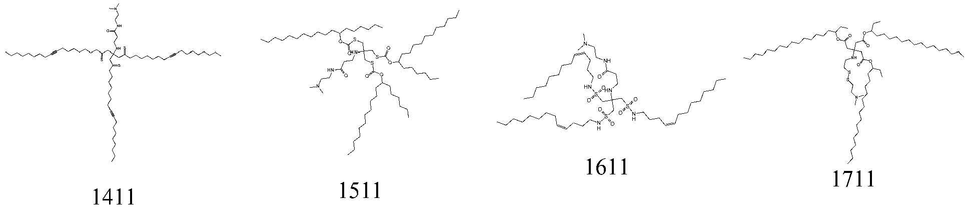

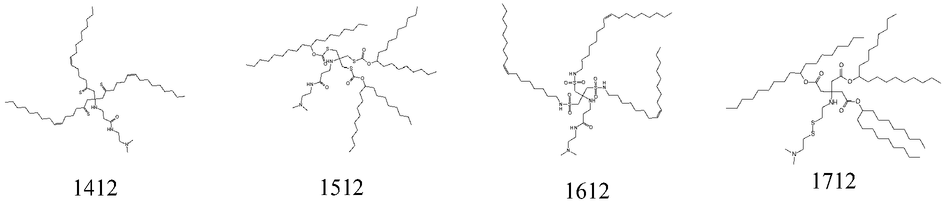

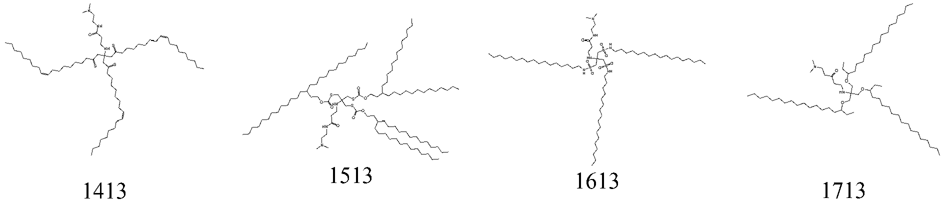

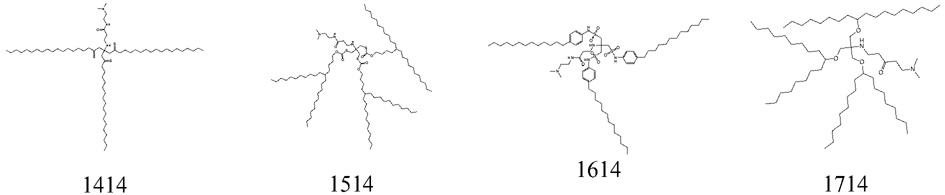

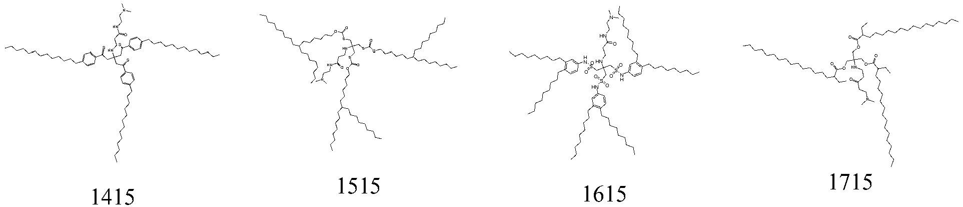

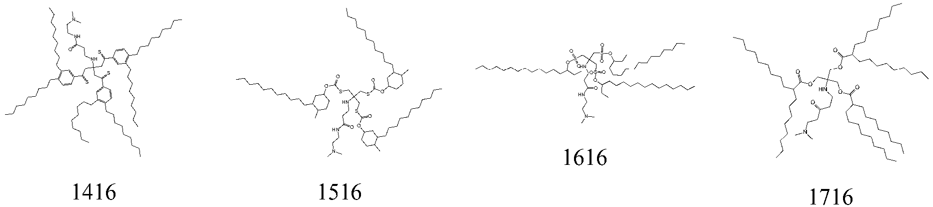

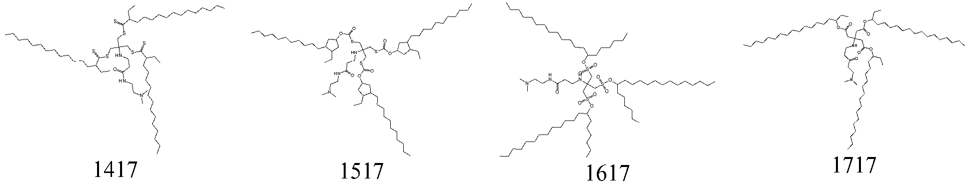

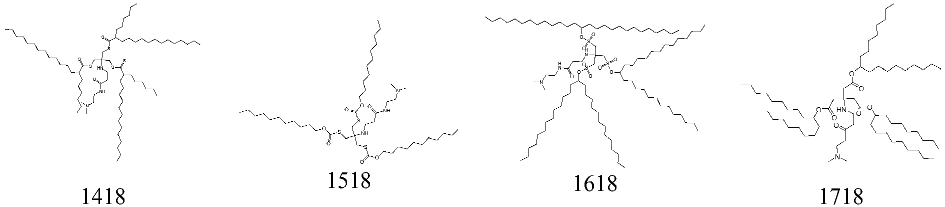

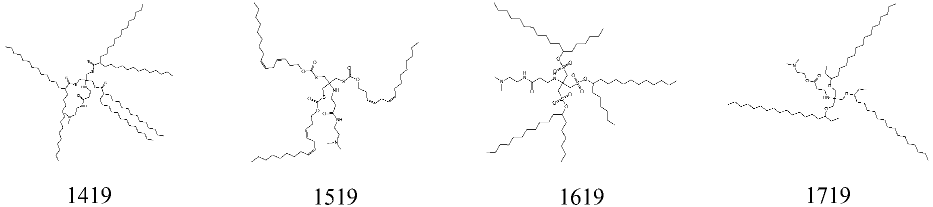

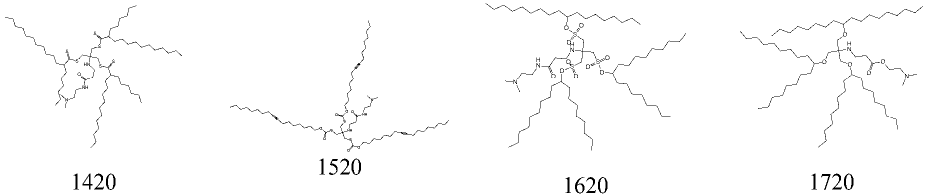

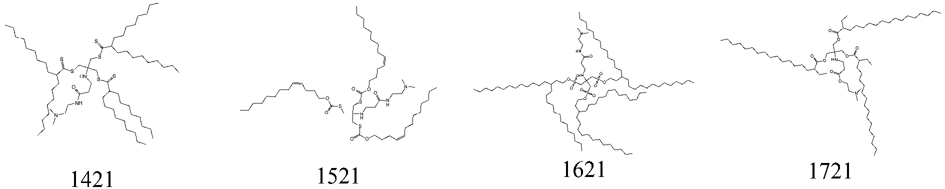

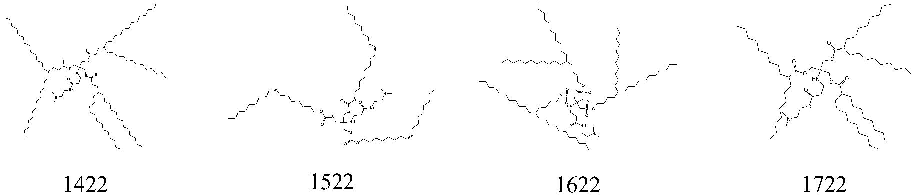

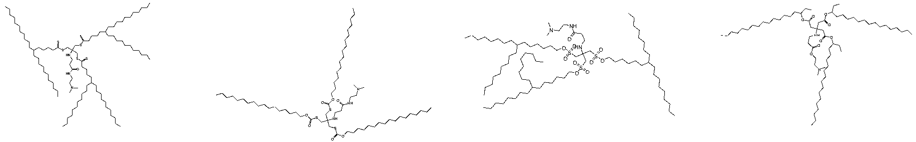

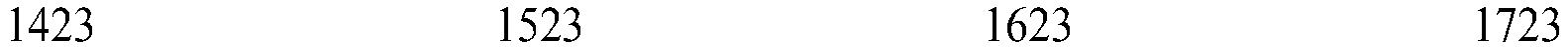

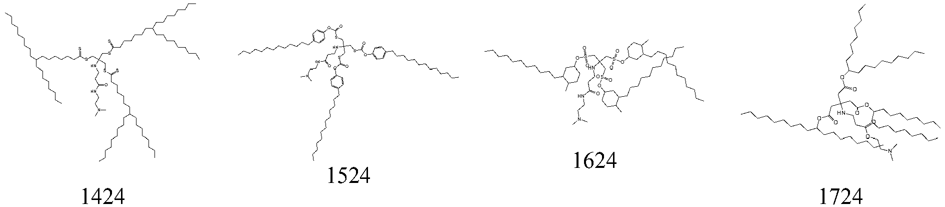

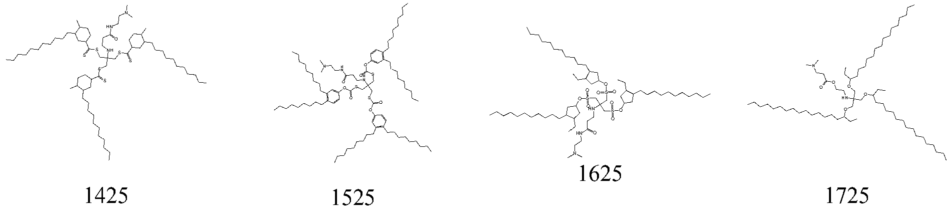

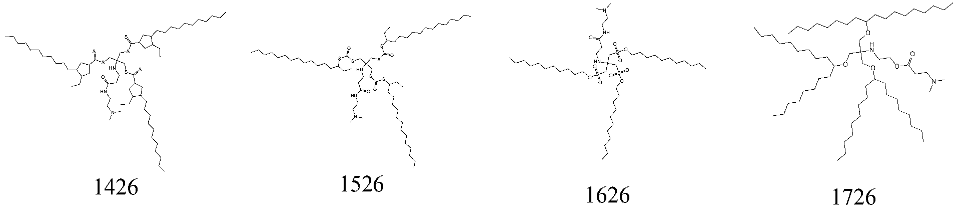

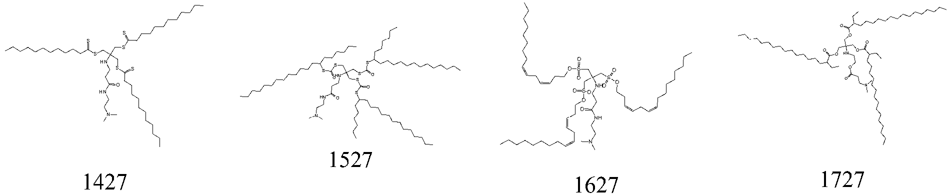

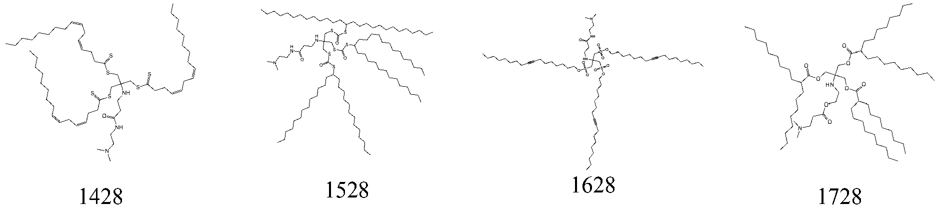

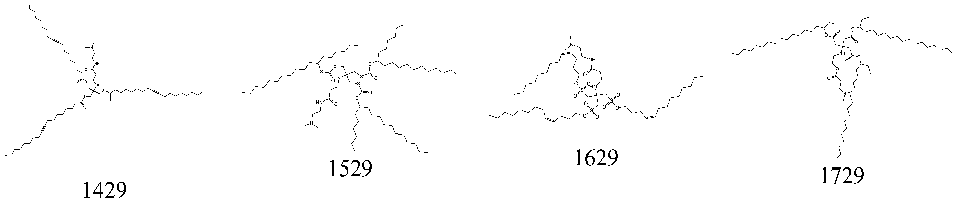

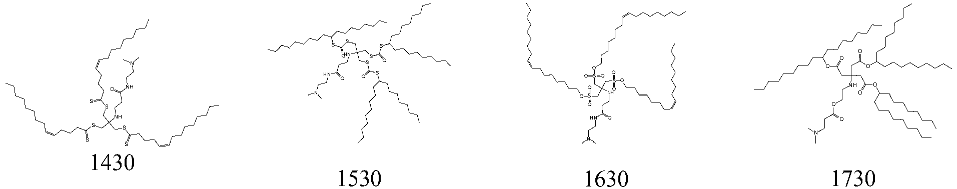

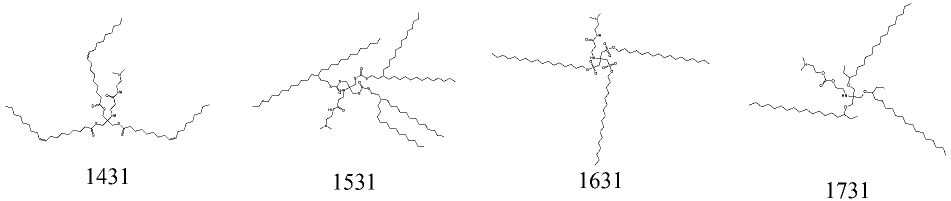

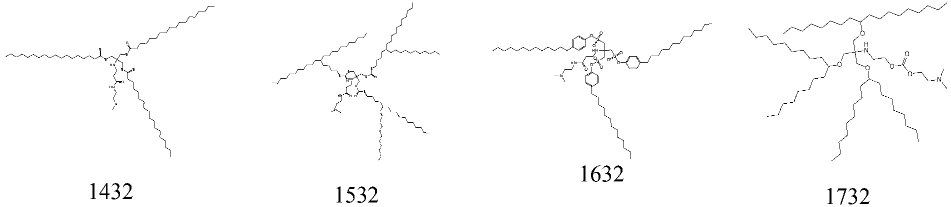

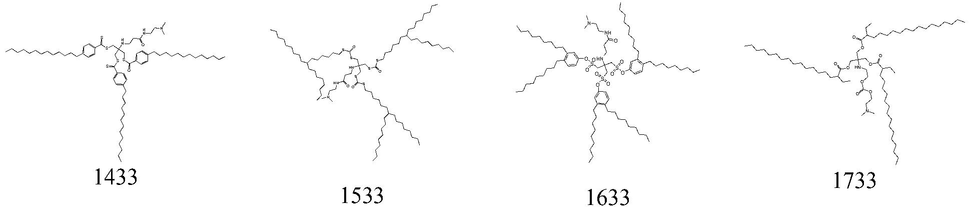

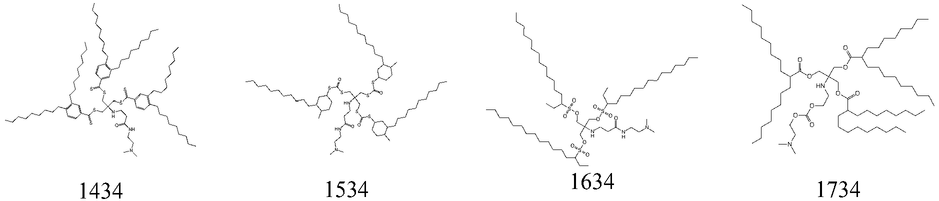

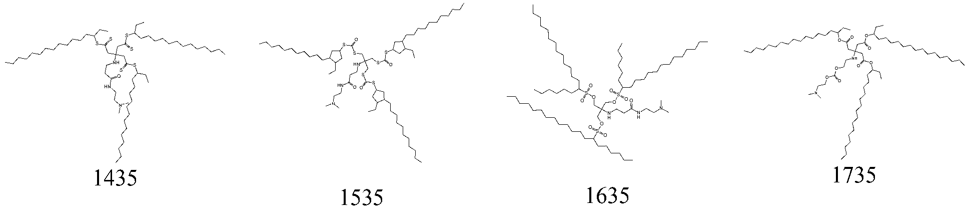

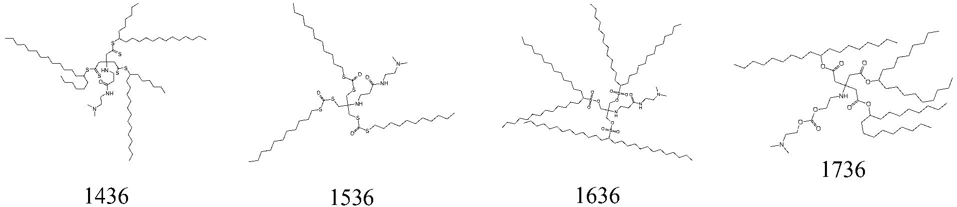

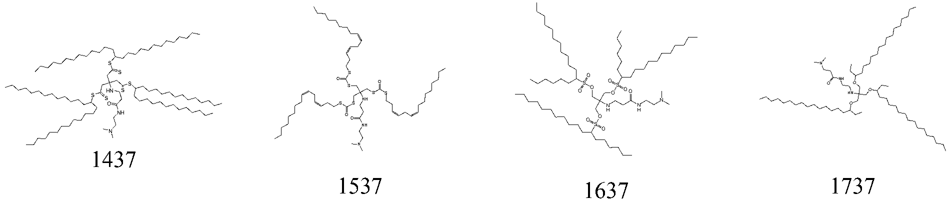

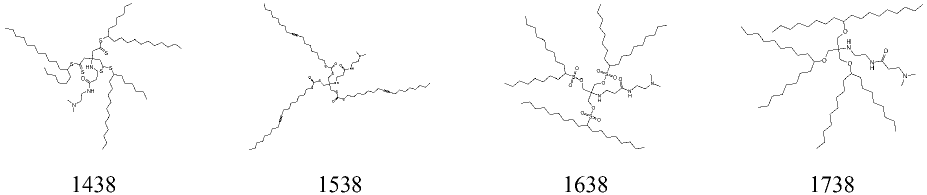

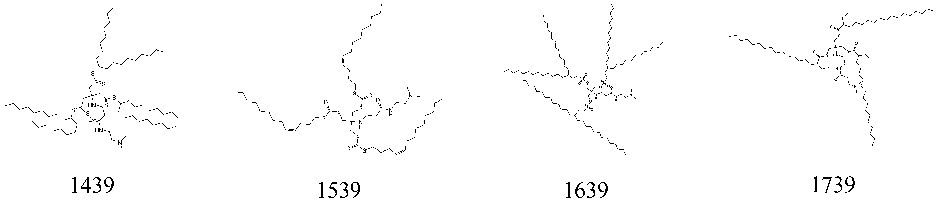

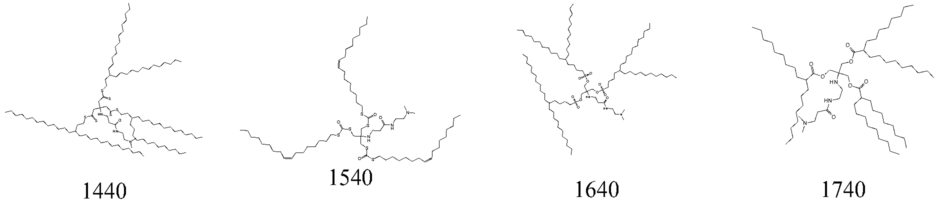

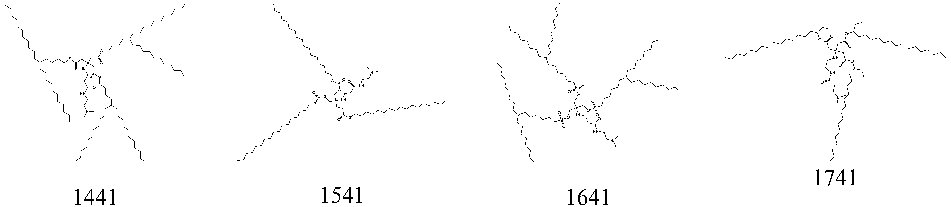

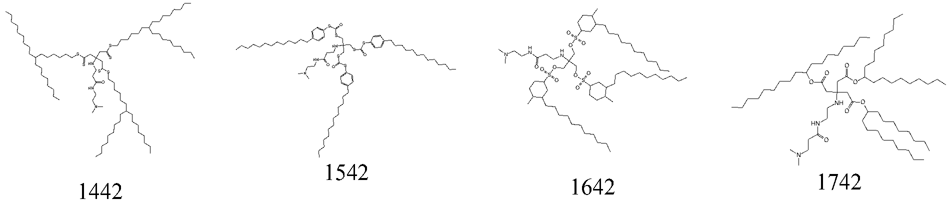

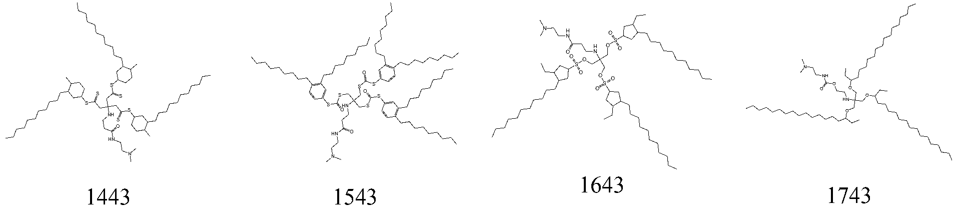

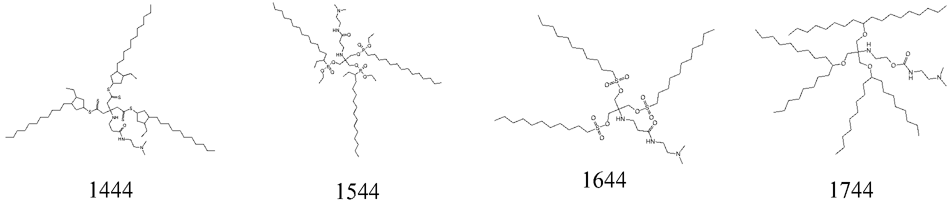

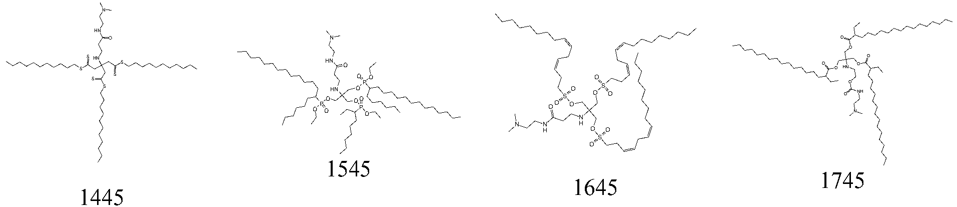

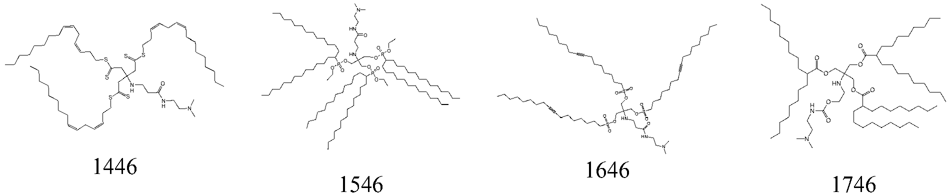

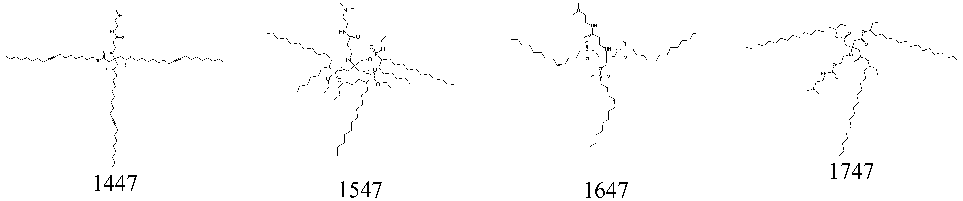

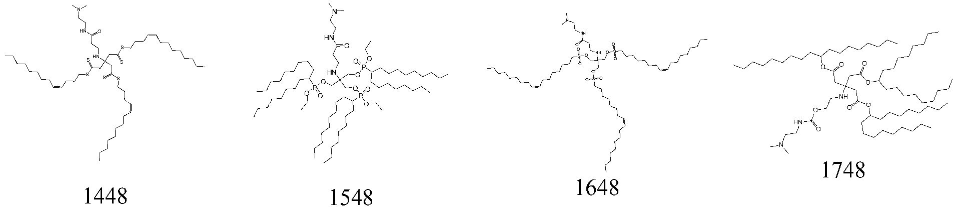

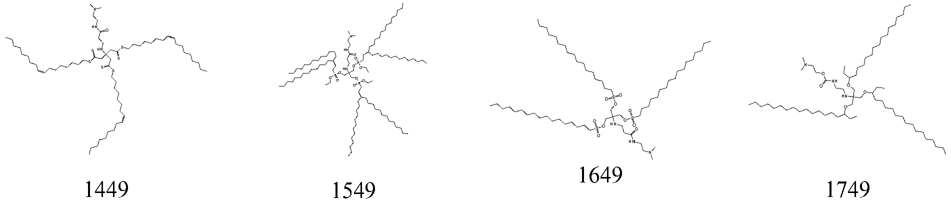

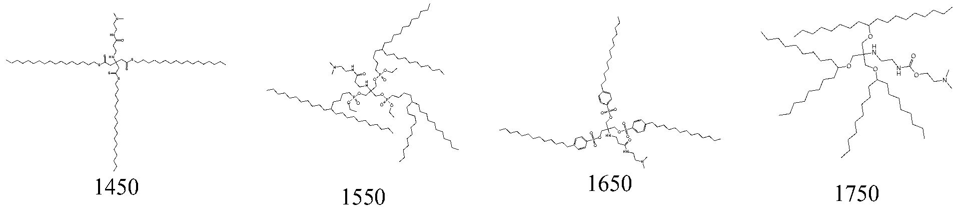

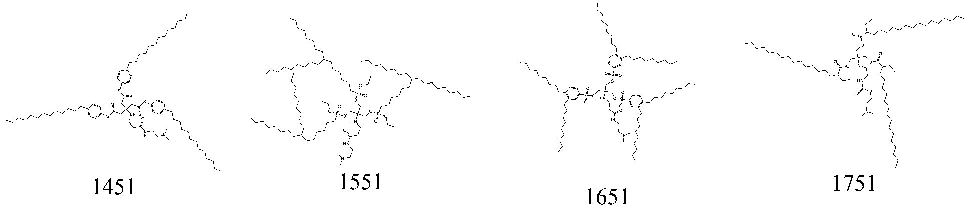

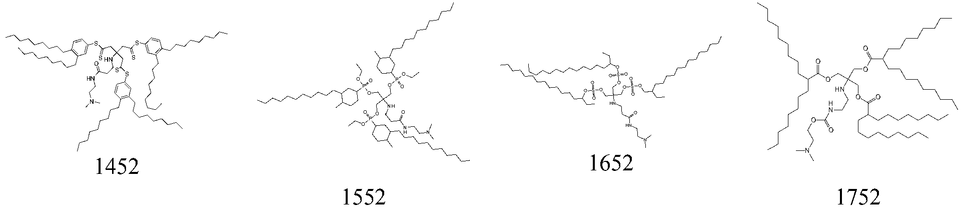

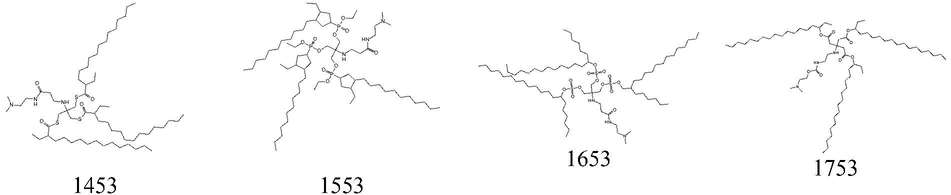

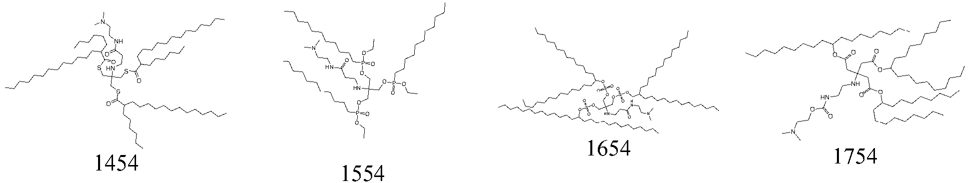

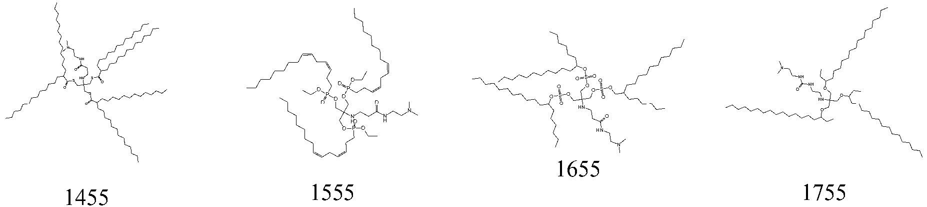

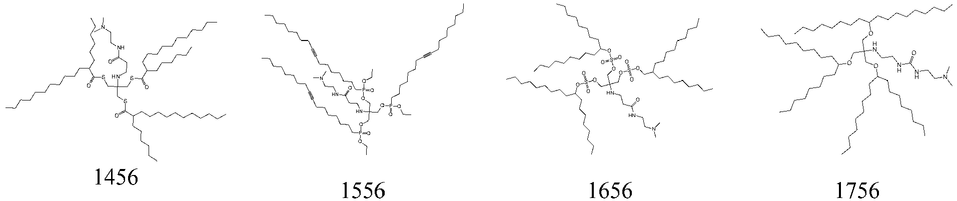

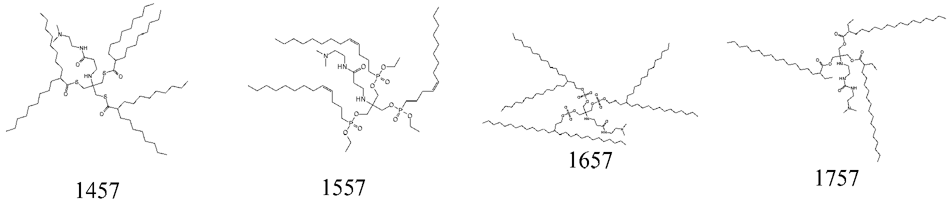

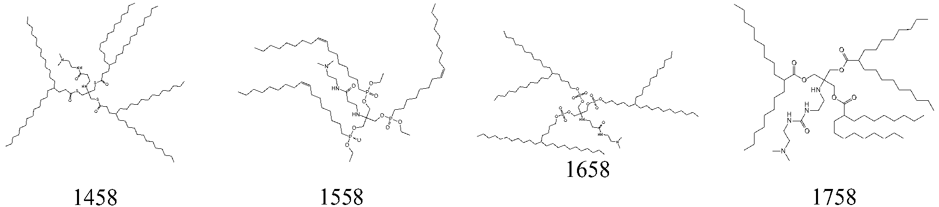

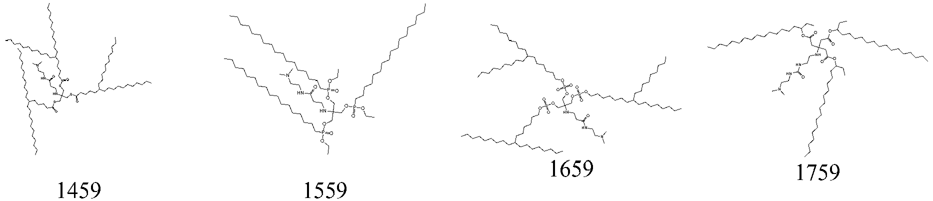

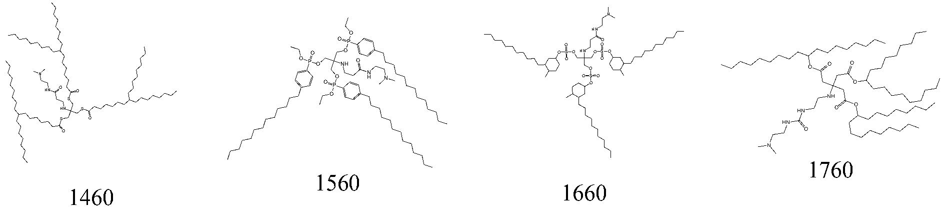

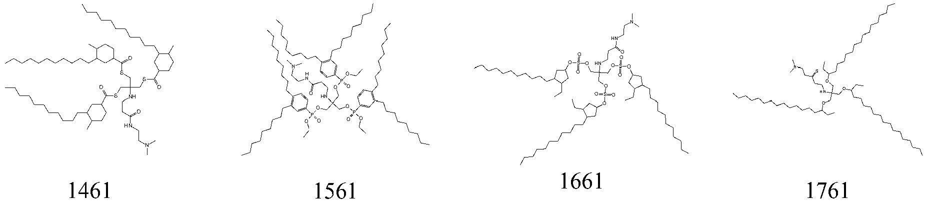

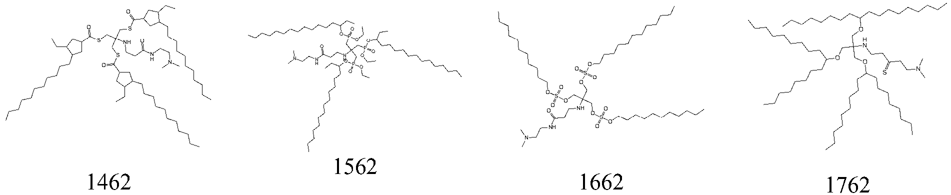

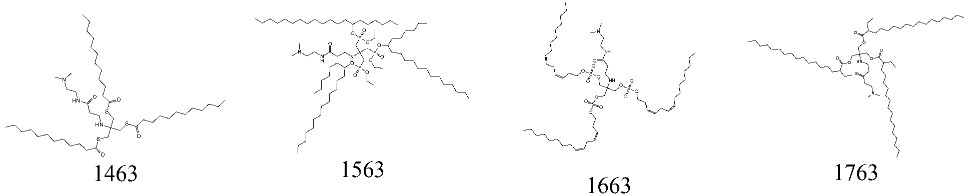

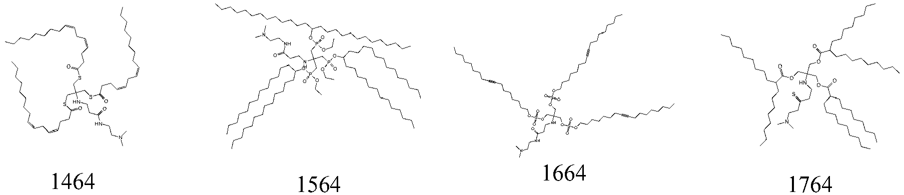

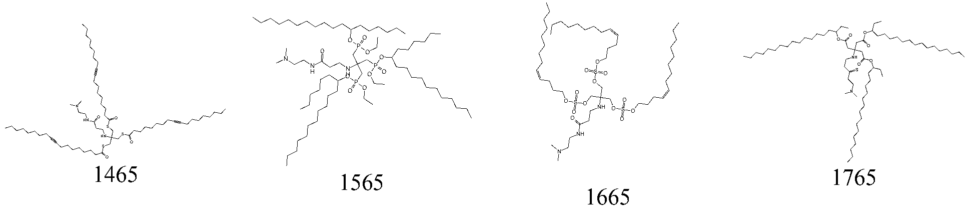

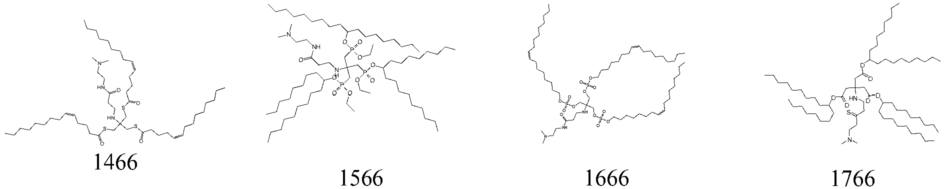

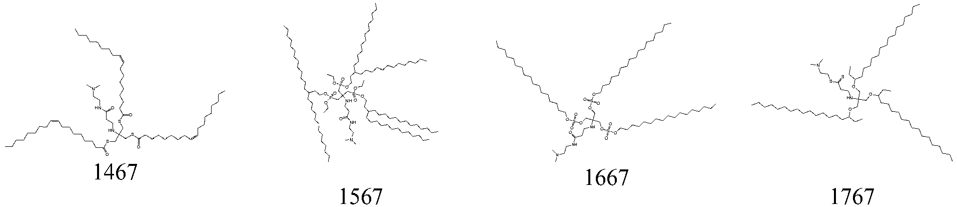

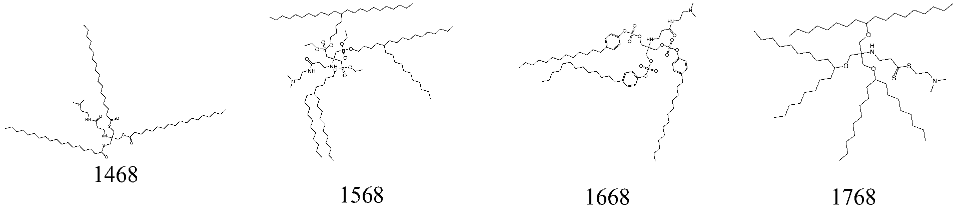

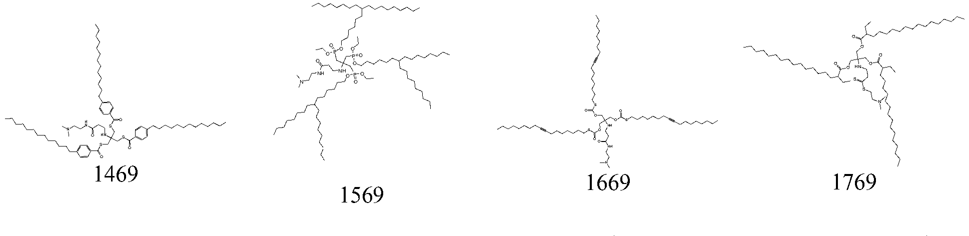

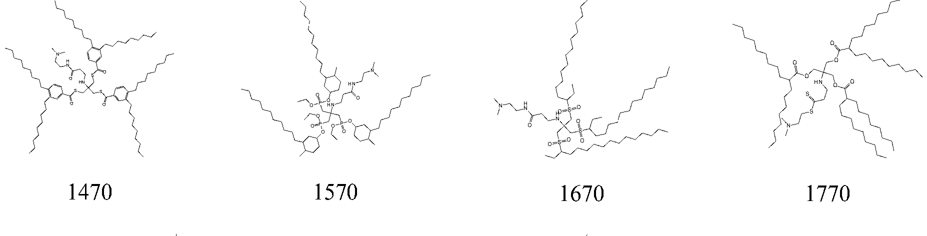

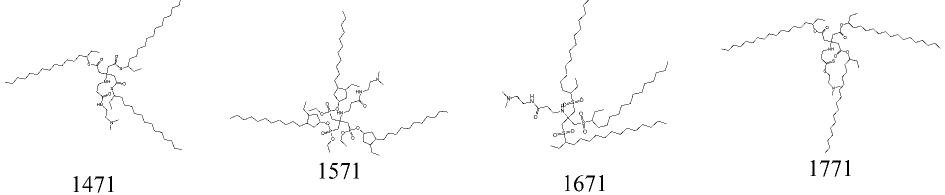

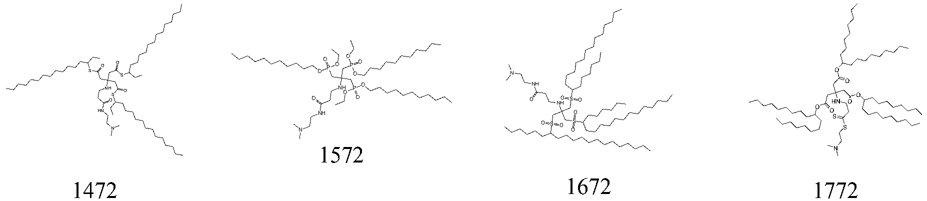

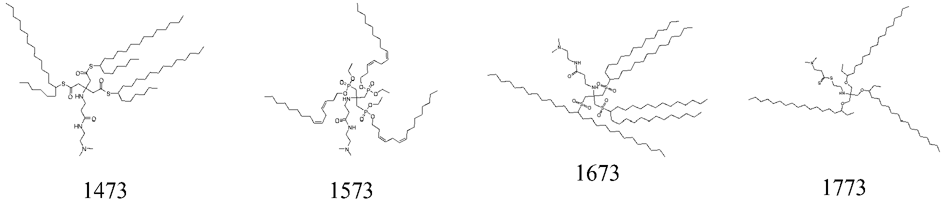

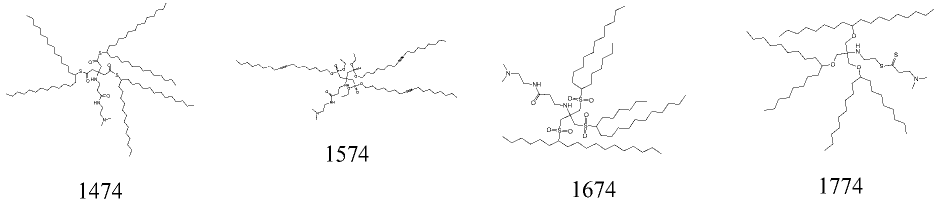

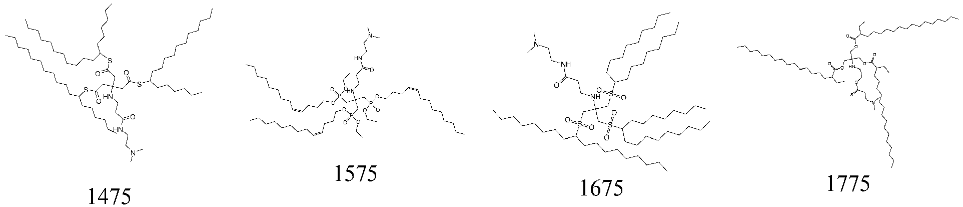

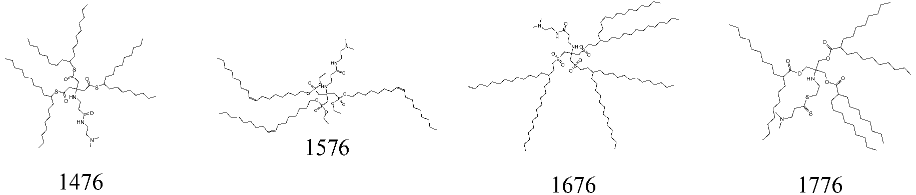

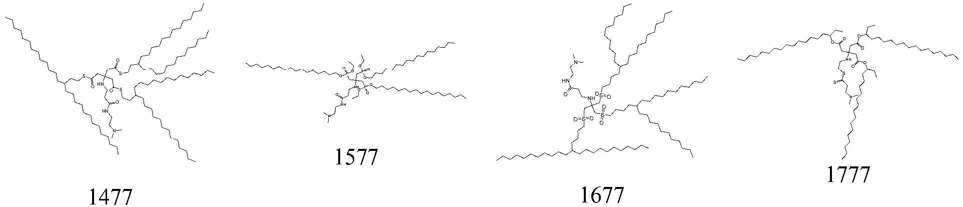

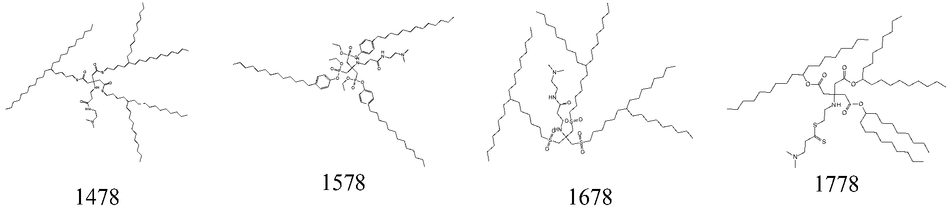

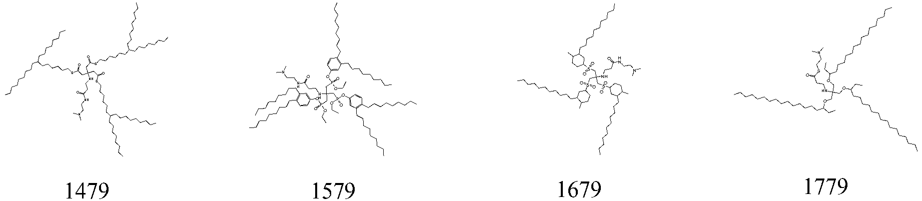

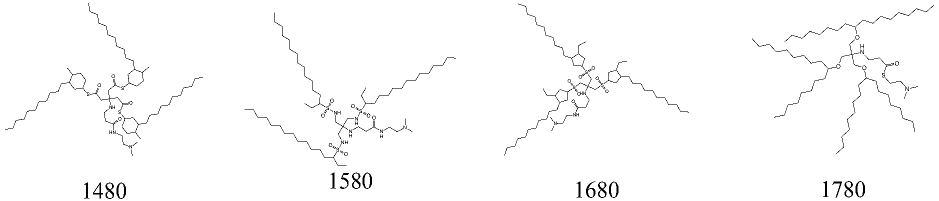

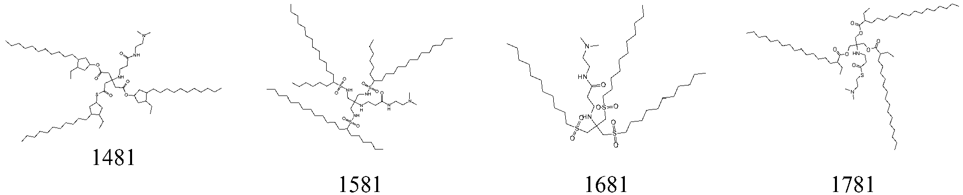

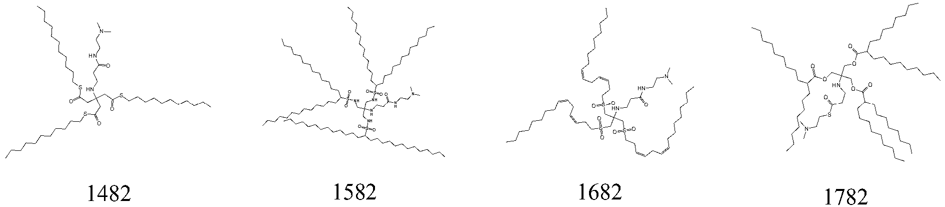

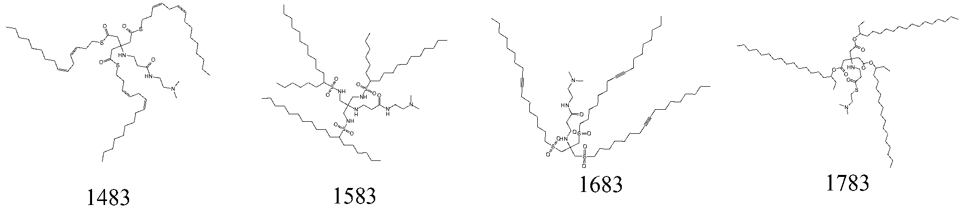

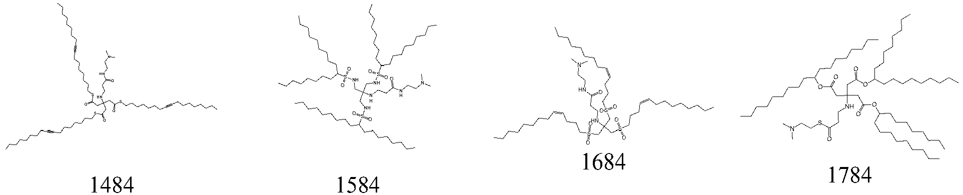

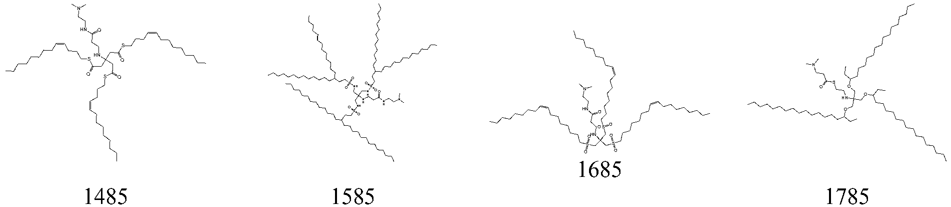

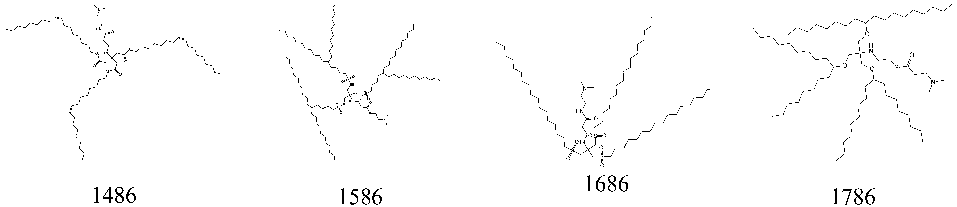

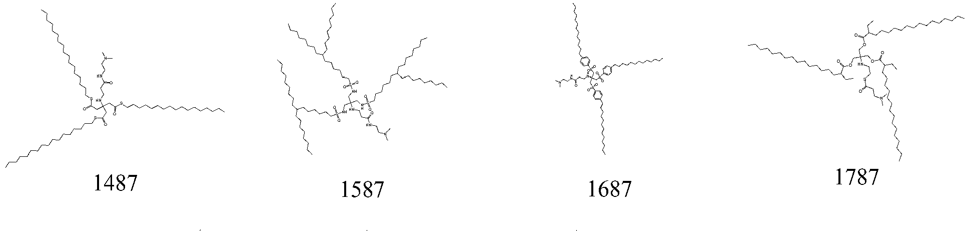

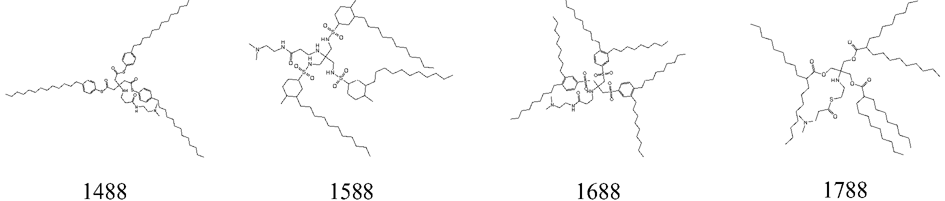

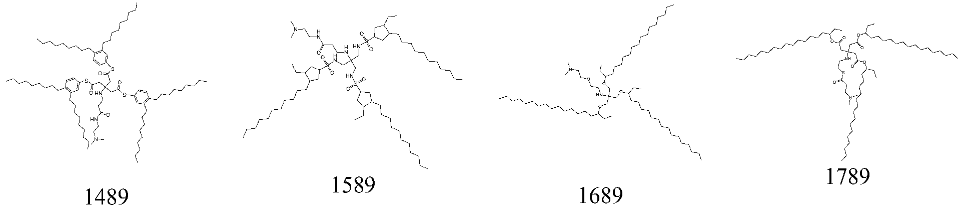

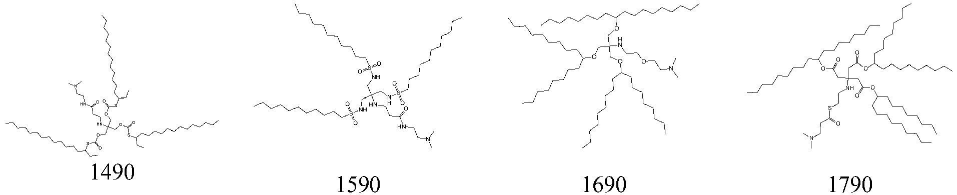

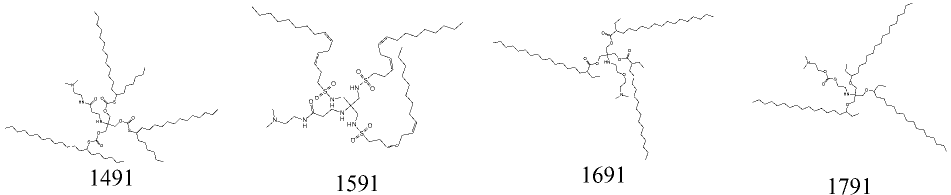

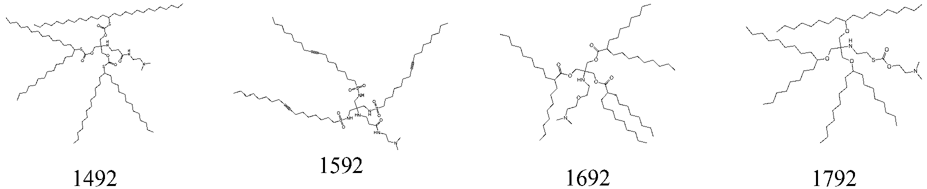

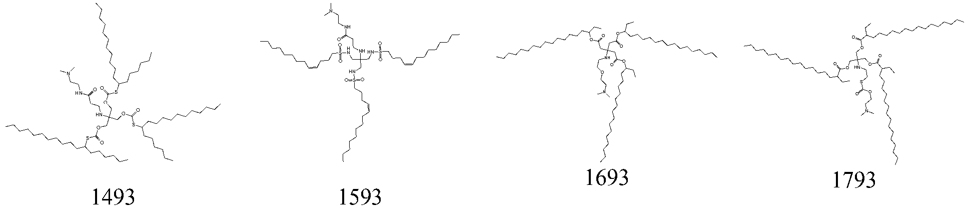

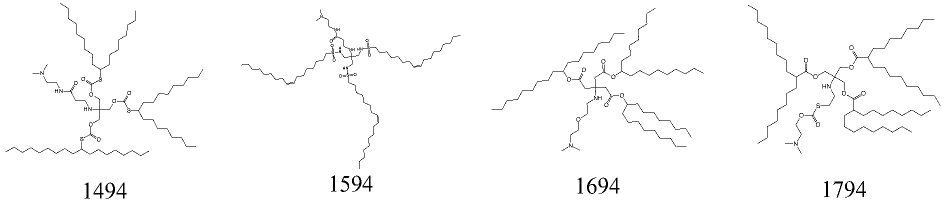

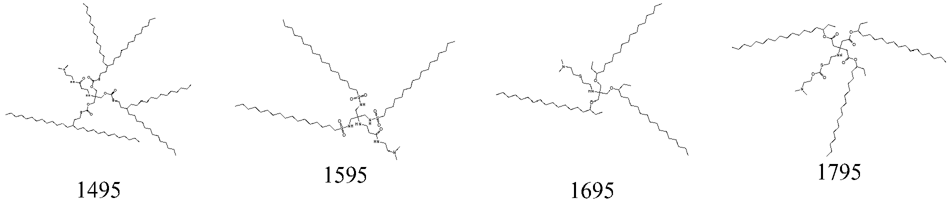

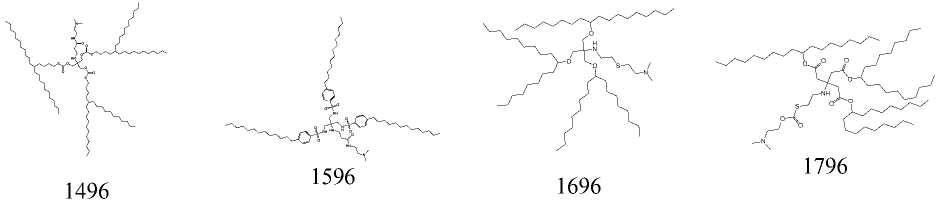

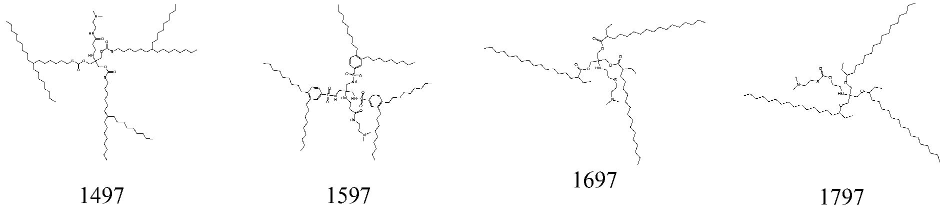

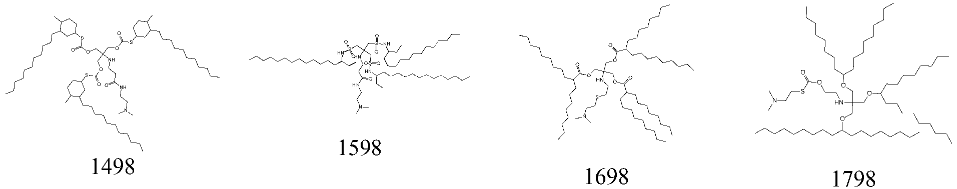

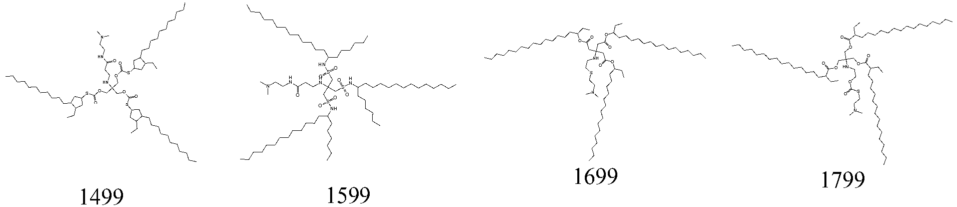

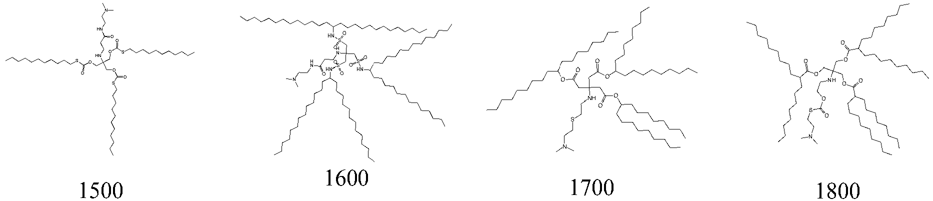

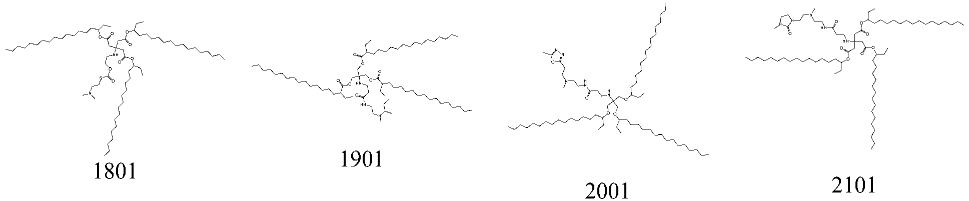

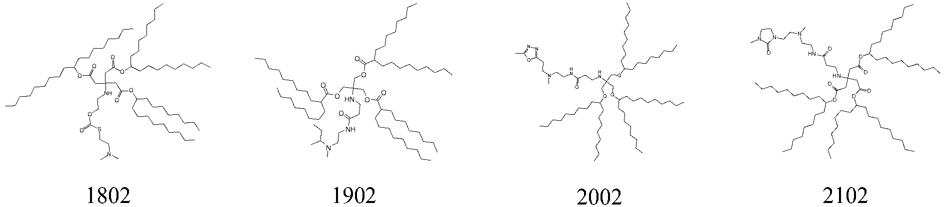

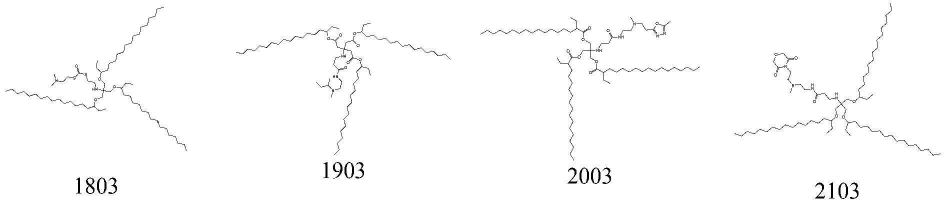

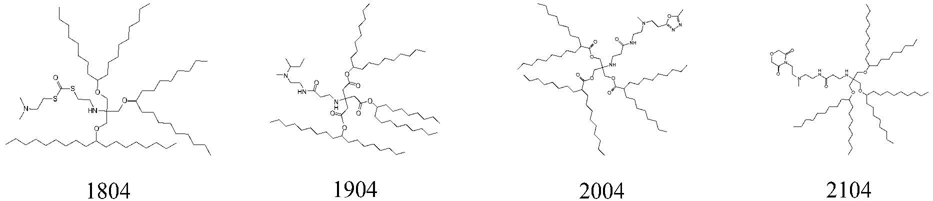

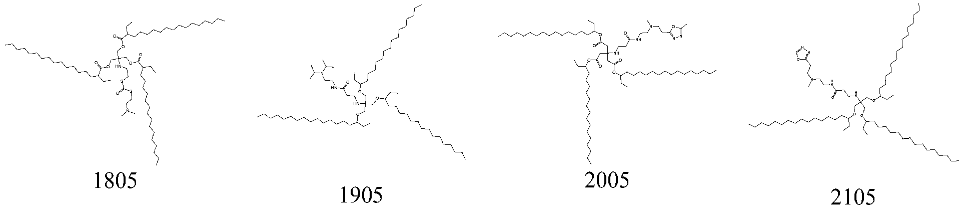

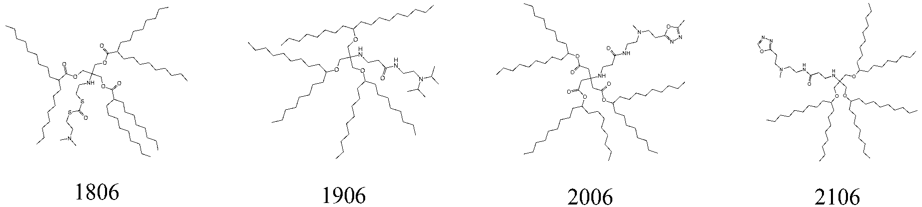

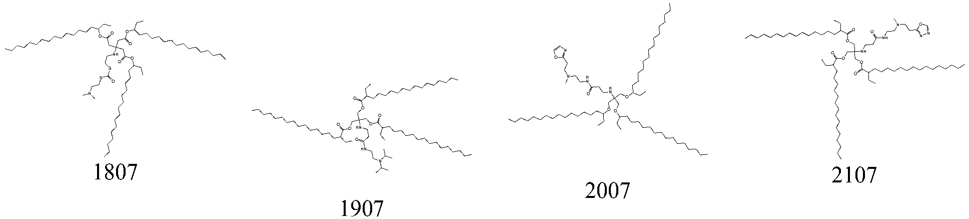

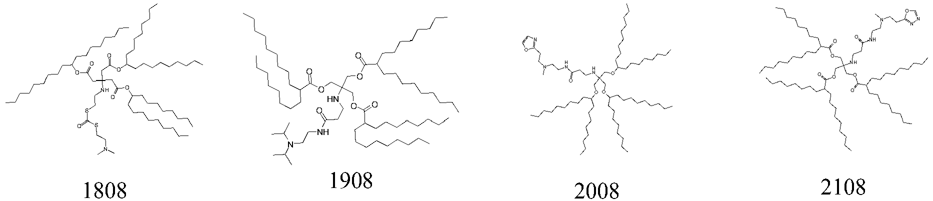

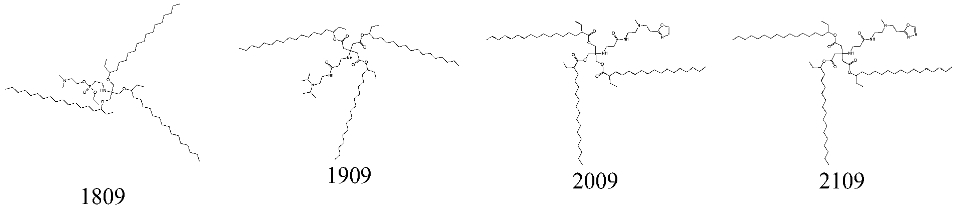

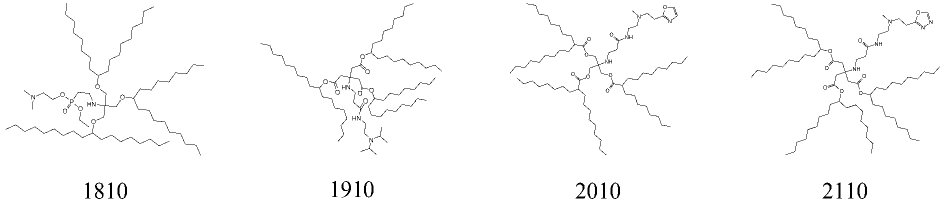

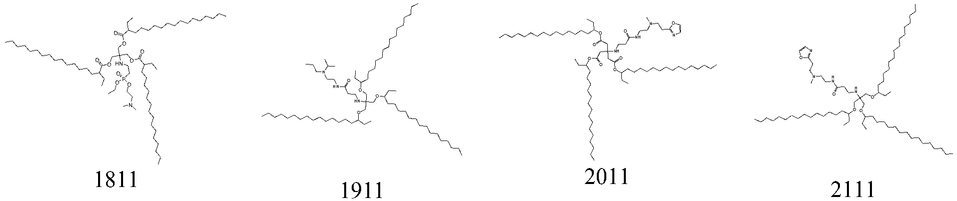

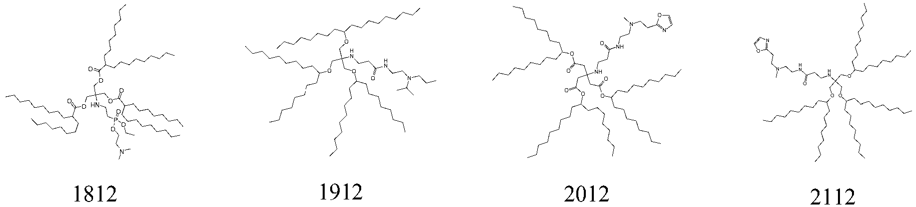

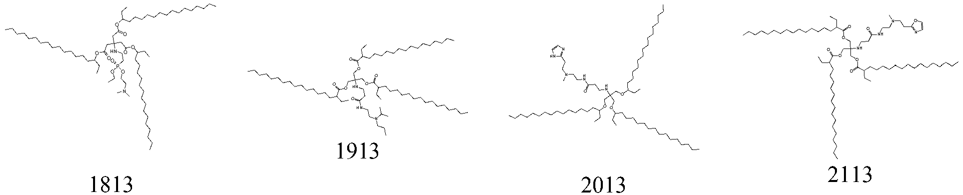

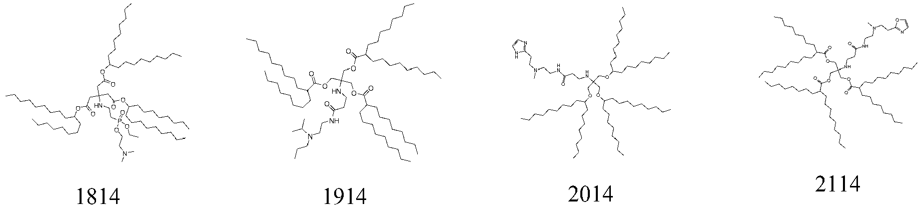

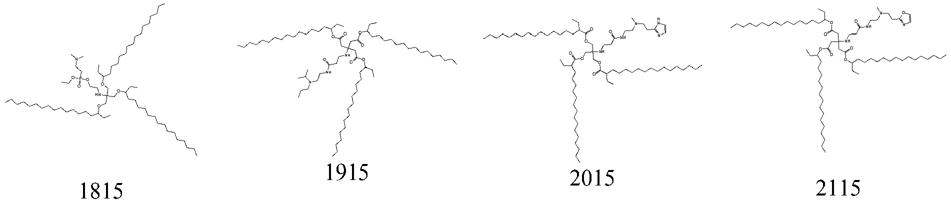

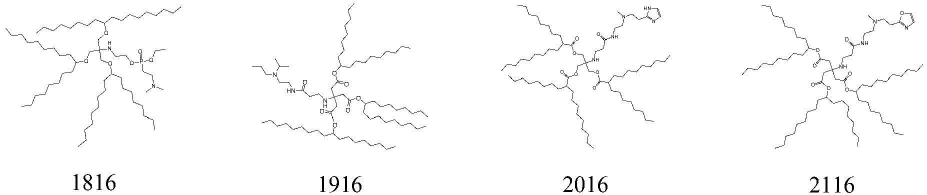

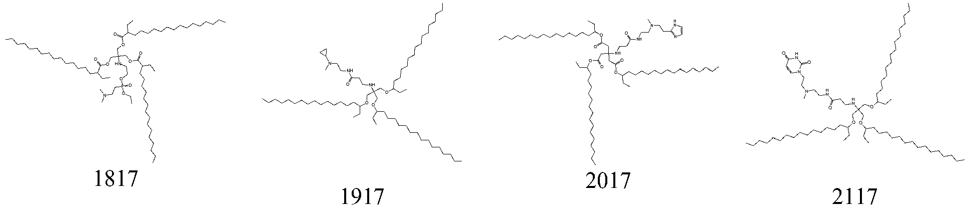

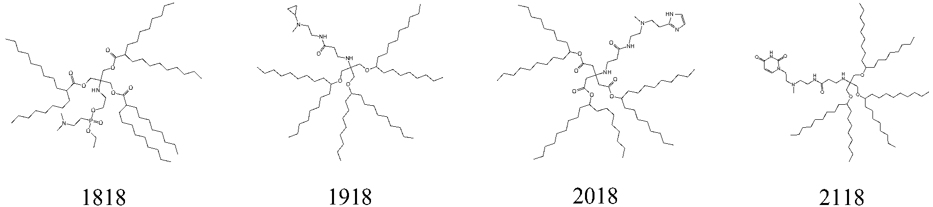

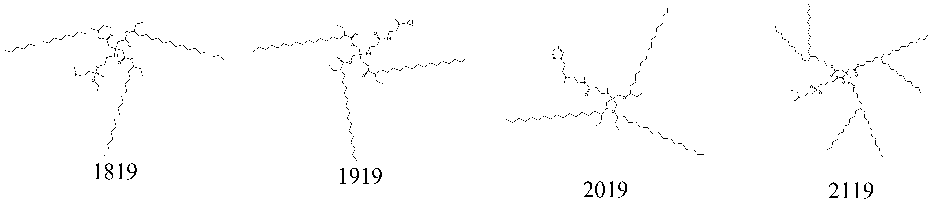

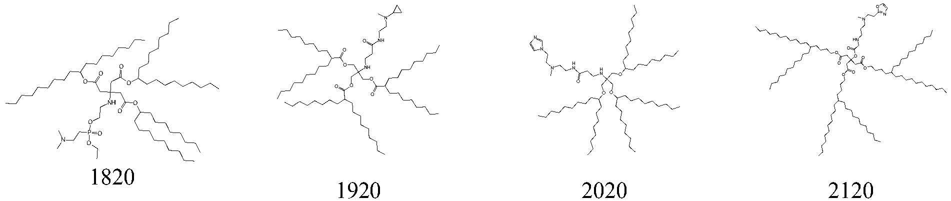

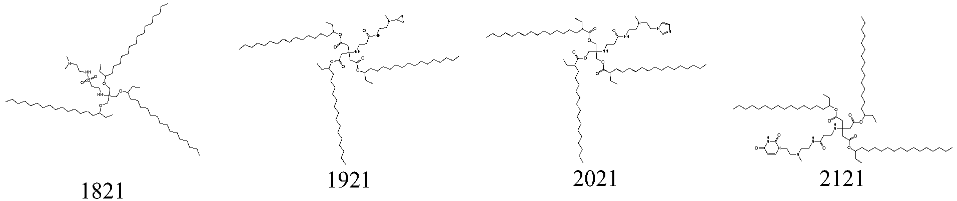

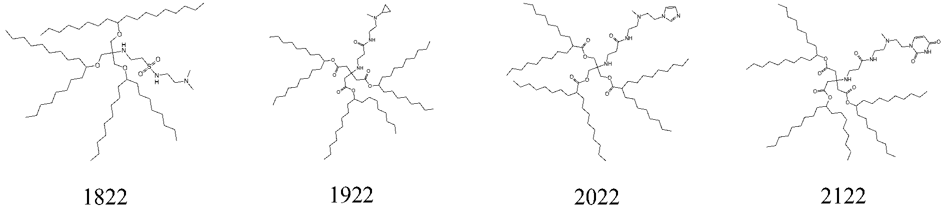

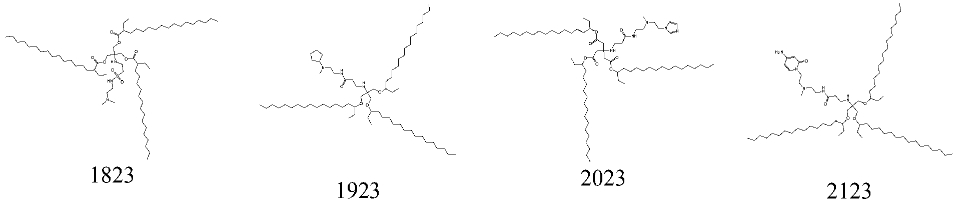

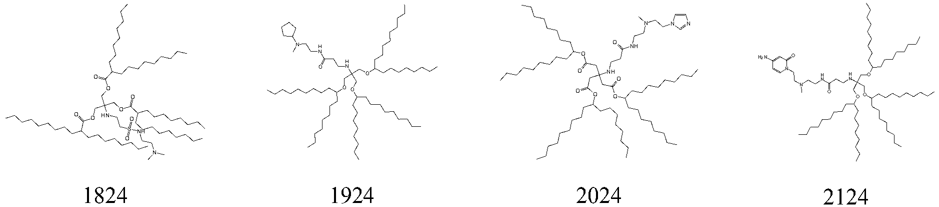

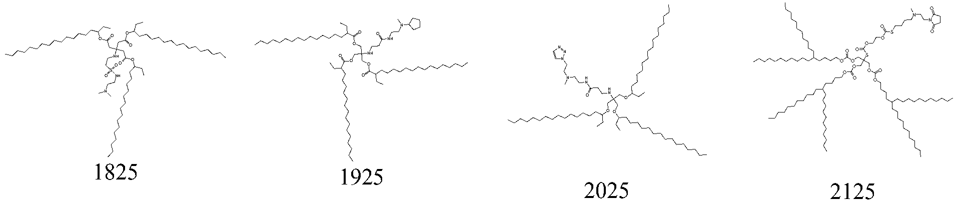

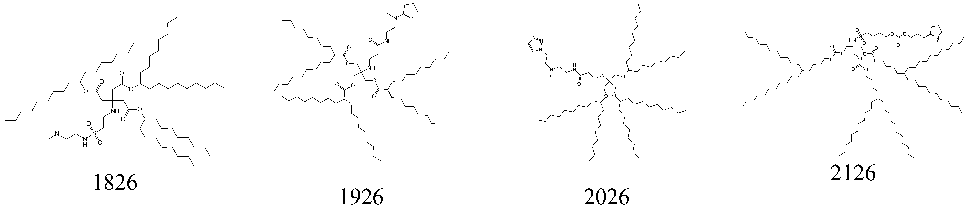

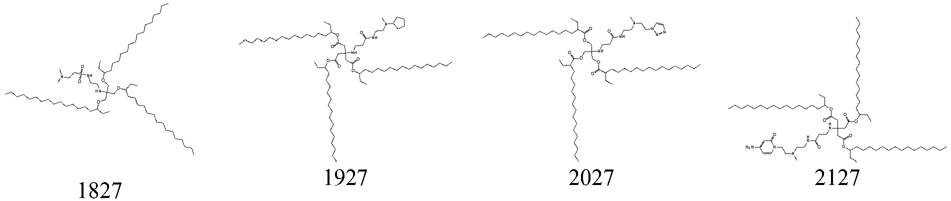

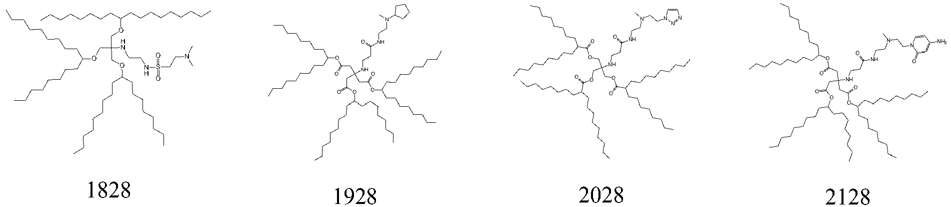

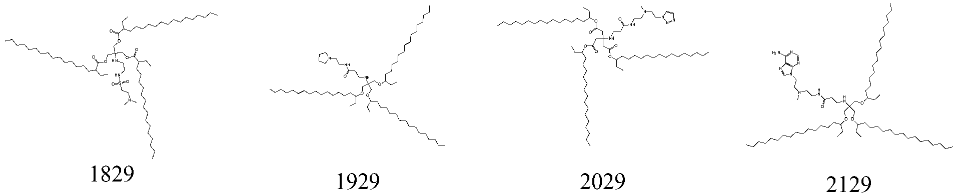

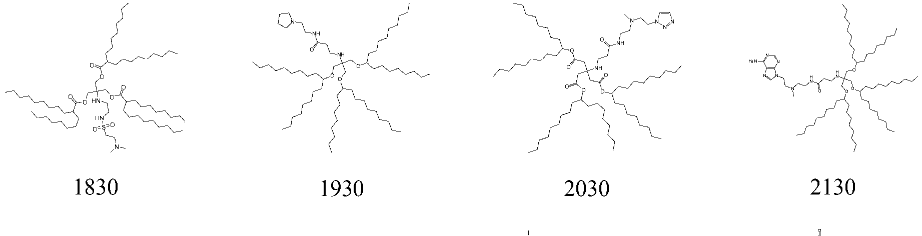

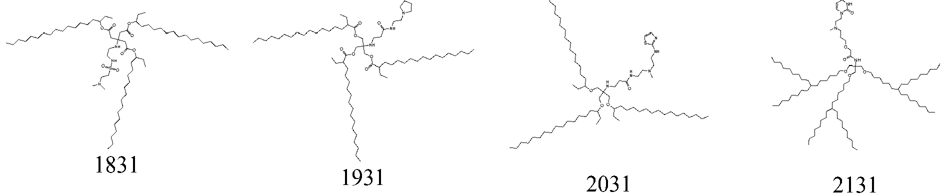

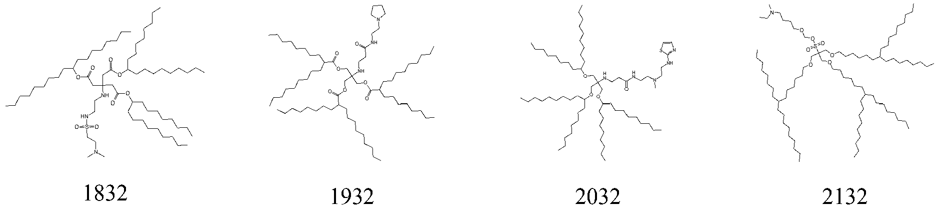

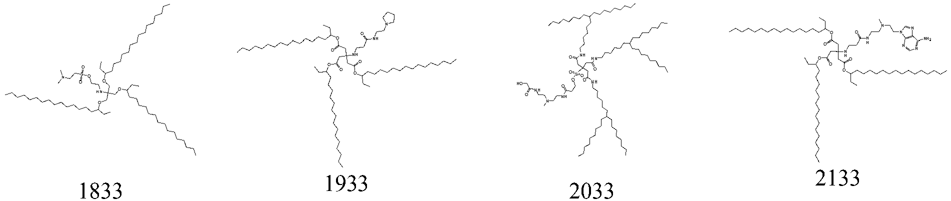

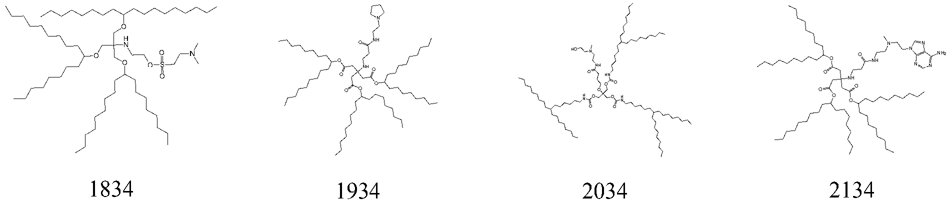

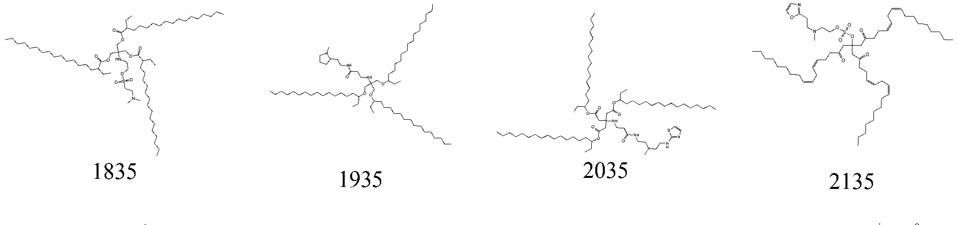

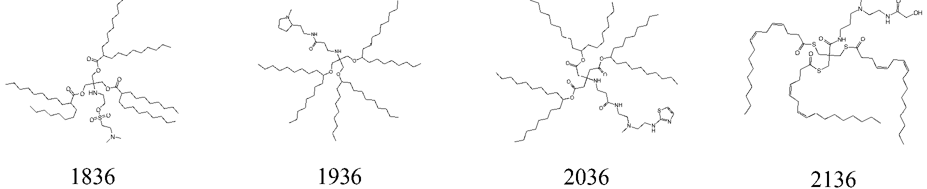

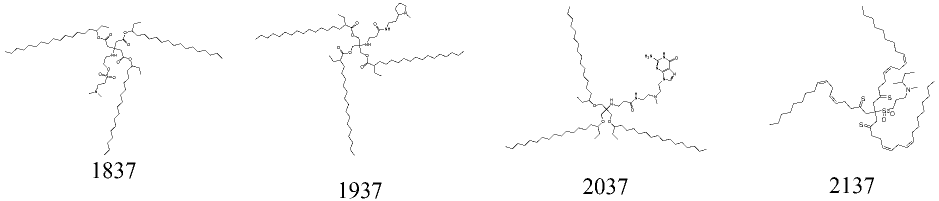

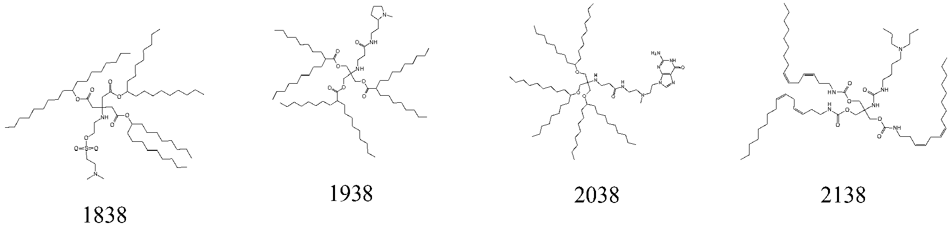

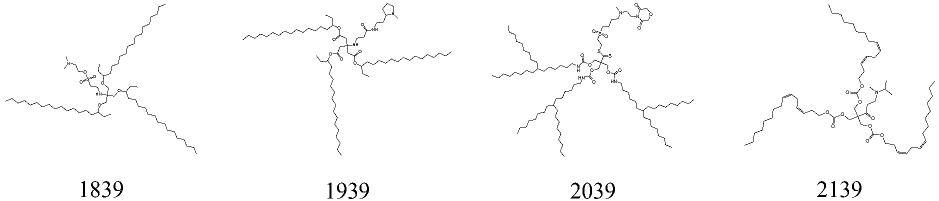

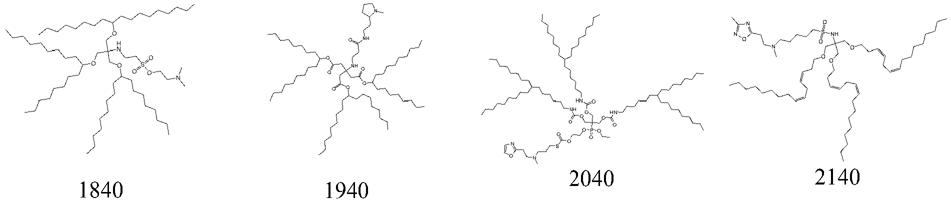

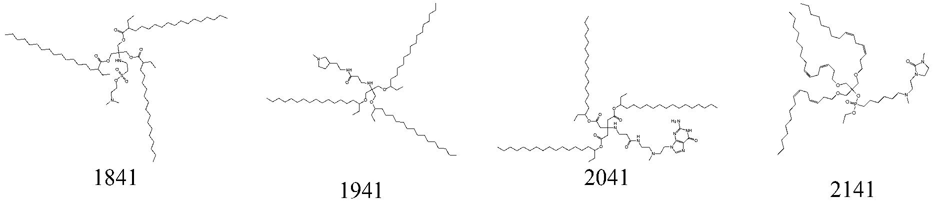

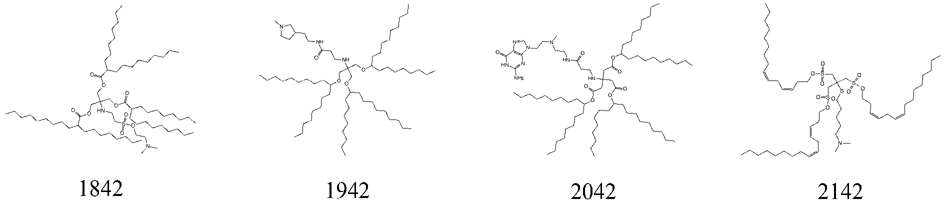

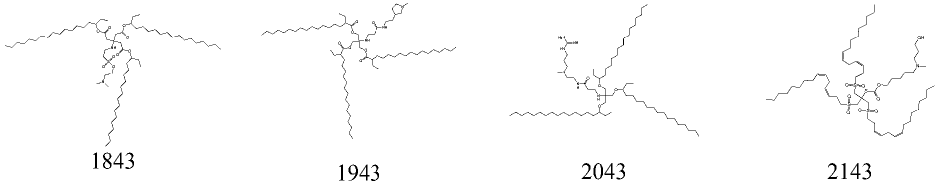

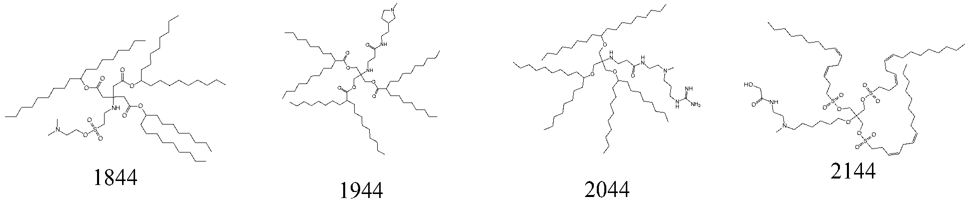

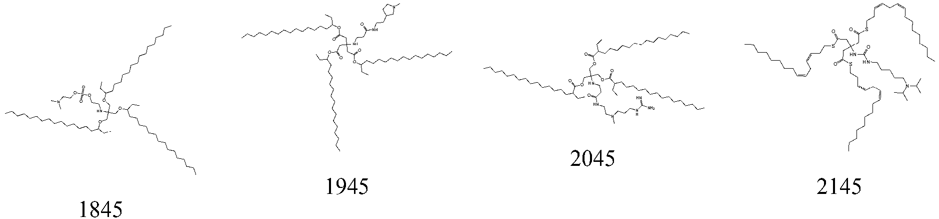

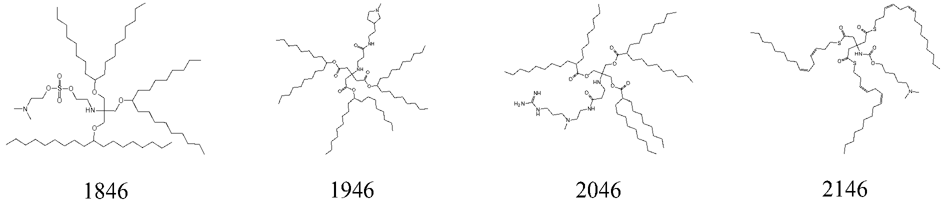

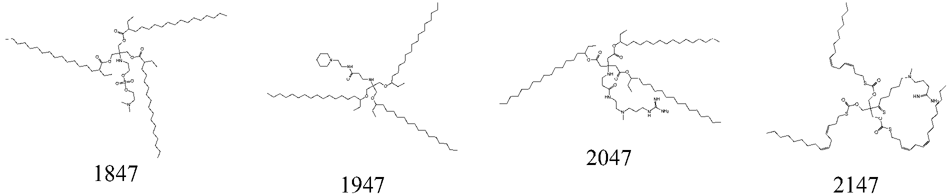

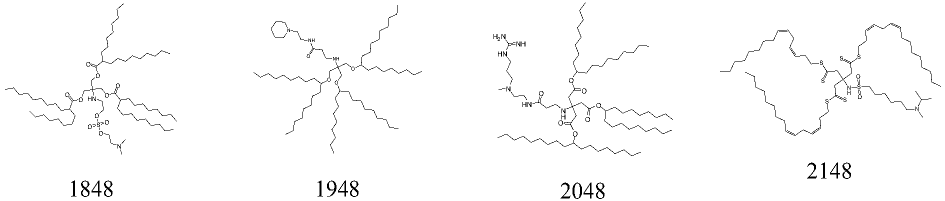

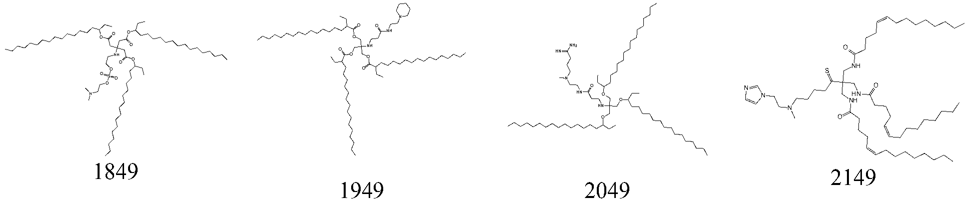

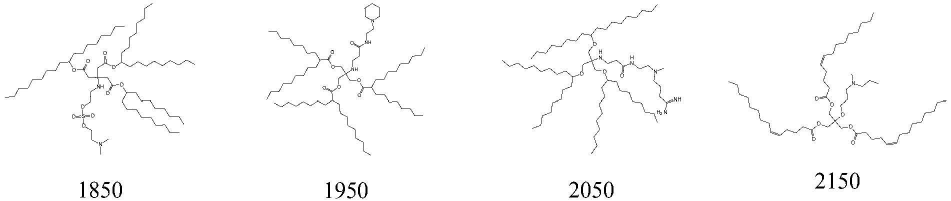

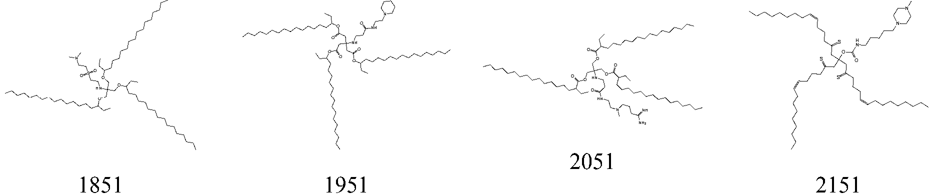

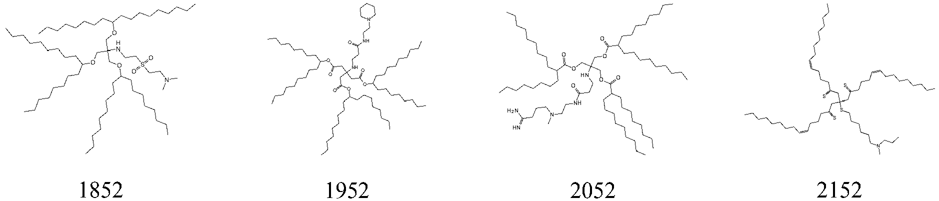

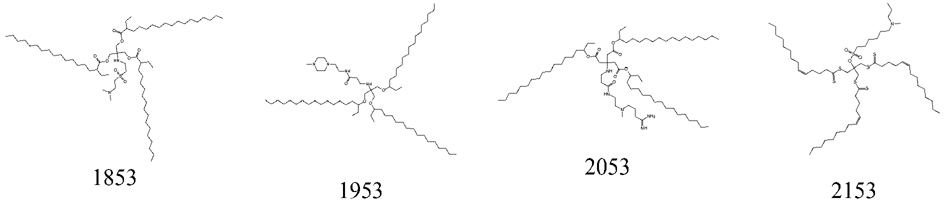

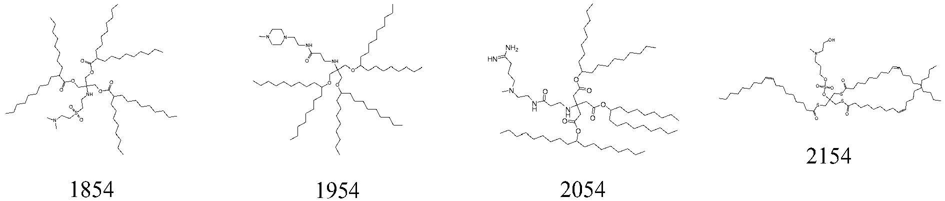

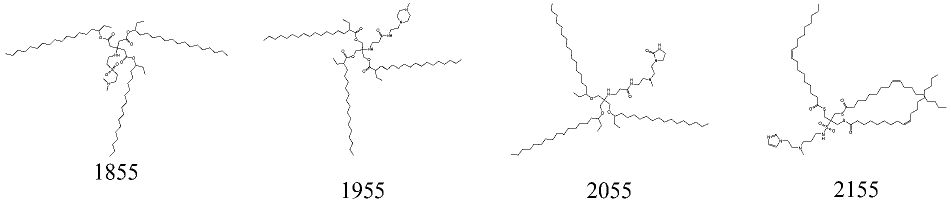

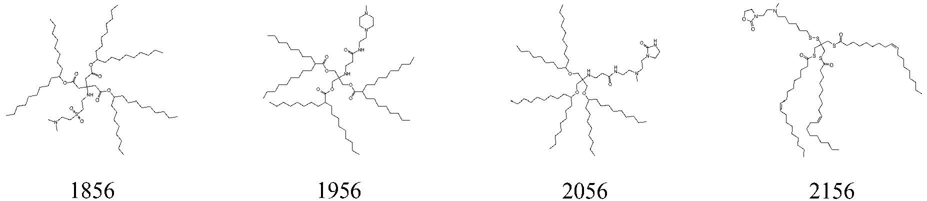

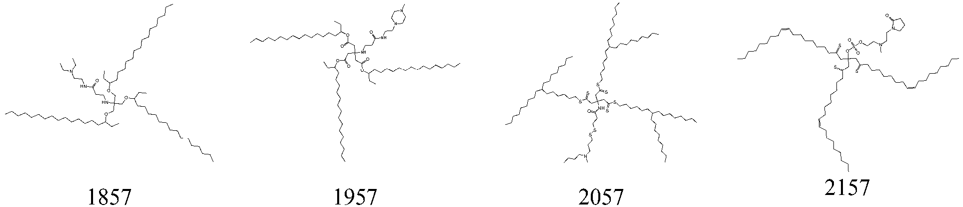

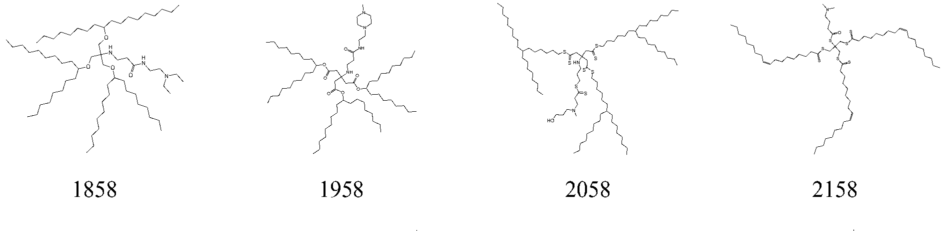

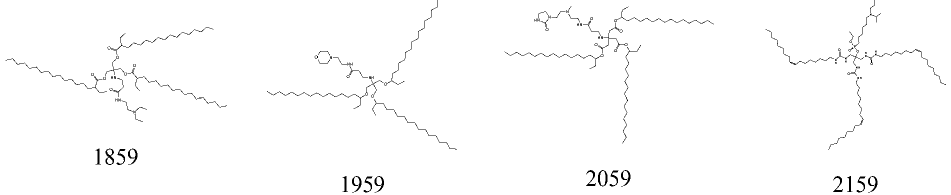

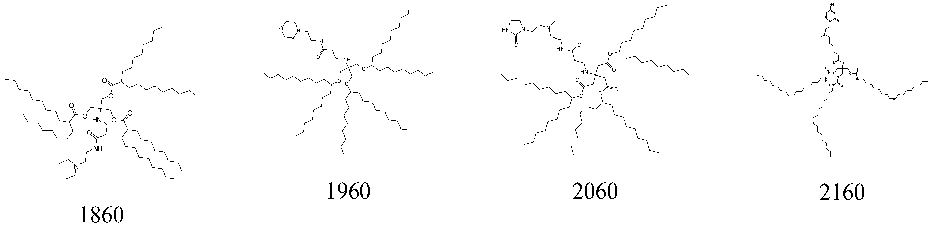

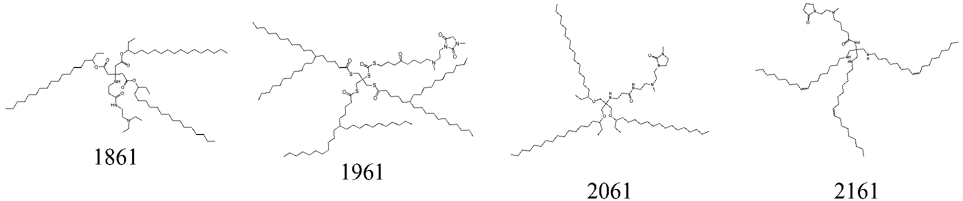

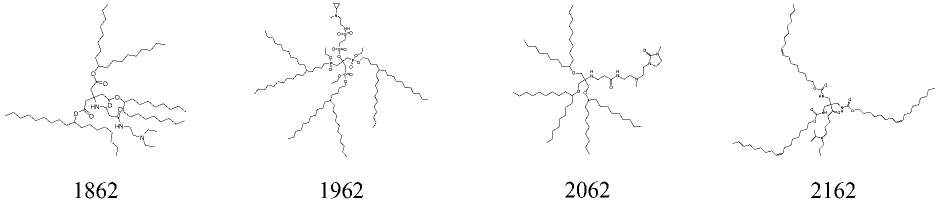

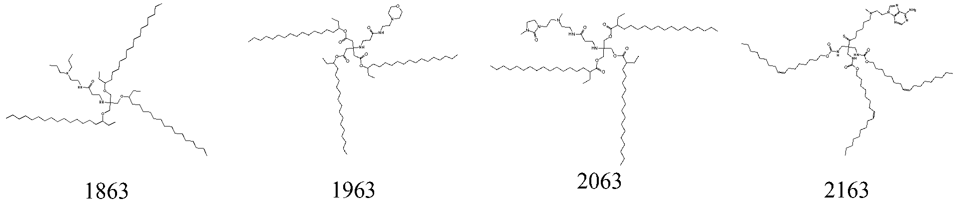

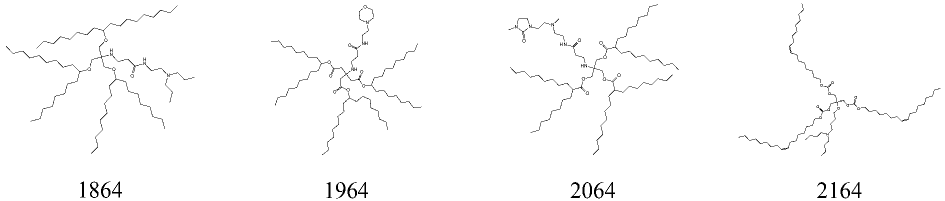

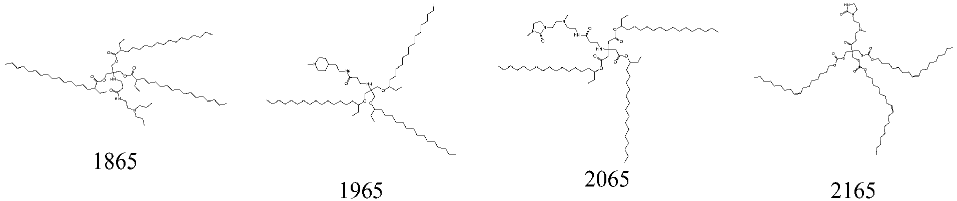

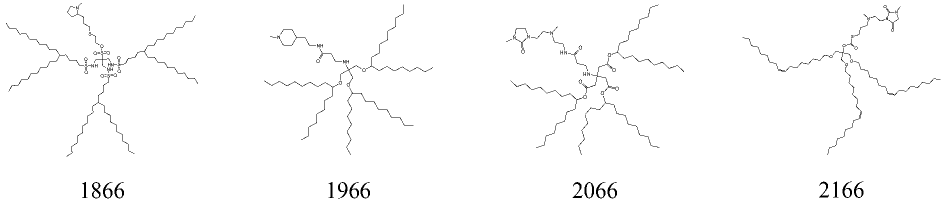

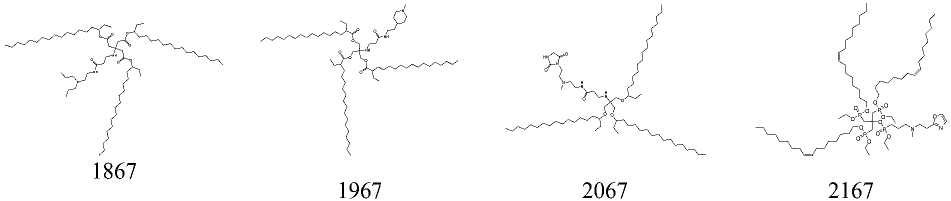

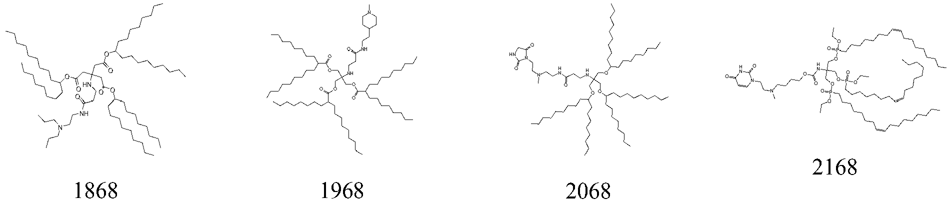

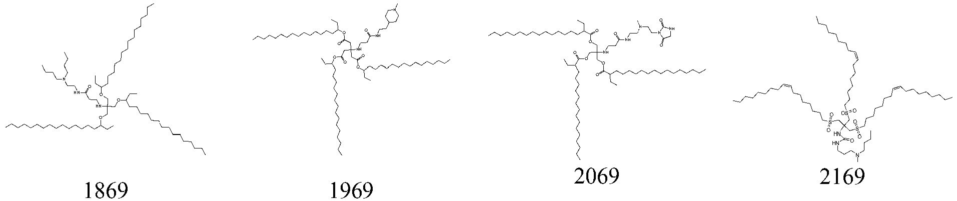

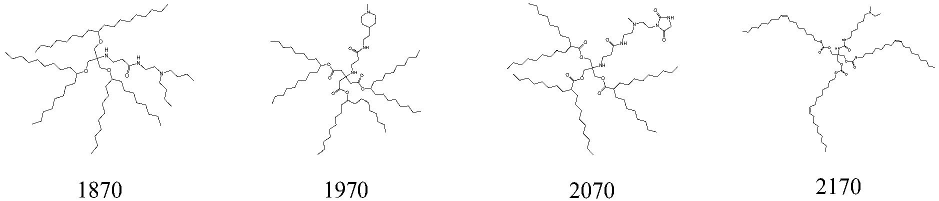

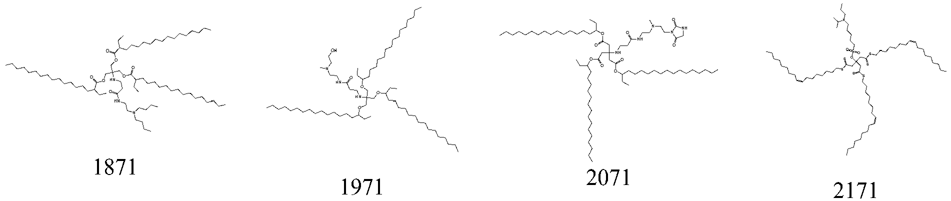

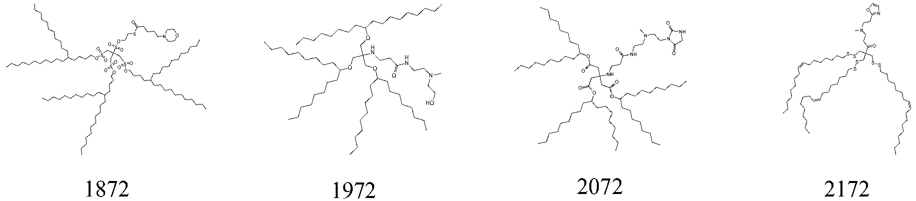

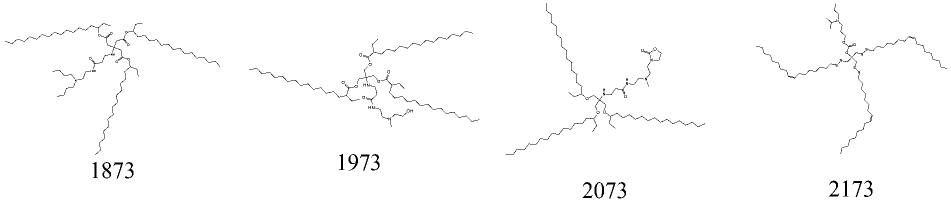

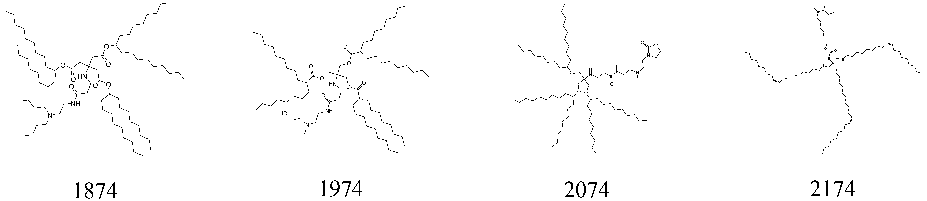

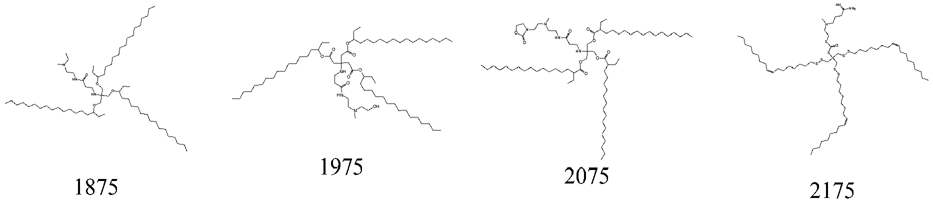

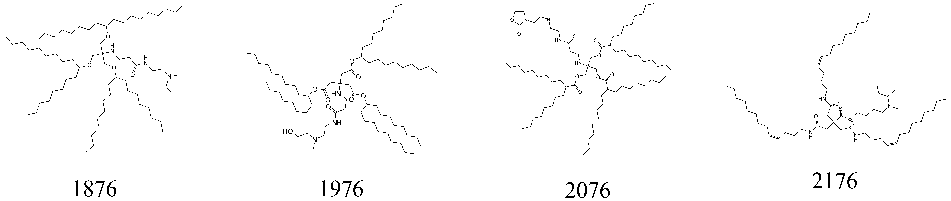

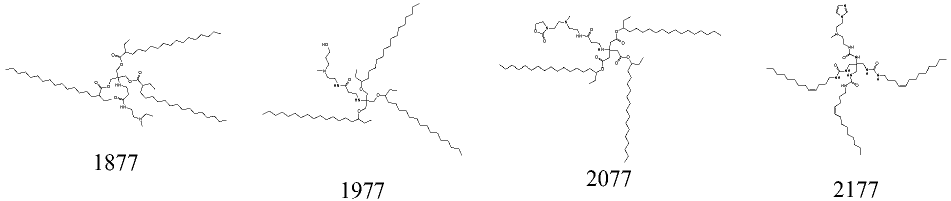

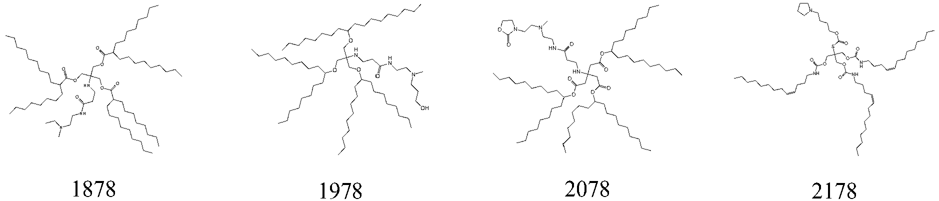

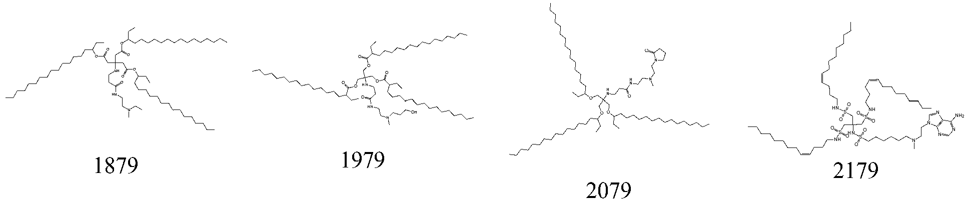

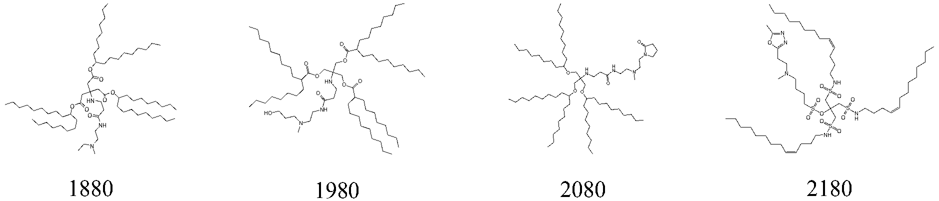

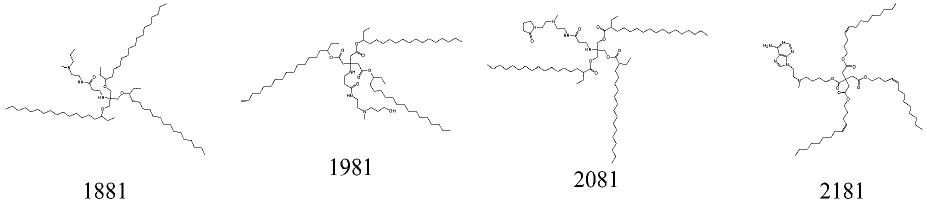

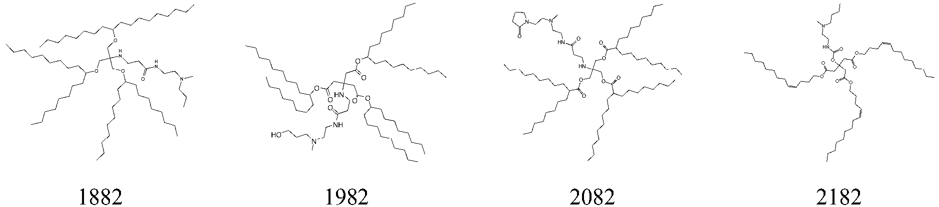

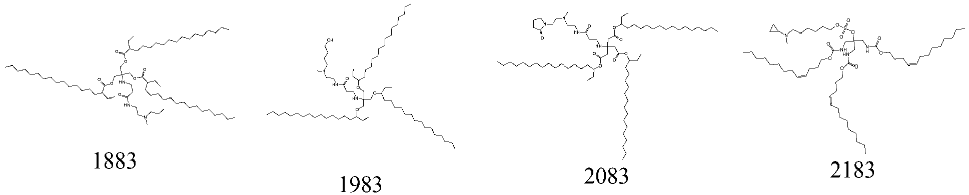

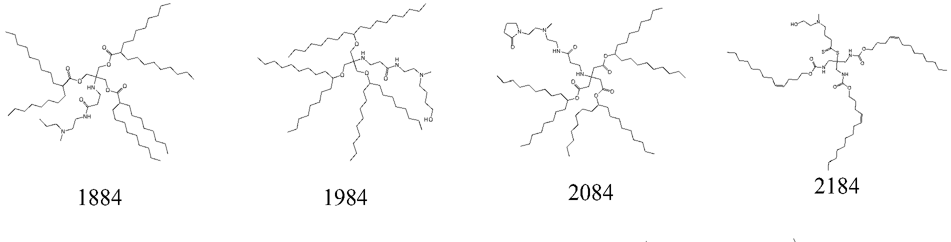

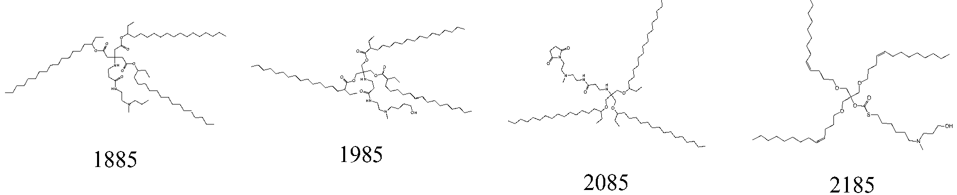

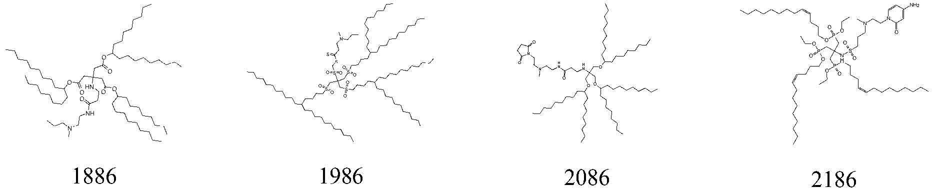

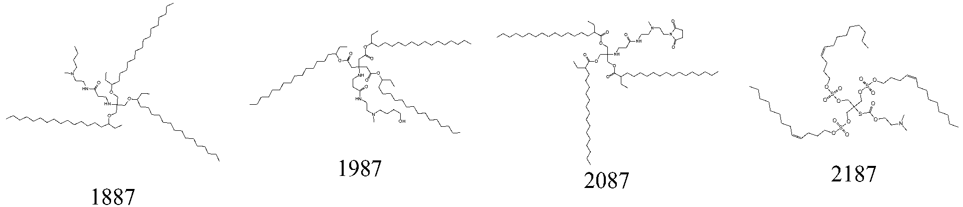

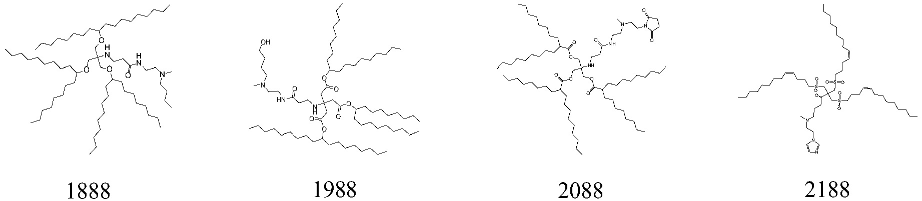

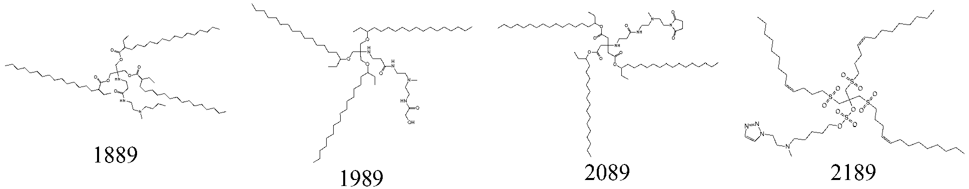

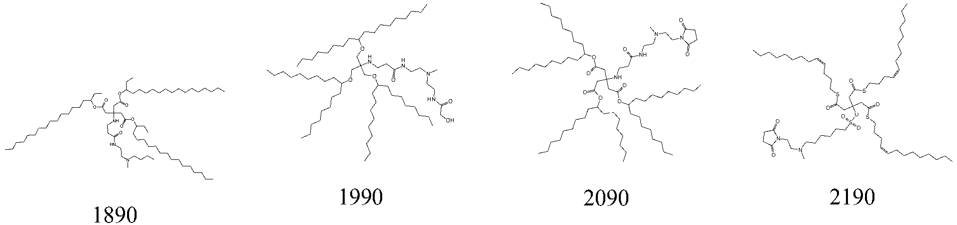

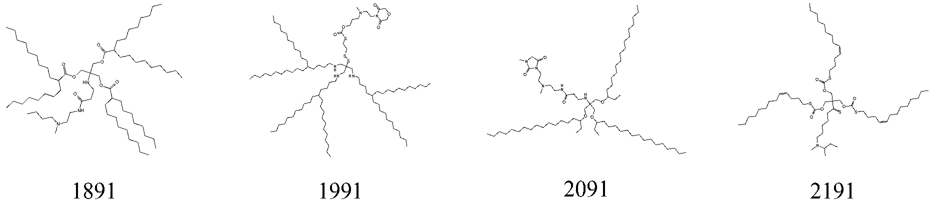

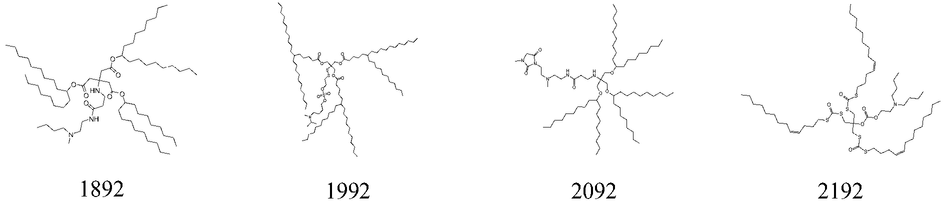

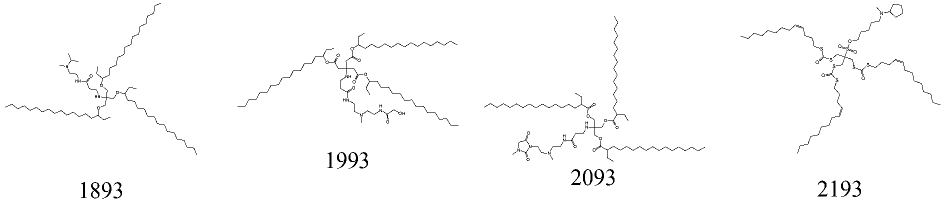

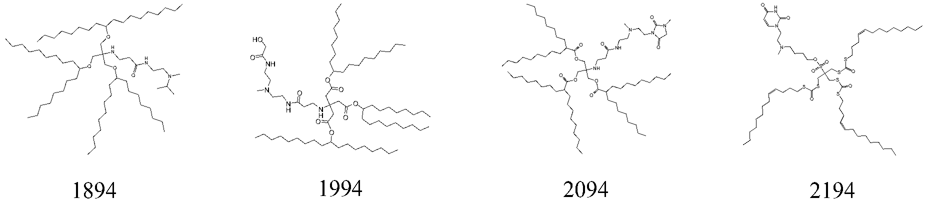

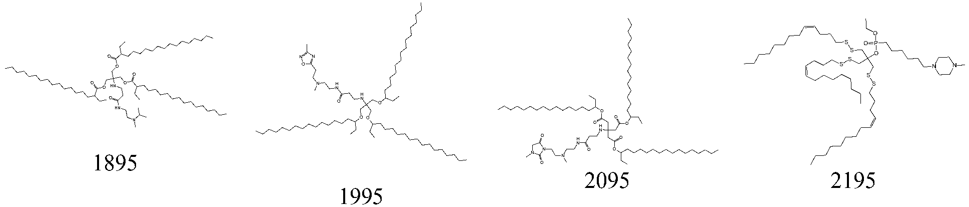

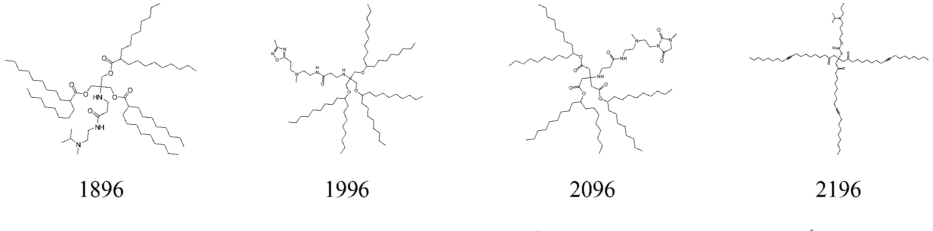

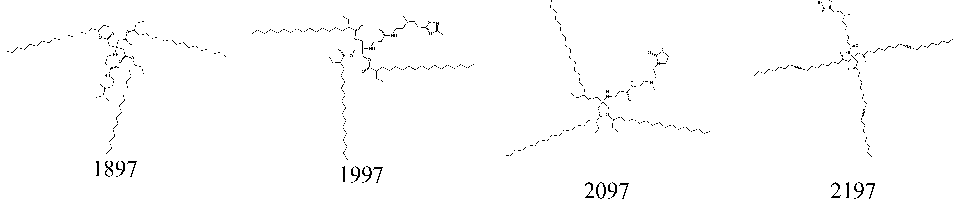

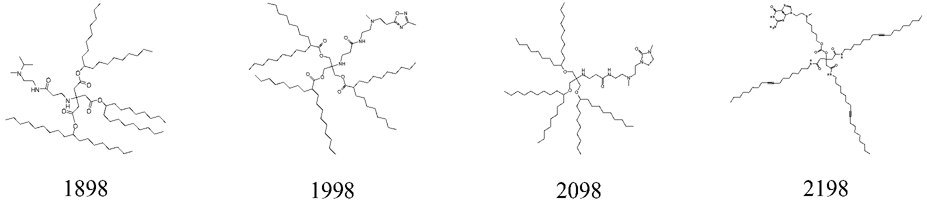

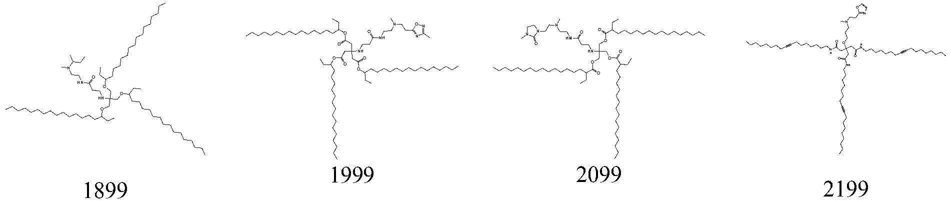

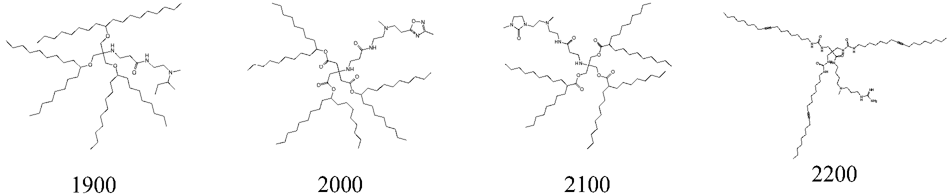

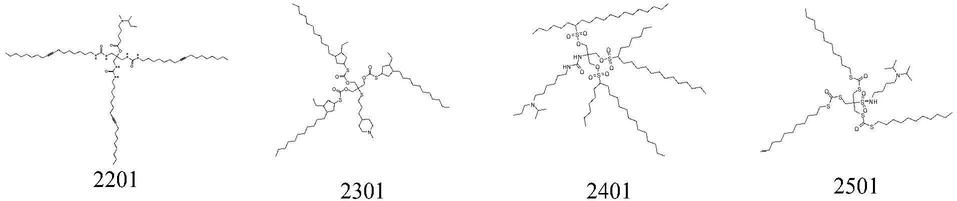

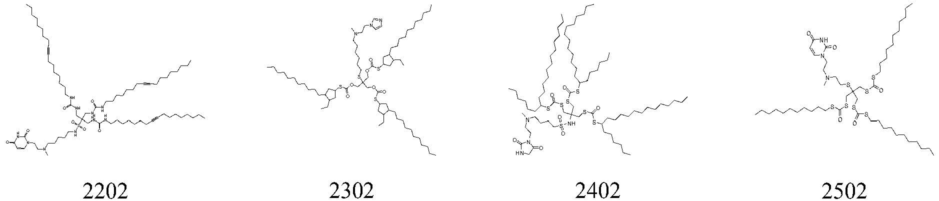

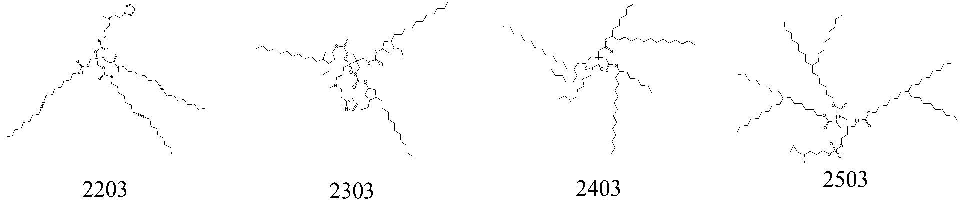

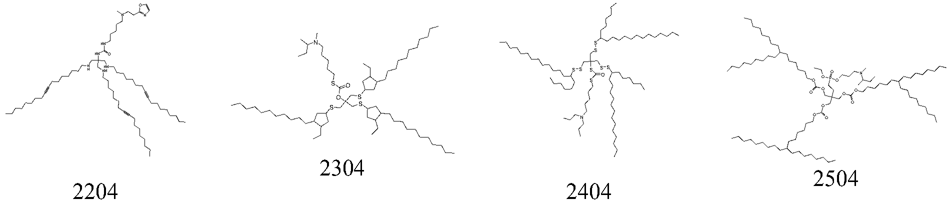

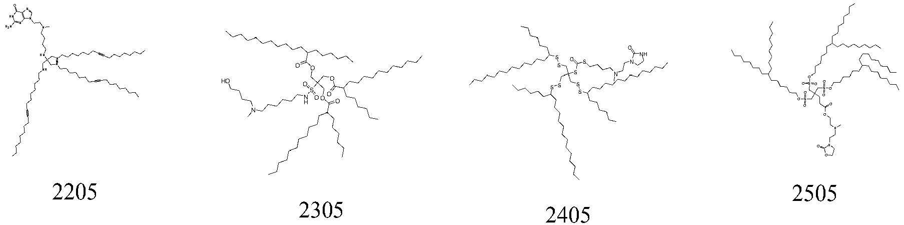

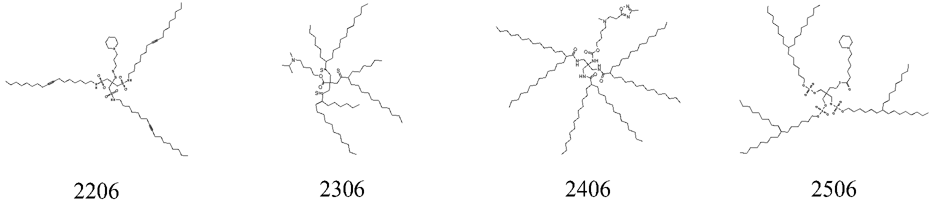

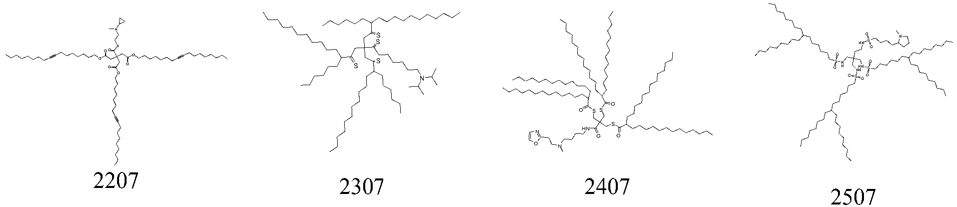

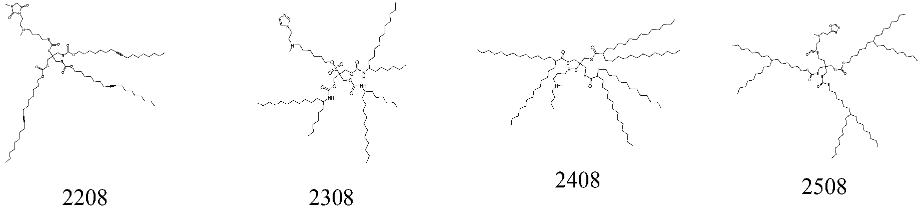

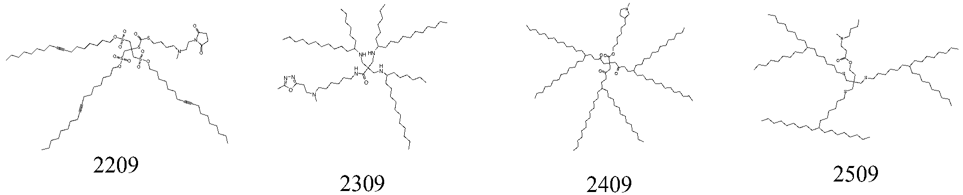

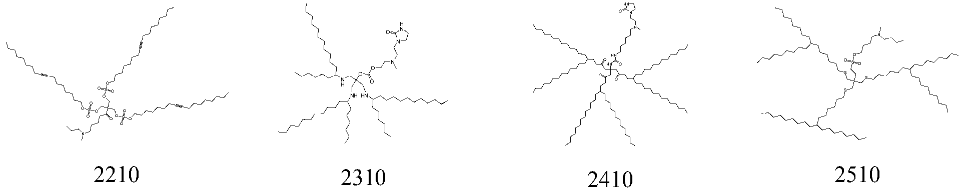

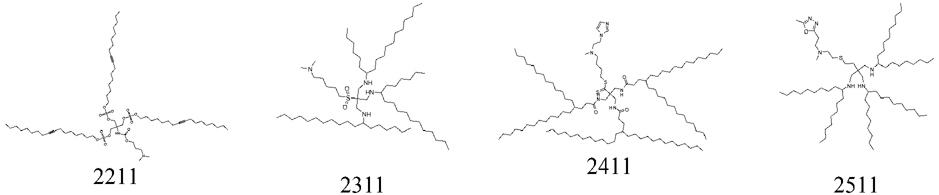

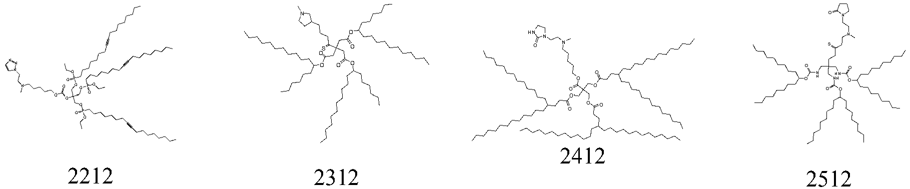

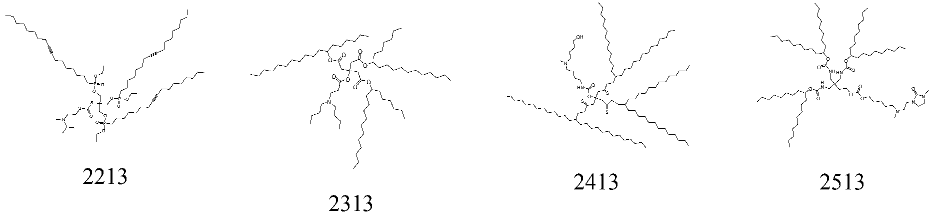

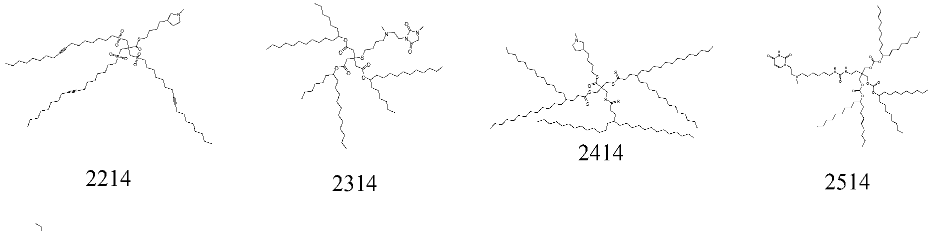

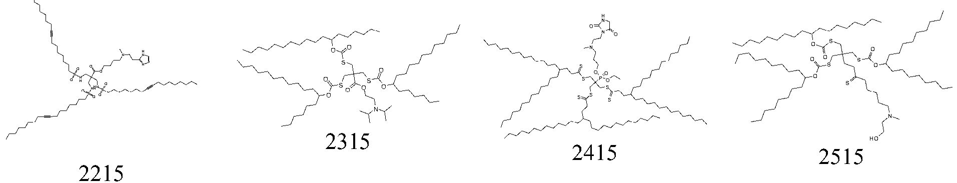

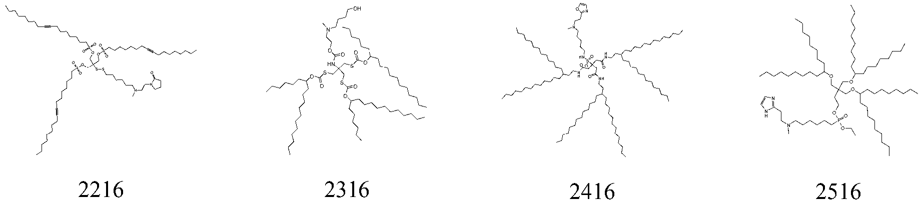

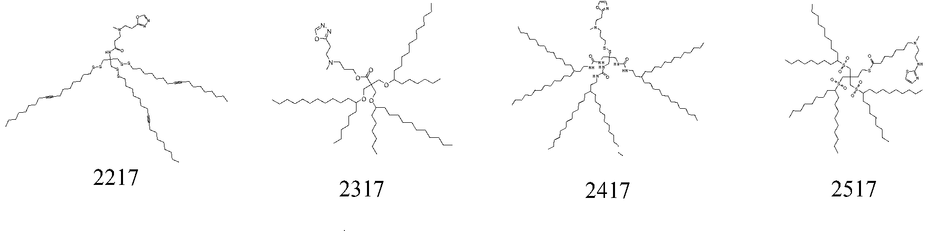

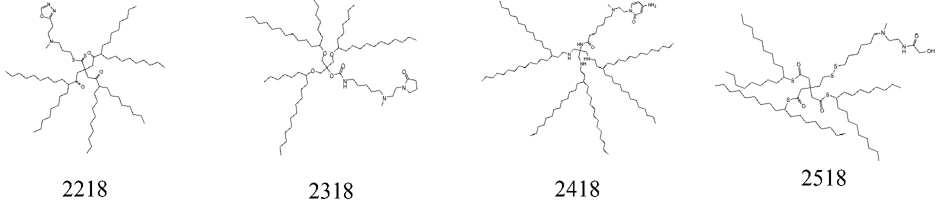

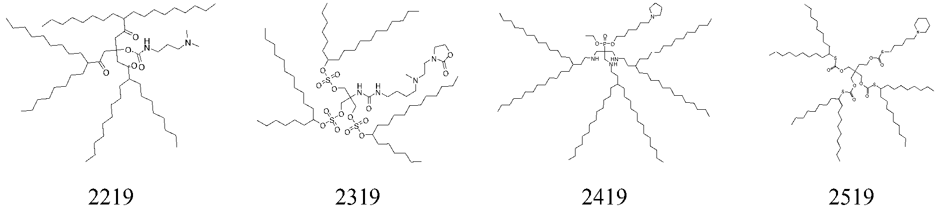

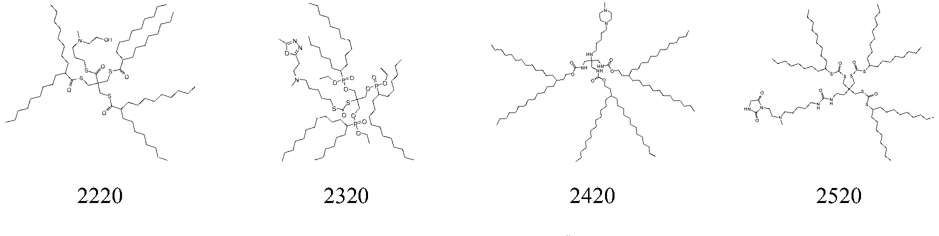

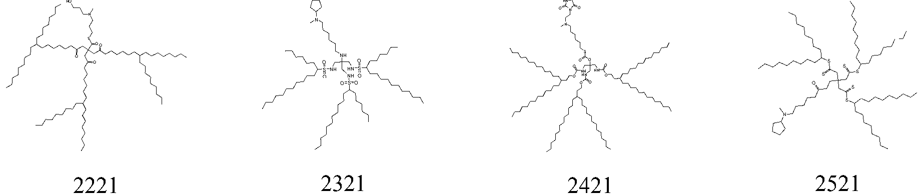

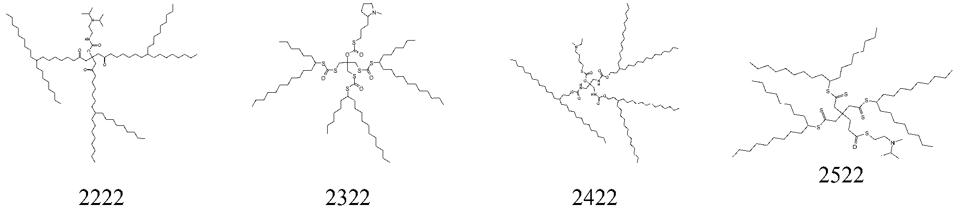

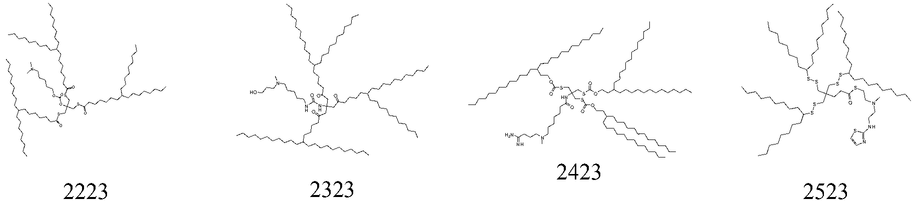

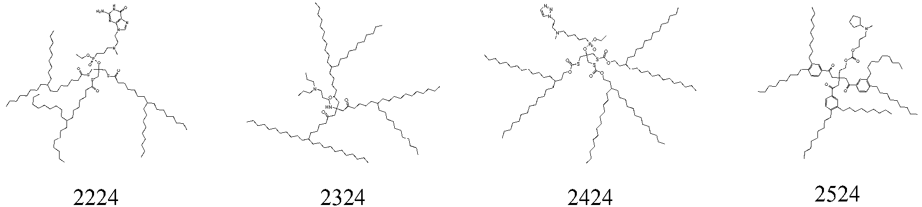

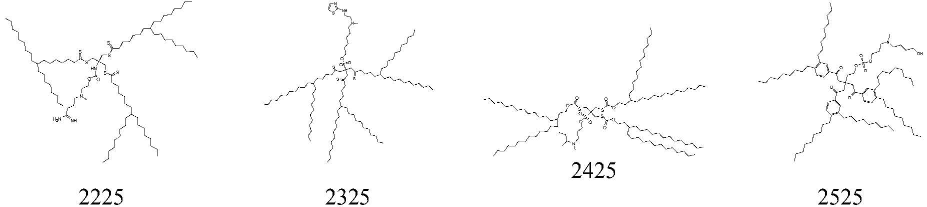

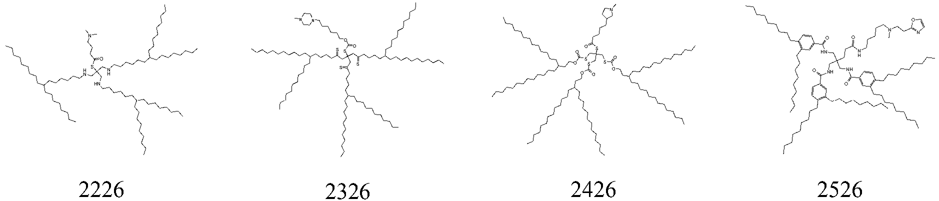

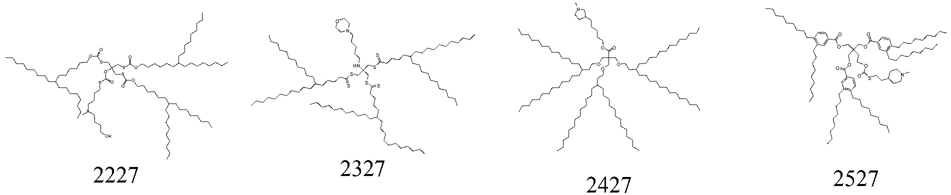

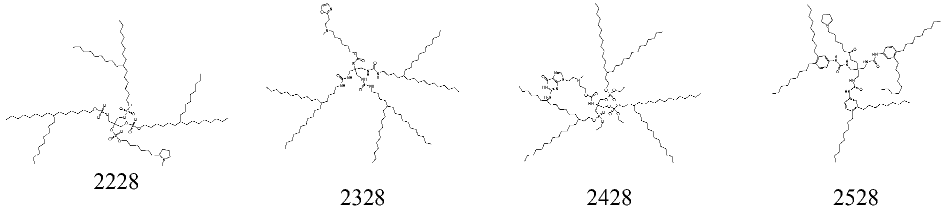

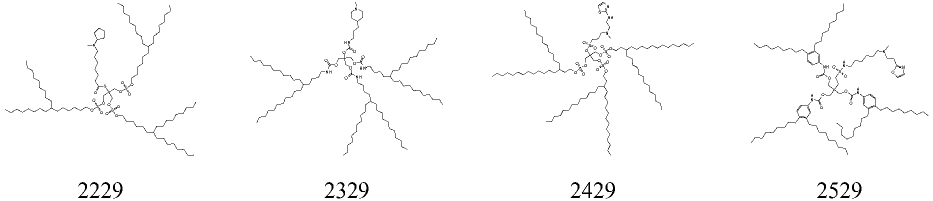

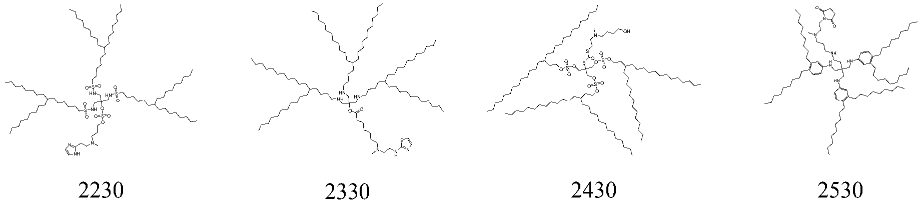

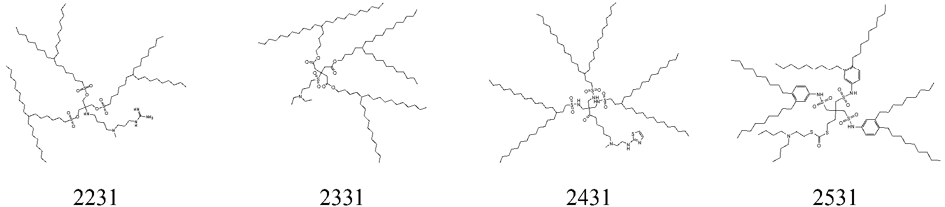

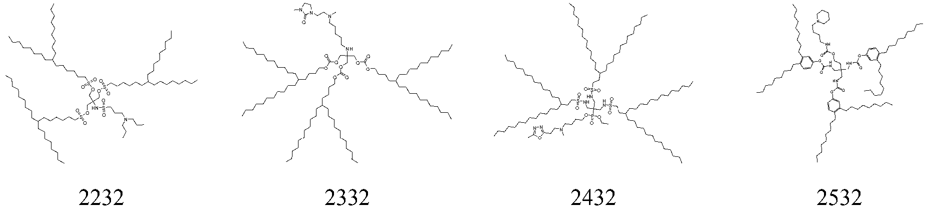

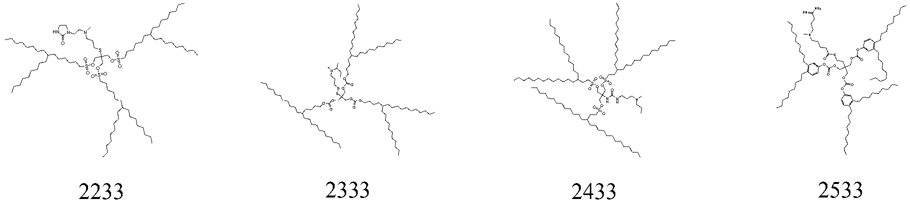

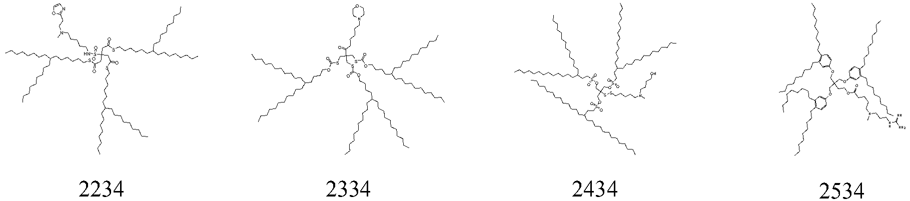

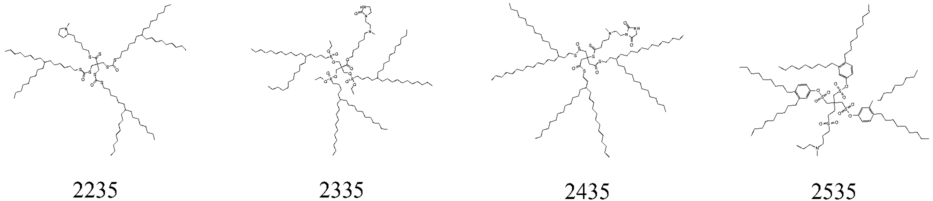

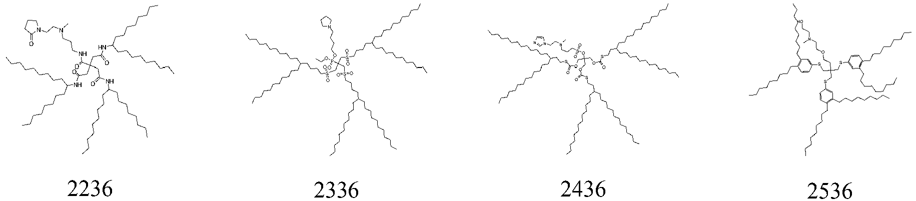

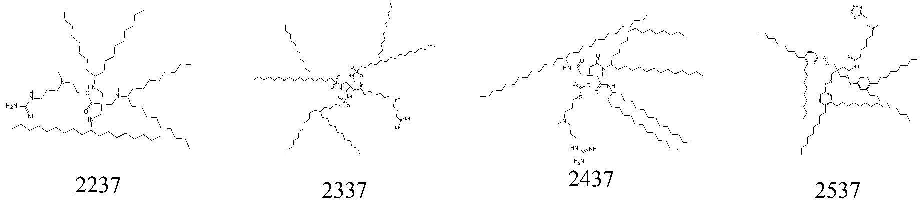

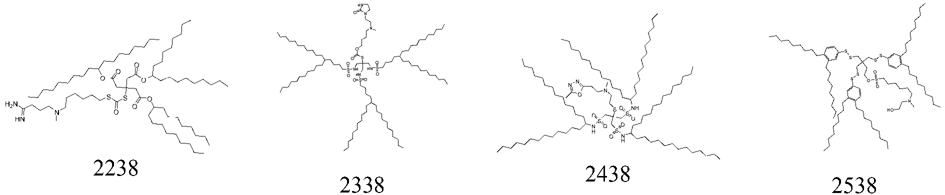

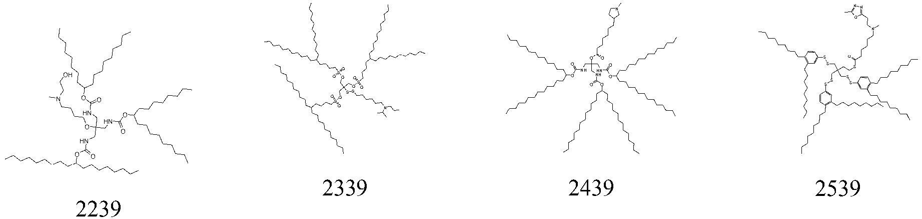

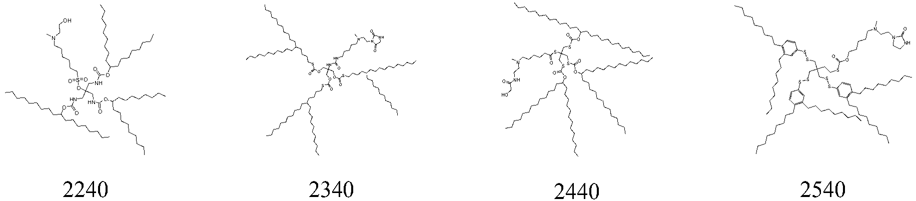

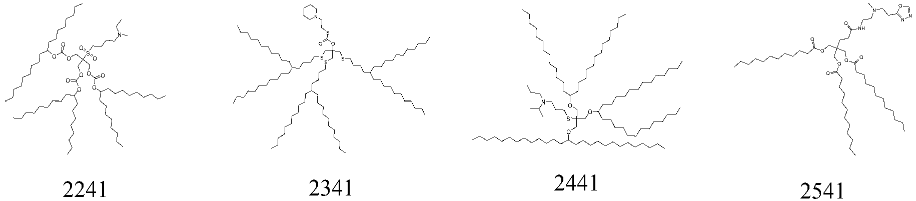

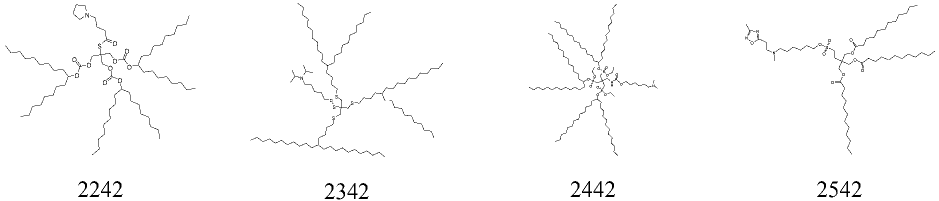

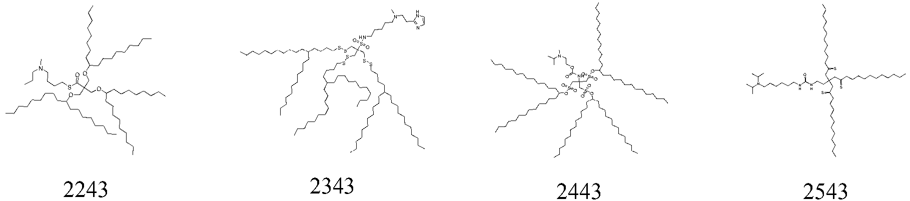

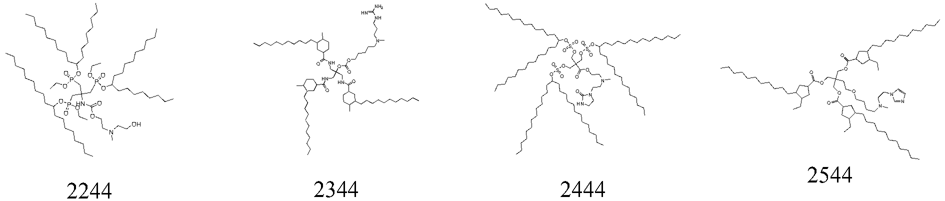

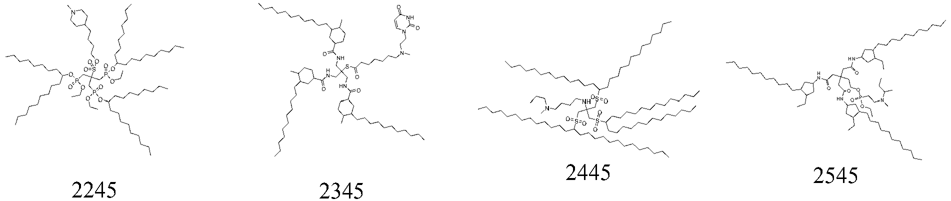

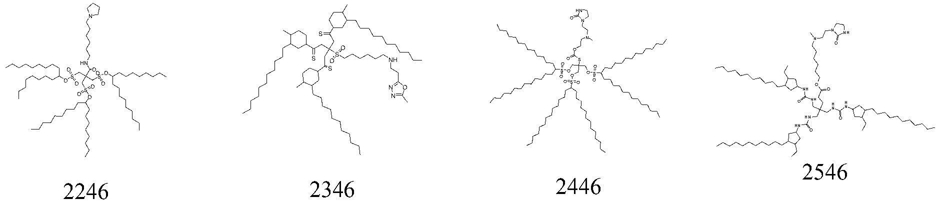

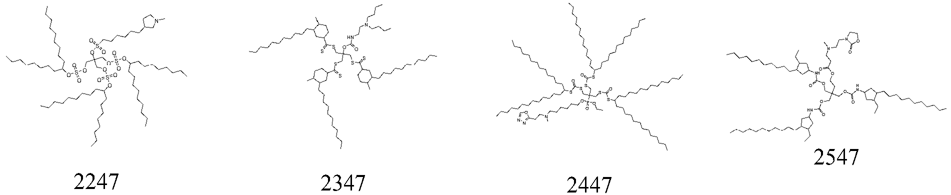

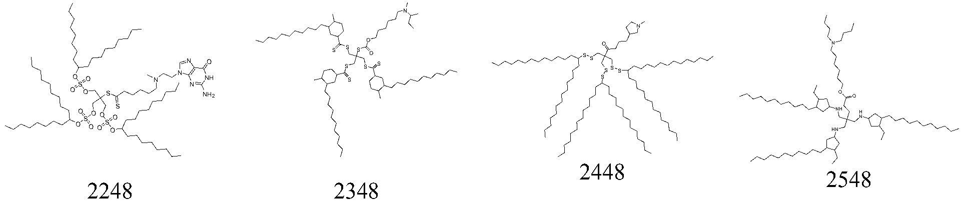

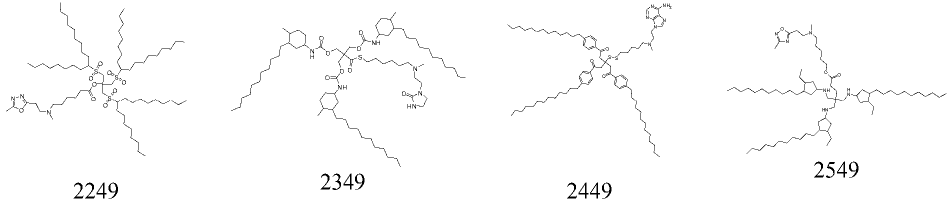

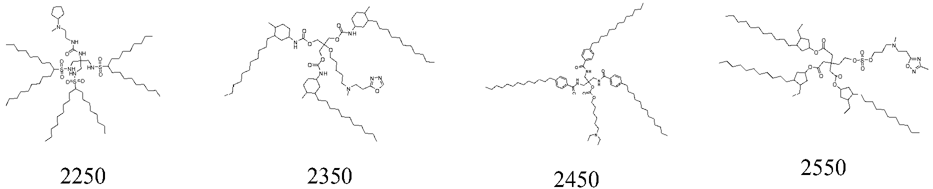

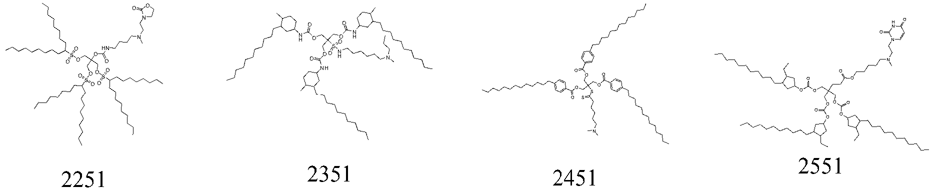

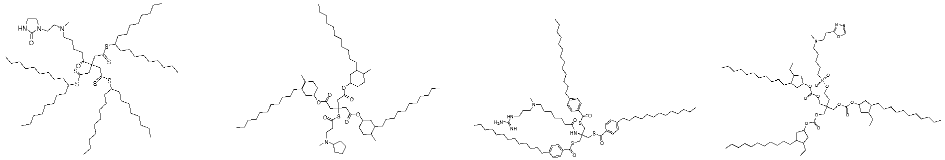

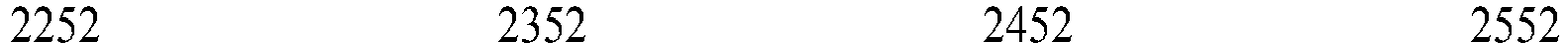

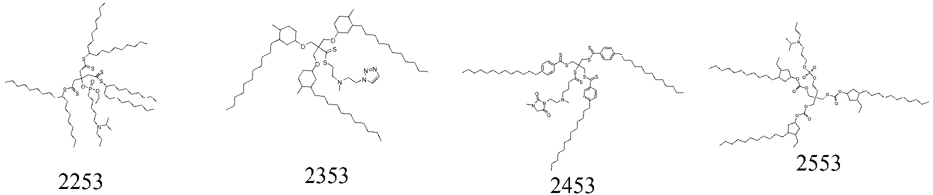

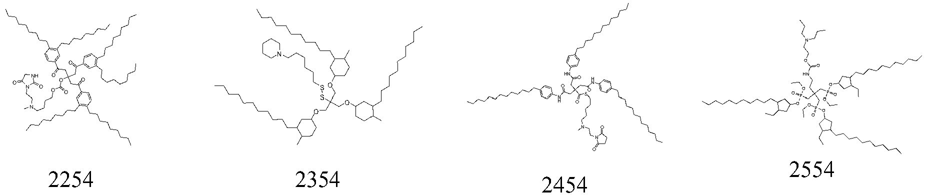

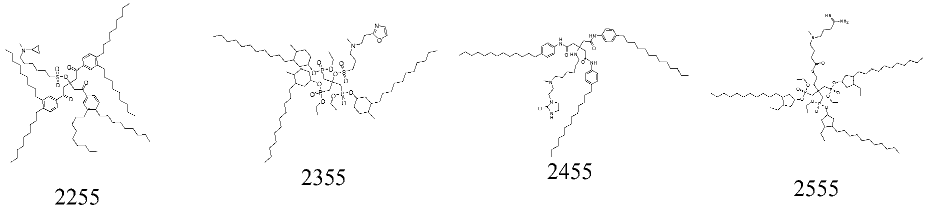

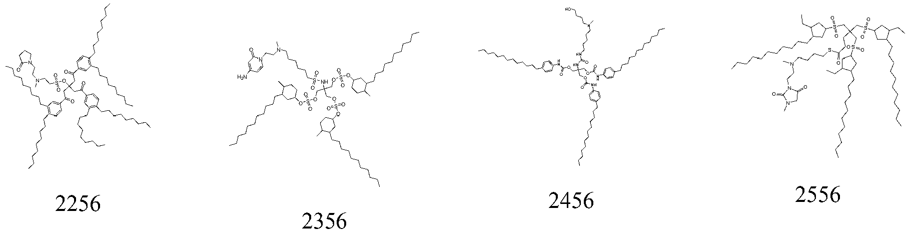

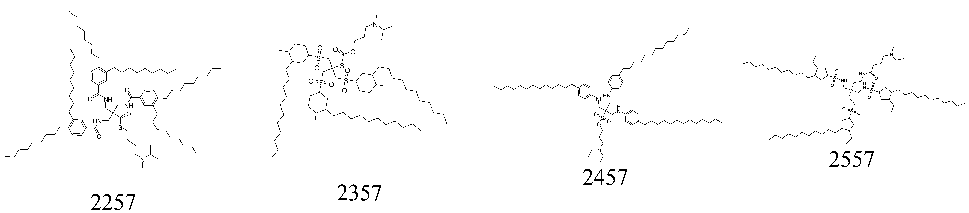

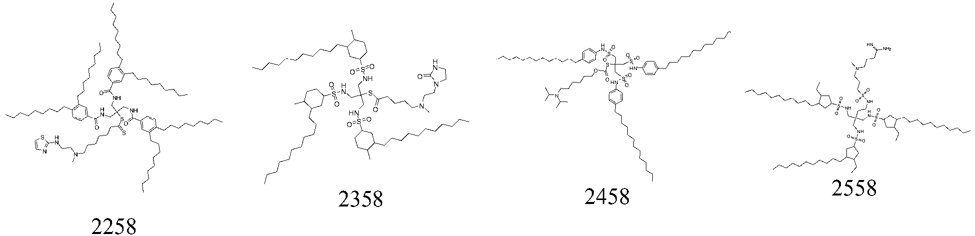

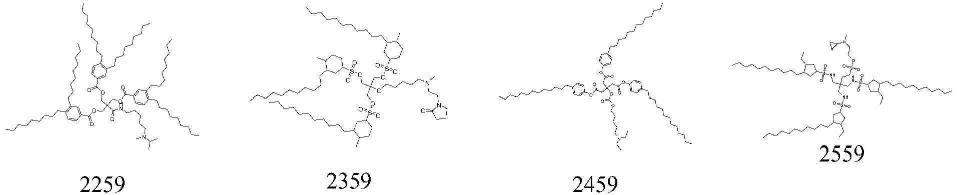

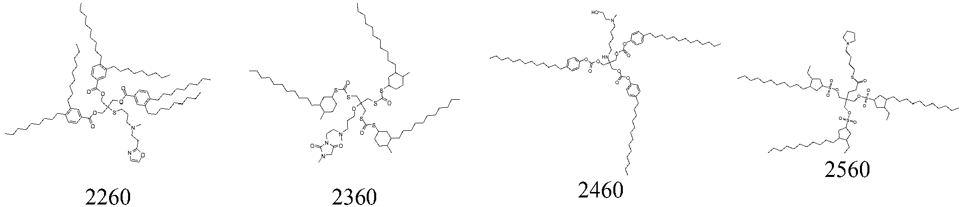

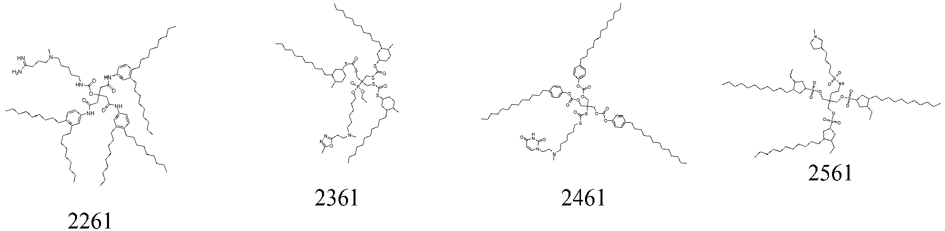

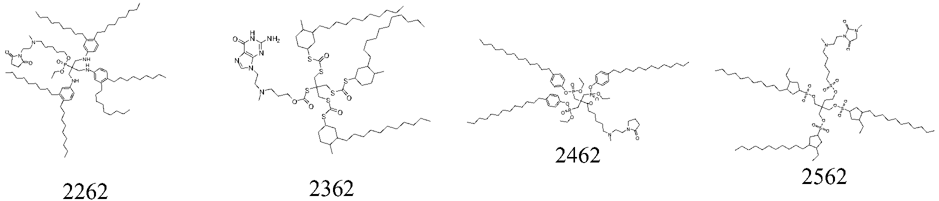

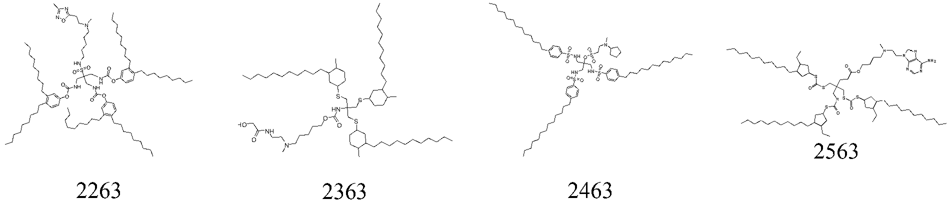

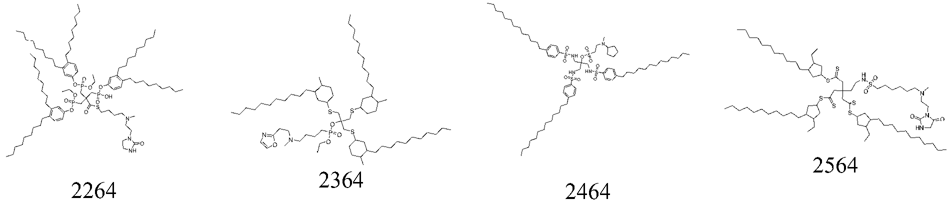

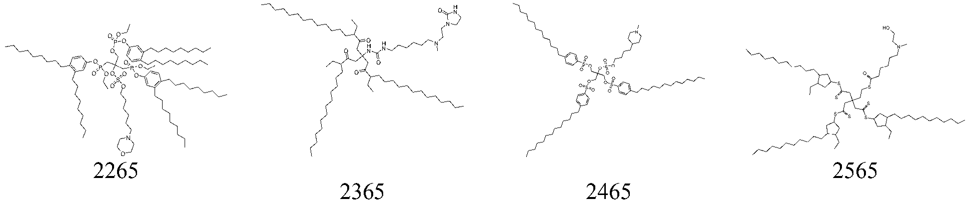

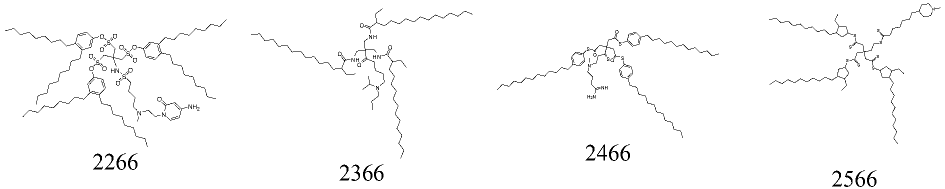

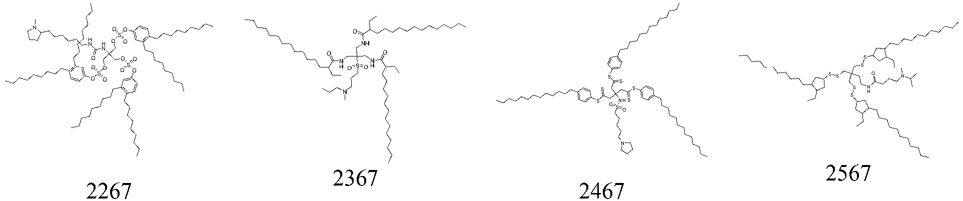

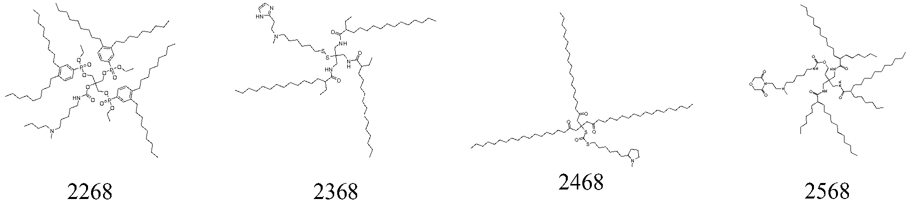

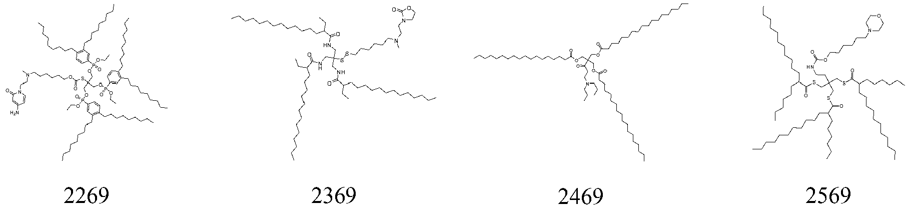

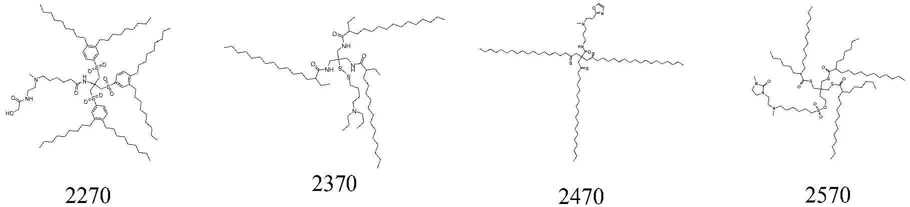

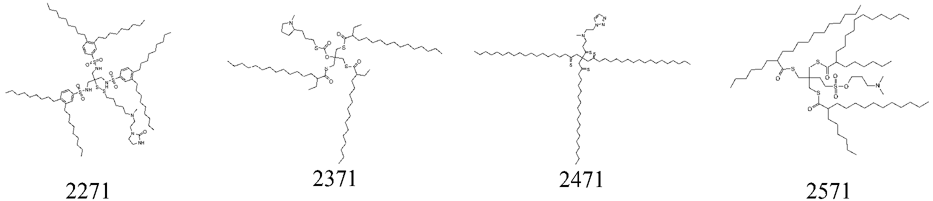

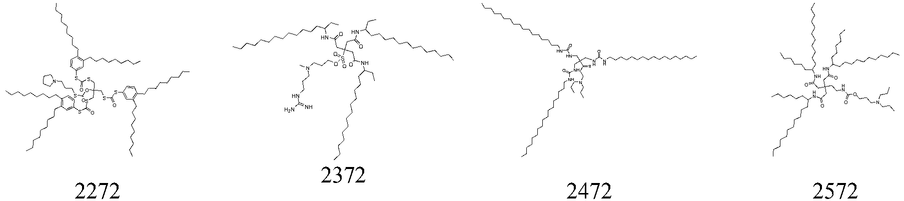

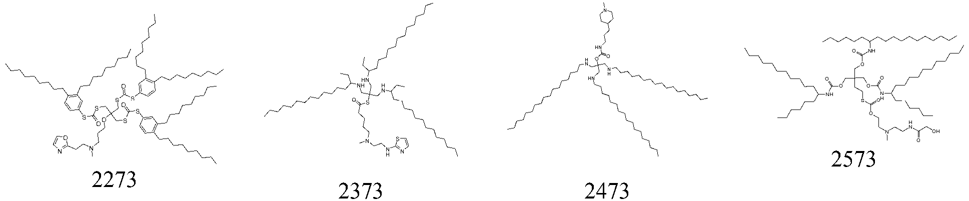

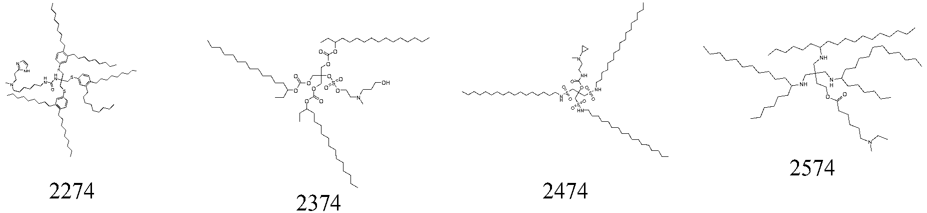

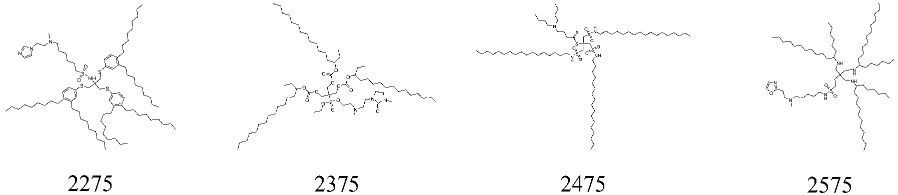

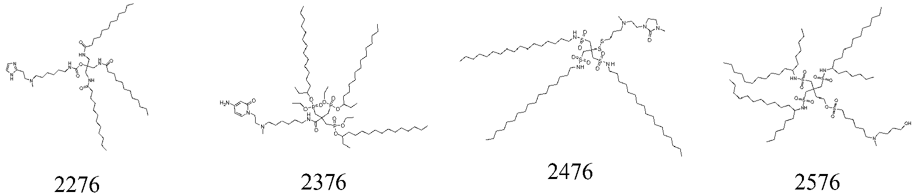

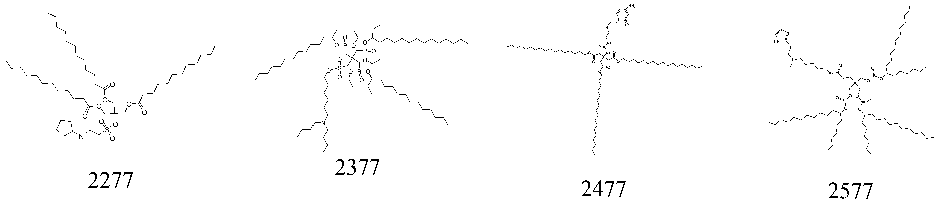

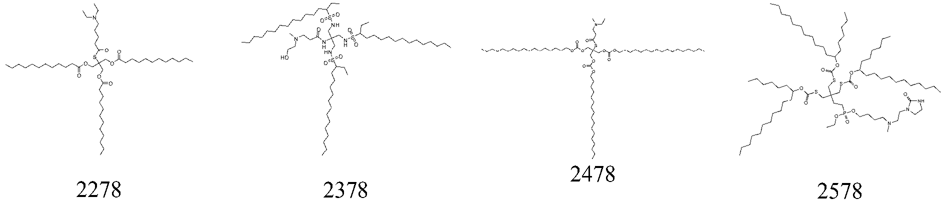

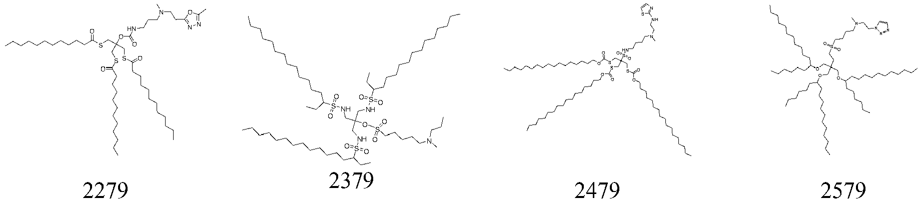

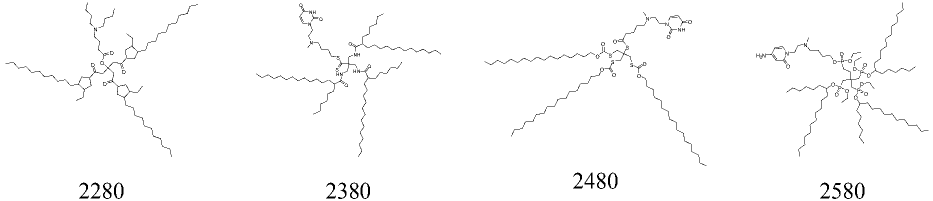

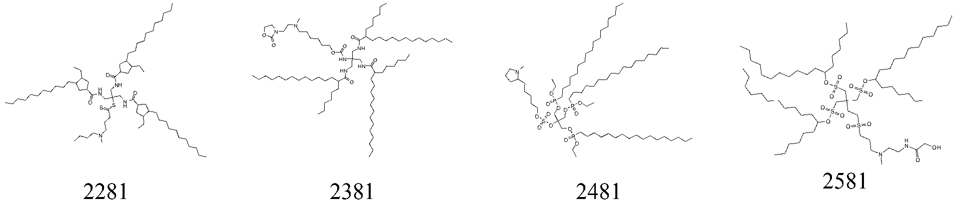

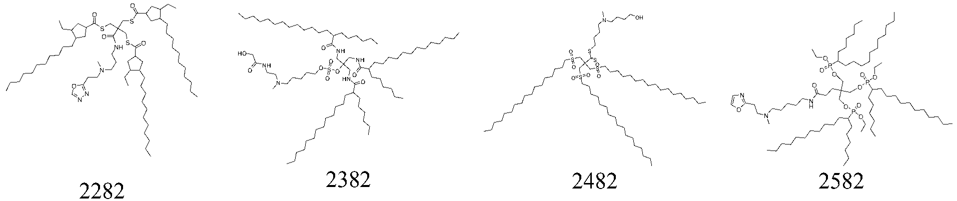

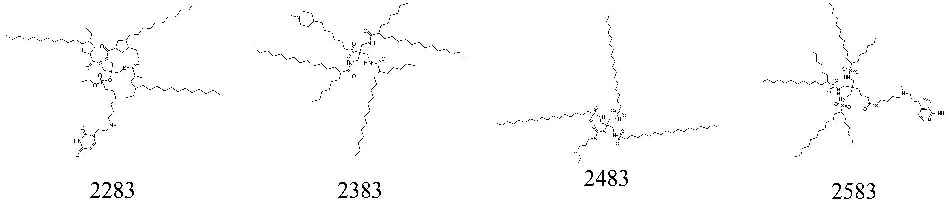

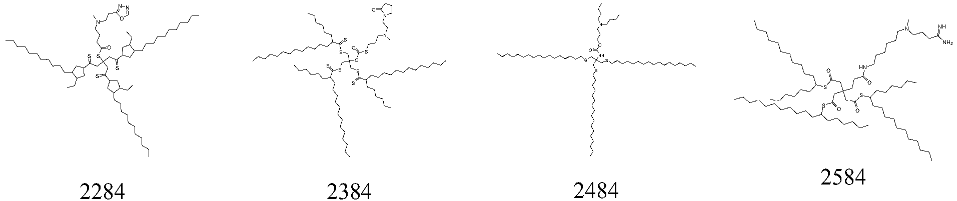

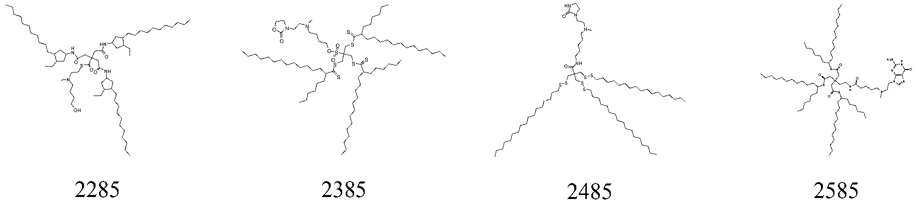

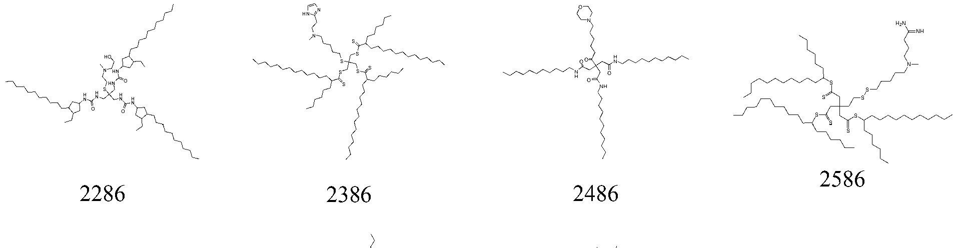

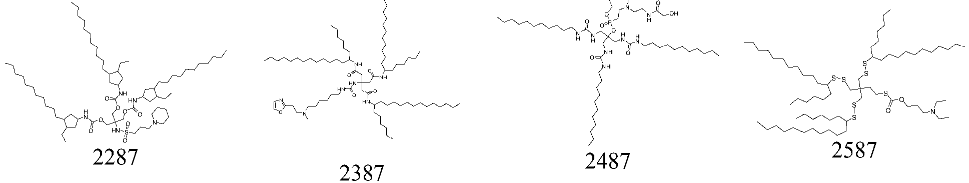

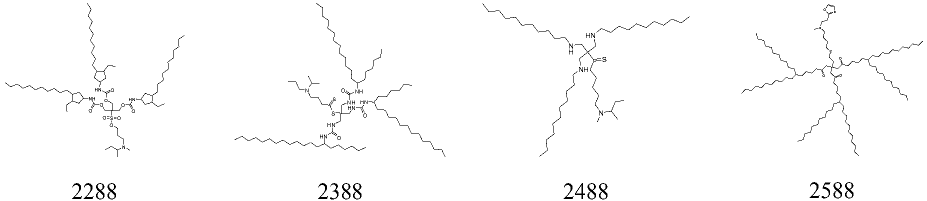

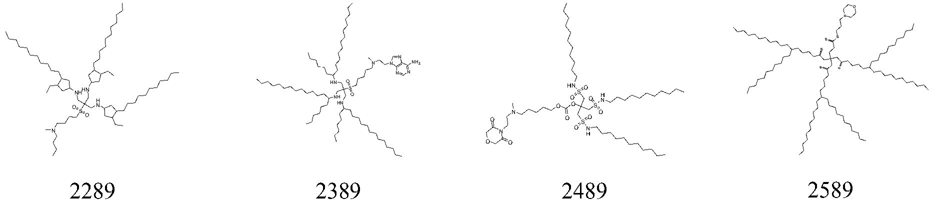

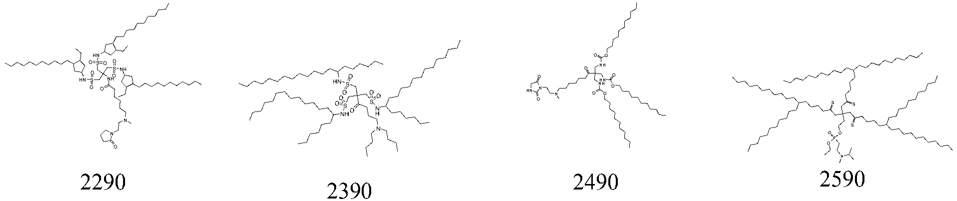

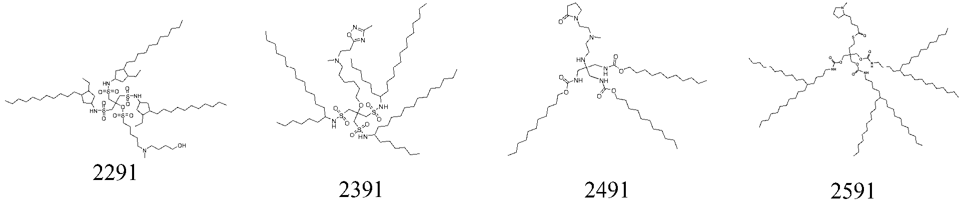

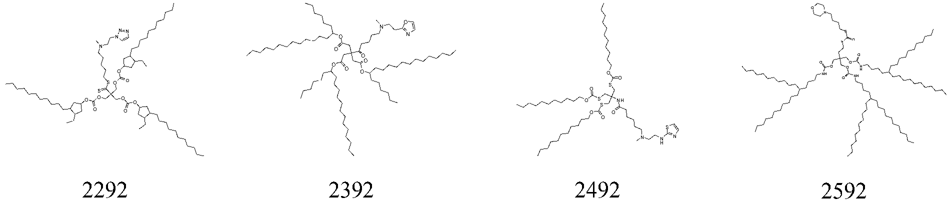

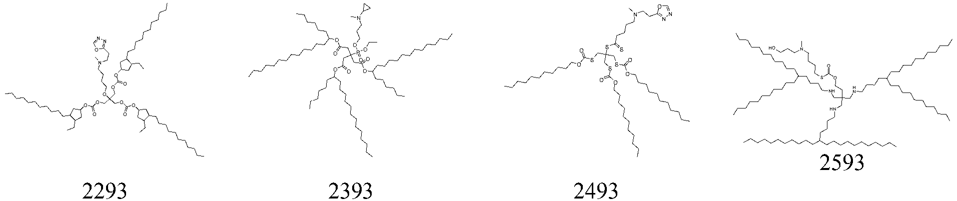

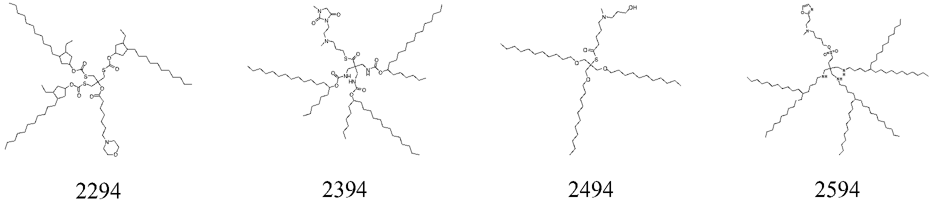

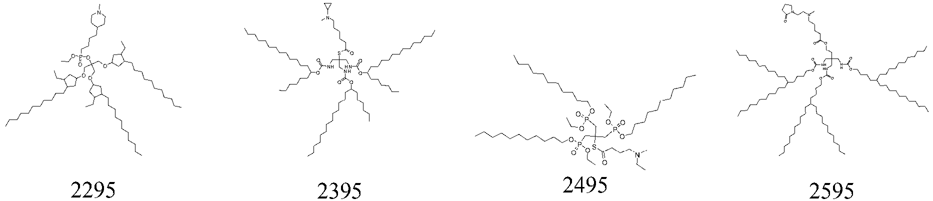

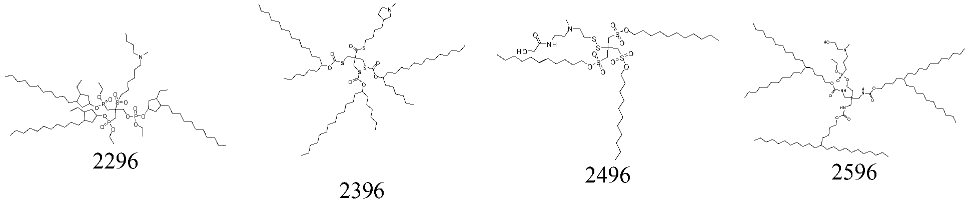

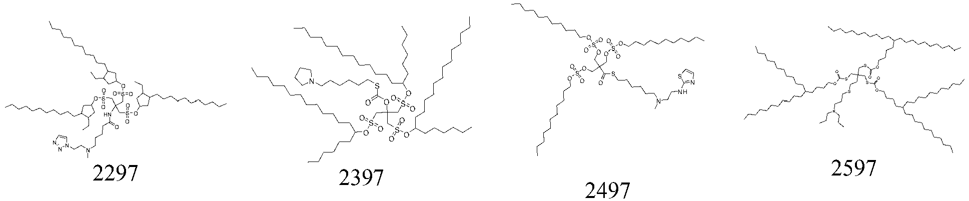

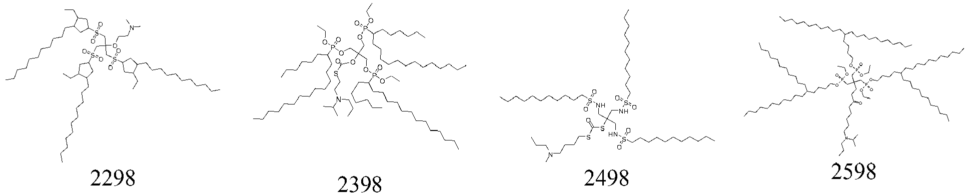

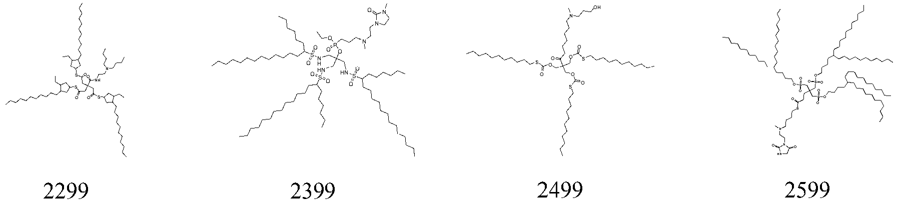

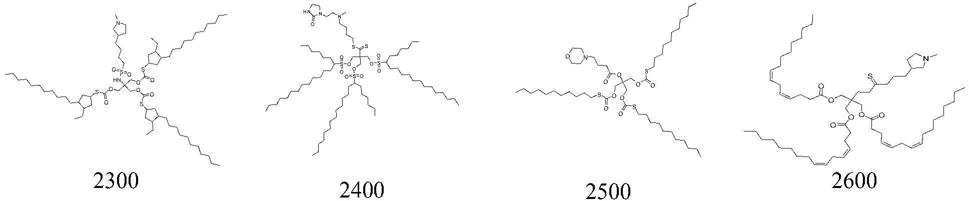

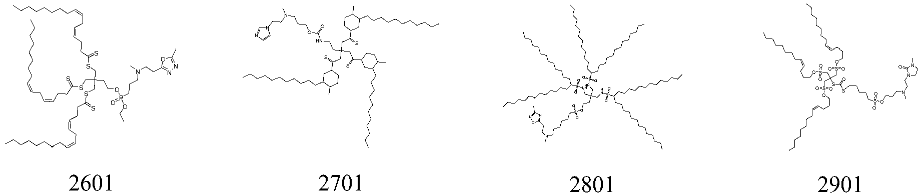

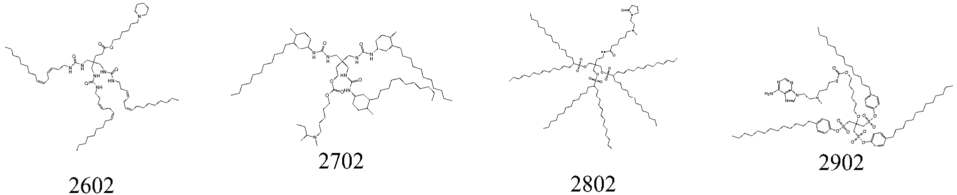

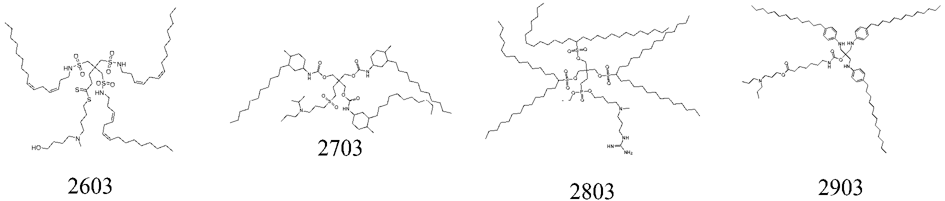

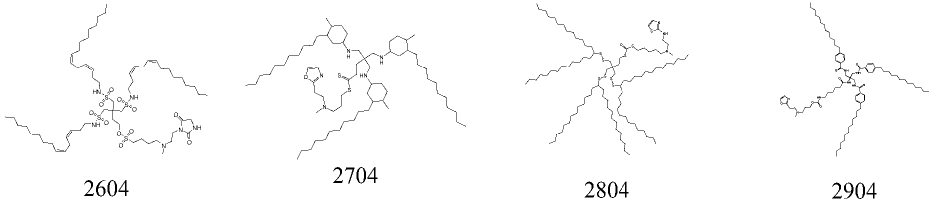

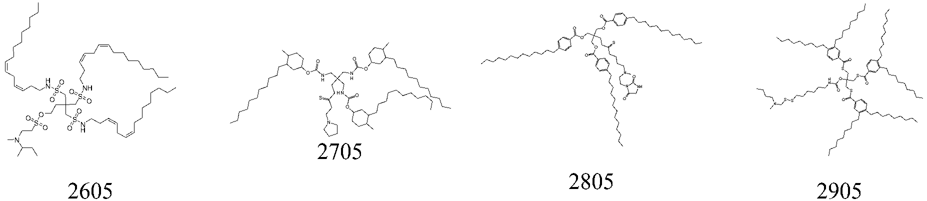

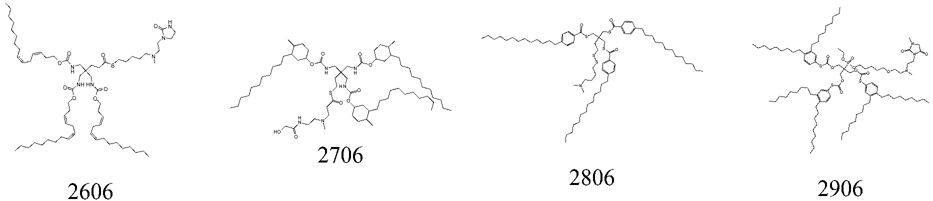

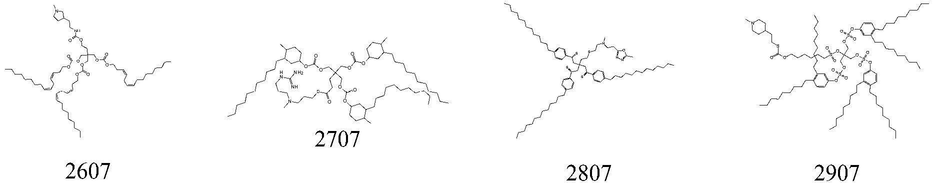

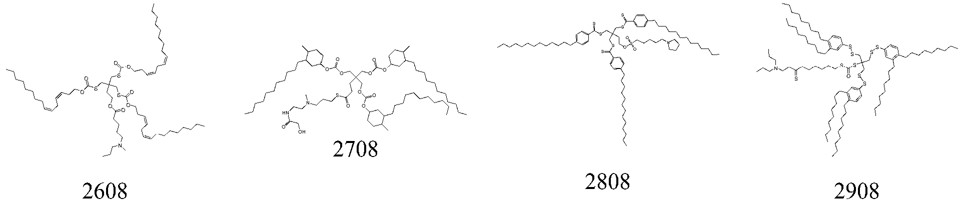

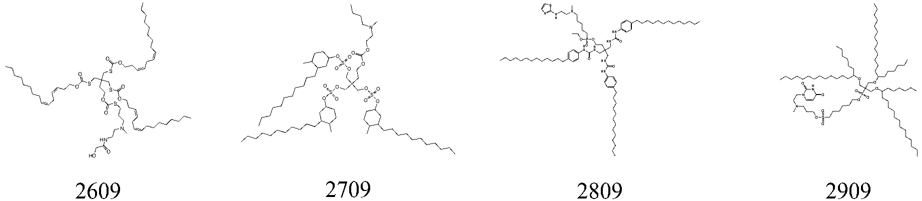

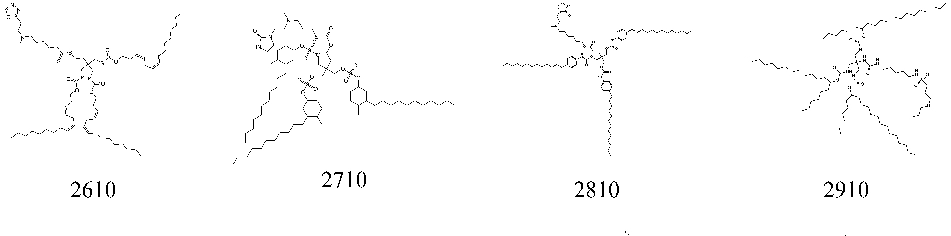

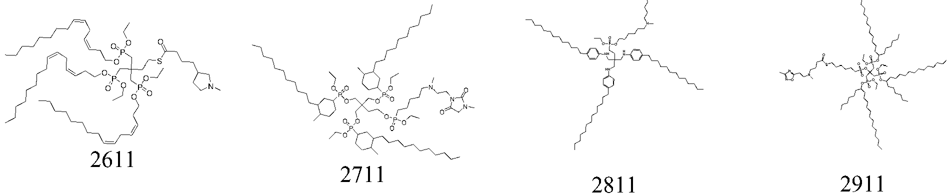

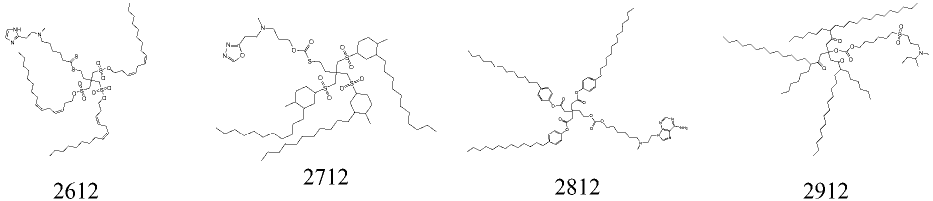

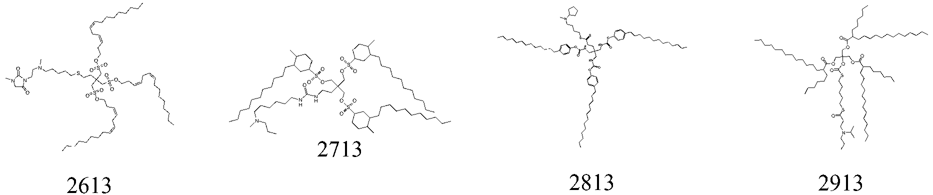

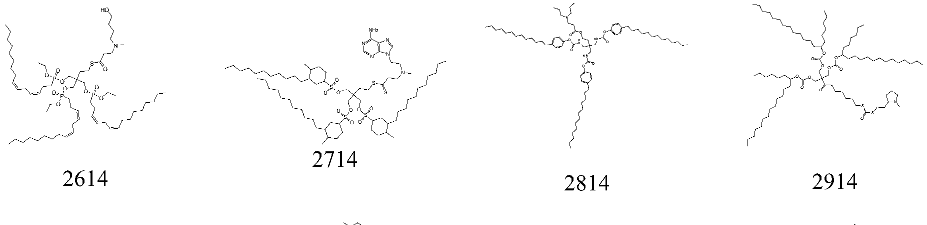

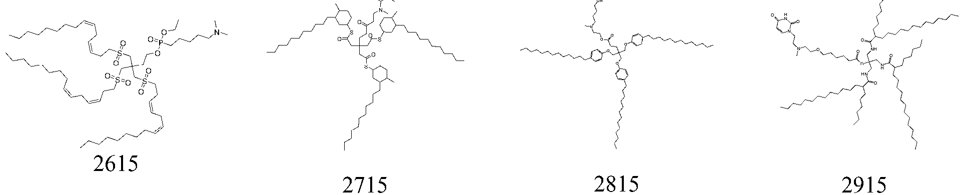

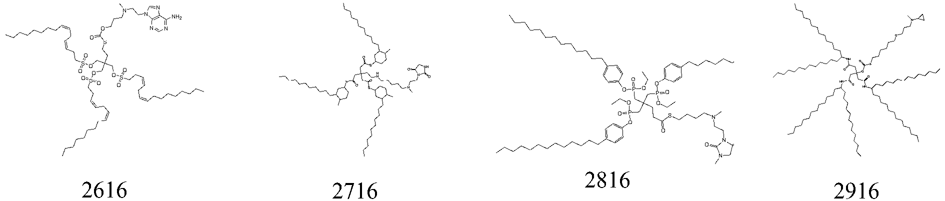

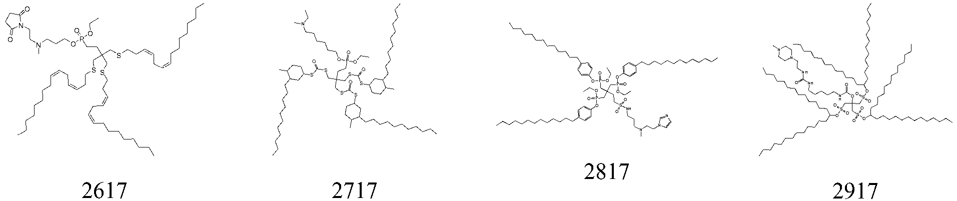

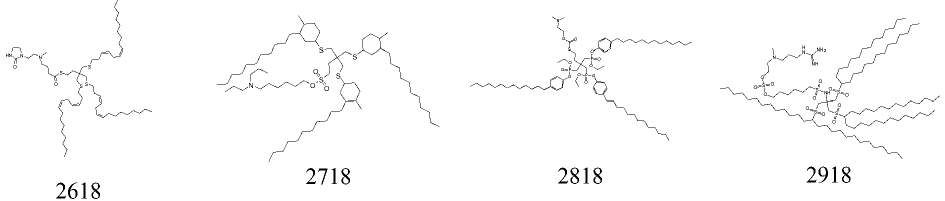

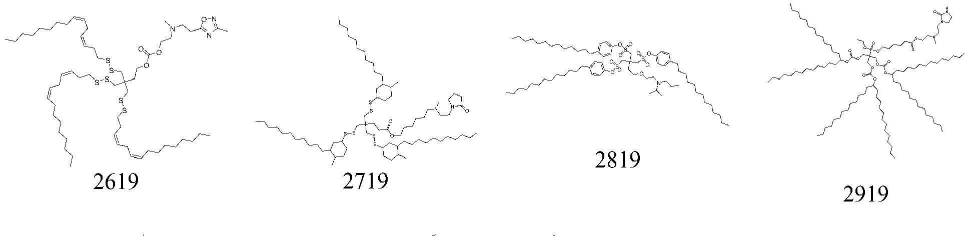

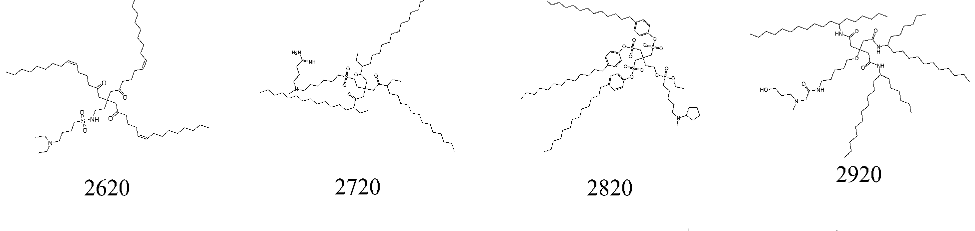

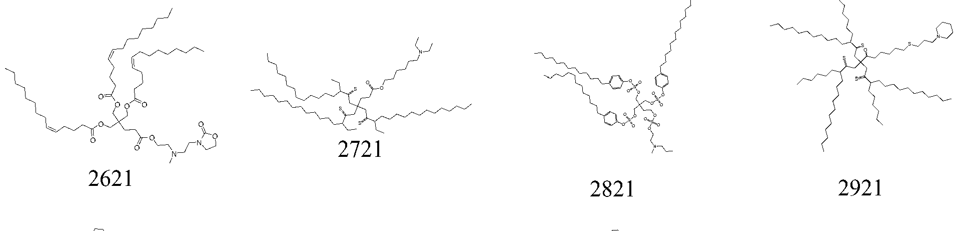

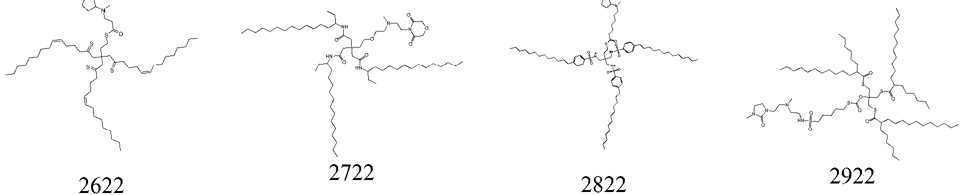

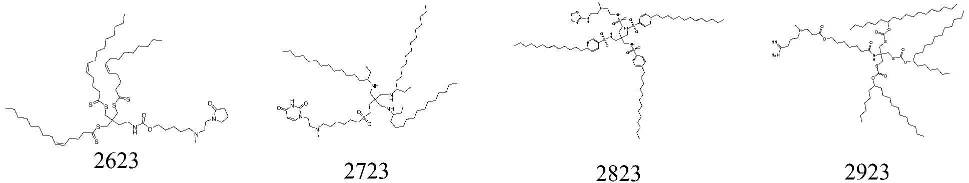

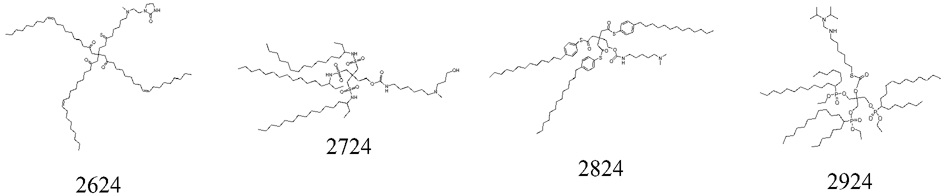

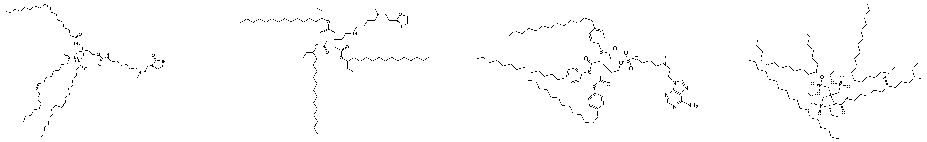

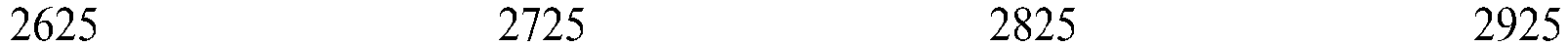

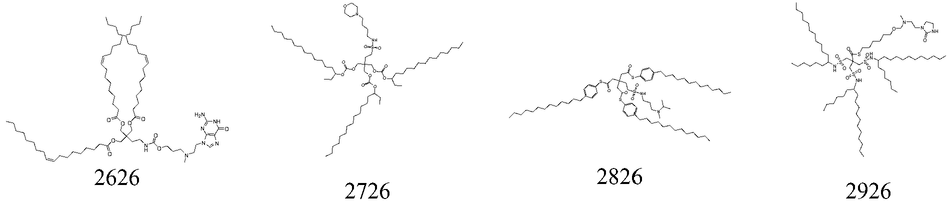

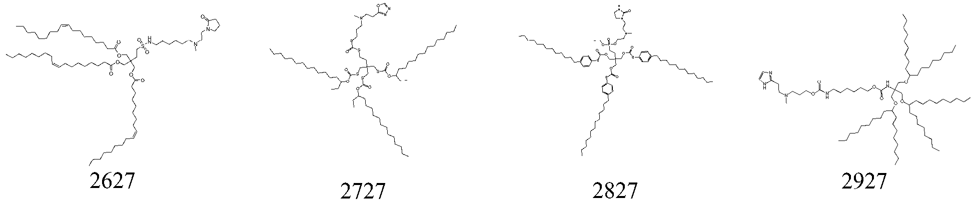

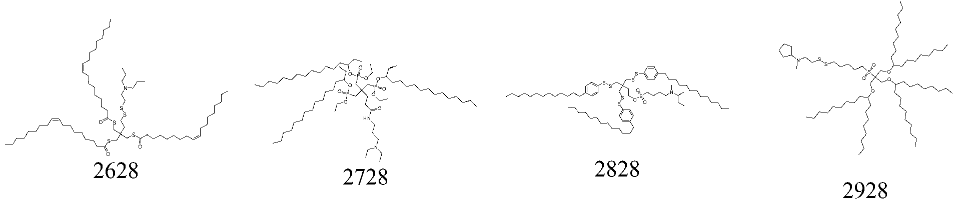

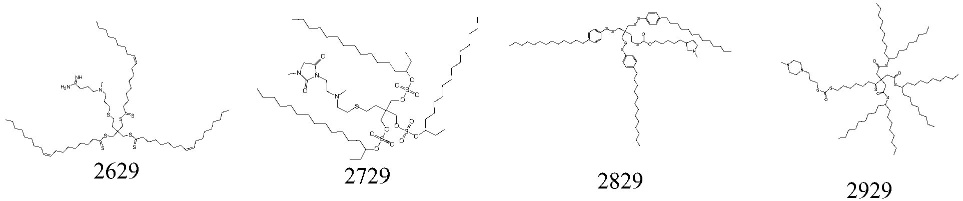

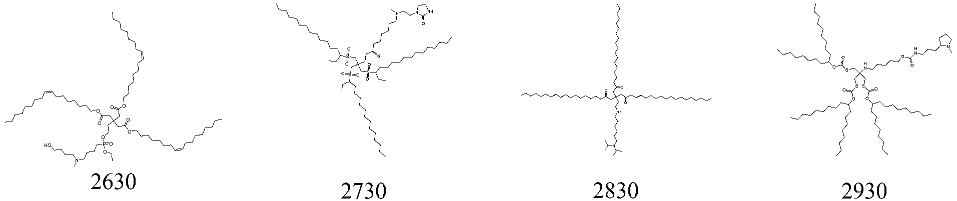

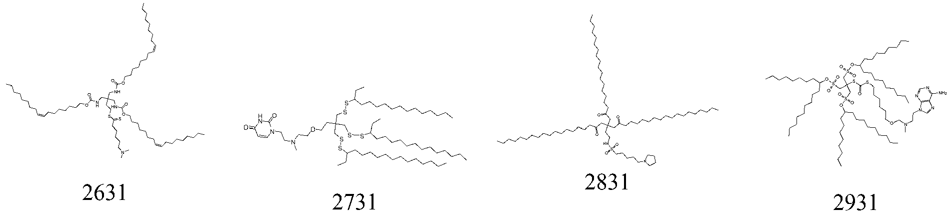

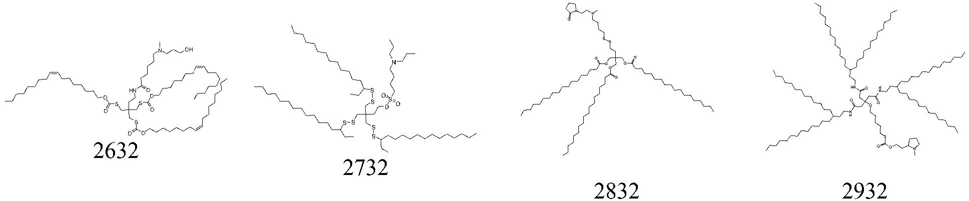

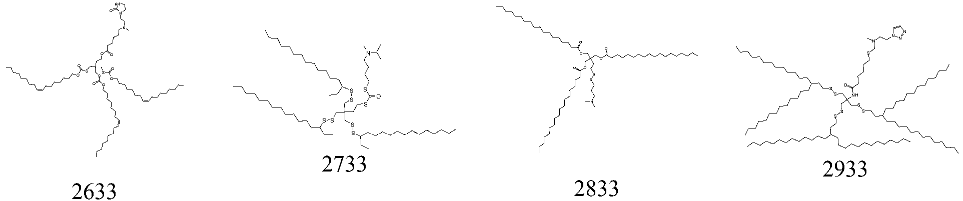

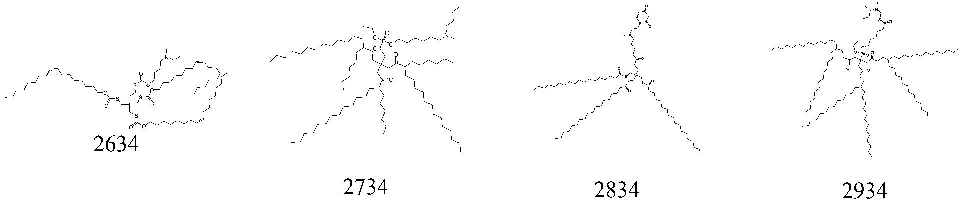

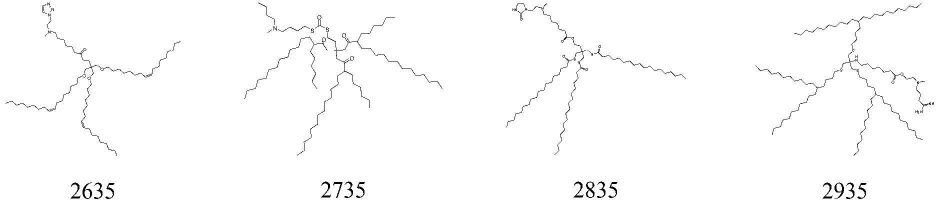

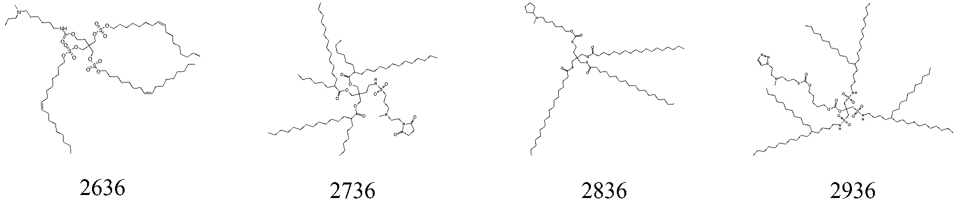

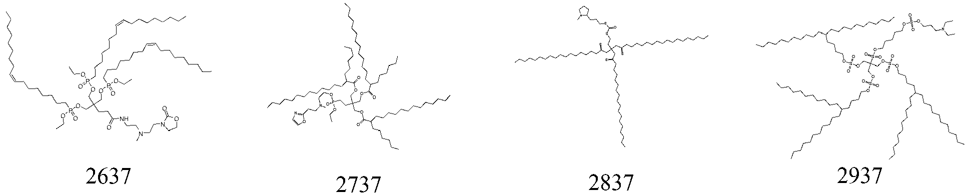

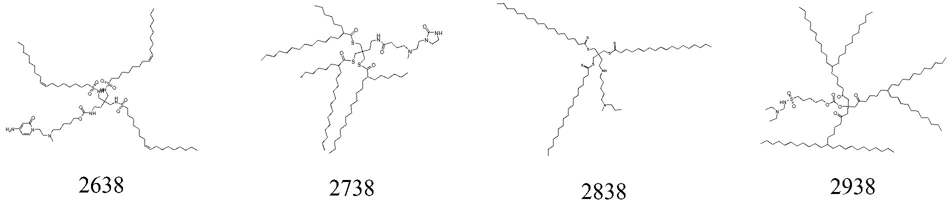

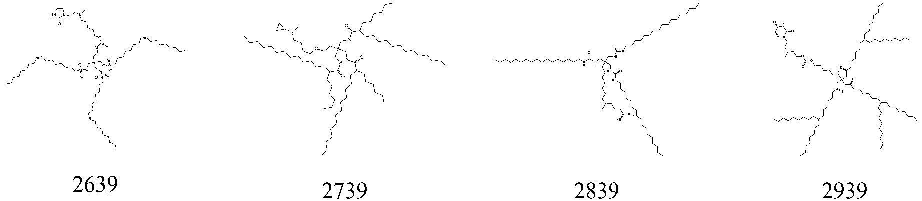

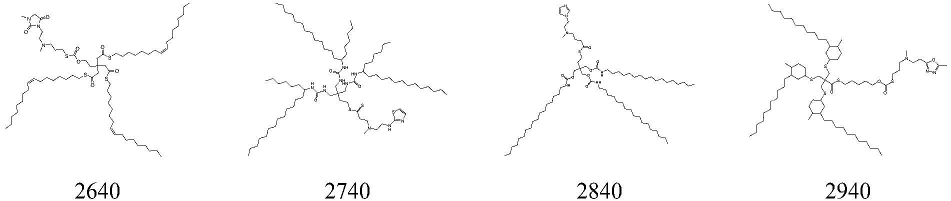

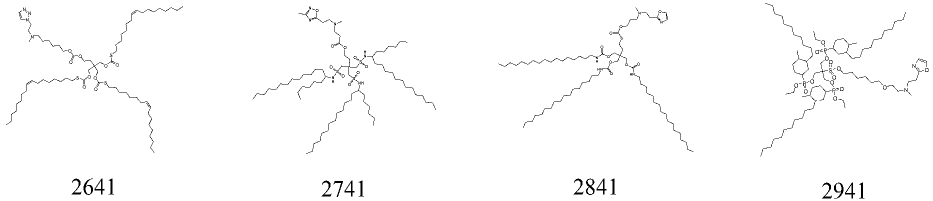

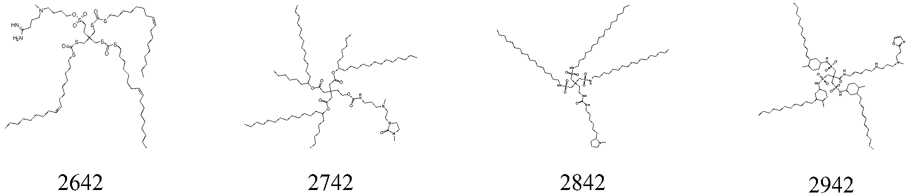

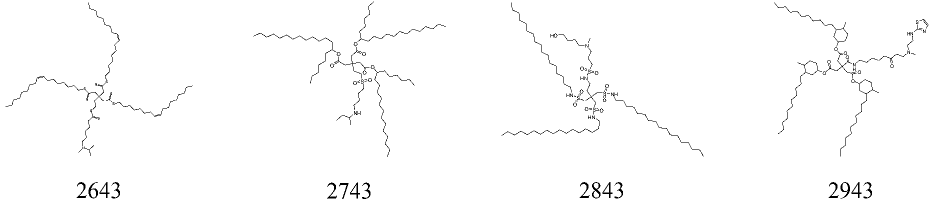

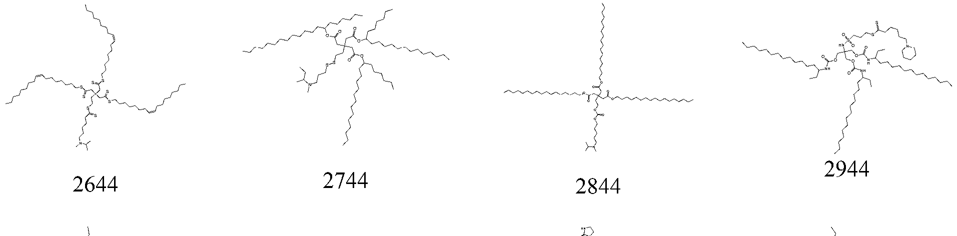

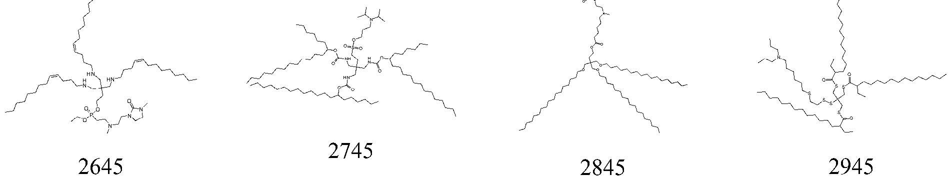

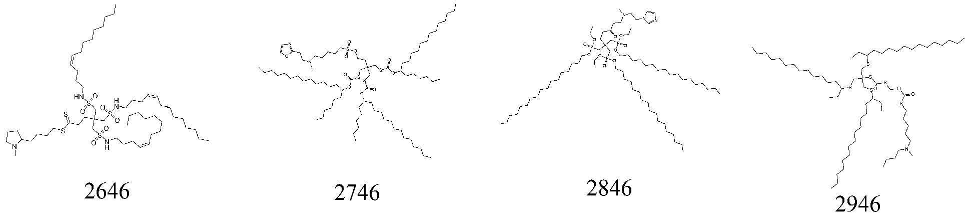

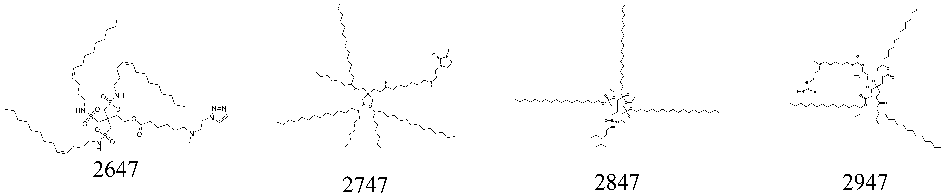

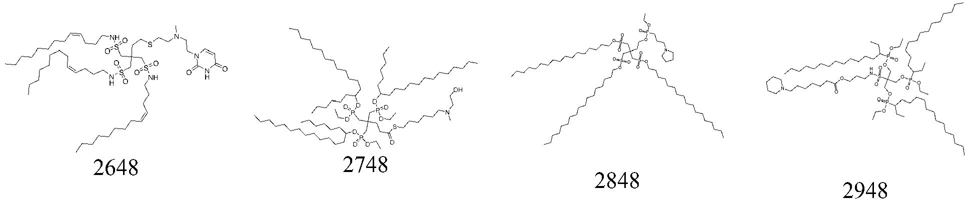

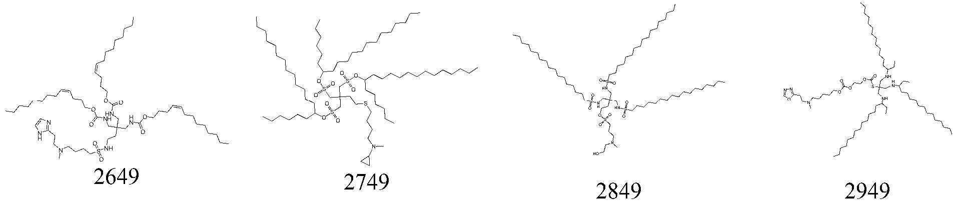

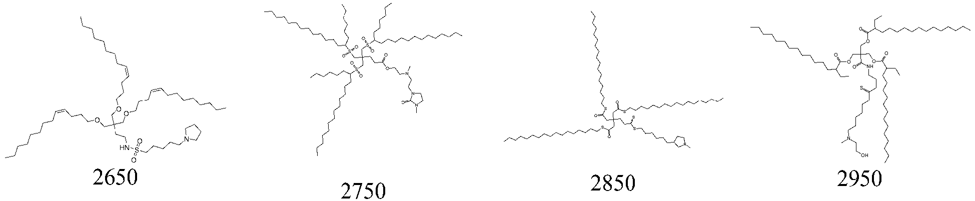

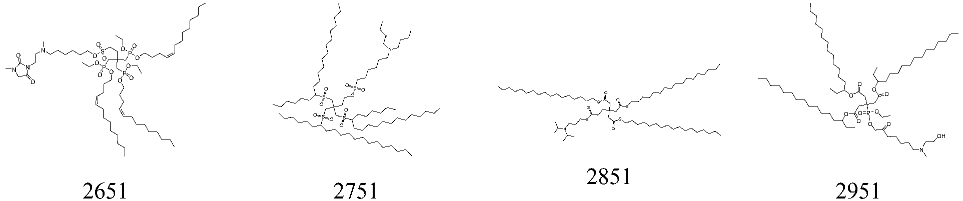

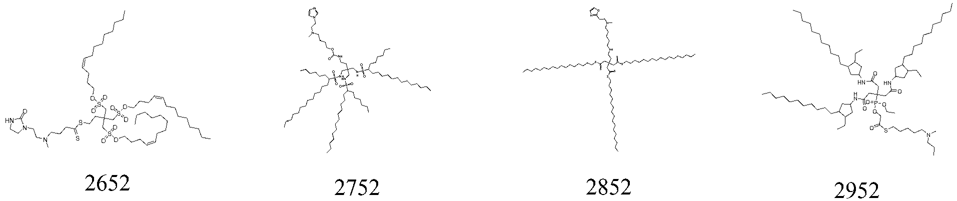

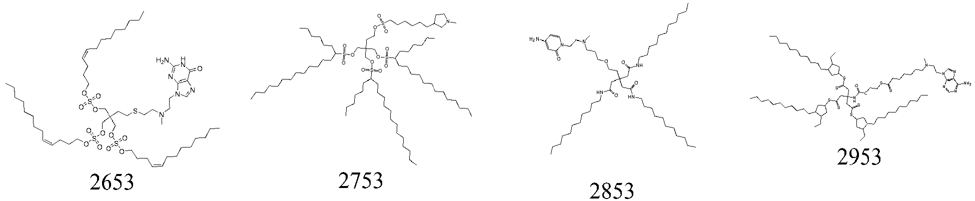

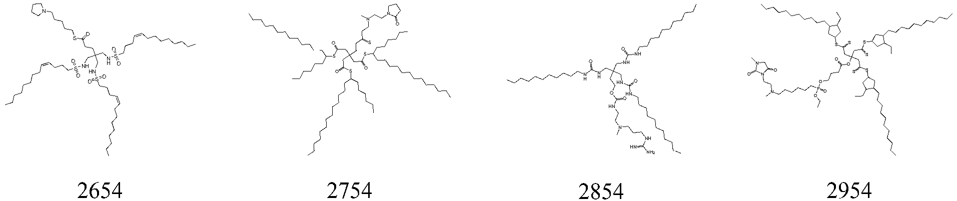

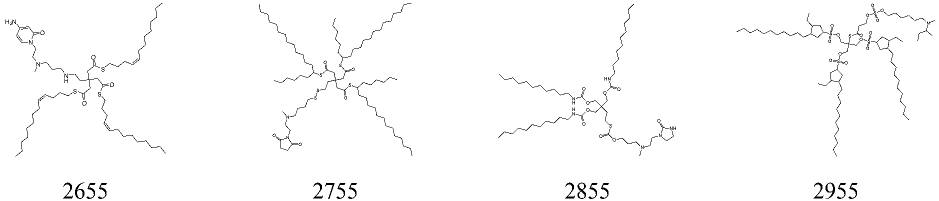

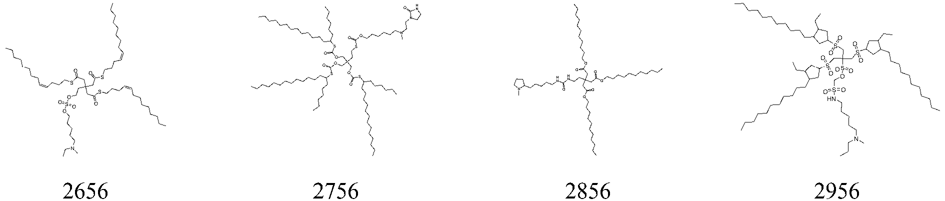

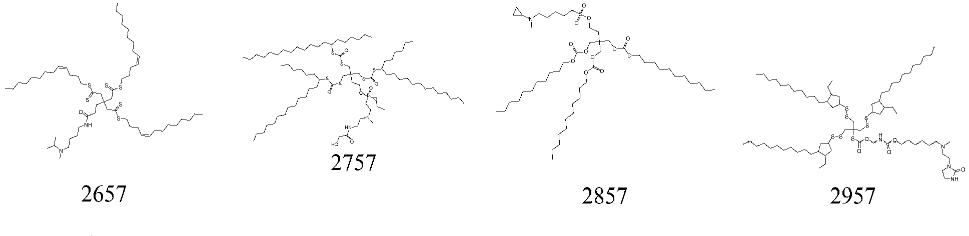

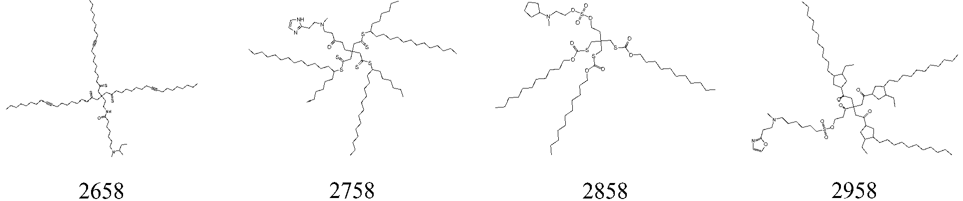

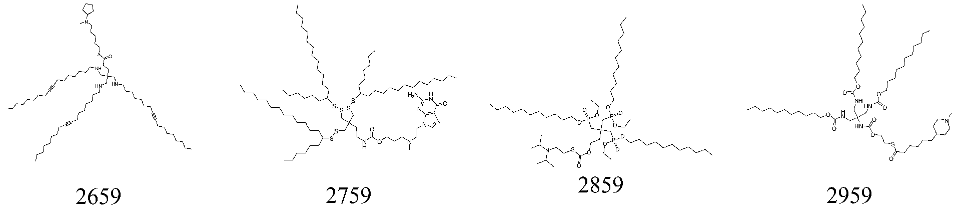

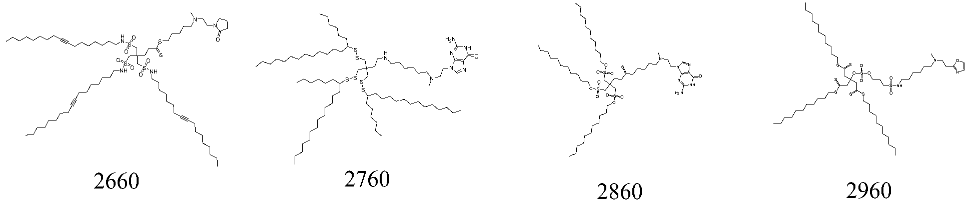

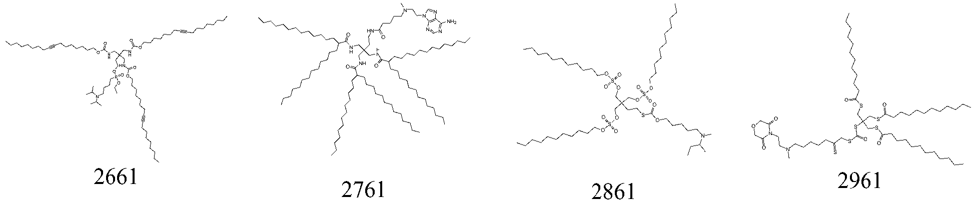

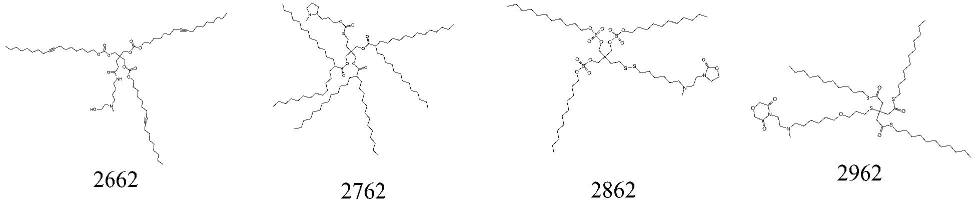

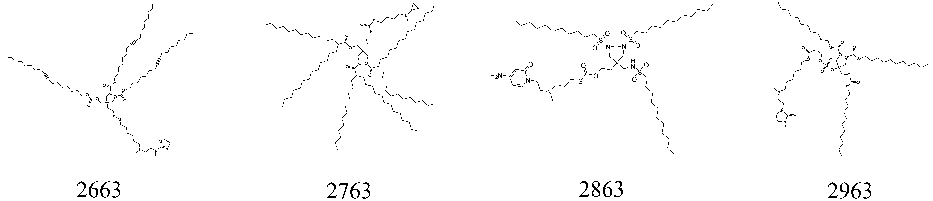

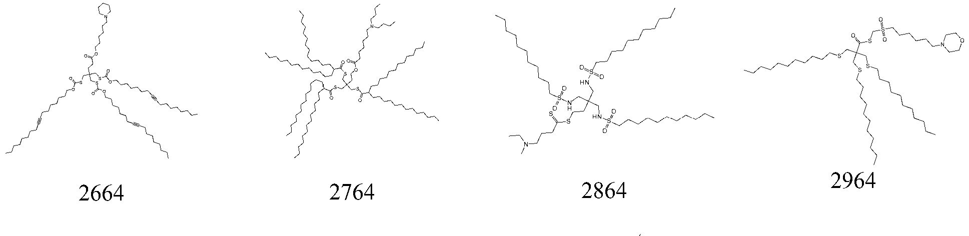

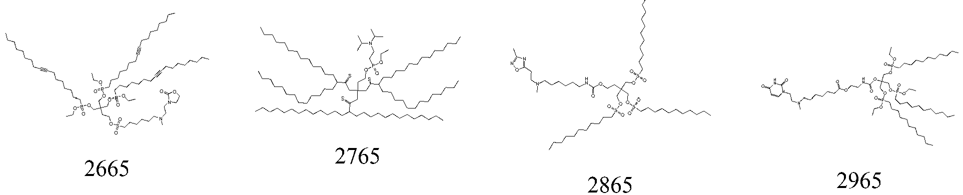

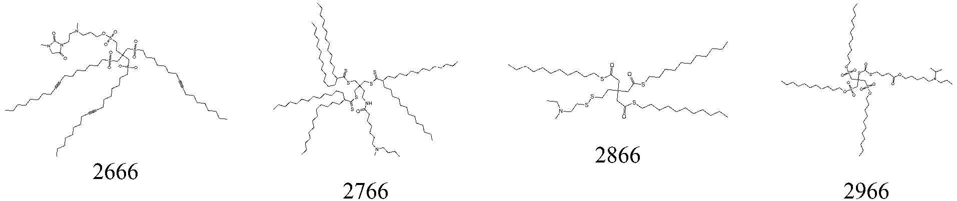

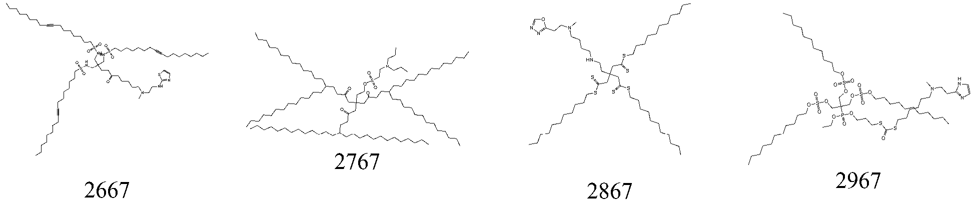

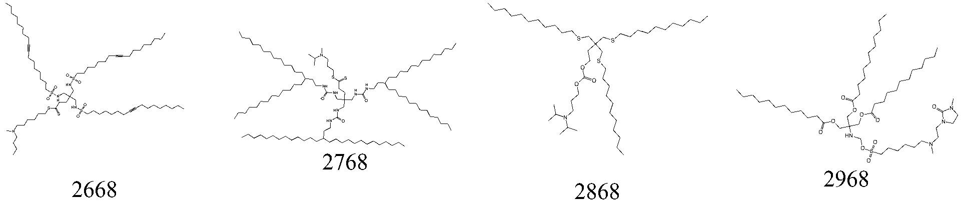

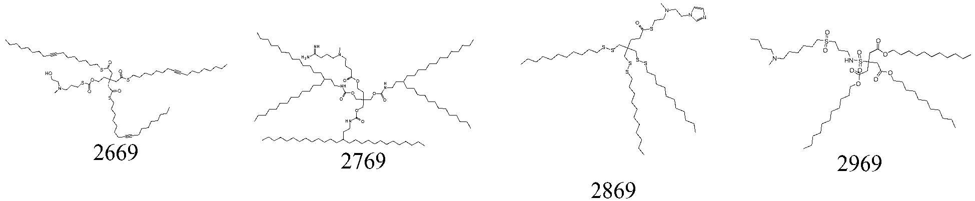

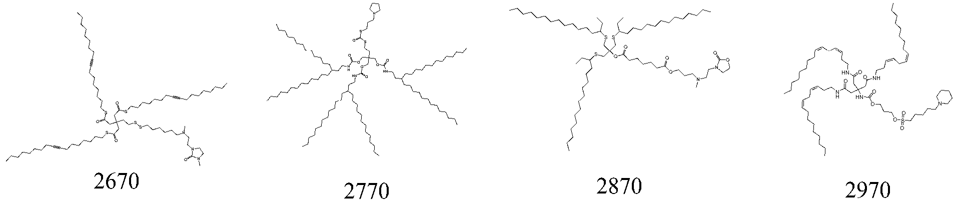

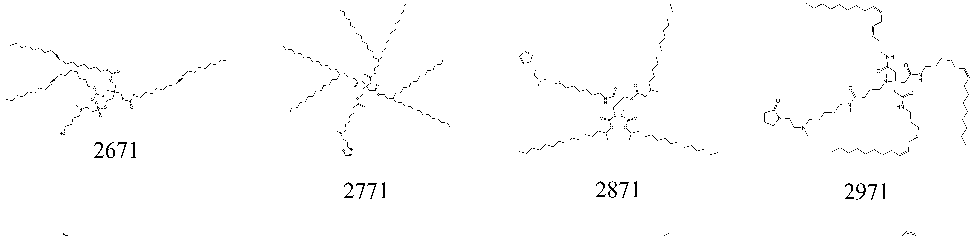

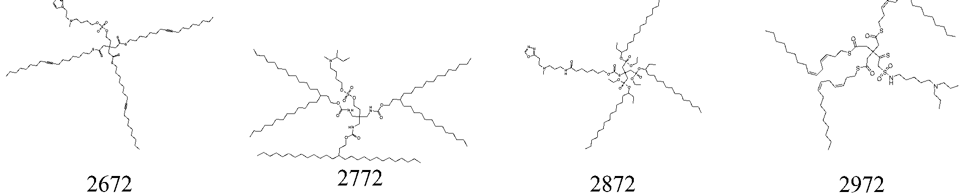

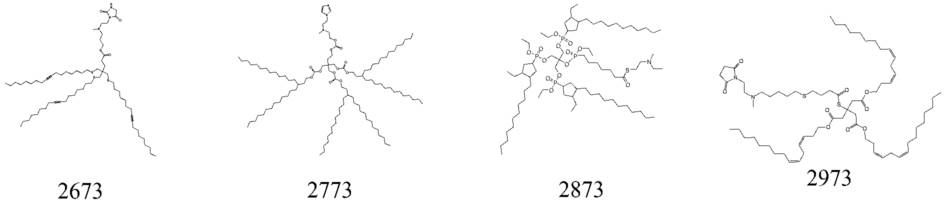

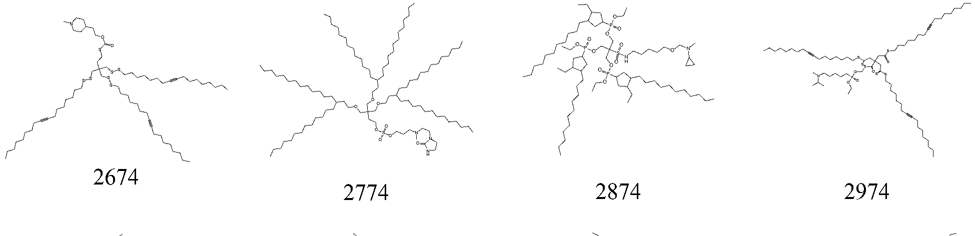

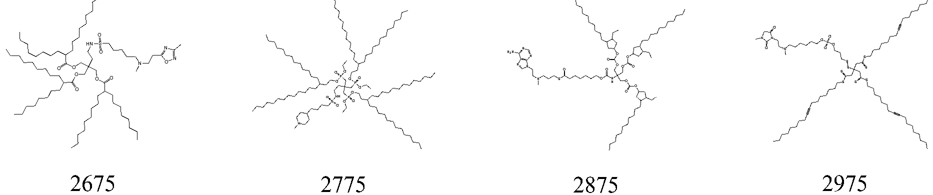

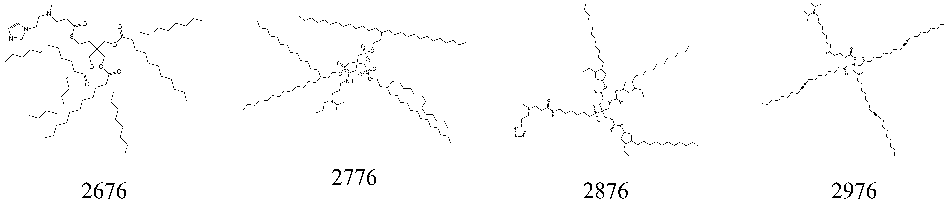

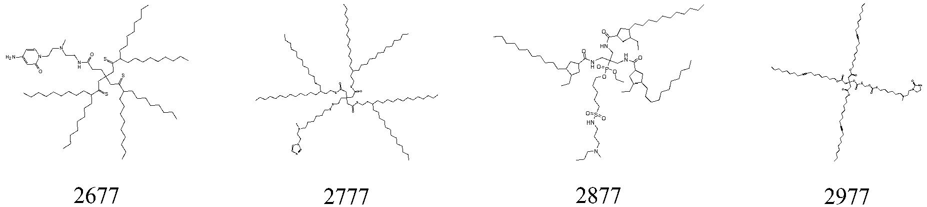

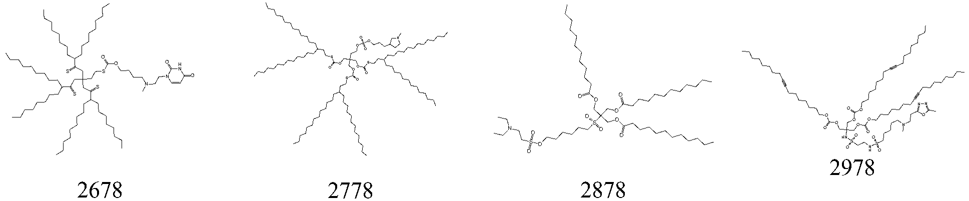

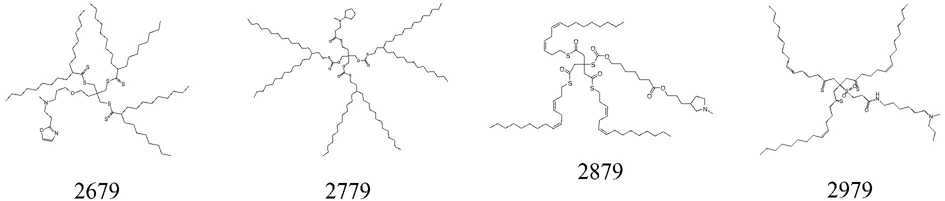

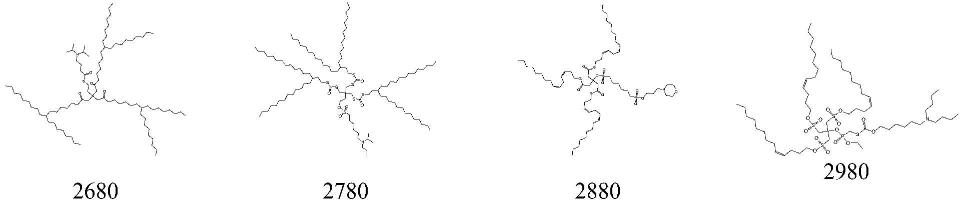

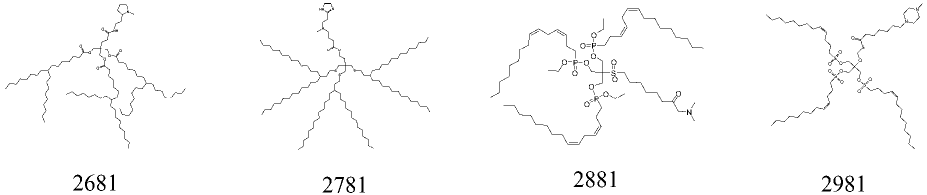

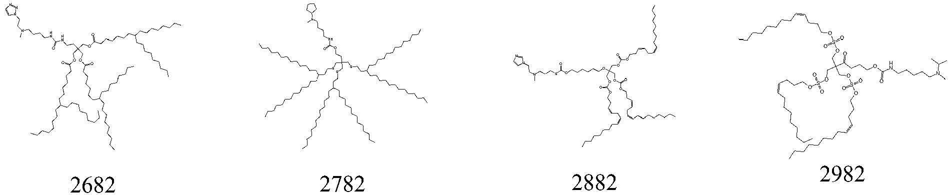

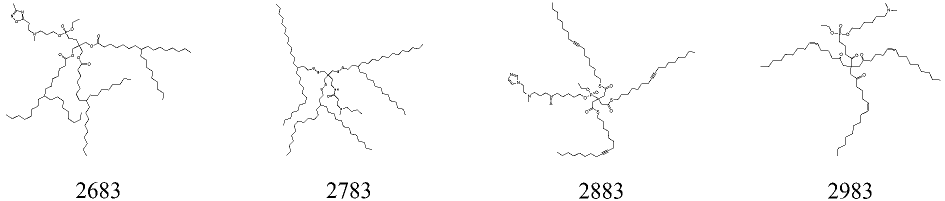

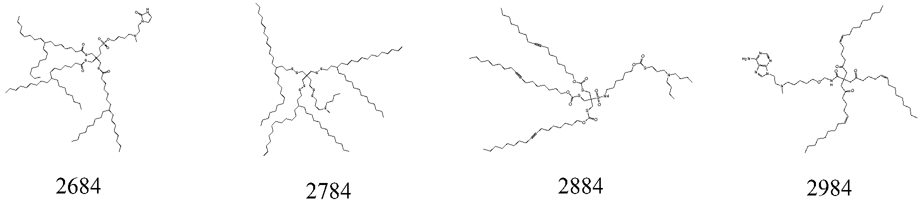

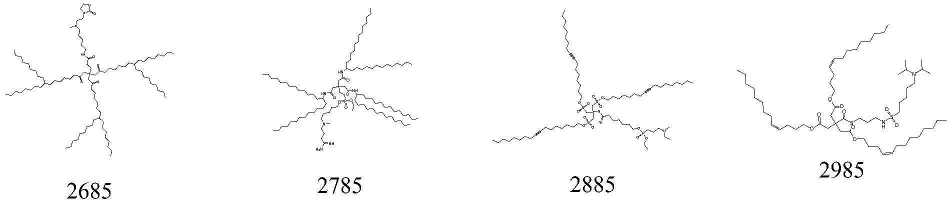

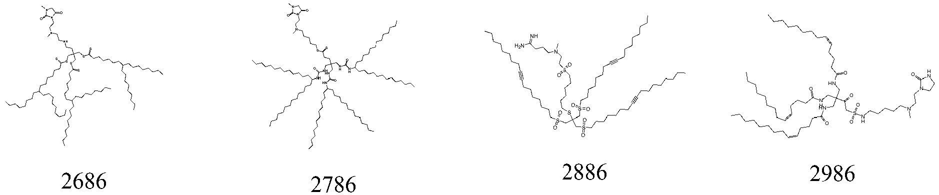

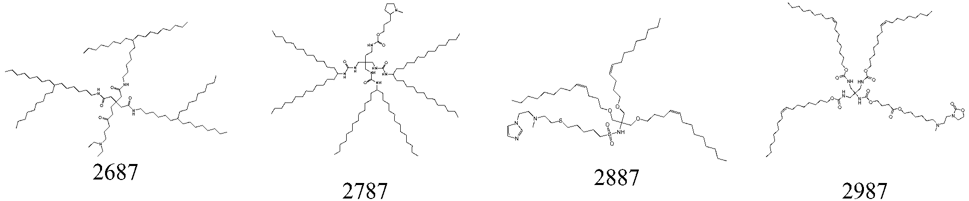

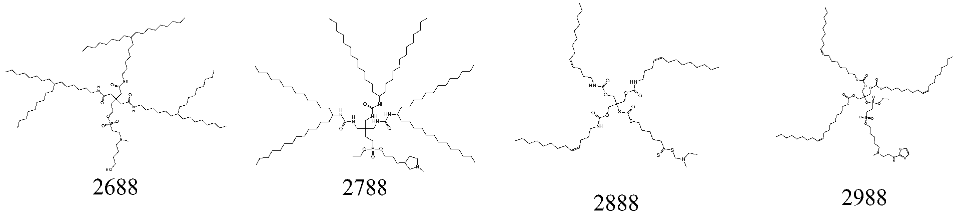

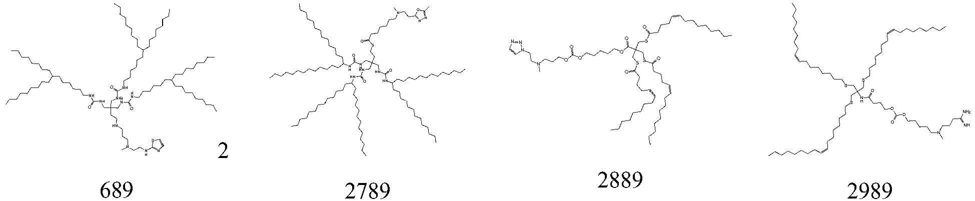

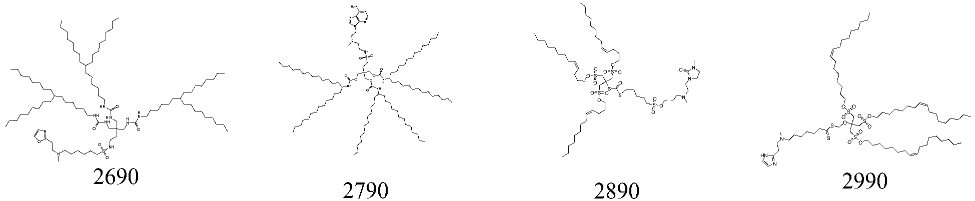

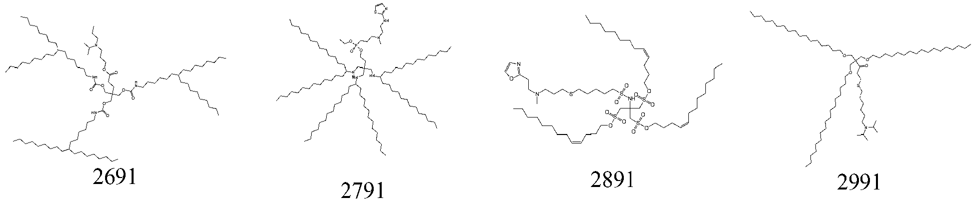

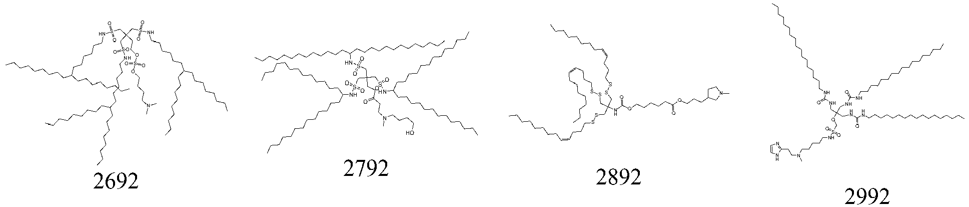

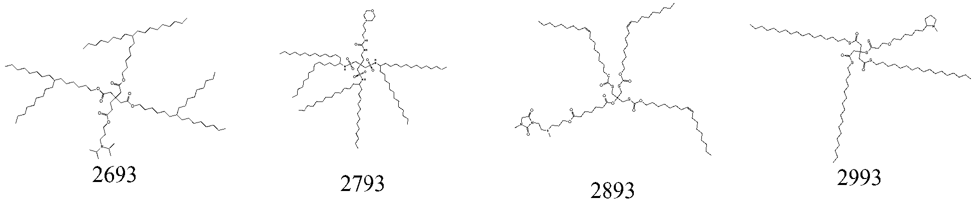

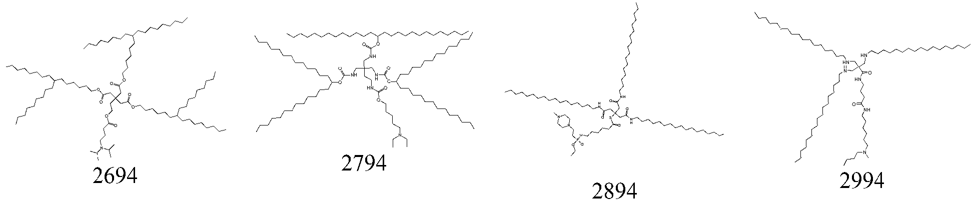

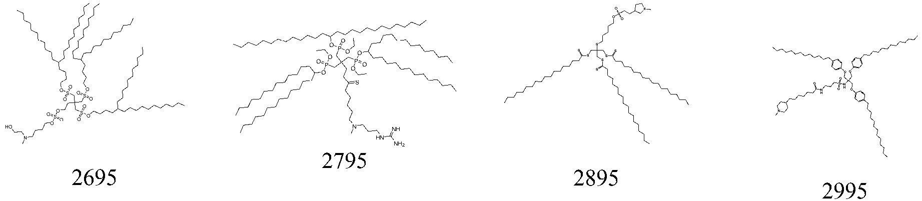

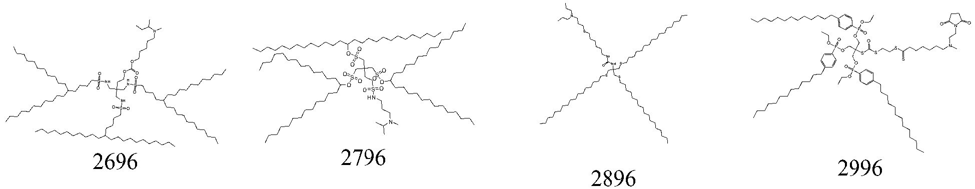

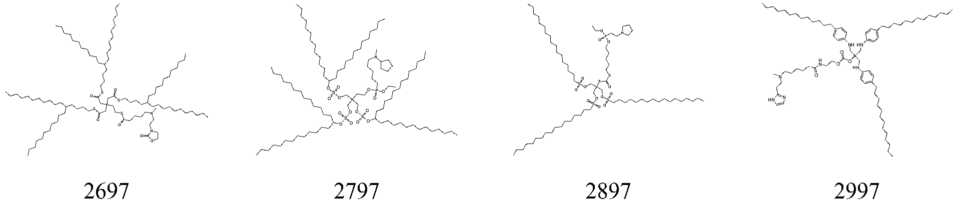

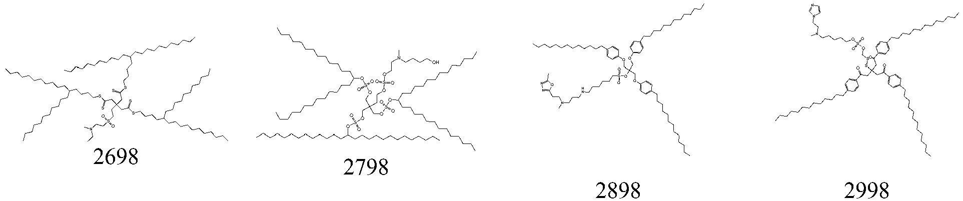

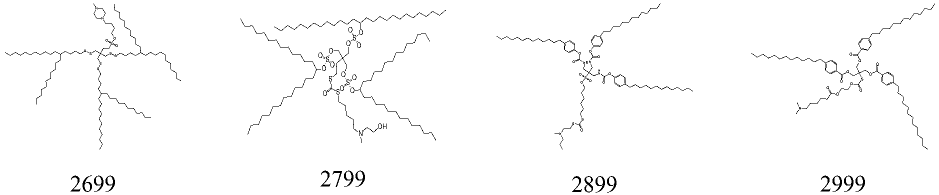

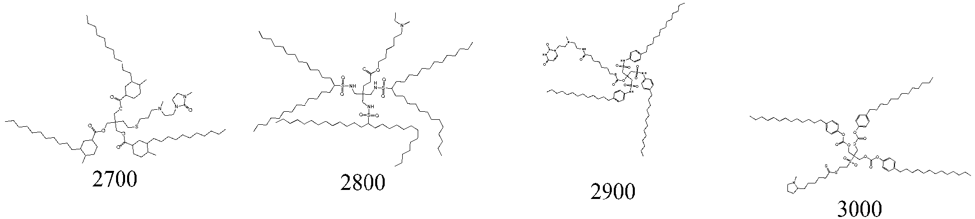

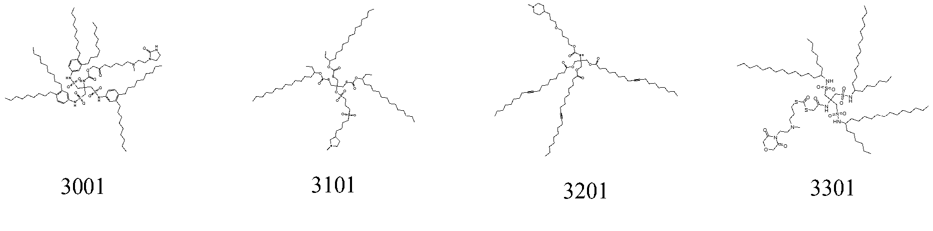

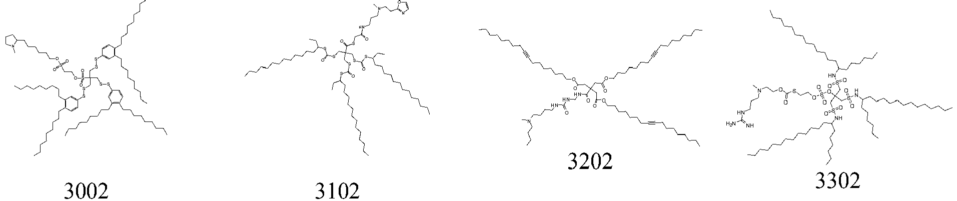

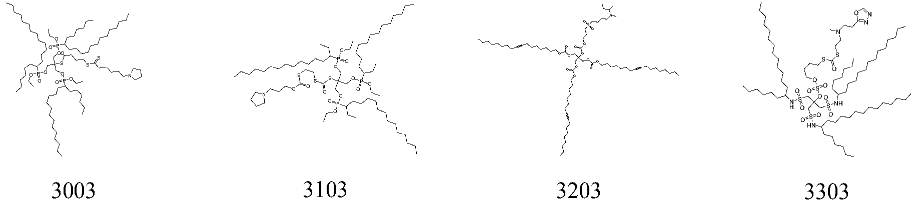

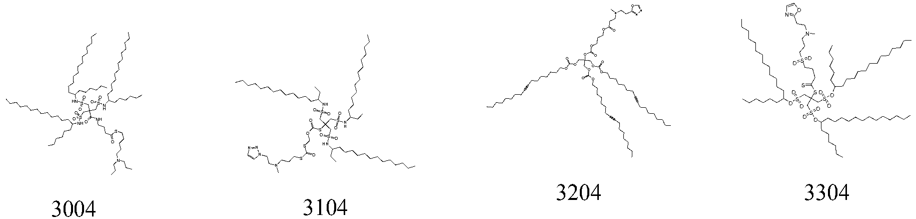

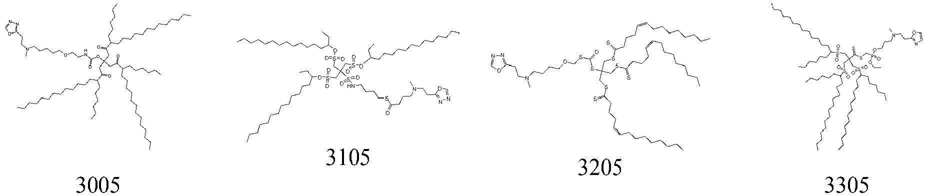

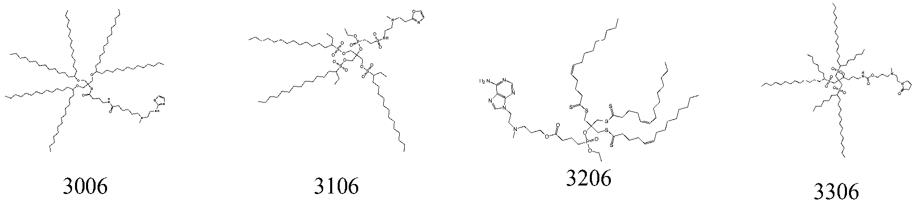

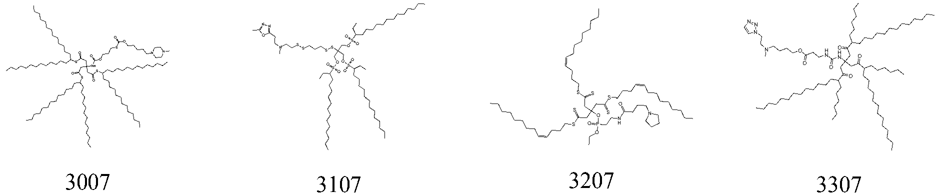

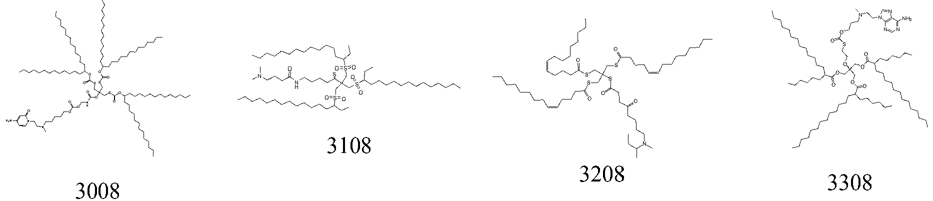

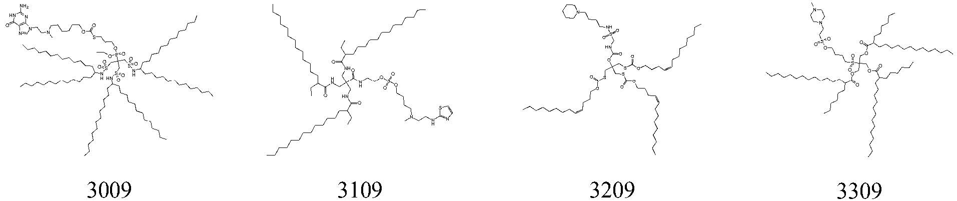

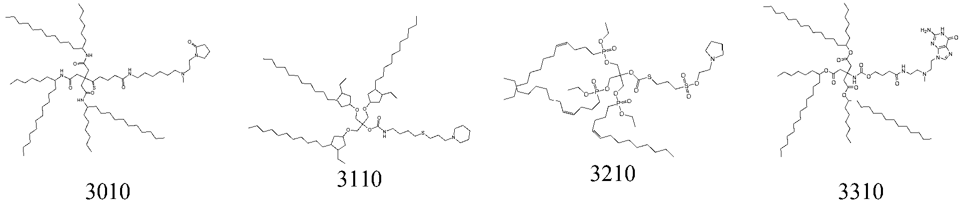

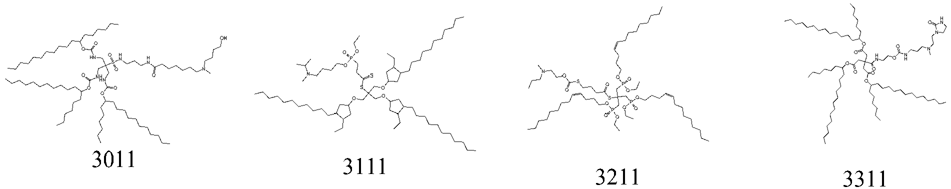

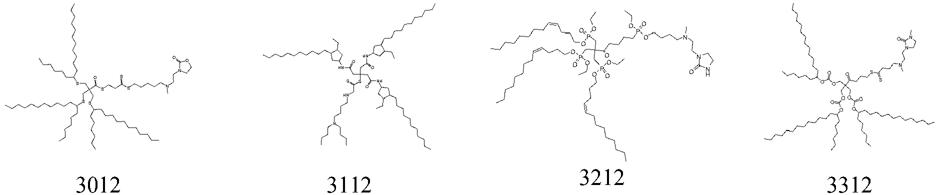

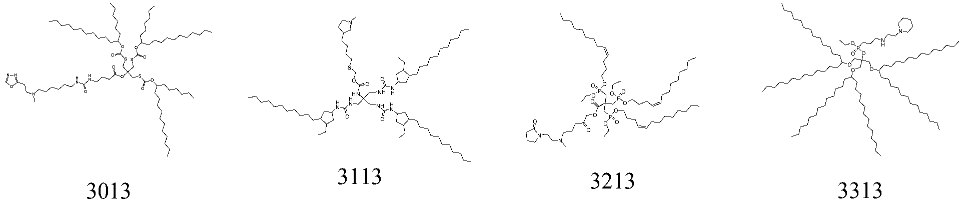

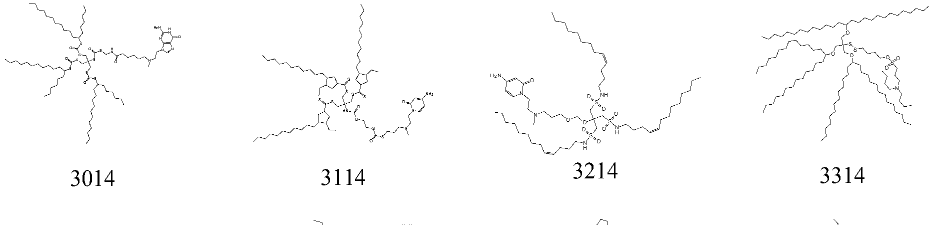

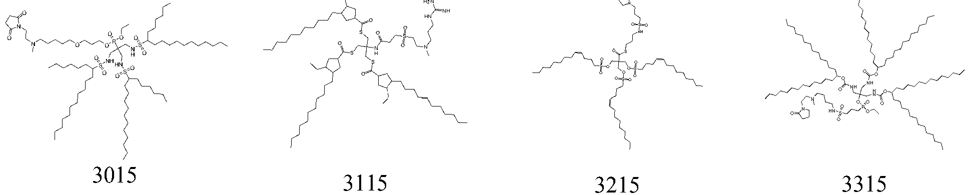

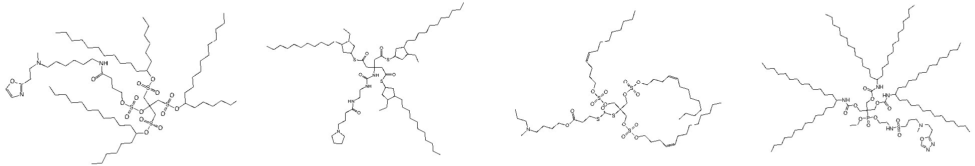

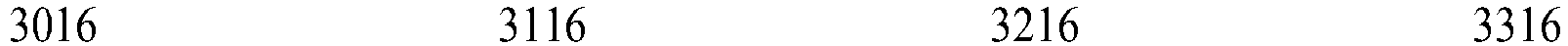

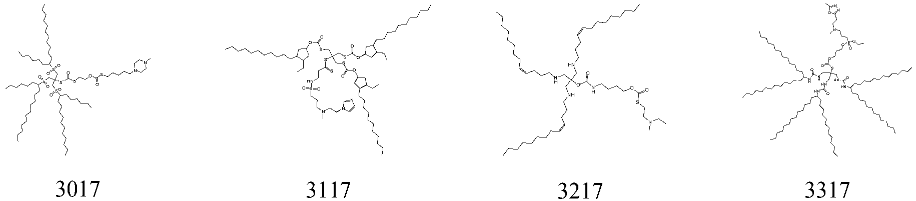

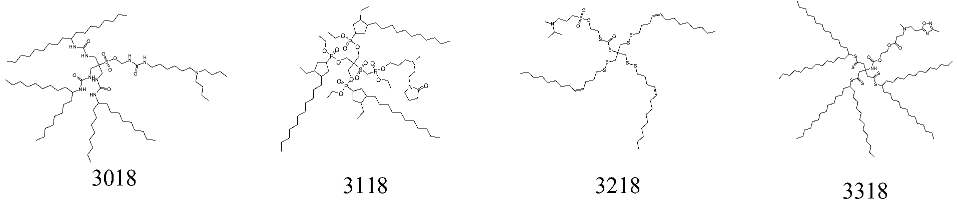

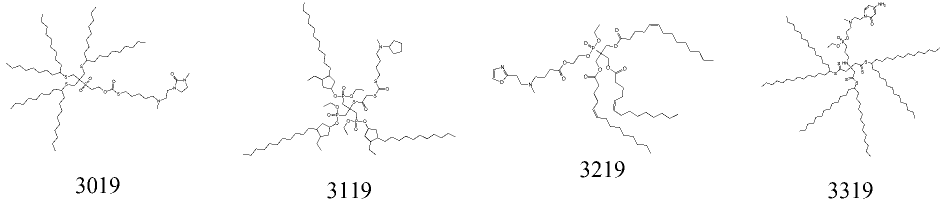

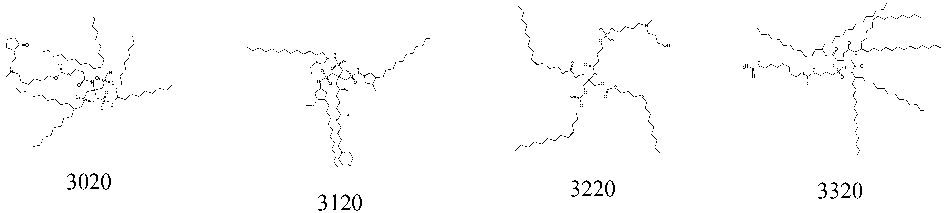

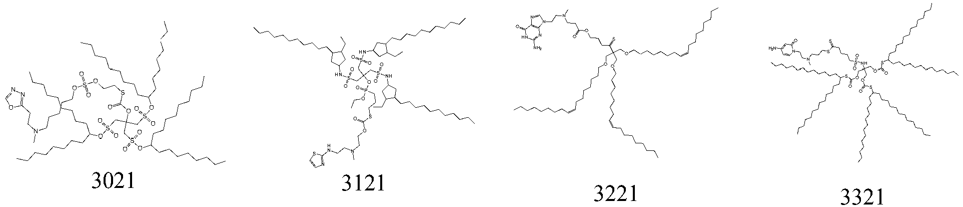

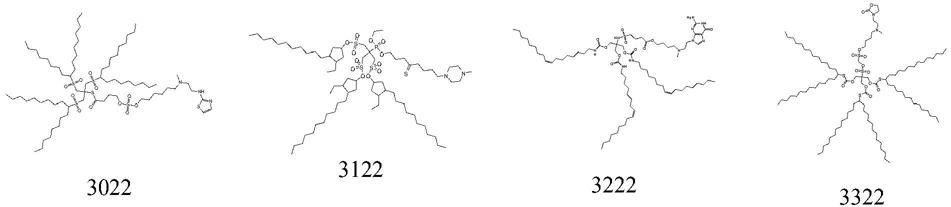

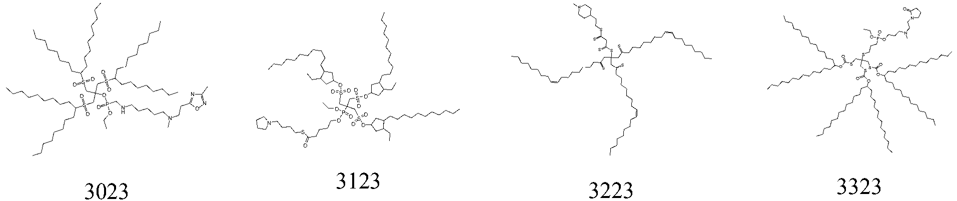

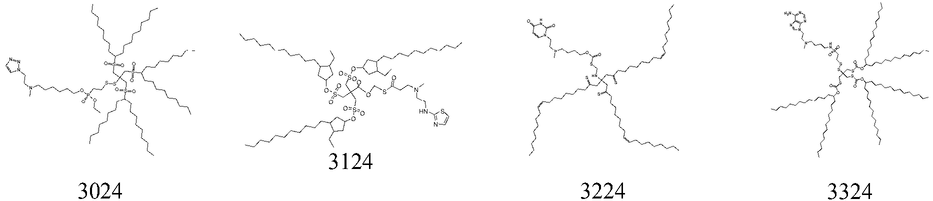

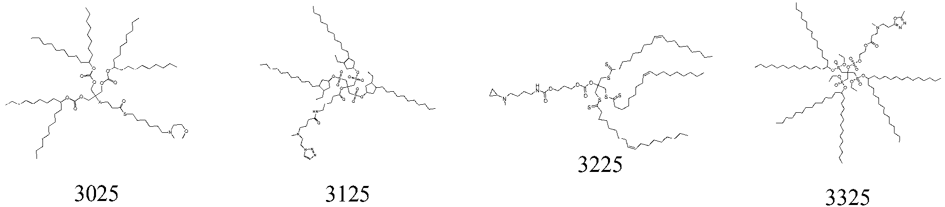

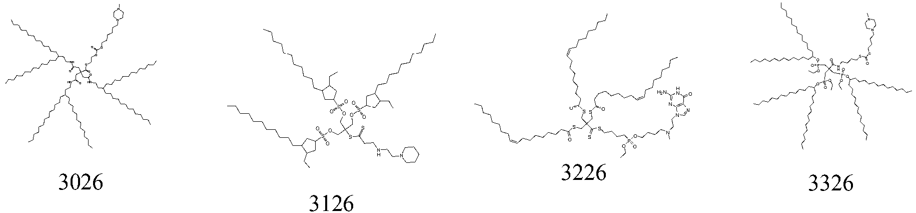

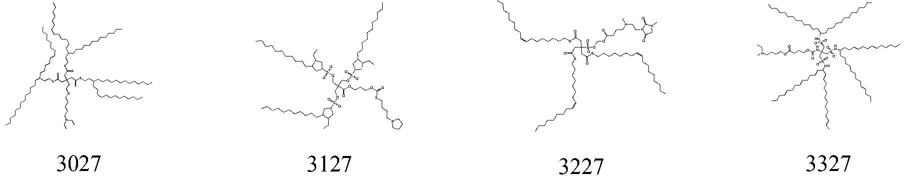

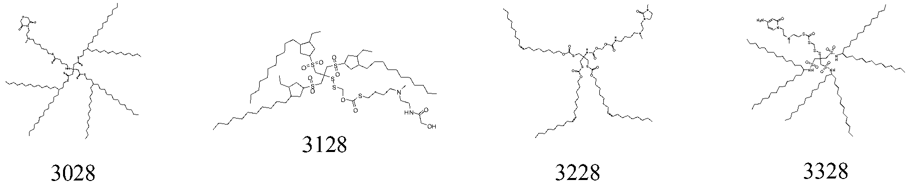

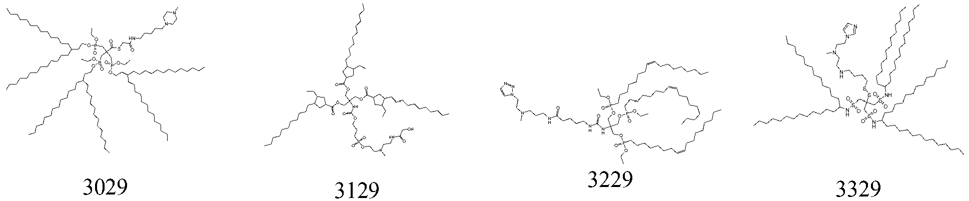

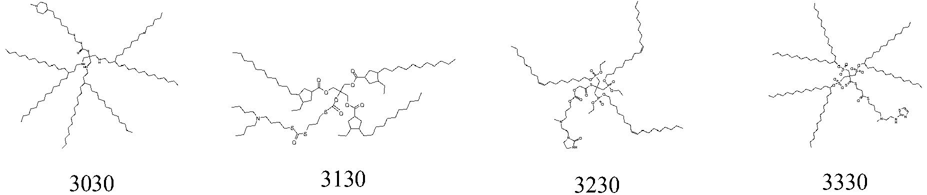

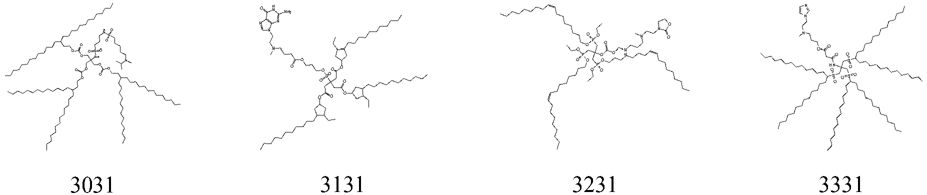

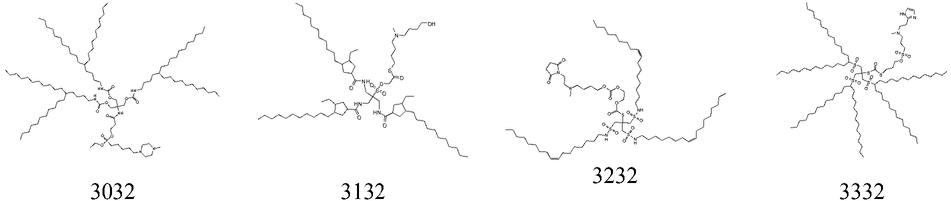

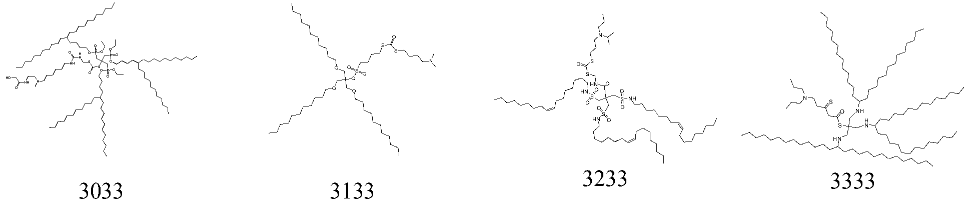

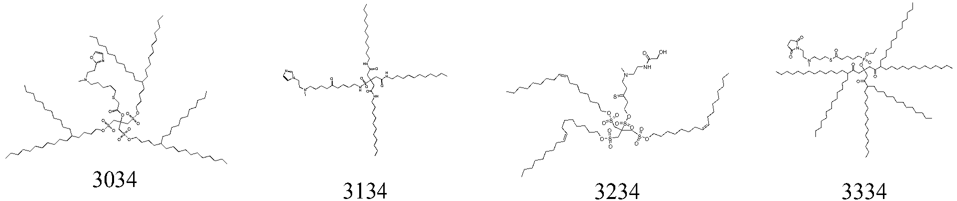

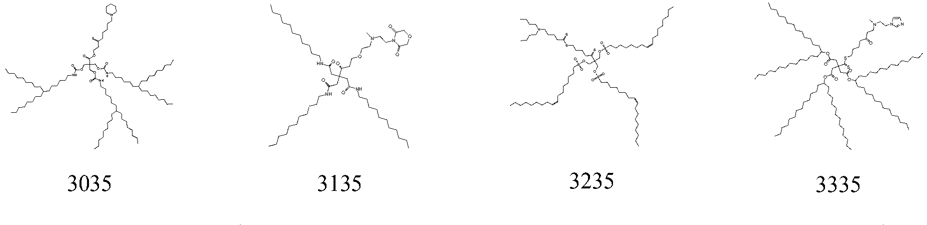

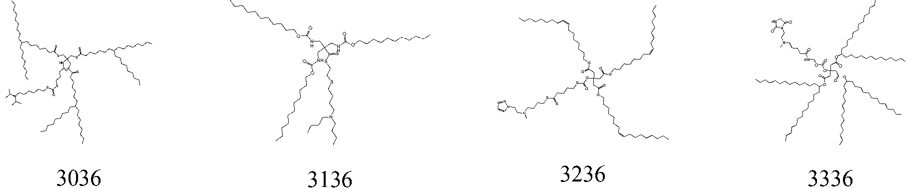

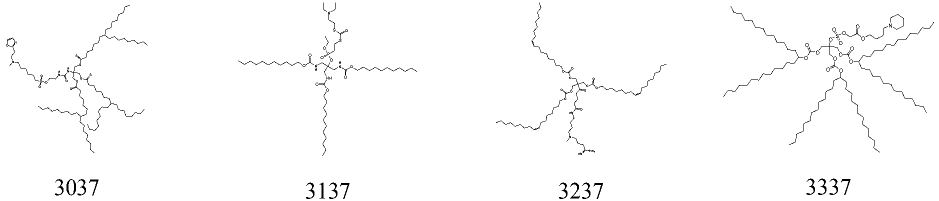

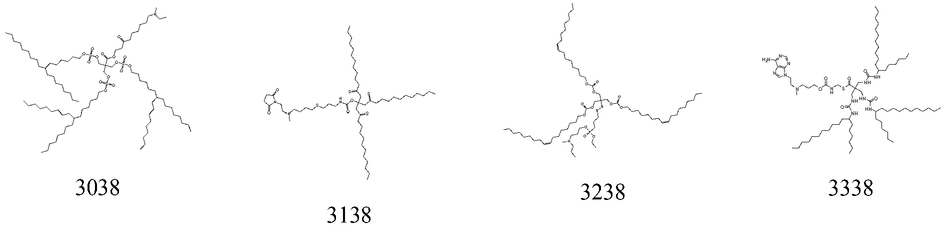

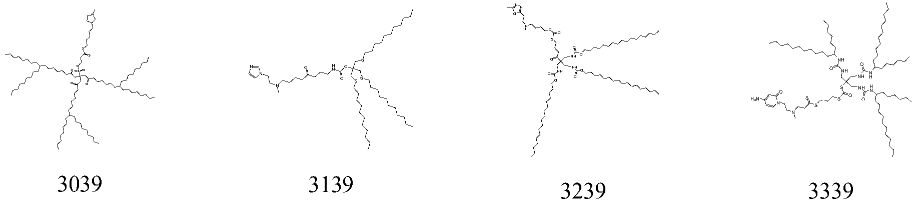

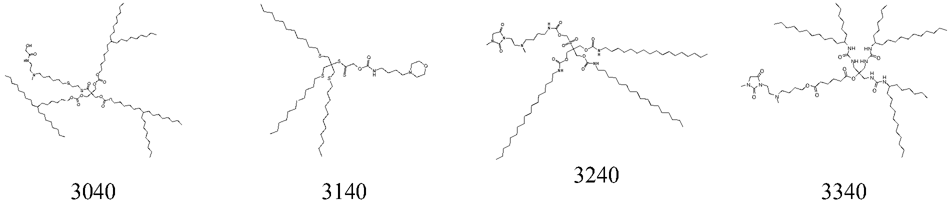

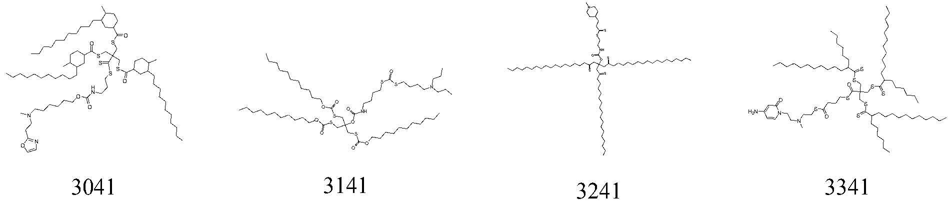

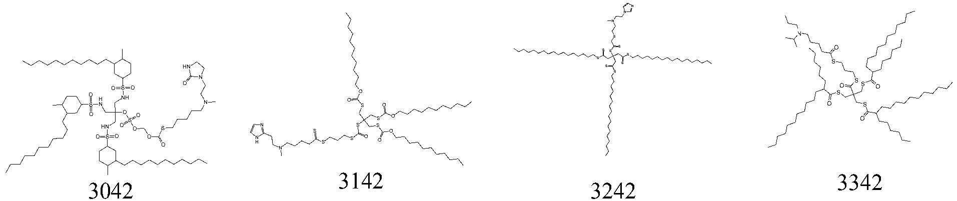

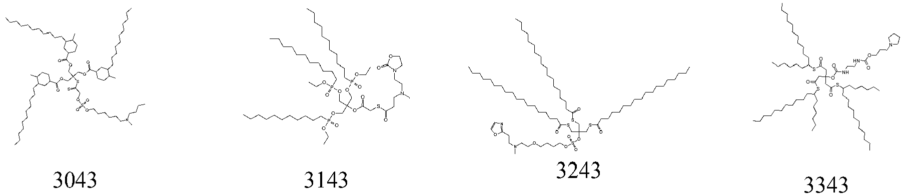

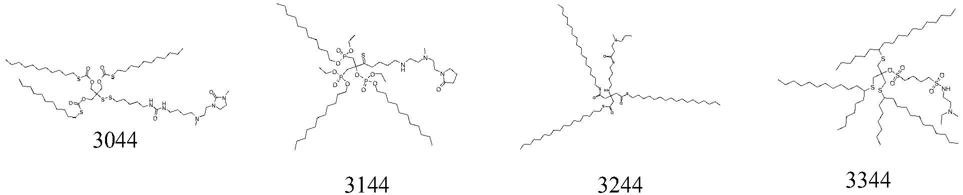

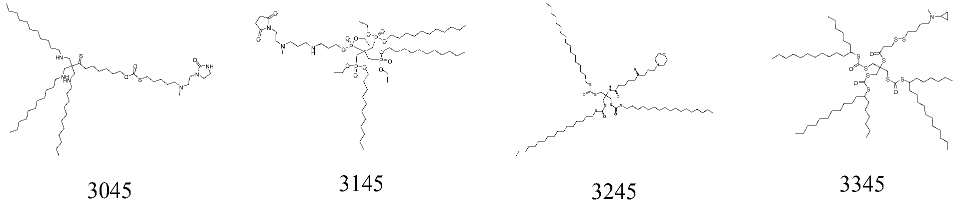

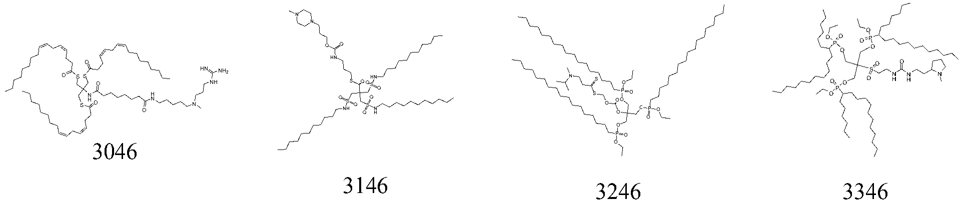

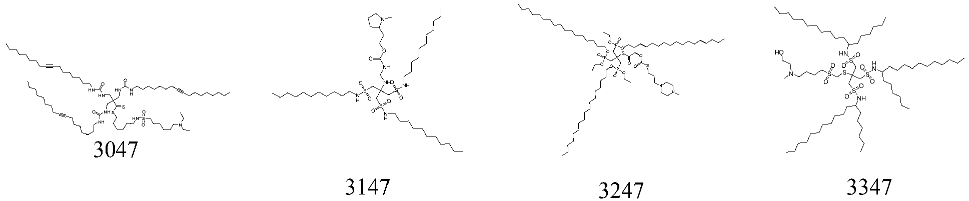

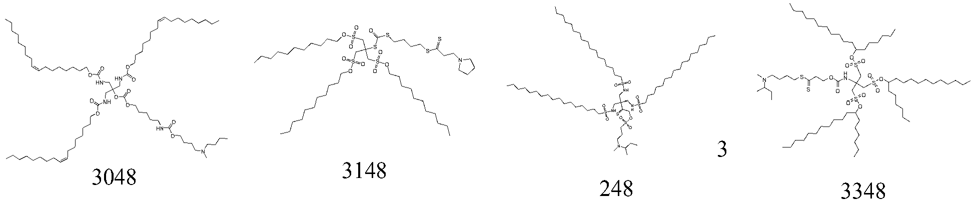

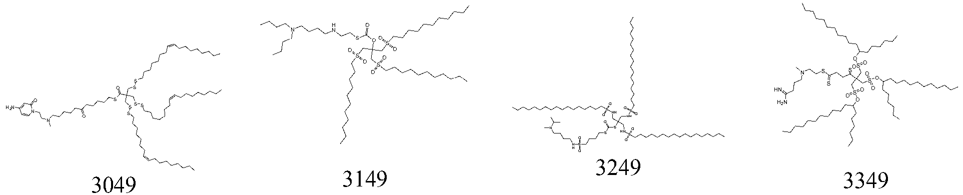

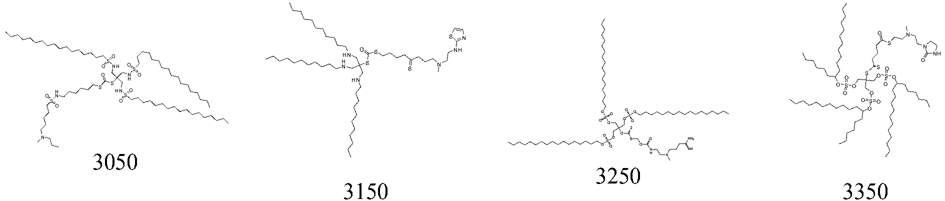

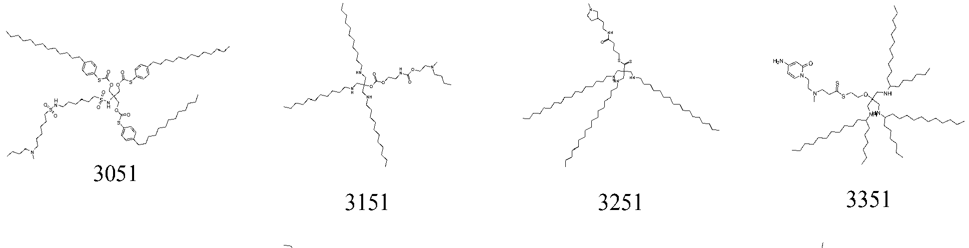

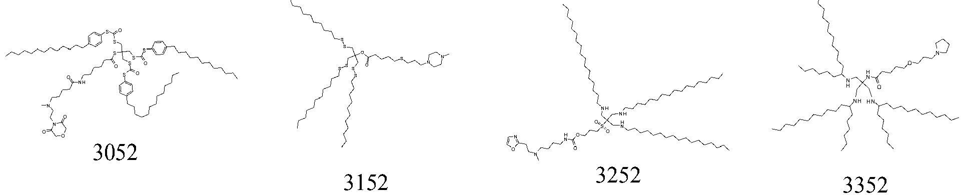

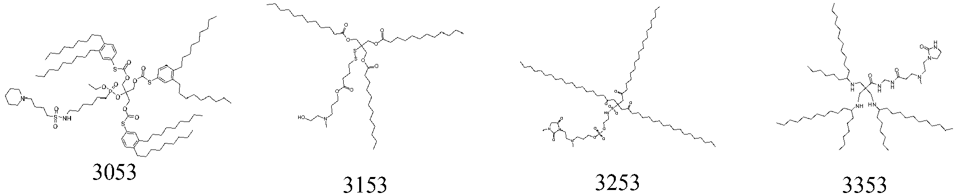

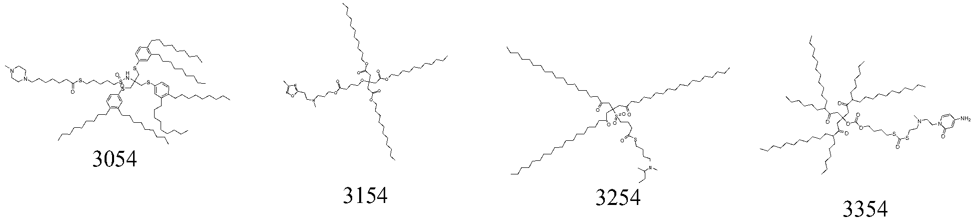

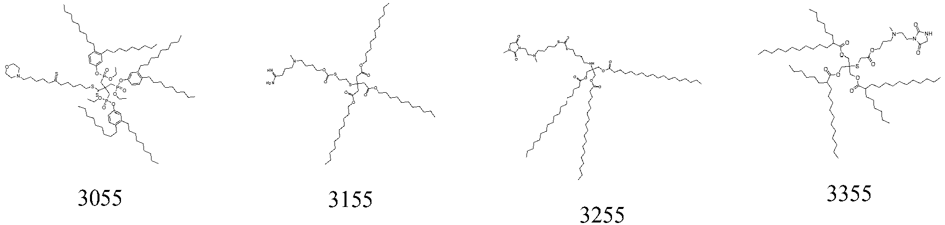

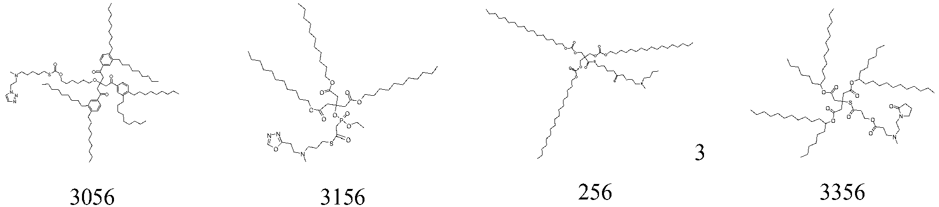

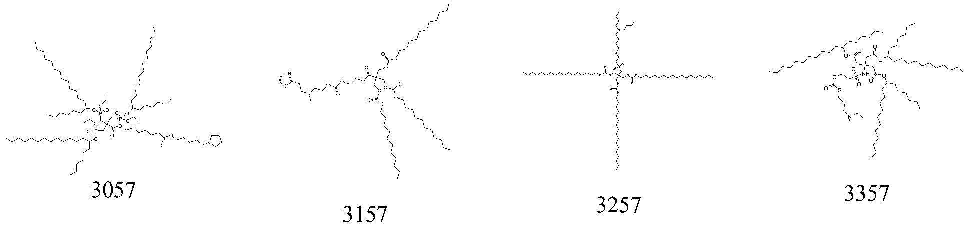

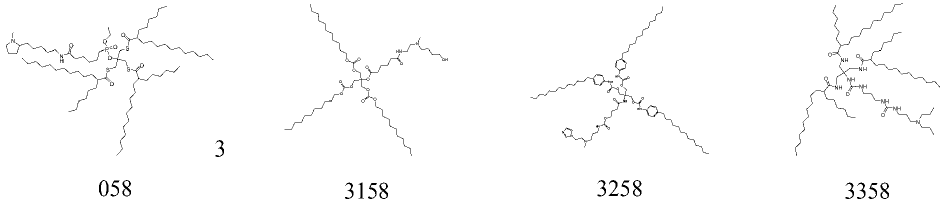

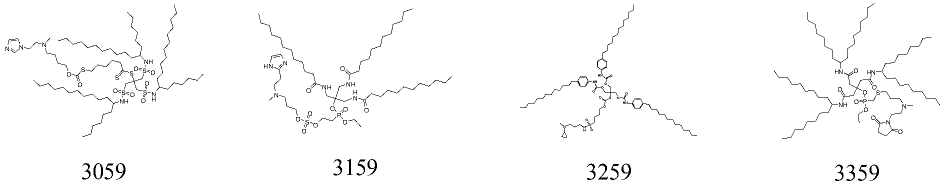

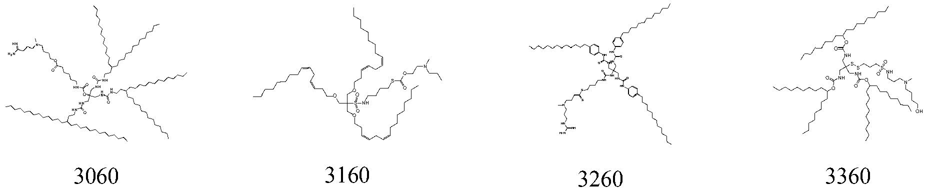

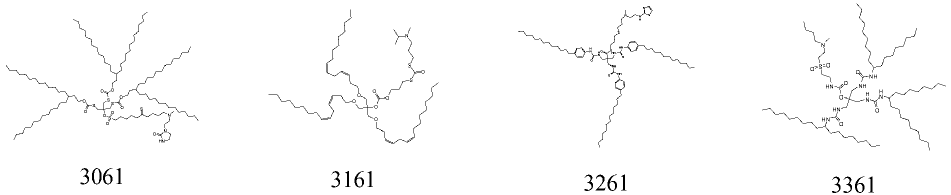

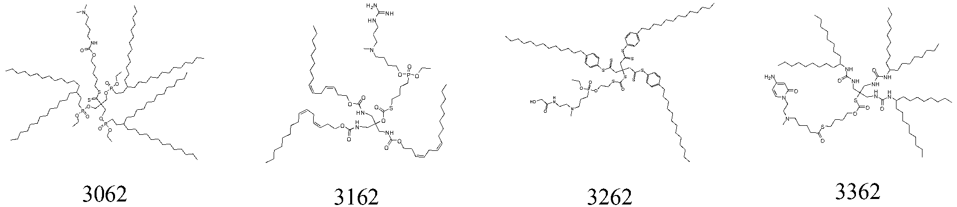

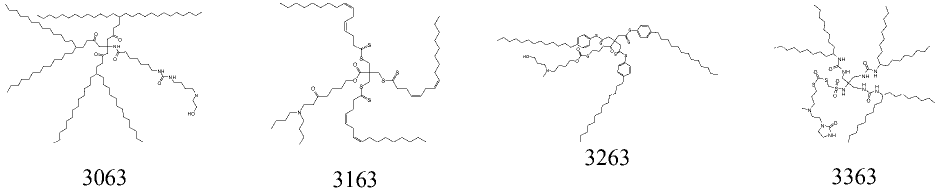

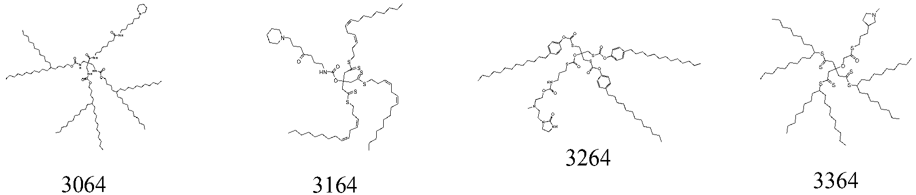

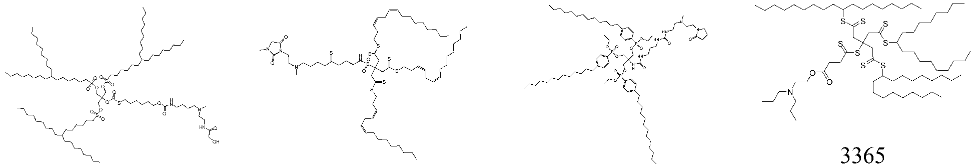

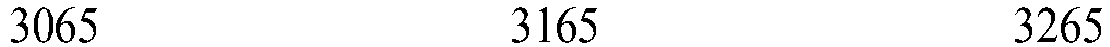

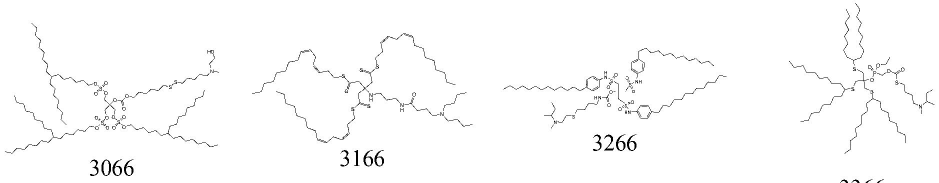

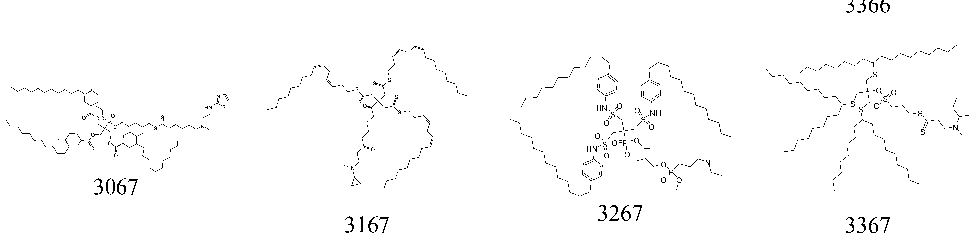

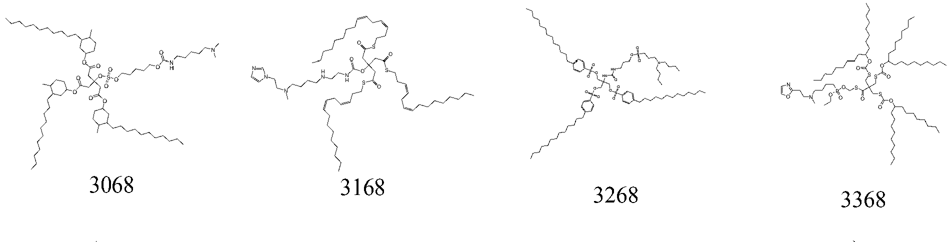

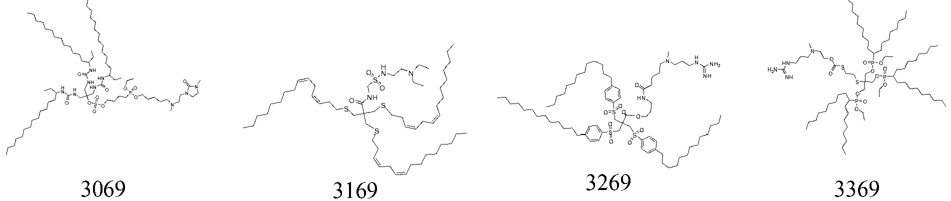

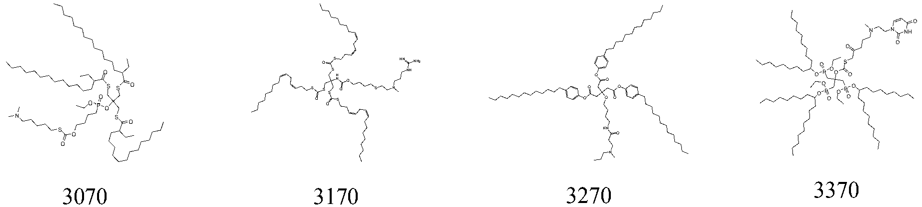

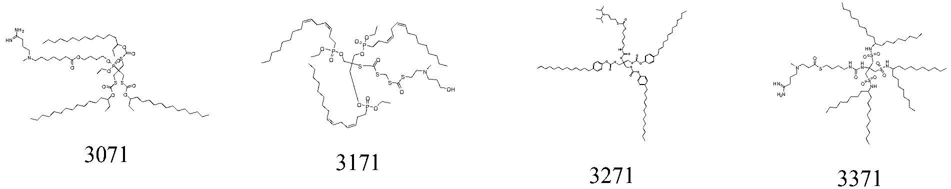

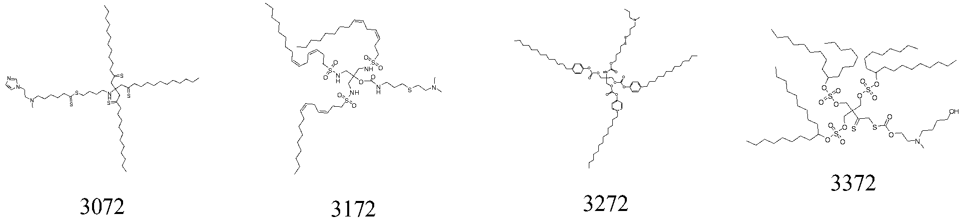

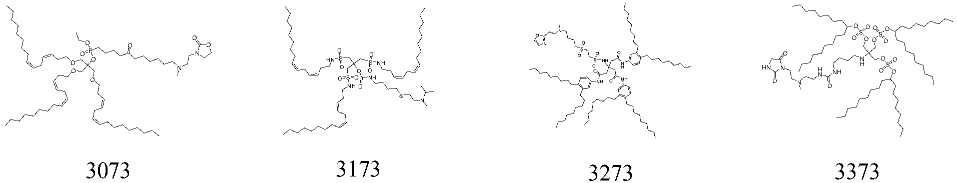

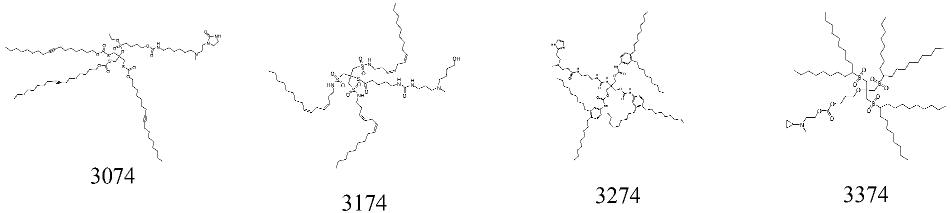

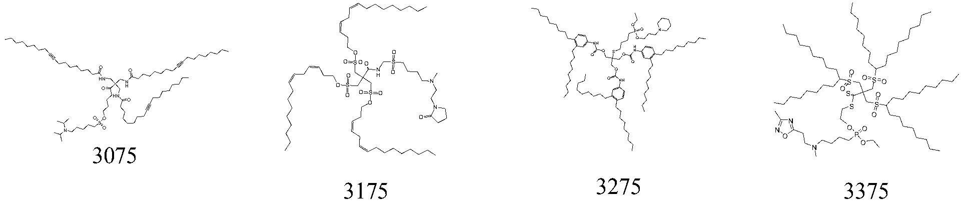

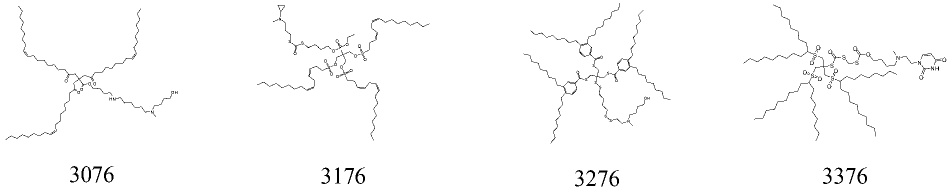

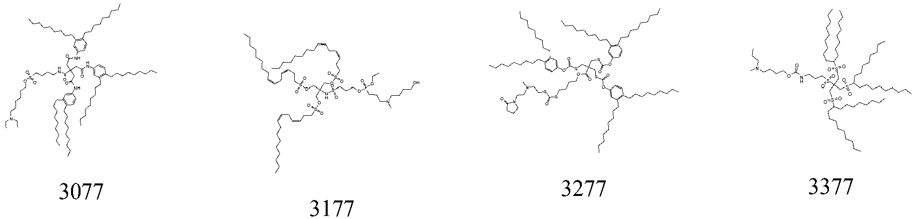

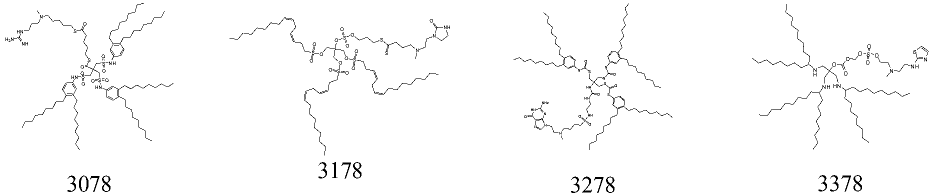

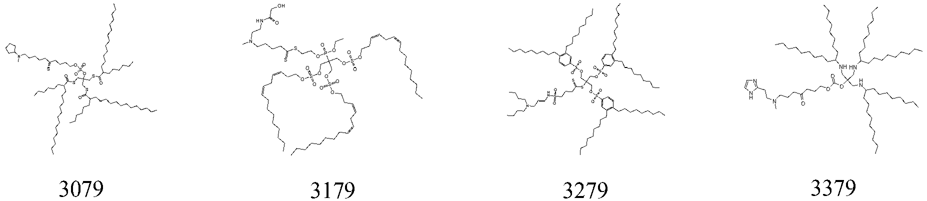

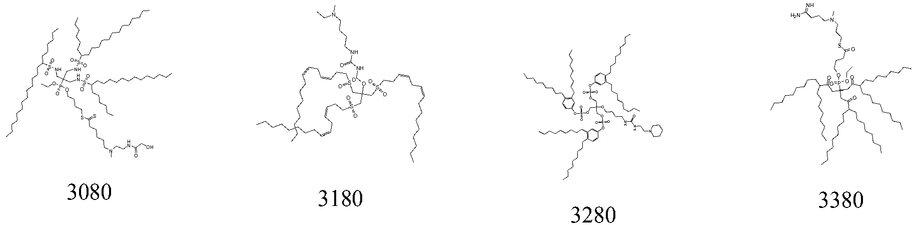

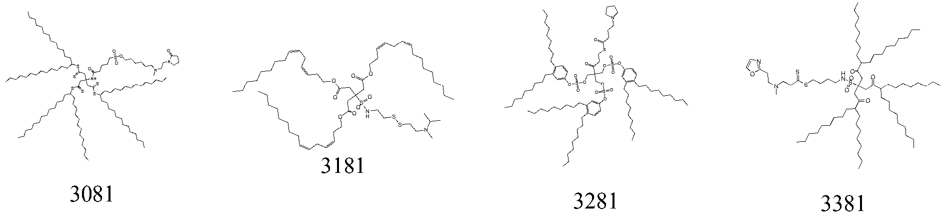

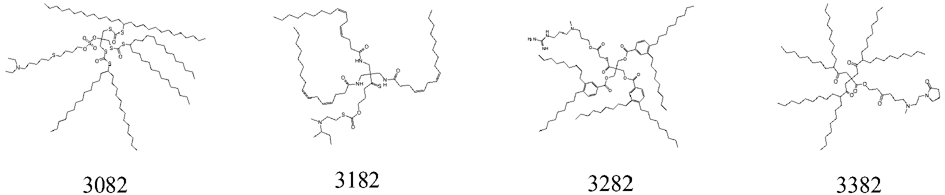

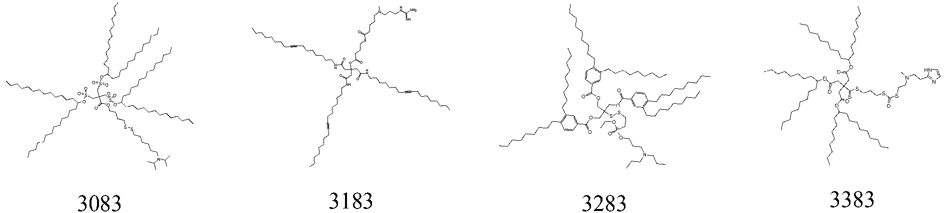

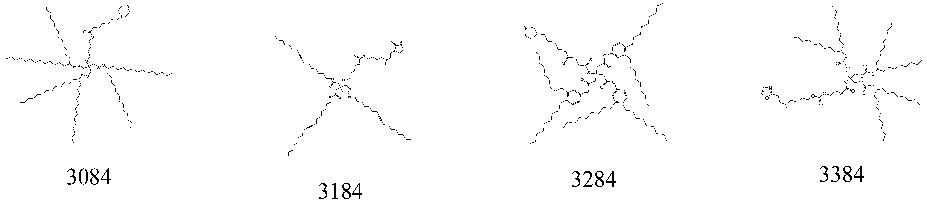

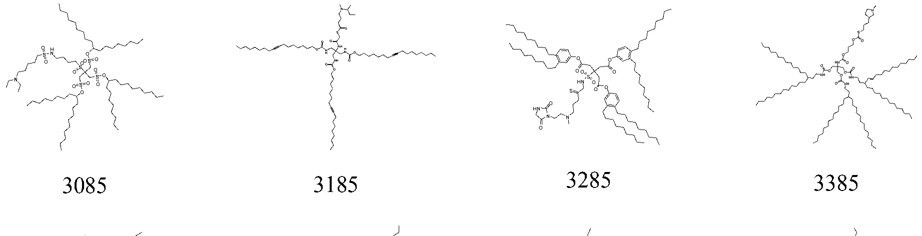

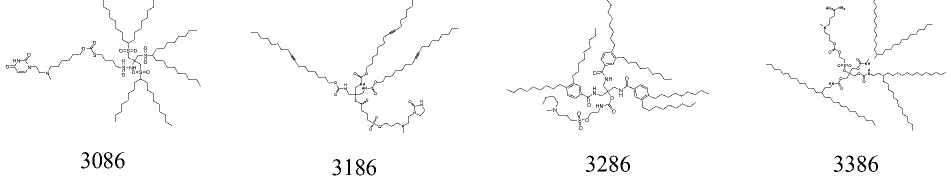

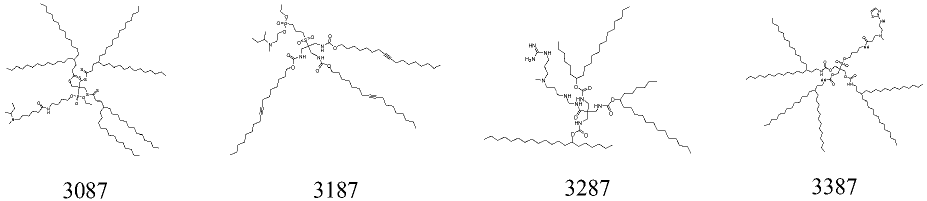

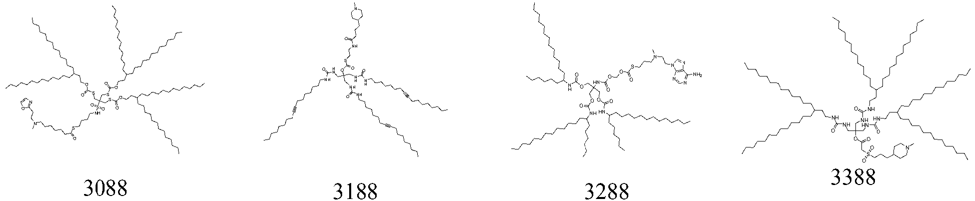

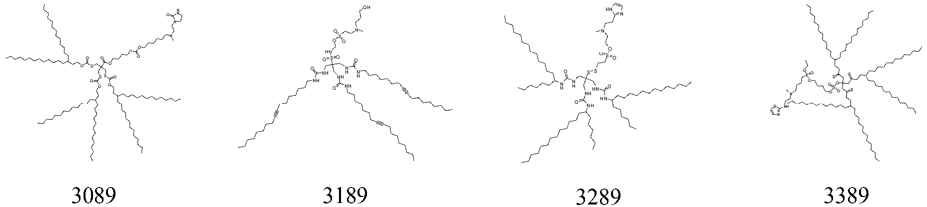

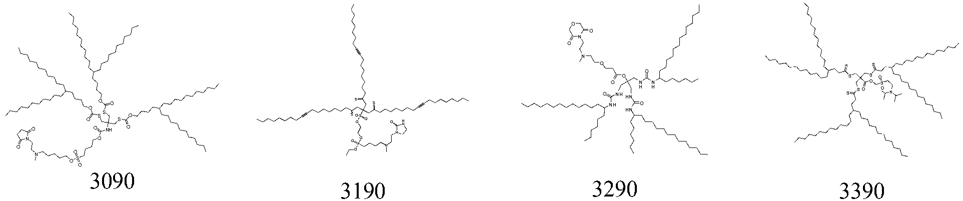

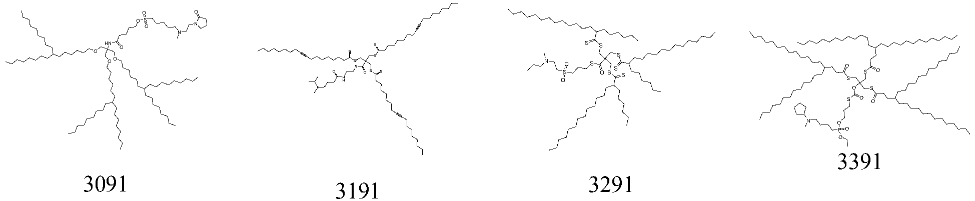

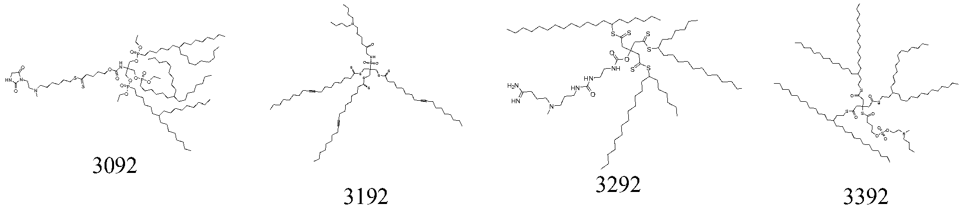

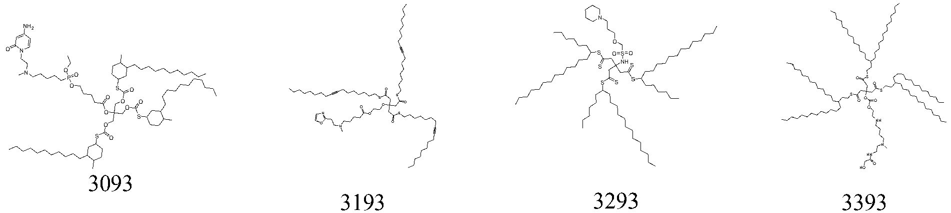

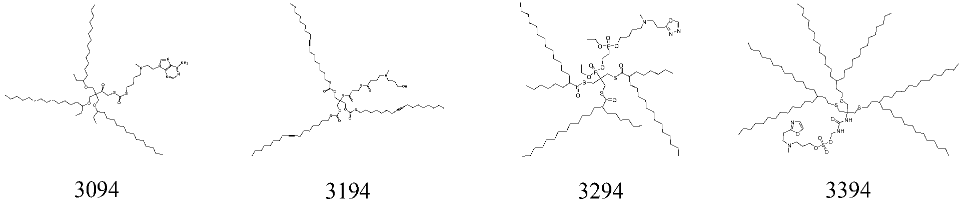

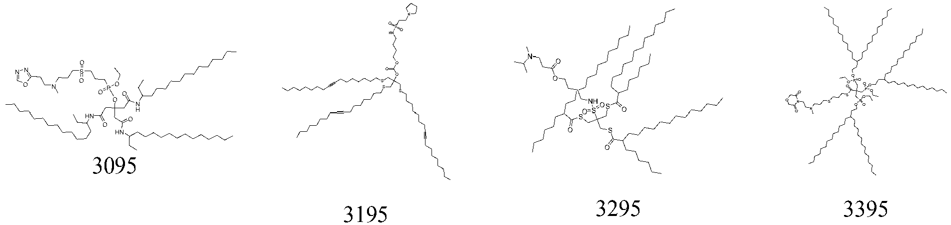

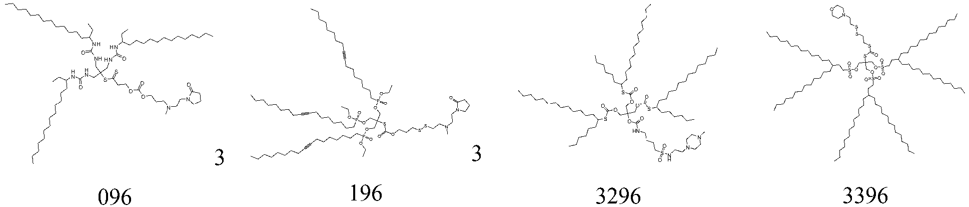

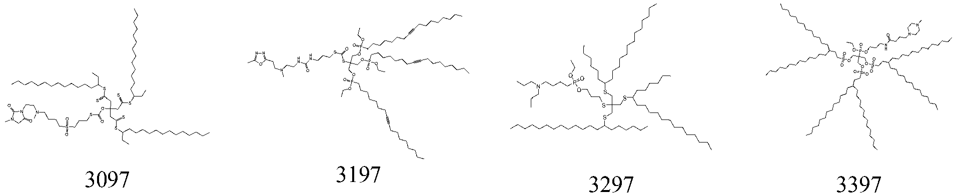

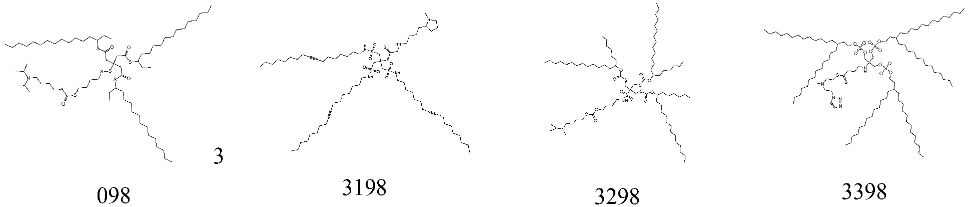

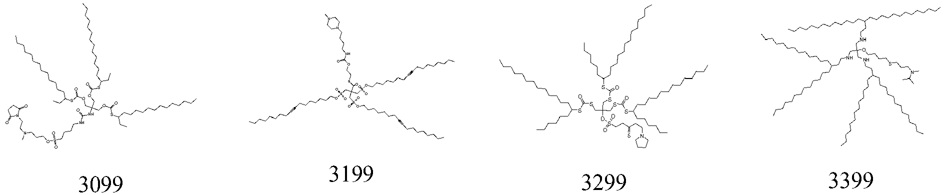

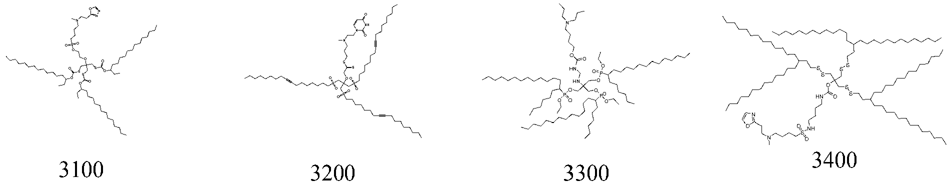

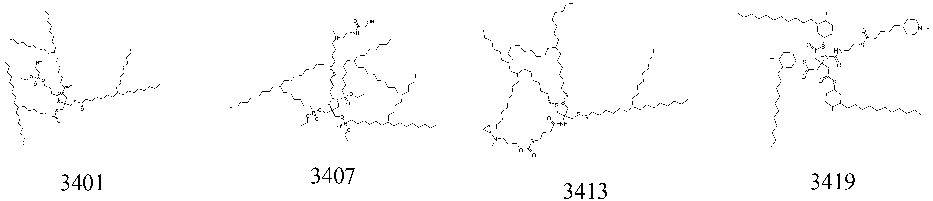

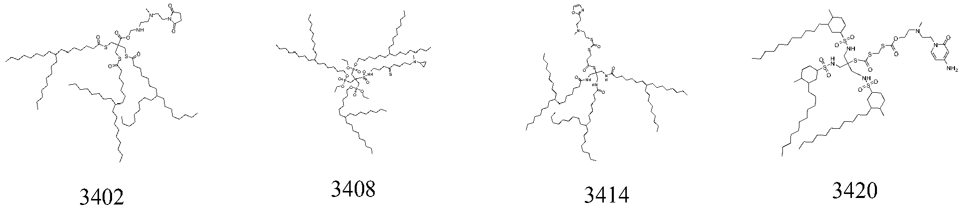

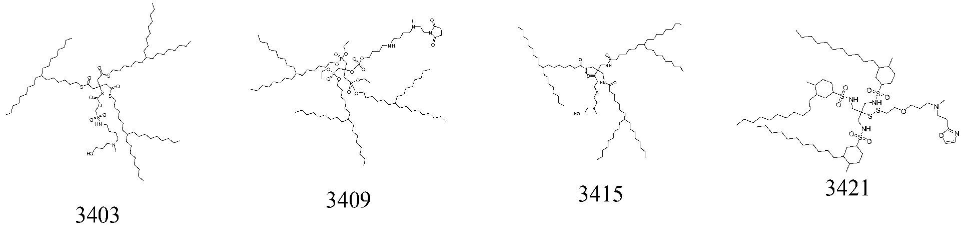

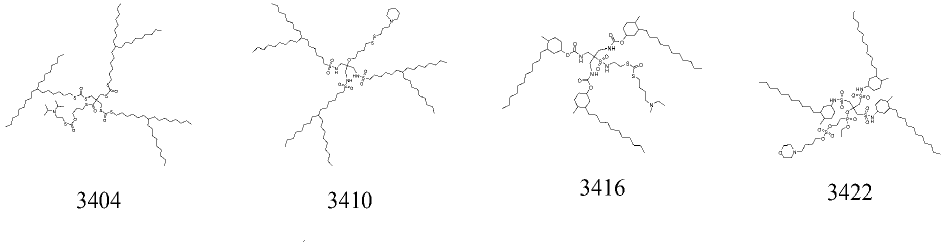

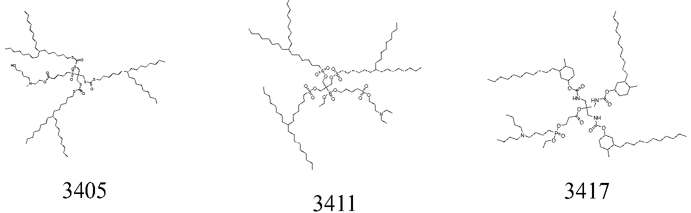

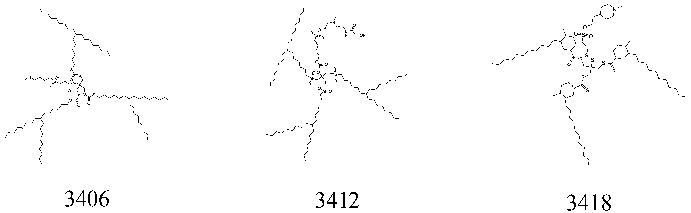

In a preferred embodiment, the preparation method of the first ionizable lipid comprises a step of reacting a compound of Formula (II) with a compound of Formula (III): 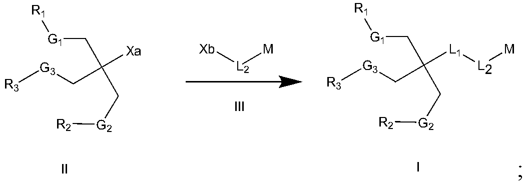 wherein Xₐ and X_{b} are groups bearing leaving groups or nucleophilic groups, and Xₐ and X_{b} form L₁ via a nucleophilic or condensation reaction.

In a preferred embodiment, the preparation method of the first ionizable lipid comprises a step of reacting a compound of Formula (IV) with a compound of Formula (V): 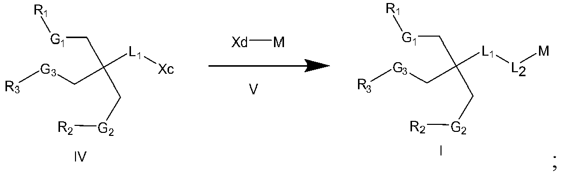 wherein X_{c} and X_{d} are groups bearing leaving groups or nucleophilic groups, and X_{c} and X_{d} form L₂ via a nucleophilic or condensation reaction.

In a preferred embodiment, the preparation method of the first ionizable lipid comprises a step of reacting a compound of Formula (VI): 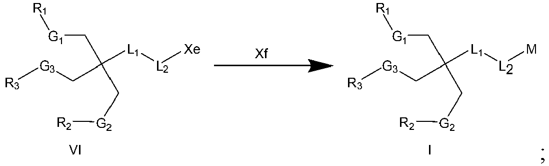 wherein Xₑ is a group bearing a leaving group or nucleophilic group and X_{f} is a compound bearing a leaving group or nucleophilic group; Xₑ and X_{f} form M via a nucleophilic or condensation reaction.

In a preferred embodiment, the preparation method of the first ionizable lipid comprises a step of sequentially reacting a compound of Formula (VII) with a compound of Formula (VIII), a compound of Formula (IX) and a compound of Formula (X): 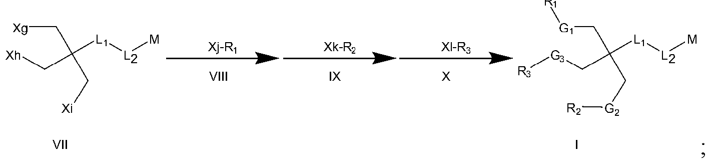 wherein X_{g}, Xₕ, Xᵢ, Xⱼ, Xₖ and Xₗ are groups bearing leaving groups or nucleophilic groups; X_{g} and Xⱼ form Gₗ, Xₕ and Xₖ form G₂, and Xᵢ and Xₗ form G₃ via a nucleophilic or condensation reaction respectively.

In a preferred embodiment, the preparation method of the first ionizable lipid comprises a step of reacting a compound of Formula (XI) with a compound of Formula (XII): 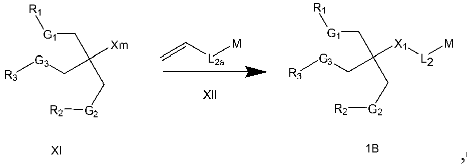 or a step of reacting a compound of Formula (XIII) with a compound of Formula (XII): 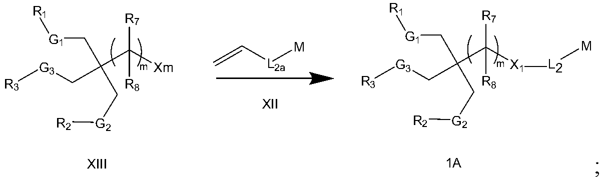 wherein Xₘ is a group bearing a nucleophilic group, and Xₘ undergoes an addition reaction with the C=C-L₂ₐ of Formula (XII) to form X₁-L₂.

In a preferred embodiment, R₁, R₂ and R₃ are each independently selected from groups as shown below: 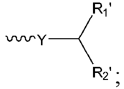
wherein Y, R₁' and R₂' are defined as above; the method further comprises a step of forming side chains R₁, R₂ and R₃: 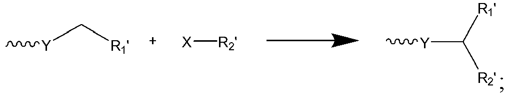
wherein X denotes a leaving group.

In a preferred embodiment, the second ionizable lipid is a compound of Formula (2), or a salt, stereoisomer or tautomer thereof: 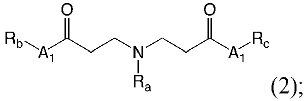
wherein N₁ is NH or O;
Rₐ is selected from C₆-C₂₄ alkyl, C₆-C₂₄ alkenyl, C₆-C₂₄ cycloalkyl, C₆-C₂₄ alcohol and C₆-C₂₄ short-chain polyoxyethylene; the C₆-C₂₄ alkyl, C₆-C₂₄ alkenyl, C₆-C₂₄ cycloalkyl, C₆-C₂₄ alcohol and C₆-C₂₄ short-chainpolyoxyethylene are linear or branched;R_{b} and R_{c} are each independently selected from C₁-C₁₂ alkyl, C₂-C₁₂ alkenyl, C₁-C₁₂ alkynyl, C₃-C₁₂ cycloalkyl, C₆-C₁₂ aryl, C₁-C₁₂ alkyl alcohol, C₁-C₁₂ heterocyclyl and alkylamine;
the alkylamino has the structure of 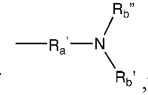 wherein Rₐ' is C₁-C₁₂ alkyl; R_{b}' and R_{b}" are each independently selected from H, C₁-C₆ alkylamino, 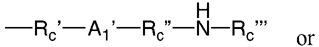 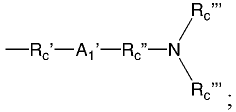 R_{c}" is selected from unsubstituted or amino-substituted C₁-C₆ alkyl; R_{c}‴ is H, 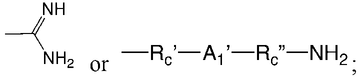
provided that when Ai' is -CO-NH-, -NH-CO- or -CO-O-, R_{c}' is C₁-C₆ alkyl; when A₁' is -CO-, R_{c}' is absent.

In a preferred embodiment, R_{b} and R_{c} are each independently selected from C₁-C₁₂ alkyl alcohol or alkylamino groups.

In a preferred embodiment, the proviso is: when Rₐ is C₆-C₂₄ linear alkyl, N₁ is not NH, or R_{b} and R_{c} are not C₁-C₁₂ alcohols; when Rₐ is C₆-C₂₄ alcohol, N₁ is not O, or R_{b} and R_{c} are not C₆-C₂₄ alkyl.

In a preferred embodiment, the proviso is: when Rₐ is C₆-C₂₄ alkenyl, A₁ is NH, and R_{b} and R_{c} are each independently selected from C₁-C₁₂ alkyl alcohols;
when Rₐ is branched C₆-C₂₄ alkyl, A₁ is NH, and R_{b} and R_{c} are each independently selected from C₁-C₁₂ alkyl alcohols or alkylamino groups;
when Rₐ is C₆-C₂₄ linear alkyl, A₁ is NH or O, and R_{b} and R_{c} are each independently selected from alkylamino groups;
when Rₐ is C₆-C₂₄ linear alkyl, A₁ is O, and R_{b} and R_{c} are each independently selected from C₁-C₁₂ alkyl alcohols;
when Rₐ is short C₆-C₂₄ polyethylene glycol, A₁ is NH or O, and R_{b} and R_{c} are each independently selected from alkylamino groups;
when Rₐ is C₆-C₂₄ alkyl alcohol, A₁ is NH or O, R_{b} and R_{c} are each independently selected from alkylamino groups.

In a preferred embodiment, Rₐ is selected from the following structures:

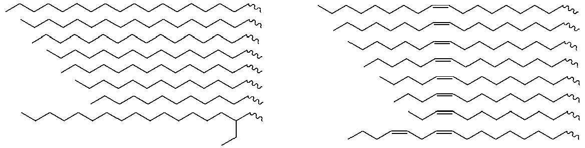

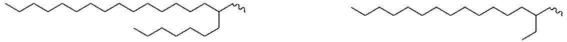

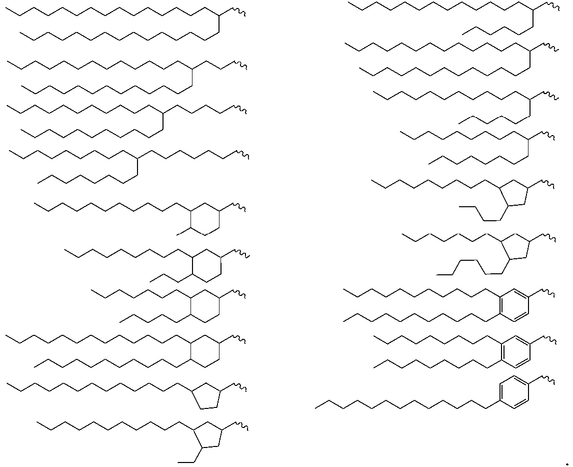

In a preferred embodiment, R_{b} and R_{c} are selected from the following structures:

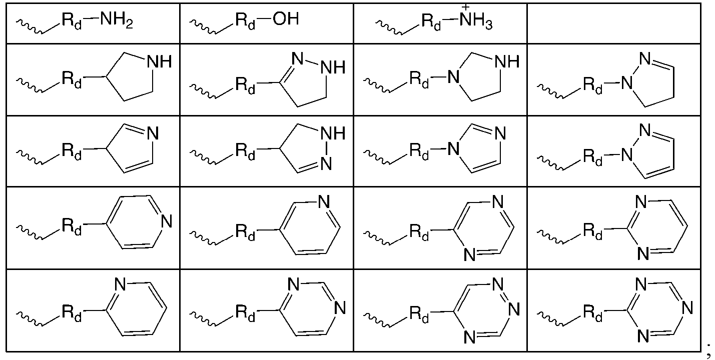

wherein Rₐ is selected from C₁-C₆ alkane or cycloalkane.

In a preferred embodiment, the compound of Formula (2) is selected from at least one of compounds below:

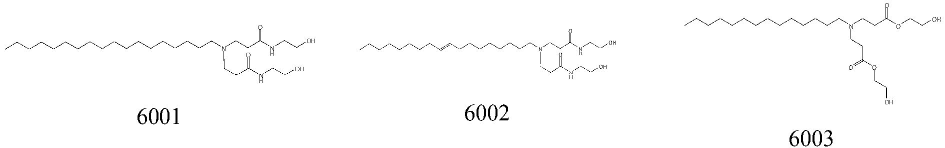

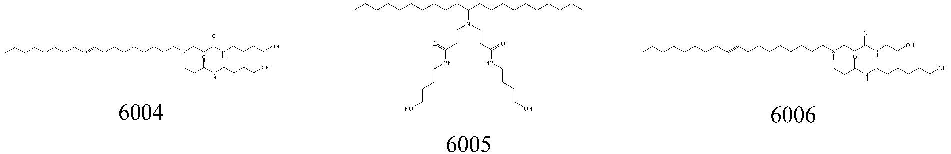

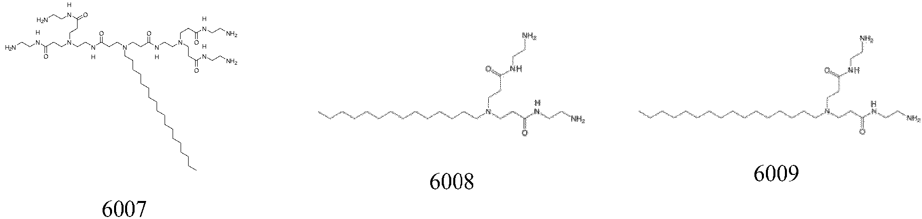

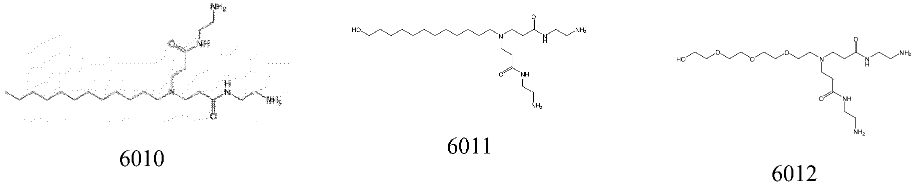

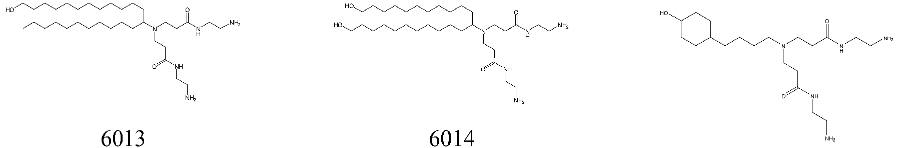

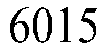

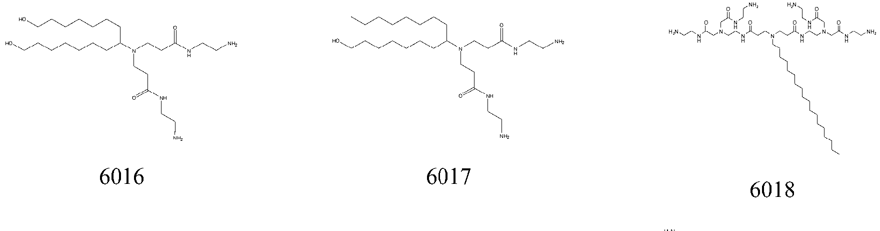

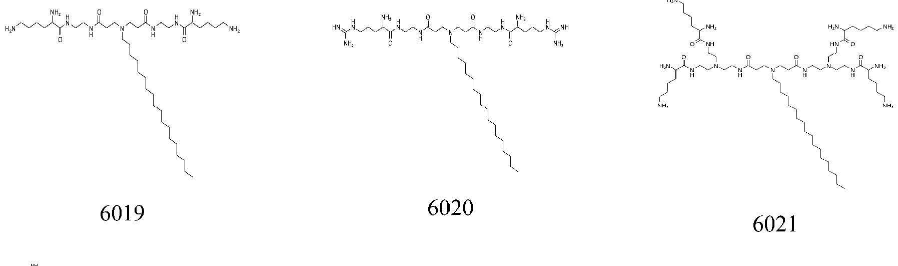

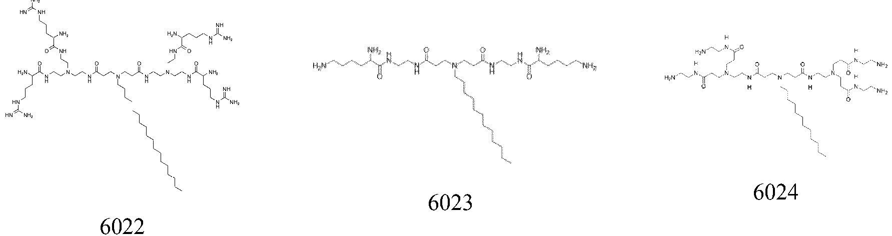

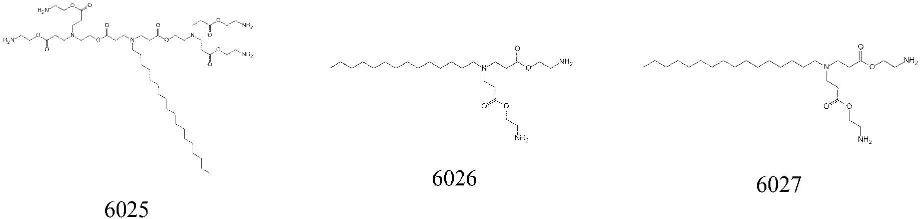

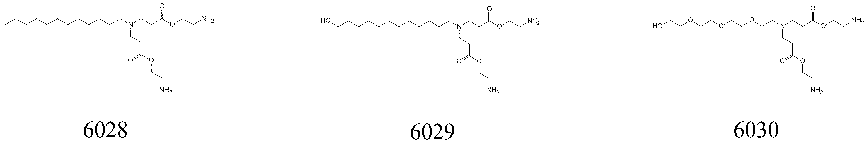

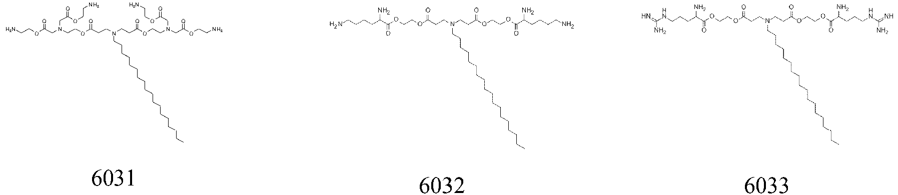

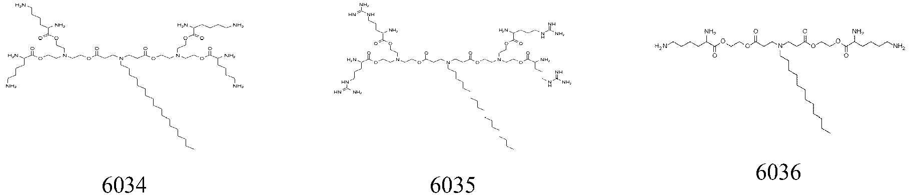

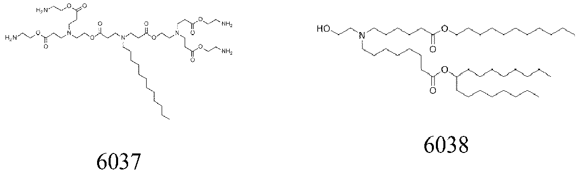

In a preferred embodiment, the preparation method of the second ionizable lipid comprises the following reaction step:
Rₐ"-NH₂ ① reacts with an α,β-unsaturated carbonyl compound ② to form ionizable lipid compound ③: 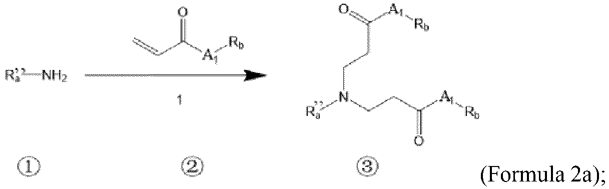
wherein Rₐ"-NH₂ is Rₐ-NH₂ or 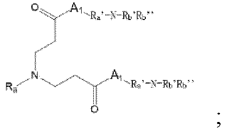
R_{b}' and R_{b}" are both H, or R_{b}' and R_{b}" are both C₁-C₆ amino groups, or R_{b}' and R_{b}" are both -R_{c}'-A₁'-R_{c}"-NH₂ .

In a preferred embodiment, the preparation method of the second ionizable lipid comprises:
1) Rₐ"-NH₂ ① reacts with an α,β-unsaturated carbonyl compound ④ to form compound ⑤;
2) compound ⑤ reacts with nucleophile ⑥ to form ionizable lipid compound ③; 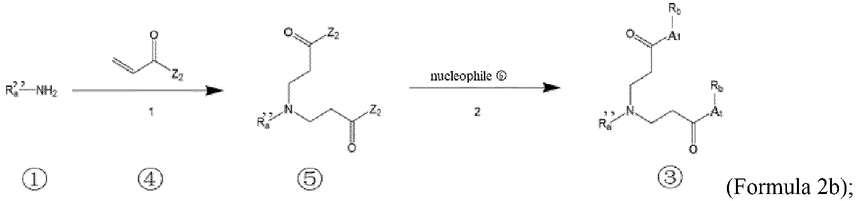
   wherein nucleophile ⑥ is R_{b}-NH₂ or R_{b}-OH; Rₐ"-NH₂ is Rₐ-NH₂ or 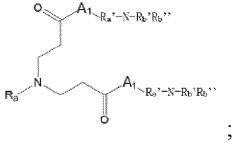
   R_{b}' and R_{b}" are both H, or R_{b}' and R_{b}" are both C₁-C₆ amino groups, or R_{b}' and R_{b}" are both -R_{c}'-A₁'-R_{c}"-NH₂; Z₂ is a leaving group and Z₂reacts with NH₂ to form A₁.

In a preferred embodiment, the preparation method of the second ionizable lipid comprises:
1) Rₐ"-NH₂ ① reacts with α,β-unsaturated carbonyl compound ② to form compound ⑦;
2) compound ⑦ reacts with α,β-unsaturated carbonyl compound ⑧ to form compound ⑨;
3) compound ⑨ reacts with nucleophile ⑩ to form ionizable lipid compound ⑪; 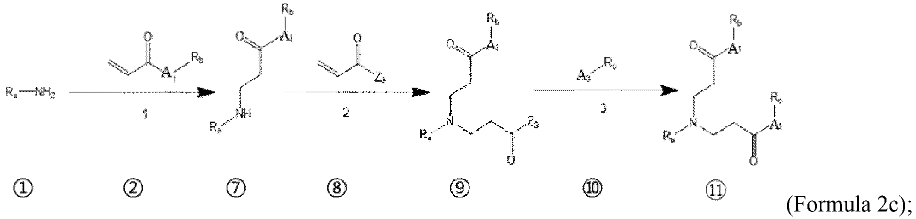 wherein Rₐ"-NH₂ is Rₐ-NH₂ and Z₃ is a leaving group; A₃ reacts with Z₃ to form A₁.

In a preferred embodiment, in Formula 2a-Formula 2c, at least one nucleophile undergoes Michael addition reaction with the β-carbon of at least one α,β-unsaturated carbonyl compound to form the branched ionizable lipid bearing C-C, C-O, C-N, C-S, orC-Se bonds.

In a preferred embodiment, the reaction schemes of Formula 2a-Formula 2b further comprise iterative reaction steps using the terminal amino-containing Formula (2) compounds obtained from Formula 2a-Formula 2b as starting materials, following Step 1 of Formula 2a or Steps 1-2 of Formula 2b;
the terminal amino-containing Formula (2) compound is 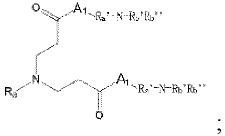
wherein R_{b}' and R_{b}" are both H, or R_{b}' and R_{b}" are both C₁-C₆ amino groups, or R_{b}' and R_{b}" are both -R_{c}'-A₁'-R_{c}"-NH₂ .

In a preferred embodiment, the reaction schemes of Formula 2a-2c further comprise reacting the terminal amino-containing Formula (2) products from Formula 2a-2c as starting materialswith 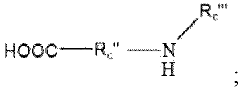
the terminal amino-containing Formula (2) compound is 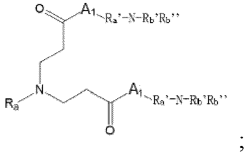
wherein R_{b}' and R_{b}" are both H, or R_{b}' and R_{b}" are both C₁-C₆ amino groups, or R_{b}' and R_{b}" are both -R_{c}'-A₁'-R_{c}"-NH₂.

The leaving group as described above refers to a group that leaves during nucleophilic or condensation reactions, including but not limited to H, OH, H₂O, halogens (F, Cl, Br, I), cyanate anions, mineral acids (nitric acid, sulfuric acid, phosphoric acid), carboxylic acids (acetic acid, trifluoroacetic acid, benzoic acid, etc.), sulfonic acids (methanesulfonic acid, trifluoromethanesulfonic acid, benzenesulfonic acid, p-toluenesulfonic acid, p-nitrobenzenesulfonic acid, etc.), CO₂, N₂, imidazole, alkoxy groups (R-O-), amino groups (-NHR, R = alkyl/aryl minus H), phenoxy groups, tertiary carbocations (tert-butyl carbocation), carbocations stabilized by unsaturated systems or heteroatoms, and various protecting groups mentioned above.

The nucleophilic groups as described above refer to molecules or ions capable of donating an electron pair to form new chemical bonds in chemical reactions. Common nucleophilic groups include hydroxide (HO⁻), ammonia (NH₃), hydroxylamine (NH₂OH), hydrazine (NH₂-NH₂), substituted hydrazines, nucleophilic halide anions (e.g., Cl⁻, Br⁻, I⁻), hydride (H⁻), azide (N₃⁻), cyanide (CN⁻), alcohols and alkoxide anions (e.g., alcohols with the proton removed from hydroxyl groups), amino groups (including primary, secondary and tertiary amines) or amide anions, carbanions (e.g., carbanions from organometallic reagents such as Grignard reagents, organolithium reagents and Gilman reagents), thiols or thiolate anions, thioethers, enols or enolate anions, vinyl ethers, enamines, carboxylic acids or carboxylate anions, alkyl and aryl phosphines (e.g., triphenylphosphine), and heteroaromatic rings bearing lone electron pairs (e.g., pyridine).

In a preferred embodiment, raw materials used in the reactions further contain protecting groups, and the reaction steps include protection and/or deprotection procedures.

In a preferred embodiment, the third ionizable lipid is s at least one compound selected from the list below:

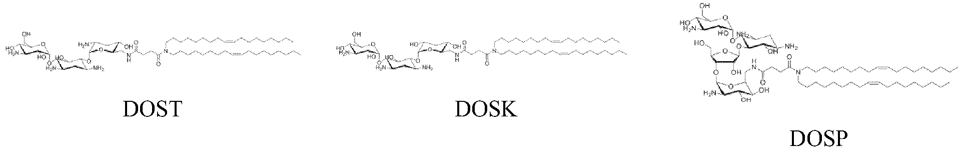

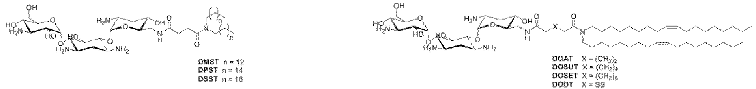

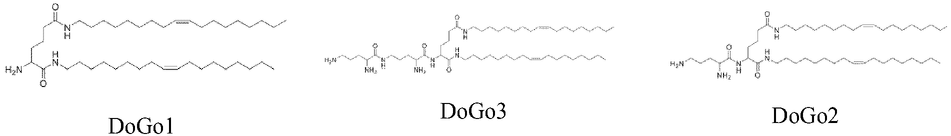

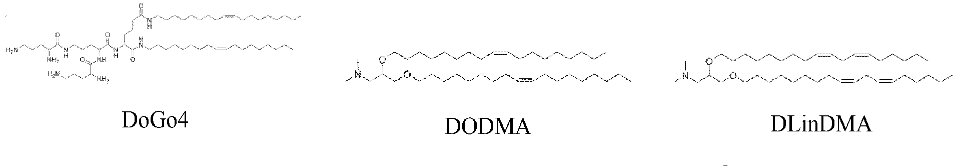

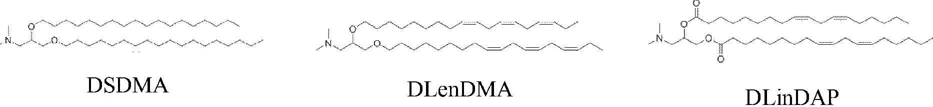

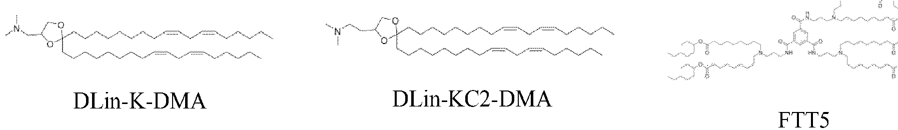

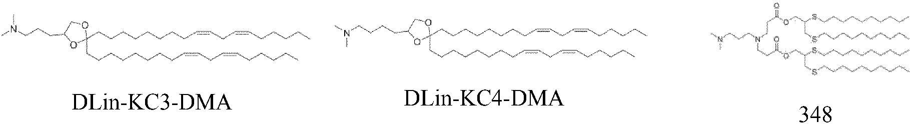

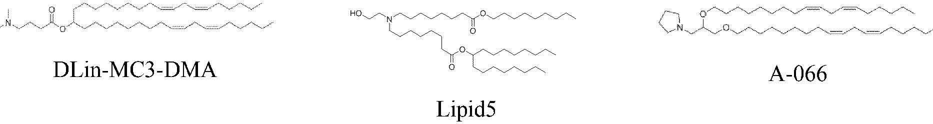

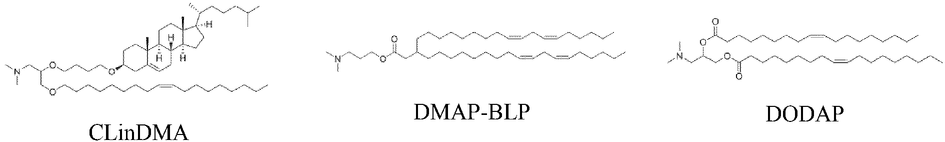

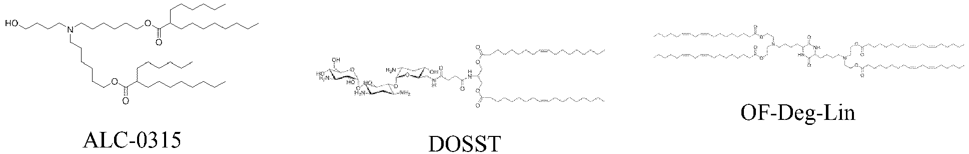

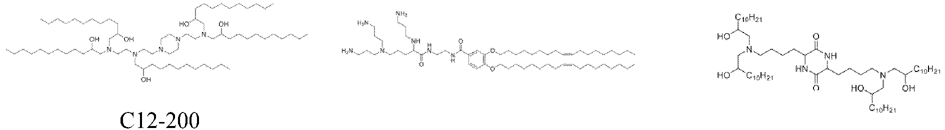

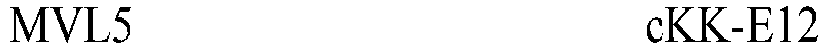

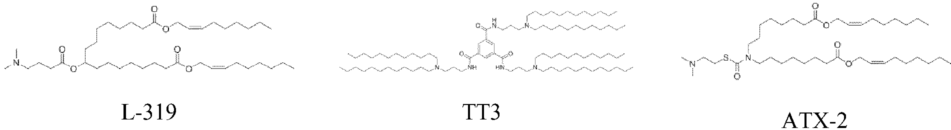

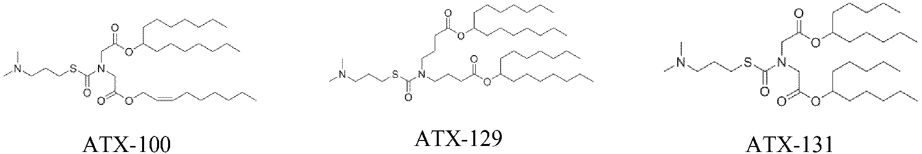

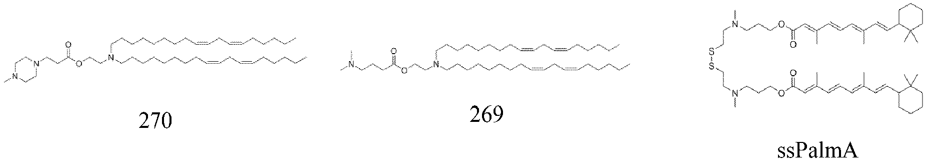

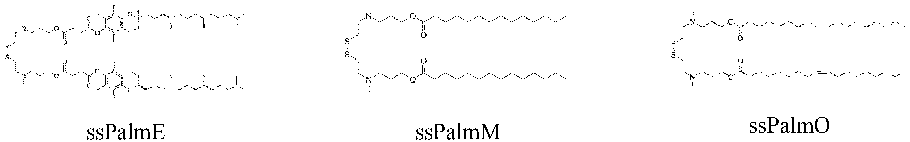

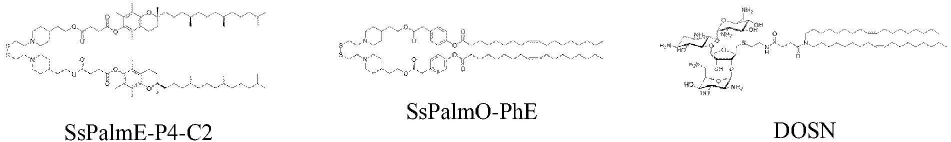

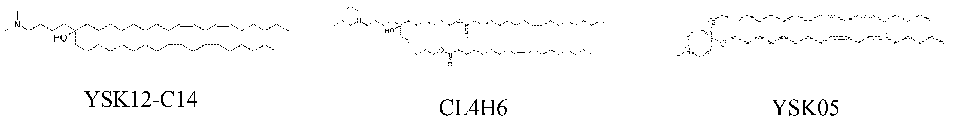

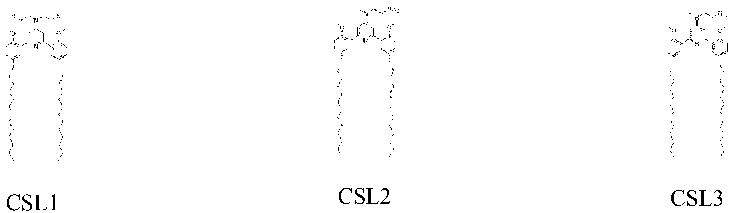

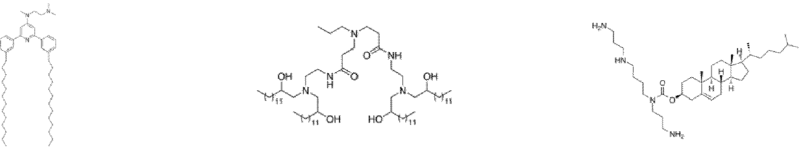

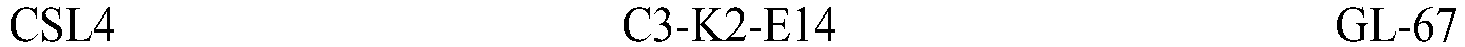

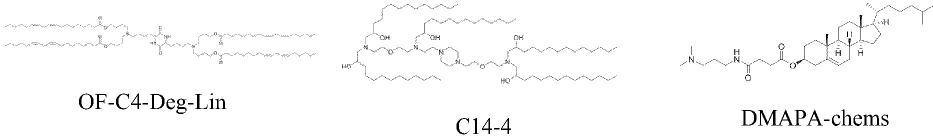

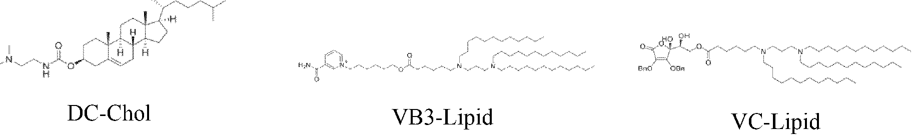

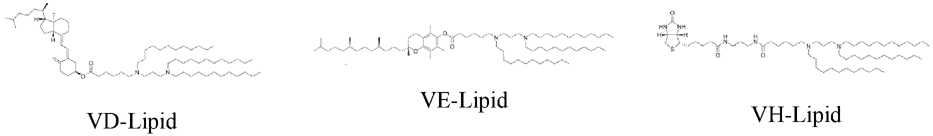

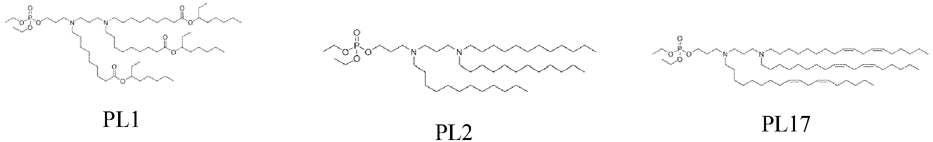

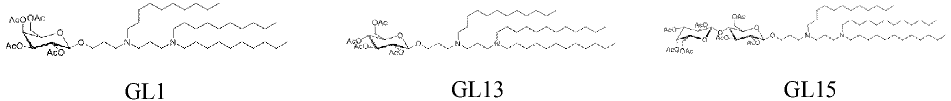

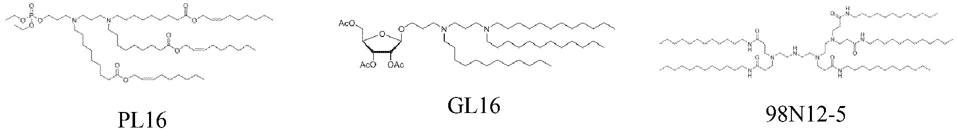

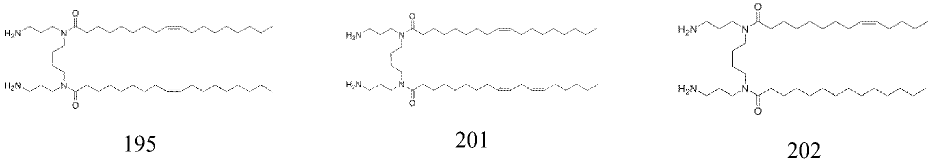

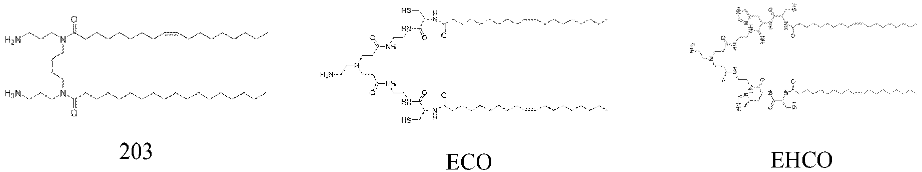

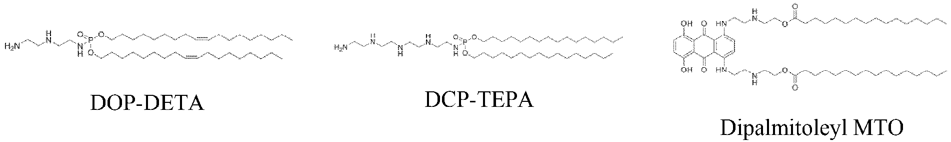

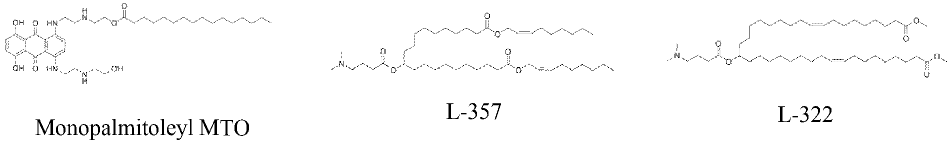

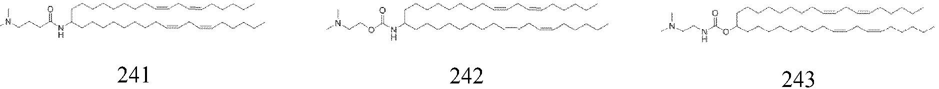

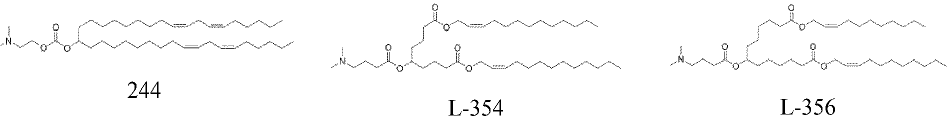

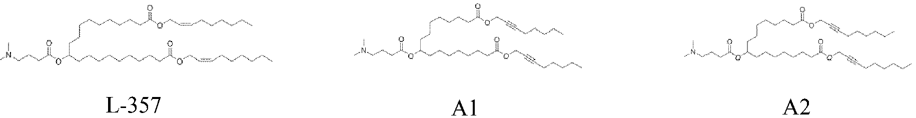

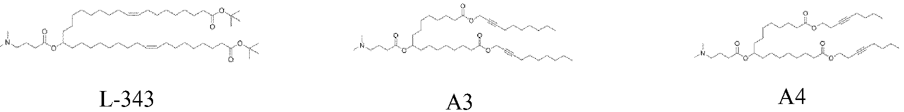

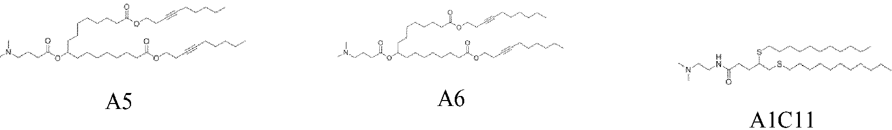

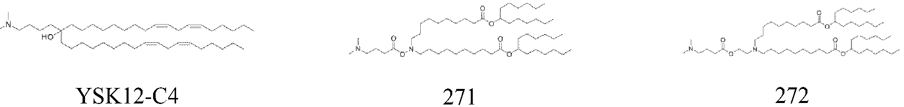

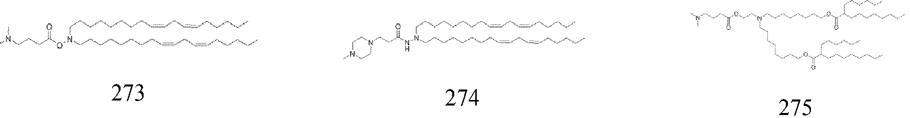

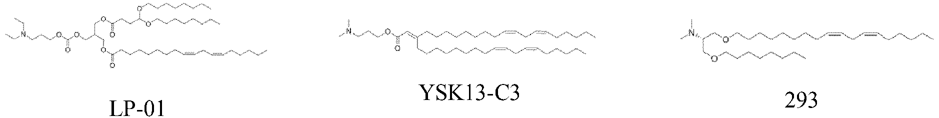

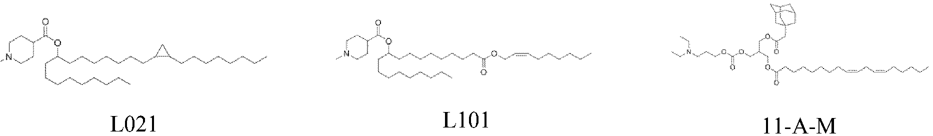

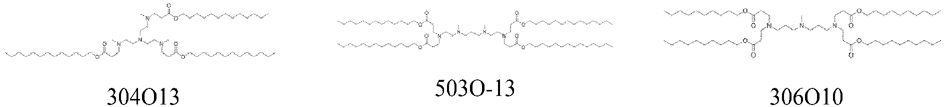

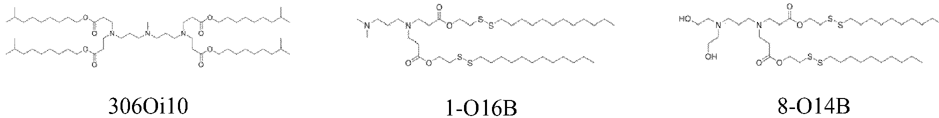

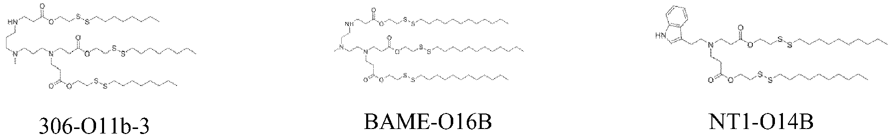

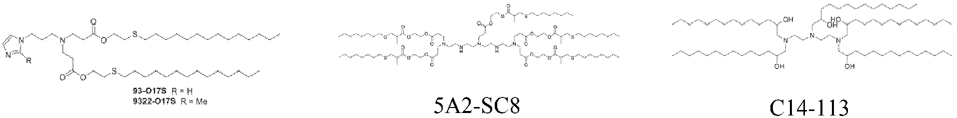

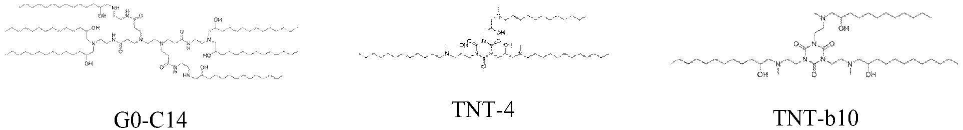

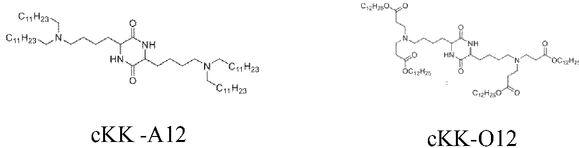

In a preferred embodiment, the helper lipid is a mixture of phospholipids and steroids or derivatives thereof.

In a preferred embodiment, the phospholipid comprises at least one of the following substances: 1,2-dilinoleoyl-sn-glycero-3-phosphocholine (DLPC), 1,2-dimyristoyl-sn-glycero-3-phosphocholine (DMPC), 1,2-dioleoyl-sn-glycero-3-phosphocholine (DOPC), 1,2-dipalmitoyl-sn-glycero-3-phosphocholine (DPPC), 1,2-distearoyl-sn-glycero-3-phosphocholine (DSPC), 1,2-diundecanoyl-sn-glycero-3-phosphocholine (DUPC), 1-palmitoyl-2-oleoyl-sn-glycero-3-phosphocholine (POPC), 1,2-di-O-octadecenyl-sn-glycero-3-phosphocholine (18:0 Diether PC), 1-oleoyl-2-cholesteryl hemisuccinyl-sn-glycero-3-phosphocholine (OChemsPC), 1-hexadecyl-sn-glycero-3-phosphocholine (C16 Lyso PC), 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine (DOPE), 1,2-diphytanoyl-sn-glycero-3-phosphoethanolamine (ME 16.0 PE), 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, sodium 1,2-dioleoyl-sn-glycero-3-phospho-(1'-rac-glycerol) (DOPG), dipalmitoylphosphatidylglycerol (DPPG), palmitoyloleoylphosphatidylethanolamine (POPE), distearoylphosphatidylethanolamine (DSPE), dipalmitoylphosphatidylethanolamine (DPPE), dimyristoylphosphatidylethanolamine (DMPE), 1-stearoyl-2-oleoylphosphatidylethanolamine (SOPE), 1-stearoyl-2-oleoyl-sn-glycero-3-phosphocholine (SOPC), sphingomyelin, phosphatidylcholine, phosphatidylethanolamine, phosphatidylserine, phosphatidylinositol, phosphatidic acid, palmitoyloleoylphosphatidylcholine, lysophosphatidylcholine, lysophosphatidylethanolamine (LPE).

In a preferred embodiment, the steroid or derivative thereof comprises at least one selected from cholesterol, cholesterol stearate, coprosterol, sitosterol, ergosterol, campesterol, stigmasterol, brassicasterol, fucosterol, tomatidine, ursolic acid and α-tocopherol.

In a preferred embodiment, the pulmonary delivery system containing ionizable lipids further comprises an alcohol compound.

In a preferred embodiment, the alcohol compound comprises at least one selected from the group consisting of C₆-C₁₈ alcohol and polyethylene glycol; the molar ratio of the alcohol compound to the ionizable lipids is 0-2:1, more preferably 0.01-0.5:1.

In a preferred embodiment, the amount of the ionizable lipid is 5-80 mol% of the total lipids in the formulation; and the amount of the helper lipid is 20-95 mol% of the total lipids in the formulation.

In a preferred embodiment, the helper lipid comprises phospholipid and steroids or a derivative thereof in a molar ratio of (5-60):(10-80).

In a preferred embodiment, the ionizable lipid is a first ionizable lipid, a second ionizable lipid, a third ionizable lipid, a mixture of the first ionizable lipid and the second ionizable lipid, or a mixture of the second ionizable lipid and the third ionizable lipid; in the mixture of the first ionizable lipid and the second ionizable lipid, the molar ratio of the first ionizable lipid to the second ionizable lipid is (5-90):(1-30); in the mixture of the second ionizable lipid and the third ionizable lipid, the molar ratio of the third ionizable lipid to the second ionizable lipid is (5-90):(1-30).

In a preferred embodiment, the pulmonary delivery system comprising ionizable lipids has a particle size of 20-400 nm, a polydispersity index (PDI) of less than 0.5.

In a preferred embodiment, the zeta potential of the delivery system ranges from -20 mV to 60 mV.

In a preferred embodiment, the pulmonary delivery system containing ionizable lipids further comprises other excipients.

In a preferred embodiment, the excipients are pharmaceutically acceptable excipients including but not limited to buffer salts, carriers, diluents, absorbents, colorants, adjuvants, preservatives, fillers, disintegrants, wetting agents, emulsifiers, suspending agents, sweeteners, flavorants, fragrances, antimicrobials, lubricants, dispersants, temperature stabilizers, adherents, stabilizers and suspending agents.

In a preferred embodiment, the pulmonary delivery system comprising ionizable lipids is in a liquid form or solid form.

In a second aspect, the present application provides a method for preparing the pulmonary delivery system comprising ionizable lipids, comprising the step of mixing a bioactive substance, an ionizable lipid and an helper lipid in a solvent.

In a preferred embodiment, the preparation method comprises dissolving ionizable lipids and helper lipids in a solvent to prepare a lipid solution; dissolving bioactive substances in a solvent to prepare a bioactive solution; mixing the lipid solution with the bioactive solution and performing ultrafiltration.

In a preferred embodiment, the solvent comprises at least one selected from alcohol, water and buffer solution.

In a third aspect, the present application provides use of the pulmonary delivery system comprising ionizable lipids in the manufacture of a medicament for delivering a bioactive substance by intranasal, intrabronchial or pulmonary administration.

In a preferred embodiment, the medicament is administered to mammals.

In a preferred embodiment, the medicament is administered to cells.

In a preferred embodiment, the cells are at least one selected from respiratory epithelial cell populations, nasal cell populations, alveolar epithelial cell populations, lung cell populations, bronchial epithelial cell populations and human bronchial epithelial(HBE) cell populations.

In a preferred embodiment, the medicament is in liquid form.

In a preferred embodiment, the medicament is in solid form.

In a preferred embodiment, the medicament is suitable for inhalation.

In a preferred embodiment, the medicament is administered by a nebulizer or an inhaler.

Compared with the prior art, the present application achieves the following advantageous effects:
1. The pulmonary delivery system of the present application comprises ionizable lipids, helper lipids and bioactive substances without any polymer-conjugated lipids. It can be administered intratracheally or via nebulized inhalation, both routes achieving efficient pulmonary delivery of bioactive substances with significant pulmonary expression and negligible expression in other visceral organs.
2. The pulmonary delivery system of the present application has a particle size of 20-400 nm and PDI < 0.5, with zeta potential ranging from -20 to 60 mV, exhibiting uniform particle size and excellent biosafety.
3. The preparation process of the pulmonary delivery system of the present application is simple and highly reproducible.
4. The pulmonary delivery system of the present application efficiently delivers therapeutic agents to the lungs and maintains consistent particle properties (particle size, polydispersity index, zeta potential, encapsulation efficiency, etc.) before and after nebulization, offering a safe and effective therapeutic strategy for pulmonary diseases.
5. The pulmonary delivery system of the present application disclosed possesses favorable safety and efficacy. For example, diseases can be treated via intracellular nanoparticle accumulation and target protein/mRNA expression, opening new therapeutic avenues for pulmonary disorders with broad application prospects.

### BRIEF DESCRIPTION OF THE DRAWINGS

FIG. *1**: In vivo* imaging of mice after intratracheal administration;
FIG. 2: *In vivo* imaging of mice after nebulized inhalation administration;
FIG. 3: Schematic diagram of IVIS imaging;
FIG. 4: IVIS imaging of pulmonary distribution after spray administration in Example 6.

### DETAILED DESCRIPTION OF THE EMBODIMENTS

The synthetic routes described herein tolerate a wide range of functional groups, and various substituted starting materials can be utilized. The general synthetic procedures described below afford the target final compounds at or near the end of the synthetic sequence; in certain cases, further conversion to pharmaceutically acceptable salts is required. The compounds of the present application can be synthesized from commercially available starting materials or intermediates readily prepared via literature protocols, using standard organic synthesis methods well known to a person skilled in the art or readily derivable from the teachings herein. Standard synthetic manipulations, functional group transformations and references can be retrieved from scientific literature and standard organic chemistry textbooks. The synthetic schemes outlined below are illustrative rather than restrictive for preparing the disclosed compounds.

Compounds of the general formulas described herein can be prepared from commercially available starting materials or starting materials synthesizable via literature procedures according to the processes illustrated in the corresponding general synthetic routes. Variables in each general synthetic route (such as R₁, R₂, R₃, etc.) are defined herein. A person skilled in the art will recognize that the sequence of certain steps in the reaction procedures and synthetic schemes described herein can be adjusted, for example the introduction and removal of protecting groups.

Multiple stereoisomers may be generated in the reaction schemes described herein. Where no specific stereoisomer is indicated, all stereoisomers obtainable from the reaction are intended to be encompassed. A person skilled in the art will appreciate that the reactions can be optimized to preferentially form a single isomer, or alternative synthetic schemes can be designed to afford a single isomer. Where an isomeric mixture is obtained, separation techniques including preparative thin-layer chromatography, preparative HPLC, preparative chiral HPLC and preparative SFC can be employed to isolate individual stereoisomers.

### Preparation of Example 1: Synthesis of the First Ionizable Lipid

General synthetic routes for the first ionizable lipid are shown in General Synthetic Routes 1-5.

### General Synthetic Route 1

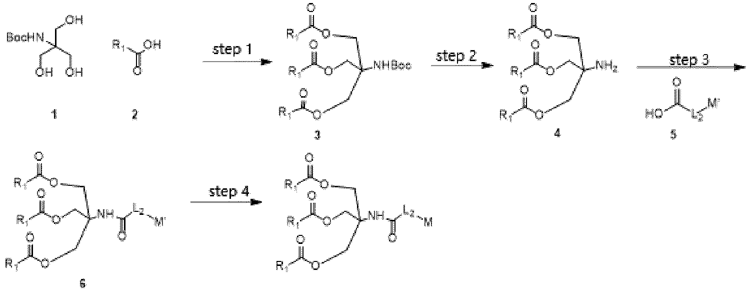

wherein M' denotes M or M bearing protecting groups.

As illustrated in General Synthetic Route 1 above, Boc-aminotris(hydroxymethyl)methane undergoes a condensation reaction with carboxylic acid (Compound 2) to afford Compound 3. Step 1 can be carried out in an organic solvent such as dichloromethane (DCM) in the presence of 1-ethyl-3-(3-dimethylaminopropyl)carbodiimide hydrochloride (EDCI) and 4-dimethylaminopyridine (DMAP), and the reaction of step 1 may be performed at room temperature for 24 hours.

Subsequently, Compound 3 undergoes Boc deprotection to yield Compound 4. Step 2 may be carried out in an organic solvent such as DCM under the catalysis of an acid like trifluoroacetic acid.

Thereafter, Compound 4 is subjected to a condensation reaction with Compound 5 to form Compound 6. Step 3 can be performed in an organic solvent such as DCM or DMF with EDCI and DMAP, or dicyclohexylcarbodiimide (DCC) as a catalyst.

If the M' of Compound 6 carries the aforementioned protecting groups, deprotection is performed to afford the target lipid compound. Step 4 is carried out under deprotection conditions matching the selected protecting groups.

### General Synthetic Route 2

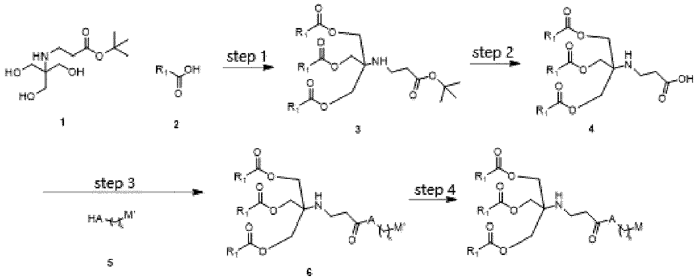

wherein M' = M or Mbearing protecting groups; A = O, NH or S.

As illustrated in General Synthetic Route 2 above, Compound 1 undergoes a condensation reaction with Compound 2 to obtain Compound 3. Step 1 can be carried out in an organic solvent such as DCM in the presence of EDCI and DMAP.

Subsequently, Compound 3 is deprotected to remove tert-butoxy protecting groups, affording Compound 4. Step 2 may be conducted in an organic solvent such as DCM with an acid (e.g., trifluoroacetic acid) and a cation scavenger (e.g., triisopropylsilane (TiPS)).

Thereafter, Compound 4 reacts with Compound 5 via condensation to yield Compound 6. Step 3 can be performed in an organic solvent such as DCM or DMF in the presence of EDCI and DMAP, or DCC.

If the M' of Compound 6 bears any of the aforementioned protecting groups, deprotection is carried out to obtain the target lipid compound. Step 4 is implemented under deprotection conditions suitable for the protecting groups employed.

### General Synthetic Route 3

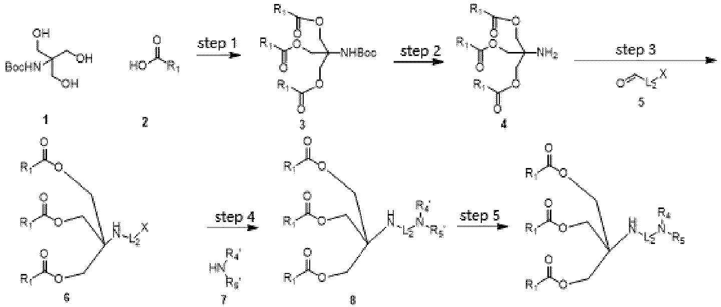

wherein X is a halogen such as Cl, Br or I; R₄' is R₄ or R₄ bearing protecting groups; R₅' is R₅ or R₅ bearing protecting group.

As illustrated in General Synthetic Route 3 above, Boc-aminotris(hydroxymethyl)methane undergoes a condensation reaction with Compound 2 to form Compound 3. Step 1 may be carried out in an organic solvent such as DCM in the presence of EDCI and DMAP, and the reaction can be held at room temperature for 24 hours.

Subsequently, Compound 3 undergoes Boc deprotection to afford Compound 4. Step 2 can be performed in an organic solvent such as DCM under acid catalysis with trifluoroacetic acid.

Then, Compound 4 reacts with a halogen-substituted aldehyde (Compound 5) via condensation-reduction to obtain Compound 6. Step 3 is conducted in an organic solvent such as 1,2-dichloroethane (DCE) with a reducing agent such as sodium triacetoxyborohydride (NaBH(OAc)₃).

Thereafter, Compound 6 undergoes a nucleophilic substitution reaction with an amine (Compound 7) to yield Compound 8. Step 4 can be implemented in an organic solvent such as DMF in the presence of a base and a catalyst; the base is selected from non-nucleophilic organic bases (e.g., N,N-diisopropylethylamine, iPr2EtN) or inorganic bases (e.g., K₂CO₃), and the catalyst is potassium iodide (KI) or sodium iodide (NaI).

If the R₄' and/or R₅' of Compound 8 contain any of the aforementioned protecting groups, deprotection is performed to provide the target lipid compound. Step 5 is carried out under deprotection conditions corresponding to the protecting groups adopted.

### General Synthetic Route 4

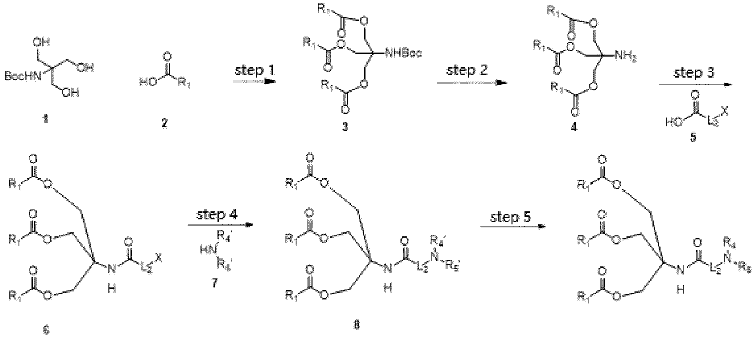

wherein X is a halogen such as Cl, Br or I; R₄' is R₄ or R₄ bearing protecting groups; R₅' is R₅ or R₅ bearing protecting group.

As set forth in General Synthetic Route 4 above, Boc-aminotris(hydroxymethyl)methane undergoes a condensation reaction with Compound 2 to form Compound 3. Step 1 can be carried out in an organic solvent such as DCM in the presence of EDCI and DMAP, and the reaction may be performed at room temperature for 24 hours.

Subsequently, Compound 4 is obtained after Boc deprotection of Compound 3. Step 2 can be conducted in an organic solvent such as DCM under catalysis of an acid like trifluoroacetic acid.

Thereafter, Compound 4 is subjected to a condensation reaction with halogen-substituted Compound 5 to afford Compound 6. Step 3 can be implemented in an organic solvent such as DCM or DMF with EDCI and DMAP, or DCC as the catalyst.

Then, Compound 6 reacts with an amine (Compound 7) via nucleophilic substitution to yield Compound 8. Step 4 may be performed in an organic solvent such as DMF in the presence of a base and a catalyst. The base is a non-nucleophilic organic base (e.g., N,N-diisopropylethylamine, iPr2EtN) or an inorganic base (e.g., K₂CO₃), and the catalyst is potassium iodide (KI) or sodium iodide (NaI).

If the R₄" and/or R₅' of Compound 8 contain any of the aforementioned protecting groups, deprotection is performed to obtain the target lipid compound. Step 5 is carried out under deprotection conditions matching the protecting groups utilized.

### General Synthetic Route 5

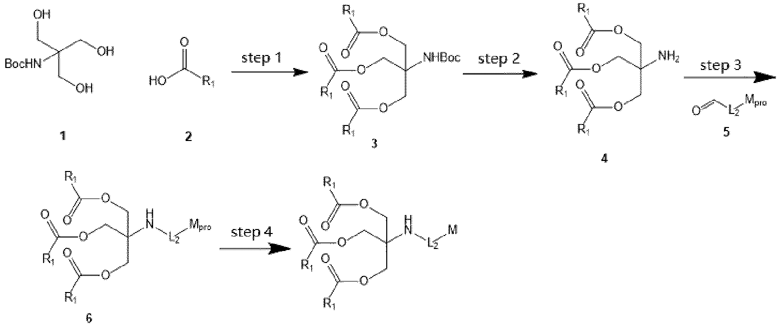

wherein Mₚᵣₒ = M or M bearing protecting groups.

As illustrated in General Synthetic Route 5 above, Boc-aminotris(hydroxymethyl)methane undergoes a condensation reaction with Compound 2 to form Compound 3. Step 1 can be carried out in an organic solvent such as DCM in the presence of EDCI and DMAP, and the reaction may be maintained at room temperature for 24 hours.

Subsequently, Compound 3 is deprotected by removal of the Boc group to afford Compound 4. Step 2 can be carried out in an organic solvent (e.g., DCM) in the presence of an acid (e.g., TFA) as a catalyst.

Thereafter, Compound 4 reacts with Compound 5 via condensation-reduction to obtain Compound 6. Step 3 is conducted in an organic solvent such as 1,2-dichloroethane (DCE) in the presence of a reducing agent, for example sodium triacetoxyborohydride (NaBH(OAc)₃).

If the Mₚᵣₒ of Compound 6 bears any of the aforementioned protecting groups, deprotection is carried out to yield the target lipid compound. Step 4 is implemented under deprotection conditions suitable for the selected protecting groups.

Furthermore, it should also be understood that any specific embodiment of the present application falling within the scope of the prior art may be expressly excluded from any one or more claims. Such embodiments may be excluded as they are deemed known to a person skilled in the art, even if such exclusion is not explicitly set forth herein.

All cited sources, such as references, publications, databases, database entries and techniques cited herein, are incorporated into the present application by reference, even where such incorporation is not explicitly stated in the citation. In the event of any conflict between the disclosure of a cited source and the statements of the present application, the statements of the present application shall prevail.

### (1) Synthesis of compounds represented by Formula (1), (1A), (1B), (1C), (1D), (1E), (1F), (1G), (1H), (1I), (1J), (1K), (1L) or (1M)

### A. General Considerations

It should be noted that all raw materials used in the present application are commercially available common products, and there is no specific limitation on their sources.

The synthetic routes described below can be adopted to prepare Compounds 1001-3422 of the present application.

The following abbreviations are used herein:
THF: Tetrahydrofuran
MeCN: Acetonitrile
MeOH: Methanol
PE: Petroleum ether
EA: Ethyl acetate
DMF: N,N-Dimethylformamide
EDCI: 1-Ethyl-3-(3-dimethylaminopropyl)carbodiimide hydrochloride
LAH: Lithium aluminum hydride
DCM: Dichloromethane
DMAP: 4-Dimethylaminopyridine
LDA: Lithium diisopropylamide
rt: Room temperature
DCE: 1,2-Dichloroethane
n-BuLi: n-Butyllithium
i-Pr2EtN: N,N-Diisopropylethylamine

### B. Intermediate Synthesis

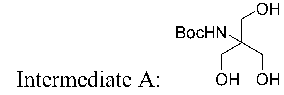

The intermediate A was obtained by the following synthetic route

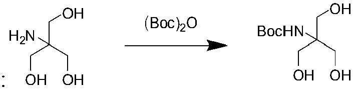

Tris(hydroxymethyl)aminomethane (50.0 g) and di-tert-butyl dicarbonate (Boc2O) (99.1 g) were dissolved in a mixed solvent of methanol (300 mL) and water (30 mL). The mixture was reacted at room temperature for 72 h, then purified by silica gel column chromatography (eluent: DCM/MeOH = 20:1-10:1) to afford Intermediate A (N-Boc-tris(hydroxymethyl)aminomethane) as a white solid (75.0 g). LCMS (ESI): calculated for C9H19NO5, [M+H]⁺ m/z 222.13; found 222.25.

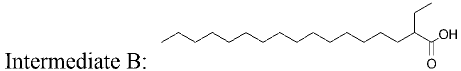

The intermediate B was obtained by the following synthetic route:

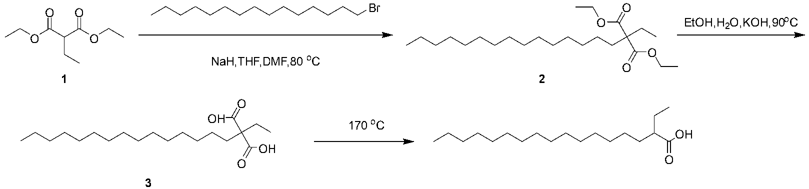

Diethyl 2-ethylmalonate (29.0 g), THF (90 mL) and DMF (30 mL) were charged into a single-neck flask. NaH (3.7 g) was added under ice cooling, and the mixture was stirred at room temperature for 30 min under nitrogen protection. 1-Bromopentadecane (15.0 g) was then added, and the reaction was heated at 80°C for 2 h under nitrogen. THF was removed by concentration under reduced pressure, and the residue was dropwise added into ice water, followed by addition of ethyl acetate and stirring. The mixture was filtered through celite, and the organic layer was collected and separated. The organic phase was washed with saturated brine and separated, then purified by silica gel column chromatography (eluent: PE/EA = 100:1-50:1) to afford diethyl 2-ethyl-2-pentadecylmaleate (16.5 g). Diethyl 2-ethyl-2-pentadecylmaleate (8.0 g) was combined with EtOH (50 mL), water (50 mL) and KOH (11.3 g), and heated at 90°C for 12 h. EtOH was removed by concentration under reduced pressure, and dilute hydrochloric acid was added to adjust the system pH to 4-5. Water and ethyl acetate were added for stirring and liquid separation; the organic layer was washed with saturated brine, separated, and purified by column chromatography (eluent: PE/EA = 5:1 to 1:1) to obtain 2-ethyl-2-pentadecylmaleic acid (5.2 g).2-Ethyl-2-pentadecylmaleic acid (10.0 g) was heated at 170°C for 6 h under open conditions, then cooled to room temperature. Water and ethyl acetate were added for stirring and phase separation. The organic layer was washed with saturated brine, separated and concentrated, then purified by column chromatography (eluent: DCM/MeOH = 20:1-10:1) to provide Intermediate B (2-ethylheptadecanoic acid) (8.2 g). ¹H NMR (400 MHz, CDCl₃): δ 2.31 (tt, J = 8.6, 5.3 Hz, 1H), 1.71-1.46 (m, 4H), 1.28 (s, 26H), 0.96 (t, J = 7.4 Hz, 3H), 0.90 (t, J = 6.7 Hz, 3H).

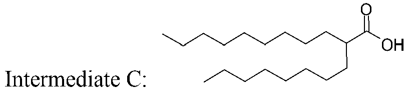

The intermediate C was obtained by the following synthetic route:

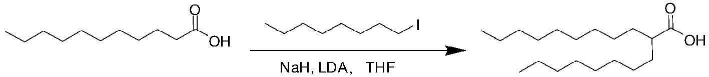

Decanoic acid (50 g) and THF (500 mL) were added to a three-neck flask. The system was cooled to 0 °C, then NaH (23.22 g) was slowly charged. After stirring at 0 °C under nitrogen protection for 1 h, LDA (62.19 g) was added dropwise slowly, and stirring was maintained at 0 °C under nitrogen for another 1 h. Nonyl iodide (88.52 g) was added dropwise lastly, the mixture was warmed to room temperature and stirred overnight. The reaction mixture was diluted with 1 L of DCM and washed successively with saturated aqueous ammonium chloride (NH₄ Cl) solution and water. The organic layer was dried over anhydrous sodium sulfate (Na₂ SO₄ ), filtered and concentrated, then purified by silica gel column chromatography (eluent: PE/EA = 50:1) to afford Intermediate C (2-octylundecanoic acid) (11 g). ¹H NMR (400 MHz, Chloroform-d): δ 2.37 (tt, J = 8.7, 5.1 Hz, 1H), 1.71-1.58 (m, 2H), 1.48 (dt, J = 13.3, 6.7 Hz, 2H), 1.29 (d, J = 9.4 Hz, 25H), 0.90 (t, J = 6.8 Hz, 6H).

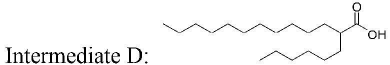

The intermediate D was obtained by the following synthetic route:

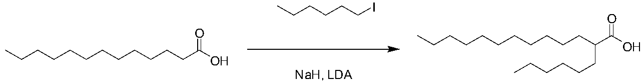

Tridecanoic acid (79.5 g) and THF (800 mL) were charged into a three-neck flask, and the system was cooled to 0 °C before slow addition of NaH (22.25 g). The reaction mixture was stirred at 0 °C under nitrogen protection for 1 h, followed by slow dropwise addition of LDA (317.87 g), and stirring was continued at 0 °C under nitrogen for another 1 h. n-Hexyl iodide (94.39 g) was added dropwise, then the mixture was warmed to room temperature and stirred overnight. The reaction solution was diluted with 1 L of DCM and washed sequentially with saturated ammonium chloride aqueous solution and water. The organic layer was dried over anhydrous sodium sulfate, filtered and concentrated, then purified by silica gel column chromatography (eluent: PE/EA = 50:1) to afford Intermediate D (2-hexyltridecanoic acid) (26 g). ¹H NMR (400 MHz, Chloroform-d): δ 2.36 (tt, J = 8.7, 5.4 Hz, 1H), 1.63 (ddd, J = 14.3, 8.7, 5.5 Hz, 2H), 1.49 (dq, J = 13.5, 6.6 Hz, 2H), 1.29 (d, J = 10.1 Hz, 26H), 0.90 (t, J = 6.6 Hz, 6H).

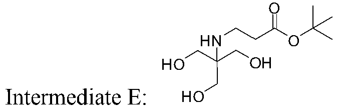

The intermediate E was obtained by the following synthetic route:

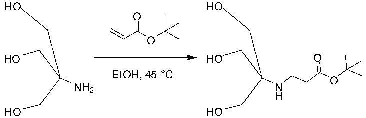

Tris(hydroxymethyl)aminomethane (10.0 g) and tert-butyl acrylate (21.1 g) were dissolved in EtOH (150 mL). The mixture was reacted at 45 °C under nitrogen protection for 30 h, then EtOH was removed by concentration under reduced pressure. The residue was triturated with a solvent mixture (PE/EA = 20:1, 100 mL × 3) and filtered to afford Intermediate E as a white solid (11.5 g). ¹H NMR (400 MHz, CDCl₃): δ 3.60 (s, 6H), 2.84 (t, J = 5.9 Hz, 2H), 2.47 (t, J = 5.8 Hz, 2H), 1.48 (s, 9H).

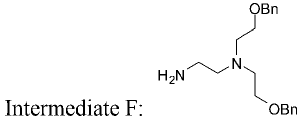

The intermediate F was obtained by the following synthetic route:

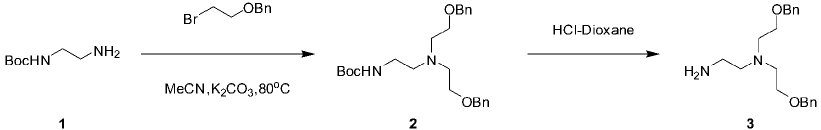

tert-Butyl (2-aminoethyl)carbamate (2.7 g), MeCN (90 mL), benzyl 2-bromoethyl ether (7.99 g) and K₂CO₃ (11.65 g) were charged into a single-neck flask. The mixture was heated at 80 °C and stirred overnight. Water and ethyl acetate were added, and the mixture was stirred and subjected to liquid-liquid separation. The organic layer was washed with saturated brine, separated and concentrated, then purified by silica gel column chromatography (eluent: PE/EA = 10:1 to 5:1) to obtain Compound 2 (5.5 g). Dioxane (30 mL) and HCl solution in dioxane (30 mL) were added to Compound 2. After stirring at room temperature for 3 h, the mixture was concentrated under reduced pressure to yield Compound 3, namely Intermediate F,(6 g). LCMS (ESI): calculated for C₂₀H₂₈N₂O₂, [M+H]⁺ m/z 329.22; found 329.24.

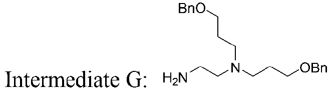

The intermediate G was obtained by the following synthetic route:

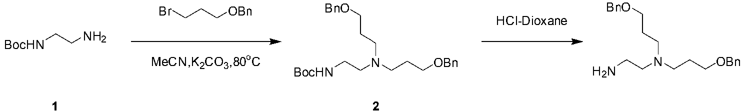

Compound 1 (3.0 g), MeCN (90 mL), 3-benzyloxypropyl bromide (9.45 g) and K₂CO₃ (12.94 g) were added to a single-neck flask. The mixture was heated at 80 °C and stirred overnight. Water and ethyl acetate were added, the mixture was stirred and separated. The organic phase was washed with saturated brine, separated and concentrated, then purified by column chromatography (eluent: PE/EA = 10:1 to 5:1) to afford Compound 2 (6.9 g). Dioxane (30 mL) and HCl solution in dioxane (30 mL) were added to Compound 2 (6.9 g). After stirring at room temperature for 3 h, the mixture was concentrated under reduced pressure to obtain Intermediate G (7.5 g). LCMS (ESI): calculated for C₂₂H₃₂N₂O₂, [M+H]⁺ m/z 357.25; found 357.51.

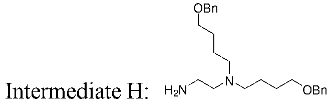

The intermediate H was obtained by the following synthetic route:

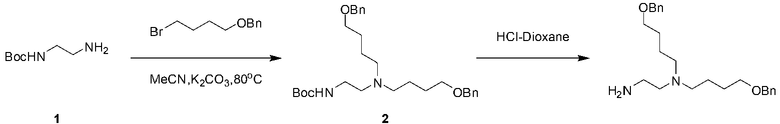

Compound 1 (1.5 g), MeCN (90 mL), benzyl 4-bromobutyl ether (5.02 g) and K₂CO₃ (6.47 g) were charged into a single-neck flask. The mixture was heated at 80 °C and stirred overnight. Water and ethyl acetate were added, and the mixture was stirred before phase separation. The organic layer was washed with saturated brine, separated and concentrated, then purified by column chromatography (eluent: PE/EA = 20:1 to 10:1) to afford Compound 2 (4.1 g). Dioxane (30 mL) and HCl solution in dioxane (30 mL) were added to Compound 2 (4.1 g). After stirring at room temperature for 3 h, the mixture was concentrated under reduced pressure to obtain Intermediate H (4.9 g). LCMS (ESI): calculated for C₂₄H₃₆N₂O₂, [M+H]⁺ m/z 385.28; found 385.56.

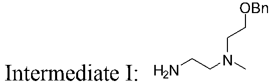

The intermediate I was obtained by the following synthetic route:

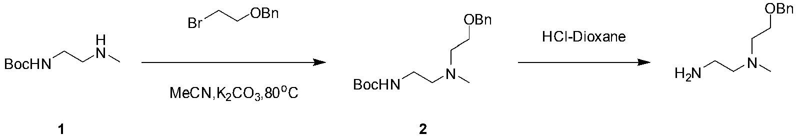

Compound 1 (3.9 g), MeCN (90 mL), benzyl 2-bromoethyl ether (5.78 g) and K₂CO₃ (15.46 g) were charged into a single-neck flask. The mixture was heated at 80 °C and stirred overnight. Water and ethyl acetate were added, and the mixture was stirred prior to phase separation. The organic layer was washed with saturated brine, separated and concentrated, then purified by column chromatography (eluent: PE/EA = 20:1 to 10:1) to afford Compound 2 (6.8 g). Dioxane (30 mL) and HCl solution in dioxane (30 mL) were added to Compound 2 (6.8 g). After stirring at room temperature for 3 h, the mixture was concentrated under reduced pressure to yield Intermediate I (6.5 g). LCMS (ESI): calculated for C₁₂H₂₀N₂O, [M+H]⁺ m/z 209.16; found 209.31.

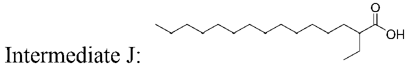

The intermediate J was obtained by the following synthetic route:

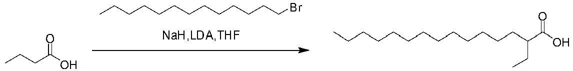

Butyric acid (5.0 g) and THF (100 mL) were added to a single-neck flask. NaH (2.73 g) was charged at 0 °C, followed by slow dropwise addition of LDA (56.8 mL). The mixture was stirred at room temperature for 30 min, then 1-bromotridecane was added, and stirring was continued overnight at room temperature. Ice water and ethyl acetate were introduced for stirring and phase separation. The organic layer was washed with saturated brine, separated and concentrated, then purified by column chromatography (eluent: PE/EA = 10:1 to 5:1) to afford Intermediate J (5.0 g). LCMS (ESI): calculated for C₁₇H₃₄O₂, [M+H]⁺ m/z 271.26; found 271.46.

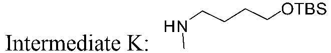

The intermediate K was obtained by the following synthetic route:

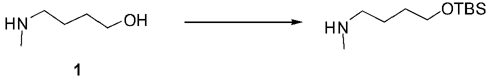

Reactant 1 (24 g), imidazole (19.00 g) and DCM (200 mL) were added to a three-neck flask. The mixture was cooled to 0 °C, then TBDMSCl (38.57 g) was added slowly. After stirring at 25 °C for 4 h, the reaction mixture was diluted with 300 mL DCM. The organic layer was washed twice with 1 L water, dried over anhydrous Na₂SO₄, filtered and concentrated. The residue was loaded onto silica gel and purified by column chromatography with eluent DCM/MeOH = 1:20. The target fractions were combined and concentrated to afford Intermediate K (28.00 g). ¹H NMR (400 MHz, CDCl₃): δ 4.89 (s, 1H), 3.58 (t, J = 6.1 Hz, 2H), 2.66 (t, J = 7.3 Hz, 2H), 2.45 (s, 3H), 1.67-1.40 (m, 4H), 0.84 (s, 9H).

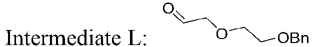

The intermediate L was obtained by the following synthetic route:

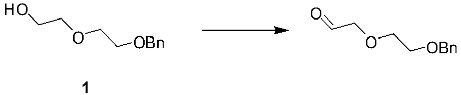

DMSO (3.58 g) and anhydrous DCM (30 mL) were charged into a three-neck flask. The system was cooled to -78 °C, then oxalyl chloride (2.91 g) was added slowly. The reaction mixture was stirred at -78 °C under nitrogen protection for 10 min, followed by slow dropwise addition of Compound 1 (3 g), and stirring was maintained at -78 °C under nitrogen for another 1 h. TEA (9.28 g) was added dropwise lastly, and the mixture was stirred at -78 °C under nitrogen for 0.5 h. The reaction solution was diluted with 100 mL DCM and washed sequentially with saturated aqueous NH₄Cl solution and water. The organic layer was dried over anhydrous Na₂SO₄, filtered and concentrated to afford Intermediate L (3.0 g). LCMS (ESI): calculated for C₁₁H₁₄O₃, [M+H]⁺ m/z 195.09; found 195.23.

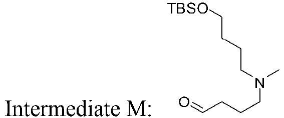

The intermediate M was obtained by the following synthetic route:

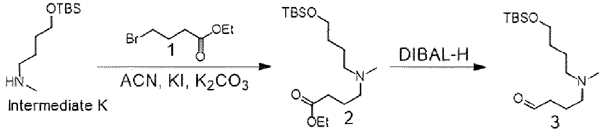

Intermediate K (2.0 g), Compound 1 (1.79 g) and ACN (20 mL) were charged into a sealed tube. K₂CO₃ (3.81 g) and KI (1.52 g) were added, and the mixture was stirred overnight at 70 °C under nitrogen protection. The reaction mixture was diluted with 100 mL EA, then washed successively with saturated aqueous NH₄Cl solution and water. The organic layer was dried over anhydrous Na₂SO₄, filtered and concentrated. The residue was purified by silica gel column chromatography with eluent DCM/MeOH = 15:1. Combined target fractions were concentrated to obtain Compound 2 (2.0 g). Compound 2 (1.80 g) and DCM (20 mL) were added to a three-neck flask. The flask was purged with nitrogen and cooled to -78 °C, then DIBAL-H (1.00 g) was added dropwise. The mixture was stirred at -78 °C for 4 h, then quenched by dropwise addition of methanol and aqueous sodium carbonate solution. The mixture was extracted with DCM (100 mL). The organic layer was dried over anhydrous sodium sulfate, filtered and concentrated to afford Intermediate M (1.36 g). LCMS (ESI): calculated for C₁₅H₃₃NO₂Si, [M+H]⁺ m/z 288.52; found 288.23.

Compound 1003 was synthesized according to General Synthetic Route 1. 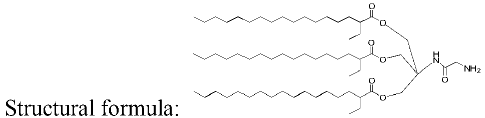
Molecular formula: C₆₃H₁₂₂N₂O₇
Molecular weight: 1019.68

### Step 1: Synthesis of Compound 3 in General Synthetic Route 1

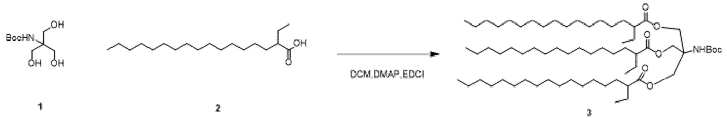

Compound 1 (Intermediate A) (1.0 g), DCM (20 mL), DMAP (2.2 g) and EDCI (3.4 g) were added to Compound 2 (Intermediate B) (4.3 g). The mixture was stirred at room temperature under nitrogen protection for 12 h. Water and dichloromethane were added, and the mixture was stirred for phase separation. The organic layer was washed with saturated brine, dried over anhydrous sodium sulfate and concentrated, then purified by silica gel column chromatography (eluent: PE/EA = 40:1 to 30:1) to afford Compound 3 (2.6 g).

### Step 2: Synthesis of Compound 4 in General Synthetic Route 1

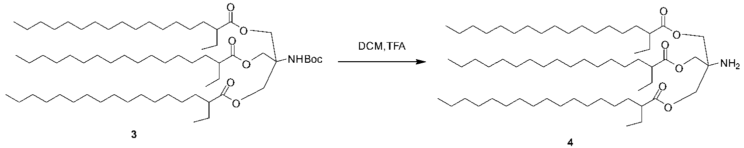

DCM (15 mL) and TFA (5 mL) were added to Compound 3 (2.6 g). The mixture was stirred at room temperature for 3 h and concentrated. The residue was purified by silica gel column chromatography (eluent: PE/EA = 10:1 to 5:1) to obtain Compound 4 (3.2 g).

### Step 3: Synthesis of Compound 6 in General Synthetic Route 1

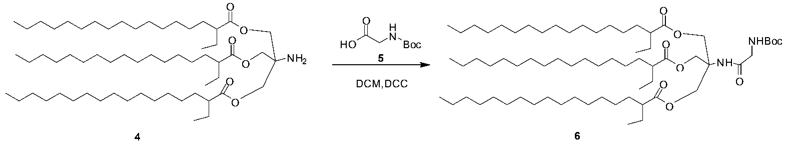

DCM (20 mL), Boc-glycine (Compound 5, 578 mg) and DCC (6.8 g) were added to Compound 4 (3.2 g) and stirred at room temperature under nitrogen protection for 12 h. Water and ethyl acetate were added, and the mixture was stirred for phase separation. The organic layer was washed with saturated brine, dried over anhydrous sodium sulfate and concentrated, then purified by column chromatography (eluent: PE/EA = 5:1 to 2:1) to afford Compound 6 (2.2 g).

### Step 4: Synthesis of Compound 1003

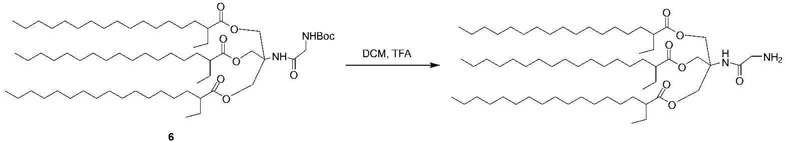

Compound 6 (2.2 g) was mixed with DCM (15 mL) and TFA (5 mL). The mixture was stirred at room temperature for 12 h and concentrated. The residue was purified by column chromatography (eluent: DCM/MeOH = 30:1 to 20:1) to obtain Compound 1003 (1.3 g). ¹H NMR (400 MHz, CDCl₃): δ 4.49 (d, J = 2.6 Hz, 6H), 3.28 (s, 2H), 2.32 (tt, J = 8.6, 5.5 Hz, 3H), 1.62 (q, J = 7.1 Hz, 12H), 1.27 (d, J = 3.4 Hz, 78H), 0.94-0.86 (m, 18H).

D. Compound 1002 was synthesized according to General Synthetic Route 1. 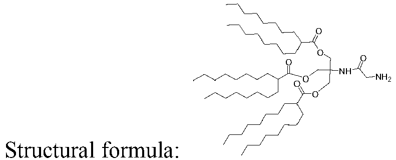
Molecular formula: C₆₀H₁₁₆N₂O₇
Molecular weight: 977.60

It was synthesized following General Synthetic Route 1 with a procedure similar to that of Compound 1003, except that Intermediate C was used as Compound 2 in General Synthetic Route 1. ¹H NMR (400 MHz, CDCl₃): δ 4.48 (s, 6H), 3.27 (s, 2H), 2.37 (tt, J = 8.5, 5.5 Hz, 3H), 1.64 (d, J = 6.9 Hz, 6H), 1.50-1.42 (m, 6H), 1.27 (s, 72H), 0.90 (t, J = 6.7 Hz, 18H).

E. Compound 1004 was synthesized according to General Synthetic Route 1. 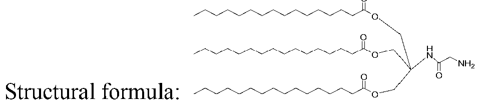
Molecular formula: C₅₄H₁₀₄N₂O₇
Molecular weight: 893.43

It was synthesized following General Synthetic Route 1 with a procedure similar to that of Compound 1003, except that palmitic acid was used as Compound 2 in General Synthetic Route 1. ¹H NMR (400 MHz, CDCl₃): δ 7.55 (s, 1H), 4.45 (s, 6H), 3.74 (s, 2H), 2.35 (t, J = 7.6 Hz, 6H), 1.60 (p, J = 6.9 Hz, 6H), 1.27 (s, 72H), 0.90 (t, J = 6.7 Hz, 9H).

F. Compound 1001 was synthesized according to General Synthetic Route 1. 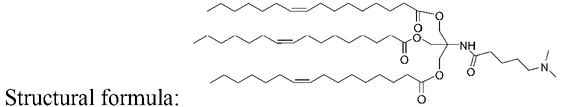
Molecular formula: C₅₉H₁₀₈N₂O₇
Molecular weight: 957.52

It was synthesized following General Synthetic Route 1 with a synthetic procedure similar to that of Compound 1003,except that (9Z)-hexadec-9-enoic acid was used as Compound 2 and 5-(N,N-dimethylamino)pentanoic acid as Compound 5 in General Synthetic Route 1. ¹H NMR (400 MHz, CDCl₃): δ 5.42-5.30 (m, 6H), 4.51-4.38 (m, 6H), 3.19 (dt, J = 11.3, 5.6 Hz, 2H), 2.97 (t, J = 5.9 Hz, 6H), 2.46 (t, J = 6.6 Hz, 1H), 2.35 (td, J = 7.6, 3.1 Hz, 5H), 2.20 (t, J = 7.6 Hz, 2H), 2.03 (d, J = 6.2 Hz, 12H), 1.89-1.81 (m, 3H), 1.74 (q, J = 7.8 Hz, 2H), 1.67-1.51 (m, 6H), 1.31 (d, J = 7.8 Hz, 48H), 0.90 (t, J = 6.7 Hz, 9H).

G. Compound 1014 was synthesized according to General Synthetic Route 2. 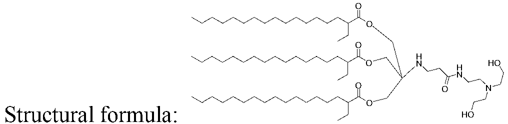
Molecular formula: C₇₀H₁₃₇N₃O₉
Molecular weight: 1164.88

### Step 1: Synthesis of Intermediate 3 in General Synthetic Route 2

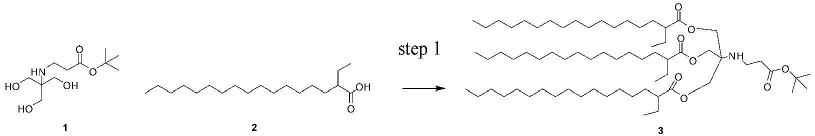

Intermediate 3 was synthesized following the procedure described in Step 1 of General Synthetic Route 1, with the only modification that Intermediate E was used as Compound 1 in General Synthetic Route 1.

### Step 2: Synthesis of Compound 4 in General Synthetic Route 2

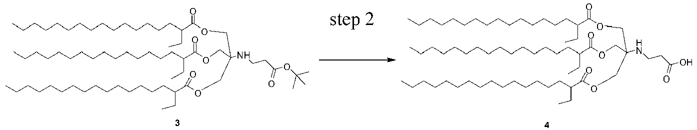

DCM (15 mL), TFA (12 mL) and TiPS (3 mL) were added to Compound 3 (4.8 g). The mixture was reacted at room temperature for 12 h, then water and dichloromethane were added and stirred for phase separation. The organic layer was washed with saturated brine, separated and concentrated. The residue was purified by silica gel column chromatography (eluent: DCM/MeOH = 20:1 to 10:1) to afford Compound 4 (3.3 g).

### Step 3: Synthesis of Compound 6 in General Synthetic Route 2

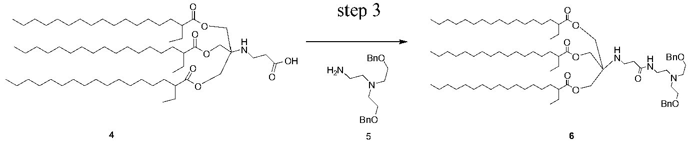

Compound 4 (4.0 g), DCM (80 mL), EDCI (1.1 g), 1-hydroxybenzotriazole (HOBt) (0.78 g) and DIEA (2.5 g) were added to Compound 5 (1.9 g) and stirred at room temperature for 12 h. Water and dichloromethane were added and stirred for phase separation. The organic phase was washed with saturated brine, separated and concentrated. The residue was purified by silica gel column chromatography (eluent: DCM/MeOH = 30:1 to 20:1) to obtain Compound 6 (4.0 g).

### Step 4: Synthesis of Compound 1014

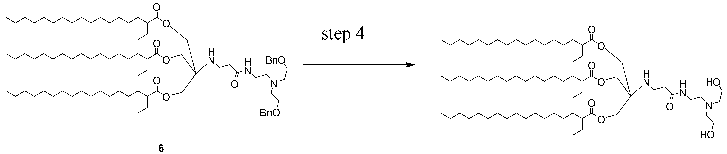

MeOH (30 mL) and DCM (10 mL) and Pd/C (4.73 g (10%)) were added to Compound 6 (4.0 g). The mixture was stirred overnight at room temperature under hydrogen atmosphere. The suspension was filtered through celite and concentrated. The residue was purified by silica gel column chromatography with eluent DCM/MeOH = 30:1 to 15:1 to yield Compound 1014 (2 g). ¹H NMR (400 MHz, CDCl₃): δ 7.38 (t, J = 5.8 Hz, 1H), 4.14-4.05 (m, 6H), 3.58 (t, J = 4.9 Hz, 4H), 3.31 (q, J = 5.7 Hz, 2H), 2.89 (t, J = 6.3 Hz, 2H), 2.64 (q, J = 5.2 Hz, 6H), 2.29 (dq, J = 10.8, 4.4 Hz, 6H), 1.66-1.37 (m, 12H), 1.24 (d, J = 4.3 Hz, 78H), 0.87 (t, J = 7.1 Hz, 18H).

H. Compound 1015 was synthesized according to General Synthetic Route 2. 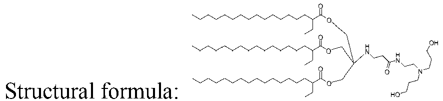
Molecular formula: C₇₂H₁₄₁N₃O₉
Molecular weight: 1192.93

It was synthesized following General Synthetic Route 2 with a procedure similar to that of Compound 1014, except that Intermediate G was used as Compound 5 in General Synthetic Route 2. ¹H NMR (400 MHz, CDCl₃): δ 7.58 (t, J = 5.7 Hz, 1H), 4.15-4.01 (m, 6H), 3.70 (t, J = 5.4 Hz, 4H), 3.39 (q, J = 5.7 Hz, 2H), 2.86 (t, J = 6.2 Hz, 2H), 2.57 (dt, J = 20.5, 5.8 Hz, 6H), 2.36-2.22 (m, 5H), 1.76-1.67 (m, 4H), 1.62-1.35 (m, 13H), 1.23 (d, J = 4.6 Hz, 78H), 0.92-0.80 (m, 18H).

I. Compound 1020 was synthesized according to General Synthetic Route 2. 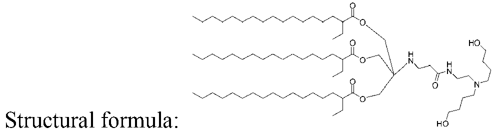
Molecular formula: C₇₄H₁₄₅N₃O₅
Molecular weight: 1220.99

It was synthesized according to General Synthetic Route 2 with a procedure similar to that of Compound 1014, except that Intermediate H was used as Compound 5 in General Synthetic Route 2.

J. Compound 1025 was synthesized according to General Synthetic Route 2. 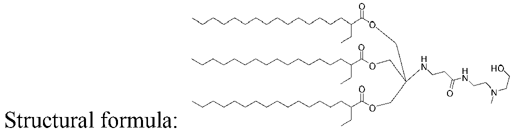
Molecular formula: C₆₉H₁₃₅N₃O₈
Molecular weight: 1134.85

It was synthesized following General Synthetic Route 2 with a synthetic procedure similar to that of Compound 1014, except that Intermediate I was used as Compound 5 in General Synthetic Route 2. ¹H NMR (400 MHz, CDCl₃): δ 6.98 (t, J = 5.7 Hz, 1H), 4.10 (m, J = 3.8 Hz, 6H), 3.63 (t, J = 5.2 Hz, 2H), 3.35 (q, J = 5.8 Hz, 2H), 2.89 (t, J = 6.1 Hz, 2H), 2.57 (q, J = 5.3 Hz, 4H), 2.32 (s, 3H), 2.28 (q, J = 4.1 Hz, 5H), 1.65-1.41 (m, 13H), 1.24 (d, J = 4.4 Hz, 78H), 0.87 (t, J = 7.1 Hz, 18H).

K. Compound 1005 was synthesized according to General Synthetic Route 2. 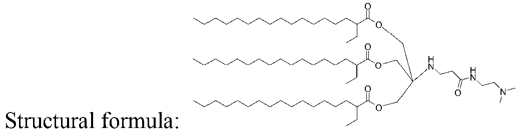
Molecular formula: C₆₈H₁₃₃N₃O₇
Molecular weight: 1104.83

It was synthesized according to General Synthetic Route 2 with a procedure similar to that of Compound 1014, except that N,N-dimethylethylenediamine was used as Compound 5 in General Synthetic Route 2. ¹H NMR (400 MHz, CDCl₃): δ 4.19-4.07 (m, 6H), 3.40 (d, J = 5.7 Hz, 2H), 2.95-2.87 (m, 2H), 2.82 (s, 4H), 2.64-2.55 (m, 2H), 2.38 (s, 6H), 2.33-2.29 (m, 3H), 1.63-1.55 (m, 6H), 1.49-1.44 (m, 2H), 1.26 (d, J = 5.4 Hz, 78H), 0.89 (td, J = 7.0, 2.8 Hz, 18H).

L. Compound 1006 was synthesized according to General Synthetic Route 2. 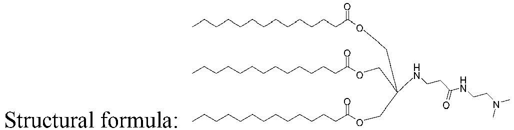
Molecular formula: C₅₃H₁₀₃N₃O₇
Molecular weight: 894.42

It was synthesized via General Synthetic Route 2 with a procedure similar to that of Compound 1014, except that myristic acid was used as Compound 2 and N,N-dimethylethylenediamine as Compound 5 in General Synthetic Route 2. ¹H NMR (400 MHz, CDCl₃): δ 7.99 (t, J = 5.8 Hz, 1H), 4.13 (s, 6H), 3.60 (q, J = 5.3 Hz, 2H), 3.38-3.34 (m, 2H), 3.00 (s, 6H), 2.94 (t, J = 5.8 Hz, 2H), 2.45 (t, J = 5.8 Hz, 2H), 2.35 (t, J = 7.6 Hz, 6H), 1.66-1.56 (m, 6H), 1.33-1.23 (m, 60H), 0.90 (t, J = 6.8 Hz, 9H).

M. Compound 1007 was synthesized according to General Synthetic Route 2. 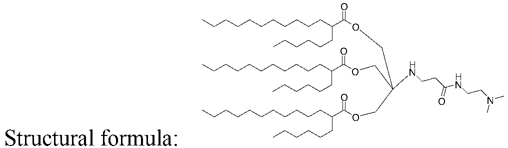
Molecular formula: C₆₈H₁₃₃N₃O₇
Molecular weight: 1104.83

It was synthesized following General Synthetic Route 2 with a procedure similar to that of Compound 1014, except that Intermediate D was used as Compound 2 and N,N-dimethylethylenediamine as Compound 5 in General Synthetic Route 2. ¹H NMR (400 MHz, Chloroform-d): δ 7.13 (t, J = 5.0 Hz, 1H), 4.13 (s, 6H), 3.33 (q, J = 5.6 Hz, 2H), 2.92 (t, J = 6.0 Hz, 2H), 2.47-2.28 (m, 7H), 2.24 (s, 6H), 1.60 (dq, J = 14.7, 7.2 Hz, 6H), 1.47 (dd, J = 14.2, 6.7 Hz, 6H), 1.27 (d, J = 3.4 Hz, 78H), 0.89 (td, J = 6.7, 2.5 Hz, 18H).

N. Compound 1008 was synthesized according to General Synthetic Route 2. 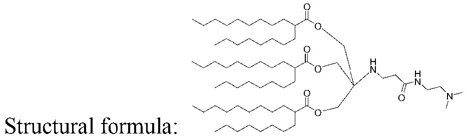
Molecular formula: C₆₈H₁₃₃N₃O₇
Molecular weight: 1104.01

It was synthesized with General Synthetic Route 2 by a procedure similar to that of Compound 1014, except that Intermediate C served as Compound 2 and N,N-dimethylethylenediamine as Compound 5 in General Synthetic Route 2. ¹H NMR (400 MHz, Chloroform-d): δ 4.13 (s, 6H), 3.33 (q, J = 5.6 Hz, 2H), 2.92 (t, J = 5.9 Hz, 2H), 2.51-2.26 (m, 7H), 2.24 (s, 6H), 1.59 (ddt, J = 14.8, 10.9, 6.4 Hz, 6H), 1.47 (tq, J = 11.0, 5.4 Hz, 6H), 1.26 (s, 77H), 0.89 (t, J = 6.7 Hz, 18H).

O. Compound 1009 was synthesized according to General Synthetic Route 2. 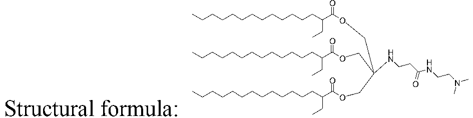
Molecular formula: C₆₂H₁₂₁N₃O₇
Molecular weight: 1020.66

It was synthesized following General Synthetic Route 2 with a procedure similar to that of Compound 1014, except that Intermediate J was used as Compound 2 and N,N-dimethylethylenediamine as Compound 5 in General Synthetic Route 2. ¹H NMR (400 MHz, CDCl₃): δ 7.04 (t, J = 5.0 Hz, 1H), 4.12 (m, J = 3.3 Hz, 6H), 3.30 (q, J = 5.6 Hz, 2H), 2.91 (t, J = 6.0 Hz, 2H), 2.39 (t, J = 6.0 Hz, 2H), 2.33-2.25 (m, 5H), 2.22 (s, 6H), 1.76-1.54 (m, 12H), 1.24 (m, J = 3.7 Hz, 66H), 0.90-0.84 (m, 18H).

P. Compound 1011 was synthesized according to General Synthetic Route 2. 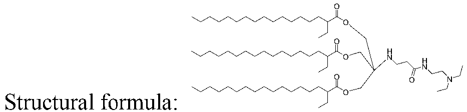
Molecular formula: C₇₀H₁₃₇N₃O₇
Molecular weight: 1132.88

It was synthesized following General Synthetic Route 2 with a procedure similar to that of Compound 1014, except that N,N-diethylethylenediamine was used as Compound 5 in General Synthetic Route 2. ¹H NMR (400 MHz, CDCl₃): δ 4.19-4.08 (m, 6H), 3.31 (q, J = 5.8 Hz, 2H), 2.92 (t, 2H), 2.57 (t, J = 6.9 Hz, 6H), 2.37-2.26 (m, 5H), 1.62 (ddd, J = 18.3, 9.0, 4.3 Hz, 12H), 1.27 (d, J = 4.7 Hz, 78H), 1.04 (t, J = 7.1 Hz, 6H), 0.94-0.86 (m, 18H).

Q. Compound 1012 was synthesized according to General Synthetic Route 2. 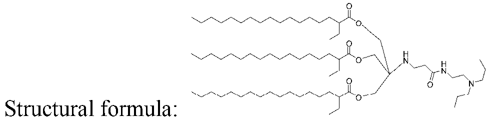
Molecular formula: C₇₂H₁₄₁N₃O₇
Molecular weight: 1160.93

It was synthesized according to General Synthetic Route 2 with a procedure similar to that of Compound 1014, except that N,N-diethylethylenediamine was used as Compound 5 in General Synthetic Route 2. ¹H NMR (400 MHz, CDCl₃): δ 6.59 (s, 1H), 4.18-4.07 (m, 6H), 3.29 (q, J = 5.7 Hz, 2H), 2.91 (t, J = 6.2 Hz, 2H), 2.54 (t, J = 6.1 Hz, 2H), 2.40 (t, J = 7.5 Hz, 4H), 2.35-2.27 (m, 5H), 1.63-1.42 (m, 16H), 1.27 (m, J = 4.5 Hz, 78H), 0.93-0.85 (m, 24H).

R. Compound 1013 was synthesized according to General Synthetic Route 2. 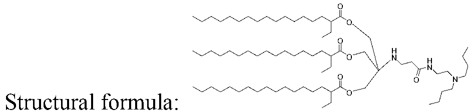
Molecular formula: C₇₄H₁₄₅N₃O₇
Molecular weight: 1188.99

It was synthesized following General Synthetic Route 2 with a procedure similar to that of Compound 1014, except that N,N-diethylethylenediamine was used as Compound 5 in General Synthetic Route 2. ¹H NMR (400 MHz, CDCl₃): δ 4.10 (d, J = 4.1 Hz, 6H), 3.26 (q, J = 5.8 Hz, 2H), 2.89 (t, J = 6.2 Hz, 2H), 2.52 (d, J = 6.6 Hz, 2H), 2.41 (t, J = 7.4 Hz, 4H), 2.33-2.25 (m, 5H), 1.60 (d, J = 11.8 Hz, 16H), 1.24 (d, J = 4.5 Hz, 82H), 0.96-0.79 (m, 24H).

S. Compound 1029 was synthesized according to General Synthetic Route 2. 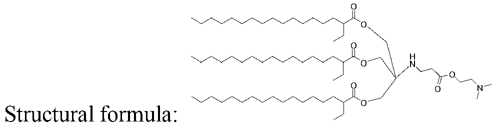
Molecular formula: C₆₈H₁₃₂N₂O₈
Molecular weight: 1105.81

It was synthesized via General Synthetic Route 2 with a procedure similar to that of Compound 1014, except that N,N-diethyl-2-hydroxyethylamine was used as Compound 5 in General Synthetic Route 2. ¹H NMR (400 MHz, CDCl₃): δ 4.16 (t, J = 5.8 Hz, 2H), 4.13-4.05 (m, 6H), 2.86 (t, J = 6.3 Hz, 2H), 2.55 (t, J = 5.8 Hz, 2H), 2.44 (t, J = 6.3 Hz, 2H), 2.35-2.20 (m, 9H), 1.60-1.39 (m, 11H), 1.24 (d, J = 4.5 Hz, 80H), 1.02-0.79 (m, 18H).

T. Compound 1111 was synthesized according to General Synthetic Route 2. 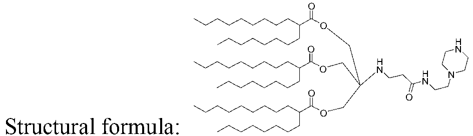
Molecular formula: C₇₀H₁₃₆N₄O₇
Molecular weight: 1145.88

It was synthesized following General Synthetic Route 2 with a procedure similar to that of Compound 1014, except that Intermediate C was used as Compound 2 and tert-butyl 4-(2-aminoethyl)piperazine-1-carboxylate as Compound 5 in General Synthetic Route 2. ¹H NMR (400 MHz, Chloroform-d): δ 4.28 (s, 6H), 3.41 (s, 2H), 3.31 (s, 4H), 3.22 (s, 3H), 2.97-2.80 (m, 5H), 2.70 (s, 5H), 2.46-2.32 (m, 4H), 1.60 (t, J = 7.1 Hz, 6H), 1.54-1.40 (m, 6H), 1.27 (s, 78H), 0.89 (t, J = 6.7 Hz, 18H).

U. Compound 1118 was synthesized according to General Synthetic Route 2. 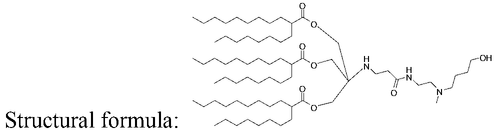
Molecular formula: C₇₁H₁₃₉N₃O₈
Molecular weight: 1162.91

It was synthesized according to General Synthetic Route 2 with a procedure similar to that of Compound 1014, except that Intermediate C was used as Compound 2 and Intermediate K as Compound 5 in General Synthetic Route 2. ¹H NMR (400 MHz, Chloroform-d): δ 4.11 (s, 6H), 3.61 (s, 2H), 3.42 (d, J = 33.5 Hz, 2H), 2.91 (t, J = 6.2 Hz, 3H), 2.36 (dd, J = 9.2, 3.6 Hz, 6H), 1.47 (dd, J = 13.9, 6.2 Hz, 14H), 1.27 (s, 71H), 0.90 (t, J = 6.7 Hz, 18H).

V. Compound 1010 was synthesized according to General Synthetic Route 3. 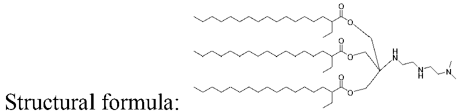
Molecular formula: C₆₇H₁₃₃N₃O₆
Molecular weight: 1076.82

### Steps 1 and 2: Synthesis of Compound 3 and Compound 4 in General Synthetic Route 3

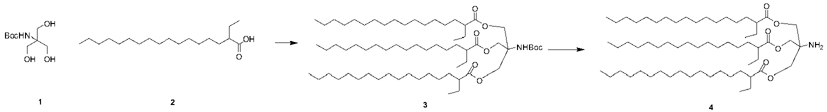

Synthesis was carried out following Steps 1 and 2 of General Synthetic Route 1, with the identical procedure as that for Compound 1003.

### Step 3: Synthesis of Compound 6 in General Synthetic Route 3

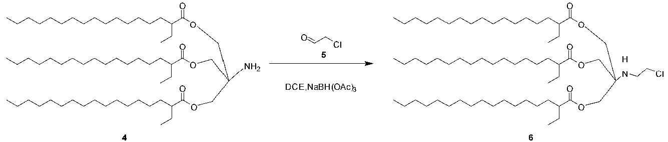

Compound 4 (600 mg), chloroacetaldehyde (Compound 5, 242 mg (40%)) and sodium triacetoxyborohydride (NaBH(OAc)₃, 394 mg) were added to 1,2-dichloroethane (DCE, 60 mL). The mixture was reacted overnight at room temperature, then water and dichloromethane were added and stirred for phase separation. The organic layer was washed with saturated brine, separated and concentrated. Purification by silica gel column chromatography (eluent: DCM/MeOH = 30:1 to 15:1) afforded Compound 6 (270 mg).

### Step 4: Synthesis of Compound 1010

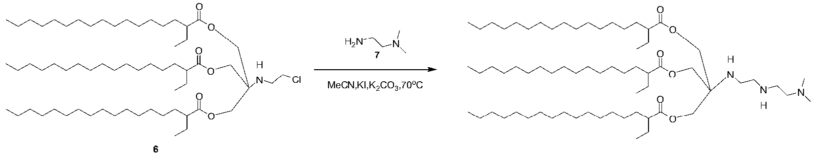

Acetonitrile (MeCN, 20 mL), N,N-dimethylethylenediamine (Compound 7, 229 mg), potassium iodide (KI, 43 mg) and potassium carbonate (K₂CO₃, 179 mg) were added to Compound 6 (270 mg). The reaction mixture was heated to 70 °C under nitrogen atmosphere and stirred overnight. The crude product was purified by silica gel column chromatography to obtain Compound 1010 (16 mg). ¹H NMR (400 MHz, CDCl₃): δ 4.10 (m, J = 2.9 Hz, 6H), 2.76-2.64 (m, 6H), 2.40 (t, J = 6.2 Hz, 2H), 2.29 (ddd, J = 8.5, 5.6, 2.9 Hz, 3H), 2.22 (s, 6H), 1.97 (s, 6H), 1.50-1.41 (m, 6H), 1.24 (d, J = 4.2 Hz, 78H), 0.87 (t, J = 7.1 Hz, 18H).

W. Compound 1059 was synthesized according to General Synthetic Route 3. 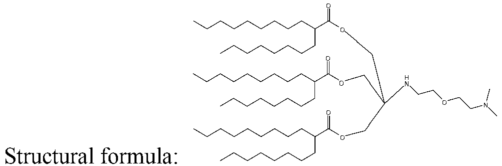
Molecular formula: C₆₇H₁₃₂N₂O₇
Molecular weight: 1077.80

It was synthesized following General Synthetic Route 3 with a procedure similar to that of Compound 1010, except that Intermediate C and Intermediate L were used as Compound 2 and Compound 5 in General Synthetic Route 3, respectively. In addition, Compound 6 obtained from General Synthetic Route 3 requires the following process steps for functional group transformation:

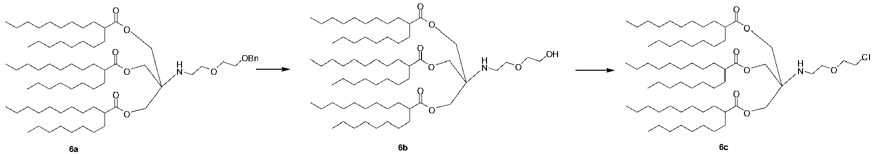

To Intermediate 6a (1.5 g) were added methanol (MeOH, 5 mL) and tetrahydrofuran (THF, 5 mL), followed by 10% Pd(OH)₂ /C (0.46 g) and 10% Pd/C (0.35 g). The mixture was stirred under hydrogen atmosphere at 25 °C overnight. The mixture was filtered filtration through celite, and the filtrate was concentrated. Purification via silica gel column chromatography (eluent: petroleum ether/ethyl acetate = 30:1 to 10:1) afforded Intermediate 6b (520 mg). To Intermediate 6b (470 mg) in dichloromethane (DCM, 5 mL), thionyl chloride (SOCl₂, 1064.26 mg, 8.946 mmol) was added dropwise at 0 °C. The reaction mixture was warmed to room temperature and stirred for 3 h, then extracted with water and DCM. The organic layer was concentrated and purified by silica gel column chromatography (eluent: petroleum ether/ethyl acetate = 20:1) to obtain Intermediate 6c (181 mg). To Intermediate 6c (170 mg) were added dimethylamine (143.39 mg), DMF (2 mL), potassium carbonate (K₂CO₃, 43.96 mg) and potassium iodide (KI, 26.39 mg). The mixture was stirred at 70 °C, then filtered and concentrated. Silica gel column chromatography (eluent: DCM/methanol = 20:1) provided Compound 1059 (91 mg, yield 53.22 %). ¹H NMR (400 MHz, Chloroform-d): δ 4.11 (s, 6H), 3.51 (dt, J = 18.3, 5.6 Hz, 4H), 2.81 (dd, J = 10.3, 5.1 Hz, 2H), 2.50 (t, J = 6.0 Hz, 2H), 2.42-2.32 (m, 3H), 2.28 (s, 6H), 2.08-1.98 (m, 1H), 1.53-1.38 (m, 7H), 1.27 (s, 77H), 0.90 (t, J = 6.7 Hz, 18H).

X. Compound 1112 was synthesized according to General Synthetic Route 3. 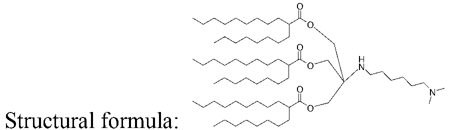
Molecular formula: C₆₉H₁₃₆N₂O₆
Molecular weight: 1089.85

It was synthesized following General Synthetic Route 3 with a procedure similar to that of Compound 1010, except that Intermediate C and 6-bromohexanal were used as Compound 2 and Compound 5 in General Synthetic Route 3, respectively. ¹H NMR (400 MHz, Chloroform-d): δ 4.11 (s, 6H), 3.11 (s, 1H), 2.57 (t, J = 6.8 Hz, 2H), 2.44-2.19 (m, 12H), 2.04 (q, J = 6.5 Hz, 2H), 1.53-1.38 (m, 13H), 1.27 (s, 92H), 0.90 (t, J = 6.7 Hz, 20H).

Y. Compound 1113 was synthesized according to General Synthetic Route 4. 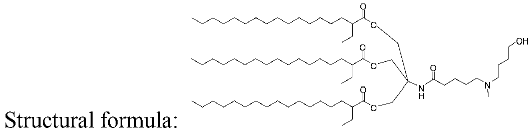
Molecular formula: C₇₁H₁₃₈N₂O₈
Molecular weight: 1147.89

### Steps 1 and 2: Synthesis of Compound 3 and Compound 4 in General Synthetic Route 4

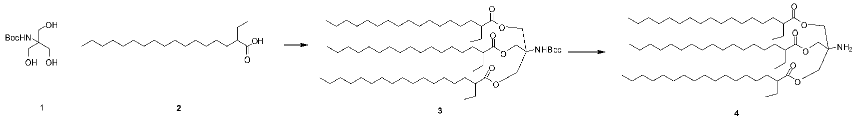

Synthesis was carried out following Steps 1 and 2 of General Synthetic Route 1, adopting the identical procedure used for Compound 1003.

### Step 3: Synthesis of Compound 6 in General Synthetic Route 4

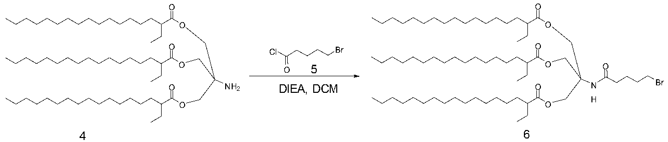

To Compound 4 were added N,N-diisopropylethylamine (DIEA, 0.30 g) and dichloromethane (DCM, 20 mL). Under nitrogen protection, the mixture was cooled to 0 °C, then 5-bromopentanoyl chloride (Compound 5, 0.27 g) was added. The reaction system was stirred at 25 °C for 2 h, diluted with 100 mL DCM, and the organic layer was washed twice with 100 mL water. The organic phase was dried over anhydrous sodium sulfate, filtered and concentrated. Purification by silica gel column chromatography (eluent: petroleum ether/ethyl acetate = 20:1) afforded Compound 6 (800 mg).

### Step 4: Synthesis of Compound 8 in General Synthetic Route 4

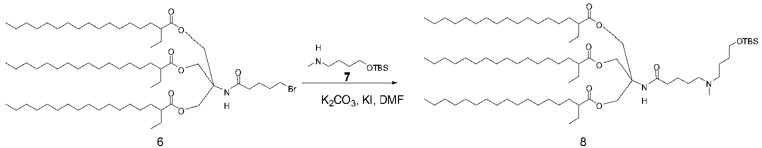

Compound 6 (750.00 mg), Intermediate K (Compound 7, 217.31 mg) and DMF (20 mL) were charged into a three-neck flask, followed by potassium carbonate (K₂CO₃, 184.16 mg) and potassium iodide (KI, 110.60 mg). The mixture was stirred at 25 °C for 16 h and then heated to 100 °C with stirring for another 3 h. The reaction mixture was diluted with 200 mL ethyl acetate (EA), and the organic layer was washed twice with 300 mL saturated brine. The organic phase was dried over anhydrous sodium sulfate, filtered and concentrated. The crude residue was loaded onto silica gel and purified by column chromatography with eluent DCM/MeOH = 20:1. The target fractions were concentrated to obtain Compound 8 (700.00 mg).

### Step 5: Synthesis of Compound 1113

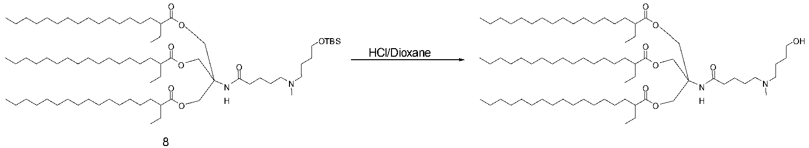

Compound 8 (700.00 mg) and tetrahydrofuran (THF, 5 mL) were added to a three-neck flask, followed by 1 M HCl solution in dioxane (5.55 mL). The reaction mixture was stirred at 25 °C for 2 h, diluted with 50 mL ethyl acetate (EA). The organic layer was washed sequentially with saturated aqueous sodium bicarbonate solution and water, then dried over anhydrous sodium sulfate, filtered and concentrated. Purification via silica gel column chromatography with eluent DCM/MeOH = 20:1 provided Compound 1113 (201.1 mg, yield 31.59 %). ¹H NMR (400 MHz, CDCl₃): δ 4.50-4.39 (m, 6H), 3.59 (t, J = 4.6 Hz, 2H), 2.49 (s, 3H), 2.35-2.26 (m, 6H), 2.14 (t, J = 7.5 Hz, 2H), 1.75-1.69 (m, 4H), 1.66-1.43 (m, 17H), 1.27 (d, J = 3.7 Hz, 78H), 0.94-0.87 (m, 18H).

Z. Compound 1115 was synthesized according to General Synthetic Route 4. 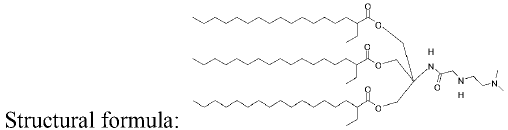
Molecular formula: C₆₇H₁₃₁N₃O₇
Molecular weight: 1090.80

It was synthesized following General Synthetic Route 4 with a procedure similar to that of Compound 1010, except that 2-bromoacetyl chloride and N,N-dimethylethylenediamine were used as Compound 5 and Compound 7 in General Synthetic Route 4, respectively. ¹H NMR (400 MHz, CDCl₃): δ 7.42 (s, 1H), 4.48 (tt, J = 11.9, 5.7 Hz, 6H), 3.27 (s, 2H), 3.13 (s, 2H), 3.01 (s, 2H), 2.83 (s, 6H), 2.38-2.25 (m, 3H), 1.55 (dtt, J = 36.3, 14.1, 7.3 Hz, 12H), 1.27 (d, J = 3.3 Hz, 78H), 0.90 (td, J = 7.2, 3.4 Hz, 18H).

AA. Compound 1114 was synthesized according to General Synthetic Route 5. 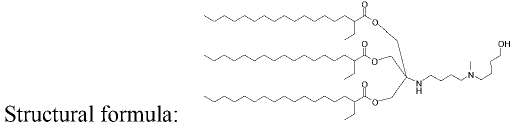
Molecular formula: C₇₀H₁₃₈N₂O₇
Molecular weight: 1119.88

### Steps 1 and 2: Synthesis of Compound 3 and Compound 4 in General Synthetic Route 5

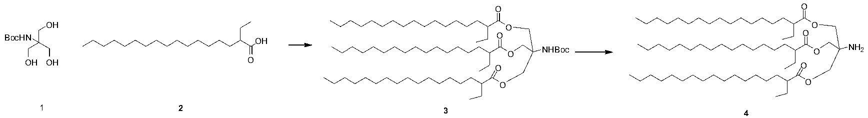

Synthesis was carried out following Steps 1 and 2 of General Synthetic Route 1 with the identical operation procedure for Compound 1003.

### Step 3: Synthesis of Compound 6 in General Synthetic Route 5

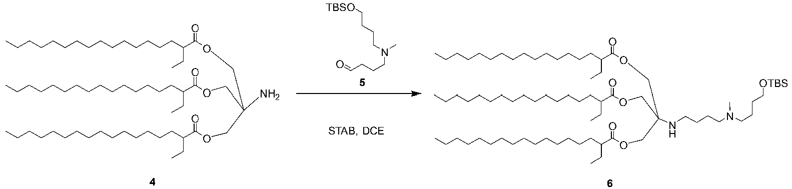

Compound 4 (500 mg), Intermediate M (Compound 5, 298.68 mg) and sodium triacetoxyborohydride (STAB, 328.68 mg) were charged into a three-neck flask, followed by 1,2-dichloroethane (DCE, 10 mL). The mixture was stirred at 25 °C under nitrogen atmosphere for 16 h. Afterwards, 100 mL water was added to the reaction mixture, which was extracted twice with ethyl acetate (EA, 100 mL). The combined organic layers were concentrated, loaded onto silica gel and purified by column chromatography using eluent DCM/MeOH = 1:20. The collected target fractions were concentrated to afford Compound 6 (500 mg).

### Step 4: Synthesis of Compound 1114

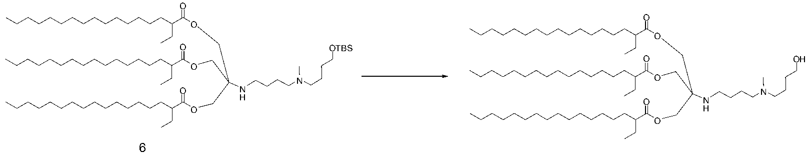

Compound 6 (450 mg) and tetrahydrofuran (THF, 5 mL) were added into a three-neck flask, then 1 M HCl solution in dioxane (3.65 mL) was added. The mixture was stirred at 25 °C for 2 h, diluted with 50 mL ethyl acetate (EA). The organic phase was washed successively with 50 mL saturated aqueous sodium bicarbonate solution and water, dried over anhydrous sodium sulfate, filtered and concentrated. The crude residue was purified by silica gel column chromatography with eluent DCM/MeOH = 20:1 to yield Compound 1114 (107 mg, 24.84 %). ¹H NMR (400 MHz, CDCl₃): δ 4.16-4.07 (m, 6H), 3.60 (t, J = 4.8 Hz, 2H), 2.62 (t, J = 7.0 Hz, 2H), 2.49 (s, 3H), 2.31 (tt, J = 8.4, 5.5 Hz, 6H), 1.70 (d, J = 4.6 Hz, 3H), 1.64-1.39 (m, 18H), 1.27 (d, J = 4.0 Hz, 78H), 0.90 (t, J = 7.1 Hz, 18H).

Z. Compound 1120 was synthesized according to General Synthetic Route 5. 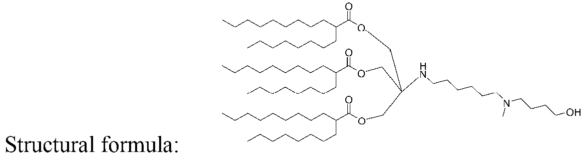
Molecular formula: C₇₂H₁₄₂N₂O₇
Molecular weight: 1147.90

It was synthesized following General Synthetic Route 5 with a procedure similar to that of Compound 1114, except that Intermediate C and 6-bromohexanal were used as Compound 2 and Compound 5 in General Synthetic Route 5, respectively. ¹H NMR (400 MHz, Chloroform-d): δ 4.11 (s, 6H), 3.63 (s, 2H), 2.57 (t, J = 6.8 Hz, 4H), 2.37 (ddd, J = 8.5, 7.1, 4.3 Hz, 5H), 1.67-1.41 (m, 24H), 1.27 (s, 79H), 0.90 (t, J = 6.7 Hz, 18H).

### (2) Preparation Example 2 Synthesis of the Second Ionizable Lipid

### 1. Preparation of Compound 6001

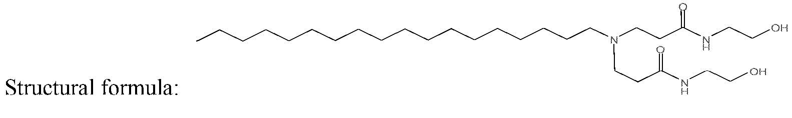

Molecular weight: 499.43

0.8 g oleylamine and 0.8 g N-(2-hydroxyethyl)acrylamide were added to 6.4 mL ethanol. The mixture was heated to 70 °C under stirring. After 3.5 h, an additional 0.8 g N-(2-hydroxyethyl)acrylamide was added, and the reaction was allowed to proceed for another 16 h. Purification via reversed-phase column chromatography afforded Compound 6002.

0.7 g Compound 6002, 0.4 g palladium on carbon and 28 mL tetrahydrofuran were charged into a reaction flask with stirring. The atmosphere was replaced with hydrogen and maintained under a hydrogen balloon for 16 h. The mixture was filtered, and the filtrate was purified by normal-phase column chromatography (eluent: MeOH/DCM) to obtain 200 mg Compound 6001. ¹H NMR (400 MHz, CDCl₃): δ 7.54 (t, J = 5.5 Hz, 2H), 3.79-3.68 (m, 4H), 3.42 (dd, J = 10.0, 5.4 Hz, 4H), 2.89 (t, J = 6.0 Hz, 4H), 2.55 (dt, J = 11.8, 6.8 Hz, 6H), 1.54 (s, 2H), 1.27 (d, J = 11.2 Hz, 32H), 0.91 (t, J = 6.8 Hz, 3H).

### 2. Preparation of Compound 6002

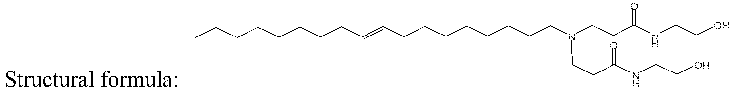

Molecular weight: 497.42

0.8 g oleylamine and 0.8 g N-(2-hydroxyethyl)acrylamide were added to 6.4 mL ethanol, and the mixture was heated to 70 °C with stirring. After 3.5 h, an extra 0.8 g N-(2-hydroxyethyl)acrylamide was added, and the reaction was further carried out for 16 h. The organic solvent was concentrated, the residue was purified by reversed-phase column chromatography and lyophilized to afford 400 mg of Compound 6002. ¹H NMR (400 MHz, CDCl₃): δ 7.31 (s, 2H), 5.38 (dd, J = 13.0, 7.4 Hz, 2H), 3.79-3.66 (m, 4H), 3.41 (dd, J = 10.1, 5.4 Hz, 4H), 2.82-2.72 (m, 4H), 2.44 (dd, J = 15.1, 9.2 Hz, 6H), 2.10-1.99 (m, 4H), 1.46 (s, 2H), 1.29 (s, 24H), 0.90 (t, J = 6.8 Hz, 3H).

### 3. Preparation of Compound 6003

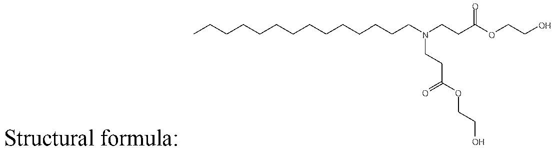

Molecular weight: 445.34

4.00 g tetradecylamine, 6.53 g 2-hydroxyethyl acrylate and 80 mL tert-butanol were added to a reaction flask. The mixture was heated to 70 °C with stirring. After 29 h, the organic solvent was concentrated, the crude product was purified via reversed-phase column chromatography and lyophilized to obtain 1.5 g Compound 6003. ¹H NMR (400 MHz, CDCl₃): δ 4.29 (t, J = 7.5 Hz, 4H), 3.89-3.75 (m, 4H), 2.81 (t, J = 6.2 Hz, 4H), 2.50 (ddd, J = 23.2, 14.1, 7.1 Hz, 6H), 1.46 (s, 2H), 1.28 (s, 24H), 0.91 (t, J = 6.3 Hz, 3H).

### 4. Preparation of Compound 6004

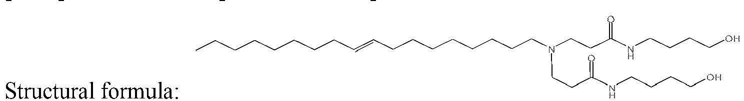

Molecular weight: 553.48

0.8 g oleylamine, 1.5 g n-butyl acrylate and 5.6 mL n-butanol were added to a reaction flask, heated to 100 °C under stirring. After 4 h, an additional 1 mL n-butyl acrylate was added, and the reaction proceeded for another 1 h. The mixture was purified by normal-phase column chromatography with eluent petroleum ether/ethyl acetate. To the obtained intermediate were added 0.4 g sodium hydroxide, 1 mL water and 10 mL methanol, and the mixture was stirred for 30 min for hydrolysis. Then 1 mL concentrated hydrochloric acid and 10 mL methanol were added to neutralize the pH, followed by rotary evaporation to remove solvents. The residue was dissolved in DCM, dried over anhydrous magnesium sulfate, filtered and concentrated. 10 mL DCM, 1.06 g 4-amino-1-butanol, 0.81 g HOBT and 2.3 g EDCI were added to the above residue, and the reaction mixture was stirred at room temperature for 18 h. After concentration of organic solvent, the crude product was purified by reversed-phase column chromatography and lyophilized to afford 240 mg Compound 6004. ¹H NMR (400 MHz, CDCl₃): δ 7.58 (d, J = 16.6 Hz, 2H), 5.44-5.31 (m, 2H), 3.72 (d, J = 16.5 Hz, 4H), 3.31 (d, J = 5.6 Hz, 4H), 3.17 (s, 4H), 2.84 (s, 2H), 2.69 (s, 4H), 2.09-2.01 (m, 4H), 1.67 (d, J = 2.7 Hz, 10H), 1.37-1.26 (m, 24H), 0.91 (t, J = 6.8 Hz, 3H).

### 5. Preparation of Compound 6005

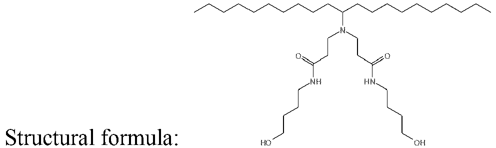

Molecular weight:597.54

25.00 g 11-heneicosanone, 62.05 g ammonium acetate and 500 mL methanol were charged into a reaction flask under stirring, followed by addition of 6.55 g sodium cyanoborohydride. After 16 h of reaction, 250 mL water and 250 mL DCM were added for liquid separation. The aqueous layer was extracted with 50 mL DCM, and all organic phases were combined. Purification via normal-phase column chromatography (eluent: MeOH/DCM) afforded 21.00 g Compound 6005-A.

21.00 g Compound 6005-A, 58 mL n-butyl acrylate and 100 mL n-butanol were added to a reaction flask and heated to 100 °C with stirring. An additional 10 mL n-butyl acrylate was added, and the mixture reacted for 16 h. Column chromatography (eluent: petroleum ether/ethyl acetate) provided 31.00 g Compound 6005-B.

A solution consisting of 31.00 g Compound 6005-B, 6.55 g sodium hydroxide, 310 mL methanol and 31 mL water was added to a reaction flask and stirred for 20 min, then 150 mL THF was added and the mixture was heated to 50 °C. After stirring for 30 min, a premixed solution of 16.13 g concentrated hydrochloric acid and 160 mL methanol was added, and stirring continued for another 30 min. All solvents were removed by rotary evaporation, and the residue was dissolved in 300 mL DCM, dried over anhydrous magnesium sulfate, filtered and concentrated to yield 27.00 g oily Compound 6005-C.

27.00 g Compound 6005-C, 25.00 g 4-amino-1-butanol, 41.85 g EDCI, 14.74 g HOBT and 310 mL DCM were added to a reaction flask and stirred for 18 h. The organic solvent was concentrated, and the crude product was purified by column chromatography (eluent: MeOH/DCM) to remove excess 4-amino-1-butanol, giving 6.80 g Compound 6005. ¹H NMR (400 MHz, CDCl₃): δ 3.70 (t, J = 5.6 Hz, 4H), 3.28 (t, J = 5.6 Hz, 4H), 2.74 (t, J = 6.0 Hz, 4H), 2.40-2.45 (m, 1H), 2.34 (t, J = 6.4 Hz, 4H), 1.63-1.65 (m, 8H), 1.25-1.35 (m, 36H), 0.91 (t, J = 6.4 Hz, 6H).

### 6. Preparation of Compound 6006

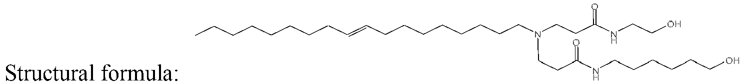

Molecular weight: 553.48

1.00 g oleylamine, 0.47 g N-(2-hydroxyethyl)acrylamide and 8 mL ethanol were added into a reaction flask, heated to 70 °C under stirring. After 13 h, the solvent was removed by rotary evaporation, and the residue was purified by column chromatography (eluent: MeOH/DCM) to afford 800 mg Intermediate 1. 1.5 mL n-butyl acrylate and 5 mL n-butanol were added, and the mixture was heated to 100 °C with stirring. After 3 h, the solvent was evaporated and the crude product was subjected to column chromatography (MeOH/DCM system). A solution of 0.4 g sodium hydroxide, 10 mL methanol and 1 mL water was added and stirred for 1 h, followed by a premixed solution of 1 mL concentrated hydrochloric acid and 8 mL methanol. The mixture was stirred for another 30 min, then all solvents were evaporated. The residue was dissolved in 100 mL DCM, dried over anhydrous magnesium sulfate, filtered and concentrated. 0.45 g 6-amino-1-hexanol, 0.27 g HOBT, 0.78 g EDCI and 10 mL DCM were added and the reaction mixture was stirred for 14 h. After concentration of organic solvent, column chromatography (MeOH/DCM eluent) was performed to yield 300 mg Compound 6006. ¹H NMR (400 MHz, CDCl₃): δ 7.83 (s, 1H), 6.95 (s, 1H), 5.43-5.28 (m, 2H), 3.74-3.67 (m, 2H), 3.64 (t, J = 6.3 Hz, 2H), 3.39 (dd, J = 10.0, 5.3 Hz, 2H), 3.24 (dd, J = 12.9, 6.7 Hz, 2H), 2.74 (t, J = 5.8 Hz, 4H), 2.48-2.42 (m, 2H), 2.41-2.33 (m, 4H), 2.01 (dd, J = 13.6, 6.8 Hz, 4H), 1.66-1.17 (m, 34H), 0.89 (t, J = 6.7 Hz, 3H).

### 7. Compound 6007

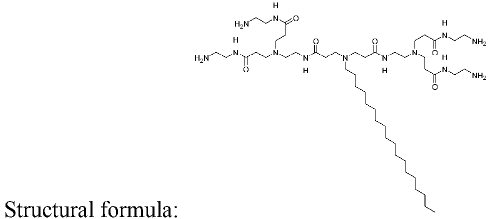

Molecular weight: 953.78

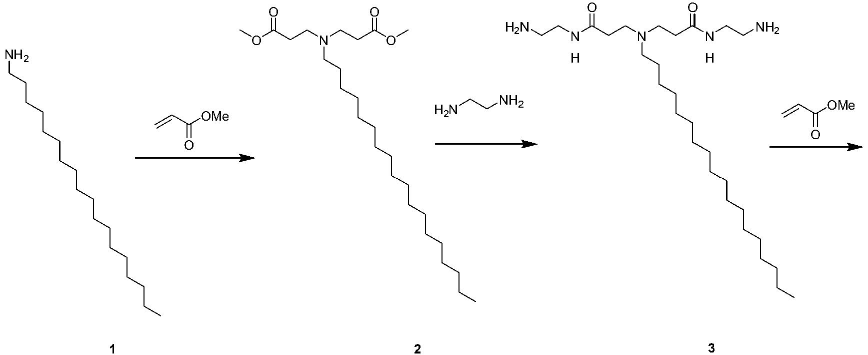

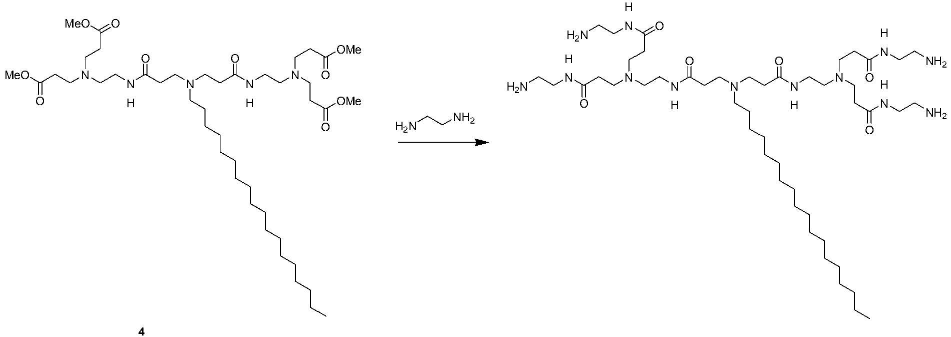

At room temperature, octadecylamine (50 g) and methanol (200 mL) were charged into a three-neck flask and stirred. The mixture was cooled to 5 °C with an ice-water bath, then methyl acrylate (31.5 g) was added dropwise slowly under stirring. The mixture was allowed to warm to room temperature and reacted for 4 h. The solvent was removed under reduced pressure, and the residue was purified by column chromatography with eluent PE/EA = 20:1 to 10:1 to obtain Product 2 (68 g). Product 2 (15.0 g), methanol (100 mL) and ethylenediamine (1.0 g) were added to a single-neck flask at room temperature. The mixture was heated to 60 °C and stirred overnight. After concentration under reduced pressure, 30 mL toluene was added and heated to 60 °C to fully dissolve the crude product, followed by slow cooling to room temperature. All solvents were evaporated under vacuum to afford Compound 3 (15 g). Compound 3 (2 g) and methanol (20 mL) were added to a single-neck flask at room temperature. Methyl acrylate (2.5 g) was added slowly with stirring, and the mixture was heated to 60 °C and reacted overnight. The reaction mixture was concentrated under reduced pressure and purified by column chromatography (eluent DCM/MeOH = 50:1 to 20:1) to obtain Compound 4 (2.5 g). Compound 4 (2.3 g) and ethylenediamine (20 mL) were stirred in a single-neck flask at room temperature overnight. The reaction was stopped, and the mixture was directly concentrated under reduced pressure to yield Compound 6007 (24 mg). ¹H NMR (400 MHz, Methanol-d₄ ): δ 3.87 (t, J = 6.6 Hz, 2H), 3.55-3.36 (m, 8H), 3.33 (s, 8H), 3.27-3.16 (m, 2H), 3.06-2.90 (m, 4H), 2.77 (t, J = 6.5 Hz, 4H), 1.90 (dtdd, J = 21.2, 14.0, 9.5, 6.9 Hz, 4H), 1.73 (p, J = 7.7 Hz, 4H), 1.49 (tdd, J = 9.2, 7.9, 7.1, 4.0 Hz, 4H), 1.31 (s, 28H), 0.92 (t, J = 6.8 Hz, 3H).

### 8. Compounds 6008-6018

### Synthesis of Compound 6009

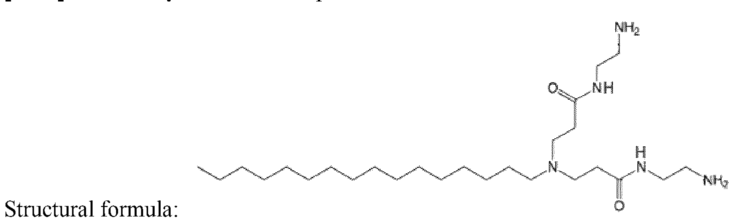

A methanol (20 mL) solution of freshly recrystallized hexadecylamine (0.03 mol) was added dropwise under nitrogen atmosphere to a stirred solution of methyl methacrylate (6 mL) in methanol (20 mL) at room temperature, and the mixture was reacted overnight. The solvent was removed by rotary evaporation at room temperature, and the residue was dissolved in chloroform, which was washed twice with 0.1 M aqueous NaOH solution. The chloroform layer was collected and dried over anhydrous calcium chloride, followed by purification via column chromatography to afford a colorless oil. ¹H NMR (300 MHz, CDCl₃): δ 0.78 (t, 3H), 1.16 (s, 30H), 2.38 (m, 6H), 2.71 (t, 4H), 3.57 (m, 6H).

A methanol (20 mL) solution of the above colorless oil (11.05 g) was added to a vigorously stirred solution of 1,2-ethanediamine (75 g) in methanol (100 mL) at room temperature. After complete addition, the mixture was stirred at room temperature for an additional 24 h. The solvent was removed under reduced pressure with the temperature controlled below 40 °C. Excess 1,2-ethanediamine was eliminated by azeotropic distillation with toluene/methanol (9:1), and residual toluene was further stripped via azeotropy with methanol to obtain a white powder (10.5 g). The crude solid was recrystallized repeatedly from chloroform and cyclohexane to yield a white solid target product. ¹H NMR (300 MHz, CDCl₃): δ 0.88 (t, 3H), 1.25 (s, 30H), 1.84 (s, 4H), 2.38 (m, 6H), 2.73 (m, 4H), 2.82 (m, 4H), 3.29 (m, 4H), 7.47 (s, 2H).

The synthetic procedure for Compound 6008 differs from that of 6009 only in that an equimolar amount of tetradecylamine was used to replace hexadecylamine. Compounds 6010-6017 were prepared following the synthetic route of Compound 6009, where hexadecylamine was substituted with equimolar corresponding primary alkyl amines Rₐ-NH₂ respectively.

Compound 6018 was synthesized in accordance with the preparation method of Compound 6007, by replacing octadecylamine with an equimolar amount of the corresponding alkyl amine Rₐ-NH₂.

### 9. Compound 6019

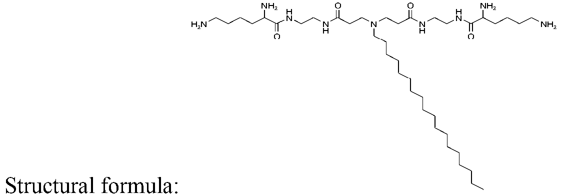

Molecular weight: 753.66

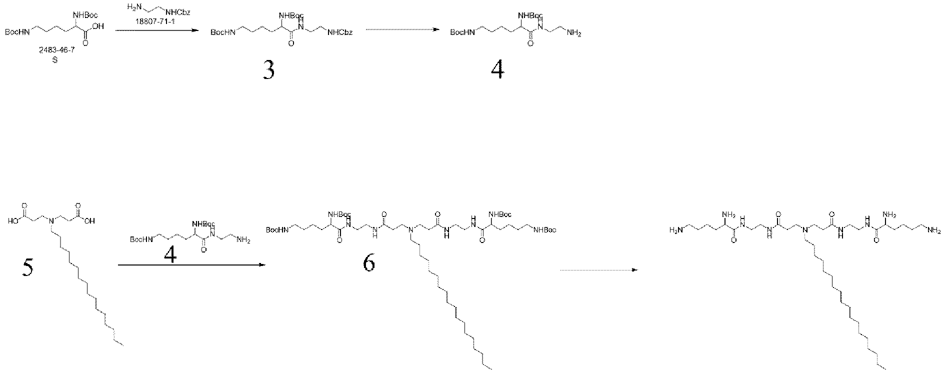

At room temperature, Compound CAS 2483-46-7 (3 g) and dichloromethane (DCM, 50 mL) were charged into a single-neck flask and stirred. Then CAS 18807-71-1 (2.5 g), dicyclohexylcarbodiimide (DCC, 2.7 g) and 4-dimethylaminopyridine (DMAP, 1.6 g) were added, and the mixture was stirred and reacted overnight at room temperature. The solvent was removed under reduced pressure, and the residue was purified by column chromatography with eluent DCM/MeOH = 5:1 to afford Product 3 (4 g). Product 3 (5.0 g), methanol (MeOH, 50 mL) and palladium on carbon (Pd/C, 1.0 g) were added to a three-neck flask at room temperature. The atmosphere was replaced with hydrogen, and the mixture was heated to 50 °C with stirring for 1 h. The reaction was stopped, the system cooled to room temperature and filtered. The filtrate was concentrated under vacuum to obtain Product 4 (3.5 g). Starting Material 5 (1.2 g) and N,N-dimethylformamide (DMF, 50 mL) were added to a single-neck flask at room temperature. Product 4 (3.3 g), O-(7-azabenzotriazol-1-yl)-N,N,N',N'-tetramethyluronium hexafluorophosphate (HATU, 3.2 g) and triethylamine (TEA, 0.9 g) were added, and the reaction mixture was stirred overnight at room temperature. Afterwards, 500 mL water and 500 mL ethyl acetate (EA) were added for liquid-liquid extraction. The organic layer was dried over anhydrous sodium sulfate and concentrated under reduced pressure. The crude product was purified by column chromatography (eluent DCM/MeOH = 20:1 to 10:1) to yield solid Compound 6 (380 mg). Solid Compound 6 (380 mg) and 4 M HCl in dioxane (1.6 mL) were mixed in a single-neck flask and reacted at room temperature for 2 h. The solvent was evaporated under reduced pressure, followed by purification to obtain Compound 6019 (220 mg). ¹H NMR (400 MHz, Methanol-d₄): δ 3.87 (t, J = 6.6 Hz, 2H), 3.55-3.36 (m, 8H), 3.33 (s, 8H), 3.27-3.16 (m, 2H), 3.06-2.90 (m, 4H), 2.77 (t, J = 6.5 Hz, 4H), 1.90 (dtdd, J = 21.2, 14.0, 9.5, 6.9 Hz, 4H), 1.73 (p, J = 7.7 Hz, 4H), 1.49 (tdd, J = 9.2, 7.9, 7.1, 4.0 Hz, 4H), 1.31 (s, 28H), 0.92 (t, J = 6.8 Hz, 3H).

### 10. Compound 6020

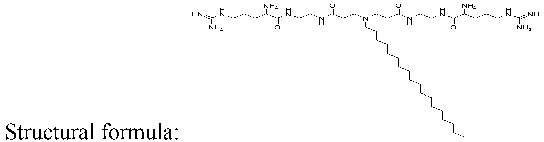

Molecular weight: 810.19

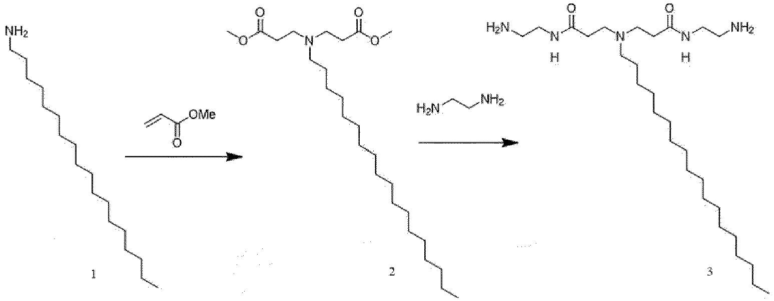

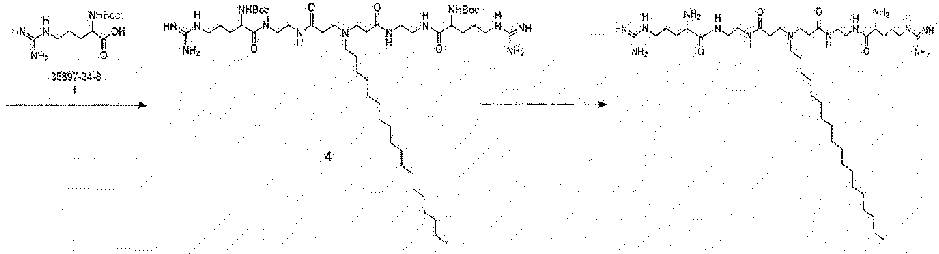

At room temperature, octadecylamine (50 g) and methanol (200 mL) were added to a three-neck flask and stirred. The mixture was cooled to 5 °C with an ice-water bath, then methyl acrylate (31.5 g) was added dropwise with stirring. The mixturewas allowed to warm to room temperature and reacted for 4 h. The reaction was stopped, the solvent was directly removed under reduced pressure, and the residue was purified by column chromatography with eluent PE/EA = 20:1 to 10:1 to obtain Product 2 (68 g). Product 2 (15.0 g), methanol (100 mL) and ethylenediamine (1.0 g) were charged into a single-neck flask at room temperature, heated to 60 °C and stirred overnight. The reaction was stopped, the mixture was concentrated under reduced pressure, 30 mL toluene was added and heated to 60 °C to dissolve the crude product, then slowly cooled to room temperature and filtered. The filtrate was evaporated under vacuum to afford Compound 3 (15 g). Compound 3 (1.3 g) and DMF (20 mL) were added to a single-neck flask at room temperature, followed by addition of CAS 35897-34-8 (3.6 g), EDCI (3.0 g), HOBt (2.1 g) and DIEA (2.0 g). The mixture was stirred overnight at room temperature. 200 mL water was added, and the mixture was directly lyophilized under reduced pressure to obtain Compound 4 (4 g). Compound 4 (100 mg) and 4 M HCl in dioxane (2 mL) were added to a single-neck flask and reacted at room temperature for 1 h. The reaction mixture was concentrated and purified under reduced pressure to yield Compound 6020. ¹H NMR (400 MHz, Methanol-d₄ ): δ 3.90 (t, J = 6.4 Hz, 2H), 3.57-3.34 (m, 10H), 3.24 (dt, J = 16.1, 8.0 Hz, 8H), 2.77 (t, J = 6.5 Hz, 4H), 2.03-1.58 (m, 10H), 1.31 (s, 30H), 1.01-0.82 (m, 3H).

### 11. Compound 6021

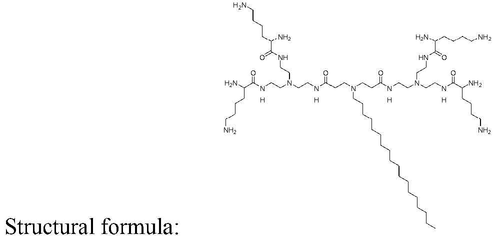

Molecular weight: 1182.79

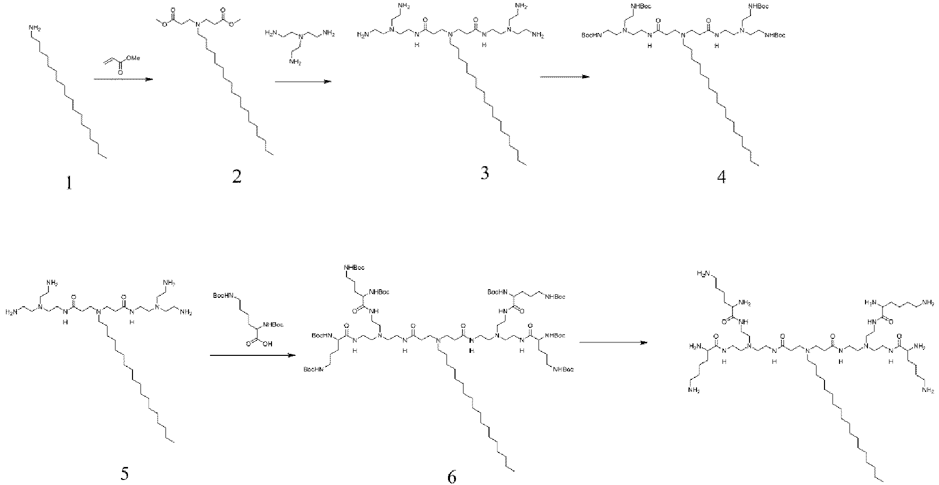

At room temperature, Product 2 (3.5 g, synthesized via the same procedure as Product 2 described for Compound 6020) and methanol (20 mL) were charged into a single-neck flask and stirred. Tris(2-aminoethyl)amine (TREN, 23.0 g) was added, and the mixture was heated to 60 °C with stirring overnight. The solvent was removed under reduced pressure, and the crude residue was lyophilized to afford Product 3 (25 g). Product 3 (22.0 g) and dichloromethane (DCM, 200 mL) were added to a single-neck flask and cooled to 0 °C. Di-tert-butyl dicarbonate ((Boc)₂O, 71.7 g) was added dropwise slowly. The mixturewas allowed to warm to room temperature and stirred for 3 h. The reaction was quenched by adding 200 mL water, followed by further extraction with 100 mL DCM. The organic layer was dried over anhydrous sodium sulfate, concentrated under reduced pressure, and purified by column chromatography to yield yellow oily Product 4 (5.8 g). Yellow oily Product 4 (1.4 g) was placed in a single-neck flask, and 4 M HCl in dioxane (15 mL) was slowly added under an ice-water bath with stirring. Upon completion of addition, the mixture was warmed to room temperature and reacted for 0.5 h, then concentrated under vacuum to obtain Compound 5 (800 mg). (S)-2,6-di(tert-butoxycarbonylamino)hexanoic acid (2.6 g), EDCI (1.4 g), HOBt (1.0 g), DIEA (1.0 g) and DMF (10 mL) were added to Compound 5 (1.0 g) in a single-neck flask and stirred overnight at room temperature. 100 mL water and 100 mL ethyl acetate (EA) were added for liquid-liquid extraction. The EA layer was washed once with 100 mL saturated brine, concentrated under reduced pressure, and purified by column chromatography with eluent DCM/MeOH = 10:1 to obtain Compound 6 (1.1 g). Compound 6 (1.2 g), 4 M HCl in dioxane (10 mL) and DCM (10 mL) were charged into a single-neck flask and reacted overnight at room temperature. The mixture was concentrated under reduced pressure and purified to furnish Compound 6021 (220 mg). ¹H NMR (400 MHz, Methanol-d₄ ): δ 3.96 (t, J = 6.6 Hz, 4H), 3.75 (dt, J = 14.0, 6.6 Hz, 4H), 3.62-3.45 (m, 12H), 3.36-3.33 (m, 14H), 3.25-3.17 (m, 2H), 3.04-2.90 (m, 8H), 2.83 (t, J = 6.9 Hz, 4H), 2.05-1.82 (m, 8H), 1.73 (p, J = 7.7 Hz, 10H), 1.53 (qd, J = 8.3, 7.7, 4.3 Hz, 8H), 1.30 (s, 28H), 0.97-0.87 (m, 3H).

### 12. Compound 6023

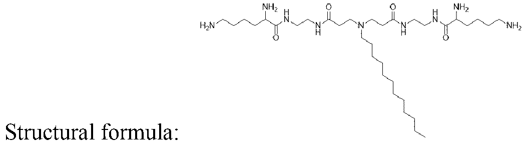

Molecular weight: 670

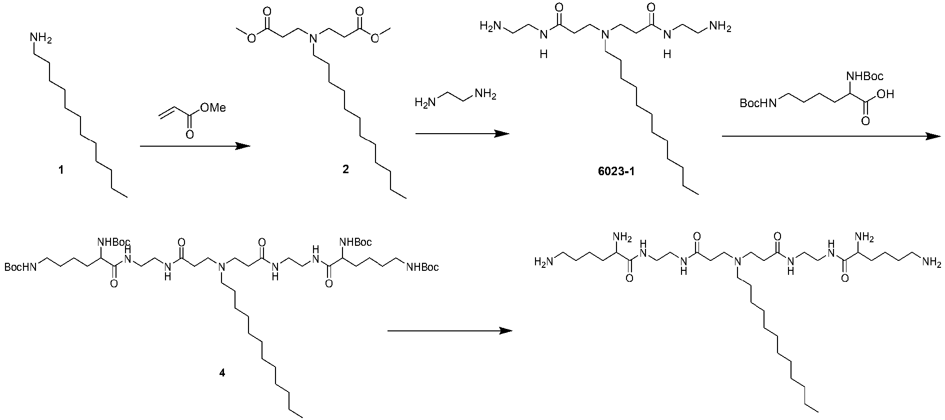

At room temperature, dodecylamine (10 g) and methanol (100 mL) were added to a three-neck flask and stirred. The system was cooled to 5 °C with an ice-water bath, then methyl acrylate (10.2 g) was added dropwise slowly under stirring. The mixture was allowed to warm to room temperature and reacted for 4 h. The reaction was stopped, the solvent was removed under reduced pressure, and the residue was purified by column chromatography with eluent PE/EA = 20:1 to 10:1 to obtain Product 2 (19 g). Product 2 (19.0 g), methanol (200 mL) and ethylenediamine (127.7 g) were charged into a single-neck flask at room temperature, heated to 60 °C and stirred overnight. The mixture was concentrated under reduced pressure to afford Compound 3 (20 g). A 500 mg portion of Compound 3 was further purified and lyophilized to yield Intermediate 6023-1 (218 mg). Intermediate 6023-1 (1.4 g) and DCM (20 mL) were added to a single-neck flask at room temperature, then (S)-2,6-di(tert-butoxycarbonylamino)hexanoic acid (3.4 g), EDCI (1.9 g) and DMAP (1.2 g) were added, and the mixture was stirred overnight at room temperature. Liquid-liquid extraction was performed, the organic layer was dried and concentrated under vacuum to obtain Compound 4 (3.0 g). Compound 4 (2.8 g) and 4 M HCl in 1,4-dioxane (20 mL) were mixed in a single-neck flask and reacted at room temperature for 2 h. The mixture was concentrated under reduced pressure, purified and lyophilized to furnish Compound 6023 (123 mg). ¹H NMR (400 MHz, Methanol-d₄): δ 3.88 (t, J = 6.6 Hz, 2H), 3.55-3.36 (m, 8H), 3.36-3.33 (m, 2H), 3.31-3.14 (m, 4H), 3.04-2.93 (m, 4H), 2.77 (t, J = 6.5 Hz, 4H), 2.00-1.82 (m, 4H), 1.82-1.66 (m, 6H), 1.50 (qd, J = 8.2, 7.8, 4.2 Hz, 4H), 1.45-1.25 (m, 18H), 0.96-0.87 (m, 3H).

### 13. Compound 6024

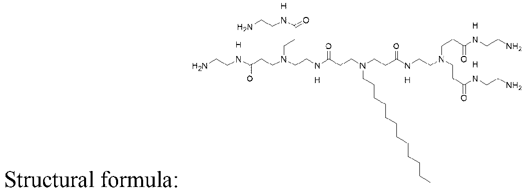

Molecular weight: 870.24

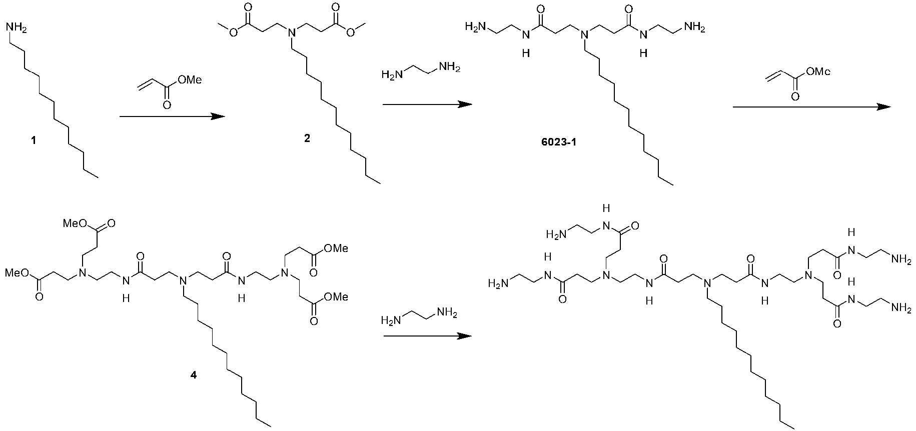

At room temperature, Intermediate 6023-1 (1.4 g, prepared following the synthetic procedure for 6023-1 described in Compound 6023) and methanol (20 mL) were added to a single-neck flask. Methyl acrylate (2.8 g) was added slowly under stirring, and the mixture was heated to 60 °C and reacted overnight at this temperature. The solvent was removed under reduced pressure to obtain Compound 4 (2.0 g). Compound 4 (2.4 g) and ethylenediamine (20 mL) were added to a single-neck flask and stirred overnight at room temperature. The mixture was concentrated under reduced pressure and purified to yield Compound 6024 (191 mg). ¹H NMR (400 MHz, Methanol-d₄): δ 3.69 (t, J = 6.0 Hz, 4H), 3.60-3.48 (m, 20H), 3.42 (t, J = 6.1 Hz, 4H), 3.25-3.19 (m, 2H), 3.12 (t, J = 5.8 Hz, 8H), 2.83 (t, J = 6.5 Hz, 12H), 1.78 (tt, J = 11.0, 6.4 Hz, 2H), 1.51-1.18 (m, 20H), 0.91 (t, J = 6.7 Hz, 3H).

### 14. Compound 6026

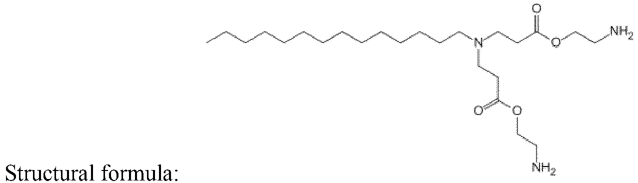

Molecular weight: 443.67

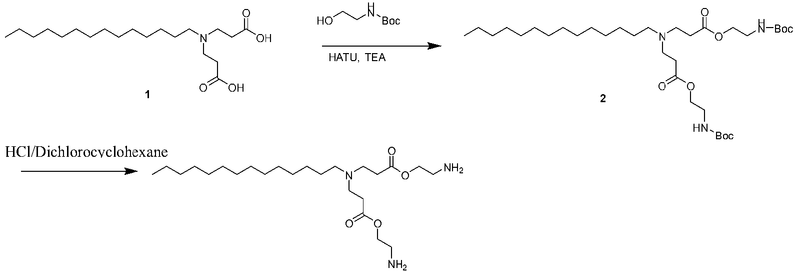

Compound 6026 was synthesized referring to the synthetic route of Compound 6019, except that Raw Material 5 used in the synthesis of 6019 was replaced with an equimolar amount of Compound 1, and Intermediate 4 of 6019 was substituted by an equimolar quantity of N-(tert-butoxycarbonyl)ethanolamine. ¹H NMR (300 MHz, DMSO): δ 4.51 (t, J = 7.3 Hz, 4H), 3.76 (t, J = 6.1 Hz, 4H), 3.18 (t, J = 6.7 Hz, 4H), 3.01 (t, J = 5.8 Hz, 2H), 2.49 (t, J = 7.1 Hz, 4H), 1.36-1.26 (m, 24H), 0.89 (t, J = 6.2 Hz, 3H).

Compounds 6025-6034 were prepared by referring to the aforementioned synthetic procedures of previous compounds. The modification lies in that ethanolamine was used to replace the corresponding ethylenediamine. The amino group of ethanolamine was first protected with Fmoc, Boc or other protecting groups, and the protecting groups were removed by standard deprotection protocols after the completion of all reactions.

### (3) Exemplary Compounds of the First Ionizable Lipid and Their Properties

The exemplary compounds of the present application and their properties are summarized in Table 1, all of which were synthesized according to the synthetic routes described in Preparation Example 1.

The calculated c-pKa (molnetwork) and LogP (cLogP driver) values of the exemplary compounds were generated by the ChemDraw module of Chemoffice.

**Table 1: Properties of Exemplary Compounds of the First Ionizable Lipid**

| **No.** | **Molecular Formula** | **Molecular Weight** | **c-pKa** | **cLog P** | **No.** | **Molecular Formula** | **Molecular Weight** | **c-pKa** | **cLog P** |
|---|---|---|---|---|---|---|---|---|---|
| 1001 | C₅₉H₁₀₈N₂O₇ | 957.52 | 9.83 | 21.43 | 1201 | C₇₁H₁₄₈N₆O | 1102.01 | 9.01 | 28.48 |
| 1002 | C₆₀H₁₁₆N₂O₇ | 977.6 | 6.97 | 24.49 | 1202 | C₉₂H₁₉₀N₆O | 1396.57 | 9.01 | 39.59 |
| 1003 | C₆₃H₁₂₂N₂O₇ | 1019.68 | 6.97 | 26.07 | 1203 | C₆₅H₁₃₆N₆O | 1017.84 | 9.01 | 25.31 |
| 1004 | C₅₄H₁₀₄N₂O₇ | 893.43 | 6.97 | 21.97 | 1204 | C₆₅H₁₃₆N₆O | 1017.84 | 9.01 | 25.31 |
| 1005 | C₆₈H₁₃₃N₃O₇ | 1104.83 | 9.01 | 26.86 | 1205 | C₉₈H₂₀₂N₆O | 1480.73 | 9.01 | 43.03 |
| 1006 | C₅₃H₁₀₃N₃O₇ | 894.42 | 9.01 | 19.58 | 1206 | C₉₂H₁₉₀N₆O | 1396.57 | 9.01 | 39.86 |
| 1007 | C₆₈H₁₃₃N₃O₇ | 1104.83 | 9.01 | 26.86 | 1207 | C₈₃H₁₇₂N₆O | 1270.33 | 9.01 | 35.1 |
| 1008 | C₆₈H₁₃₃N₃O₇ | 1104.01 | 9.01 | 26.86 | 1208 | C₆₅H₁₃₀N₆O | 1011.8 | 9.01 | 24.06 |
| 1009 | C₆₂H₁₂₁N₃O₇ | 1020.66 | 9.01 | 23.69 | 1209 | C₆₅H₁₃₀N₆O | 1011.8 | 9.01 | 23.97 |
| 1010 | C₆₇H₁₃₃N₃O₆ | 1076.82 | 9.78 | 27.13 | 1210 | C₄₄H₉₄N₆O | 723.28 | 9.01 | 14.86 |
| 1011 | C₇₀H₁₃₇N₃O₇ | 1132.88 | 9.07 | 27.92 | 1211 | C₅₆H₁₀₆N₆O | 879.5 | 9.01 | 18.3 |
| 1012 | C₇₂H₁₄₁N₃O₇ | 1160.93 | 9.1 | 28.98 | 1212 | C₆₂H₁₁₈N₆O | 963.67 | 9.01 | 20.3 |
| 1013 | C₇₄H₁₄₅N₃O₄ | 1188.99 | 9.12 | 30.03 | 1213 | C₅₀H₁₀₀N₆O | 801.39 | 9.01 | 16.58 |
| 1014 | C₇₀H₁₃₇N₃O₉ | 1164.88 | 7.4 | 26.03 | 1214 | C₆₂H₁₂₄N₆O | 969.71 | 9.01 | 22.93 |
| 1015 | C₇₂H₁₄₁N₃O₉ | 1192.93 | 8.33 | 26.61 | 1215 | C₆₂H₁₃₀N₆O | 975.76 | 9.01 | 24.38 |
| 1016 | C₆₅H₁₂₉N₃O₈ | 1080.8 | 9.11 | 23.7 | 1216 | C₆₈H₁₁₈N₆O | 1035.73 | 9.01 | 25.13 |
| 1017 | C₇₁H₁₄₁N₃O₈ | 1164.9 | 9.1 | 26.87 | 1217 | C₈₃H₁₄₈N₆O | 1246.14 | 9.01 | 32.83 |
| 1018 | C₇₂H₁₄₀N₂O₁₀ | 1193.92 | 8.15 | 27.21 | 1218 | C₅₉H₁₂₁N₃OS₆ | 1081 | 9.01 | 27.15 |
| 1019 | C₇₂H₁₄₀N₂O₁₀ | 1193.92 | 8.15 | 27.21 | 1219 | C₅₉H₁₂₁N₃OS₆ | 1081 | 9.01 | 27.15 |
| 1020 | C₇₄H₁₄₅N₃O₉ | 1220.99 | 8.74 | 26.06 | 1220 | C₇₁H₁₄₅N₃OS₆ | 1249.32 | 9.01 | 33.5 |
| 1021 | C₇₄H₁₄₅N₃O₉ | 1221 | 8.74 | 26.06 | 1221 | C₉₂H₁₈₇N₃OS₆ | 1543.89 | 9.01 | 44.61 |
| 1022 | C₇₃H₁₄₅N₃O₅ | 1193 | 9.51 | 26.33 | 1222 | C₆₅H₁₃₃N₃OS₆ | 1165.16 | 9.01 | 30.32 |
| 1023 | C₇₄H₁₄₄N₂O₁₀ | 1221.97 | 8.55 | 26.66 | 1223 | C₁₀₀H₁₉₈N₄O₁₀S ₂ | 1680.82 | 8.9 | 43.24 |
| 1024 | C₇₄H₁₄₄N₂O₁₀ | 1221.97 | 8.55 | 26.66 | 1224 | C₉₈H₁₉₉N₃OS₆ | 1628.05 | 9.01 | 48.05 |
| 1025 | C₆₉H₁₃₅N₃O₈ | 1134.85 | 8.2 | 26.44 | 1225 | C₉₂H₁₈₇N₃OS₆ | 1543.89 | 9.01 | 44.88 |
| 1026 | C₆₉H₁₃₄N₂O₉ | 1135.84 | 8.02 | 27.05 | 1226 | C₈₃H₁₆₉N₃OS₆ | 1417.64 | 9.01 | 40.11 |
| 1027 | C₆₉H₁₃₄N₂O₉ | 1135.84 | 8.02 | 27.05 | 1227 | C₆₅H₁₂₇N₃OS₆ | 1159.11 | 9.01 | 29.08 |
| 1028 | C₆₄H₁₂₅N₃O₇ | 1048.7 | 9.07 | 24.74 | 1228 | C₆₅H₁₂₇N₃OS₆ | 1159.11 | 9.01 | 28.99 |
| 1029 | C₆₈H₁₃₂N₂O₈ | 1105.81 | 8.83 | 27.46 | 1229 | C₄₄H₉₁N₃OS₆ | 870.59 | 9.01 | 19.87 |
| 1030 | C₆₆H₁₂₉N₃O₇ | 1076.8 | 9.1 | 25.8 | 1230 | C₅₆H₁₀₃N₃OS₆ | 1026.82 | 9.01 | 23.32 |
| 1031 | C₆₅H₁₂₉N₃O₆ | 1048.8 | 9.86 | 26.07 | 1231 | C₆₂H₁₁₅N₃OS₆ | 1110.98 | 9.01 | 25.32 |
| 1032 | C₆₉H₁₃₅N₃O₇ | 1118.9 | 9.1 | 27.39 | 1232 | C₅₀H₉₇N₃OS₆ | 948.71 | 9.01 | 21.6 |
| 1033 | C₆₆H₁₂₈N₂O₈ | 1077.8 | 8.91 | 26.41 | 1233 | C₆₂H₁₂₁N₃OS₆ | 1117.03 | 9.01 | 27.94 |
| 1034 | C₆₈H₁₃₃N₃O₇ | 1104.83 | 9.12 | 26.86 | 1234 | C₆₂H₁₂₇N₃OS₆ | 1123.08 | 9.01 | 29.4 |
| 1035 | C₇₁H₁₃₉N₃O₇ | 1146.91 | 9.12 | 28.45 | 1235 | C₆₈H₁₁₅N₃OS₆ | 1183.05 | 9.01 | 27.86 |
| 1036 | C₆₈H₁₃₂N₂O₈ | 1105.8 | 8.93 | 27.46 | 1236 | C₈₃H₁₄₅N₃OS₆ | 1393.45 | 9.01 | 35.56 |
| 1037 | C₆₄H₁₂₅N₃O₉ | 1080.7 | 7.4 | 22.85 | 1237 | C₆₂H₁₂₁N₃O₄ | 972.67 | 9.01 | 23.48 |
| 1038 | C₆₇H₁₃₁N₃O₉ | 1122.8 | 7.4 | 24.44 | 1238 | C₇₄H₁₄₅N₃O₄ | 1140.99 | 9.01 | 29.83 |
| 1039 | C₆₄H₁₂₄N₂O₁₀ | 1081.7 | 7.21 | 23.46 | 1239 | C₉₅H₁₈₇N₃O₄ | 1435.56 | 9.01 | 40.94 |
| 1040 | C₆₉H₁₃₅N₃O₉ | 1150.9 | 8.33 | 25.02 | 1240 | C₆₈H₁₃₃N₃O₄ | 1056.83 | 9.01 | 26.65 |
| 1041 | C₆₆H₁₂₈N₂O₁₀ | 1109.8 | 8.15 | 24.04 | 1241 | C₆₈H₁₃₃N₃O₄ | 1056.83 | 9.01 | 26.65 |
| 1042 | C₆₈H₁₃₂N₂O₁₀ | 1137.8 | 8.55 | 23.49 | 1242 | C₁₀₁H₁₉₉N₃O₄ | 1519.72 | 9.01 | 43.72 |
| 1043 | C₇₀H₁₃₈N₄O₈ | 1163.89 | 9.4 | 25.69 | 1243 | C₉₅H₁₈₇N₃O₄ | 1435.56 | 9.01 | 40.55 |
| 1044 | C₆₄H₁₂₆N₄O₈ | 1079.73 | 9.4 | 22.52 | 1244 | C₈₆H₁₆₉N₃O₄ | 1309.32 | 9.01 | 35.79 |
| 1045 | C₇₀H₁₃₈N₄O₈ | 1163.89 | 9.4 | 25.69 | 1245 | C₆₈H₁₂₇N₃O₄ | 1050.78 | 9.01 | 24.75 |
| 1046 | C₇₀H₁₃₈N₄O₈ | 1163.89 | 9.4 | 25.69 | 1246 | C₆₈H₁₂₇N₃O₄ | 1050.78 | 9.01 | 24.66 |
| 1047 | C₆₇H₁₃₁N₃O₈ | 1106.8 | 8.95 | 25.14 | 1247 | C₄₇H₉₁N₃O₄ | 762.26 | 9.01 | 15.54 |
| 1048 | C₆₁H₁₁₉N₃O₈ | 1022.64 | 8.95 | 21.96 | 1248 | C₅₉H₁₀₃N₃O₄ | 918.49 | 9.01 | 18.99 |
| 1049 | C₆₇H₁₃₁N₃O₈ | 1106.8 | 8.95 | 25.14 | 1249 | C₆₅H₁₁₅N₃O₄ | 1002.65 | 9.01 | 20.99 |
| 1050 | C₇₂H₁₃₉NO₉ | 1162.9 | 9.1 | 28.52 | 1250 | C₅₃H₉₇N₃O₄ | 840.38 | 9.01 | 17.27 |
| 1051 | C₇₄H₁₃₉N₃O₁₃ | 1278.9 | 9.01 | 23.74 | 1251 | C₆₅H₁₂₁N₃O₄ | 1008.7 | 9.01 | 23.61 |
| 1052 | C₇₇H₁₅₀N₂O₇ | 1216.1 | 9.83 | 32.01 | 1252 | C₆₅H₁₂₇N₃O₄ | 1014.75 | 9.01 | 25.07 |
| 1053 | C₇₃H₁₃₇N₂O₆P | 1169.88 | 9.68 | 25.77 | 1253 | C₇₁H₁₁₅N₃O₄ | 1074.72 | 9.01 | 25 |
| 1054 | C₇₄H₁₃₈N₃O₇P | 1212.91 | 9.95 | 26.18 | 1254 | C₈₆H₁₄₅N₃O₄ | 1285.12 | 9.01 | 32.69 |
| 1055 | C₇₁H₁₃₆N₃O₃PS₉ | 1399.4 | 8.85 | 32.41 | 1255 | C₉₉H₁₉₅N₃O₃S₄ | 1603.91 | 8.68 | 43.96 |
| 1056 | C₆₈H₁₃₈N₂O₅ | 1063.9 | 9.7 | 26.03 | 1256 | C₇₄H₁₄₅N₃O₇ | 1188.99 | 9.01 | 30.03 |
| 1057 | C₇₀H₁₂₀N₄O₆S₂ | 1198.03 | 9.67 | 24.35 | 1257 | C₉₅H₁₈₇N₃O₇ | 1483.55 | 9.01 | 41.14 |
| 1058 | C₆₇H₁₃₂N₂O₇ | 1077.8 | 9.45 | 27.29 | 1258 | C₁₀₄H₂₀₀N₃O₇P S₄ | 1763.97 | 7.94 | 41.36 |
| 1059 | C₆₇H₁₃₂N₂O₇ | 1077.8 | 9.45 | 27.29 | 1259 | C₁₀₃H₂₀₀N₂OS₇ | 1707.17 | 10.09 | 46.14 |
| 1060 | C₆₇H₁₃₃N₃O₈S | 1140.87 | 8.74 | 26.96 | 1260 | C₁₀₁H₁₉₉N₃O₇ | 1567.72 | 9.01 | 44.59 |
| 1061 | C₇₃H₁₂₅N₃O₅ | 1124.82 | 9.77 | 23.41 | 1261 | C₉₅H₁₈₇N₃O₇ | 1483.55 | 9.01 | 41.41 |
| 1062 | C₆₇H₁₁₃N₃O₁₀S₃ | 1216.83 | 9.73 | 22.69 | 1262 | C₈₆H₁₆₉N₃O₇ | 1357.31 | 9.01 | 36.65 |
| 1063 | C₆₅H₁₀₉N₃O₁₀S₃ | 1188.78 | 9.01 | 21.48 | 1263 | C₆₈H₁₂₇N₃O₇ | 1098.78 | 9.01 | 25.62 |
| 1064 | C₆₈H₁₃₉N₃ OS₆ | 1207.24 | 9.01 | 32.18 | 1264 | C₆₈H₁₂₇N₃O₇ | 1098.78 | 9.01 | 25.53 |
| 1065 | C₆₈H₁₃₉N₃OS₆ | 1207.24 | 9.01 | 32.18 | 1265 | C₄₇H₉₁N₃O₇ | 810.26 | 9.01 | 16.41 |
| 1066 | C₇₄H₁₅₄N₃O₁₃P₃ | 1386.98 | 9.01 | 26.51 | 1266 | C₅₉H₁₀₃N₃O₇ | 966.49 | 9.01 | 19.85 |
| 1067 | C₇₄H₁₅₄N₃O₁₃P₃ | 1386.98 | 9.01 | 26.51 | 1267 | C₆₅H₁₁₅N₃O₇ | 1050.65 | 9.01 | 21.86 |
| 1068 | C₇₁H₁₃₁N₃O₇ | 1138.84 | 5.07 | 26.81 | 1268 | C₅₃H₉₇N₃O₇ | 888.37 | 9.01 | 18.13 |
| 1069 | C₇₁H₁₃₁N₃O₇ | 1138.84 | 5.04 | 26.81 | 1269 | C₆₅H₁₂₁N₃O₇ | 1056.7 | 9.01 | 24.48 |
| 1070 | C₆₉H₁₃₀N₄O₇ | 1127.82 | 5.03 | 26.38 | 1270 | C₆₅H₁₂₇N₃O₇ | 1062.74 | 9.01 | 25.93 |
| 1071 | C₆₉H₁₃₀N₄O₇ | 1127.82 | 5 | 26.38 | 1271 | C₇₁H₁₁₅N₃O₇ | 1122.71 | 9.01 | 26.17 |
| 1072 | C₇₀H₁₃₅N₃O₈ | 1146.86 | 6.83 | 26.82 | 1272 | C₈₆H₁₄₅N₃O₇ | 1333.12 | 9.01 | 33.87 |
| 1073 | C₇₀H₁₃₅N₃O₈ | 1146.86 | 6.83 | 26.82 | 1273 | C₆₂H₁₂₁N₃O₇ | 1020.66 | 9.01 | 23.69 |
| 1074 | C₇₁H₁₃₈N₄O₈ | 1175.91 | 7.89 | 26.41 | 1274 | C₇₄H₁₄₅N₃O₄ | 1188.99 | 9.01 | 30.03 |
| 1075 | C₇₁H₁₃₈N₄O₈ | 1175.91 | 7.89 | 26.41 | 1275 | C₉₅H₁₈₇N₃O₇ | 1483.55 | 9.01 | 41.14 |
| 1076 | C₇₁H₁₃₈N₄O₉ | 1191.9 | 7.8 | 26.25 | 1276 | C₆₈H₁₃₃N₃O₇ | 1104.83 | 9.01 | 26.86 |
| 1077 | C₇₁H₁₃₈N₄O₉ | 1191.9 | 7.8 | 26.25 | 1277 | C₆₈H₁₃₃N₃O₇ | 1104.83 | 9.01 | 26.86 |
| 1078 | C₇₁H₁₃₈N₄O₉ | 1191.9 | 7.75 | 27.09 | 1278 | C₁₀₁H₁₉₉N₃O₇ | 1567.72 | 9.01 | 44.59 |
| 1079 | C₇₁H₁₃₈N₄O₉ | 1191.9 | 7.75 | 27.09 | 1279 | C₉₅H₁₈₇N₃O₇ | 1483.55 | 9.01 | 41.41 |
| 1080 | C₇₀H₁₃₈N₄O₉S | 1211.95 | 7.61 | 26.56 | 1280 | C₈₆H₁₆₉N₃O₇ | 1357.31 | 9.01 | 36.65 |
| 1081 | C₇₀H₁₃₈N₄O₉S | 1211.95 | 7.61 | 26.56 | 1281 | C₆₈H₁₂₇N₃O₇ | 1098.78 | 9.01 | 25.62 |
| 1082 | C₇₂H₁₃₉N₅O₈ | 1202.93 | 7.82 | 26.46 | 1282 | C₆₈H₁₂₇N₃O₇ | 1098.78 | 9.01 | 25.53 |
| 1083 | C₇₂H₁₃₉N₅O₈ | 1202.93 | 7.82 | 26.46 | 1283 | C₄₇H₉₁N₃O₇ | 810.26 | 9.01 | 16.41 |
| 1084 | C₇₂H₁₃₇N₅O₇S | 1216.98 | 7.95 | 28.08 | 1284 | C₅₉H₁₀₃N₃O₇ | 966.49 | 9.01 | 19.85 |
| 1085 | C₇₂H₁₃₇N₅O₇S | 1216.98 | 7.95 | 28.08 | 1285 | C₆₅H₁₁₅N₃O₇ | 1050.65 | 9.01 | 21.86 |
| 1086 | C₇₄H₁₃₈N₈O₇ | 1251.97 | 5.88 | 25 | 1286 | C₅₃H₉₇N₃O₇ | 888.37 | 9.01 | 18.13 |
| 1087 | C₇₄H₁₃₈N₈O₇ | 1251.97 | 5.88 | 25 | 1287 | C₆₅H₁₂₁N₃O₇ | 1056.7 | 9.01 | 24.48 |
| 1088 | C₇₄H₁₃₈N₃O₈ | 1267.97 | 7.59 | 25.58 | 1288 | C₆₅H₁₂₇N₃O₇ | 1062.74 | 9.01 | 25.93 |
| 1089 | C₇₄H₁₃₈N₃O₈ | 1267.97 | 7.59 | 25.58 | 1289 | C₇₁H₁₁₅N₃O₇ | 1122.71 | 9.01 | 24.8 |
| 1090 | C₇₃H₁₃₇N₅O₉ | 1228.93 | 7.07 | 26.15 | 1290 | C₈₆H₁₄₅N₃O₇ | 1333.12 | 9.01 | 32.49 |
| 1091 | C₇₃H₁₃₇N₅O₉ | 1228.93 | 7.07 | 26.15 | 1291 | C₆₂H₁₂₁N₃O₁₀ | 1068.66 | 9.01 | 23.51 |
| 1092 | C₇₄H₁₃₉N₅O₈ | 1226.95 | 7.47 | 26.45 | 1292 | C₇₄H₁₄₅N₃O₁₀ | 1236.98 | 9.01 | 29.86 |
| 1093 | C₇₄H₁₃₉N₅O₈ | 1226.95 | 7.47 | 26.45 | 1293 | C₉₅H₁₈₇N₃O₁₀ | 1531.55 | 9.01 | 40.96 |
| 1094 | C₇₀H₁₃₈N₆O₇ | 1175.91 | 8.12 | 25.9 | 1294 | C₆₈H₁₃₃N₃O₁₀ | 1152.82 | 9.01 | 26.68 |
| 1095 | C₇₀H₁₃₈N₆O₇ | 1175.91 | 8.12 | 25.9 | 1295 | C₆₈H₁₃₃N₃O₁₀ | 1152.82 | 9.01 | 26.68 |
| 1096 | C₇₂H₁₄₂N₆O₉S | 1268.02 | 7.63 | 26.4 | 1296 | C₁₀₁H₁₉₉N₃O₁₀ | 1615.71 | 9.01 | 44.41 |
| 1097 | C₇₂H₁₄₂N₆O₉S | 1268.02 | 7.63 | 26.4 | 1297 | C₉₅H₁₈₇N₃O₁₀ | 1531.55 | 9.01 | 41.23 |
| 1098 | C₇₃H₁₄₃N₅O₉S | 1267.03 | 7.51 | 27.09 | 1298 | C₈₆H₁₆₉N₃O₁₀ | 1405.31 | 9.01 | 36.47 |
| 1099 | C₇₃H₁₄₃N₅O₉S | 1267.03 | 7.51 | 27.09 | 1299 | C₆₈H₁₂₇N₃O₁₀ | 1146.77 | 9.01 | 25.44 |
| 1100 | C₇₄H₁₄₅N₅O₉S | 1281.06 | 8.31 | 27.25 | 1300 | C₆₈H₁₂₇N₃O₁₀ | 1146.77 | 9.01 | 25.35 |
| 1101 | C₇₃H₁₄₃N₅O₉S | 1267.03 | 7.51 | 27.09 | 1301 | C₄₇H₉₁N₃O₁₀ | 858.26 | 9.01 | 16.23 |
| 1102 | C₇₂H₁₃₉N₃O₇ | 1158.92 | 10.26 | 27.41 | 1302 | C₅₉H₁₀₃N₃O₁₀ | 1014.48 | 9.01 | 19.68 |
| 1103 | C₇₁H₁₃₈N₄O₇ | 1159.91 | 9.78 | 26.04 | 1303 | C₆₅H₁₁₅N₃O₁₀ | 1098.65 | 9.01 | 21.68 |
| 1104 | C₆₉H₁₃₃N₃O₇ | 1116.84 | 9.32 | 25.52 | 1304 | C₅₃H₉₇N₃O₁₀ | 936.37 | 9.01 | 17.95 |
| 1105 | C₆₉H₁₃₃N₃O₇ | 1116.84 | 9.32 | 25.52 | 1305 | C₆₅H₁₂₁N₃O₁₀ | 1104.69 | 9.01 | 24.3 |
| 1106 | C₆₉H₁₃₆N₆O₇ | 1161.88 | 7.46 | 24.33 | 1306 | C₆₅H₁₂₇N₃O₁₀ | 1110.74 | 9.01 | 25.75 |
| 1107 | C₆₃H₁₂₄N₆O₇ | 1077.72 | 7.46 | 21.15 | 1307 | C₇₁H₁₁₅N₃O₁₀ | 1170.71 | 9.01 | 24.31 |
| 1108 | C₆₉H₁₃₆N₆O₇ | 1161.88 | 7.46 | 24.33 | 1308 | C₈₆H₁₄₅N₃O₁₀ | 1381.12 | 9.01 | 32 |
| 1109 | C₆₉H₁₃₆N₆O₇ | 1161.88 | 7.46 | 24.33 | 1309 | C₆₂H₁₂₄N₆O₄ | 1017.71 | 9.01 | 20.89 |
| 1110 | C₇₀H₁₃₆N₄O₇ | 1145.88 | 8.82 | 26.41 | 1310 | C₇₄H₁₄₈N₆O₄ | 1186.04 | 9.01 | 27.24 |
| 1111 | C₇₀H₁₃₆N₄O₇ | 1145.88 | 8.82 | 26.41 | 1311 | C₉₅H₁₉₀N₆O₄ | 1480.6 | 9.01 | 38.35 |
| 1112 | C₆₉H₁₃₆N₂O₆ | 1089.85 | 10.19 | 28.16 | 1312 | C₆₈H₁₃₆N₆O₄ | 1101.87 | 9.01 | 24.06 |
| 1113 | C₇₁H₁₃₈N₂O₈ | 1147.89 | 9.69 | 26.58 | 1313 | C₆₈H₁₃₆N₆O₄ | 1101.87 | 9.01 | 24.06 |
| 1114 | C₇₀H₁₃₈N₂O₇ | 1119.88 | 9.84 | 26.71 | 1314 | C₁₀₁H₂₀₂N₆O₄ | 1564.76 | 9.01 | 41.79 |
| 1115 | C₆₇H₁₃₁N₃O₇ | 1090.8 | 9.5 | 26.91 | 1315 | C₉₅H₁₉₀N₆O₄ | 1480.6 | 9.01 | 38.62 |
| 1116 | C₆₉H₁₃₅N₃O₈ | 1134.85 | 9.52 | 27.58 | 1316 | C₈₆H₁₇₂N₆O₄ | 1354.36 | 9.01 | 33.85 |
| 1117 | C₇₁H₁₄₁N₃O₆ | 1132.92 | 9.88 | 29.03 | 1317 | C₆₈H₁₃₀N₆O₄ | 1095.83 | 9.01 | 22.82 |
| 1118 | C₇₁H₁₃₉N₃O₈ | 1162.91 | 8.87 | 26.46 | 1318 | C₆₈H₁₃₀N₆O₄ | 1095.83 | 9.01 | 22.73 |
| 1119 | C₇₁H₁₃₃NO₉S₅ | 1305.14 | 9.96 | 25.12 | 1319 | C₄₇H₉₄N₆O₄ | 807.31 | 9.01 | 13.61 |
| 1120 | C₇₂H₁₄₂N₂O₇ | 1147.9 | 10.05 | 27.76 | 1320 | C₅₉H₁₀₆N₆O₄ | 963.53 | 9.01 | 17.06 |
| 1121 | C₇₃H₁₃₉N₅O₁₅S₄ | 1455.17 | 7.8 | 23.62 | 1321 | C₅₉H₁₀₆N₆O₄ | 963.53 | 9.01 | 17.06 |
| 1122 | C₆₈H₁₂₉NO₁₃S₃ | 1264.95 | 8.49 | 25.91 | 1322 | C₅₃H₁₀₀N₆O₄ | 885.42 | 9.01 | 15.34 |
| 1123 | C₇₇H₁₅₂N₂O₇ | 1218.07 | 9.99 | 30.68 | 1323 | C₆₅H₁₂₄N₆O₄ | 1053.74 | 9.01 | 21.68 |
| 1124 | C₆₇H₁₃₃N₃O₈S | 1140.87 | 8.74 | 26.96 | 1324 | C₆₅H₁₃₀N₆O₄ | 1059.79 | 9.01 | 23.14 |
| 1125 | C₆₉H₁₃₆N₄O₇ | 1133.87 | 9.61 | 27.37 | 1325 | C₇₁H₁₁₈N₆O₄ | 1119.76 | 9.01 | 23.4 |
| 1126 | C₆₅H₁₂₁N₃O₇ | 1056.7 | 9.01 | 23.82 | 1326 | C₈₆H₁₄₈N₆O₄ | 1330.17 | 9.01 | 31.09 |
| 1127 | C₆₉H₁₃₆N₂O₉ | 1137.85 | 8.31 | 26.05 | 1327 | C₆₂H₁₂₄N₆O₄ | 1017.71 | 9.01 | 20.89 |
| 1128 | C₇₀H₁₃₉N₂O₁₁P | 1215.86 | 7.77 | 26.53 | 1328 | C₇₄H₁₄₈N₆O₄ | 1186.04 | 9.01 | 27.24 |
| 1129 | C₆₈H₁₃₄N₂O₇S₂ | 1155.95 | 8.84 | 27.8 | 1329 | C₉₅H₁₉₀N₆O₄ | 1480.6 | 9.01 | 38.35 |
| 1130 | C₇₄H₁₅₄N₃O₁₃P₃ | 1386.98 | 9.01 | 26.51 | 1330 | C₆₈H₁₃₆N₆O₄ | 1101.87 | 9.01 | 24.06 |
| 1131 | C₈₄H₁₅₉N₃O₈ | 1339.21 | 9.46 | 31.7 | 1331 | C₆₈H₁₃₆N₆O₄ | 1101.87 | 9.01 | 24.06 |
| 1132 | C₈₇H₁₄₇N₃O₈ | 1363.15 | 9.46 | 32.11 | 1332 | C₁₀₁H₂₀₁N₅O₅ | 1565.75 | 9.01 | 42.72 |
| 1133 | C₇₁H₁₃₁N₃O₇ | 1138.84 | 5.07 | 26.81 | 1333 | C₉₅H₁₈₉N₅O₅ | 1481.59 | 9.01 | 39.55 |
| 1134 | C₆₉H₁₃₀N₄O₇ | 1127.82 | 5 | 26.38 | 1334 | C₈₆H₁₇₁N₅O₅ | 1355.34 | 9.01 | 34.79 |
| 1135 | C₇₁H₁₃₈N₄O₈ | 1175.91 | 7.89 | 26.41 | 1335 | C₆₈H₁₃₀N₆O₄ | 1095.83 | 9.01 | 22.82 |
| 1136 | C₁₀₈H₂₀₄N₂O₉S₂ | 1738.94 | 8.56 | 46.05 | 1336 | C₆₈H₁₂₉N₅O₅ | 1096.81 | 9.01 | 23.66 |
| 1137 | C₉₉H₁₉₅NO₆S₃ | 1591.83 | 7.68 | 42.68 | 1337 | C₄₇H₉₃N₅O₅ | 808.29 | 9.01 | 14.55 |
| 1138 | C₇₂H₁₃₇N₅O₇S | 1216.98 | 7.95 | 28.08 | 1338 | C₅₉H₁₀₅N₅O₅ | 964.52 | 9.01 | 17.99 |
| 1139 | C₇₄H₁₃₈N₈O₇ | 1251.97 | 5.88 | 25 | 1339 | C₆₅H₁₁₇N₅O₅ | 1048.68 | 9.01 | 19.99 |
| 1140 | C₇₀H₁₃₈N₆O₇ | 1175.91 | 8.12 | 25.9 | 1340 | C₅₃H₉₉N₅O₅ | 886.4 | 9.01 | 16.27 |
| 1141 | C₇₃H₁₄₃N₅O₉S | 1267.03 | 7.51 | 27.09 | 1341 | C₆₅H₁₂₃N₅O₅ | 1054.73 | 9.01 | 22.62 |
| 1142 | C₇₀H₁₃₅N₃O₇ | 1130.86 | 9.98 | 26.32 | 1342 | C₆₅H₁₂₉N₅O₅ | 1060.78 | 9.01 | 24.07 |
| 1143 | C₆₅H₁₁₅N₃O₇ | 1050.6 | 9.01 | 21.2 | 1343 | C₇₁H₁₁₇N₅O₅ | 1120.75 | 9.01 | 24.31 |
| 1144 | C₇₅H₁₄₁N₃O₈ | 1212.97 | 9.18 | 28.86 | 1344 | C₈₆H₁₄₈N₆O₄ | 1330.17 | 9.01 | 31.76 |
| 1145 | C₇₀H₁₁₈N₂O₁₁S₃ | 1259.9 | 9.49 | 22.27 | 1345 | C₆₂H₁₂₄N₆O₇ | 1065.71 | 9.01 | 22.93 |
| 1146 | C₆₈H₁₃₄N₂O₇S₂ | 1155.95 | 9.14 | 27.39 | 1346 | C₇₄H₁₄₈N₆O₇ | 1234.03 | 9.01 | 29.28 |
| 1147 | C₈₆H₁₆₇N₃O₅S₂ | 1387.4 | 6.75 | 35.1 | 1347 | C₉₅H₁₉₀N₆O₇ | 1528.6 | 9.01 | 40.39 |
| 1148 | C₆₉H₁₂₀N₆O₂S₄ | 1194 | 8.63 | 24.08 | 1348 | C₆₈H₁₃₆N₆O₇ | 1149.87 | 9.01 | 26.1 |
| 1149 | C₇₆H₁₅₄N₆O₃S | 1232.2 | 7.65 | 30.39 | 1349 | C₆₈H₁₃₆N₆O₇ | 1149.87 | 9.01 | 26.1 |
| 1150 | C₉₁H₁₈₆N₄S₆ | 1528.9 | 9.77 | 43.45 | 1350 | C₁₀₁H₂₀₂N₆O₇ | 1612.76 | 9.01 | 43.83 |
| 1151 | C₇₈H₁₄₂N₆O₅ | 1244 | NA | 27.09 | 1351 | C₉₅H₁₉₀N₆O₇ | 1528.6 | 9.01 | 40.66 |
| 1152 | C₁₀₅H₁₈₁N₃O₁₂ | 1677.6 | 9.16 | 40.21 | 1352 | C₈₆H₁₇₂N₆O₇ | 1402.36 | 9.01 | 35.9 |
| 1153 | C₆₉H₁₃₁N₅O₅ | 1110.8 | 7.41 | 22.95 | 1353 | C₆₈H₁₃₀N₆O₇ | 1143.82 | 9.01 | 24.86 |
| 1154 | C₇₆H₁₃₉N₃S₃ | 1191.1 | 8.91 | 30.35 | 1354 | C₆₈H₁₃₀N₆O₇ | 1143.82 | 9.01 | 24.77 |
| 1155 | C₆₆H₁₃₂N₅O₁₀P | 1186.8 | 8.65 | 24.15 | 1355 | C₄₇H₉₄N₆O₇ | 855.3 | 9.01 | 15.66 |
| 1156 | C₇₀H₁₂₀N₇O₈P | 1218.7 | 9.55 | 21.96 | 1356 | C₅₉H₁₀₆N₆O₇ | 1011.53 | 9.01 | 19.1 |
| 1157 | C₇₂H₁₃₅N₅O₁₂S₃ | 1359.08 | 7.99 | 23.39 | 1357 | C₆₅H₁₁₈N₆O₇ | 1095.69 | 9.01 | 21.1 |
| 1158 | C₇₂H₁₃₀N₂O₉ | 1167.84 | 8.05 | 25.65 | 1358 | C₅₃H₁₀₀N₆O₇ | 933.42 | 9.01 | 17.38 |
| 1159 | C₇₁H₁₃₉N₃O₃S₆ | 1275.3 | 9.04 | 31.51 | 1359 | C₆₅H₁₂₄N₆O₇ | 1101.74 | 9.01 | 23.73 |
| 1160 | C₆₉H₁₃₈N₄O₉S₃ | 1264.1 | 7.48 | 28.35 | 1360 | C₆₅H₁₃₀N₆O₇ | 1107.79 | 9.01 | 25.18 |
| 1161 | C₇₃H₁₄₂N₂O₈ | 1175.9 | 9.39 | 27.24 | 1361 | C₇₁H₁₁₈N₆O₇ | 1167.76 | 9.01 | 24.18 |
| 1162 | C₈₅H₁₆₇N₅O₁₀ | 1419.3 | 9.49 | 27.91 | 1362 | C₈₆H₁₄₈N₆O₇ | 1378.16 | 9.01 | 31.87 |
| 1163 | C₅₉H₁₂₁N₃O₄ | 936.63 | 9.01 | 22.86 | 1363 | C₆₂H₁₂₄N₆O₇ | 1065.71 | 9.01 | 22.93 |
| 1164 | C₇₁H₁₄₅N₃O₄ | 1104.96 | 9.01 | 29.21 | 1364 | C₇₄H₁₄₈N₆O₇ | 1234.03 | 9.01 | 29.28 |
| 1165 | C₉₂H₁₈₇N₃O₄ | 1399.53 | 9.01 | 40.32 | 1365 | C₉₅H₁₉₀N₆O₇ | 1528.6 | 9.01 | 40.39 |
| 1166 | C₆₅H₁₃₃N₃O₄ | 1020.8 | 9.01 | 26.04 | 1366 | C₆₈H₁₃₆N₆O₇ | 1149.87 | 9.01 | 26.1 |
| 1167 | C₆₅H₁₃₃N₃O₄ | 1020.8 | 9.01 | 26.04 | 1367 | C₆₈H₁₃₆N₆O₇ | 1149.87 | 9.01 | 26.1 |
| 1168 | C₉₈H₁₉₉N₃O₄ | 1483.69 | 9.01 | 43.77 | 1368 | C₁₀₁H₂₀₂N₆O₇ | 1612.76 | 9.01 | 43.83 |
| 1169 | C₉₂H₁₈₇N₃O₄ | 1399.53 | 9.01 | 40.59 | 1369 | C₉₅H₁₉₀N₆O₇ | 1528.6 | 9.01 | 40.66 |
| 1170 | C₈₃H₁₆₉N₃O₄ | 1273.28 | 9.01 | 35.83 | 1370 | C₈₆H₁₇₂N₆O₇ | 1402.36 | 9.01 | 35.9 |
| 1171 | C₆₅H₁₂₇N₃O₄ | 1014.75 | 9.01 | 24.8 | 1371 | C₆₈H₁₃₀N₆O₇ | 1143.82 | 9.01 | 24.86 |
| 1172 | C₆₅H₁₂₇N₃O₄ | 1014.75 | 9.01 | 24.71 | 1372 | C₆₈H₁₃₀N₆O₇ | 1143.82 | 9.01 | 24.77 |
| 1173 | C₄₄H₉₁N₃O₄ | 726.23 | 9.01 | 15.59 | 1373 | C₄₇H₉₄N₆O₇ | 855.3 | 9.01 | 15.66 |
| 1174 | C₅₆H₁₀₃N₃O₄ | 882.46 | 9.01 | 19.03 | 1374 | C₅₉H₁₀₆N₆O₇ | 1011.53 | 9.01 | 19.1 |
| 1175 | C₆₂H₁₁₅N₃O₄ | 966.62 | 9.01 | 21.04 | 1375 | C₆₅H₁₁₈N₆O₇ | 1095.69 | 9.01 | 21.1 |
| 1176 | C₅₀H₉₇N₃O₄ | 804.34 | 9.01 | 17.31 | 1376 | C₅₃H₁₀₀N₆O₇ | 933.42 | 9.01 | 17.38 |
| 1177 | C₆₂H₁₂₁N₃O₄ | 972.67 | 9.01 | 23.66 | 1377 | C₆₅H₁₂₄N₆O₇ | 1101.74 | 9.01 | 23.73 |
| 1178 | C₆₂H₁₂₇N₃O₄ | 978.71 | 9.01 | 25.11 | 1378 | C₆₅H₁₃₀N₆O₇ | 1107.79 | 9.01 | 25.18 |
| 1179 | C₆₈H₁₁₅N₃O₄ | 1038.68 | 9.01 | 26.06 | 1379 | C₇₁H₁₁₈N₆O₇ | 1167.76 | 9.01 | 25.06 |
| 1180 | C₈₃H₁₄₅N₃O₄ | 1249.09 | 9.01 | 33.76 | 1380 | C₈₆H₁₄₈N₆O₇ | 1378.16 | 9.01 | 32.76 |
| 1181 | C₅₉H₁₂₁N₃OS₃ | 984.82 | 9.01 | 25.15 | 1381 | C₆₂H₁₂₇N₉O₄ | 1062.76 | 9.01 | 22.06 |
| 1182 | C₇₁H₁₄₅N₃OS₃ | 1153.14 | 9.01 | 31.5 | 1382 | C₇₄H₁₅₁N₉O₄ | 1231.08 | 9.01 | 28.41 |
| 1183 | C₉₂H₁₈₇N₃OS₃ | 1447.71 | 9.01 | 42.61 | 1383 | C₉₅H₁₉₃N₉O₄ | 1525.65 | 9.01 | 39.52 |
| 1184 | C₆₅H₁₃₃N₃OS₃ | 1068.98 | 9.01 | 28.32 | 1384 | C₆₈H₁₃₉N₉O₄ | 1146.92 | 9.01 | 25.24 |
| 1185 | C₆₅H₁₃₃N₃OS₃ | 1068.98 | 9.01 | 28.32 | 1385 | C₆₈H₁₃₉N₉O₄ | 1146.92 | 9.01 | 25.24 |
| 1186 | C₉₈H₁₉₉N₃OS₃ | 1531.87 | 9.01 | 46.05 | 1386 | C₁₀₁H₂₀₅N₉O₄ | 1609.81 | 9.01 | 42.97 |
| 1187 | C₉₂H₁₈₇N₃OS₃ | 1447.71 | 9.01 | 42.88 | 1387 | C₉₅H₁₉₃N₉O₄ | 1525.65 | 9.01 | 39.79 |
| 1188 | C₈₃H₁₆₉N₃OS₃ | 1321.46 | 9.01 | 38.12 | 1388 | C₈₆H₁₇₅N₉O₄ | 1399.41 | 9.01 | 35.03 |
| 1189 | C₆₅H₁₂₇N₃OS₃ | 1062.93 | 9.01 | 27.08 | 1389 | C₆₈H₁₃₃N₉O₄ | 1140.87 | 9.01 | 24 |
| 1190 | C₆₅H₁₂₇N₃OS₃ | 1062.93 | 9.01 | 26.99 | 1390 | C₆₈H₁₃₃N₉O₄ | 1140.87 | 9.01 | 23.91 |
| 1191 | C₄₄H₉₁N₃OS₃ | 774.41 | 9.01 | 17.88 | 1391 | C₄₇H₉₇N₉O₄ | 852.35 | 9.01 | 14.79 |
| 1192 | C₅₆H₁₀₃N₃OS₃ | 930.64 | 9.01 | 21.32 | 1392 | C₅₉H₁₀₉N₉O₄ | 1008.58 | 9.01 | 18.23 |
| 1193 | C₆₂H₁₁₅N₃OS₃ | 1014.8 | 9.01 | 23.32 | 1393 | C₆₅H₁₂₁N₉O₄ | 1092.74 | 9.01 | 20.24 |
| 1194 | C₅₀H₉₇N₃OS₃ | 852.53 | 9.01 | 19.6 | 1394 | C₅₃H₁₀₃N₉O₄ | 930.47 | 9.01 | 16.51 |
| 1195 | C₆₂H₁₂₁N₃OS₃ | 1020.85 | 9.01 | 25.95 | 1395 | C₆₅H₁₂₇N₉O₄ | 1098.79 | 9.01 | 22.86 |
| 1196 | C₆₂H₁₂₇N₃OS₃ | 1026.9 | 9.01 | 27.4 | 1396 | C₆₅H₁₃₃N₉O₄ | 1104.84 | 9.01 | 24.31 |
| 1197 | C₆₈H₁₁₅N₃OS₃ | 1086.87 | 9.01 | 27.48 | 1397 | C₇₁H₁₂₁N₉O₄ | 1164.81 | 9.01 | 23.93 |
| 1198 | C₈₃H₁₄₅N₃OS₃ | 1297.27 | 9.01 | 35.18 | 1398 | C₈₆H₁₅₁N₉O₄ | 1375.21 | 9.01 | 31.63 |
| 1199 | C₅₉H₁₂₄N₆O | 933.68 | 9.01 | 22.13 | 1399 | C₆₂H₁₂₁N₃OS₃ | 1020.85 | 9.01 | 24 |
| 1200 | C₅₉H₁₂₄N₆O | 933.68 | 9.01 | 22.13 | 1400 | C₇₄H₁₄₅N₃OS₃ | 1189.17 | 9.01 | 30.34 |
| 1401 | C₉₅H₁₈₇N₃OS₃ | 1483.74 | 9.01 | 41.45 | 1601 | C₆₅H₁₃₆N₆O₇S₃ | 1210.04 | 9.01 | 24.53 |
| 1402 | C₆₈H₁₃₃N₃OS₃ | 1105.01 | 9.01 | 27.17 | 1602 | C₆₅H₁₃₆N₆O₇S₃ | 1210.04 | 9.01 | 24.53 |
| 1403 | C₆₈H₁₃₃N₃OS₃ | 1105.01 | 9.01 | 27.17 | 1603 | C₉₈H₂₀₂N₆O₇S₃ | 1672.93 | 9.01 | 42.26 |
| 1404 | C₁₀₁H₁₉₉N₃OS₃ | 1567.9 | 9.01 | 44.9 | 1604 | C₉₂H₁₉₀N₆O₇S₃ | 1588.77 | 9.01 | 39.08 |
| 1405 | C₉₅H₁₈₇N₃OS₃ | 1483.74 | 9.01 | 41.72 | 1605 | C₈₃H₁₇₂N₆O₇S₃ | 1462.52 | 9.01 | 34.32 |
| 1406 | C₈₆H₁₆₉N₃OS₃ | 1357.5 | 9.01 | 36.96 | 1606 | C₆₅H₁₃₀N₆O₇S₃ | 1203.99 | 9.01 | 23.29 |
| 1407 | C₆₈H₁₂₇N₃OS₃ | 1098.96 | 9.01 | 25.93 | 1607 | C₆₅H₁₃₀N₆O₇S₃ | 1203.99 | 9.01 | 23.2 |
| 1408 | C₆₈H₁₂₇N₃OS₃ | 1098.96 | 9.01 | 25.84 | 1608 | C₄₄H₉₄N₆O₇S₃ | 915.47 | 9.01 | 14.08 |
| 1409 | C₄₇H₉₁N₃OS₃ | 810.44 | 9.01 | 16.72 | 1609 | C₅₆H₁₀₆N₆O₇S₃ | 1071.7 | 9.01 | 17.52 |
| 1410 | C₅₉H₁₀₃N₃OS₃ | 966.67 | 9.01 | 20.17 | 1610 | C₆₂H₁₁₈N₆O₇S₃ | 1155.86 | 9.01 | 19.53 |
| 1411 | C₆₅H₁₁₅N₃OS₃ | 1050.83 | 9.01 | 22.17 | 1611 | C₅₀H₁₀₀N₆O₇S₃ | 993.59 | 9.01 | 15.8 |
| 1412 | C₅₃H₉₇N₃OS₃ | 888.56 | 9.01 | 18.44 | 1612 | C₆₂H₁₂₄N₆O₇S₃ | 1161.91 | 9.01 | 22.15 |
| 1413 | C₆₅H₁₂₁N₃OS₃ | 1056.88 | 9.01 | 24.79 | 1613 | C₆₂H₁₃₀N₆O₇S₃ | 1167.96 | 9.01 | 23.6 |
| 1414 | C₆₅H₁₂₇N₃OS₃ | 1062.93 | 9.01 | 26.24 | 1614 | C₆₈H₁₁₈N₆O₇S₃ | 1227.93 | 9.01 | 23.6 |
| 1415 | C₇₁H₁₁₅N₃OS₃ | 1122.9 | 9.01 | 25.38 | 1615 | C₈₃H₁₄₈N₆O₇S₃ | 1438.33 | 9.01 | 31.29 |
| 1416 | C₈₆H₁₄₅N₃OS₃ | 1333.31 | 9.01 | 33.07 | 1616 | C₅₉H₁₂₁N₃O₁₀S₃ | 1128.83 | 9.01 | 22.22 |
| 1417 | C₆₂H₁₂₁N₃OS₆ | 1117.03 | 9.01 | 26.24 | 1617 | C₇₁H₁₄₅N₃O₁₀S₃ | 1297.15 | 9.01 | 28.57 |
| 1418 | C₇₄H₁₄₅N₃OS₆ | 1285.35 | 9.01 | 32.59 | 1618 | C₉₂H₁₈₇N₃O₁₀S₃ | 1591.72 | 9.01 | 39.68 |
| 1419 | C₉₅H₁₈₇N₃OS₆ | 1579.92 | 9.01 | 43.7 | 1619 | C₆₅H₁₃₃N₃O₁₀S₃ | 1212.99 | 9.01 | 25.39 |
| 1420 | C₆₈H₁₃₃N₃OS₆ | 1201.19 | 9.01 | 29.41 | 1620 | C₆₅H₁₃₃N₃O₁₀S₃ | 1212.99 | 9.01 | 25.39 |
| 1421 | C₆₈H₁₃₃N₃OS₆ | 1201.19 | 9.01 | 29.41 | 1621 | C₉₈H₁₉₉N₃O₁₀S₃ | 1675.88 | 9.01 | 43.12 |
| 1422 | C₁₀₁H₁₉₉N₃OS₆ | 1664.08 | 9.01 | 47.14 | 1622 | C₉₂H₁₈₇N₃O₁₀S₃ | 1591.72 | 9.01 | 39.95 |
| 1423 | C₉₅H₁₈₇N₃OS₆ | 1579.92 | 9.01 | 43.97 | 1623 | C₈₃H₁₆₉N₃O₁₀S₃ | 1465.48 | 9.01 | 35.19 |
| 1424 | C₈₆H₁₆₉N₃OS₆ | 1453.68 | 9.01 | 39.2 | 1624 | C₆₅H₁₂₇N₃O₁₀S₃ | 1206.94 | 9.01 | 24.15 |
| 1425 | C₆₈H₁₂₇N₃OS₆ | 1195.14 | 9.01 | 28.17 | 1625 | C₆₅H₁₂₇N₃O₁₀S₃ | 1206.94 | 9.01 | 24.06 |
| 1426 | C₆₈H₁₂₇N₃OS₆ | 1195.14 | 9.01 | 28.08 | 1626 | C₄₄H₉₁N₃O₁₀S₃ | 918.42 | 9.01 | 14.95 |
| 1427 | C₄₇H₉₁N₃OS₆ | 906.62 | 9.01 | 18.96 | 1627 | C₅₆H₁₀₃N₃O₁₀S₃ | 1074.65 | 9.01 | 18.39 |
| 1428 | C₅₉H₁₀₃N₃OS₆ | 1062.85 | 9.01 | 22.41 | 1628 | C₆₂H_{11S}N₃O₁₀S₃ | 1158.81 | 9.01 | 20.39 |
| 1429 | C₆₅H₁₁₅N₃OS₆ | 1147.01 | 9.01 | 24.41 | 1629 | C₅₀H₉₇N₃O₁₀S₃ | 996.54 | 9.01 | 16.67 |
| 1430 | C₅₃H₉₇N₃OS₆ | 984.74 | 9.01 | 20.69 | 1630 | C₆₂H₁₂₁N₃O₁₀S₃ | 1164.86 | 9.01 | 23.02 |
| 1431 | C₆₅H₁₂₁N₃OS₆ | 1153.06 | 9.01 | 27.03 | 1631 | C₆₂H₁₂₇N₃O₁₀S₃ | 1170.91 | 9.01 | 24.47 |
| 1432 | C₆₅H₁₂₇N₃OS₆ | 1159.11 | 9.01 | 28.49 | 1632 | C₆₈H₁₁₅N₃O₁₀S₃ | 1230.88 | 9.01 | 24.27 |
| 1433 | C₇₁H₁₁₅N₃O₂S₅ | 1203.02 | 9.01 | 26.6 | 1633 | C₈₃H₁₄₅N₃O₁₀S₃ | 1441.28 | 9.01 | 31.96 |
| 1434 | C₈₆H₁₄₅N₃OS₆ | 1429.49 | 9.01 | 34.45 | 1634 | C₅₉H₁₂₁N₃O₁₀S₃ | 1128.83 | 9.01 | 22.22 |
| 1435 | C₆₂H₁₂₁N₃OS₆ | 1117.03 | 9.01 | 26.24 | 1635 | C₇₁H₁₄₅N₃O₁₀S₃ | 1297.15 | 9.01 | 28.57 |
| 1436 | C₇₄H₁₄₅N₃OS₆ | 1285.35 | 9.01 | 32.59 | 1636 | C₉₂H₁₈₇N₃O₁₀S₃ | 1591.72 | 9.01 | 39.68 |
| 1437 | C₉₅H₁₈₇N₃OS₆ | 1579.92 | 9.01 | 43.7 | 1637 | C₆₅H₁₃₃N₃O₁₀S₃ | 1212.99 | 9.01 | 25.39 |
| 1438 | C₆₈H₁₃₃N₃OS₆ | 1201.19 | 9.01 | 29.41 | 1638 | C₆₅H₁₃₃N₃O₁₀S₃ | 1212.99 | 9.01 | 25.39 |
| 1439 | C₆₈H₁₃₃N₃OS₆ | 1201.19 | 9.01 | 29.41 | 1639 | C₉₈H₁₉₉N₃O₁₀S₃ | 1675.88 | 9.01 | 43.12 |
| 1440 | C₁₀₁H₁₉₉N₃OS₆ | 1664.08 | 9.01 | 47.14 | 1640 | C₉₂H₁₈₇N₃O₁₀S₃ | 1591.72 | 9.01 | 39.95 |
| 1441 | C₉₅H₁₈₇N₃OS₆ | 1579.92 | 9.01 | 43.97 | 1641 | C₈₃H₁₆₉N₃O₁₀S₃ | 1465.48 | 9.01 | 35.19 |
| 1442 | C₈₆H₁₆₉N₃OS₆ | 1453.68 | 9.01 | 39.2 | 1642 | C₆₅H₁₂₇N₃O₁₀S₃ | 1206.94 | 9.01 | 24.15 |
| 1443 | C₆₈H₁₂₇N₃OS₆ | 1195.14 | 9.01 | 28.17 | 1643 | C₆₅H₁₂₇N₃O₁₀S₃ | 1206.94 | 9.01 | 24.06 |
| 1444 | C₆₈H₁₂₇N₃OS₆ | 1195.14 | 9.01 | 28.08 | 1644 | C₄₄H₉₁N₃O₁₀S₃ | 918.42 | 9.01 | 14.95 |
| 1445 | C₄₇H₉₁N₃OS₆ | 906.62 | 9.01 | 18.96 | 1645 | C₅₆H₁₀₃N₃O₁₀S₃ | 1074.65 | 9.01 | 18.39 |
| 1446 | C₅₉H₁₀₃N₃OS₆ | 1062.85 | 9.01 | 22.41 | 1646 | C₆₂H₁₁₅N₃O₁₀S₃ | 1158.81 | 9.01 | 20.39 |
| 1447 | C₆₅H₁₁₅N₃OS₆ | 1147.01 | 9.01 | 24.41 | 1647 | C₅₀H₉₇N₃O₁₀S₃ | 996.54 | 9.01 | 16.67 |
| 1448 | C₅₃H₉₇N₃OS₆ | 984.74 | 9.01 | 20.69 | 1648 | C₆₂H₁₂₁N₃O₁₀S₃ | 1164.86 | 9.01 | 23.02 |
| 1449 | C₆₅H₁₂₁N₃OS₆ | 1153.06 | 9.01 | 27.03 | 1649 | C₆₂H₁₂₇N₃O₁₀S₃ | 1170.91 | 9.01 | 24.47 |
| 1450 | C₆₅H₁₂₇N₃OS₆ | 1159.11 | 9.01 | 28.49 | 1650 | C₆₈H₁₁₅N₃O₁₀S₃ | 1230.88 | 9.01 | 23.31 |
| 1451 | C₇₁H₁₁₅N₃OS₆ | 1219.08 | 9.01 | 27.44 | 1651 | C₈₃H₁₄₅N₃O₁₀S₃ | 1441.28 | 9.01 | 31.01 |
| 1452 | C₈₆H₁₄₅N₃OS₆ | 1429.49 | 9.01 | 35.13 | 1652 | C₆₀H₁₂₃N₃O₁₃S₃ | 1190.85 | 9.01 | 23.97 |
| 1453 | C₆₂H₁₂₁N₃O₄S₃ | 1068.85 | 9.01 | 25.13 | 1653 | C₇₂H₁₄₇N₃O₁₃S₃ | 1359.18 | 9.01 | 30.32 |
| 1454 | C₇₄H_{14S}N₃O₄S₃ | 1237.17 | 9.01 | 31.48 | 1654 | C₉₃H₁₈₉N₃O₁₃S₃ | 1653.74 | 9.01 | 41.43 |
| 1455 | C₉₅H₁₃₇N₃O₄₈S₃ | 1531.74 | 9.01 | 42.59 | 1655 | C₆₆H₁₃₅N₃O₁₃S₃ | 1275.01 | 9.01 | 27.15 |
| 1456 | C₆₈H₁₃₃N₃O₄S₃ | 1153.01 | 9.01 | 28.3 | 1656 | C₆₆H₁₃₅N₃O₁₃S₃ | 1275.01 | 9.01 | 27.15 |
| 1457 | C₆₈H₁₃₃N₃O₄S₃ | 1153.01 | 9.01 | 28.3 | 1657 | C₉₉H₂₀₁N₃O₁₃S₃ | 1737.91 | 9.01 | 44.78 |
| 1458 | C₁₀₁H₁₉₉N₃O₄S₃ | 1615.9 | 9.01 | 46.03 | 1658 | C₉₃H₁₈₉N₃O₁₃S₃ | 1653.74 | 9.01 | 41.61 |
| 1459 | C₉₅H₁₈₇N₃O₄S₃ | 1531.74 | 9.01 | 42.86 | 1659 | C₈₄H₁₇₁N₃O₁₃S₃ | 1527.5 | 9.01 | 36.85 |
| 1460 | C₈₆H₁₆₉N₃O₄S₃ | 1405.5 | 9.01 | 38.09 | 1660 | C₆₅H₁₂₇N₃O₁₃S₃ | 1254.94 | 9.01 | 25.28 |
| 1461 | C₆₈H₁₂₇N₃O₄S₃ | 1146.96 | 9.01 | 27.06 | 1661 | C₆₅H₁₂₇N₃O₁₃S₃ | 1254.94 | 9.01 | 25.19 |
| 1462 | C₆₈H₁₂₇N₃O₄S₃ | 1146.96 | 9.01 | 26.97 | 1662 | C₄₅H₉₃N₃O₁₃S₃ | 980.45 | 9.01 | 16.61 |
| 1463 | C₄₇H₉₁N₃O₄S₃ | 858.44 | 9.01 | 17.85 | 1663 | C₅₆H₁₀₃N₃O₁₃S₃ | 1122.65 | 9.01 | 19.52 |
| 1464 | C₅₉H₁₀₃N₃O₄S₃ | 1014.67 | 9.01 | 21.3 | 1664 | C₆₂H₁₁₅N₃O₁₃S₃ | 1206.81 | 9.01 | 21.53 |
| 1465 | C₆₅H₁₁₅N₃O₄S₃ | 1098.83 | 9.01 | 23.3 | 1665 | C₅₀H₉₇N₃O₁₃S₃ | 1044.53 | 9.01 | 17.8 |
| 1466 | C₅₃H₉₇N₃O₄S₃ | 936.56 | 9.01 | 19.58 | 1666 | C₆₂H₁₂₁N₃O₁₃S₃ | 1212.86 | 9.01 | 24.15 |
| 1467 | C₆₅H₁₂₁N₃O₄S₃ | 1104.88 | 9.01 | 25.92 | 1667 | C₆₂H₁₂₇N₃O₁₃S₃ | 1218.91 | 9.01 | 25.6 |
| 1468 | C₆₅H₁₂₇N₃O₄S₃ | 1110.93 | 9.01 | 27.38 | 1668 | C₆₈H₁₁₅N₃O₁₃S₃ | 1278.88 | 9.01 | 25.8 |
| 1469 | C₇₁H₁₁₅N₃O₄S₃ | 1170.9 | 9.01 | 26.31 | 1669 | C₈₃H₁₄₅N₃O₁₃S₃ | 1489.28 | 9.01 | 33.49 |
| 1470 | C₈₆H_{14S}N₃O₄S₃ | 1381.3 | 9.01 | 34 | 1670 | C₅₉H₁₂₁N₃O₇S₃ | 1080.83 | 9.01 | 19.96 |
| 1471 | C₆₂H₁₂₁N₃O₄S₃ | 1068.85 | 9.01 | 25.13 | 1671 | C₅₉H₁₂₁N₃O₇S₃ | 1080.83 | 9.01 | 19.96 |
| 1472 | C₆₂H₁₂₁N₃O₄S₃ | 1068.85 | 9.01 | 25.13 | 1672 | C₇₁H₁₄₅N₃O₇S₃ | 1249.16 | 9.01 | 26.3 |
| 1473 | C₇₄H₁₄₅N₃O₄S₃ | 1237.17 | 9.01 | 31.48 | 1673 | C₉₂H₁₈₇N₃O₇S₃ | 1543.72 | 9.01 | 37.41 |
| 1474 | C₉₅H₁₈₇N₃O₄S₃ | 1531.74 | 9.01 | 42.59 | 1674 | C₆₅H₁₃₃N₃O₇S₃ | 1164.99 | 9.01 | 23.13 |
| 1475 | C₆₈H₁₃₃N₃O₄S₃ | 1153.01 | 9.01 | 28.3 | 1675 | C₆₅H₁₃₃N₃O₇S₃ | 1164.99 | 9.01 | 23.13 |
| 1476 | C₆₈H₁₃₃N₃O₄S₃ | 1153.01 | 9.01 | 28.3 | 1676 | C₉₈H₁₉₉N₃o₇S₃ | 1627.88 | 9.01 | 40.86 |
| 1477 | C₁₀₁H₁₉₉N₃O₄S₃ | 1615.9 | 9.01 | 46.03 | 1677 | C₉₂H₁₈₇N₃O₇S₃ | 1543.72 | 9.01 | 37.68 |
| 1478 | C₉₅H₁₈₇N₃O₄S₃ | 1531.74 | 9.01 | 42.86 | 1678 | C₈₃H₁₆₉N₃O₇S₃ | 1417.48 | 9.01 | 32.92 |
| 1479 | C₈₆H₁₆₉N₃O₄S₃ | 1405.5 | 9.01 | 38.09 | 1679 | C₆₅H₁₂₇N₃O₇S₃ | 1158.95 | 9.01 | 21.89 |
| 1480 | C₆₈H₁₂₇N₃O₄S₃ | 1146.96 | 9.01 | 27.06 | 1680 | C₆₅H₁₂₇N₃O₇S₃ | 1158.95 | 9.01 | 21.8 |
| 1481 | C₆₈H₁₂₇N₃O₆S | 1114.84 | 9.01 | 26.01 | 1681 | C₄₄H₉₁N₃O₇S₃ | 870.43 | 9.01 | 12.68 |
| 1482 | C₄₇H₉₁N₃O₄S₃ | 858.46 | 9.01 | 17.85 | 1682 | C₅₆H₁₀₃N₃O₇S₃ | 1026.65 | 9.01 | 16.12 |
| 1483 | C₅₉H₁₀₃N₃O₄S₃ | 1014.69 | 9.01 | 21.3 | 1683 | C₆₂H₁₁₅N₃O₇S₃ | 1110.82 | 9.01 | 18.13 |
| 1484 | C₆₅H₁₁₅N₃O₄S₃ | 1098.85 | 9.01 | 23.3 | 1684 | C₅₀H₉₇N₃O₇S₃ | 948.54 | 9.01 | 14.4 |
| 1485 | C₅₃H₉₇N₃O₄S₃ | 936.58 | 9.01 | 19.58 | 1685 | C₆₂H₁₂₁N₃O₇S₃ | 1116.86 | 9.01 | 20.75 |
| 1486 | C₆₅H₁₂₁N₃O₄S₃ | 1104.9 | 9.01 | 25.92 | 1686 | C₆₂H₁₂₇N₃O₇S₃ | 1122.91 | 9.01 | 22.2 |
| 1487 | C₆₅H₁₂₇N₃O₄S₃ | 1110.95 | 9.01 | 27.38 | 1687 | C₆₈H₁₁₅N₃O₇S₃ | 1182.88 | 9.01 | 22.6 |
| 1488 | C₇₁H₁₁₅N₃O₄S₃ | 1170.92 | 9.01 | 26.44 | 1688 | C₈₃H₁₄₅N₃O₇S₃ | 1393.29 | 9.01 | 30.3 |
| 1489 | C₈₆H₁₄₅N₃O₄S₃ | 1381.32 | 9.01 | 34.14 | 1689 | C₆₄H₁₃₂N₂O₄ | 993.77 | 9.45 | 26.47 |
| 1490 | C₆₂H₁₂₁N₃O₇S₃ | 1116.86 | 9.01 | 26.05 | 1690 | C₆₄H₁₃₂N₂O₄ | 993.77 | 9.45 | 26.47 |
| 1491 | C₇₄H₁₄₅N₃O₇S₃ | 1285.19 | 9.01 | 32.39 | 1691 | C₆₇H₁₃₂N₂O₇ | 1077.8 | 9.45 | 27.29 |
| 1492 | C₉₅H₁₈₇N₃O₇S₃ | 1579.76 | 9.01 | 43.5 | 1692 | C₆₇H₁₃₂N₂O₇ | 1077.8 | 9.45 | 27.29 |
| 1493 | C₆₈H₁₃₃N₃O₇S₃ | 1201.03 | 9.01 | 29.22 | 1693 | C₆₇H₁₃₂N₂O₇ | 1077.8 | 9.46 | 27.29 |
| 1494 | C₆₈H₁₃₃N₃O₇S₃ | 1201.03 | 9.01 | 29.22 | 1694 | C₆₇H₁₃₂N₂O₇ | 1077.8 | 9.46 | 27.29 |
| 1495 | C₁₀₁H₁₉₉N₃O₇S₃ | 1663.92 | 9.01 | 46.95 | 1695 | C₆₄H₁₃₂N₂O₃S | 1009.83 | 9.8 | 26.96 |
| 1496 | C₉₅H₁₈₇N₃O₇S₃ | 1579.76 | 9.01 | 43.77 | 1696 | C₆₄H₁₃₂N₂O₃S | 1009.83 | 9.8 | 26.96 |
| 1497 | C₈₆H₁₆₉N₃O₇S₃ | 1453.51 | 9.01 | 39.01 | 1697 | C₆₇H₁₃₂N₂O₆S | 1093.86 | 9.79 | 27.78 |
| 1498 | C₆₈H₁₂₇N₃O₇S₃ | 1194.98 | 9.01 | 27.98 | 1698 | C₆₇H₁₃₂N₂O₆S | 1093.86 | 9.79 | 27.78 |
| 1499 | C₆₈H₁₂₇N₃O₇S₃ | 1194.98 | 9.01 | 27.89 | 1699 | C₆₇H₁₃₂N₂O₆S | 1093.86 | 9.81 | 27.78 |
| 1500 | C₄₇H₉₁N₃O₇S₃ | 906.46 | 9.01 | 18.77 | 1700 | C₆₇H₁₃₂N₂O₆S | 1093.86 | 9.81 | 27.78 |
| 1501 | C₅₉H₁₀₃N₃O₇S₃ | 1062.69 | 9.01 | 22.21 | 1701 | C₆₄H₁₃₃N₃O₃ | 992.79 | 9.78 | 26.31 |
| 1502 | C₆₅H₁₁₅N₃O₇S₃ | 1146.85 | 9.01 | 24.22 | 1702 | C₆₄H₁₃₃N₃O₃ | 992.79 | 9.78 | 26.31 |
| 1503 | C₅₃H₉₇N₃O₇S₃ | 984.57 | 9.01 | 20.49 | 1703 | C₆₇H₁₃₃N₃O₆ | 1076.82 | 9.78 | 27.13 |
| 1504 | C₆₅H₁₂₁N₃O₇S₃ | 1152.9 | 9.01 | 26.84 | 1704 | C₆₇H₁₃₃N₃O₆ | 1076.82 | 9.78 | 27.13 |
| 1505 | C₆₅H₁₂₇N₃O₇S₃ | 1158.95 | 9.01 | 28.29 | 1705 | C₆₇H₁₃₃N₃O₆ | 1076.82 | 9.79 | 27.13 |
| 1506 | C₇₁H₁₁₅N₃O₇S₃ | 1218.92 | 9.01 | 27.44 | 1706 | C₆₇H₁₃₃N₃O₆ | 1076.82 | 9.79 | 27.13 |
| 1507 | C₈₆H₁₄₅N₃O₇S₃ | 1429.32 | 9.01 | 35.13 | 1707 | C₆₄H₁₃₂N₂O₃S₂ | 1041.89 | 9.65 | 27.39 |
| 1508 | C₆₂H₁₂₁N₃O₇S₃ | 1116.86 | 9.01 | 26.05 | 1708 | C₆₄H₁₃₂N₂O₃S₂ | 1041.89 | 9.65 | 27.39 |
| 1509 | C₇₄H₁₄₅N₃O₇S₃ | 1285.19 | 9.01 | 32.39 | 1709 | C₆₇H₁₃₂N₂O₆S₂ | 1125.92 | 9.65 | 28.21 |
| 1510 | C₉₅H₁₈₇N₃O₇S₃ | 1579.76 | 9.01 | 43.5 | 1710 | C₆₇H₁₃₂N₂O₆S₂ | 1125.92 | 9.65 | 28.21 |
| 1511 | C₆₈H₁₃₃N₃O₇S₃ | 1201.03 | 9.01 | 29.22 | 1711 | C₆₇H₁₃₂N₂O₆S₂ | 1125.92 | 9.65 | 28.21 |
| 1512 | C₆₈H₁₃₃N₃O₇S₃ | 1201.03 | 9.01 | 29.22 | 1712 | C₆₇H₁₃₂N₂O₆S₂ | 1125.92 | 9.65 | 28.21 |
| 1513 | C₁₀₁H₁₉₉N₃O₇S₃ | 1663.92 | 9.01 | 46.95 | 1713 | C₆₅H₁₃₂N₂O₄ | 1005.78 | 8.95 | 26.46 |
| 1514 | C₉₅H₁₈₇N₃O₇S₃ | 1579.76 | 9.01 | 43.77 | 1714 | C₆₅H₁₃₂N₂O₄ | 1005.78 | 8.95 | 26.46 |
| 1515 | C₈₆H₁₆₉N₃O₇S₃ | 1453.51 | 9.01 | 39.01 | 1715 | C₆₈H₁₃₂N₂O₇ | 1089.81 | 8.95 | 27.28 |
| 1516 | C₆₈H₁₂₇N₃O₇S₃ | 1194.98 | 9.01 | 27.98 | 1716 | C₆₈H₁₃₂N₂O₇ | 1089.81 | 8.95 | 27.28 |
| 1517 | C₆₈H₁₂₇N₃O₇S₃ | 1194.98 | 9.01 | 27.89 | 1717 | C₆₈H₁₃₂N₂O₇ | 1089.81 | 8.96 | 27.28 |
| 1518 | C₄₇H₉₁N₃O₇S₃ | 906.46 | 9.01 | 18.77 | 1718 | C₆₈H₁₃₂N₂O₇ | 1089.81 | 8.96 | 27.28 |
| 1519 | C₅₉H₁₀₃N₃O₇S₃ | 1062.69 | 9.01 | 22.21 | 1719 | C₆₅H₁₃₂N₂O₅ | 1021.78 | 8.83 | 26.64 |
| 1520 | C₆₅H₁₁₅N₃O₇S₃ | 1146.85 | 9.01 | 24.22 | 1720 | C₆₅H₁₃₂N₂O₅ | 1021.78 | 8.83 | 26.64 |
| 1521 | C₅₃H₉₇N₃O₇S₃ | 984.57 | 9.01 | 20.49 | 1721 | C₆₈H₁₃₂N₂O₈ | 1105.81 | 8.83 | 27.46 |
| 1522 | C₆₅H₁₂₁N₃O₇S₃ | 1152.9 | 9.01 | 26.84 | 1722 | C₆₈H₁₃₂N₂O₈ | 1105.81 | 8.83 | 27.46 |
| 1523 | C₆₅H₁₂₇N₃O₇S₃ | 1158.95 | 9.01 | 28.29 | 1723 | C₆₈H₁₃₂N₂O₈ | 1105.81 | 8.83 | 27.46 |
| 1524 | C₇₁H₁₁₅N₃O₇S₃ | 1218.92 | 9.01 | 25.64 | 1724 | C₆₈H₁₃₂N₂O₈ | 1105.81 | 8.83 | 27.46 |
| 1525 | C₈₆H₁₄₅N₃O₇S₃ | 1429.32 | 9.01 | 33.34 | 1725 | C₆₅H₁₃₂N₂O₅ | 1021.78 | 8.67 | 26.64 |
| 1526 | C₆₂H₁₂₁N₃O₄S₆ | 1165.07 | 9.01 | 27.16 | 1726 | C₆₅H₁₃₂N₂O₅ | 1021.78 | 8.67 | 26.64 |
| 1527 | C₇₄H₁₄₅N₃O₄S₆ | 1333.39 | 9.01 | 33.5 | 1727 | C₆₈H₁₃₂N₂O₈ | 1105.81 | 8.67 | 27.46 |
| 1528 | C₉₅H₁₈₇N₃O₄S₆ | 1627.96 | 9.01 | 44.61 | 1728 | C₆₈H₁₃₂N₂O₈ | 1105.81 | 8.67 | 27.46 |
| 1529 | C₆₈H₁₃₃N₃O₄S₆ | 1249.23 | 9.01 | 30.33 | 1729 | C₆₈H₁₃₂N₂O₈ | 1105.81 | 8.67 | 27.46 |
| 1530 | C₆₈H₁₃₃N₃O₄S₆ | 1249.23 | 9.01 | 30.33 | 1730 | C₆₈H₁₃₂N₂O₈ | 1105.81 | 8.67 | 27.46 |
| 1531 | C₁₀₁H₁₉₉N₃O₄S₆ | 1712.12 | 9.01 | 48.06 | 1731 | C₆₅H₁₃₂N₂O₆ | 1037.78 | 8.66 | 26.6 |
| 1532 | C₉₅H₁₈₇N₃O₄S₆ | 1627.96 | 9.01 | 44.88 | 1732 | C₆₅H₁₃₂N₂O₆ | 1037.78 | 8.66 | 26.6 |
| 1533 | C₈₆H₁₆₉N₃O₄S₆ | 1501.72 | 9.01 | 40.12 | 1733 | C₆₈H₁₃₂N₂O₉ | 1121.81 | 8.66 | 27.43 |
| 1534 | C₆₈H₁₂₇N₃O₄S₆ | 1243.18 | 9.01 | 29.09 | 1734 | C₆₈H₁₃₂N₂O₉ | 1121.81 | 8.66 | 27.43 |
| 1535 | C₆₈H₁₂₇N₃O₄S₆ | 1243.18 | 9.01 | 29 | 1735 | C₆₈H₁₃₂N₂O₉ | 1121.81 | 8.66 | 27.43 |
| 1536 | C₄₇H₉₁N₃O₄S₆ | 954.66 | 9.01 | 19.88 | 1736 | C₆₈H₁₃₂N₂O₉ | 1121.81 | 8.66 | 27.43 |
| 1537 | C₅₉H₁₀₃N₃O₄S₆ | 1110.89 | 9.01 | 23.32 | 1737 | C₆₅H₁₃₃N₃O₄ | 1020.8 | 8.84 | 26.04 |
| 1538 | C₆₅H₁₁₅N₃O₄S₆ | 1195.05 | 9.01 | 25.33 | 1738 | C₆₅H₁₃₃N₃O₄ | 1020.8 | 8.84 | 26.04 |
| 1539 | C₅₃H₉₇N₃O₄S₆ | 1032.78 | 9.01 | 21.6 | 1739 | C₆₈H₁₃₃N₃O₇ | 1104.83 | 8.83 | 26.86 |
| 1540 | C₆₅H₁₂₁N₃O₄S₆ | 1201.1 | 9.01 | 27.95 | 1740 | C₆₈H₁₃₃N₃O₇ | 1104.83 | 8.84 | 26.86 |
| 1541 | C₆₅H₁₂₇N₃O₄S₆ | 1207.15 | 9.01 | 29.4 | 1741 | C₆₈H₁₃₃N₃O₇ | 1104.83 | 8.84 | 26.86 |
| 1542 | C₇₁H₁₁₅N₃O₄S₆ | 1267.12 | 9.01 | 28.55 | 1742 | C₆₈H₁₃₃N₃O₇ | 1104.83 | 8.84 | 26.86 |
| 1543 | C₈₆H₁₄₅N₃O₄S₆ | 1477.52 | 9.01 | 36.24 | 1743 | C₆₅H₁₃₃N₃O₅ | 1036.79 | 8.85 | 26.48 |
| 1544 | C₆₅H₁₃₆N₃O₁₀P₃ | 1212.74 | 9.01 | 21.98 | 1744 | C₆₅H₁₃₃N₃O₅ | 1036.79 | 8.85 | 26.48 |
| 1545 | C₇₇H₁₆₀N₃O₁₀P₃ | 1381.06 | 9.01 | 28.33 | 1745 | C₆₈H₁₃₃N₃O₈ | 1120.82 | 8.85 | 27.3 |
| 1546 | C₉₈H₂₀₂N₃O₁₀P₃ | 1675.63 | 9.01 | 39.44 | 1746 | C₆₈H₁₃₃N₃O₈ | 1120.82 | 8.85 | 27.3 |
| 1547 | C₇₁H₁₄₈N₃O₁₀P₃ | 1296.9 | 9.01 | 25.16 | 1747 | C₆₈H₁₃₃N₃O₈ | 1120.82 | 8.85 | 27.3 |
| 1548 | C₇₁H₁₄₈N₃O₁₀P₃ | 1296.9 | 9.01 | 25.16 | 1748 | C₆₈H₁₃₃N₃O₈ | 1120.82 | 8.85 | 27.3 |
| 1549 | C₁₀₄H₂₁₄N₃O₁₀P ₃ | 1759.79 | 9.01 | 42.88 | 1749 | C₆₅H₁₃₃N₃O₅ | 1036.79 | 8.75 | 26.48 |
| 1550 | C₉₈H₂₀₂N₃O₁₀P₃ | 1675.63 | 9.01 | 39.71 | 1750 | C₆₅H₁₃₃N₃O₅ | 1036.79 | 8.75 | 26.48 |
| 1551 | C₈₉H₁₈₄N₃O₁₀P₃ | 1549.38 | 9.01 | 34.95 | 1751 | C₆₈H₁₃₃N₃O₈ | 1120.82 | 8.75 | 27.3 |
| 1552 | C₇₁H₁₄₂N₃O₁₀P₃ | 1290.85 | 9.01 | 23.91 | 1752 | C₆₈H₁₃₃N₃O₈ | 1120.82 | 8.75 | 27.3 |
| 1553 | C₇₁H₁₄₂N₃O₁₀P₃ | 1290.85 | 9.01 | 23.82 | 1753 | C₆₈H₁₃₃N₃O₈ | 1120.82 | 8.75 | 27.3 |
| 1554 | C₅₀H₁₀₆N₃O₁₀P₃ | 1002.33 | 9.01 | 14.71 | 1754 | C₆₈H₁₃₃N₃O₈ | 1120.82 | 8.75 | 27.3 |
| 1555 | C₆₂H₁₁₈N₃O₁₀P₃ | 1158.56 | 9.01 | 18.15 | 1755 | C₆₅H₁₃₄N₄O₄ | 1035.81 | 8.95 | 26.29 |
| 1556 | C₆₈H₁₃₀N₃O₁₀P₃ | 1242.72 | 9.01 | 20.16 | 1756 | C₆₅H₁₃₄N₄O₄ | 1035.81 | 8.95 | 26.29 |
| 1557 | C₅₆H₁₁₂N₃O₁₀P₃ | 1080.44 | 9.01 | 16.43 | 1757 | C₆₈H₁₃₄N₄O₇ | 1119.84 | 8.95 | 27.11 |
| 1558 | C₆₈H₁₃₆N₃O₁₀P₃ | 1248.77 | 9.01 | 22.78 | 1758 | C₆₈H₁₃₄N₄O₇ | 1119.84 | 8.95 | 27.11 |
| 1559 | C₆₈H₁₄₂N₃O₁₀P₃ | 1254.82 | 9.01 | 24.23 | 1759 | C₆₈H₁₃₄N₄O₇ | 1119.84 | 8.95 | 27.11 |
| 1560 | C₇₄H₁₃₀N₃O₁₀P₃ | 1314.79 | 9.01 | 23.74 | 1760 | C₆₈H₁₃₄N₄O₇ | 1119.84 | 8.95 | 27.11 |
| 1561 | C₈₉H₁₆₀N₃O₁₀P₃ | 1525.19 | 9.01 | 31.43 | 1761 | C₆₅H₁₃₂N₂O₃S | 1021.84 | 9.29 | 26.71 |
| 1562 | C₆₅H₁₃₆N₃O₁₀P₃ | 1212.74 | 9.01 | 21.98 | 1762 | C₆₅H₁₃₂N₂O₃S | 1021.84 | 9.29 | 26.71 |
| 1563 | C₇₇H₁₆₀N₃O₁₀P₃ | 1381.06 | 9.01 | 28.33 | 1763 | C₆₈H₁₃₂N₂O₆S | 1105.87 | 9.29 | 27.53 |
| 1564 | C₉₈H₂₀₂N₃O₁₀P₃ | 1675.63 | 9.01 | 39.44 | 1764 | C₆₈H₁₃₂N₂O₆S | 1105.87 | 9.29 | 27.53 |
| 1565 | C₇₁H₁₄₈N₃O₁₀P₃ | 1296.9 | 9.01 | 25.16 | 1765 | C₆₈H₁₃₂N₂O₆S | 1105.87 | 9.31 | 27.53 |
| 1566 | C₇₁H₁₄₈N₃O₁₀P₃ | 1296.9 | 9.01 | 25.16 | 1766 | C₆₈H₁₃₂N₂O₆S | 1105.87 | 9.31 | 27.53 |
| 1567 | C₁₀₄H₂₁₄N₃O₁₀P ₃ | 1759.79 | 9.01 | 42.88 | 1767 | C₆₅H₁₃₂N₂O₃S₂ | 1053.9 | 9.35 | 27.19 |
| 1568 | C₉₈H₂₀₂N₃O₁₀P₃ | 1675.63 | 9.01 | 39.71 | 1768 | C₆₅H₁₃₂N₂O₃S₂ | 1053.9 | 9.35 | 27.19 |
| 1569 | C₈₉H₁₈₄N₃O₁₀P₃ | 1549.38 | 9.01 | 34.95 | 1769 | C₆₈H₁₃₂N₂O₆S₂ | 1137.93 | 9.35 | 28.02 |
| 1570 | C₇₁H₁₄₂N₃O₁₀P₃ | 1290.85 | 9.01 | 23.91 | 1770 | C₆₈H₁₃₂N₂O₆S₂ | 1137.93 | 9.35 | 28.02 |
| 1571 | C₇₁H₁₄₂N₃O₁₀P₃ | 1290.85 | 9.01 | 23.82 | 1771 | C₆₈H₁₃₂N₂O₆S₂ | 1137.93 | 9.35 | 28.02 |
| 1572 | C₅₀H₁₀₆N₃O₁₀P₃ | 1002.33 | 9.01 | 14.71 | 1772 | C₆₈H₁₃₂N₂O₆S₂ | 1137.93 | 9.35 | 28.02 |
| 1573 | C₆₂H₁₁₈N₃O₁₀P₃ | 1158.56 | 9.01 | 18.15 | 1773 | C₆₅H₁₃₂N₂O₃S₂ | 1053.9 | 9.13 | 27.19 |
| 1574 | C₆₈H₁₃₀N₃O₁₀P₃ | 1242.72 | 9.01 | 20.16 | 1774 | C₆₅H₁₃₂N₂O₃S₂ | 1053.9 | 9.13 | 27.19 |
| 1575 | C₅₆H₁₁₂N₃O₁₀P₃ | 1080.44 | 9.01 | 16.43 | 1775 | C₆₈H₁₃₂N₂O₆S₂ | 1137.93 | 9.13 | 28.02 |
| 1576 | C₆₈H₁₃₆N₃O₁₀P₃ | 1248.77 | 9.01 | 22.78 | 1776 | C₆₈H₁₃₂N₂O₆S₂ | 1137.93 | 9.13 | 28.02 |
| 1577 | C₆₈H₁₄₂N₃O₁₀P₃ | 1254.82 | 9.01 | 24.23 | 1777 | C₆₈H₁₃₂N₂O₆S₂ | 1137.93 | 9.14 | 28.02 |
| 1578 | C₇₄H₁₃₀N₃O₁₀P₃ | 1314.79 | 9.01 | 24.34 | 1778 | C₆₈H₁₃₂N₂O₆S₂ | 1137.93 | 9.14 | 28.02 |
| 1579 | C₈₉H₁₆₀N₃O₁₀P₃ | 1525.19 | 9.01 | 32.03 | 1779 | C₆₅H₁₃₂N₂O₄S | 1037.84 | 9.2 | 26.95 |
| 1580 | C₅₉H₁₂₄N₆O₇S₃ | 1125.88 | 9.01 | 21.35 | 1780 | C₆₅H₁₃₂N₂O₄S | 1037.84 | 9.2 | 26.95 |
| 1581 | C₇₁H₁₄₈N₆O₇S₃ | 1294.2 | 9.01 | 27.7 | 1781 | C₆₈H₁₃₂N₂O₇S | 1121.87 | 9.2 | 27.78 |
| 1582 | C₉₂H₁₉₀N₆O₇S₃ | 1588.77 | 9.01 | 38.81 | 1782 | C₆₈H₁₃₂N₂O₇S | 1121.87 | 9.2 | 27.78 |
| 1583 | C₆₅H₁₃₆N₆O₇S₃ | 1210.04 | 9.01 | 24.53 | 1783 | C₆₈H₁₃₂N₂O₇S | 1121.87 | 9.2 | 27.78 |
| 1584 | C₆₅H₁₃₆N₆O₇S₃ | 1210.04 | 9.01 | 24.53 | 1784 | C₆₈H₁₃₂N₂O₇S | 1121.87 | 9.2 | 27.78 |
| 1585 | C₉₈H₂₀₂N₆O₇S₃ | 1672.93 | 9.01 | 42.26 | 1785 | C₆₅H₁₃₂N₂O₄S | 1037.84 | 8.8 | 26.95 |
| 1586 | C₉₂H₁₉₀N₆O₇S₃ | 1588.77 | 9.01 | 39.08 | 1786 | C₆₅H₁₃₂N₂O₄S | 1037.84 | 8.8 | 26.95 |
| 1587 | C₈₃H₁₇₂N₆O₇S₃ | 1462.52 | 9.01 | 34.32 | 1787 | C₆₈H₁₃₂N₂O₇S | 1121.87 | 8.8 | 27.78 |
| 1588 | C₆₅H₁₃₀N₆O₇S₃ | 1203.99 | 9.01 | 23.29 | 1788 | C₆₈H₁₃₂N₂O₇S | 1121.87 | 8.8 | 27.78 |
| 1589 | C₆₅H₁₃₀N₆O₇S₃ | 1203.99 | 9.01 | 23.2 | 1789 | C₆₈H₁₃₂N₂O₇S | 1121.87 | 8.8 | 27.78 |
| 1590 | C₄₄H₉₄N₆O₇S₃ | 915.47 | 9.01 | 14.08 | 1790 | C₆₈H₁₃₂N₂O₇S | 1121.87 | 8.8 | 27.78 |
| 1591 | C₅₆H₁₀₆N₆O₇S₃ | 1071.7 | 9.01 | 17.52 | 1791 | C₆₅H₁₃₂N₂O₅S | 1053.84 | 8.7 | 27.15 |
| 1592 | C₆₂H₁₁₈N₆O₇S₃ | 1155.86 | 9.01 | 19.53 | 1792 | C₆₅H₁₃₂N₂O₅S | 1053.84 | 8.7 | 27.15 |
| 1593 | C₅₀H₁₀₀N₆O₇S₃ | 993.59 | 9.01 | 15.8 | 1793 | C₆₈H₁₃₂N₂O₈S | 1137.87 | 8.7 | 27.97 |
| 1594 | C₆₂H₁₂₄N₆O₇S₃ | 1161.91 | 9.01 | 22.15 | 1794 | C₆₈H₁₃₂N₂O₈S | 1137.87 | 8.7 | 27.97 |
| 1595 | C₆₂H₁₃₀N₆O₇S₃ | 1167.96 | 9.01 | 23.6 | 1795 | C₆₈H₁₃₂N₂O₈S | 1137.87 | 8.7 | 27.97 |
| 1596 | C₆₈H₁₁₈N₆O₇S₃ | 1227.93 | 9.01 | 23.53 | 1796 | C₆₈H₁₃₂N₂O₈S | 1137.87 | 8.7 | 27.97 |
| 1597 | C₈₃H₁₄₈N₆O₇S₃ | 1438.33 | 9.01 | 31.23 | 1797 | C₆₅H₁₃₂N₂O₅S | 1053.84 | 9 | 27.15 |
| 1598 | C₅₉H₁₂₄N₆O₇S₃ | 1125.88 | 9.01 | 21.35 | 1798 | C₆₅H₁₃₂N₂O₅S | 1053.84 | 9 | 27.15 |
| 1599 | C₇₁H₁₄₈N₆O₇S₃ | 1294.2 | 9.01 | 27.7 | 1799 | C₆₈H₁₃₂N₂O₈S | 1137.87 | 9 | 27.97 |
| 1600 | C₉₂H₁₉₀N₆O₇S₃ | 1588.77 | 9.01 | 38.81 | 1800 | C₆₈H₁₃₂N₂O₈S | 1137.87 | 9 | 27.97 |
| 1801 | C₆₈H₁₃₂N₂O₈S | 1137.87 | 9 | 27.97 | 2001 | C₆₉H₁₃₇N₅O₅ | 1116.88 | 7.65 | 24.63 |
| 1802 | C₆₈H₁₃₂N₂O₈S | 1137.87 | 9 | 27.97 | 2002 | C₆₉H₁₃₇N₅O₅ | 1116.88 | 7.65 | 24.63 |
| 1803 | C₆₅H₁₃₂N₂O₄S₂ | 1069.9 | 9.06 | 27.39 | 2003 | C₇₂H₁₃₇N₅O₈ | 1200.91 | 7.65 | 25.45 |
| 1804 | C₆₅H₁₃₂N₂O₄S₂ | 1069.9 | 9.06 | 27.39 | 2004 | C₇₂H₁₃₇N₅O₈ | 1200.91 | 7.65 | 25.45 |
| 1805 | C₆₈H₁₃₂N₂O₇S₂ | 1153.93 | 9.06 | 28.21 | 2005 | C₇₂H₁₃₇N₅O₈ | 1200.91 | 7.65 | 25.45 |
| 1806 | C₆₈H₁₃₂N₂O₇S₂ | 1153.93 | 9.06 | 28.21 | 2006 | C₇₂H₁₃₇N₅O₈ | 1200.91 | 7.65 | 25.45 |
| 1807 | C₆₈H₁₃₂N₂O₇S₂ | 1153.93 | 9.06 | 28.21 | 2007 | C₆₉H₁₃₆N₄O₅ | 1101.87 | 7.9 | 25.58 |
| 1808 | C₆₈H₁₃₂N₂O₇S₂ | 1153.93 | 9.06 | 28.21 | 2008 | C₆₉H₁₃₆N₄O₅ | 1101.87 | 7.9 | 25.58 |
| 1809 | C₆₆H₁₃₇N₂O₆P | 1085.8 | 8.79 | 26.39 | 2009 | C₇₂H₁₃₆N₄O₈ | 1185.9 | 7.9 | 26.41 |
| 1810 | C₆₆H₁₃₇N₂O₆P | 1085.8 | 8.79 | 26.39 | 2010 | C₇₂H₁₃₆N₄O₈ | 1185.9 | 7.9 | 26.41 |
| 1811 | C₆₉H₁₃₇N₂O₉P | 1169.83 | 8.79 | 27.21 | 2011 | C₇₂H₁₃₆N₄O₅ | 1185.9 | 7.9 | 26.41 |
| 1812 | C₆₉H₁₃₇N₂O₉P | 1169.83 | 8.79 | 27.21 | 2012 | C₇₂H₁₃₆N₄O₈ | 1185.9 | 7.9 | 26.41 |
| 1813 | C₆₉H₁₃₇N₂O₉P | 1169.83 | 8.79 | 27.21 | 2013 | C₆₉H₁₃₇N₅O₄ | 1100.89 | 8.23 | 25.73 |
| 1814 | C₆₉H₁₃₇N₂O₉P | 1169.83 | 8.79 | 27.21 | 2014 | C₆₉H₁₃₇N₅O₄ | 1100.89 | 8.23 | 25.73 |
| 1815 | C₆₆H₁₃₇N₂O₆P | 1085.8 | 8.41 | 26.39 | 2015 | C₇₂H₁₃₇N₅O₇ | 1184.92 | 8.23 | 26.56 |
| 1816 | C₆₆H₁₃₇N₂O₆P | 1085.8 | 8.41 | 26.39 | 2016 | C₇₂H₁₃₇N₅O₇ | 1184.92 | 8.23 | 26.56 |
| 1817 | C₆₉H₁₃₇N₂O₉P | 1169.83 | 8.41 | 27.21 | 2017 | C₇₂H₁₃₇N₅O₇ | 1184.92 | 8.23 | 26.56 |
| 1818 | C₆₉H₁₃₇N₂O₉P | 1169.83 | 8.41 | 27.21 | 2018 | C₇₂H₁₃₇N₅O₇ | 1184.92 | 8.23 | 26.56 |
| 1819 | C₆₉H₁₃₇N₂O₉P | 1169.83 | 8.41 | 27.21 | 2019 | C₆₉H₁₃₇N₅O₄ | 1100.89 | 7.75 | 26.13 |
| 1820 | C₆₉H₁₃₇N₂O₉P | 1169.83 | 8.41 | 27.21 | 2020 | C₆₉H₁₃₇N₅O₄ | 1100.89 | 7.75 | 26.13 |
| 1821 | C₆₄H₁₃₃N₃O₅S | 1056.84 | 8.74 | 26.14 | 2021 | C₇₂H₁₃₇N₅O₇ | 1184.92 | 7.75 | 26.95 |
| 1822 | C₆₄H₁₃₃N₃O₅S | 1056.84 | 8.74 | 26.14 | 2022 | C₇₂H₁₃₇N₅O₇ | 1184.92 | 7.75 | 26.95 |
| 1823 | C₆₇H₁₃₃N₃O₈S | 1140.87 | 8.74 | 26.96 | 2023 | C₇₂H₁₃₇N₅O₇ | 1184.92 | 7.75 | 26.95 |
| 1824 | C₆₇H₁₃₃N₃O₈S | 1140.87 | 8.74 | 26.96 | 2024 | C₇₂H₁₃₇N₅O₇ | 1184.92 | 7.75 | 26.95 |
| 1825 | C₆₇H₁₃₃N₃O₈S | 1140.87 | 8.74 | 26.96 | 2025 | C₆₈H₁₃₆N₆O₄ | 1101.87 | 7.19 | 25.85 |
| 1826 | C₆₇H₁₃₃N₃O₈S | 1140.87 | 8.74 | 26.96 | 2026 | C₆₈H₁₃₆N₆O₄ | 1101.87 | 7.19 | 25.85 |
| 1827 | C₆₄H₁₃₃N₃O₅S | 1056.84 | 7.78 | 26.14 | 2027 | C₇₁H₁₃₆N₆O₇ | 1185.9 | 7.19 | 26.67 |
| 1828 | C₆₄H₁₃₃N₃O₅S | 1056.84 | 7.78 | 26.14 | 2028 | C₇₁H₁₃₆N₆O₇ | 1185.9 | 7.19 | 26.67 |
| 1829 | C₆₇H₁₃₃N₃O₈S | 1140.87 | 7.78 | 26.96 | 2029 | C₇₁H₁₃₆N₆O₇ | 1185.9 | 7.19 | 26.67 |
| 1830 | C₆₇H₁₃₃N₃O₈S | 1140.87 | 7.78 | 26.96 | 2030 | C₇₁H₁₃₆N₆O₇ | 1185.9 | 7.19 | 26.67 |
| 1831 | C₆₇H₁₃₃N₃O₈S | 1140.87 | 7.78 | 26.96 | 2031 | C₆₉H₁₃₇N₅O₄S | 1132.95 | 7.95 | 27.26 |
| 1832 | C₆₇H₁₃₃N₃O₈S | 1140.87 | 7.78 | 26.96 | 2032 | C₆₉H₁₃₇N₅O₄S | 1132.95 | 7.95 | 27.26 |
| 1833 | C₆₄H₁₃₂N₂O₆S | 1057.83 | 7.6 | 26.33 | 2033 | C₈₉H₁₇₆N₆O₉S | 1506.48 | 7.74 | 33.24 |
| 1834 | C₆₄H₁₃₂N₂O₆S | 1057.83 | 7.6 | 26.33 | 2034 | C₈₈H₁₇₅N₅O₉ | 1447.39 | 8.17 | 35.73 |
| 1835 | C₆₇H₁₃₂N₂O₉S | 1141.86 | 7.6 | 27.15 | 2035 | C₇₂H₁₃₇N₅O₇S | 1216.98 | 7.96 | 28.08 |
| 1836 | C₆₇H₁₃₂N₂O₉S | 1141.86 | 7.6 | 27.15 | 2036 | C₇₂H₁₃₇N₅O₇S | 1216.98 | 7.96 | 28.08 |
| 1837 | C₆₇H₁₃₂N₂O₉S | 1141.86 | 7.6 | 27.15 | 2037 | C₇₁H₁₃₈N₈O₅ | 1183.94 | 7.59 | 24.76 |
| 1838 | C₆₇H₁₃₂N₂O₉S | 1141.86 | 7.6 | 27.15 | 2038 | C₇₁H₁₃₈N₈O₅ | 1183.94 | 7.59 | 24.76 |
| 1839 | C₆₄H₁₃₂N₂O₆S | 1057.83 | 8.32 | 26.33 | 2039 | C₉₃H₁₇₉N₅O₁₁S₃ | 1639.66 | 7.56 | 35.27 |
| 1840 | C₆₄H₁₃₂N₂O₆S | 1057.83 | 8.32 | 26.33 | 2040 | C₉₃H₁₈₀N₅O₁₂P S | 1623.52 | 8.52 | 37.28 |
| 1841 | C₆₇H₁₃₂N₂O₉S | 1141.86 | 8.32 | 27.15 | 2041 | C₇₄H₁₃₈N₃O₈ | 1267.97 | 7.59 | 25.58 |
| 1842 | C₆₇H₁₃₂N₂O₉S | 1141.86 | 8.32 | 27.15 | 2042 | C₇₄H₁₃₈N₈O₈ | 1267.97 | 7.59 | 25.58 |
| 1843 | C₆₇H₁₃₂N₂O₉S | 1141.86 | 8.32 | 27.15 | 2043 | C₆₈H₁₄₀N₆O₄ | 1105.91 | 8.62 | 25.32 |
| 1844 | C₆₇H₁₃₂N₂O₉S | 1141.86 | 8.32 | 27.15 | 2044 | C₆₈H₁₄₀N₆O₄ | 1105.91 | 8.62 | 25.32 |
| 1845 | C₆₄H₁₃₂N₂O₇S | 1073.83 | 8.15 | 26.57 | 2045 | C₇₁H₁₄₀N₆O₇ | 1189.94 | 8.62 | 26.14 |
| 1846 | C₆₄H₁₃₂N₂O₇S | 1073.83 | 8.15 | 26.57 | 2046 | C₇₁H₁₄₀N₆O₇ | 1189.94 | 8.62 | 26.14 |
| 1847 | C₆₇H₁₃₂N₂O₁₀S | 1157.86 | 8.15 | 27.39 | 2047 | C₇₁H₁₄₀N₆O₇ | 1189.94 | 8.62 | 26.14 |
| 1848 | C₆₇H₁₃₂N₂O₁₀S | 1157.86 | 8.15 | 27.39 | 2048 | C₇₁H₁₄₀N₆O₇ | 1189.94 | 8.62 | 26.14 |
| 1849 | C₆₇H₁₃₂N₂O₁₀S | 1157.86 | 8.15 | 27.39 | 2049 | C₆₈H₁₃₉N₅O₄ | 1090.89 | 8.61 | 25.54 |
| 1850 | C₆₇H₁₃₂N₂O₁₀S | 1157.86 | 8.15 | 27.39 | 2050 | C₆₈H₁₃₉N₅O₄ | 1090.89 | 8.61 | 25.54 |
| 1851 | C₆₄H₁₃₂N₂O₅S | 1041.83 | 7.83 | 25.84 | 2051 | C₇₁H₁₃₉N₅O₇ | 1174.92 | 8.61 | 26.36 |
| 1852 | C₆₄H₁₃₂N₂O₅S | 1041.83 | 7.83 | 25.84 | 2052 | C₇₁H₁₃₉N₅O₇ | 1174.92 | 8.61 | 26.36 |
| 1853 | C₆₇H₁₃₂N₂O₈S | 1125.86 | 7.83 | 26.66 | 2053 | C₇₁H₁₃₉N₅O₇ | 1174.92 | 8.61 | 26.36 |
| 1854 | C₆₇H₁₃₂N₂O₈S | 1125.86 | 7.83 | 26.66 | 2054 | C₇₁H₁₃₉N₅O₇ | 1174.92 | 8.61 | 26.36 |
| 1855 | C₆₇H₁₃₂N₂O₈S | 1125.86 | 7.84 | 26.66 | 2055 | C₆₉H₁₃₉N₅O₅ | 1118.9 | 7.82 | 25.64 |
| 1856 | C₆₇H₁₃₂N₂O₈S | 1125.86 | 7.84 | 26.66 | 2056 | C₆₉H₁₃₉N₅O₅ | 1118.9 | 7.82 | 25.64 |
| 1857 | C₆₇H₁₃₇N₃O₄ | 1048.85 | 9.07 | 27.1 | 2057 | C₉₀H₁₇₆N₂OS₈ | 1558.89 | 9.96 | 42.56 |
| 1858 | C₆₇H₁₃₇N₃O₄ | 1048.85 | 9.07 | 7.1 | 2058 | C₈₉H₇₄N₂OS₈ | 1544.86 | 8.77 | 40.62 |
| 1859 | C₇₀H₁₃₇N₃O₇ | 1132.88 | 9.07 | 27.92 | 2059 | C₇₂H₁₃₉N₅O₈ | 1202.93 | 7.82 | 26.46 |
| 1860 | C₇₀H₁₃₇N₃O₇ | 1132.88 | 9.07 | 27.92 | 2060 | C₇₂H₁₃₉N₅O₈ | 1202.93 | 7.82 | 26.46 |
| 1861 | C₇₀H₁₃₇N₃O₇ | 1132.88 | 9.07 | 27.92 | 2061 | C₇₀H₁₄₁N₅O₅ | 1132.93 | 7.84 | 26.35 |
| 1862 | C₇₀H₁₃₇N₃O₇ | 1132.88 | 9.07 | 27.92 | 2062 | C₇₀H₁₄₁N₅O₅ | 1132.93 | 7.84 | 26.35 |
| 1863 | C₆₉H₁₄₁N₃O₄ | 1076.9 | 9.1 | 28.15 | 2063 | C₇₃H₁₄₁N₅O₈ | 1216.96 | 7.84 | 27.17 |
| 1864 | C₆₉H₁₄₁N₃O₄ | 1076.9 | 9.09 | 28.15 | 2064 | C₇₃H₁₄₁N₅O₈ | 1216.96 | 7.84 | 27.17 |
| 1865 | C₇₂H₁₄₁N₃O₇ | 1160.93 | 9.1 | 28.98 | 2065 | C₇₃H₁₄₁N₅O₈ | 1216.96 | 7.84 | 27.17 |
| 1866 | C₉₅H₁₉₄N₄O₉S₅ | 1696.92 | 9.86 | 40.86 | 2066 | C₇₃H₁₄₁N₅O₈ | 1216.96 | 7.84 | 27.17 |
| 1867 | C₇₂H₁₄₁N₃O₄ | 1160.93 | 9.1 | 28.98 | 2067 | C₆₉H₁₃₇N₅O₆ | 1132.88 | 7.19 | 25.38 |
| 1868 | C₇₂H₁₄₁N₃O₇ | 1160.93 | 9.1 | 28.98 | 2068 | C₆₉H₁₃₇N₅O₆ | 1132.88 | 7.19 | 25.38 |
| 1869 | C₇₁H₁₄₅N₃O₄ | 1104.96 | 9.12 | 29.21 | 2069 | C₇₂H₁₃₇N₅O₉ | 1216.91 | 7.06 | 26.2 |
| 1870 | C₇₁H₁₄₅N₃O₄ | 1104.96 | 9.12 | 29.21 | 2070 | C₇₂H₁₃₇N₅O₉ | 1216.91 | 7.06 | 26.2 |
| 1871 | C₇₄H₁₄₅N₃O₄ | 1188.99 | 9.12 | 30.03 | 2071 | C₇₂H₁₃₇N₅O₉ | 1216.91 | 7.06 | 26.2 |
| 1872 | C₉₆H₁₉₁NO₁₄S₅ | 1743.88 | 7.67 | 41.25 | 2072 | C₇₂H₁₃₇N₅O₉ | 1216.91 | 7.06 | 26.2 |
| 1873 | C₇₄H₁₄₅N₃O₇ | 1188.99 | 9.12 | 30.03 | 2073 | C₆₉H₁₃₈N₄O₆ | 1119.88 | 7.66 | 26.29 |
| 1874 | C₇₄H₁₄₅N₃O₇ | 1188.99 | 9.12 | 30.03 | 2074 | C₆₉H₁₃₈N₄O₆ | 1119.88 | 7.66 | 26.29 |
| 1875 | C₆₆H₁₃₅N₃O₄ | 1034.82 | 9.04 | 26.57 | 2075 | C₇₂H₁₃₈N₄O₉ | 1203.91 | 7.66 | 27.11 |
| 1876 | C₆₆H₁₃₅N₃O₄ | 1034.82 | 9.04 | 26.57 | 2076 | C₇₂H₁₃₈N₄O₉ | 1203.91 | 7.66 | 27.11 |
| 1877 | C₆₉H₁₃₅N₃O₇ | 1118.85 | 9.04 | 27.39 | 2077 | C₇₂H₁₃₈N₄O₉ | 1203.91 | 7.66 | 27.11 |
| 1878 | C₆₉H₁₃₅N₃O₇ | 1118.85 | 9.04 | 27.39 | 2078 | C₇₂H₁₃₈N₄O₉ | 1203.91 | 7.66 | 27.11 |
| 1879 | C₆₉H₁₃₅N₃O₇ | 1118.85 | 9.04 | 27.39 | 2079 | C₇₀H₁₄₀N₄O₅ | 1117.91 | 7.94 | 26.15 |
| 1880 | C₆₉H₁₃₅N₃O₇ | 1118.85 | 9.04 | 27.39 | 2080 | C₇₀H₁₄₀N₄O₅ | 1117.91 | 7.93 | 26.15 |
| 1881 | C₆₇H₁₃₇N₃O₄ | 1048.85 | 9.05 | 27.1 | 2081 | C₇₃H₁₄₀N₄O₈ | 1201.94 | 7.94 | 26.97 |
| 1882 | C₆₇H₁₃₇N₃O₄ | 1048.85 | 9.05 | 27.1 | 2082 | C₇₃H₁₄₀N₄O₈ | 1201.94 | 7.93 | 26.97 |
| 1883 | C₇₀H₁₃₇N₃O₇ | 1132.88 | 9.05 | 27.92 | 2083 | C₇₃H₁₄₀N₄O₈ | 1201.94 | 7.94 | 26.97 |
| 1884 | C₇₀H₁₃₇N₃O₇ | 1132.88 | 9.05 | 27.92 | 2084 | C₇₃H₁₄₀N₄O₈ | 1201.94 | 7.94 | 26.97 |
| 1885 | C₇₀H₁₃₇N₃O₇ | 1132.88 | 9.05 | 27.92 | 2085 | C₇₀H₁₃₈N₄O₆ | 1131.9 | 7.19 | 25.85 |
| 1886 | C₇₀H₁₃₇N₃O₇ | 1132.88 | 9.05 | 27.92 | 2086 | C₇₀H₁₃₈N₄O₆ | 1131.9 | 7.19 | 25.85 |
| 1887 | C₆₈H₁₃₉N₃O₄ | 1062.88 | 9.06 | 27.63 | 2087 | C₇₃H₁₃₈N₄O₉ | 1215.93 | 7.17 | 26.67 |
| 1888 | C₆₈H₁₃₉N₃O₄ | 1062.88 | 9.06 | 27.63 | 2088 | C₇₃H₁₃₈N₄O₉ | 1215.93 | 7.17 | 26.67 |
| 1889 | C₇₁H₁₃₉N₃O₇ | 1146.91 | 9.06 | 28.45 | 2089 | C₇₃H₁₃₈N₄O₉ | 1215.93 | 7.17 | 26.67 |
| 1890 | C₇₁H₁₃₉N₃O₇ | 1146.91 | 9.06 | 28.45 | 2090 | C₇₃H₁₃₈N₄O₉ | 1215.93 | 7.17 | 26.67 |
| 1891 | C₇₁H₁₃₉N₃O₇ | 1146.91 | 9.06 | 28.45 | 2091 | C₇₀H₁₃₉N₅O₆ | 1146.91 | 7.19 | 25.65 |
| 1892 | C₇₁H₁₃₉N₃O₇ | 1146.91 | 9.06 | 28.45 | 2092 | C₇₀H₁₃₉N₅O₆ | 1146.91 | 7.19 | 25.65 |
| 1893 | C₆₇H₁₃₇N₃O₄ | 1048.85 | 9.11 | 26.88 | 2093 | C₇₃H₁₃₉N₅O₉ | 1230.94 | 7.08 | 26.47 |
| 1894 | C₆₇H₁₃₇N₃O₄ | 1048.85 | 9.11 | 26.88 | 2094 | C₇₃H₁₃₉N₅O₉ | 1230.94 | 7.08 | 26.47 |
| 1895 | C₇₀H₁₃₇N₃O₇ | 1132.88 | 9.11 | 27.7 | 2095 | C₇₃H₁₃₉N₅O₉ | 1230.94 | 7.08 | 26.47 |
| 1896 | C₇₀H₁₃₇N₃O₇ | 1132.88 | 9.11 | 27.7 | 2096 | C₇₃H₁₃₉N₅O₉ | 1230.94 | 7.08 | 26.47 |
| 1897 | C₇₀H₁₃₇N₃O₇ | 1132.88 | 9.11 | 27.7 | 2097 | C₇₀H₁₄₁N₅O₅ | 1132.93 | 7.84 | 26.35 |
| 1898 | C₇₀H₁₃₇N₃O₇ | 1132.88 | 9.11 | 27.7 | 2098 | C₇₀H₁₄₁N₅O₅ | 1132.93 | 7.84 | 26.35 |
| 1899 | C₆₈H₁₃₉N₃O₄ | 1062.88 | 9.12 | 27.41 | 2099 | C₇₃H₁₄₁N₅O₈ | 1216.96 | 7.84 | 27.17 |
| 1900 | C₆₈H₁₃₉N₃O₄ | 1062.88 | 9.12 | 27.41 | 2100 | C₇₃H₁₄₁N₅O₈ | 1216.96 | 7.84 | 27.17 |
| 1901 | C₇₁H₁₃₉N₃O₇ | 1146.91 | 9.12 | 28.23 | 2101 | C₇₃H₁₄₁N₅O₈ | 1216.96 | 7.84 | 27.17 |
| 1902 | C₇₁H₁₃₉N₃O₇ | 1146.91 | 9.12 | 28.23 | 2102 | C₇₃H₁₄₁N₅O₈ | 1216.96 | 7.84 | 27.17 |
| 1903 | C₇₁H₁₃₉N₃O₇ | 1146.91 | 9.12 | 28.23 | 2103 | C₇₀H₁₃₈N₄O₇ | 1147.89 | 7.19 | 26.06 |
| 1904 | C₇₁H₁₃₉N₃O₇ | 1146.91 | 9.12 | 28.23 | 2104 | C₇₀H₁₃₈N₄O₇ | 1147.89 | 7.19 | 26.06 |
| 1905 | C₆₉H₁₄₁N₃O₄ | 1076.9 | 9.2 | 27.71 | 2105 | C₆₈H₁₃₅N₅O₅ | 1102.86 | 7.65 | 24.36 |
| 1906 | C₆₉H₁₄₁N₃O₄ | 1076.9 | 9.2 | 27.71 | 2106 | C₆₇H₁₃₅N₅O₅ | 1102.86 | 7.65 | 24.36 |
| 1907 | C₇₂H₁₄₁N₃O₇ | 1160.93 | 9.2 | 28.54 | 2107 | C₇₁H₁₃₅N₅O₈ | 1186.89 | 7.65 | 25.18 |
| 1908 | C₇₂H₁₄₁N₃O₄ | 1160.93 | 9.2 | 28.54 | 2108 | C₇₁H₁₃₅N₅O₈ | 1186.89 | 7.65 | 25.18 |
| 1909 | C₇₂H₁₄₁N₃O₄ | 1160.93 | 9.2 | 28.54 | 2109 | C₇₁H₁₃₅N₅O₈ | 1186.89 | 7.65 | 25.18 |
| 1910 | C₇₂H₁₄₁N₃O₇ | 1160.93 | 9.2 | 28.54 | 2110 | C₇₁H₁₃₅N₅O₈ | 1186.89 | 7.65 | 25.18 |
| 1911 | C₆₉H₁₄₁N₃O₄ | 1076.9 | 9.15 | 27.93 | 2111 | C₆₉H₁₃₆N₄O₅ | 1101.87 | 7.9 | 25.58 |
| 1912 | C₆₉H₁₄₁N₃O₄ | 1076.9 | 9.15 | 27.93 | 2112 | C₆₉H₁₃₆N₄O₅ | 1101.87 | 7.9 | 25.58 |
| 1913 | C₇₂H₁₄₁N₃O₄ | 1160.93 | 9.15 | 28.76 | 2113 | C₇₂H₁₃₆N₄O₅ | 1185.9 | 7.9 | 26.41 |
| 1914 | C₇₂H₁₄₁N₃O₇ | 1160.93 | 9.15 | 28.76 | 2114 | C₇₂H₁₃₆N₄O₈ | 1185.9 | 7.9 | 26.41 |
| 1915 | C₇₂H₁₄₁N₃O₇ | 1160.93 | 9.15 | 28.76 | 2115 | C₇₂H₁₃₆N₄O₈ | 1185.9 | 7.9 | 26.41 |
| 1916 | C₇₂H₁₄₁N₃O₄ | 1160.93 | 9.15 | 28.76 | 2116 | C₇₂H₁₃₆N₄O₅ | 1185.9 | 7.9 | 26.41 |
| 1917 | C₆₇H₁₃₅N₃O₄ | 1046.83 | 8.42 | 26.62 | 2117 | C₇₀H₁₃₇N₅O₆ | 1144.89 | 7.19 | 25.33 |
| 1918 | C₆₇H₁₃₅N₃O₄ | 1046.83 | 8.42 | 26.62 | 2118 | C₇₀H₁₃₇N₅O₆ | 1144.89 | 7.19 | 25.33 |
| 1919 | C₇₀H₁₃₅N₃O₇ | 1130.86 | 8.42 | 27.44 | 2119 | C₁₀₀H₁₉₅NO₉S₂ | 1619.78 | 9.12 | 42.45 |
| 1920 | C₇₀H₁₃₅N₃O₇ | 1130.86 | 8.42 | 27.44 | 2120 | C₉₉H₁₈₉N₃O₉ | 1565.61 | 8.77 | 41.14 |
| 1921 | C₇₀H₁₃₅N₃O₇ | 1130.86 | 8.42 | 27.44 | 2121 | C₇₃H₁₃₇N₅O₉ | 1228.92 | 7.07 | 26.15 |
| 1922 | C₇₀H₁₃₅N₃O₇ | 1130.86 | 8.42 | 27.44 | 2122 | C₇₃H₁₃₇N₅O₉ | 1228.92 | 7.07 | 26.15 |
| 1923 | C₆₉H₁₃₉N₃O₄ | 1074.89 | 8.99 | 27.51 | 2123 | C₇₁H₁₃₉N₅O₅ | 1142.92 | 7.47 | 25.63 |
| 1924 | C₆₉H₁₃₉N₃O₄ | 1074.89 | 8.99 | 27.51 | 2124 | C₇₁H₁₃₉N₅O₅ | 1142.92 | 7.47 | 25.63 |
| 1925 | C₇₂H₁₃₉N₃O₇ | 1158.92 | 8.99 | 28.33 | 2125 | C₁₀₃H₁₉₄N₂O₁₅S ₂ | 1764.8 | 8.09 | 42.63 |
| 1926 | C₇₂H₁₃₉N₃O₇ | 1158.92 | 8.99 | 28.33 | 2126 | C₁₀₁H₁₉₆N₂O₁₄S | 1694.74 | 9.65 | 42.49 |
| 1927 | C₇₂H₁₃₉N₃O₇ | 1158.92 | 9 | 28.33 | 2127 | C₇₄H₁₃₀N₅O₈ | 1226.95 | 7.47 | 26.45 |
| 1928 | C₇₂H₁₃₉N₃O₇ | 1158.92 | 9 | 28.33 | 2128 | C₇₄H₁₃₀N₅O₈ | 1226.95 | 7.47 | 26.45 |
| 1929 | C₆₇H₁₃₅N₃O₄ | 1046.83 | 8.77 | 26.65 | 2129 | C₇₁H₁₃₈N₈O₄ | 1167.94 | 7.58 | 25.74 |
| 1930 | C₆₇H₁₃₅N₃O₄ | 1046.83 | 8.77 | 26.65 | 2130 | C₇₁H₁₃₈N₈O₄ | 1167.94 | 7.58 | 25.74 |
| 1931 | C₇₀H₁₃₅N₃O₇ | 1130.86 | 8.77 | 27.47 | 2131 | C₈₈H₁₇₂N₄O₇ | 1398.36 | 7.81 | 35.59 |
| 1932 | C₇₀H₁₃₅N₃O₇ | 1130.86 | 8.77 | 27.47 | 2132 | C₈₄H₁₇₁NO₇S | 1339.35 | 10.02 | 36.46 |
| 1933 | C₇₀H₁₃₅N₃O₇ | 1130.86 | 8.77 | 27.47 | 2133 | C₇₄H₁₃₈N₈O₇ | 1251.97 | 7.58 | 26.56 |
| 1934 | C₇₀H₁₃₅N₃O₇ | 1130.86 | 8.77 | 27.47 | 2134 | C₇₄H₁₃₈N₈O₇ | 1251.97 | 7.58 | 26.56 |
| 1935 | C₆₈H₁₃₇N₃O₄ | 1060.86 | 9.4 | 26.63 | 2135 | C₆₀H₁₀₀N₂O₈S | 1009.53 | 7.01 | 18.88 |
| 1936 | C₆₈H₁₃₇N₃O₄ | 1060.86 | 9.4 | 26.63 | 2136 | C₆₁H₁₀₅N₃O₆S₃ | 1072.71 | 8.34 | 21.3 |
| 1937 | C₇₁H₁₃₇N₃O₇ | 1144.89 | 9.4 | 27.45 | 2137 | C₆₀H₁₀₅NO₂S₄ | 1000.75 | 8.96 | 22.13 |
| 1938 | C₇₁H₁₃₇N₃O₇ | 1144.89 | 9.4 | 27.45 | 2138 | C₆₃H₁₁₄N₆O₇ | 1067.64 | 10 | 21.45 |
| 1939 | C₇₁H₁₃₇N₃O₇ | 1144.89 | 9.4 | 27.45 | 2139 | C₅₉H₁₀₁NO₁₀ | 984.45 | 8.35 | 21.09 |
| 1940 | C₇₁H₁₃₇N₃O₇ | 1144.89 | 9.4 | 27.45 | 2140 | C₆₀H₁₀₈N₄O₆S | 1013.61 | 8.4 | 19.22 |
| 1941 | C₆₈H₁₃₇N₃O₄ | 1060.86 | 9.62 | 25.87 | 2141 | C₆₄H₁₁₈N₃O₇P | 1072.64 | 8.79 | 20.87 |
| 1942 | C₆₈H₁₃₇N₃O₄ | 1060.86 | 9.62 | 25.87 | 2142 | C₅₆H₁₀₃NO₉S₄ | 1062.68 | 10.13 | 19.46 |
| 1943 | C₇₁H₁₃₇N₃O₇ | 1144.89 | 9.62 | 26.69 | 2143 | C₅₉H₁₀₇NO₉S₄ | 1102.74 | 9.62 | 17.16 |
| 1944 | C₇₁H₁₃₇N₃O₇ | 1144.89 | 9.62 | 26.69 | 2144 | C₆₀H₁₁₀N₂O₁₂S₃ | 1147.72 | 8.95 | 19.15 |
| 1945 | C₇₁H₁₃₇N₃O₇ | 1144.89 | 9.62 | 26.69 | 2145 | C₆₄H₁₁₃N₃O₄S₃ | 1084.8 | 10.17 | 23.74 |
| 1946 | C₇₁H₁₃₇N₃O₇ | 1144.89 | 9.62 | 26.69 | 2146 | C₆₀H₁₀₄N₂O₅S₃ | 1029.68 | 9.96 | 22.15 |
| 1947 | C₆₈H₁₃₇N₃O₄ | 1060.86 | 8.87 | 27.21 | 2147 | C₆₃H₁₀₉N₃O₆S₄ | 1132.82 | 9.65 | 22.68 |
| 1948 | C₆₈H₁₃₇N₃O₄ | 1060.86 | 8.87 | 27.21 | 2148 | C₆₂H₁₁₀N₂O₂S₇ | 1139.99 | 10.03 | 24.47 |
| 1949 | C₇₁H₁₃₇N₃O₇ | 1144.89 | 8.87 | 28.03 | 2149 | C₅₇H₁₀₂N₆O₃S | 951.54 | 8.64 | 15.85 |
| 1950 | C₇₁H₁₃₇N₃O₇ | 1144.89 | 8.87 | 28.03 | 2150 | C₅₃H₉₇NO₇ | 860.36 | 9.79 | 19.82 |
| 1951 | C₇₁H₁₃₇N₃O₇ | 1144.89 | 8.87 | 28.03 | 2151 | C₅₇H₁₀₃N₃O₂S₃ | 958.65 | 8.96 | 18.52 |
| 1952 | C₇₁H₁₃₇N₃O₇ | 1144.89 | 8.87 | 28.03 | 2152 | C₅₆H₁₀₃NS₄ | 918.69 | 10.21 | 21.1 |
| 1953 | C₆₈H₁₃₈N₄O₄ | 1075.88 | 8.77 | 25.07 | 2153 | C₅₆H₁₀₃NO₃S₇ | 1062.86 | 9.96 | 23.06 |
| 1954 | C₆₈H₁₃₈N₄O₄ | 1075.88 | 8.77 | 25.07 | 2154 | C₆₄H₁₁₉NO₈S₄ | 1158.89 | 8.4 | 26.14 |
| 1955 | C₇₁H₁₃₈N₄O₇ | 1159.91 | 8.77 | 25.89 | 2155 | C₆₇H₁₂₂N₄O₅S₄ | 1191.98 | 8.15 | 26.63 |
| 1956 | C₇₁H₁₃₈N₄O₇ | 1159.91 | 8.77 | 25.89 | 2156 | C₇₀H₁₂₈N₂O₅S₅ | 1238.1 | 8.8 | 28.64 |
| 1957 | C₇₁H₁₃₈N₄O₇ | 1159.91 | 8.77 | 25.89 | 2157 | C₆₇H₁₂₂N₂O₅S₄ | 1163.96 | 7.06 | 25.24 |
| 1958 | C₇₁H₁₃₈N₄O₇ | 1159.91 | 8.77 | 25.89 | 2158 | C₆₄H₁₁₇NOS₇ | 1141.07 | 9.43 | 28.02 |
| 1959 | C₆₇H₁₃₅N₃O₅ | 1062.83 | 7.19 | 26 | 2159 | C₇₂H₁₄₂N₇O₆P | 1232.94 | 10.1 | 26.28 |
| 1960 | C₆₇H₁₃₅N₃O₅ | 1062.83 | 7.19 | 26 | 2160 | C₇₂H₁₃₀N₆O₇S₂ | 1255.99 | 8.52 | 24.5 |
| 1961 | C₁₀₄H₁₉₇N₃O₇S₅ | 1762.03 | 7.97 | 43.84 | 2161 | C₆₇H₁₃₀N₆O₂ | 1051.82 | 8.75 | 23.16 |
| 1962 | C₉₉H₂₀₃N₂O₁₄P₃ S₂ | 1802.75 | 7.87 | 40.39 | 2162 | C₆₉H₁₃₀N₄O₇ | 1127.82 | 9.95 | 26.16 |
| 1963 | C₇₀H₁₃₅N₃O₈ | 1146.86 | 6.84 | 26.82 | 2163 | C₇₂H₁₂₉N₉O₆S | 1248.94 | 8.63 | 24.39 |
| 1964 | C₇₀H₁₃₅N₃O₈ | 1146.86 | 6.83 | 26.82 | 2164 | C₇₀H₁₃₁NO₁₀ | 1146.82 | 10.01 | 27.8 |
| 1965 | C₆₉H₁₃₉N₃O₄ | 1074.89 | 9.8 | 26.43 | 2165 | C₆₇H₁₂₁N₃O₈S₃ | 1192.9 | 7.17 | 27.01 |
| 1966 | C₆₉H₁₃₉N₃O₄ | 1074.89 | 9.8 | 26.43 | 2166 | C₆₆H₁₂₃N₃O₇S | 1102.78 | 7.67 | 23.92 |
| 1967 | C₇₂H₁₃₉N₃O₇ | 1158.92 | 9.8 | 27.25 | 2167 | C₇₂H₁₄₀N₂O₁₃P₄ | 1365.81 | 8.13 | 23.27 |
| 1968 | C₇₂H₁₃₉N₃O₇ | 1158.92 | 9.8 | 27.25 | 2168 | C₇₃H₁₃₉N₄O₁₃P₃ | 1373.85 | 7.94 | 22.39 |
| 1969 | C₇₂H₁₃₉N₃O₇++ | 1158.92 | 9.8 | 27.25 | 2169 | C₆₄H₁₂₅N₃O₇S₃ | 1144.9 | 9.63 | 22.96 |
| 1970 | C₇₂H₁₃₉N₃O₇ | 1158.92 | 9.8 | 27.25 | 2170 | C₆₈H₁₂₇N₃O₇S₃ | 1194.96 | 10.18 | 28.67 |
| 1971 | C₆₆H₁₃₅N₃O₅ | 1050.82 | 8.2 | 25.62 | 2171 | C₆₉H₁₂₉NO₃S₇ | 1245.21 | 10.02 | 29.71 |
| 1972 | C₆₆H₁₃₅N₃O₅ | 1050.82 | 8.2 | 25.62 | 2172 | C₆₄H₁₁₈N₂O₂S₆ | 1140.02 | 7.72 | 28.06 |
| 1973 | C₆₉H₁₃₅N₃O₈ | 1134.85 | 8.2 | 26.44 | 2173 | C₆₅H₁₂₅NO₃S₆ | 1161.08 | 9.59 | 30.57 |
| 1974 | C₆₉H₁₃₅N₃O₈ | 1134.85 | 8.2 | 26.44 | 2174 | C₆₇H₁₂₉NO₂S₇ | 1205.19 | 10.25 | 31.99 |
| 1975 | C₆₉H₁₃₅N₃O₈ | 1134.85 | 8.2 | 26.44 | 2175 | C₆₃H₁₂₁N₃OS₈ | 1193.16 | 8.62 | 29.09 |
| 1976 | C₆₉H₁₃₅N₃O₈ | 1134.85 | 8.2 | 26.44 | 2176 | C₅₆H₁₀₄N₄O₃S₂ | 945.59 | 10.08 | 17.35 |
| 1977 | C₆₇H₁₃₇N₃O₅ | 1064.85 | 8.67 | 25.91 | 2177 | C₅₅H₁₀₃N₁₁O₄ | 982.5 | 7.63 | 16.96 |
| 1978 | C₆₇H₁₃₇N₃O₅ | 1064.85 | 8.67 | 25.91 | 2178 | C₅₅H₁₀₀N₄O₈S | 977.48 | 9.61 | 18.82 |
| 1979 | C₇₀H₁₃₇N₃O₈ | 1148.88 | 8.67 | 26.73 | 2179 | C₅₇H₁₀₈N₁₀O₈S₄ | 1189.79 | 8.5 | 16.73 |
| 1980 | C₇₀H₁₃₇N₃O₈ | 1148.88 | 8.67 | 26.73 | 2180 | C₅₃H₁₀₂N₆O₁₀S₄ | 1111.67 | 8.18 | 14.64 |
| 1981 | C₇₀H₁₃₇N₃O₈ | 1148.88 | 8.67 | 26.73 | 2181 | C₅₉H₁₀₀N₆O₈ | 1021.48 | 8.42 | 18.23 |
| 1982 | C₇₀H₁₃₇N₃O₈ | 1148.88 | 8.67 | 26.73 | 2182 | C₅₄H₉₈N₂O₈ | 903.38 | 8.85 | 20.07 |
| 1983 | C₆₈H₁₃₉N₃O₅ | 1078.88 | 8.87 | 25.64 | 2183 | C₅₆H₁₀₄N₄O₁₀S | 1025.53 | 9.53 | 19.39 |
| 1984 | C₆₈H₁₃₉N₃O₅ | 1078.88 | 8.87 | 25.64 | 2184 | C₅₃H₉₈N₄O₇S₂ | 967.51 | 8.78 | 17.62 |
| 1985 | C₇₁H₁₃₉N₃O₈ | 1162.91 | 8.87 | 26.46 | 2185 | C₅₄H₁₀₃NO₆S | 894.48 | 9.81 | 18.88 |
| 1986 | C₉₃H₁₈₇NO₈S₆ | 1639.88 | 8.87 | 39.61 | 2186 | C₆₀H₁₁₅N₄O₁₂P₃ S | 1209.58 | 7.38 | 16.63 |
| 1987 | C₇₁H₁₃₉N₃O₈ | 1162.91 | 8.87 | 26.46 | 2187 | C₄₈H₉₁NO₁₄S₄ | 1034.49 | 8.57 | 18.09 |
| 1988 | C₇₁H₁₃₉N₃O₈ | 1162.91 | 8.87 | 26.46 | 2188 | C₅₃H₉₇N₃O₇S₃ | 984.55 | 7.65 | 15.34 |
| 1989 | C₆₈H₁₃₈N₄O₆ | 1107.87 | 7.8 | 25.43 | 2189 | C₅₃H₁₀₀N₄O₁₀S₄ | 1081.64 | 8.02 | 15.11 |
| 1990 | C₆₈H₁₃₈N₄O₆ | 1107.87 | 7.8 | 25.43 | 2190 | C₅₉H₁₀₄N₂O₈S₄ | 1097.73 | 8.07 | 20.7 |
| 1991 | C₉₈H₁₉₅N₅O₅S₃ | 1619.85 | 7.45 | 41.6 | 2191 | C₅₇H₁₀₃NO₆S₄ | 1026.69 | 10.16 | 22.82 |
| 1992 | C₉₈H₁₉₃NO₁₀S₃ | 1641.8 | 9.82 | 42.89 | 2192 | C₅₇H₁₀₃NO₆S₆ | 1090.81 | 8.72 | 25.21 |
| 1993 | C₇₁H₁₃₈N₄O₉ | 1191.9 | 7.8 | 26.25 | 2193 | C₅₇H₁₀₃NO₆S₇ | 1122.87 | 9.87 | 23.86 |
| 1994 | C₇₁H₁₃₈N₄O₉ | 1191.9 | 7.8 | 26.25 | 2194 | C₅₇H₉₉N₃O₈S₇ | 1178.85 | 7.78 | 21.15 |
| 1995 | C₆₉H₁₃₇N₅O₅ | 1116.88 | 7.49 | 25.53 | 2195 | C₅₆H₁₀₉N₂O₃PS₆ | 1081.83 | 8.96 | 22.34 |
| 1996 | C₆₉H₁₃₇N₅O₅ | 1116.88 | 7.49 | 25.53 | 2196 | C₆₉H₁₂₁NO₅S | 1076.79 | 9.98 | 23.52 |
| 1997 | C₇₂H₁₃₇N₅O₈ | 1200.91 | 7.49 | 26.35 | 2197 | C₇₀H₁₂₂N₄O₂S | 1147.95 | 8.85 | 22.42 |
| 1998 | C₇₂H₁₃₇N₅O₈ | 1200.91 | 7.49 | 26.35 | 2198 | C₇₂H₁₂₃N₉O₇ | 1226.83 | 8.52 | 18.69 |
| 1999 | C₇₂H₁₃₇N₅O₈ | 1200.91 | 7.49 | 26.35 | 2199 | C₆₇H₁₁₇N₅O₅ | 1072.7 | 8.36 | 19.23 |
| 2000 | C₇₂H₁₃₇N₅O₈ | 1200.91 | 7.49 | 26.35 | 2200 | C₆₈H₁₂₇N₁₁O₄ | 1162.84 | 9.49 | 19.64 |
| 2201 | C₆₈H₁₂₅N₇O₅ | 1120.79 | 9.91 | 22 | 2401 | C₇₇H₁₅₇N₃O₁₀S₃ | 1381.31 | 10.29 | 31.76 |
| 2202 | C₇₀H₁₂₆N₁₀O₇S | 1251.9 | 7.99 | 20.23 | 2402 | C₇₇H₁₄₈N₄O₇S₇ | 1466.52 | 7.62 | 33.01 |
| 2203 | C₆₇H₁₁₈N₈O₈ | 1163.73 | 7.71 | 21.54 | 2403 | C₇₆H₁₄₇NO₂S₆ | 1299.42 | 10.02 | 34.04 |
| 2204 | C₆₇H₁₂₃N₇O₂ | 1058.77 | 8.87 | 20.62 | 2404 | C₇₆H₁₅₃NOS₈ | 1353.6 | 10.09 | 38.26 |
| 2205 | C₆₇H₁₂₂N₁₀O | 1083.78 | 8.58 | 19.27 | 2405 | C₇₅H₁₄₉N₃O₂S₈ | 1381.57 | 8.73 | 35.21 |
| 2206 | C₆₃H₁₁₈N₄O₆S₅ | 1187.96 | 9.68 | 21.42 | 2406 | C₉₉H₁₉₃N₇O₆ | 1577.68 | 8.36 | 38.16 |
| 2207 | C₆₅H₁₁₁NO₇S | 1050.66 | 8.1 | 22.75 | 2407 | C₉₉H₁₈₉N₃O₅S₃ | 1597.82 | 8.77 | 42.25 |
| 2208 | C₇₀H₁₁₉N₃O₉S₅ | 1307.03 | 7.99 | 25.56 | 2408 | C₉₆H₁₈₉NO₃S₅ | 1565.91 | 9.91 | 45.5 |
| 2209 | C₆₇H₁₁₈N₂O₁₂S₅ | 1303.98 | 8.08 | 20.95 | 2409 | C₁₀₆H₂₀₅NO₅ | 1573.81 | 9.9 | 46.33 |
| 2210 | C₆₄H₁₁₇NO₁₃S₃ | 1204.81 | 9.78 | 23.12 | 2410 | C₁₀₇H₂₀₉N₅O₅ | 1645.88 | 8.96 | 44.62 |
| 2211 | C₆₁H₁₁₂N₂O₁₄S₃ | 1193.75 | 9.49 | 22.15 | 2411 | C₁₀₅H₂₀₄N₆O₃S₂ | 1662.96 | 8.7 | 42.53 |
| 2212 | C₇₂H₁₃₃N₄O₁₂P₃ | 1339.79 | 8.12 | 20.87 | 2412 | C₁₀₆H₂₀₅N₃O₉ | 1665.82 | 8.8 | 45.11 |
| 2213 | C₆₈H₁₂₈NO₁₀P₃S₂ | 1276.81 | 9.04 | 21.27 | 2413 | C₁₀₄H₂₀₄N₂O₃S₃ | 1626.99 | 9.75 | 44.82 |
| 2214 | C₆₅H₁₁₇NO₇S₄ | 1152.89 | 9.86 | 19.82 | 2414 | C₁₀₄H₂₀₁NOS₇ | 1706.23 | 9.86 | 49.18 |
| 2215 | C₆₆H₁₂₀N₆O₈S₃ | 1221.9 | 9.07 | 19.62 | 2415 | C₁₀₅H₂₀₄N₃O₅P S₆ | 1812.18 | 7.51 | 47.43 |
| 2216 | C₆₈H₁₂₄N₂O₁₀S₅ | 1290.04 | 9.08 | 22.31 | 2416 | C₁₀₅H₂₀₆N₆O₆S | 1680.91 | 8.82 | 42.03 |
| 2217 | C₆₃H₁₁₂N₄O₂S₆ | 1149.97 | 7.37 | 24.03 | 2417 | C₁₀₃H₂₀₄N₈O₄S₂ | 1682.95 | 8.74 | 43.18 |
| 2218 | C₇₀H₁₃₁N₃O₄S₂ | 1142.95 | 8.36 | 25.63 | 2418 | C₁₀₆H₂₁₁N₇O₂ | 1615.9 | 8.51 | 43.8 |
| 2219 | C₆₇H₁₃₀N₂O₅ | 1043.79 | 9.54 | 27.28 | 2419 | C₁₀₃H₂₁₁N₄O₃P | 1584.82 | 9.91 | 45.36 |
| 2220 | C₆₈H₁₃₁NO₅S₄ | 1171.04 | 8.83 | 28.53 | 2420 | C₁₀₄H₂₀₈N₆O₆ | 1638.84 | 8.96 | 43.83 |
| 2221 | C₈₇H₁₆₉NO₅S | 1341.37 | 9.31 | 36.31 | 2421 | C₁₀₇H₂₀₈N₆O₁₀S | 1770.94 | 8.19 | 44.86 |
| 2222 | C₈₈H₁₇₂N₂O₅ | 1338.35 | 9.01 | 37.81 | 2422 | C₁₀₂H₂₀₂N₄O₈S | 1644.82 | 9.95 | 44.99 |
| 2223 | C₈₇H₁₆₉NO₆S₃ | 1421.49 | 9.94 | 39.01 | 2423 | C₁₀₆H₂₀₈N₄O₇S₃ | 1747.05 | 9.64 | 47.63 |
| 2224 | C₉₂H₁₇₅N₆O₇PS₃ | 1604.6 | 7.83 | 37.76 | 2424 | C₁₀₆H₂₀₇N₄O₉P S₃ | 1809.02 | 8.18 | 47.75 |
| 2225 | C₈₇H₁₇₀N₄O₂S₆ | 1496.7 | 8.3 | 39.06 | 2425 | C₁₀₂H₂₀₁NO₉S₄ | 1714 | 9.33 | 48.93 |
| 2226 | C₈₂H₁₆₈N₄OS | 1258.33 | 9.43 | 35.75 | 2426 | C₁₀₄H₂₀₁NO₇S₄ | 1706.02 | 9.76 | 48.92 |
| 2227 | C₈₉H₁₇₃NO₉S₄ | 1529.6 | 9.78 | 39.84 | 2427 | C₁₀₂H₂₀₃NO₅ | 1523.75 | 9.86 | 45.85 |
| 2228 | C₈₇H₁₇₅NO₁₆S₄ | 1619.59 | 9.94 | 38.9 | 2428 | C₁₀₈H₂₁₄N₇O₁₂P₃ | 1895.86 | 7.23 | 41.61 |
| 2229 | C₈₉H₁₇₇NO₁₃ S₄ | 1597.63 | 10.04 | 39.39 | 2429 | C₁₀₀H₁₉₉N₃O₁₅S₅ | 1844.03 | 7.71 | 46.1 |
| 2230 | C₈₅H₁₇₂N₆O₁₀S₄ | 1566.58 | 8.43 | 34.65 | 2430 | C₉₉H₁₉₉NO₁₄S₅ | 1788.01 | 8.79 | 44.17 |
| 2231 | C₈₅H₁₇₅N₅O₉S₃ | 1507.54 | 9.58 | 34.71 | 2431 | C₁₀₃H₂₀₆N₆O₇S₄ | 1769.08 | 8.96 | 44.54 |
| 2232 | C₈₅H₁₇₄N₂O₁₁S₄ | 1528.6 | 8.94 | 37.92 | 2432 | C₁₀₃H₂₀₉N₆O₁₀P S₃ | 1819.01 | 8.49 | 41.31 |
| 2233 | C₈₅H₁₇₁N₃O₁₀S₄ | 1523.58 | 8.67 | 35.43 | 2433 | C₉₉H₂₀₁N₃O₁₀S₃ | 1689.91 | 9.61 | 44.8 |
| 2234 | C₉₀H₁₇₃N₃O₆S₄ | 1521.66 | 8.82 | 38.34 | 2434 | C₁₀₁H₂₀₅NO₁₀S₅ | 1754.08 | 9.99 | 43.99 |
| 2235 | C₉₀H₁₇₃NO₃S₈ | 1573.91 | 9.95 | 43.51 | 2435 | C₁₀₄H₁₉₉N₃O₅S₅ | 1732.09 | 7.64 | 46.37 |
| 2236 | C₇₂H₁₄₀N₆O₅ | 1169.95 | 8.47 | 24.78 | 2436 | C₁₀₃H₁₉₉N₃O₉S₄ | 1752 | 7.51 | 47.66 |
| 2237 | C₆₆H₁₃₇N₇O₂ | 1060.87 | 8.33 | 24.92 | 2437 | C₉₇H₁₉₃N₇O₅S | 1569.72 | 9.22 | 38.28 |
| 2238 | C₇₃H₁₄₁N₃O₇S₂ | 1237.08 | 9.74 | 28.32 | 2438 | C₉₃H₁₈₈N₆O₇S₄ | 1630.83 | 7.7 | 37.71 |
| 2239 | C₆₈H₁₃₆N₄O₈ | 1137.86 | 9.16 | 26.01 | 2439 | C₁₀₀H₁₉₆N₄O₈ | 1582.69 | 9.91 | 43 |
| 2240 | C₇₀H₁₂₀N₄O₁₀S | 1229.97 | 9.11 | 27 | 2440 | C₉₉H₁₉₂N₂O₉S₄ | 1682.9 | 8.81 | 44.24 |
| 2241 | C₆₈H₁₃₃NO₁₁S | 1172.87 | 9.51 | 27.24 | 2441 | C₉₄H₁₉₁NO₃S | 1415.63 | 10.1 | 42.86 |
| 2242 | C₆₉H₁₃₁NO₁₀S | 1166.87 | 9.12 | 27.95 | 2442 | C₁₀₀H₂₀₅N₂O₁₁P ₃ | 1704.66 | 10.14 | 43.84 |
| 2243 | C₆₇H₁₃₅NO₄S | 1050.89 | 9.96 | 27.64 | 2443 | C₉₂H₁₈₆N₂O₁₁S₃ | 1592.7 | 8.75 | 40.86 |
| 2244 | C₇₀H₁₄₅N₂O₁₂P₃ | 1299.85 | 7.89 | 25.09 | 2444 | C₉₄H₁₈₇N₃O₁₅S₃ | 1695.74 | 7.43 | 40.74 |
| 2245 | C₇₄H₁₅₂NO₁₂P₃S | 1373.01 | 9.94 | 27.12 | 2445 | C₉₃H₁₉₀N₂O₆S₃ | 1528.75 | 10.06 | 38.72 |
| 2246 | C₆₉H₁₃₈N₂O₁₀S₃ | 1252.07 | 9.92 | 27.38 | 2446 | C₉₄H₁₈₇N₃O₁₂S₄ | 1679.81 | 7.37 | 39.57 |
| 2247 | C₆₉H₁₃₉NO₁₅S₄ | 1351.13 | 9.9 | 28.99 | 2447 | C₁₀₁H₁₉₆N₃O₇P S₆ | 1788.07 | 8.79 | 44.46 |
| 2248 | C₇₂H₁₃₈N₆O₁₃S₅ | 1456.26 | 8.64 | 26.43 | 2448 | C₉₄H₁₈₇NOS₆ | 1539.94 | 9.73 | 45.76 |
| 2249 | C₇₀H₁₃₇N₃O₉S₃ | 1261.08 | 8.66 | 22.65 | 2449 | C₇₇H₁₂₀N₆O₃S₂ | 1241.98 | 8.7 | 26.41 |
| 2250 | C₆₇H₁₃₈N₆O₇S₃ | 1236.08 | 8.87 | 26.36 | 2450 | C₇₅H₁₂₄N₄O₆ | 1177.84 | 10.2 | 25.9 |
| 2251 | C₆₉H₁₃₇N₃O₁₃S₃ | 1313.06 | 8.54 | 25.97 | 2451 | C₇₂H₁₁₅NO₆S₂ | 1154.85 | 10.06 | 27.89 |
| 2252 | C₇₂H₁₃₇N₃O₂S₆ | 1269.31 | 8.65 | 29.55 | 2452 | C₇₆H₁₂₅N₅O₄S₃ | 1269.07 | 9.66 | 26.77 |
| 2253 | C₇₄H₁₄₆NO₃PS₆ | 1321.36 | 10.13 | 32.3 | 2453 | C₇₅H₁₁₇N₃O₂S₈ | 1349.32 | 7.66 | 27.75 |
| 2254 | C₉₀H₁₄₇N₃O₈ | 1399.18 | 7.9 | 32.42 | 2454 | C₇₇H₁₂₃N₅O₆S | 1246.93 | 8.19 | 24.95 |
| 2255 | C₈₉H₁₄₉NO₆S | 1361.24 | 9.32 | 34.59 | 2455 | C₇₆H₁₂₇N₇O₄ | 1202.9 | 9 | 24.97 |
| 2256 | C₈₈H₁₄₆N₂O₇S | 1376.21 | 6.19 | 33.17 | 2456 | C₇₅H₁₂₆N₆O₈ | 1239.87 | 9.84 | 25.43 |
| 2257 | C₈₈H₁₅₀N₄O₄S | 1360.26 | 10.02 | 32.47 | 2457 | C₆₈H₁₁₈N₄O₃S | 1071.78 | 9.27 | 26.81 |
| 2258 | C₉₂H₁₅₄N₆O₃S₃ | 1488.48 | 9.03 | 34.03 | 2458 | C₇₄H₁₂₈N₄O₈S₄ | 1330.13 | 10.33 | 26.97 |
| 2259 | C₈₉H₁₅₀N₂O₇ | 1360.19 | 10.09 | 35.36 | 2459 | C₇₄H₁₁₉NO₉ | 1166.76 | 10 | 26.77 |
| 2260 | C₈₈H₁₄₄N₂O₇S | 1374.19 | 8.78 | 34.21 | 2460 | C₇₂H₁₁₈N₂O₁₀ | 1171.74 | 9.32 | 24.67 |
| 2261 | C₉₀H₁₅₅N₇O₅ | 1415.27 | 9.54 | 32.12 | 2461 | C₇₈H₁₂₁N₃O₁₂S₂ | 1356.97 | 8.21 | 25.6 |
| 2262 | C₉₀H₁₅₈N₅O₅P | 1421.26 | 8.1 | 33.64 | 2462 | C₈₀H₁₃₇N₂O₁₁P₃ | 1395.9 | 8.95 | 25.51 |
| 2263 | C₉₀H₁₅₃N₇O₉S | 1509.32 | 8.41 | 32.06 | 2463 | C₇₇H₁₃₅N₄O₁₀P₃ S | 1401.93 | 7.5 | 25.37 |
| 2264 | C₉₃H₁₆₄N₃O₁₁P₃ S | 1625.33 | 8.74 | 32.17 | 2464 | C₆₉H₁₁₈N₄O₉S₄ | 1275.99 | 7.5 | 25.37 |
| 2265 | C₉₂H₁₆₄NO₁₄P₃S | 1633.31 | 7.95 | 33.25 | 2465 | C₇₁H₁₁₉NO₁₃S₄ | 1322.99 | 9.94 | 25.39 |
| 2266 | C₈₈H₁₅₀N₄O₁₂S₄ | 1584.45 | 8.03 | 31.72 | 2466 | C₇₂H₁₁₅N₃O₃S₄ | 1199 | 8.42 | 26.27 |
| 2267 | C₈₈H₁₅₃N₃O₁₃S₃ | 1557.4 | 9.96 | 35.94 | 2467 | C₇₃H₁₁₈N₂O₂S₇ | 1280.23 | 9.68 | 28.94 |
| 2268 | C₉₃H₁₆₇N₂O₁₁P₃ | 1582.28 | 10 | 33.87 | 2468 | C₇₀H₁₃₃NO₄S₂ | 1116.97 | 9.97 | 28.02 |
| 2269 | C₉₇H₁₆₈N₃O₁₂P₃ S | 1693.41 | 8.6 | 32.71 | 2469 | C₆₇H₁₂₉NO₇ | 1060.77 | 8.51 | 28.62 |
| 2270 | C₈₇H₁₅₁N₃O₉S₃ | 1479.38 | 8.83 | 30.34 | 2470 | C₆₈H₁₂₇N₃O₂S₃ | 1114.98 | 8.46 | 26.4 |
| 2271 | C₈₇H₁₅₄N₆O₇S₅ | 1556.56 | 8.94 | 32.1 | 2471 | C₆₆H₁₂₄N₄S₄ | 1102.01 | 7.24 | 26.38 |
| 2272 | C₈₇H₁₄₃NO₅S₇ | 1507.57 | 9.36 | 38.83 | 2472 | C₆₇H₁₃₅N₇O₃S | 1118.93 | 8.8 | 26.76 |
| 2273 | C₈₈H₁₄₄N₂O₅S₆ | 1502.53 | 8.66 | 36.59 | 2473 | C₆₅H₁₃₃N₅O₂ | 1016.81 | 9.94 | 25.89 |
| 2274 | C₈₉H₁₅₃N₅OS₃ | 1405.44 | 9.38 | 36.17 | 2474 | C₆₂H₁₂₇N₅O₈S₃ | 1166.93 | 8.18 | 24.53 |
| 2275 | C₈₈H₁₅₂N₄O₂S₄ | 1426.48 | 8.67 | 36.49 | 2475 | C₆₇H₁₃₈N₄O₆S₅ | 1256.2 | 9.66 | 27.43 |
| 2276 | C₅₃H₁₀₁N₇O₅ | 916.43 | 9.37 | 14.69 | 2476 | C₆₅H₁₃₄N₆O₇S₅ | 1272.16 | 8.65 | 24.58 |
| 2277 | C₄₈H₉₁NO₉S | 858.32 | 7.45 | 18.24 | 2477 | C₆₉H₁₂₉N₅O₈ | 1156.82 | 7.35 | 25.88 |
| 2278 | C₄₉H₉₃NO₇S | 840.35 | 9.87 | 18.01 | 2478 | C₆₄H₁₂₃NO₁₀S | 1098.75 | 8.66 | 26.59 |
| 2279 | C₅₀H₉₂N₄O₆S₃ | 941.51 | 8.18 | 17.07 | 2479 | C₆₈H₁₃₀N₄O₅S₅ | 1292.14 | 8.76 | 29.68 |
| 2280 | C₇₃H₁₃₅NO₅ | 1106.88 | 9.49 | 28.47 | 2480 | C₇₀H₁₂₉N₃O₉S₄ | 1285.08 | 7.88 | 28.4 |
| 2281 | C₇₀H₁₃₂N₄O₃S₂ | 1141.98 | 9.64 | 24.97 | 2481 | C₇₁H₁₄₆NO₁₃P₃ S | 1346.93 | 9.92 | 26.28 |
| 2282 | C₇₀H₁₂₆N₄O₅S₃ | 1200 | 8.18 | 25.9 | 2482 | C₆₅H₁₃₁NO₇S₅ | 1199.1 | 9.82 | 22.46 |
| 2283 | C₇₆H₁₃₈N₃O₈PS₃ | 1349.13 | 8.02 | 27.79 | 2483 | C₆₂H₁₂₈N₄O₇S₅ | 1202.06 | 9.66 | 24.8 |
| 2284 | C₇₀H₁₂₅N₃O₂S₄ | 1169.06 | 8.09 | 24.8 | 2484 | C₆₇H₁₃₆N₂O₂S₃ | 1098.04 | 9.6 | 31.2 |
| 2285 | C₆₉H₁₃₀N₄O₅S | 1127.89 | 8.62 | 22.64 | 2485 | C₆₇H₁₃₄N₄O₂S₆ | 1220.24 | 8.81 | 29.65 |
| 2286 | C₆₆H₁₂₉N₇O₄S | 1116.87 | 8.42 | 23.8 | 2486 | C₅₀H₉₆N₄O₅ | 833.34 | 7.85 | 14.83 |
| 2287 | C₆₉H₁₃₁N₅O₈S | 1190.9 | 8.72 | 26.48 | 2487 | C₄₉H₁₀₁N₈O₈P | 961.37 | 7.1 | 14.9 |
| 2288 | C₆₉H₁₃₂N₄O₉S | 1193.9 | 9.26 | 26.76 | 2488 | C₄₈H₁₀₀N₄S | 765.42 | 10.17 | 17.18 |
| 2289 | C₆₇H₁₃₄N₄O₂S | 1059.9 | 9.61 | 25.59 | 2489 | C₅₀H₉₉N₅O₁₂S₃ | 1058.57 | 7.89 | 15.01 |
| 2290 | C₇₁H₁₃₈N₆O₈S₃ | 1300.12 | 8.97 | 23.96 | 2490 | C₅₃H₁₀₀N₆O₉ | 965.42 | 8.13 | 16.59 |
| 2291 | C₆₈H₁₃₆N₄O₁₀S₄ | 1298.12 | 9.75 | 23.58 | 2491 | C₄₉H₉₆N₆O₇ | 881.34 | 8.08 | 16.13 |
| 2292 | C₇₂H₁₃₀N₄O₉S₂ | 1259.98 | 8.25 | 26.34 | 2492 | C₅₂H₉₆N₄O₇S₄ | 1017.63 | 8.99 | 20.64 |
| 2293 | C₇₀H₁₂₇N₃O₁₁ | 1186.8 | 8.54 | 23.99 | 2493 | C₅₀H₉₁N₃O₇S₅ | 1006.63 | 8.54 | 18.41 |
| 2294 | C₇₂H₁₃₁NO₉S₃ | 1251.04 | 7.85 | 29.49 | 2494 | C₄₆H₉₃NO₅S | 772.32 | 9.48 | 16 |
| 2295 | C₇₀H₁₃₆NO₆P | 1118.83 | 9.96 | 26.88 | 2495 | C₄₉H₁₀₂NO₁₀P₃ S₂ | 1022.41 | 9.45 | 15.88 |
| 2296 | C₇₅H₁₅₀NO₁₁P₃S | 1367.01 | 10.01 | 26.33 | 2496 | C₄₄H₉₀N₂O₁₁S₅ | 983.54 | 7.61 | 14.61 |
| 2297 | C₆₈H₁₂₉N₅O₁₀S₃ | 1273.01 | 8.02 | 23.99 | 2497 | C₅₀H₉₇N₃O₁₃S₅ | 1108.67 | 9.1 | 18.9 |
| 2298 | C₆₂H₁₂₁NO₇S₃ | 1088.85 | 8.69 | 22.15 | 2498 | C₄₆H₉₆N₄O₇S₅ | 977.63 | 9.97 | 15.88 |
| 2299 | C₇₂H₁₃₄N₂O₄S₃ | 1188.07 | 8.69 | 30.53 | 2499 | C₅₁H₉₇NO₈S₃ | 948.54 | 9.74 | 20.24 |
| 2300 | C₇₀H₁₃₀N₂O₈S₄ | 1256.08 | 9.65 | 29.21 | 2500 | C₄₈H₈₉NO₉S₃ | 920.44 | 7.12 | 19.34 |
| 2301 | C₇₀H₁₂₉NO₆S₄ | 1209.07 | 9.97 | 29.9 | 2501 | C₅₀H₉₈N₂O₅S₇ | 1031.81 | 10.01 | 21.72 |
| 2302 | C₇₃H₁₃₃N₃O₆S₄ | 1277.15 | 8.91 | 29.86 | 2502 | C₄₉H₈₉N₃O₅S₇ | 1024.74 | 7.38 | 20.15 |
| 2303 | C₇₀H₁₂₇N₃O₅S₇ | 1315.27 | 8.15 | 29.29 | 2503 | C₈₈H₁₇₄N₄O₁₀S | 1480.44 | 8.64 | 37.76 |
| 2304 | C₆₉H₁₃₃NO₂S₄ | 1137.1 | 10.08 | 29.8 | 2504 | C₉₁H₁₈₀NO₁₂P | 1511.41 | 9.64 | 38.49 |
| 2305 | C₇₂H₁₄₂N₂O₆S | 1212 | 10.03 | 27.69 | 2505 | C₈₇H₁₇₂N₂O₁₃S₃ | 1550.51 | 7.44 | 36.68 |
| 2306 | C₇₀H₁₃₅NO₂S₃ | 1119.06 | 9.94 | 28.41 | 2506 | C₈₉H₁₇₇NO₁₃S₄ | 1597.63 | 9.89 | 39.95 |
| 2307 | C₇₄H₁₄₃NS₄ | 1175.23 | 10.31 | 30.33 | 2507 | C₈₇H₁₇₉N₅O₈S₄ | 1551.66 | 9.72 | 35.89 |
| 2308 | C₇₃H₁₄₂N₆O₉S | 1280.04 | 8.87 | 27.51 | 2508 | C₉₁H₁₇₃N₃O₅S₇ | 1613.82 | 8.49 | 39.46 |
| 2309 | C₇₁H₁₄₃N₇O₂ | 1126.97 | 8.8 | 25.07 | 2509 | C₈₆H₁₇₃NO₂S₃ | 1349.52 | 8.69 | 40.39 |
| 2310 | C₆₇H₁₃₆N₆O₄ | 1089.86 | 7.38 | 25.24 | 2510 | C₈₇H₁₇₇NO₃S₄ | 1413.62 | 9.8 | 39.79 |
| 2311 | C₆₅H₁₃₆N₄O₂S | 1037.9 | 9.89 | 25.86 | 2511 | C₆₈H₁₃₈N₆OS | 1087.95 | 8.34 | 25.64 |
| 2312 | C₇₀H₁₃₃NO₆S | 1116.9 | 9.78 | 27.91 | 2512 | C₇₃H₁₄₁N₅O₇S | 1233.02 | 8.19 | 27.42 |
| 2313 | C₇₂H₁₃₉NO₈ | 1146.9 | 8.29 | 30.37 | 2513 | C₇₆H₁₄₈N₆O₁₀ | 1306.05 | 8.77 | 28.04 |
| 2314 | C₇₂H₁₃₇N₃O₈S | 1204.97 | 8.1 | 27.01 | 2514 | C₇₇H₁₄₅N₅O₁₂ | 1333.03 | 8.22 | 27.36 |
| 2315 | C₇₀H₁₃₅NO₈S₃ | 1215.05 | 8.87 | 31.23 | 2515 | C₇₂H₁₃₉NO₇S₄ | 1259.14 | 9.28 | 30.05 |
| 2316 | C₆₉H₁₃₄N₂O₉S₃ | 1232.04 | 8.56 | 29.17 | 2516 | C₇₄H₁₄₈N₃O₆P | 1206.99 | 9.19 | 27.09 |
| 2317 | C₆₇H₁₃₁N₃O₆ | 1074.8 | 8.05 | 24.99 | 2517 | C₇₃H₁₄₃N₃O₇S₅ | 1335.26 | 9 | 27.79 |
| 2318 | C₇₁H₁₄₁N₃O₆ | 1132.92 | 8.87 | 27 | 2518 | C₇₄H₁₄₄N₂O₅S₅ | 1302.27 | 8.94 | 31.06 |
| 2319 | C₆₉H₁₃₈N₄O₁₅S₃ | 1360.08 | 8.57 | 27.02 | 2519 | C₇₄H₁₄₁NO₈S₄ | 1301.18 | 9.84 | 32.75 |
| 2320 | C₇₆H₁₅₂N₃O₁₁P₃ S₂ | 1441.12 | 8.65 | 25.02 | 2520 | C₇₅H₁₄₃N₅O₆S₆ | 1403.36 | 8.02 | 29.84 |
| 2321 | C₇₀H₁₄₅N₅O₆S₃ | 1249.16 | 10.17 | 27.3 | 2521 | C₇₅H₁₄₃NOS₆ | 1267.34 | 10.03 | 31.57 |
| 2322 | C₇₀H₁₃₃NO₅S₇ | 1293.3 | 9.72 | 32.57 | 2522 | C₆₉H₁₃₅N₃OS₈ | 1231.28 | 9.26 | 31.21 |
| 2323 | C₉₈H₁₉₃N₃O₅ | 1493.64 | 9.64 | 41.19 | 2523 | C₆₉H₁₃₅N₃OS₈ | 1279.34 | 8.11 | 32.33 |
| 2324 | C₉₇H₁₉₂N₂O₃ | 1434.61 | 9.81 | 43.28 | 2524 | C₉₁H₁₅₁NO₆ | 1355.21 | 9.47 | 35.21 |
| 2325 | C₁₀₀H₁₉₃N₃O₃S₅ | 1646 | 9.08 | 44.26 | 2525 | C₈₉H₁₅₁NO₈S | 1395.25 | 9.1 | 33.28 |
| 2326 | C₁₀₀H₁₉₄N₂O₂S₄ | 1584.93 | 8.96 | 42.63 | 2526 | C₉₃H₁₅₆N₆O₅ | 1438.31 | 8.9 | 31.09 |
| 2327 | C₉₇H₁₉₀N₂OS₆ | 1593 | 7.95 | 44.89 | 2527 | C₉₀H₁₄₉NO₈S | 1405.24 | 9.77 | 36.24 |
| 2328 | C₁₀₁H₁₉₈N₈O₆S | 1652.81 | 9.03 | 41.03 | 2528 | C₉₁H₁₅₇N₇O₄ | 1413.3 | 9.81 | 33.65 |
| 2329 | C₉₈H₁₉₃N₅O₈ | 1569.65 | 9.94 | 42.17 | 2529 | C₉₃H₁₅₈N₆O₉S | 1536.38 | 9.06 | 33.18 |
| 2330 | C₉₈H₁₉₆N₆O₂S | 1522.75 | 8.97 | 42.54 | 2530 | C₈₈H₁₅₄N₆O₂ | 1328.24 | 9.69 | 33.16 |
| 2331 | C₉₅H₁₈₇NO₈S | 1503.61 | 8.62 | 43.07 | 2531 | C₈₉H₁₅₈N₄O₇S₅ | 1556.56 | 9.19 | 36.7 |
| 2332 | C₁₀₀H₁₉₆N₄O₁₀ | 1614.69 | 8.95 | 42.32 | 2532 | C₉₁H₁₅₅N₅O₈ | 1447.27 | 9.75 | 33.92 |
| 2333 | C₉₆H₁₈₉NO₉S₂ | 1565.7 | 9.9 | 43.36 | 2533 | C₉₁H₁₅₃N₃O₁₀S | 1481.3 | 9.45 | 33.16 |
| 2334 | C₉₈H₁₈₉NO₈S₃ | 1605.79 | 7.84 | 44.98 | 2534 | C₈₈H₁₅₄N₄O₅ | 1348.22 | 9.44 | 33.71 |
| 2335 | C₁₀₄H₂₁₀N₃O₁₂P₃ | 1787.75 | 8.95 | 40.77 | 2535 | C₈₅H₁₄₉NO₁₁S₄ | 1489.36 | 9.07 | 32.92 |
| 2336 | C₉₅H₁₉₂NO₉PS₃ | 1619.75 | 9.61 | 38.95 | 2536 | C₈₃H₁₄₅NO₂S₃ | 1285.26 | 8.63 | 34.83 |
| 2337 | C₉₆H₁₉₆N₆O₉S₃ | 1674.86 | 9.53 | 39.5 | 2537 | C₈₉H₁₅₂N₄O₂S₆ | 1502.58 | 8.69 | 33.48 |
| 2338 | C₉₆H₁₉₄N₆O₉S₄ | 1704.91 | 8.66 | 39.31 | 2538 | C₈₇H₁₅₃NO₄S₇ | 1501.6 | 9.11 | 35.87 |
| 2339 | C₉₇H₁₉₇NO₉S₅ | 1681.98 | 10.29 | 43.64 | 2539 | C₉₀H₁₅₃N₃O₂S₇ | 1533.65 | 8.67 | 35.66 |
| 2340 | C₁₀₀H₁₉₃N₅O₉S₃ | 1705.87 | 8.02 | 43.88 | 2540 | C₉₀H₁₅₅N₃O₃S₇ | 1551.67 | 8.78 | 37.08 |
| 2341 | C₉₃H₁₈₅NO₂S₄ | 1477.78 | 8.83 | 44.69 | 2541 | C₅₀H₉₂N₄O₈ | 877.31 | 7.61 | 14.76 |
| 2342 | C₉₆H₁₉₅NS₅ | 1523.96 | 10.32 | 46.5 | 2542 | C₅₄H₁₀₁N₃O₁₀S | 984.47 | 8.61 | 17.32 |
| 2343 | C₉₆H₁₉₂N₄O₂S₇ | 1659.09 | 9.15 | 45.27 | 2543 | C₅₅H₁₀₇N₃OS₃ | 922.66 | 10.35 | 19.69 |
| 2344 | C₇₂H₁₃₇N₇O₆ | 1196.93 | 9.55 | 23.01 | 2544 | C₇₃H₁₃₃N₃O₇ | 1164.88 | 8.78 | 26.27 |
| 2345 | C₇₅H₁₃₆N₆O₆S | 1250.02 | 8.12 | 23.71 | 2545 | C₇₂H₁₃₉N₄O₆P | 1187.9 | 8.5 | 25.02 |
| 2346 | C₇₃H₁₃₃N₃O₃S₄ | 1229.15 | 8.6 | 25.6 | 2546 | C₇₆H₁₄₅N₉O₆ | 1281.05 | 8.95 | 25.9 |
| 2347 | C₇₂H₁₃₄N₂O₂S₆ | 1252.28 | 8.91 | 31.69 | 2547 | C₇₂H₁₃₃N₅O₁₀ | 1228.88 | 7.28 | 26.13 |
| 2348 | C₇₃H₁₃₅NO₂S₇ | 1283.36 | 10.25 | 31.89 | 2548 | C₇₅H₁₄₈N₄O₂ | 1138.04 | 10.25 | 29.53 |
| 2349 | C₇₄H₁₃₈N₆O₈S | 1272.02 | 8.96 | 26.06 | 2549 | C₇₂H₁₃₈N₆O₃ | 1135.93 | 8.47 | 25.31 |
| 2350 | C₇₁H₁₃₂N₆O₈ | 1197.87 | 8.73 | 24.03 | 2550 | C₇₂H₁₃₁N₃O₁₁S | 1246.91 | 7.71 | 26.16 |
| 2351 | C₇₁H₁₃₇N₅O₈S | 1220.97 | 10.21 | 27.15 | 2551 | C₇₆H₁₃₅N₃O₁₃ | 1298.92 | 8.06 | 26.23 |
| 2352 | C₇₀H₁₂₇NO₇S | 1126.85 | 8.28 | 27.4 | 2552 | C₇₃H₁₃₃N₃O₁₃S | 1292.93 | 8.53 | 24.6 |
| 2353 | C₆₆H₁₂₄N₄O₃S₂ | 1085.88 | 7.45 | 24.89 | 2553 | C₇₂H₁₃₅NO₁₃S | 1254.93 | 9.37 | 28.42 |
| 2354 | C₆₉H₁₃₃NO₃S₂ | 1088.96 | 10.01 | 27.96 | 2554 | C₇₅H₁₄₉N₂O₁₁P₃ | 1347.94 | 8.85 | 27.16 |
| 2355 | C₇₃H₁₄₁N₂O₁₃P₃ S | 1379.92 | 7.73 | 24.08 | 2555 | C₇₅H₁₄₈N₃O₁₀P₃ S | 377 | 9.09 | 25.62 |
| 2356 | C₇₂H₁₃₆N₄O₁₅S₄ | 1426.16 | 8.39 | 26.1 | 2556 | C₇₂H₁₃₅N₃O₉S₄ | 1315.12 | 8.02 | 23.79 |
| 2357 | C₆₆H₁₂₇NO₈S₄ | 1191.01 | 9.56 | 23.38 | 2557 | C₆₇H₁₃₃N₅O₇S₃ | 1217.01 | 9.55 | 24.42 |
| 2358 | C₆₉H₁₃₄N₆O₈S₄ | 1304.13 | 8.63 | 23.43 | 2558 | C₆₈H₁₃₇N₇O₈S₄ | 1309.12 | 8.59 | 23.56 |
| 2359 | C₇₀H₁₃₄N₂O₁₁S₃ | 1276.05 | 9.01 | 25.11 | 2559 | C₆₆H₁₃₀N₄O₉S₄ | 1252.03 | 7.7 | 24.69 |
| 2360 | C₇₁H₁₂₉N₃O₆S₆ | 1313.23 | 7.83 | 29.32 | 2560 | C₆₉H₁₃₁NO₁₀S₄ | 1263.04 | 9.72 | 26.82 |
| 2361 | C₇₅H₁₃₈N₃O₇PS₆ | 1417.32 | 8.79 | 29.2 | 2561 | C₆₉H₁₃₄N₂O₁₁S₄ | 1296.07 | 9.65 | 25.47 |
| 2362 | C₇₃H₁₂₈N₆O₆S₇ | 1410.33 | 8.05 | 29.45 | 2562 | C₇₂H₁₃₇N₃O₁₄S₄ | 1397.13 | 7.96 | 24.84 |
| 2363 | C₆₉H₁₃₃N₃O₄S₃ | 1165.04 | 8.75 | 27.33 | 2563 | C₇₆H₁₃₄N₆O₅S₆ | 1404.3 | 8.44 | 29.07 |
| 2364 | C₇₀H₁₃₃N₂O₄PS₃ | 1194.02 | 8.64 | 27.09 | 2564 | C₇₅H₁₃₈N₄O₄S₇ | 1384.37 | 7.99 | 27.97 |
| 2365 | C₆₈H₁₃₁N₅O₅ | 1098.83 | 8.96 | 24.38 | 2565 | C₇₂H₁₃₃NO₂S₇ | 1269.28 | 9.22 | 29.39 |
| 2366 | C₆₆H₁₃₀N₄O₄ | 1043.79 | 9.95 | 23.32 | 2566 | C₇₆H₁₃₉NS₈ | 1323.43 | 9.99 | 32.54 |
| 2367 | C₆₂H₁₂₄N₄O₅S | 1037.76 | 8.99 | 22.55 | 2567 | C₆₈H₁₃₂N₂OS₆ | 1186.18 | 9.61 | 29.43 |
| 2368 | C₆₇H₁₃₀N₆O₃S₂ | 1131.95 | 9.38 | 22.39 | 2568 | C₇₆H₁₄₆N₆O₈ | 1272.04 | 7.91 | 25.83 |
| 2369 | C₆₇H₁₃₁N₅O₅S₂ | 1150.95 | 8.8 | 22.94 | 2569 | C₇₄H₁₄₂N₂O₆S₃ | 1252.14 | 7.97 | 29.92 |
| 2370 | C₆₅H₁₃₀N₄O₃S₂ | 1079.91 | 10.12 | 23.75 | 2570 | C₇₆H₁₄₇N₃O₇S₄ | 1343.27 | 8.74 | 29.77 |
| 2371 | C₆₄H₁₂₁NO₅S₄ | 1112.94 | 9.72 | 26.66 | 2571 | C₆₈H₁₃₃NO₆S₄ | 1189.05 | 9.29 | 28.76 |
| 2372 | C₆₃H₁₂₇N₇O₆S | 1110.82 | 8.83 | 20.79 | 2572 | C₇₃H₁₄₅N₅O₅ | 1172.99 | 9.61 | 27.7 |
| 2373 | C₆₃H₁₂₆N₆OS₂ | 1047.88 | 8.76 | 23.9 | 2573 | C₇₁H₁₃₉N₅O₁₀S | 1254.98 | 7.51 | 27.38 |
| 2374 | C₆₁H₁₁₉NO₁₄S | 1122.68 | 7.69 | 23.72 | 2574 | C₆₉H₁₄₂N₄O₂ | 1059.92 | 10.05 | 27.68 |
| 2375 | C₆₆H₁₂₈N₃O₁₃P | 1202.73 | 7.46 | 24.54 | 2575 | C₇₁H₁₄₄N₆O₃S | 1162.03 | 8.83 | 25.65 |
| 2376 | C₇₃H₁₄₅N₄O₁₁P₃ | 1347.9 | 8.61 | 22.75 | 2576 | C₇₁H₁₄₈N₄O₁₀S₄ | 1346.22 | 9.78 | 25.99 |
| 2377 | C₇₂H₁₅₀NO₁₃P₃S | 1362.97 | 10.23 | 26.58 | 2577 | C₇₅H₁₄₁N₃O₉S₂ | 1293.09 | 9.29 | 29.12 |
| 2378 | C₅₈H₁₂₁N₅O₈S₃ | 1112.83 | 7.95 | 21.32 | 2578 | C₇₅H₁₄₆N₃O₁₀P S₃ | 1377.16 | 8.69 | 28.51 |
| 2379 | C₆₁H₁₂₈N₄O₉S₄ | 1189.98 | 9.93 | 23.2 | 2579 | C₆₉H₁₃₈N₄O₅S | 1135.95 | 7.81 | 25.35 |
| 2380 | C₇₉H₁₅₀N₆O₅S₂ | 1328.24 | 8.02 | 27.18 | 2580 | C₈₀H₁₆₁N₃O₁₃P₄ | 1497.07 | 8.34 | 25.4 |
| 2381 | C₈₀H₁₅₆N₆O₇ | 1314.16 | 8.79 | 28.87 | 2581 | C₆₈H₁₃₈N₂O₁₃S₄ | 1320.09 | 7.81 | 24.98 |
| 2382 | C₇₇H₁₅₃N₅O₉S | 1325.16 | 8.63 | 27.52 | 2582 | C₇₈H₁₅₆N₃O₁₁P₃ | 1405.04 | 8.9 | 25.51 |
| 2383 | C₇₉H₁₅₆N₄O₅S | 1274.21 | 9.99 | 30.04 | 2583 | C₇₃H₁₄₃N₉O₇₈S₅ | 1419.3 | 8.5 | 27.26 |
| 2384 | C₇₈H₁₄₈N₂O₃S₇ | 1386.52 | 8.53 | 34.02 | 2584 | C₇₅H₁₄₆N₄O₄S₃ | 1264.2 | 9.75 | 28.36 |
| 2385 | C₇₇H₁₄₈N₂O₅S₇ | 1406.51 | 8.37 | 33.09 | 2585 | C₇₆H₁₄₁N₇O₅S₃ | 1329.19 | 8.44 | 6.51 |
| 2386 | C₇₈H₁₄₉N₃S₇ | 1353.54 | 9.35 | 33.71 | 2586 | C₇₃H₁₄₃N₃S₈ | 1319.45 | 9.73 | 31.55 |
| 2387 | C₈₀H₁₅₅N₇O₅ | 1295.16 | 9.05 | 28.08 | 2587 | C₆₈H₁₃₇NO₂S₇ | 1225.27 | 9.56 | 32.84 |
| 2388 | C₇₇H₁₅₅N₇O₃S₂ | 1291.27 | 9.72 | 30.97 | 2588 | C₁₀₀H₁₉₂N₂O₄S | 1518.71 | 9.01 | 41.94 |
| 2389 | C₇₇H₁₅₃N₉O₂S | 1269.2 | 8.46 | 28.74 | 2589 | C₉₈H₁₈₉NOS₅ | 1557.9 | 7.61 | 43.84 |
| 2390 | C₇₆H₁₅₆N₄O₇S₃ | 1334.31 | 8.94 | 31.72 | 2590 | C₉₈H₁₉₄NO₃PS₃ | 1561.79 | 8.48 | 42.9 |
| 2391 | C₇₆H₁₅₄N₆O₈S₃ | 1376.3 | 8.64 | 28.57 | 2591 | C₉₉H₁₉₄N₄O₇S | 1584.72 | 9.65 | 43.1 |
| 2392 | C₇₈H₁₄₆N₂O₈ | 1240.03 | 8.71 | 30.11 | 2592 | C₉₉H₁₉₄N₄O₈S₂ | 1632.78 | 7.76 | 43.54 |
| 2393 | C₇₆H₁₄₈NO₉P | 1250.99 | 8.86 | 31.56 | 2593 | C₉₆H₁₉₆N₄O₃S | 1486.71 | 9.59 | 42.11 |
| 2394 | C₇₈H₁₅₀N₆O₉S | 1348.16 | 7.69 | 29.53 | 2594 | C₉₇H₁₉₅N₅O₄S | 1527.72 | 8.65 | 40.06 |
| 2395 | C₇₇H₁₅₀N₄O₇S | 1276.13 | 9.43 | 30.93 | 2595 | C₁₀₂H₁₉₉N₅O₉ | 1639.74 | 8.75 | 41.94 |
| 2396 | C₇₇H₁₄₇NO₇S₄ | 1327.29 | 9.86 | 34.36 | 2596 | C₉₈H₁₉₇N₄O₁₀P | 1622.65 | 8.51 | 41 |
| 2397 | C₇₅H₁₂₀NO₁₁5₄ | 1369.28 | 9.91 | 31.06 | 2597 | C₉₉H₁₉₅NO₆S₄ | 1623.89 | 10.09 | 47.23 |
| 2398 | C₇₉H₁₆₂NO₁₁P₃S | 1427.15 | 9.02 | 30.52 | 2598 | C₁₀₅H₂₁₄NO₁₀P₃ | 1743.79 | 10.19 | 43.28 |
| 2399 | C₇₆H₁₅₇N₆O₁₀PS₃ | 1442.3 | 8.06 | 28.96 | 2599 | C₉₈H₁₉₃N₃O₁₂S₄ | 1733.87 | 8 | 41.24 |
| 2400 | C₇₅H₁₄₉N₃O₁₀S₅ | 1413.36 | 8.76 | 28.82 | 2600 | C₆₃H₁₀₇NO₆S | 1006.62 | 9.8 | 21.64 |
| 2601 | C₆₅H₁₁₂N₃O₄PS₆ | 1223 | 7.95 | 21.13 | 2801 | C₉₉H₂₀₀N₆O₁₀S₄ | 1762.96 | 8.4 | 40.16 |
| 2602 | C₆₆H₁₁₉N₇O₅ | 1090.72 | 10 | 21.99 | 2802 | C₁₀₀H₁₉₉N₃O₁₁S₃ | 1715.88 | 8.98 | 40.36 |
| 2603 | C₆₁H₁₁₄N₄O₇S₅ | 1175.94 | 9.86 | 19.74 | 2803 | C₉₈H₂₀₁N₄O₁₂P S₃ | 1754.86 | 9.49 | 39.38 |
| 2604 | C₆₁H₁₁₂N₆O₁S₄ | 1233.87 | 7.61 | 17.67 | 2804 | C₉₉H₁₉₅N₃OS₉ | 1732.21 | 8.95 | 48.29 |
| 2605 | C₅₈H₁₁₀N₄O₉S₄ | 1135.8 | 7.67 | 19.8 | 2805 | C₇₇H₁₂₁N₃O₈S | 1248.89 | 8.03 | 26.31 |
| 2606 | C₆₆H₁₁₆N₆O₈S | 1153.75 | 8.85 | 21.05 | 2806 | C₇₂H₁₁₇NO₃S₅ | 1205.03 | 10.07 | 28.49 |
| 2607 | C₆₂H₁₀₆N₂O₁₁ | 1055.53 | 9.6 | 20.49 | 2807 | C₇₄H₁₁₇N₃OS₄ | 1193.01 | 8.45 | 25.19 |
| 2608 | C₆₃H₁₀₉NO₈S₃ | 1104.76 | 9.87 | 23.85 | 2808 | C₇₅H₁₂₁NO₃S₇ | 1309.22 | 9.65 | 27.97 |
| 2609 | C₆₃H₁₀₈N₂O₁₀S₄ | 1181.83 | 8.42 | 23.27 | 2809 | C₇₉H₁₃₄N₉O₆PS | 1369.03 | 8.95 | 25.87 |
| 2610 | C₆₅H₁₀₉N₃O₇S₅ | 1204.94 | 8.71 | 22.79 | 2810 | C₇₉H₁₃₀N₆O₉ | 1307.94 | 8.95 | 26.58 |
| 2611 | C₆₆H₁₂₂NO₁₀P₃S | 1214.69 | 9.74 | 20.96 | 2811 | C₇₆H₁₃₅N₄O₄P | 1199.91 | 10.01 | 25.75 |
| 2612 | C₆₃H₁₁₁N₃O₉S₅ | 1214.93 | 9.29 | 21.07 | 2812 | C₈₁H₁₂₆N₆O₉ | 1327.93 | 8.71 | 26.39 |
| 2613 | C₆₃H₁₁₃N₃O₁₁S₄ | 1216.87 | 8.22 | 20.49 | 2813 | C₇₇H₁₂₆N₄O₆S₂ | 1268 | 9.98 | 27.83 |
| 2614 | C₆₅H₁₂₂NO₁₁P₃S | 1218.68 | 8.62 | 19.71 | 2814 | C₇₅H₁₂₄N₄O₈ | 1209.84 | 8.73 | 27.26 |
| 2615 | C₆₀H₁₁₂NO₉PS₃ | 1118.73 | 10 | 17.5 | 2815 | C₇₁H₁₁₉NO₆ | 1082.73 | 8.65 | 26.27 |
| 2616 | C₆₃H₁₀₈N₆O₁₁S₄ | 1253.86 | 8.07 | 20.48 | 2816 | C₈₁H₁₃₈N₃O₁₂P₃ S | 1470.99 | 8.02 | 25.79 |
| 2617 | C₆₃H₁₁₃N₂O₅PS₃ | 1105.78 | 7.69 | 21.48 | 2817 | C₇₈H₁₃₅N₄O₁₁P₃ S | 1429.94 | 8.22 | 25.01 |
| 2618 | C₆₁H₁₀₉N₃O₂S₄ | 1044.83 | 8.31 | 22.42 | 2818 | C₇₄H₁₂₈NO₁₁P₃ S | 1332.82 | 8.72 | 26.25 |
| 2619 | C₆₀H₁₀₅N₃O₄S₆ | 1124.92 | 7.15 | 23.05 | 2819 | C₇₁H₁₂₁NO₁₀S₃ | 1244.93 | 9.53 | 26.84 |
| 2620 | C₅₆H₁₀₄N₂O₅S | 917.52 | 9.66 | 18.46 | 2820 | C₇₆H₁₃₀NO₁₂PS₃ | 1377.02 | 9.99 | 26.39 |
| 2621 | C₅₇H₁₀₀N₂O₁₀ | 973.43 | 7.44 | 19.35 | 2821 | C₆₉H₁₁₇NO₁₆S₄ | 1344.93 | 8.21 | 27.4 |
| 2622 | C₅₇H₁₀₁NOS₄ | 944.71 | 8.76 | 21.3 | 2822 | C₇₆H₁₃₀N₄O₈S₃ | 1324.08 | 10.01 | 27.21 |
| 2623 | C₆₁H₁₀₉N₃O₃S₆ | 1124.96 | 8.89 | 21.53 | 2823 | C₇₁H₁₂₁N₇O₈S₅ | 1361.09 | 7.65 | 25.06 |
| 2624 | C₇₂H₁₃₁N₃O₄S | 1134.92 | 8.9 | 25.07 | 2824 | C₇₄H₁₂₀N₂O₅S₃ | 1213.96 | 9.95 | 27.23 |
| 2625 | C₇₃H₁₃₇N₇O₆ | 1208.94 | 8.96 | 23.56 | 2825 | C₇₇H₁₂₀N₆O₇S₄ | 1370.08 | 7.8 | 26.86 |
| 2626 | C₇₂H₁₂₇N₇O₉ | 1234.85 | 8.1 | 24.19 | 2826 | C₇₃H₁₂₀N₂O_{S}S₄ | 1234.01 | 9.58 | 27.28 |
| 2627 | C₇₃H₁₃₅N₃O₉S | 1230.96 | 9.09 | 25.63 | 2827 | C₇₆H₁₂₄N₃O₁₀P S₃ | 1366.99 | 7.57 | 26.82 |
| 2628 | C₆₈H₁₂₇NO₃S₅ | 1167.1 | 9.7 | 29.88 | 2828 | C₇₂H₁₂₃NO₃S₇ | 1275.2 | 9.67 | 28.9 |
| 2629 | C₆₈H₁₂₇N₃S₇ | 1211.25 | 9.6 | 27.84 | 2829 | C₇₄H₁₂₃NO₂S₇ | 1283.22 | 9.86 | 30.94 |
| 2630 | C₇₁H₁₃₄NO₁₀P | 1192.82 | 9.64 | 24.22 | 2830 | C₇₂H₁₄₄N₂O₃ | 1083.94 | 9.36 | 28.29 |
| 2631 | C₆₈H₁₂₈N₄O₆S₂ | 1161.93 | 10.07 | 26.57 | 2831 | C₆₉H₁₃₄N₂O₅S | 1103.9 | 9.68 | 26.54 |
| 2632 | C₇₀H₁₃₀N₂O₈S₃ | 1224.02 | 9.71 | 26.56 | 2832 | C₇₁H₁₃₆N₂O₇S₂ | 1194 | 8.99 | 28.63 |
| 2633 | C₇₂H₁₃₁N₃O₉S₃ | 1279.05 | 8.83 | 27.55 | 2833 | C₆₅H₁₂₇NO₆S₂ | 1082.85 | 9.93 | 28.44 |
| 2634 | C₆₈H₁₂₅NO₇S₅ | 1229.08 | 9.96 | 29.51 | 2834 | C₇₂H₁₃₃N₃O₆S₃ | 1233.05 | 7.96 | 27.03 |
| 2635 | C₆₉H₁₃₀N₄O₄ | 1079.82 | 8.27 | 25.32 | 2835 | C₇₃H₁₃₉N₃O₆S₃ | 1251.11 | 8.84 | 28.79 |
| 2636 | C₆₈H₁₃₂N₂O₁₄S₃ | 1298 | 10.19 | 27.01 | 2836 | C₇₃H₁₃₉NO₅S₄ | 1239.16 | 10.13 | 31.72 |
| 2637 | C₇₂H₁₄₀N₃O₁₂P₃ | 1332.84 | 7.62 | 23.57 | 2837 | C₆₉H₁₃₁NOS₅ | 1151.11 | 9.73 | 29.4 |
| 2638 | C₇₁H₁₃₅N₇O₉S₃ | 1327.1 | 8.42 | 23.39 | 2838 | C₆₉H₁₃₆N₂S₆ | 1186.22 | 10.2 | 29.92 |
| 2639 | C₆₉H₁₃₁N₃O₁₂S₄ | 1323.08 | 8.78 | 25.51 | 2839 | C₆₈H₁₃₉N₉O₃S₂ | 1195.04 | 9.53 | 26.63 |
| 2640 | C₇₁H₁₂₇N₃O₇S₄ | 1263.08 | 7.7 | 27.37 | 2840 | C₇₀H₁₃₄N₆O₇S | 1203.94 | 8.24 | 27.19 |
| 2641 | C₇₂H₁₃₀N₄O₉S₃ | 1292.05 | 8.32 | 28.21 | 2841 | C₇₀H₁₃₃N₅O₉S | 1220.92 | 8.39 | 26.98 |
| 2642 | C₆₉H₁₂₉N₃O₆S₇ | 1321.27 | 9.35 | 27.16 | 2842 | C₆₉H₁₄₂N₆O₇S₃ | 1264.11 | 9.97 | 26.55 |
| 2643 | C₇₀H₁₂₉NS₈ | 1241.29 | 10.16 | 30.09 | 2843 | C₆₅H₁₃₇N₅O₉S₄ | 1261.08 | 8.86 | 24.06 |
| 2644 | C₅₈H₁₀₈N₄O₈S | 1021.59 | 10.16 | 30.09 | 2844 | C₇₁H₁₃₇NO₉ | 1148.88 | 10.05 | 28.77 |
| 2645 | C₅₆H₁₁₁N₆O₄P | 963.52 | 7.19 | 17.58 | 2845 | C₆₉H₁₃₇N₃O₆ | 1104.87 | 8.83 | 26.42 |
| 2646 | C₅₅H₁₀₆N₄O₆S₅ | 1079.81 | 9.87 | 19.57 | 2846 | C₇₂H₁₄₄N₃O₁₀P₃ | 1304.88 | 7.67 | 25.5 |
| 2647 | C₅₆H₁₀₇N₇O₈S₃ | 1102.71 | 8.21 | 17.61 | 2847 | C₇₁H₁₄₀N₂O₁₁P₃ S | 1331.96 | 8.9 | 26.6 |
| 2648 | C₅₄H₁₀₂N₆O₈S₄ | 1091.71 | 7.81 | 16.98 | 2848 | C₆₆H₁₃₄NO₁₂PS₃ | 1260.95 | 9.07 | 25.89 |
| 2649 | C₅₈H₁₀₇N₇O₈S | 1062.6 | 8.83 | 17.19 | 2849 | C₆₃H₁₃₂N₄O₁₀S₄ | 1234.01 | 8.1 | 24.36 |
| 2650 | C₅₄H₁₀₄N₂O₅S | 893.5 | 9.68 | 18.78 | 2850 | C₇₂H₁₃₇NO₃S₅ | 1225.19 | 9.9 | 31.49 |
| 2651 | C₆₄H₁₂₄N₃O₁₄P₃ S | 1284.69 | 8.2 | 17.97 | 2851 | C₇₀H₁₃₅NO₃S₅ | 1199.15 | 9.97 | 30.96 |
| 2652 | C₅₅H₁₀₃N₃O₁₀S₅ | 1126.77 | 8.47 | 18.72 | 2852 | C₇₁H₁₃₅N₃OS₆ | 1239.24 | 9.56 | 28.4 |
| 2653 | C₅₅H₁₀₀N₆O₁₃S₄ | 1181.7 | 8.28 | 18.03 | 2853 | C₅₃H₁₀₀N₆O₅ | 901.42 | 8.32 | 14.69 |
| 2654 | C₅₅H₁₀₆N₄O₇S₄ | 1063.74 | 9.8 | 19.25 | 2854 | C₅₀H₁₀₃N₁₁O₅ | 938.45 | 8.48 | 15.09 |
| 2655 | C₅₉H₁₀₄N₄O₄S₃ | 1029.71 | 9.13 | 19.59 | 2855 | C₅₂H₁₀₀N₆O₉S | 985.46 | 8.31 | 17.51 |
| 2656 | C₅₆H₁₀₃NO₇S₄ | 1030.71 | 9.87 | 21.24 | 2856 | C₅₃H₁₀₁N₃O₇ | 892.4 | 9.95 | 18.39 |
| 2657 | C₅₇H₁₀₄N₂OS₆ | 1025.87 | 10.01 | 20.81 | 2857 | C₅₁H₉₇NO₁₂S | 948.39 | 9.3 | 17.75 |
| 2658 | C₇₂H₁₂₈N₂OS₃ | 1134.03 | 10.16 | 24.3 | 2858 | C₅₀H₉₅NO₁₀S₄ | 998.55 | 8.15 | 20.65 |
| 2659 | C₆₉H₁₂₈N₄OS | 1061.88 | 10.02 | 24.28 | 2859 | C₅₄H₁₁₂NO₁₁P₃ S | 1076.47 | 9.21 | 18.64 |
| 2660 | C₇₀H₁₂₉N₅O₇S₅ | 1313.16 | 8.99 | 22.78 | 2860 | C₅₃H₁₀₀N₆O₁₀S₄ | 1109.65 | 8.67 | 15.63 |
| 2661 | C₇₂H₁₃₃N₄O₉P | 1229.85 | 9.96 | 23.06 | 2861 | C₅₀H₁₀₁NO₁₄S₄ | 1068.59 | 10.07 | 20.13 |
| 2662 | C₆₈H₁₂₀N₂O₁₁ | 1141.71 | 9.11 | 21.06 | 2862 | C₅₁H₁₀₂N₂O₁₄S₅ | 1127.68 | 8.8 | 20.04 |
| 2663 | C₇₂H₁₂₅N₃O₉S₃ | 1273 | 9.1 | 26.58 | 2863 | C₅₁H₁₀₀N₆O₉S₄ | 1069.63 | 8.09 | 16.22 |
| 2664 | C₇₂H₁₂₅NO₈S₃ | 1228.99 | 10 | 27.22 | 2864 | C₄₆H₉₆N₄O₆S₅ | 961.6 | 9.69 | 17.23 |
| 2665 | C₇₅H₁₄₁N₂O₁₄P₃ S | 1419.94 | 8.57 | 22.34 | 2865 | C₅₂H₁₀₂N₄O₁₂S₃ | 1071.58 | 8.63 | 16.45 |
| 2666 | C₆₇H₁₂₁N₃O₁₁S₄ | 1272.95 | 7.36 | 19.18 | 2866 | C₄₇H₉₁NO₃S₅ | 878.55 | 9.65 | 20.22 |
| 2667 | C₆₉H₁₂₆N₆O₇S₄ | 1280.04 | 9 | 22.36 | 2867 | C₅₁H₉₆N₄OS₆ | 973.72 | 9.5 | 17.13 |
| 2668 | C₆₉H₁₃₀N₄O₆S₅ | 1272.12 | 10.21 | 24.79 | 2868 | C₄₉H₉₉NO₃S₃ | 846.51 | 9.67 | 20.47 |
| 2669 | C₆₇H₁₁₇NO₆S₄ | 1160.91 | 8.83 | 24.72 | 2869 | C₄₈H₉₃N₃OS₇ | 952.71 | 7.9 | 20.75 |
| 2670 | C₇₃H₁₂₉N₃O₄S₅ | 1273.15 | 8.98 | 26.99 | 2870 | C₆₈H₁₃₂N₂O₆S₃ | 1169.99 | 8.2 | 27.11 |
| 2671 | C₆₇H₁₁₉NO₇S₇ | 1275.11 | 7.43 | 24.74 | 2871 | C₆₉H₁₃₁N₅O₇S₄ | 1271.07 | 8.05 | 28 |
| 2672 | C₇₀H₁₂₁N₃O₄S₇ | 1293.17 | 8.47 | 24.89 | 2872 | C₇₄H₁₄₇N₄O₁₃P₃ S | 1425.99 | 8.26 | 21.77 |
| 2673 | C₆₇H₁₁₉N₃O₃S₄ | 1142.95 | 7.75 | 24.17 | 2873 | C₇₈H₁₅₅NO₁₃P₄ S | 1471.05 | 9.23 | 26.84 |
| 2674 | C₆₆H₁₁₉NO₂S₇ | 1183.1 | 9.76 | 27.41 | 2874 | C₇₄H₁₄₇N₂O₁₂P₃ S | 1381.97 | 7.99 | 25.04 |
| 2675 | C₇₄H₁₄₂N₄O₉S | 1264.03 | 8.4 | 26.85 | 2875 | C₈₀H₁₄₁N₇O₁₂S | 1425.1 | 8.28 | 27.74 |
| 2676 | C₇₂H₁₃₅N₃O₇S | 1186.95 | 7.5 | 28.39 | 2876 | C₇₅H₁₃₇N₅O₁₂S | 1333 | 7.02 | 25.8 |
| 2677 | C₇₄H₁₃₈N₄O₂S₃ | 1212.12 | 7.44 | 26.73 | 2877 | C₇₅H₁₄₆N₅O₈PS | 1309.05 | 9.53 | 24.72 |
| 2678 | C₇₅H₁₃₉N₃O₄S₄ | 1275.19 | 7.91 | 28.49 | 2878 | C₅₂H₁₀₁NO₁₁S₂ | 980.5 | 7.66 | 18.55 |
| 2679 | C₇₂H₁₃₆N₂O₂S₆ | 1254.25 | 8.75 | 29.44 | 2879 | C₆₈H₁₁₅NO₇S₄ | 1186.91 | 9.71 | 24.43 |
| 2680 | C₉₁H₁₇₇NO₄S | 1381.48 | 9.69 | 39.41 | 2880 | C₆₅H₁₁₃NO₁₀S₅ | 1228.92 | 7.12 | 22.76 |
| 2681 | C₈₉H₁₇₂N₂O₇ | 1382.36 | 9.42 | 37.27 | 2881 | C₆₅H₁₂₂NO₁₂P₃ S | 1234.67 | 7.76 | 19.16 |
| 2682 | C₉₁H₁₇₆N₆O₇ | 1466.44 | 8.11 | 36.76 | 2882 | C₆₈H₁₁₅N₃O₁₂S | 1198.74 | 8.37 | 22.77 |
| 2683 | C₉₂H₁₇₈N₃O₁₀P | 1517.42 | 8.01 | 36.76 | 2883 | C₇₅H₁₃₀N₃O₆PS₄ | 1329.09 | 8.51 | 25.28 |
| 2684 | C₉₁H₁₇₇N₃O₇S₄ | 1553.67 | 8.56 | 37.79 | 2884 | C₇₆H₁₃₆N₂O₁₀S₅ | 1398.23 | 9.74 | 30.25 |
| 2685 | C₉₃H₁₇₉N₃O₃S₃ | 1483.65 | 8.65 | 37.45 | 2885 | C₇₀H₁₃₀NO₁₂PS₅ | 1369.08 | 9.35 | 22.77 |
| 2686 | C₉₀H₁₇₄N₄O₂S₆ | 1536.77 | 9.13 | 38.77 | 2886 | C₆₈H₁₂₇N₃O₈S₅ | 1275.08 | 7.49 | 18.47 |
| 2687 | C₈₉H₁₇₆N₄O₄ | 1366.41 | 9.65 | 36.39 | 2887 | C₅₆H₁₀₆N₄O₅S₂ | 979.61 | 8.6 | 19.07 |
| 2688 | C₈₈H₁₇₆N₄O₇S | 1434.46 | 7.44 | 34.46 | 2888 | C₅₇H₁₀₄N₄O₇S₄ | 1085.72 | 8.19 | 20.14 |
| 2689 | C₉₀H₁₈₀N₁₀O₃S | 1482.56 | 9.39 | 37.26 | 2889 | C₆₁H₁₀₆N₄O₈S₃ | 1119.72 | 7.67 | 21.03 |
| 2690 | C₉₃H₁₈₅N₉O₆S | 1557.62 | 8.83 | 35.92 | 2890 | C₅₉H₁₁₁N₃O₁₄S₆ | 1278.9 | 8.13 | 18.51 |
| 2691 | C₉₁H₁₈₀N₄O₈ | 1458.46 | 9.68 | 39.2 | 2891 | C₅₇H₁₀₇N₃O₁₂S₅ | 1186.79 | 8.93 | 18.22 |
| 2692 | C₈₄H₁₇₄N₄O₁₀S₄ | 1528.57 | 9.73 | 35.64 | 2892 | C₅₈H₁₀₈N₂O₄S₆ | 1089.87 | 9.71 | 23.62 |
| 2693 | C₉₁H₁₇₇NO₈ | 1413.42 | 9.74 | 39.59 | 2893 | C₇₅H₁₃₃N₃O₁₂S₃ | 1365.08 | 7.62 | 28.16 |
| 2694 | C₉₁H₁₇₇NO₈ | 1413.42 | 9.63 | 39.59 | 2894 | C₇₃H₁₄₄N₅O₇PS | 1267.02 | 8.77 | 23.15 |
| 2695 | C₉₄H₁₉₁NO₁₄S₄ | 1687.8 | 8.92 | 40.68 | 2895 | C₇₀H₁₃₅NO₃S₈ | 1295.33 | 9.28 | 29.89 |
| 2696 | C₉₈H₂₀₀N₄O₉S₃ | 1674.88 | 10.05 | 42.36 | 2896 | C₇₁H₁₄₅N₃OS₄ | 1185.2 | 10.13 | 32.12 |
| 2697 | C₁₀₁H₁₉₄N₂O₆S₄ | 1660.91 | 8.5 | 44.51 | 2897 | C₆₉H₁₃₈NO₁₃PS₅ | 1381.13 | 8.17 | 26.27 |
| 2698 | C₉₅H₁₈₇NO₂S₇ | 1599.97 | 7.87 | 43.73 | 2898 | C₇₆H₁₂₈N₄O₇S | 1241.94 | 9.76 | 26.18 |
| 2699 | C₉₆H₁₉₄N₂O₃S₇ | 1649.04 | 8.91 | 43.81 | 2899 | C₇₇H₁₂₈N₄O₁₀S₃ | 1366.07 | 9.13 | 28.18 |
| 2700 | C₇₃H₁₃₅N₃O₇S | 1198.96 | 8.83 | 27.72 | 2900 | C₇₈H₁₂₉N₇O₁₁S₄ | 1469.17 | 7.08 | 24.13 |
| 2701 | C₇₃H₁₃₂N₄O₂S₃ | 1194.07 | 8.26 | 26.95 | 2901 | C₇₆H₁₃₀N₆O₁₀S₆ | 1480.27 | 8.32 | 26.25 |
| 2702 | C₇₄H₁₄₃N₇O₆ | 1227 | 10.05 | 27.15 | 2902 | C₇₉H₁₂₈N₆O₁₂S₄ | 1482.16 | 8.21 | 26.97 |
| 2703 | C₇₂H₁₃₈N₄O₈S | 1219.98 | 9.16 | 26.82 | 2903 | C₇₃H₁₃₅N₅O₄ | 1206.97 | 9.64 | 29.41 |
| 2704 | C₇₀H₁₃₃N₅OS₂ | 1124.99 | 8.67 | 26.08 | 2904 | C₈₀H₁₂₀N₇O₇ | 1300.95 | 8.42 | 24.11 |
| 2705 | C₇₀H₁₃₀N₄O₆S | 1155.89 | 9.03 | 26.72 | 2905 | C₉₂H₁₅₆N₂O₅S₅ | 1530.57 | 8.87 | 38.21 |
| 2706 | C₇₁H₁₃₃N₅O₉S | 1232.93 | 7.56 | 25.84 | 2906 | C₉₅H₁₆₀N₃O₁₂P S₃ | 1663.49 | 7.57 | 35.94 |
| 2707 | C₇₂H₁₃₄N₄O₁₀S | 1247.94 | 9.32 | 26.54 | 2907 | C₉₁H₁₅₇NO₁₇S₅ | 1697.55 | 9.77 | 37.07 |
| 2708 | C₇₂H₁₃₂N₂O₁₂S | 1249.91 | 8.5 | 26.64 | 2908 | C₉₂H₁₅₉NOS₉ | 1583.83 | 9.44 | 41.5 |
| 2709 | C₆₈H₁₃₁NO₁₅S₃ | 1298.97 | 8.73 | 27.84 | 2909 | C₈₀H₁₅₇N₃O₁₁S₂ | 1401.27 | 7.36 | 30.07 |
| 2710 | C₇₀H₁₃₃N₃O₁₅S₄ | 1385.08 | 8.44 | 27.06 | 2910 | C₈₀H₁₆₁N₇O₉S | 1397.27 | 9.05 | 31.05 |
| 2711 | C₈₀H₁₅₇N₃O₁₄P₄ | 1509.04 | 8.07 | 24.7 | 2911 | C₈₆H₁₇₂N₃O₁₄P₃ S₂ | 1629.37 | 7.98 | 30.43 |
| 2712 | C₆₉H₁₂₉N₃O₉S₄ | 1273.04 | 8.14 | 23.02 | 2912 | C₈₂H₁₅₉NO₈S | 1319.23 | 9.17 | 32.07 |
| 2713 | C₇₁H₁₃₉N₃O₁₀S₃ | 1291.08 | 10.2 | 26.84 | 2913 | C₈₂H₁₅₇NO₉S₂ | 1365.28 | 8.94 | 34.05 |
| 2714 | C₇₁H₁₃₀N₆O₉S₅ | 1372.15 | 7.66 | 25.94 | 2914 | C₈₂H₁₅₅NO₁₀S₃ | 1411.32 | 9.46 | 33.89 |
| 2715 | C₇₁H₁₃₁NO₄S₃ | 1159.01 | 9.05 | 28.83 | 2915 | C₈₂H₁₅₆N₆O₈ | 1354.18 | 7.58 | 27.67 |
| 2716 | C₇₄H₁₃₆N₄O₅S₃ | 1258.11 | 9.28 | 26.98 | 2916 | C₁₀₂H₂₀₀N₄O₆S | 1610.8 | 9.3 | 41.11 |
| 2717 | C₇₄H₁₄₀NO₆PS₆ | 1363.27 | 10.18 | 30.16 | 2917 | C₉₉H₁₉₉N₅O₁₂S₃ | 1747.88 | 8.74 | 39.52 |
| 2718 | C₇₂H₁₄₁NO₃S₄ | 1197.16 | 10.22 | 30.35 | 2918 | C₉₈H₂₀₁N₅O₁₂S₅ | 1802.01 | 7.78 | 38.29 |
| 2719 | C₇₄H₁₂₀N₂O₃S₆ | 1298.3 | 9.07 | 30.62 | 2919 | C₁₀₄H₂₀₂N₃O₁₃P S₂ | 1797.86 | 8.16 | 42.89 |
| 2720 | C₆₈H₁₃₃N₃O₅S | 1104.89 | 9.56 | 23.55 | 2920 | C₇₂H₁₄₃N₅O₆ | 1174.96 | 7.02 | 24.11 |
| 2721 | C₆₈H₁₃₁NO₂S₃ | 1090.98 | 10.2 | 27.08 | 2921 | C₇₅H₁₄₃NOS₄ | 1203.22 | 9.9 | 30.72 |
| 2722 | C₆₆H₁₂₇N₅O₇ | 1102.77 | 7.4 | 22.13 | 2922 | C₇₆H₁₄₆N₄O₈S₅ | 1404.32 | 7.57 | 29.76 |
| 2723 | C₆₇H₁₃₄N₆O₄S | 1119.91 | 8.03 | 22.12 | 2923 | C₇₆H₁₄₆N₄O₉S₃ | 1356.2 | 8.27 | 30.39 |
| 2724 | C₆₅H₁₃₅N₅O₉S₃ | 1227 | 9.81 | 23.41 | 2924 | C₇₈H₁₆₁N₂O₁₁P₃ S | 1428.13 | 9.86 | 28.23 |
| 2725 | C₆₇H₁₂₇N₃O₇ | 1086.77 | 9.14 | 23.56 | 2925 | C₇₇H₁₅₆NO₁₁P₃ S₂ | 1429.13 | 9.8 | 27.74 |
| 2726 | C₆₅H₁₂₆N₂O₁₂S | 1159.78 | 7.67 | 23.67 | 2926 | C₇₂H₁₄₆N₆O₉S₄ | 1368.23 | 7.44 | 25.66 |
| 2727 | C₆₆H₁₂₃N₃O₈S₅ | 1247.02 | 8.3 | 26.43 | 2927 | C₇₅H₁₄₇N₅O₇ | 1231.03 | 8.75 | 27.63 |
| 2728 | C₆₈H₁₄₁N₂O₁₀P₃ | 1239.8 | 9.68 | 23.81 | 2928 | C₇₁H₁₄₃NO₅S₃ | 1187.11 | 9.63 | 29.7 |
| 2729 | C₆₃H₁₂₅N₃O₁₄S₄ | 1276.94 | 7.77 | 24.47 | 2929 | C₇₇H₁₄₆N₂O₄S₆ | 1356.38 | 8.92 | 30.9 |
| 2730 | C₆₇H₁₃₃N₃O₇S₄ | 1221.05 | 8.93 | 22.48 | 2930 | C₇₅H₁₄₅N₃O₈S₃ | 1313.18 | 9.69 | 30.82 |
| 2731 | C₆₃H₁₂₃N₃O₃S₆ | 1163.05 | 7.51 | 26.47 | 2931 | C₇₄H₁₄₂N₆O₁₁S₅ | 1452.28 | 7.2 | 26.83 |
| 2732 | C₆₄H₁₃₁NO₃S₇ | 1187.18 | 9.65 | 28.98 | 2932 | C₁₀₇H₂₁₀N₄O₆S | 1680.94 | 9.3 | 44.75 |
| 2733 | C₆₃H₁₂₇NOS₈ | 1171.19 | 10.03 | 29.92 | 2933 | C₁₀₃H₂₀₅N₅OS₇ | 1754.23 | 7.43 | 49.14 |
| 2734 | C₈₂H₁₆₂NO₆P | 1289.17 | 10.2 | 32.78 | 2934 | C₁₀₈H₂₁₂NO₇PS | 1699.92 | 7.96 | 46.71 |
| 2735 | C₇₉H₁₅₃NO₄S₂ | 1245.22 | 9.98 | 34.17 | 2935 | C₉₉H₂₀₀N₄O₂S₃ | 1574.89 | 9.31 | 44.17 |
| 2736 | C₇₉H₁₅₁N₃O₁₀S | 1335.15 | 7.16 | 30.27 | 2936 | C₁₀₀H₁₉₉N₇O₁₁S₄ | 1803.97 | 7.82 | 41.43 |
| 2737 | C₇₉H₁₅₁N₂O₁₀P | 1320.05 | 7.65 | 29.97 | 2937 | C₉₇H₁₉₇NO₁₉S₅ | 1841.93 | 9.29 | 43.58 |
| 2738 | C₇₉H₁₅₂N₄O₅S₃ | 1334.29 | 8.33 | 31.06 | 2938 | C₉₉H₁₉₄N₂O₈S | 1572.71 | 7.31 | 41.98 |
| 2739 | C₇₇H₁₄₆NO₄S₃ | 1249.22 | 9.44 | 32.44 | 2939 | C₉₄H₁₇₈N₄O₅S₃ | 1540.66 | 7.7 | 37.45 |
| 2740 | C₇₈H₁₅₃N₉O₃S₃ | 1361.32 | 8.05 | 31.75 | 2940 | C₇₄H₁₃₇N₃O₄S₅ | 1293.23 | 8.28 | 28.37 |
| 2741 | C₇₅H₁₅₀N₆O₉S₃ | 1376.24 | 7.11 | 28.59 | 2941 | C₇₈H₁₅₁N₂O₁₄P₃ S | 1466.05 | 8.41 | 24.98 |
| 2742 | C₈₀H₁₅₄N₄O₉ | 1316.13 | 8.39 | 31.34 | 2942 | C₇₃H₁₄₁N₇O₈S₃ | 1341.15 | 9.86 | 23.7 |
| 2743 | C₇₈H₁₅₃NO₈S | 1265.14 | 9.73 | 30.75 | 2943 | C₇₆H₁₃₆N₄O₈S | 1266 | 7.96 | 27.68 |
| 2744 | C₇₇H₁₅₁NO₆S₂ | 1251.18 | 10.04 | 33.91 | 2944 | C₆₉H₁₃₅N₅O₈S₃ | 1259.05 | 9.93 | 26.72 |
| 2745 | C₇₈H₁₅₆N₄O₉S | 1326.18 | 9.48 | 31.83 | 2945 | C₆₉H₁₃₅NO₃S₆ | 1219.2 | 10.26 | 30.26 |
| 2746 | C₈₀H₁₅₂N₂O₁₀S₄ | 1430.34 | 8.79 | 32.39 | 2946 | C₆₅H₁₂₉NO₃S₆ | 1165.11 | 10.03 | 29.3 |
| 2747 | C₇₉H₁₆₀N₄O₄ | 1230.17 | 9.9 | 31.06 | 2947 | C₇₀H₁₃₇N₄O₁₁P S₄ | 1370.1 | 9.59 | 27.67 |
| 2748 | C₈₂H₁₆₈NO₁₁P₃S | 1469.22 | 9.35 | 31.16 | 2948 | C₇₃H₁₄₉N₂O₁₃P S | 1387.98 | 9.89 | 25.38 |
| 2749 | C₇₄H₁₄₉NO₉S₄ | 1325.24 | 9.52 | 31.12 | 2949 | C₆₆H₁₃₀N₆O₆S | 1135.86 | 8.58 | 21.52 |
| 2750 | C₇₆H₁₅₁N₃O₉S₃ | 1347.24 | 7.62 | 28.28 | 2950 | C₆₉H₁₃₂N₂O₈S | 1149.88 | 9.31 | 25.41 |
| 2751 | C₈₀H₁₆₃NO₉S₄ | 1411.42 | 10.02 | 31.61 | 2951 | C₆₇H₁₃₀NO₁₁P | 1156.75 | 9.2 | 24.28 |
| 2752 | C₇₇H₁₅₅N₇O₈S₃ | 1403.31 | 8.62 | 28.92 | 2952 | C₇₄H₁₄₁N₄O₇PS | 1262 | 10.07 | 25.5 |
| 2753 | C₇₇H₁₅₅NO₁₂S₄ | 1415.32 | 9.9 | 31.42 | 2953 | C₇₉H₁₃₉N₇O₂S₈ | 1475.51 | 8.66 | 30.35 |
| 2754 | C₇₉H₁₅₀N₂O₄S₄ | 1320.32 | 8.2 | 32.38 | 2954 | C₈₀H₁₄₆N₃O₇PS₆ | 1485.4 | 8.03 | 29.1 |
| 2755 | C₈₀H₁₅₂N₂O₅S₅ | 1382.4 | 8.23 | 33.6 | 2955 | C₇₂H₁₃₉NO₁₄S₅ | 1403.2 | 10.24 | 26.99 |
| 2756 | C₈₂H₁₅₇N₃O₉S₄ | 1457.41 | 8.95 | 34.69 | 2956 | C₆₈H₁₃₄N₂O₁₁S₅ | 1316.12 | 9.97 | 23.02 |
| 2757 | C₇₈H₁₅₃N₂O₈PS₆ | 1470.42 | 7.17 | 32.63 | 2957 | C₇₃H₁₃₈N₄O₅S₇ | 1376.35 | 8.95 | 30.58 |
| 2758 | C₇₈H₁₄₇N₃OS₆ | 1335.41 | 8.18 | 32.47 | 2958 | C₇₆H₁₃₆N₂O₈S | 1237.99 | 8.81 | 25.65 |
| 2759 | C₇₈H₁₅₁N₇O₃S₆ | 1427.47 | 8.1 | 32.55 | 2959 | C₅₅H₁₀₅N₅O₉S | 1012.53 | 9.99 | 19.03 |
| 2760 | C₈₀H₁₅₇N₇OS₆ | 1425.54 | 9.93 | 32.94 | 2960 | C₅₅H₁₀₃N₃O₇S₈ | 1174.92 | 9.06 | 19.72 |
| 2761 | C₁₀₃H₁₉₈N₁₀O₄ | 1640.78 | 8.43 | 38.34 | 2961 | C₅₅H₉₈N₂O₇S₆ | 1091.76 | 8.02 | 19.51 |
| 2762 | C₉₉H₁₉₁NO₈S | 1555.68 | 9.65 | 43.86 | 2962 | C₅₆H₁₀₄N₂O₇S₄ | 1045.7 | 8.13 | 19.65 |
| 2763 | C₉₉H₁₉₁NO₇S₂ | 1571.74 | 9.34 | 44.32 | 2963 | C₅₅H₁₀₃N₃O₁₃S₄ | 1142.68 | 8.95 | 20.08 |
| 2764 | C₁₀₅H₂₀₅NO₅S₃ | 1657.98 | 10.13 | 47.57 | 2964 | C₄₉H₉₇NO₄S₅ | 924.62 | 7.78 | 18.16 |
| 2765 | C₁₀₀H₁₉₈NO₃PS₃ | 1589.84 | 8.58 | 43.46 | 2965 | C₆₀H₁₁₇N₄O₁₅P₃ | 1227.53 | 8.09 | 15.71 |
| 2766 | C₁₀₂H₂₀₀N₂OS₆ | 1663.09 | 10.1 | 45.62 | 2966 | C₅₃H₁₀₅NO₁₂S₅ | 1108.72 | 10.13 | 18.4 |
| 2767 | C₁₀₄H₂₀₅NO₆S | 1597.85 | 7.66 | 46.47 | 2967 | C₅₅H₁₀₈N₃O₁₆PS₅ | 1258.75 | 9.37 | 19.39 |
| 2768 | C₁₀₄H₂₀₉N₇O₃S₂ | 1669.98 | 9.88 | 46.29 | 2968 | C₅₄H₁₀₄N₄O₁₀S | 1001.5 | 8.74 | 17.69 |
| 2769 | C₁₀₅H₂₀₈N₆O₈ | 1682.85 | 9.03 | 44.74 | 2969 | C₅₄H₁₀₆N₂O₁₀S₂ | 1007.57 | 10.02 | 18.69 |
| 2770 | C₁₀₄H₂₀₄N₄O₇S₂ | 1686.92 | 9.42 | 47.15 | 2970 | C₆₆H₁₁₇N₅O₈S | 1140.75 | 9.75 | 19.41 |
| 2771 | C₁₀₉H₂₀₈N₂O₉ | 1690.87 | 8.92 | 46.22 | 2971 | C₆₈H₁₂₁N₇O₅ | 1116.76 | 8.93 | 17.67 |
| 2772 | C₁₀₅H₂₁₀N₄O₁₀S | 1720.91 | 9.84 | 46.22 | 2972 | C₆₅H₁₁₄N₂O₅S₅ | 1163.94 | 10.02 | 23.5 |
| 2773 | C₁₀₅H₂₀₁N₃O₁₁S | 1713.83 | 7.46 | 46.28 | 2973 | C₆₈H₁₁₄N₂O₉S₂ | 1167.78 | 8.32 | 21.74 |
| 2774 | C₁₀₂H₂₀₅N₃O₈S | 1633.83 | 8.04 | 44.66 | 2974 | C₇₁H₁₂₆NO₄PS₇ | 1313.19 | 10.09 | 25.85 |
| 2775 | C₁₀₉H₂₂₃N₂O₁₁P ₃S | 1862.97 | 9.95 | 45.08 | 2975 | C₇₄H₁₂₉N₃O₆S₉ | 1445.4 | 8.19 | 27.04 |
| 2776 | C₁₀₂H₂₀₈N₂O₉S₃ | 1702.97 | 10.14 | 46 | 2976 | C₇₄H₁₂₉NO₆S₂ | 1192.97 | 10.35 | 24.9 |
| 2777 | C₁₀₇H₂₀₈N₄O₃S₅ | 1759.17 | 8.34 | 49.21 | 2977 | C₇₃H₁₂₅N₃O₁₁S₂ | 1284.93 | 8.96 | 23.77 |
| 2778 | C₁₀₄H₂₀₃NO₁₀S₄ | 1756.01 | 9.55 | 48 | 2978 | C₇₁H₁₂₅N₅O₁₄S₂ | 1336.92 | 8.57 | 20.7 |
| 2779 | C₁₀₅H₂₀₃NO₇S₄ | 1720.02 | 8.74 | 49.41 | 2979 | C₅₉H₁₀₈N₂O₃S₄ | 1021.76 | 10.2 | 20.34 |
| 2780 | C₁₀₈H₂₁₃NO₆S₇ | 1846.31 | 10.05 | 50.72 | 2980 | C₆₁H₁₁₈NO₁₄PS₄ | 1248.82 | 10.25 | 22.37 |
| 2781 | C₁₀₃H₂₀₃N₃O₂S₃ | 1611.96 | 8.66 | 46.73 | 2981 | C₅₆H₁₀₆N₂O₁₁S₄ | 1111.71 | 8.96 | 17.71 |
| 2782 | C₁₀₄H₂₀₈N₂O₂S₃ | 1615 | 9.88 | 47.93 | 2982 | C₅₇H₁₀₈N₂O₁₅S₃ | 1157.67 | 10.05 | 20.1 |
| 2783 | C₁₀₁H₂₀₄N₂OS₆ | 1655.12 | 8.86 | 49.01 | 2983 | C₆₀H₁₁₀NO₇P | 988.51 | 10.15 | 18.89 |
| 2784 | C₁₀₁H₂₀₅NS₈ | 1690.24 | 9.98 | 51.49 | 2984 | C₆₁H₁₀₅N₇O₅ | 1016.55 | 8.67 | 17.66 |
| 2785 | C₁₀₂H₂₀₅N₆O₆P | 1642.77 | 9.54 | 39.05 | 2985 | C₆₁H₁₁₂N₂O₉S₂ | 1081.69 | 10.11 | 20.51 |
| 2786 | C₁₀₃H₂₀₃N₉O₅S₂ | 1711.93 | 8.13 | 42.14 | 2986 | C₅₉H₁₀₉N₇O₇S | 1060.62 | 8.74 | 15.29 |
| 2787 | C₉₉H₁₉₈N₈O₅ | 1580.72 | 9.68 | 41.14 | 2987 | C₇₅H₁₃₈N₆O₁₂ | 1315.96 | 8.8 | 26.03 |
| 2788 | C₁₀₀H₂₀₂N₇O₆P | 1629.73 | 9.66 | 40.59 | 2988 | C₇₃H₁₃₄N₃O₁₂P S₅ | 1437.16 | 8.84 | 29.13 |
| 2789 | C₁₀₃H₂₀₃N₉O₅S | 1679.88 | 8.7 | 41.15 | 2989 | C₇₀H₁₃₄N₄O₄S₃ | 1192.05 | 9.54 | 26.89 |
| 2790 | C₁₀₀H₁₉₄N₁₀O₈S | 1696.77 | 7.27 | 40.42 | 2990 | C₆₉H₁₂₉N₃O₁₀S₅ | 1321.1 | 9.31 | 24.87 |
| 2791 | C₉₈H₁₉₉N₆O₃PS | 1572.74 | 8.26 | 41.72 | 2991 | C₇₀H₁₄₁NO₄S | 1092.96 | 10.37 | 29.21 |
| 2792 | C₉₅H₁₉₄N₄O₈S₃ | 1616.8 | 8.77 | 39.52 | 2992 | C₇₀H₁₄₀N₁₀O₆S | 1250.01 | 9.15 | 24.15 |
| 2793 | C₉₅H₁₉₄N₆O₈S₃ | 1644.81 | 7.43 | 39.83 | 2993 | C₇₂H₁₃₇NO₄S₅ | 1241.19 | 9.95 | 30.25 |
| 2794 | C₁₀₀H₁₉₉N₅O₈ | 1599.72 | 10.02 | 42.8 | 2994 | C₇₁H₁₄₆N₆O₂ | 1115.99 | 10.21 | 26.99 |
| 2795 | C₁₀₄H₂₁₃N₄O₉P₃ S | 1788.85 | 9.7 | 40.93 | 2995 | C₇₇H₁₃₁N₃O₆S | 1226.97 | 9.99 | 28.8 |
| 2796 | C₉₄H₁₉₂N₂O₁₁S₄ | 1654.81 | 9.58 | 41.21 | 2996 | C₈₄H₁₄₁N₂O₁₂P₃ S₄ | 1592.22 | 8.25 | 26.39 |
| 2797 | C₁₀₀H₂₀₂NO₁₅PS₃ | 1785.86 | 9.99 | 43.01 | 2997 | C₇₆H₁₂₇N₇O₄ | 1202.9 | 9.28 | 25.07 |
| 2798 | C₉₄H₁₉₁NO₁₇S₄ | 1735.79 | 8.03 | 41.32 | 2998 | C₇₆H₁₂₁N₃O₇S₃ | 1285 | 8.58 | 26.2 |
| 2799 | C₉₆H₁₉₃NO₁₄S₅ | 1745.89 | 9.2 | 43.57 | 2999 | C₇₅H₁₁₉NO₁₀S | 1226.83 | 10.03 | 27.76 |
| 2800 | C₉₇H₁₉₈N₄O₈S₃ | 1644.85 | 10.17 | 41.87 | 3000 | C₇₇H₁₂₃NO₁₁S₃ | 1335.01 | 9.89 | 27.55 |
| 3001 | C₉₀H₁₅₇N₇O₁₀S₃ | 1593.47 | 8.81 | 31.48 | 3201 | C₇₂H₁₂₆N₂O₉ | 1163.81 | 9.97 | 23.54 |
| 3002 | C₈₉H₁₅₅NO₆S₈ | 1591.7 | 9.94 | 37.95 | 3202 | C₇₀H₁₂₄N₄O₈ | 1149.78 | 9.97 | 23.36 |
| 3003 | C₈₄H₁₆₈NO₁₀P₃S₃ | 1541.37 | 9.83 | 31.59 | 3203 | C₇₁H₁₂₅NO₁₄S | 1248.83 | 9.73 | 22.7 |
| 3004 | C₈₀H₁₆₃N₅O₈S₄ | 1451.45 | 10.12 | 31.84 | 3204 | C₇₀H₁₁₇N₃O₁₅ | 1240.71 | 7.26 | 20.93 |
| 3005 | C₈₀H₁₅₂N₄O₇ | 1282.12 | 8.76 | 29.06 | 3205 | C₅₇H₉₉N₃O₃S₈ | 1130.92 | 8.63 | 19.99 |
| 3006 | C₁₀₁H₁₉₈N₄O₅S₂ | 1612.84 | 9 | 42.07 | 3206 | C₆₃H₁₀₉N₆O₅PS₆ | 1253.94 | 8.12 | 21.93 |
| 3007 | C₁₀₃H₁₉₉N₃O₇S₄ | 1719.98 | 8.96 | 44.17 | 3207 | C₅₈H₁₀₅N₂O₄PS₆ | 1117.82 | 9.26 | 21.79 |
| 3008 | C₁₀₄H₁₉₆N₄O₁₂S₃ | 1790.91 | 8.4 | 43.89 | 3208 | C₅₉H₁₀₅NO₅S₄ | 1036.73 | 9.99 | 21.27 |
| 3009 | C₁₀₅H₂₀₈N₉O₁₂P S₄ | 1948.08 | 8.72 | 40.74 | 3209 | C₅₇H₁₀₃N₃O₁₀S₄ | 1118.7 | 9.68 | 21.46 |
| 3010 | C₇₉H₁₅₂N₆O₅S | 1298.18 | 9.08 | 25.22 | 3210 | C₅₉H₁₁₄NO₁₄P₃ S₂ | 1218.6 | 8.06 | 17.66 |
| 3011 | C₇₆H₁₅₂N₆O₁₀S | 1342.14 | 9.91 | 26.73 | 3211 | C₆₀H₁₁₆NO₁₂P₃ S₂ | 1200.62 | 8.76 | 19.01 |
| 3012 | C₇₃H₁₄₂N₂O₃S₆ | 1288.31 | 8.72 | 30.69 | 3212 | C₆₆H₁₂₉N₃ O₁₄P₄ | 1312.66 | 8.69 | 16.22 |
| 3013 | C₇₇H₁₄₅N₅O₁₀S₃ | 1397.21 | 8.79 | 29.01 | 3213 | C₆₂H₁₁₇N₂O₁₃P₃ | 1191.54 | 8.34 | 16.88 |
| 3014 | C₇₇H₁₄₁N₇O₆S₈ | 1517.5 | 8.62 | 30.23 | 3214 | C₅₅H₁₀₄N₆O₉S₃ | 1089.65 | 8.26 | 15.77 |
| 3015 | C₇₅H₁₅₂N₅O₁₂PS₃ | 1443.22 | 8.29 | 25.88 | 3215 | C₅₄H₁₀₂N₂O₁₂S₅ | 1131.71 | 8.76 | 17.82 |
| 3016 | C₇₄H₁₄₅N₃O₁₅S₄ | 1445.22 | 9.05 | 25.98 | 3216 | C₅₅H₁₀₃NO₁₅S₅ | 1178.72 | 9.92 | 19.53 |
| 3017 | C₇₂H₁₄₂N₂O₉S₆ | 1372.29 | 8.96 | 24.57 | 3217 | C₅₄H₁₀₅N₅O₄S | 920.52 | 9.05 | 18.7 |
| 3018 | C₇₇H₁₅₇N₉O₇S | 1353.22 | 10.26 | 29.33 | 3218 | C₅₃H₁₀₁NO₄S₉ | 1104.93 | 8.98 | 24.27 |
| 3019 | C₇₃H₁₄₅N₃O₅S₅ | 1305.28 | 8.8 | 30.66 | 3219 | C₆₀H₁₀₅N₂O₁₂P | 1077.48 | 8.29 | 18.94 |
| 3020 | C₇₄H₁₄₉N₇O₁₀S₄ | 1425.28 | 8.95 | 26.66 | 3220 | C₅₈H₁₀₅NO₁₆S | 1104.53 | 9.39 | 18.7 |
| 3021 | C₇₁H₁₃₉N₃O₁₆S₅ | 1451.2 | 8.48 | 24.73 | 3221 | C₇₀H₁₂₆N₆O₆S | 1179.87 | 7.23 | 23.31 |
| 3022 | C₇₄H₁₄₅N₃O₁₁S₆ | 1445.34 | 9.07 | 26.3 | 3222 | C₇₄H₁₃₄N₁₀O₁₁S | 1372 | 8.45 | 23.42 |
| 3023 | C₇₂H₁₄₅N₄O₁₀PS₃ | 1354.12 | 8.61 | 23.41 | 3223 | C₆₉H₁₂₃NS₇ | 1191.17 | 9.86 | 26.81 |
| 3024 | C₇₃H₁₄₇N₄O₉PS₅ | 1416.27 | 8.33 | 24.01 | 3224 | C₇₁H₁₂₆N₄O₄S₄ | 1228.05 | 7.91 | 25.16 |
| 3025 | C₇₄H₁₄₁NO₁₁S₂ | 1285.06 | 7.98 | 28.99 | 3225 | C₇₁H₁₂₈N₂O₅S₆ | 1282.17 | 9.3 | 28.34 |
| 3026 | C₁₀₉H₂₁₃N₅O₈ | 1721.93 | 8.96 | 42.65 | 3226 | C₇₇H₁₃₇N₆O₇PS₅ | 1450.25 | 8.46 | 25.3 |
| 3027 | C₁₀₄H₂₀₅NOS₇ | 1710.21 | 10.17 | 49.05 | 3227 | C₇₀H₁₂₅N₃O₁₀S₄ | 1297.02 | 7.45 | 26.12 |
| 3028 | C₁₁₁H₂₁₃N₃O₃S₈ | 1894.42 | 8.11 | 50.12 | 3228 | C₇₂H₁₃₁N₅O₁₁S₃ | 1339.04 | 8.71 | 27.24 |
| 3029 | C₁₁₀H₂₂₂N₃O₁₁P ₃S | 1887.98 | 8.96 | 42 | 3229 | C₇₅H₁₄₆N₇O₁₁P₃ | 1414.95 | 7.81 | 22.6 |
| 3030 | C₁₁₀H₂₂₂N₃O₁₁P ₃S | 1887.98 | 8.96 | 42 | 3230 | C₇₃H₁₄₀N₃O₁₄P₃ S | 1408.91 | 8.27 | 22.79 |
| 3031 | C₁₀₆H₂₁₁N₃O₁₃S ₂ | 1799.98 | 10.02 | 45.91 | 3231 | C₇₂H₁₄₀N₃O₁₄P₃ | 1364.84 | 8.46 | 23.22 |
| 3032 | C₁₀₃H₂₀₅N₆O₁₀P | 1718.78 | 8.96 | 40.53 | 3232 | C₇₀H₁₃₁N₅O₁₃S₄ | 1379.08 | 8.01 | 22.5 |
| 3033 | C₁₀₅H₂₁₃N₄O₁₃P ₃S₂ | 1896.92 | 8.94 | 39.79 | 3233 | C₆₇H₁₃₁N₅O₈S₅ | 1295.11 | 9.77 | 25.44 |
| 3034 | C₉₈H₁₉₂N₂O₁₂S₄ | 1718.86 | 9.02 | 40.76 | 3234 | C₆₅H₁₂₄N₂O₁₄S₅ | 1318.01 | 7.85 | 23.07 |
| 3035 | C₉₃H₁₈₀N₄O₅S | 1514.54 | 9.95 | 38.53 | 3235 | C₇₃H₁₃₉NO₉S₆ | 1367.27 | 10.04 | 28.32 |
| 3036 | C₉₆H₁₈₆N₂O₂S₈ | 1657.04 | 10.34 | 44.16 | 3236 | C₇₃H₁₃₀N₄O₈S₃ | 1288.04 | 8.18 | 26.62 |
| 3037 | C₉₄H₁₈₀N₄O₅S₇ | 1670.92 | 8.81 | 39.99 | 3237 | C₇₂H₁₃₂N₄O₁₀S | 1245.93 | 9.51 | 23.8 |
| 3038 | C₈₉H₁₇₇NO₁₅S₃ | 1597.57 | 10.12 | 38.26 | 3238 | C₇₀H₁₃₂NO₁₂PS ₂ | 1274.91 | 9.58 | 26.32 |
| 3039 | C₉₂H₁₇₇NO₆S₃ | 1489.61 | 9.88 | 38.99 | 3239 | C₇₃H₁₃₈N₆O₁₀S | 1292 | 8.5 | 25.82 |
| 3040 | C₉₃H₁₈₀N₂O₁₂S₂ | 1582.59 | 8.97 | 38.02 | 3240 | C₇₁H₁₃₇N₇O₁₂S | 1312.97 | 7.95 | 25.01 |
| 3041 | C₇₈H₁₃₉N₃O₆S₅ | 1375.28 | 9.03 | 28.75 | 3241 | C₇₀H₁₃₂N₂O₂S₅ | 1194.14 | 9.86 | 28.4 |
| 3042 | C₇₁H₁₃₈N₆O₁₃S₅ | 1444.21 | 8.79 | 24.44 | 3242 | C₆₈H₁₂₇N₃S₉ | 1275.32 | 8.31 | 29.94 |
| 3043 | C₇₄H₁₃₇NO₁₀S₃ | 1297.09 | 10.18 | 28.33 | 3243 | C₇₀H₁₃₂N₂O₉S₄ | 1274.07 | 8.35 | 27.5 |
| 3044 | C₅₇H₁₀₉N₅O₈S₅ | 1152.83 | 8.75 | 21.16 | 3244 | C₆₉H₁₃₄N₂O₄S₄ | 1184.08 | 8.84 | 29.64 |
| 3045 | C₅₆H₁₁₂N₆O₃S₂ | 981.67 | 8.79 | 18.08 | 3245 | C₇₁H₁₃₄N₂O₅S₇ | 1320.28 | 7.6 | 30.36 |
| 3046 | C₇₀H₁₂₄N₆O₅S₃ | 1225.98 | 9.62 | 21.57 | 3246 | C₇₀H₁₄₂NO₁₂P₃ S₂ | 1346.94 | 8.97 | 25.79 |
| 3047 | C₇₅H₁₄₀N₈O₅S₃ | 1330.18 | 10 | 23.56 | 3247 | C₇₂H₁₄₅N₂O₁₂P₃ S₂ | 1388 | 8.91 | 24.47 |
| 3048 | C₇₄H₁₃₉N₅O₁₁ | 1274.95 | 9.94 | 26.43 | 3248 | C₆₅H₁₃₄N₄O₉S₅ | 1276.11 | 8.95 | 25.16 |
| 3049 | C₇₅H₁₃₇N₃O₃S₇ | 1353.36 | 8.51 | 29.95 | 3249 | C₆₈H₁₄₁N₅O₉S₆ | 1365.26 | 9.94 | 24.68 |
| 3050 | C₇₁H₁₄₇N₅O₉S₆ | 1407.34 | 9.97 | 26.49 | 3250 | C₆₅H₁₃₀N₄O₁₆S₄ | 1352.01 | 8.42 | 25.08 |
| 3051 | C₈₁H₁₃₇N₃O₁₀S₅ | 1473.3 | 9.99 | 30.81 | 3251 | C₆₇H₁₃₅N₅OS₂ | 1090.97 | 9.62 | 24.49 |
| 3052 | C₈₂H₁₂₉N₃O₈S₇ | 1509.37 | 7.82 | 29.9 | 3252 | C₆₉H₁₃₈N₆O₅S | 1163.96 | 8.79 | 24.62 |
| 3053 | C₉₆H₁₆₅N₂O₁₁PS ₄ | 1682.59 | 9.47 | 37.52 | 3253 | C₇₀H₁₃₄N₄O₁₁S₂ | 1271.98 | 7.29 | 25.51 |
| 3054 | C₉₃H₁₆₃N₃O₃S₅ | 1531.64 | 8.96 | 37.35 | 3254 | C₇₁H₁₃₇NO₆S₂ | 1165 | 10.07 | 27.92 |
| 3055 | C₉₈H₁₇₂NO₁₀P₃S ₃ | 1713.55 | 7.92 | 34.58 | 3255 | C₇₄H₁₄₀N₄O₉S₂ | 1294.07 | 8 | 27.9 |
| 3056 | C₉₆H₁₆₀N₄O₆S | 1498.42 | 8.16 | 36.02 | 3256 | C₇₃H₁₃₉NO₁₀S₂ | 1255.03 | 10.04 | 28.57 |
| 3057 | C₈₇H₁₇₄NO₁₃P₃ | 1535.26 | 9.76 | 31.67 | 3257 | C₇₂H₁₄₁NO₁₃S | 1260.97 | 10.11 | 29.32 |
| 3058 | C₇₉H₁₅₃N₂O₇PS₃ | 1370.25 | 9.95 | 30.27 | 3258 | C₇₈H₁₂₆N₈O₉ | 1319.91 | 8.3 | 26.16 |
| 3059 | C₇₅H₁₄₈N₆O₈S₆ | 1454.4 | 8.59 | 27.41 | 3259 | C₇₆H₁₂₅N₅O₁₀S₂ | 1332.98 | 8.9 | 26.29 |
| 3060 | C₁₁₀H₂₂₀N₁₀O₇ | 1795.03 | 9.47 | 43.87 | 3260 | C₇₈H₁₃₄N₁₂O₄S₂ | 1368.13 | 9.28 | 25.15 |
| 3061 | C₁₁₀H₂₁₃N₃O₁₀S ₅ | 1898.22 | 8.79 | 48.01 | 3261 | C₇₇H₁₂₇N₉O₅S₂ | 1323.04 | 8.94 | 26.78 |
| 3062 | C₁₁₀H₂₂₃N₂O₁₁P ₃S₂ | 1907.04 | 9.9 | 44.27 | 3262 | C₇₇H₁₂₅N₂O₆PS ₈ | 1462.31 | 8.08 | 28.61 |
| 3063 | C₁₀₉H₂₁₄N₄O₆ | 1676.93 | 9.12 | 44.3 | 3263 | C₇₇H₁₂₃NO₄S₇ | 1351.25 | 9.51 | 29.37 |
| 3064 | C₁₀₇H₂₁₀N₆O₈ | 1708.89 | 10.02 | 43.55 | 3264 | C₇₇H₁₂₂N₄O₁₁S₄ | 1408.08 | 7.58 | 26.28 |
| 3065 | C₉₃H₁₈₅N₃O₁₅S₄ | 1713.75 | 8.68 | 36.47 | 3265 | C₈₁H₁₄₁N₆O₁₂P₃ | 1483.97 | 7.9 | 24.37 |
| 3066 | C₉₁H₁₈₃NO₁₆S₅ | 1707.76 | 9.33 | 39.98 | 3266 | C₇₃H₁₂₇N₅O₈S₄ | 1331.09 | 9.94 | 26.26 |
| 3067 | C₈₀H₁₄₆N₃O₉PS₃ | 1421.21 | 9.01 | 30.06 | 3267 | C₇₄H₁₃₂N₄O₁₂P₂ S₃ | 1428.01 | 9.34 | 25.27 |
| 3068 | C₇₄H₁₃₈N₂O₁₂S | 1279.98 | 9.95 | 27.25 | 3268 | C₇₆H₁₃₁N₃O₁₃S₄ | 1423.13 | 9.02 | 27.31 |
| 3069 | C₇₃H₁₄₈N₉O₁₁PS | 1391.07 | 8.77 | 22.93 | 3269 | C₇₅H₁₂₇N₅O₉S₃ | 1339.05 | 9.48 | 21.87 |
| 3070 | C₆₉H₁₃₄NO₈PS₄ | 1265.04 | 9.98 | 27.73 | 3270 | C₇₅H₁₂₂N₂O₈ | 1179.81 | 8.88 | 25.72 |
| 3071 | C₇₃H₁₄₂N₃O₁₁PS₃ | 1365.1 | 9.62 | 27.62 | 3271 | C₇₉H₁₂₈N₂O₁₂S₂ | 1362.01 | 9.85 | 28.34 |
| 3072 | C₅₆H₁₀₄N₄S₅ | 993.78 | 8.81 | 19.28 | 3272 | C₇₆H₁₂₄N₂O₁₁S₂ | 1305.95 | 9.98 | 27.72 |
| 3073 | C₆₈H₁₂₃N₂O₉P | 1143.71 | 8.74 | 20.97 | 3273 | C₉₁H₁₅₄N₆O₈S₂ | 1524.39 | 8.48 | 30.75 |
| 3074 | C₇₇H₁₃₇N₄O₁₂PS ₃ | 1438.11 | 8.96 | 25.55 | 3274 | C₉₂H₁₅₅N₉O₈ | 1515.31 | 8.03 | 32.69 |
| 3075 | C₇₄H₁₃₄N₄O₇S | 1223.97 | 10.08 | 21.69 | 3275 | C₉₃H₁₆₁N₄O₉PS | 1542.36 | 9.4 | 34.53 |
| .3076 | C₇₄H₁₃₈N₂O₆ | 1151.93 | 10.06 | 24.52 | 3276 | C₉₀H₁₅₃NO₄S₇ | 1537.64 | 9.51 | 36.88 |
| 3077 | C₉₄H₁₆₃N₅O₇S | 1507.43 | 10.19 | 33.88 | 3277 | C₉₄H₁₅₄N₂O₁₀S₅ | 1632.57 | 8.01 | 35.49 |
| 3078 | C₉₂H₁₆₃N₇O₈S₅ | 1655.66 | 9.65 | 32.84 | 3278 | C₉₄H₁₅₅N₉O₇S₇ | 1747.75 | 8.18 | 34.85 |
| 3079 | C₈₃H₁₅₉NO₇S₅ | 1443.48 | 10.08 | 34.53 | 3279 | C₉₁H₁₆₀N₂O₁₁S₆ | 1650.64 | 9.59 | 34.8 |
| 3080 | C₈₁H₁₆₆N₅O₁₁PS ₅ | 1577.52 | 8.86 | 29.78 | 3280 | C₈₈H₁₅₃N₃O₁₄S₃ | 1573.38 | 8.83 | 34.94 |
| 3081 | C₁₀₆H₂₀₅N₃O₅S₇ | 1826.24 | 9.06 | 44.87 | 3281 | C₈₇H₁₄₇NO₁₄S₄ | 1559.37 | 9.24 | 35.58 |
| 3082 | C₁₀₁H₁₉₉NO₇S₈ | 1796.18 | 10.22 | 47.81 | 3282 | C₉₀H₁₅₀N₄O₉S | 1464.27 | 9.12 | 33.75 |
| 3083 | C₁₀₂H₂₀₅NO₁₁S₅ | 1782.06 | 10.35 | 44.92 | 3283 | C₉₂H₁₅₈NO₉PS₂ | 1517.37 | 9.6 | 37.32 |
| 3084 | C₁₀₀H₁₉₉NO₂S₈ | 1704.18 | 7.88 | 48.22 | 3284 | C₉₂H₁₅₁NO₆S₄ | 1495.46 | 9.87 | 35.38 |
| 3085 | C₇₁H₁₄₆N₂O₁₃S₅ | 1396.25 | 9.98 | 26.51 | 3285 | C₉₀H₁₄₈N₄O₁₀S₂ | 1510.31 | 7.57 | 32.53 |
| 3086 | C₇₆H₁₄₈N₄O₁₂S₅ | 1470.34 | 8.2 | 25.26 | 3286 | C₉₀H₁₅₅N₅O₈S | 1467.32 | 8.94 | 33.22 |
| 3087 | C₁₁₁H₂₁₉N₂O₄PS ₆ | 1869.32 | 10.16 | 48.79 | 3287 | C₇₇H₁₅₅N₉O₇ | 1319.14 | 9.48 | 28.87 |
| 3088 | C₁₁₁H₂₁₃N₃O₁₀S ₅ | 1910.24 | 8.95 | 48.91 | 3288 | C₈₁H₁₅₂N₁₀O₁₀S | 1458.23 | 8.16 | 30.05 |
| 3089 | C₁₁₂H₂₁₅N₃O₁₂S ₃ | 1892.14 | 8.95 | 48.47 | 3289 | C₇₇H₁₅₃N₉O₆S₃ | 1397.31 | 7.51 | 28.31 |
| 3090 | C₁₀₅H₂₀₁N₃O₁₃S ₄ | 1842.01 | 8.05 | 44.33 | 3290 | C₇₉H₁₅₄N₈O₉ | 1360.15 | 7.29 | 28.84 |
| 3091 | C₉₃H₁₈₅N₃O₈S | 1505.58 | 8.82 | 36.48 | 3291 | C₇₇H₁₄₉NO₃S₈ | 1393.52 | 7.9 | 34.02 |
| 3092 | C₁₀₀H₁₉₉N₄O₁₃P ₃S₂ | 1822.75 | 8.21 | 37.37 | 3292 | C₇₉H₁₅₄N₆O₃S₆ | 1428.5 | 9.2 | 32.59 |
| 3093 | C₈₁H₁₄₆N₃O₁₂PS ₃ | 1481.22 | 8.46 | 28.15 | 3293 | C₇₆H₁₄₈N₂O₃S₇ | 1362.45 | 9.46 | 34.16 |
| 3094 | C₆₇H₁₂₆N₆O₅S₂ | 1159.9 | 8.5 | 24.22 | 3294 | C₈₂H₁₅₉N₃O₁₀P₂ S₃ | 1505.31 | 8.51 | 30.25 |
| 3095 | C₆₈H₁₃₃N₆O₉PS | 1241.88 | 7.67 | 19.74 | 3295 | C₇₈H₁₅₂N₂O₇S₄ | 1358.32 | 8.76 | 32.96 |
| 3096 | C₆₉H₁₃₄N₈O₇S₂ | 1252 | 8.41 | 22.96 | 3296 | C₇₉H₁₅₄N₄O₁₀S₄ | 1448.36 | 8.7 | 31.46 |
| 3097 | C₇₀H₁₃₁N₃O₆S₈ | 1367.31 | 7.69 | 26.18 | 3297 | C₇₉H₁₆₂NO₃PS₄ | 1333.38 | 9.86 | 33.98 |
| 3098 | C₇₀H₁₃₅NO₄S₇ | 1279.27 | 10.12 | 30.32 | 3298 | C₈₀H₁₅₄N₂O₁₁S₄ | 1448.35 | 9.26 | 33.61 |
| 3099 | C₇₀H₁₃₂N₄O₁₂S₄ | 1350.08 | 7.46 | 26.35 | 3299 | C₇₆H₁₄₅NO₆S₈ | 1425.48 | 8.81 | 34.83 |
| 3100 | C₆₆H₁₂₅N₃O₁₁S₄ | 1264.98 | 8.2 | 24.53 | 3300 | C₈₂H₁₇₀N₃O₁₁P₃ | 1467.19 | 9.97 | 30.57 |
| 3101 | C₆₇H₁₂₉NO₁₁S₅ | 1285.06 | 9.67 | 26.84 | 3301 | C₇₇H₁₅₂N₆O₁₁S₅ | 1498.39 | 7.59 | 29.12 |
| 3102 | C₆₇H₁₂₃N₃O₆S₇ | 1291.16 | 8.47 | 27.22 | 3302 | C₇₄H₁₅₃N₇O₁₂S₅ | 1493.38 | 8.28 | 28.08 |
| 3103 | C₆₉H₁₃₈NO₁₂P₃S ₃ | 1362.96 | 9.26 | 24.34 | 3303 | C₇₆H₁₅₂N₆O₁₂S₆ | 1534.44 | 7.73 | 28.03 |
| 3104 | C₆₃H₁₂₅N₇O₉S₆ | 1317.09 | 7.8 | 22.84 | 3304 | C₇₇H₁₅₀N₂O₁₂S₆ | 1488.41 | 7.93 | 28.36 |
| 3105 | C₆₅H₁₂₈N₄O₁₃S₅ | 1334.05 | 8.14 | 22.01 | 3305 | C₇₇H₁₅₁N₂O₁₀P S₅ | 1456.33 | 8.39 | 26.01 |
| 3106 | C₆₄H₁₂₈N₃O₁₅PS ₄ | 1338.95 | 7.4 | 22.22 | 3306 | C₇₉H₁₅₅N₃O₁₀S₃ | 1403.3 | 8.45 | 27.66 |
| 3107 | C₆₃H₁₂₅N₃O₁₀S₇ | 1309.12 | 8.27 | 22.61 | 3307 | C₈₀H₁₅₂N₆O₆ | 1294.13 | 8.06 | 30.31 |
| 3108 | C₆₃H₁₂₆N₂O₇S₄ | 1151.95 | 9.53 | 20.16 | 3308 | C₈₁H₁₅₀N₆O₉S | 1384.18 | 8.07 | 31.2 |
| 3109 | C₆₈H₁₃₁N₇O₈S₂ | 1238.96 | 8.67 | 22.69 | 3309 | C₇₈H₁₅₂N₂O₁₁S₂ | 1358.2 | 8.66 | 29.1 |
| 3110 | C₇₁H₁₃₆N₂O₅S | 1129.94 | 9.9 | 27.52 | 3310 | C₈₂H₁₅₂N₈O₁₀ | 1410.16 | 7.54 | 29 |
| 3111 | C₇₁H₁₃₈NO₆PS₂ | 1196.98 | 9.97 | 25.57 | 3311 | C₈₀H₁₅₃N₅O₁₀ | 1345.13 | 8.37 | 30.38 |
| 3112 | C₇₅H₁₄₃N₅O₃S₂ | 1227.12 | 10.12 | 25.6 | 3312 | C₈₂H₁₅₅N₃O₁₁S₂ | 1423.27 | 8.49 | 32.3 |
| 3113 | C₇₂H₁₃₈N₈O₅S | 1228.01 | 9.88 | 25.83 | 3313 | C₉₇H₁₉₇N₂O₆P | 1518.62 | 9.48 | 42.37 |
| 3114 | C₇₆H₁₃₄N₄O₄S₈ | 1424.41 | 8.11 | 29.4 | 3314 | C₁₀₀H₂₀₃NO₆S₃ | 1611.9 | 9.02 | 44.49 |
| 3115 | C₇₁H₁₃₃N₅O₆S₄ | 1281.11 | 7.36 | 26.07 | 3315 | C₁₀₄H₂₀₇N₆O₁₂P S | 1796.86 | 8.77 | 40.97 |
| 3116 | C₇₂H₁₃₂N₄O₅S₃ | 1230.05 | 9.26 | 27.72 | 3316 | C₁₀₀H₁₉₈N₇O₁₂P S | 1753.75 | 7.59 | 39.4 |
| 3117 | C₇₃H₁₃₂N₄O₈S₆ | 1386.24 | 7.72 | 28.9 | 3317 | C₁₀₃H₂₀₄N₉O₉P S | 1775.85 | 7.96 | 38.97 |
| 3118 | C₇₇H₁₅₂N₂O₁₅P₄ S | 1502.02 | 8.23 | 24.37 | 3318 | C₁₀₀H₁₉₀N₄O₅S₆ | 1721 | 7.09 | 43.77 |
| 3119 | C₇₇H₁₄₈NO₁₁P₃S ₃ | 1453.13 | 9.91 | 26.96 | 3319 | C₁₀₃H₁₉₉N₄O₄P S₆ | 1781.08 | 7.23 | 43.88 |
| 3120 | C₇₁H₁₃₇N₅O₈S₅ | 1349.2 | 7.82 | 25.24 | 3320 | C₁₀₀H₁₉₇N₅O₈S₄ | 1725.94 | 9.14 | 42.88 |
| 3121 | C₇₂H₁₃₉N₆O₁₁PS ₅ | 1456.21 | 7.67 | 26.24 | 3321 | C₁₀₄H₁₉₈N₄O₉S₆ | 1841.11 | 8.25 | 44.96 |
| 3122 | C₇₃H₁₄₁N₂O₁₂PS ₄ | 1398.15 | 8.95 | 24.7 | 3322 | C₉₉H₁₉₂N₂O₁₄S₅ | 1794.92 | 8.35 | 43.66 |
| 3123 | C₇₃H₁₄₀NO₁₃PS₄ | 1399.13 | 9.72 | 26.53 | 3323 | C₁₀₃H₂₀₁N₂O₁₀P S₄ | 1786.96 | 8.46 | 44.74 |
| 3124 | C₆₉H₁₂₇N₃O₁₂S₅ | 1351.08 | 7.54 | 25.78 | 3324 | C₁₀₂H₁₉₅N₇O₈S₆ | 1840.08 | 8.41 | 43.99 |
| 3125 | C₇₂H₁₃₅N₅O₁₂S₃ | 1359.08 | 7.71 | 24.04 | 3325 | C₁₀₂H₂₀₄N₃O₁₆P ₃S | 1853.74 | 7.25 | 38.61 |
| 3126 | C₆₈H₁₃₀N₂O₉S₅ | 1280.09 | 9.58 | 25.45 | 3326 | C₁₀₆H₂₁₄N₃O₁₁P ₃S₂ | 1863.93 | 8.94 | 40.89 |
| 3127 | C₇₁H₁₃₃NO₁₃S₄ | 1337.08 | 9.69 | 26.71 | 3327 | C₉₇H₁₉₇N₅O₁₀S₃ | 1689.85 | 9.91 | 40.33 |
| 3128 | C₆₉H₁₃₂N₂O₁₀S₆ | 1342.18 | 8.71 | 22.55 | 3328 | C₉₇H₁₉₂N₆O₈S₇ | 1795.06 | 7.5 | 39.79 |
| 3129 | C₇₂H₁₃₃N₃O₁₃S | 1280.92 | 7.06 | 25.48 | 3329 | C₉₇H₁₉₇N₇O₆S₅ | 1717.99 | 9.7 | 39.61 |
| 3130 | C₇₈H₁₄₃NO₉S₃ | 1335.18 | 10.04 | 30.97 | 3330 | C₉₉H₁₉₃N₃O₁₀S₇ | 1810.06 | 8.9 | 42.09 |
| 3131 | C₇₆H₁₃₄N₆O₁₂S | 1356 | 8.01 | 25.46 | 3331 | C₉₇H₁₉₀N₄O₉S₃ | 1652.79 | 8.14 | 38.15 |
| 3132 | C₇₂H₁₃₆N₄O₈S₂ | 1250.02 | 9.77 | 23.12 | 3332 | C₉₈H₁₉₃N₃O₁₀S₆ | 1765.99 | 8.13 | 37.98 |
| 3133 | C₄₈H₉₇NO₇S₃ | 896.48 | 9.93 | 17.95 | 3333 | C₉₆H₁₉₄N₄OS₂ | 1484.76 | 9.09 | 41.92 |
| 3134 | C₅₅H₁₀₅N₇O₆S | 992.55 | 8.64 | 14.03 | 3334 | C₁₀₅H₂₀₁N₂O₉P S | 1698.8 | 7.87 | 42.52 |
| 3135 | C₅₂H₉₇N₅O₈ | 920.37 | 7.3 | 14.27 | 3335 | C₁₀₂H₁₉₃N₃O₇S₂ | 1637.8 | 7.73 | 42.08 |
| 3136 | C₅₅H₁₀₈N₄O₆S₃ | 1017.67 | 10.2 | 20.03 | 3336 | C₁₀₁H₁₉₂N₄O₁₂ | 1654.66 | 7.87 | 39.92 |
| 3137 | C₅₁H₁₀₁N₄O₁₀PS ₂ | 1025.48 | 9.12 | 18.42 | 3337 | C₉₉H₁₉₁NO₁₅S | 1667.67 | 9.69 | 42.63 |
| 3138 | C₅₅H₁₀₁N₃O₄S₄ | 996.67 | 8.27 | 18.05 | 3338 | C₇₅H₁₄₃N₁₃O₆S | 1355.11 | 8.07 | 25.23 |
| 3139 | C₅₂H₁₀₀N₄O₃S₃ | 925.58 | 8.64 | 18.64 | 3339 | C₇₆H₁₄₅N₉O₅S₄ | 1393.29 | 7.57 | 26.41 |
| 3140 | C₄₈H₉₄N₂O₃S₅ | 907.59 | 7.71 | 18.31 | 3340 | C₇₃H₁₅₁N₉O₉ | 1359.12 | 7.94 | 25.79 |
| 3141 | C₅₆H₁₀₆N₂O₉S₅ | 1111.77 | 10.02 | 23.32 | 3341 | C₇₆H₁₃₉N₃O₃S₈ | 1399.45 | 7.63 | 30.64 |
| 3142 | C₅₅H₉₉N₃O₇S₇ | 1138.83 | 9.16 | 21.4 | 3342 | C₇₆H₁₄₅NO₅S₅ | 1313.3 | 9.99 | 31.73 |
| 3143 | C₅₄H₁₀₇N₂O₁₄P₃ S | 1133.43 | 7.27 | 15.46 | 3343 | C₇₂H₁₃₇N₃O₇S₃ | 1253.08 | 9.28 | 29.58 |
| 3144 | C₅₇H₁₁₆N₃O₁₀P₃S | 1128.55 | 9.57 | 15.21 | 3344 | C₆₇H₁₃₈N₂O₅S₅ | 1212.15 | 8.77 | 28.81 |
| 3145 | C₅₈H₁₁₉N₃O₁₄P₄ | 1206.49 | 9.37 | 15.64 | 3345 | C₇₂H₁₃₇NO₄S₉ | 1369.43 | 9.47 | 33.57 |
| 3146 | C₅₀H₁₀₂N₆O₉S₄ | 1059.64 | 8.91 | 14.11 | 3346 | C₇₄H₁₅₂N₃O₁₂P₃ S | 1401.02 | 9.4 | 26.1 |
| 3147 | C₄₆H₉₆N₆O₈S₃ | 957.49 | 9.3 | 14.92 | 3347 | C₆₆H₁₃₈N₄O₉S₅ | 1292.15 | 8.8 | 23.19 |
| 3148 | C₄₉H₉₅NO₁₀S₇ | 1082.72 | 8.89 | 17.71 | 3348 | C₇₁H₁₄₂N₂O₁₁S₅ | 1360.22 | 10.1 | 28.27 |
| 3149 | C₅₂H₁₀₆N₂O₈S₄ | 1015.67 | 10.2 | 15.94 | 3349 | C₆₉H₁₃₇N₃O₉S₆ | 1345.23 | 8.87 | 26.02 |
| 3150 | C₅₂H₁₀₂N₆OS₄ | 955.67 | 8.71 | 17.72 | 3350 | C₇₁H₁₃₉N₃O₁₄S₆ | 1451.26 | 7.99 | 27.43 |
| 3151 | C₄₈H₉₉N₅O₅ | 826.35 | 8.78 | 16.86 | 3351 | C₇₁H₁₄₀N₆O₂S₂ | 1174.06 | 7.56 | 26.04 |
| 3152 | C₅₀H₁₀₀N₂O₂S₇ | 985.78 | 8.94 | 20.77 | 3352 | C₇₀H₁₄₃N₅O₂ | 1086.95 | 9.65 | 26.48 |
| 3153 | C₅₀H₉₅NO₉S₂ | 918.43 | 8.74 | 17.41 | 3353 | C₆₉H₁₄₀N₈O₃ | 1129.93 | 7.47 | 24.72 |
| 3154 | C₅₃H₉₇N₃O₁₀ | 936.37 | 8.02 | 17.12 | 3354 | C₇₇H₁₄₁N₃O₈S₂ | 1301.11 | 7.55 | 28.26 |
| 3155 | C₅₂H₉₉N₃O₈S₂ | 958.5 | 9.45 | 17.41 | 3355 | C₇₂H₁₃₅N₃O₁₀S | 1234.94 | 7.57 | 26.66 |
| 3156 | C₅₂H₉₆N₃O₁₁PS | 1002.38 | 8.12 | 16.02 | 3356 | C₇₄H₁₃₈N₂O₁₀S | 1247.98 | 7.46 | 27.52 |
| 3157 | C₅₂H₉₂N₂O₁₅ | 985.31 | 7.52 | 16.32 | 3357 | C₇₀H₁₃₆N₂O₁₀S₂ | 1229.98 | 9.66 | 28.88 |
| 3158 | C₅₃H₁₀₀N₂O₁₃ | 973.38 | 8.88 | 15.78 | 3358 | C₇₅H₁₅₀N₈O₅ | 1244.08 | 9.7 | 26.96 |
| 3159 | C₅₃H₁₀₃N₆O₁₀PS | 1047.47 | 8.46 | 14.49 | 3359 | C₇₄H₁₄₄N₅O₈PS | 1295.03 | 8 | 25.17 |
| 3160 | C₆₀H₁₁₀N₂O₇S₂ | 1035.67 | 8.77 | 21.53 | 3360 | C₇₂H₁₄₅N₅O₉S₃ | 1321.16 | 9.35 | 26.34 |
| 3161 | C₆₁H₁₀₉NO₇S₂ | 1032.66 | 9.76 | 22.04 | 3361 | C₇₁H₁₄₄N₈O₇S | 1254.04 | 7.7 | 26.61 |
| 3162 | C₆₈H₁₂₄N₇O₁₁PS | 1278.81 | 9.49 | 18.68 | 3362 | C₇₉H₁₅₁N₉O₇S₂ | 1403.25 | 8.31 | 26.66 |
| 3163 | C₆₈H₁₁₇NO₃S₆ | 1189.05 | 9.07 | 26.17 | 3363 | C₇₂H₁₄₄N₁₀O₇S₃ | 1358.19 | 8.43 | 26.21 |
| 3164 | C₆₅H₁₁₀N₂O₃S₆ | 1159.97 | 9.44 | 24.53 | 3364 | C₇₁H₁₃₅NO₂S₇ | 1259.29 | 9.74 | 30.8 |
| 3165 | C₆₇H₁₁₄N₄O₄S₈ | 1296.15 | 8.05 | 23.1 | 3365 | C₇₃H₁₃₉NO₂S₈ | 1319.4 | 8.81 | 32.79 |
| 3166 | C₆₇H₁₁₉N₃OS₆ | 1175.07 | 9.63 | 26.04 | 3366 | C₇₁H₁₄₄NO₅PS₄ | 1251.15 | 10.03 | 31.16 |
| 3167 | C₆₄H₁₀₇NO₂S₆ | 1114.92 | 8.4 | 23.72 | 3367 | C₆₉H₁₃₉NO₃S₆ | 1223.23 | 9.21 | 31.23 |
| 3168 | C₆₅H₁₁₁N₅O₅S₃ | 1138.81 | 8.82 | 21.52 | 3368 | C₇₅H₁₄₁N₂O₁₁P S₄ | 1406.17 | 8.64 | 28.77 |
| 3169 | C₅₇H₁₀₅N₃O₃S₄ | 1008.72 | 8.6 | 22.28 | 3369 | C₇₄H₁₅₃N₄O₁₁P₃ S₂ | 1432.1 | 8.31 | 25.59 |
| 3170 | C₆₄H₁₁₃N₅O₅S₇ | 1257.06 | 9.42 | 23.9 | 3370 | C₇₇H₁₅₀N₃O₁₄P₃ S | 1467.04 | 7.45 | 24.68 |
| 3171 | C₆₄H₁₁₈NO₁₂P₃S ₄ | 1314.8 | 8.7 | 19.42 | 3371 | C₇₁H₁₄₆N₈O₈S₄ | 1368.24 | 8.39 | 25.23 |
| 3172 | C₅₇H₁₀₇N₅O₈S₄ | 1118.75 | 9.82 | 18.8 | 3372 | C₆₈H₁₃₅NO₁₅S₅ | 1367.12 | 8.51 | 26.84 |
| 3173 | C₆₀H₁₁₃N₅O₈S₄ | 1160.83 | 9.93 | 19.66 | 3373 | C₇₁H₁₄₂N₆O₁₅S₃ | 1416.12 | 7.02 | 26.05 |
| 3174 | C₆₃H₁₁₈N₆O₉S₄ | 1231.91 | 9.47 | 17.86 | 3374 | C₆₈H₁₃₅NO₁₀S₃ | 1223 | 8.08 | 24.6 |
| 3175 | C₆₂H₁₁₁N₃O₁₃S₄ | 1234.82 | 8.51 | 17.77 | 3375 | C₇₃H₁₄₄N₃O₁₀P S₅ | 1415.24 | 8.25 | 22.65 |
| 3176 | C₆₃H₁₁₄NO₁₃PS₅ | 1284.87 | 9.07 | 21.47 | 3376 | C₇₂H₁₃₇N₃O₁₁S₆ | 1413.26 | 7.91 | 23.79 |
| 3177 | C₆₂H₁₁₇N₂O₁₅PS ₄ | 1289.83 | 9.18 | 18.8 | 3377 | C₇₀H₁₄₂N₂O₁₀S₄ | 1300.15 | 9.93 | 24.88 |
| 3178 | C₆₂H₁₁₁N₃O₁₄S₆ | 1314.94 | 8.47 | 19.91 | 3378 | C₆₈H₁₃₆N₆O₇S₂ | 1213.99 | 7.07 | 26.69 |
| 3179 | C₆₃H₁₁₅N₂O₁₇PS ₅ | 1363.88 | 8.72 | 20.79 | 3379 | C₇₂H₁₄₂N₆O₄ | 1155.97 | 8.79 | 26 |
| 3180 | C₅₈H₁₀₇N₃O₈S₃ | 1070.69 | 9.95 | 16.48 | 3380 | C₇₅H₁₄₆N₃O₇PS | 1265.04 | 9.3 | 28.09 |
| 3181 | C₆₀H₁₀₆N₂O₈S₃ | 1079.69 | 9.71 | 22.06 | 3381 | C₇₄H₁₃₉N₃O₆S₃ | 1263.12 | 8.03 | 27.7 |
| 3182 | C₆₄H₁₁₂N₄O₅S₂ | 1081.74 | 9.13 | 19.93 | 3382 | C₇₅H₁₄₀N₂O₇ | 1181.95 | 8.49 | 27.43 |
| 3183 | C₇₂H₁₂₉N₇O₆S | 1220.93 | 9.47 | 19.86 | 3383 | C₇₄H₁₃₇N₃O₇S₄ | 1309.16 | 8.31 | 28.35 |
| 3184 | C₇₄H₁₃₁N₇O₆S₂ | 1279.03 | 8.74 | 20.89 | 3384 | C₇₄H₁₃₇N₃O₁₄S₂ | 1357.04 | 8.49 | 25.65 |
| 3185 | C₆₉H₁₂₃N₅O₈S | 1182.83 | 9.47 | 23.43 | 3385 | C₁₀₇H₂₀₉N₅O₁₀S | 1757.93 | 9.69 | 46.3 |
| 3186 | C₇₁H₁₂₆N₆O₁₀S₃ | 1320 | 8.1 | 21.56 | 3386 | C₁₀₅H₂₀₈N₆O₁₂S | 1778.91 | 9.08 | 44.2 |
| 3187 | C₇₀H₁₂₉N₄O₁₁PS | 1265.85 | 8.87 | 23.05 | 3387 | C₁₀₇H₂₀₉N₇O₁₀S ₂ | 1818.01 | 7.78 | 44.86 |
| 3188 | C₇₁H₁₂₈N₈O₆S | 1221.91 | 9.84 | 21.09 | 3388 | C₁₀₅H₂₀₉N₇O₇S | 1713.93 | 9.76 | 43.29 |
| 3189 | C₆₅H₁₂₂N₈O₉S₂ | 1223.86 | 7.01 | 20.16 | 3389 | C₁₁₀H₂₁₄N₃O₇P S₅ | 1882.21 | 8.72 | 46.15 |
| 3190 | C₇₂H₁₂₈N₃O₇PS₄ | 1307.04 | 8.58 | 21.87 | 3390 | C₁₀₄H₂₀₃NO₄S₇ | 1756.19 | 7.73 | 48.33 |
| 3191 | C₆₉H₁₂₀N₂O₂S₇ | 1234.15 | 9.59 | 25.76 | 3391 | C₁₁₀H₂₁₄NO₈PS ₄ | 1838.13 | 9.76 | 48.15 |
| 3192 | C₇₂H₁₂₈N₂O₃S₇ | 1294.25 | 9.98 | 27.71 | 3392 | C₁₀₅H₂₀₅NO₈S₅ | 1770.09 | 8.22 | 48.48 |
| 3193 | C₇₁H₁₂₀N₂O₇S₃ | 1209.93 | 8.72 | 23.5 | 3393 | C₁₀₆H₂₀₇N₃O₈S₃ | 1748.01 | 8.86 | 45.55 |
| 3194 | C₆₉H₁₁₉NO₇S₇ | 1299.13 | 9.31 | 26.45 | 3394 | C₁₀₂H₂₀₂N₄O₆S₄ | 1709 | 8.1 | 46.12 |
| 3195 | C₆₆H₁₂₀N₂O₅S₄ | 1149.93 | 7.54 | 24.03 | 3395 | C₁₁₁H₂₂₁N₂O₁₃P ₃S₂ | 1949.03 | 7.97 | 44.55 |
| 3196 | C₇₄H₁₃₇N₂O₁₂P₃ S₃ | 1436.01 | 8.52 | 22.06 | 3396 | C₁₀₀H₁₉₉NO₁₁S₇ | 1816.11 | 7.43 | 44.88 |
| 3197 | C₇₄H₁₃₆N₅O₁₂P₃ S₂ | 1444.97 | 7.61 | 19.31 | 3397 | C₁₀₅H₂₁₂N₃O₁₃P S₃ | 1852.01 | 8.82 | 42.85 |
| 3198 | C₆₆H₁₂₃N₅O₆S₅ | 1243.04 | 9.94 | 20.38 | 3398 | C₁₀₂H₂₀₃N₅O₁₂S ₅ | 1852.07 | 7.51 | 45.6 |
| 3199 | C₆₈H₁₂₃N₃O₁₂S₄ | 1302.98 | 8.93 | 19.98 | 3399 | C₁₀₂H₂₁₀N₄O_{S} | 1540.89 | 10.13 | 45.5 |
| 3200 | C₆₇H₁₁₇N₃O₉S₅ | 1268.98 | 7.82 | 18.53 | 3400 | C₁₀₆H₂₁₀N₄O₅S₇ | 1845.29 | 8.5 | 47.42 |
| 3401 | C₈₉H₁₇₄NO₇PS₄ | 1529.58 | 8.39 | 38.72 | 3413 | C₈₈H₁₇₄N₂O₃S₇ | 1532.79 | 8.91 | 42.54 |
| 3402 | C₉₀H₁₇₁N₃O₇S₃ | 1503.55 | 7.71 | 37.84 | 3414 | C₉₁H₁₇₃N₅O₆S₃ | 1529.59 | 7.96 | 34.92 |
| 3403 | C₈₉H₁₇₄N₂O₈S₅ | 1560.68 | 9.46 | 37.71 | 3415 | C₈₇H₁₇₂N₄O₅S | 1386.42 | 8.88 | 34.57 |
| 3404 | C₉₂H₁₇₇NO₆S₈ | 1649.91 | 9.21 | 43.98 | 3416 | C₇₁H₁₃₅N₅O₉S₃ | 1299.07 | 9.96 | 27.23 |
| 3405 | C₉₁H₁₇₇NO₈S₇ | 1637.84 | 8.69 | 40.23 | 3417 | C₇₈H₁₄₉N₄O₁₁P | 1350.04 | 9.88 | 28.11 |
| 3406 | C₈₈H₁₇₁NO₇S₇ | 1579.76 | 9.6 | 39.68 | 3418 | C₇₁H₁₃₁NO₃S₉ | 1335.37 | 9.68 | 29.97 |
| 3407 | C₉₄H₁₉₃N₂O₁₁P₃ S₄ | 1748.74 | 8.86 | 36.61 | 3419 | C₇₅H₁₃₇N₃O₅S₄ | 1289.18 | 9.96 | 29.92 |
| 3408 | C₉₄H₁₉₁N₂O₁₁P₃ S₂ | 1682.61 | 9.39 | 36.62 | 3420 | C₇₁H₁₃₂N₆O₁₀S₆ | 1422.23 | 7.13 | 23.9 |
| 3409 | C₉₇H₁₉₆N₃O₁₅P₃ S | 1769.62 | 9.74 | 34.98 | 3421 | C₆₉H₁₃₃N₅O₈S₅ | 1321.15 | 8.74 | 23.45 |
| 3410 | C₈₈H₁₈₀N₄O₇S₅ | 1566.73 | 9.79 | 37.89 | 3422 | C₇₀H₁₃₉N₄O₁₄P S₄ | 1420.11 | 7.55 | 24.35 |
| 3411 | C₈₉H₁₈₂NO₁₅PS₄ | 1665.64 | 9.36 | 36.96 | | | | | |
| 3412 | C₈₈H₁₇₆N₂O₁₄S₄ | 1614.62 | 7.11 | 32.69 | | | | | |

The above properties of the exemplary first ionizable lipid disclosed herein demonstrate that they can serve as excellent surfactants, and are particularly suitable for the preparation of lipid nanoparticles as drug delivery vehicles.

### (4) Preparation of Pulmonary Delivery System Containing Ionizable Lipids

| Example No. | Formulation Composition | Molar Ratio(%) | mRNA Mass Ratio |
|---|---|---|---|
| 1 | DLin-MC3-DMA / DOPC / Chol | 45 / 15 / 40 | 3.0% |
| 2 | DLin-MC3-DMA / DSPC / Chol | 40 / 20 / 40 | 6.0% |
| 3 | Compound 6008 / PEG / ALC-0315 / DOPE / Chol | 5.6 / 2.8 / 18.3 / 27.5 / 45.8 | 6.0% |
| 4 | Compound 1005 / POPC / Chol | 45 / 10 / 45 | 4.5% |
| 5 | Compound 1008 / DSPC / Chol | 47 / 10 / 43 | 3.1% |
| 6 | Compound 1011 / DOPC / Chol | 40 / 20 / 40 | 4.4% |
| 7 | Compound 6019 / Compound 1115 / DOPE / Chol | 2 / 35 / 20 / 43 | 15.0% |
| 8 | Compound 1009 / DSPC / Chol | 45 / 15 / 40 | 5.0% |
| 9 | Compound 6007 / PEG / Compound 1008 / POPE / Chol | 5.6 / 2 / 27 / 18 / 47.4 | 6.0% |
| 10 | Compound 6001 / PEG400 / Compound 1113 / DOPE / Chol | 6 / 3 / 30 / 15 / 46 | 6.5% |
| 11 | Compound 1001 / POPC / Chol | 40 / 20 / 40 | 10.0% |
| 12 | Compound 6038 / DOPE / Chol | 50 / 10 / 40 | 4.5% |
| 13 | DLin-KC3-DMA / POPC / Chol | 30 / 15 / 55 | 5.0% |
| 14 | Compound 6010 / MVL5 / DSPC / Chol | 15 / 20 / 20 / 45 | 20.0% |
| 15 | Compound 6016 / DODMA / DSPC / Chol | 20 / 20 / 25 / 35 | 10.0% |
| 16 | cKK-E12 / DOPE / Chol | 35 / 10 / 55 | 3.0% |
| 17 | Compound 6023 / Compound 1112 / POPC / Chol | 3 / 30 / 30 / 37 | 25.0% |
| 18 | Compound 1007 / DOPC / Chol | 60 / 10 / 30 | 5.0% |
| 19 | Compound 1114 / DOPE / Chol | 45 / 20 / 35 | 15.0% |
| 20 | Compound 6020 / Compound 1059 / DSPC / Chol | 5 / 30 / 10 / 55 | 10.0% |
| Comparative Example 1 | Compound 6038 / DOPE / Chol / ALC-0159 | 50 / 10 / 38.5 / 1.5 | 4.5% |
| Comparative Example 2 | Compound 6038 / DOPE / Chol / ALC-0159 | 50 / 10 / 40 / 1.5 | 4.5% |

The pulmonary delivery systems containing ionizable lipids were separately prepared according to the above molar ratios via the specific procedure described below:

All lipid components were dissolved in ethanol, with a total lipid concentration of 3 mg/mL. Firefly luciferase mRNA was dissolved in 50 mM citrate buffer (pH 4.0) to prepare luciferase mRNA solution. The concentration of luciferase mRNA solution in Example 4 was set to 0.19 mg/mL, while the concentrations for other examples were adjusted according to the corresponding formulation ratios.

The lipid ethanol solution and luciferase mRNA solution were mixed at a volume ratio of 1:3 using a nanoparticle preparation instrument. The mixed liquid was subjected to ultrafiltration, and the resulting sample was collected to obtain the pulmonary delivery system (lipid nanoparticles) containing ionizable lipids.

### Comparative Example 2

Based on the formulation of Example 12, an additional 1.5 mol% conjugated lipid (ALC-0159) (calculated with the total molar amount of lipids in Example 12 as 100%) was added as an additional lipid raw material. All lipid components were dissolved in ethanol to prepare a lipid ethanol solution with a total lipid concentration of 3.045 mg/mL. Firefly luciferase mRNA was dissolved in 50 mM citrate buffer (pH 4.0) to yield luciferase mRNA solution at a concentration of 0.19 mg/mL. The lipid ethanol solution and luciferase mRNA solution were mixed at a volume ratio of 1:3 via a nanoparticle preparation instrument. The mixture was subjected to ultrafiltration, and the final sample was collected to obtain the pulmonary delivery system (lipid nanoparticles) containing ionizable lipids.

### (5) Determination of Particle Size and Zeta Potential of Pulmonary Delivery Systems Containing Ionizable Lipids

1. Measurement of particle size and polydispersity index (PDI): The average particle size and PDI of nanoparticle sample solutions from each example were measured by dynamic light scattering (DLS) using a Malvern ZetaSizer Nano ZS90 instrument. The detection angle was 90°, the refractive index of the dispersant was 1.330, and the measurement temperature was 25 °C.
2. Measurement of Zeta potential: Zeta potential of nanoparticle sample solutions was determined by electrophoretic light scattering (ELS) equipped on Malvern ZetaSizer Nano ZS90. The refractive index of the dispersant was 1.330, and the test temperature was 25 °C.

The data of average particle size, PDI and Zeta potential of pulmonary delivery systems containing ionizable lipids prepared in each example are summarized in Table 2.

**Table 2 Summary of Particle Size, PDI and Zeta Potential of Pulmonary Delivery Systems Containing Ionizable Lipids**

| Example No. | Particle Size (nm) | PDI | Zeta Potential (mV) | Encapsulation Efficiency (%) |
|---|---|---|---|---|
| 1 | 78.11 | 0.086 | -10.1 | 76.0 |
| 2 | 108.9 | 0.261 | 32.7 | 90.5 |
| 3 | 221.2 | 0.160 | -2.14 | 99.0 |
| 4 | 45.09 | 0.157 | 42.3 | 96.4 |
| 5 | 62.39 | 0.146 | 1.77 | 94.7 |
| 6 | 62.32 | 0.135 | 12.9 | 98.3 |
| 7 | 143.5 | 0.127 | 5.61 | 89.2 |
| 8 | 90.56 | 0.124 | 10.5 | 92.4 |
| 9 | 158.5 | 0.172 | 8.01 | 86.5 |
| 10 | 126.2 | 0.133 | 5.92 | 86.3 |
| 11 | 86.4 | 0.154 | 10.3 | 92.6 |
| 12 | 158.8 | 0.059 | 20.1 | 89.5 |
| 13 | 114.7 | 0.125 | 12.5 | 80.5 |
| 14 | 120.7 | 0.103 | 12.8 | 79.8 |
| 15 | 100.5 | 0.058 | 20.3 | 85.4 |
| 16 | 115.6 | 0.102 | 18.7 | 81.3 |
| 17 | 54.93 | 0.165 | 44.0 | 96.4 |
| 18 | 48.21 | 0.185 | 35.8 | 94.1 |
| 19 | 80.51 | 0.104 | 23.5 | 86.3 |
| 20 | 95.62 | 0.127 | 28.6 | 90.5 |
| Comparative Example 1 | 86.81 | 0.104 | 4.56 | 86.7 |
| Comparative Example 2 | 102.4 | 0.127 | 6.78 | 90.4 |

It can be seen from Table 2 that the pulmonary delivery systems containing ionizable lipids provided by the present application feature small and uniform particle sizes with neutral or weak surface charge.

### (6) Investigation of Nanoparticle Characteristics After Nebulization

An Omron compressor nebulizer was used to nebulize the pulmonary delivery system containing ionizable lipids. The nebulized samples were collected for the detection of particle size, potential, encapsulation efficiency and other properties, with the same test procedures as in Section (5). The results are shown in Table 3.

**Table 3 Characteristic Data of Pulmonary Delivery Systems Containing Ionizable Lipids Before and After Nebulization**

| **Example No.** | **Nebulization Status** | **Particle Size (nm)** | **PDI** | **Potential (mV)** | **Encapsulation Efficiency (%)** |
|---|---|---|---|---|---|
| 1 | Before Nebulization | 78.11 | 0.086 | -10.1 | 76.0 |
| | After Nebulization | 94.70 | 0.174 | -11.5 | 70.5 |
| 2 | Before Nebulization | 108.9 | 0.261 | 32.7 | 90.5 |
| | After Nebulization | 105.8 | 0.221 | 29.8 | 84.9 |
| 3 | Before Nebulization | 221.2 | 0.160 | -2.14 | 99.0 |
| | After Nebulization | 235.7 | 0.130 | -5.62 | 95.6 |
| 4 | Before Nebulization | 45.09 | 0.157 | 42.3 | 96.4 |
| | After Nebulization | 39.21 | 0.270 | 41.5 | 97.4 |
| 6 | Before Nebulization | 62.32 | 0.135 | 12.9 | 98.3 |
| | After Nebulization | 70.51 | 0.201 | 10.8 | 91.4 |
| 9 | Before Nebulization | 158.5 | 0.172 | 8.01 | 86.5 |
| | After Nebulization | 149.7 | 0.210 | 12.6 | 80.7 |
| 12 | Before Nebulization | 158.8 | 0.059 | 20.1 | 89.5 |
| | After Nebulization | 153.8 | 0.102 | 18.5 | 86.7 |
| 13 | Before Nebulization | 114.7 | 0.125 | 12.5 | 80.5 |
| | After Nebulization | 102.8 | 0.210 | 15.6 | 82.3 |
| 14 | Before Nebulization | 120.7 | 0.103 | 12.8 | 79.8 |
| | After Nebulization | 132.4 | 0.159 | 18.7 | 75.6 |
| 15 | Before Nebulization | 100.5 | 0.058 | 20.3 | 85.4 |
| | After Nebulization | 105.6 | 0.146 | 26.4 | 80.4 |
| 17 | Before Nebulization | 54.93 | 0.165 | 44.0 | 96.4 |
| | After Nebulization | 62.50 | 0.197 | 32.5 | 86.9 |
| 20 | Before Nebulization | 95.62 | 0.127 | 28.6 | 90.5 |
| | After Nebulization | 102.6 | 0.189 | 31.5 | 85.4 |
| Comparativ e Example 1 | Before Nebulization | 86.81 | 0.104 | 4.56 | 86.7 |
| | After Nebulization | 191.9 | 0.471 | -6.89 | 38.0 |
| Comparativ e Example 2 | Before Nebulization | 102.4 | 0.127 | 6.78 | 90.4 |
| | After Nebulization | 212.9 | 0.357 | -10.8 | 55.3 |

It can be observed from Table 3 that the particle properties including particle size, PDI, potential and encapsulation efficiency of the pulmonary delivery systems containing ionizable lipids disclosed herein remain unchanged before and after nebulization, demonstrating outstanding stability.

### (7) In Vivo Evaluation in Mice

### 7.1 Intratracheal Administration

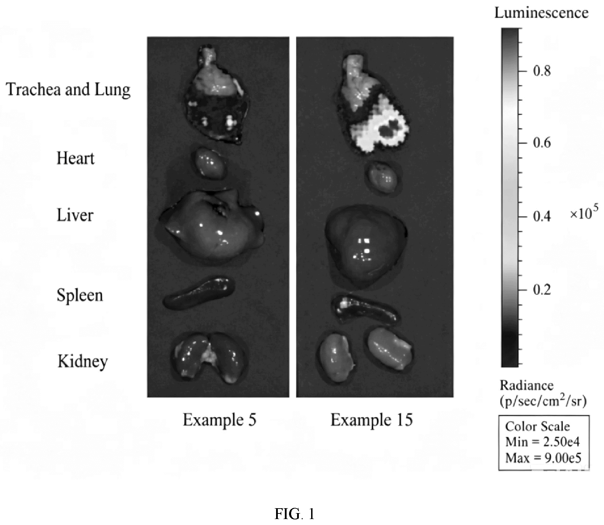

Female Balb/c mice aged 6-8 weeks were utilized to evaluate the pulmonary transfection efficiency and biosafety of the pulmonary delivery systems containing ionizable lipids prepared in each example. The formulations of Example 5 and Example 15 were administrated via intratracheal administration at a single dose of 0.375 mpk. At the designated time point (6 h post-administration), *in vivo* bioluminescence imaging was performed on the animals using a PerkinElmer small-animal imaging system to quantify bioluminescent signals in various organs and tissues. The results are summarized in Table 4 and FIG. 1.

**Table 4 In Vivo Imaging Results of Mice at 6 h After Intratracheal Administration**

| Sample ID | Average Total Flux [p/s] | | | | |
|---|---|---|---|---|---|
| | Lung & Trachea | Heart | Liver | Spleen | Kidney |
| Example 5 | 1.01E+06 | ND | ND | ND | ND |
| Example 15 | 4.58E+06 | 3.37E+04 | ND | ND | ND |

| | | | | | |
|---|---|---|---|---|---|
| *ND = Not Detected | | | | | |

The results revealed that bioluminescent expression was predominantly restricted to the lung tissues of mice sacrificed at 6 h, with negligible signals detected in other visceral organs.

### 7.2 Nebulized Inhalation Administration

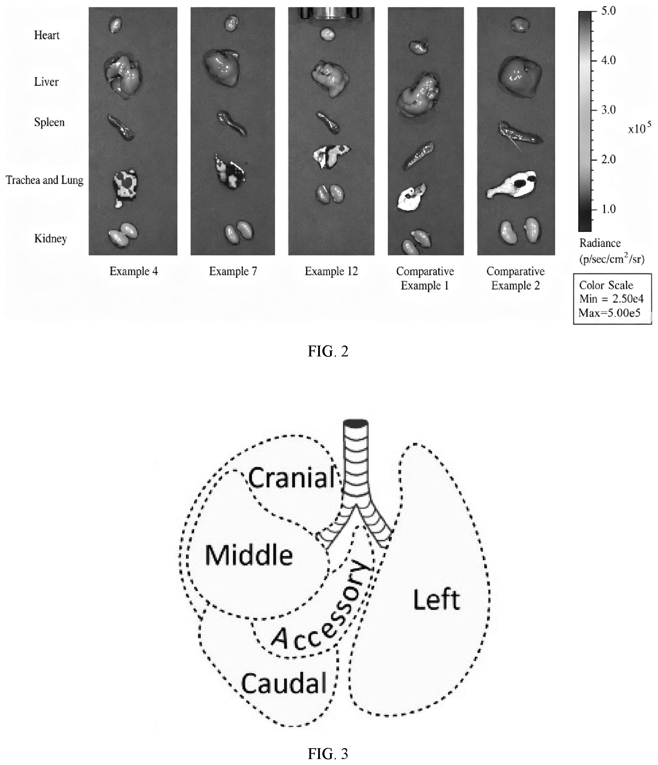

Female Balb/c mice aged 6-8 weeks were employed to assess the pulmonary transfection efficiency and biosafety of the pulmonary delivery systems containing ionizable lipids. Formulations of Example 4, Example 7, Example 12, Comparative Example 1 and Comparative Example 2 were delivered via nebulized inhalation at a single dose of 1.125 mpk. At 6 h post-administration, *in vivo* bioluminescence imaging was carried out with a PerkinElmer small-animal imaging system to measure bioluminescent signals in various organs and tissues. The data are listed in Table 5 and FIG. 2.

**Table 5 In Vivo Imaging Results of Mice at 6 h After Nebulized Inhalation**

| Sample ID | Average Total Signal Intensity [p/s] | | | | |
|---|---|---|---|---|---|
| | Lung & Trachea | Heart | Liver | Spleen | Kidney |
| Example 4 | 5.73E+06 | ND | 3.23E+05 | ND | ND |
| Example 7 | 1.94E+06 | ND | ND | ND | ND |
| Example 12 | 2.92E+06 | ND | ND | ND | ND |
| Comparative Example 1 | 8.28E+04 | ND | ND | ND | ND |
| Comparative Example 2 | 9.69E+04 | ND | ND | ND | ND |

| | | | | | |
|---|---|---|---|---|---|
| *ND = Not Detected | | | | | |

The results showed that in mice dissected at 6 h, bioluminescent expression was predominantly observed in the lungs, with almost no expression detected in other organs..

### 7.3 Pulmonary Distribution After Intratracheal Spray Administration

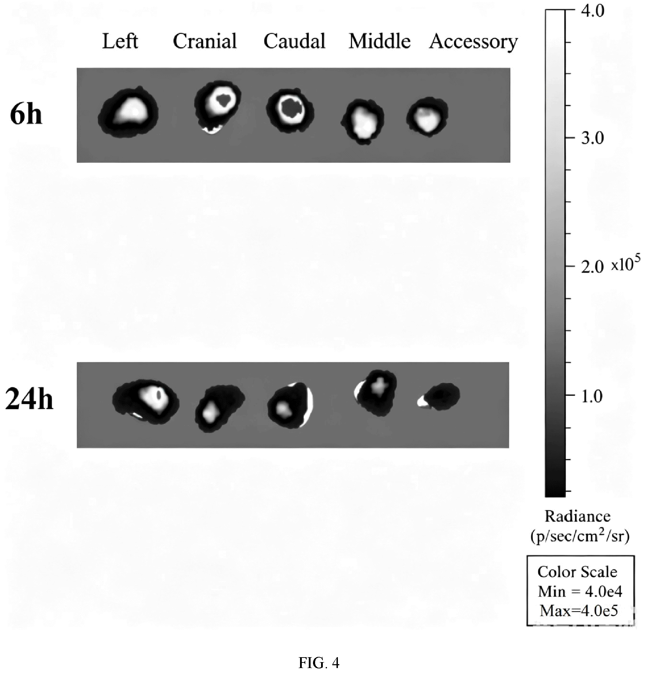

Female Balb/c mice aged 6-8 weeks were used to evaluate the pulmonary transfection efficiency of the pulmonary delivery system containing ionizable lipids prepared in the example. The pulmonary delivery system of Example 6 and normal saline (blank control) were administered via intratracheal spray at a single dose of 0.375 mpk. Lungs were harvested at predetermined time points (6 h, 24 h, 48 h and 72 h post-administration). IVIS imaging of the five lung lobes (arranged in the order 1 to 5 shown in Figure 3) was performed using a PerkinElmer small-animal imaging system, and the bioluminescent signal intensity of each lobe was quantified. The results are presented in Figure 4.

It can be observed that after intratracheal spray administration of the ionizable lipid-based pulmonary delivery system to mice, significant luciferase expression was detected in all lung lobes of the animals.

The technical solution of the present application is not limited to the technical means disclosed above, and also encompasses technical solutions formed by any arbitrary combination of the above technical features. The foregoing describes specific embodiments of the present application. It should be noted that a person skilled in the art can make several modifications and refinements without departing from the principles of the present application, and such modifications and refinements shall also fall within the protection scope of the present application.

## Claims

1. A pulmonary delivery system comprising ionizable lipids, comprises ionizable lipids, helper lipids and bioactive substances, the pulmonary delivery system comprising ionizable lipids is free of polymer-conjugated lipids; the ionizable lipids are selected from at least one of a first ionizable lipid, a second ionizable lipid and a third ionizable lipid;
the first ionizable lipid is selected from a compound having general Formula (1), or a pharmaceutically acceptable salt, stereoisomer or tautomer thereof: 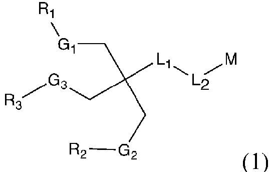
wherein R₁, R₂ and R₃ are each independently selected from H, C₅₋₄₀ linear or branchedalkyl group,C₅₋₄₀ linear or branched alkenyl group, C₅₋₄₀ linear or branched alkynyl group, 3-6membered saturated or partially unsaturated cycloaliphatic group comprising 1-3 side chains, or 6-10 membered aromatic group comprising 1-3 side chains; the side chains are each independently selected from C₁₀₋₃₀ linear or branched alkyl, C₁₀₋₃₀ linear or branched alkenyl, and C₁₀₋₃₀ linear or branched alkynyl; provided that at most one of R₁, R₂ and R₃ is H;
M is selected from -NR₄R₅, a saturated or partially unsaturated 3-6 membered heterocyclic group containing at least one nitrogen atom, and a 6-10 membered heteroaryl group containing at least one nitrogen atom; the heterocyclic group and heteroaryl group are unsubstituted or substituted with one or more substituents selected from -OH, carboxyl, amino, oxo and halogen;
R₄ and R₅ are each independently selected from H, C₁₋₆ linear or branched alkyl, C₂₋₆ linear or branched alkenyl, or C₂₋₆ linear or branched alkynyl; the C₁₋₆ linear or branched alkyl, the C₂₋₆ linear or branched alkenyl or the C₂₋₆ linear or branched alkynyl is unsubstituted or substituted with one or more substituents selected from -OH, carboxyl, aminoamido, amidino, guanidyl and halogen;
G₁, G₂ and G₃ are each independently selected from -O-, -S-, -NR₆-, -S-S-, -C(=O)-, -C(=S)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₆-, -NR₆-S(=O)₂-, -P(=O)(OR₆)O-, -OP(=O)(OR₆)- or -OP(=O)(OR₆)O-; wherein each of R₆ and R₁₃ is independently selected from H, hydroxyl, C₁₋₃₀ linear or branched alkyl or cycloalkyl, and C₂₋₃₀ linear or branched alkenyl;
L₁ is selected from -X₁- or -(CR₇R₈)ₘ-X₁-; wherein each X₁ is independently selected from -O-, -S-, -NR₁₄-, -S-S-, -C(=O)-, -C(=S)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₄-, -NR₁₄-S(=O)₂-, -P(=O)(OR₁₄)O-, -OP(=O)(OR₁₄)- or -OP(=O)(OR₁₄)O-; wherein m is an integer of 2-6; R₇ and R₈ are each independently selected from H, hydroxyl, halogen, C₁₋₆ linear or branched alkyl or cycloalkyl, or C₂₋₆ linear or branched alkenyl; wherein each of R₁₄ and R₁₅ is independently selected from H, C₁₋₃₀ linear or branched alkyl or cycloalkyl, and C₂₋₃₀ linear or branched alkenyl;
L₂ is -(CR₉R₁₀)ₙ₋ or -(CR₉R₁₀)ₙ-X₂-(CR₁₁R₁₂)ₖ-; wherein X₂ is selected from -O-, -S-, -NR₁₆-, -S-S-, -C(=O)-, -C(=S)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -C(=O)S-, -C(=S)S-, -SC(=S)-, -SC(=O)-, -OC(=O)S-, -SC(=O)O-, -SC(=O)S-, -OS(=O)₂O-, -S(=O)₂O-, -OS(=O)₂-, -S(=O)₂-, -S(=O)₂-NR₁₆-, NR₁₆-S(=O)₂-, -P(=O)(OR₁₆)O-, -OP(=O)(OR₁₆)- or -OP(=O)(OR₁₆)O-; n is an integer of 1-6; k is an integer of 1-6; R₉, R₁₀, R₁₁ and R₁₂ are each independently selected from H, hydroxyl, halogen, C₁₋₆ linear or branched alkyl or cycloalkyl, or C₂₋₆ linear or branched alkenyl; each of R₁₆ and R₁₇ is independently selected from H, C₁₋₃₀ linear or branched alkyl or cycloalkyl, and C₂₋₃₀ linear or branched alkenyl;
wherein the alkyl, cycloalkyl and alkenyl groups defined in R₄ to R₁₇ are unsubstituted or substituted with one or more groups selected from hydroxyl, mercapto, amino, substituted amino, and halogen;
the salt does not include quaternary ammonium salt.

2. The pulmonary delivery system comprising ionizable lipids according to claim 1, wherein the bioactive substance comprises at least one of DNA, RNA, proteins, peptides and small-molecule compounds.

3. The pulmonary delivery system comprising ionizable lipids according to claim 1,
wherein the dosage of the bioactive substance accounts for 1-50% (w/w) of the total mass of lipid raw materials and the bioactive substance.

4. The pulmonary delivery system comprising ionizable lipids according to claim 1, wherein R₁, R₂ and R₃ are each independently selected from the following groups: 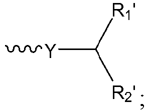 wherein Y is absent, or is C₁₋₃₀ linear or branched alkyl or cycloalkyl, C₂₋₂₀ linear or branched alkenyl, or C₂₋₂₀ linear or branched alkynyl; R₁' and R₂' are each independently selected from H, C₁₋₃₀ linear or branched alkyl, C₂₋₃₀ linear or branched alkenyl, or C₂₋₃₀ linear or branched alkynyl, and the total carbon chain length of Y, R₁' and R₂' is 8-40.

5. The pulmonary delivery system comprising ionizable lipids according to claim 1, wherein R1, R2 and R3 are each independently selected from the following groups: 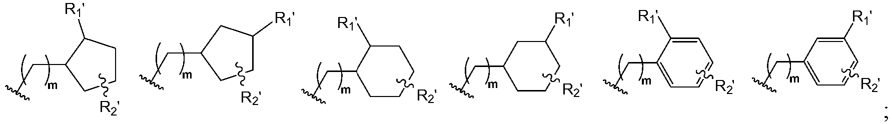 wherein R₁' and R₂' are each independently selected from H, C₁₋₃₀ linear or branched alkyl, C₂₋₃₀ linear or branched alkenyl, or C₂₋₃₀ linear or branched alkynyl, and the total carbon chain length of R₁' and R₂' is 8-30.

6. The pulmonary delivery system comprising ionizable lipids according to claim 1, wherein R₁, R₂ and R₃ are each independently selected from any one of the following groups:

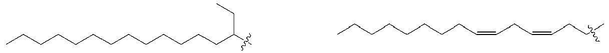

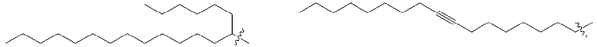

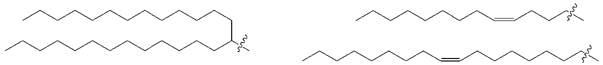

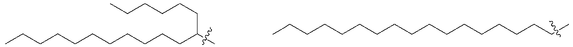

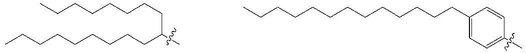

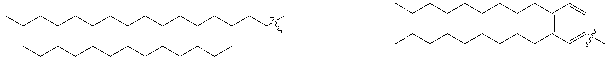

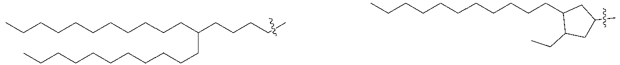

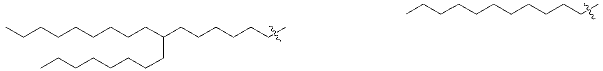

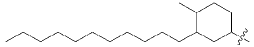

7. The pulmonary delivery system comprising ionizable lipids according to claim 1, wherein G₁, G₂ and G₃ are each independently selected from -O-, -S-, -NR₆-, -S-S-, -C(=O)-, -C(=O)O-, -CH(OH)-, -OC(=O)-, -C(=O)NR₆-, -NR₆C(=O)-, -OC(=O)O-, -NR₆C(=O)O-, -OC(=O)NR₆-, -NR₆C(=O)NR₁₃-, -P(=O)(OR₆)O-, -OP(=O)(OR₆)- or -OP(=O)(OR₆)O-.

8. The pulmonary delivery system comprising ionizable lipids according to claim 1, wherein L₁ is selected from -(CR₇R₈)ₘ-X₁-, and X₁ is selected from -O-, -S-, -NR₁₄-, -S-S-, -C(=O)-, -C(=O)O-, -OC(=O)-, -C(=O)NR₁₄-, -NR₁₄C(=O)-, -OC(=O)O-, -NR₁₄C(=O)O-, -OC(=O)NR₁₄-, -NR₁₄C(=O)NR₁₅-, -P(=O)(OR₁₄)O-, -OP(=O)(OR₁₄)- or -OP(=O)(OR₁₄)O-.

9. The pulmonary delivery system comprising ionizable lipids according to claim 1, wherein L₂ is -(CR₉R₁₀)ₙ₋X₂-(CR₁₁R₁₂)ₖ₋, and X₂ is selected from -O-, -S-, -NR₁₆-, -S-S-, -C(=O)-, -C(=O)O-, -OC(=O)-, -C(=O)NR₁₆-, -NR₁₆C(=O)-, -OC(=O)O-, -NR₁₆C(=O)O-, -OC(=O)NR₁₆-, -NR₁₆C(=O)NR₁₇-, -P(=O)(OR₁₆)O-, -OP(=O)(OR₁₆)- or -OP(=O)(OR₁₆)O-.

10. The pulmonary delivery system comprising ionizable lipids according to claim 1, wherein M is selected from the following structures: 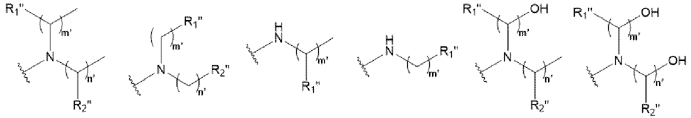 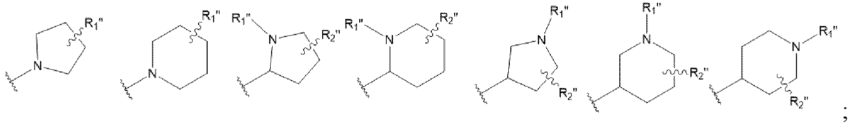 wherein m' and n' are each independently an integer of 0-6; R₁" and R₂" are each independently selected from H, C₁₋₆ alkyl, C₂₋₆ alkenyl, guanidyl, amidino, amido, aliphatic amino group, or a 3-10 membered nitrogen-containing heterocycle; the nitrogen-containing heterocycle is selected from pyrrole, imidazole, pyridine, pyrazole, triazole, oxazole, isoxazole, thiophene, isothiazole, pyridazine, pyrazine, piperazine, indole, benzimidazole, carbazole, quinoline, isoquinoline, purine, and pyrimidine, and tautomers thereof, which is unsubstituted or optionally substituted with one or more organic groups selected from hydroxyl, mercapto, amino, substituted amino, halogen, C₁-C₆ alkyl, C₂-C₆ alkenyl, C₂-C₆ alkynyl, and C₆-C₁₄ aryl.

11. The pulmonary delivery system comprising ionizable lipids according to claim 1, wherein the compound of Formula (1) is selected from compounds represented by Formula (1A):

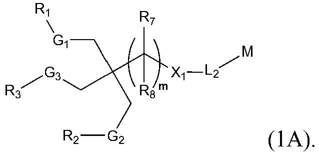

12. The pulmonary delivery system comprising ionizable lipids according to claim 1,
wherein the compound of Formula (1) is selected from compounds represented by Formula (1B):

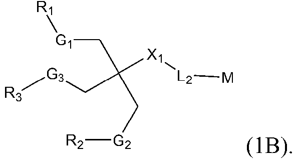

13. The pulmonary delivery system comprising ionizable lipids according to claim 1, wherein the compound of Formula (1) is selected from compounds represented by Formula (1C):

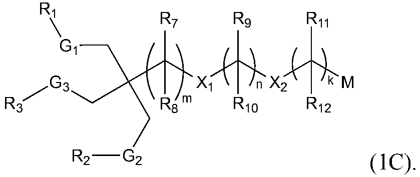

14. The pulmonary delivery system comprising ionizable lipids according to claim 12, wherein the compound of Formula (1) is selected from compounds represented by Formula (1D):

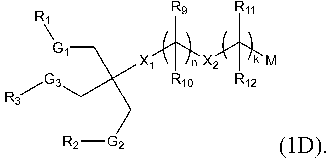

15. The pulmonary delivery system comprising ionizable lipids according to claim 14, wherein the compound of Formula (1) is selected from compounds represented by Formula (1E):

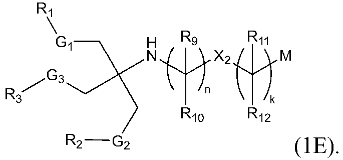

16. The pulmonary delivery system comprising ionizable lipids according to claim 15, wherein the compound of Formula (1) is selected from compounds represented by Formula (1F):

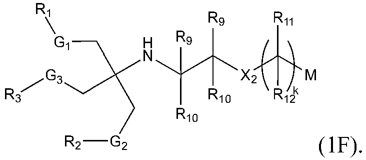

17. The pulmonary delivery system comprising ionizable lipids according to claim 16, wherein the compound of Formula (1) is selected from compounds represented by Formula (1G):

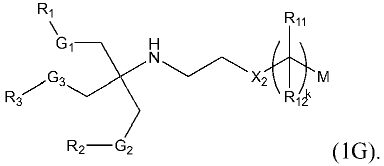

18. The pulmonary delivery system comprising ionizable lipids according to claim 16, wherein the compound of Formula (1) is selected from compounds represented by Formula (1H):

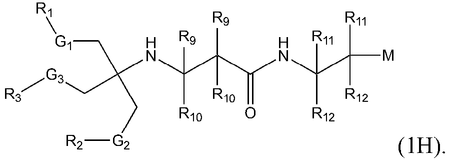

19. The pulmonary delivery system comprising ionizable lipids according to claim 1, wherein the compound of Formula (1) is selected from compounds represented by Formula (1I):

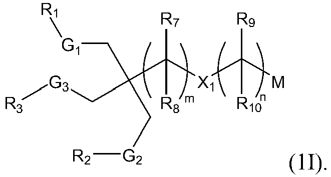

20. The pulmonary delivery system comprising ionizable lipids according to claim 12, wherein the compound of Formula (1) is selected from compounds represented by Formula (1J):

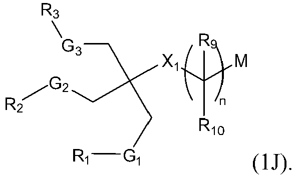

21. The pulmonary delivery system comprising ionizable lipids according to claim 19, wherein the compound of Formula (1) is selected from compounds represented by Formula (1K):

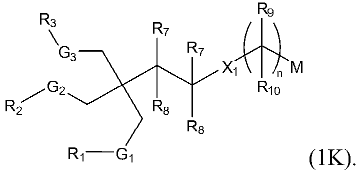

22. The pulmonary delivery system comprising ionizable lipids according to claim 4, wherein Y is absent, and the compound of Formula (1) is selected from compounds represented by Formula (1L): 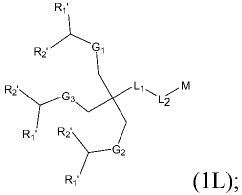 wherein R₁' and R₂' are each independently selected from H, C₁₋₃₀ linear or branched alkyl, C₂₋₃₀ linear or branched alkenyl, and C₂₋₃₀ linear or branched alkynyl, and the total carbon chain length of R₁' and R₂' is 8-40.

23. The pulmonary delivery system comprising ionizable lipids according to claim 4, wherein the compound of Formula (1) is selected from compounds represented by Formula (1M):

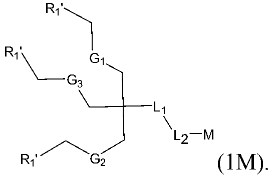

24. The pulmonary delivery system comprising ionizable lipids according to any one of claims 1 to 23, wherein the compound of Formula (1) is selected from:

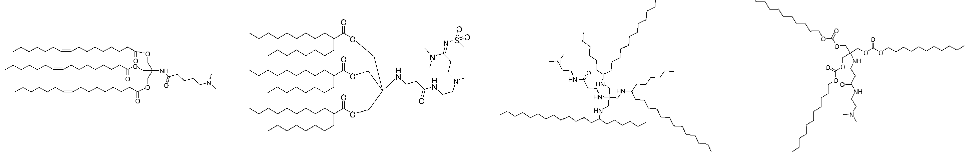

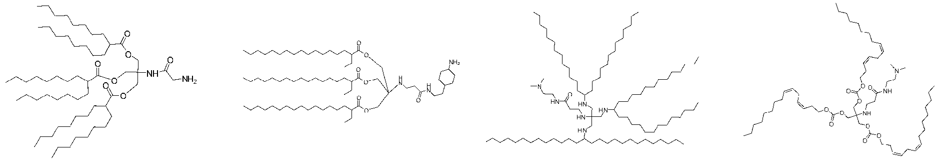

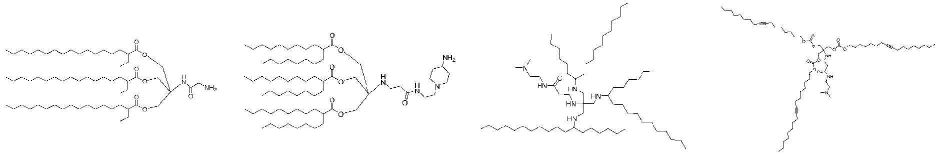

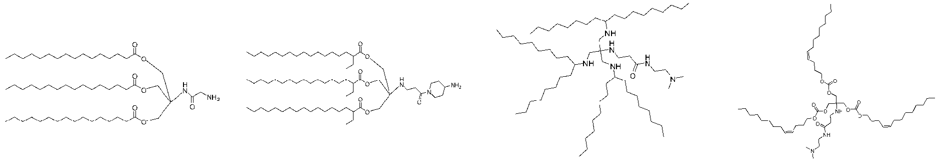

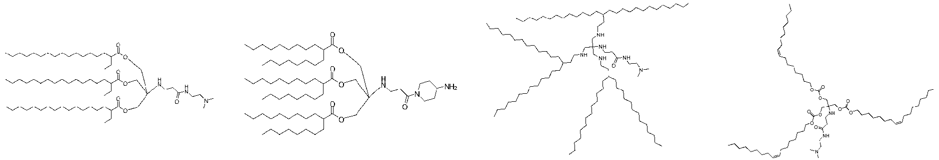

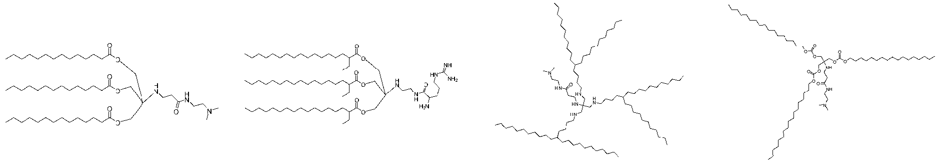

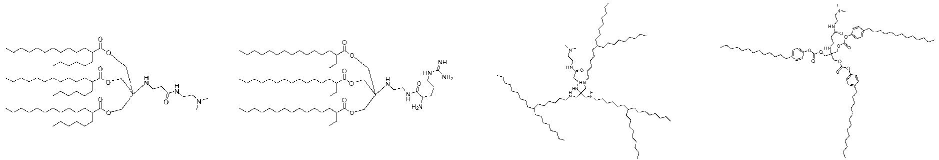

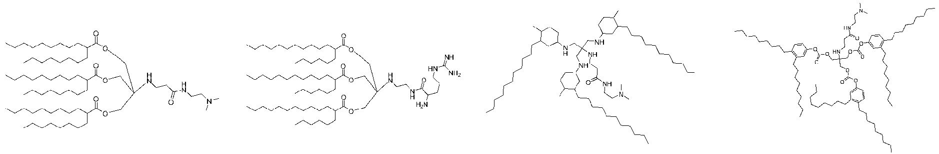

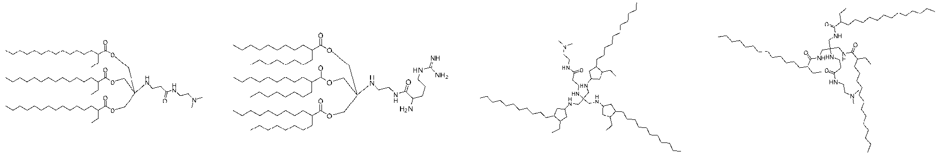

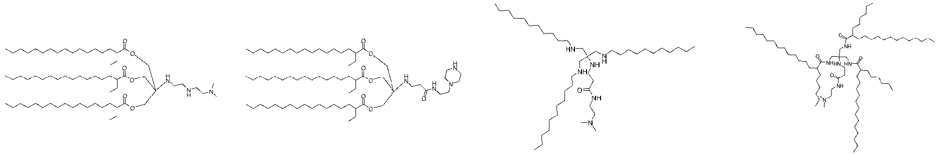

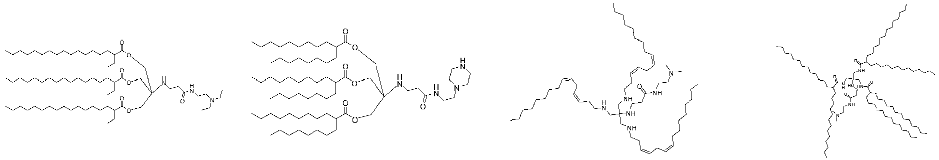

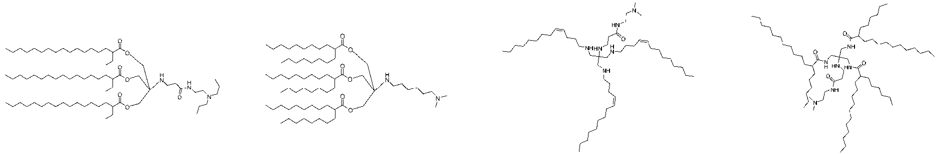

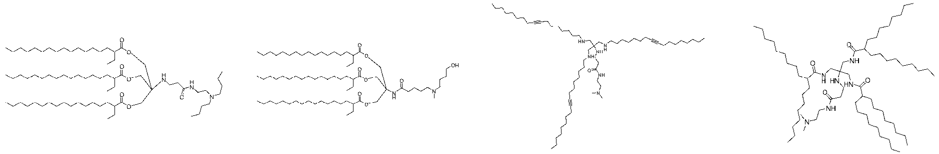

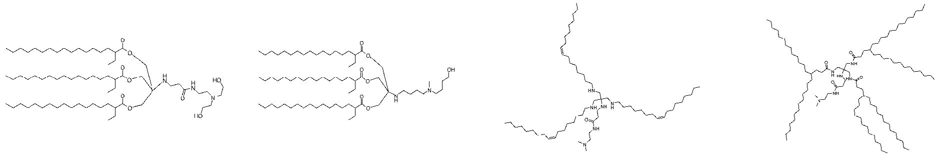

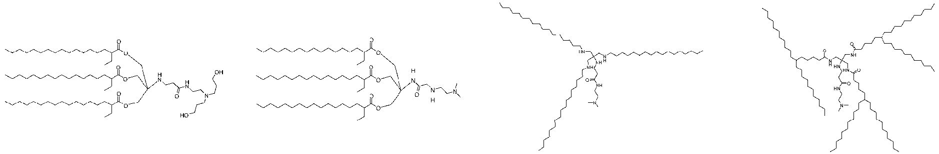

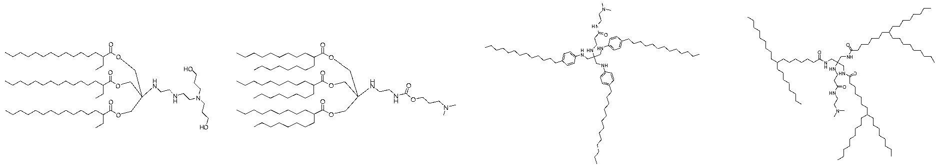

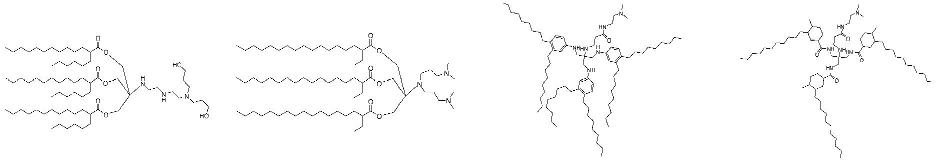

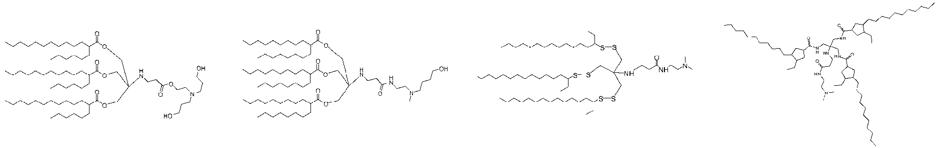

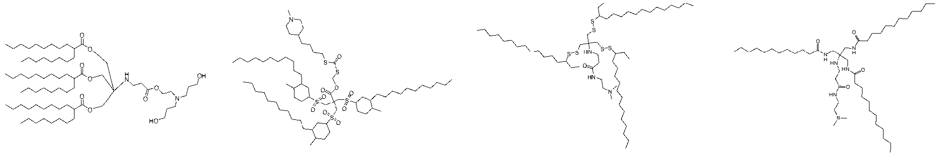

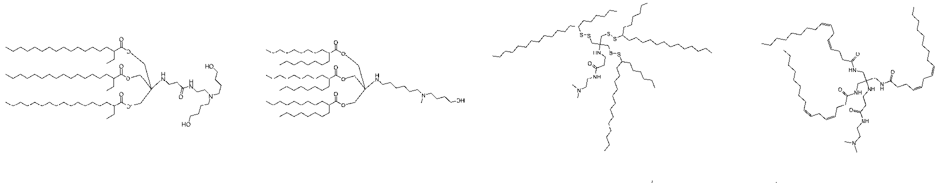

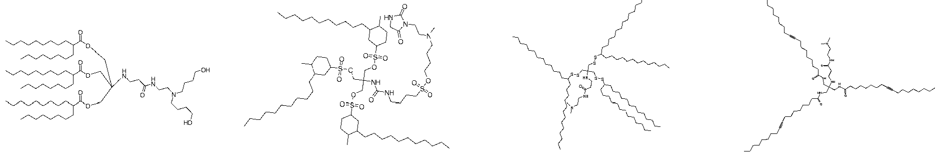

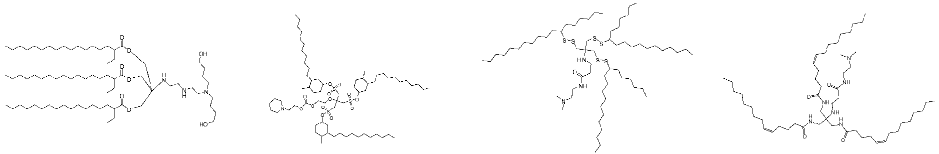

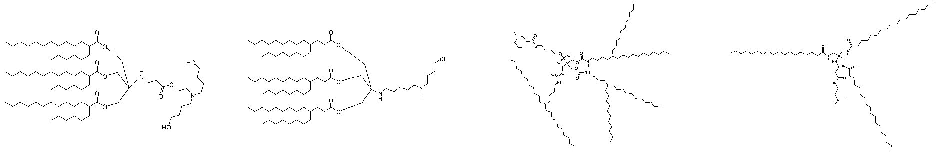

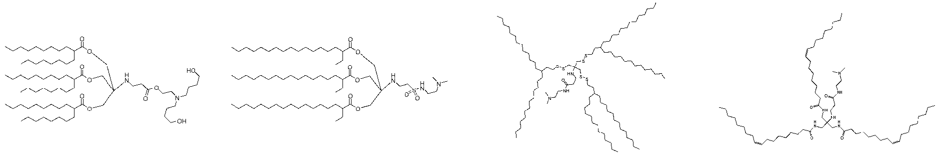

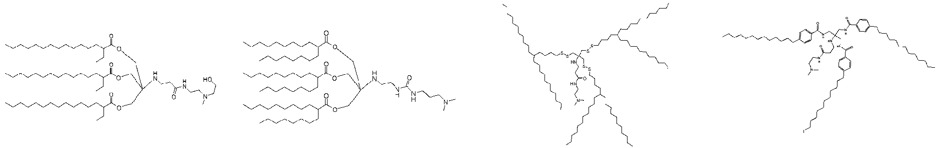

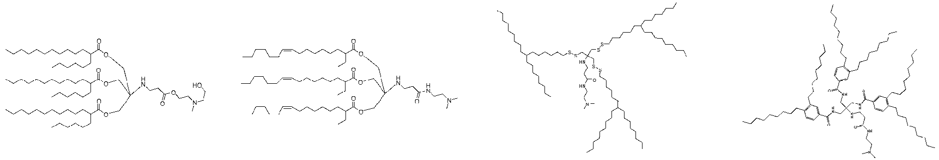

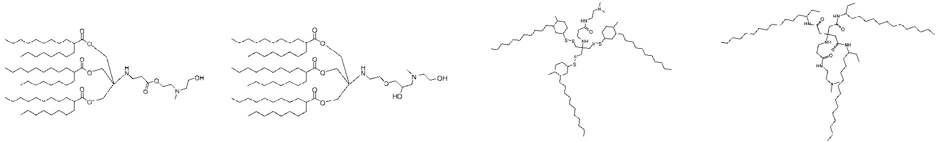

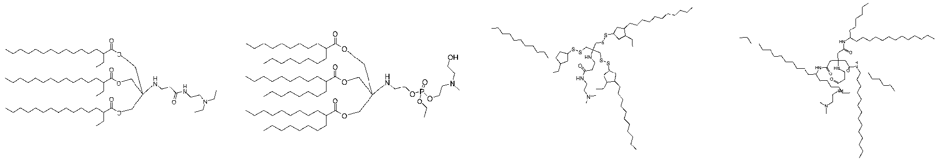

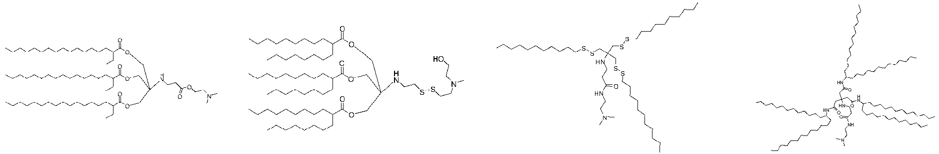

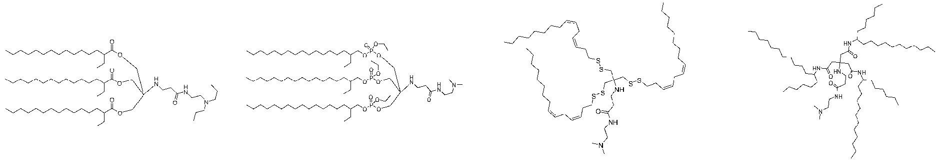

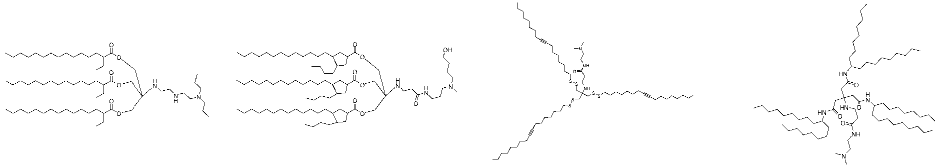

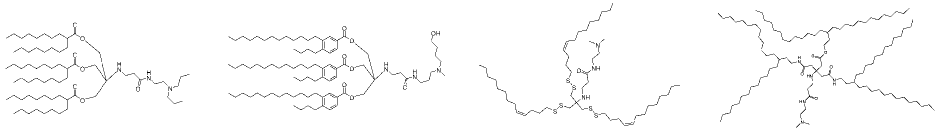

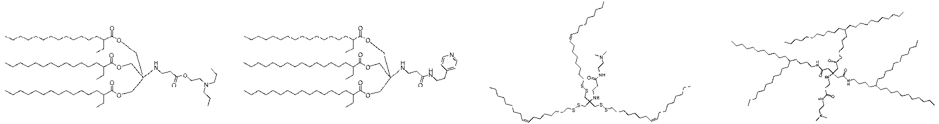

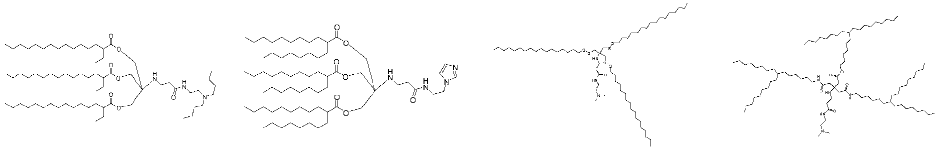

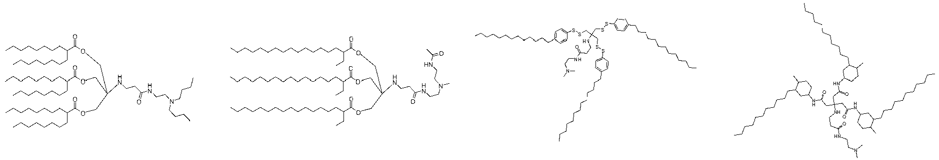

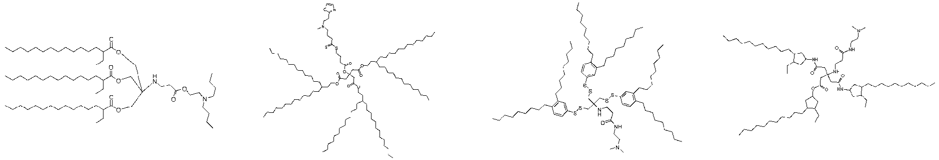

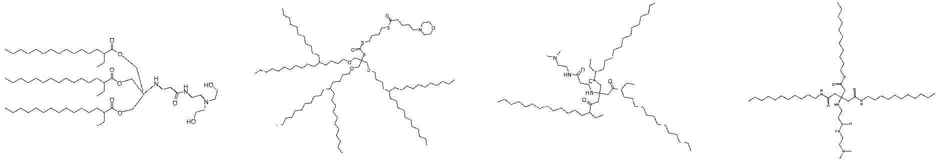

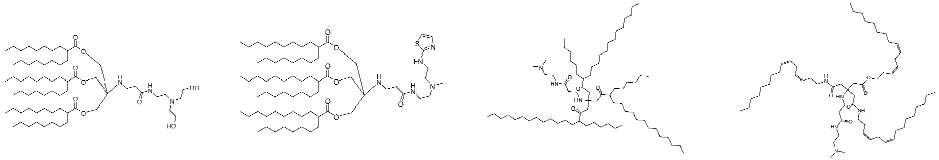

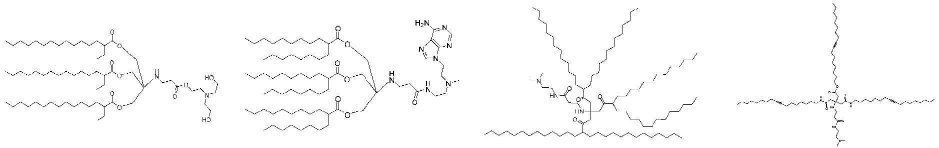

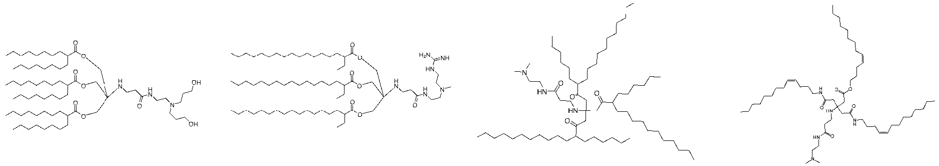

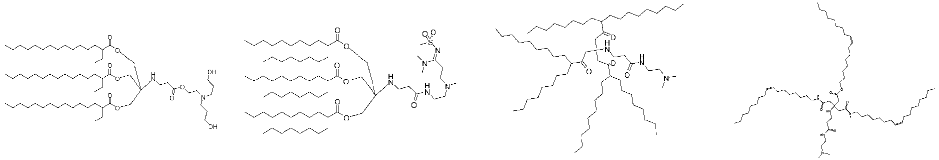

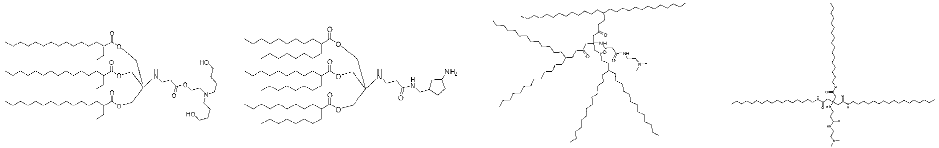

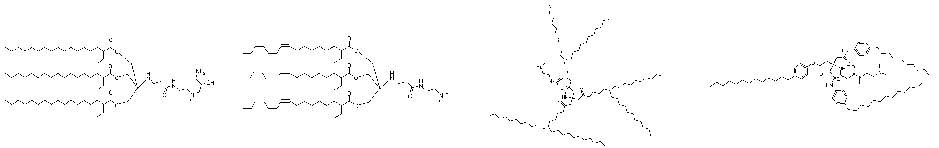

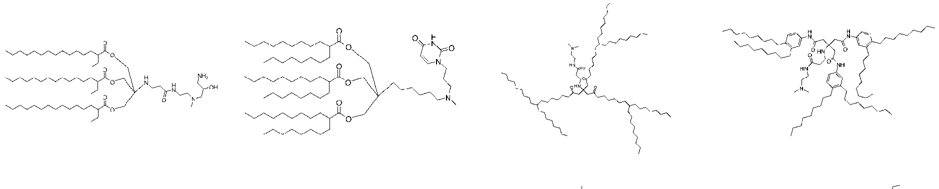

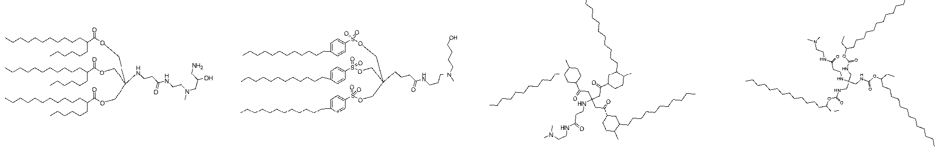

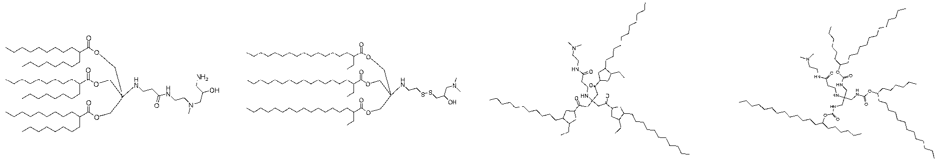

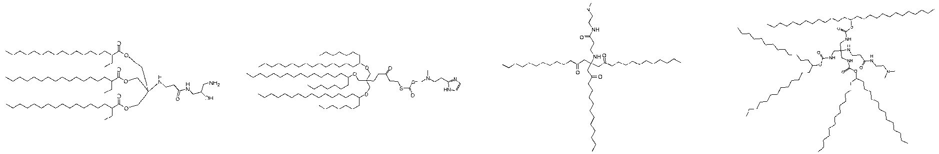

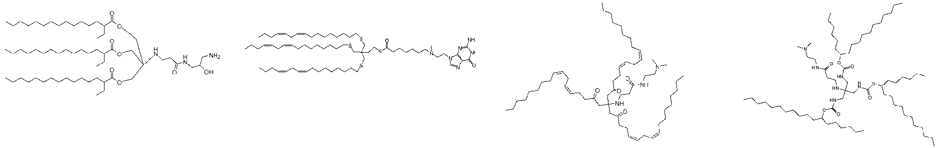

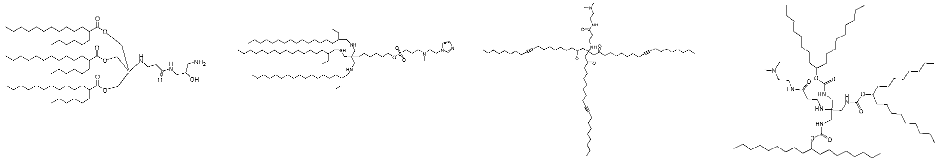

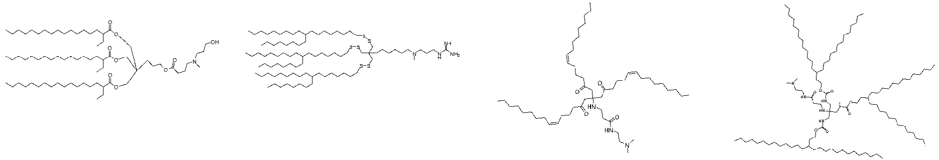

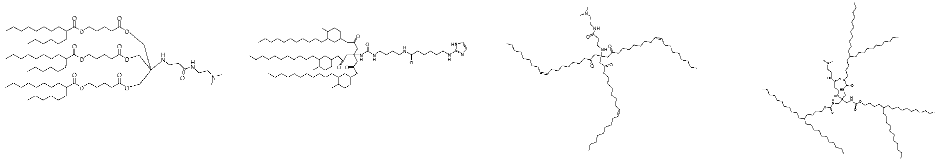

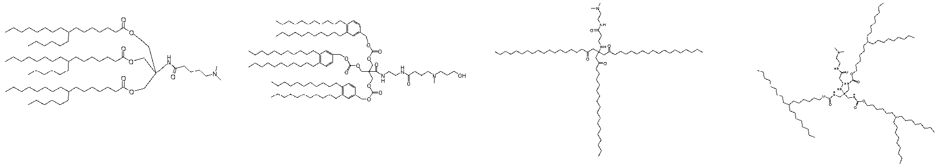

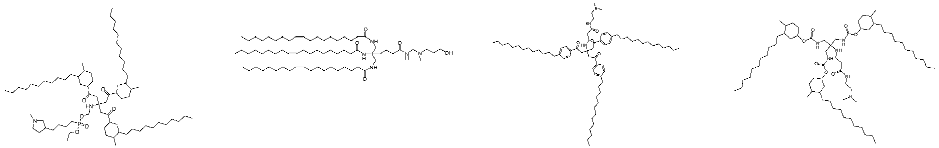

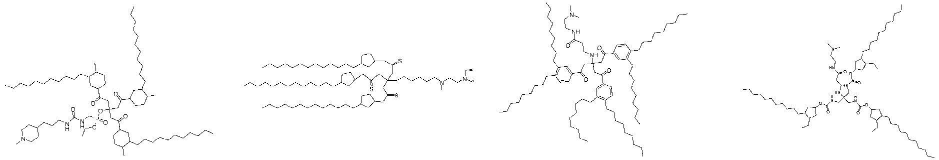

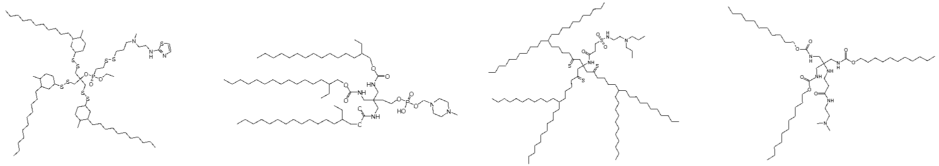

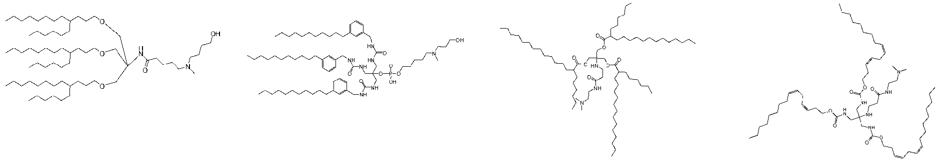

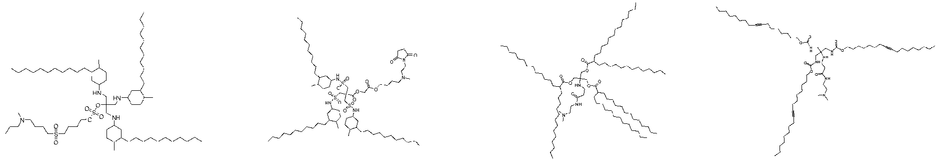

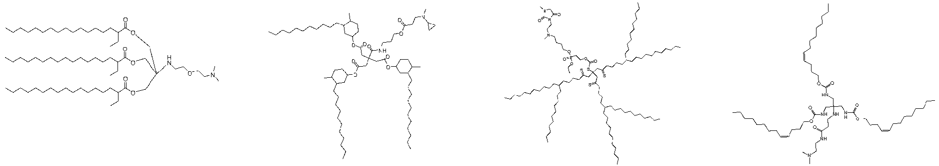

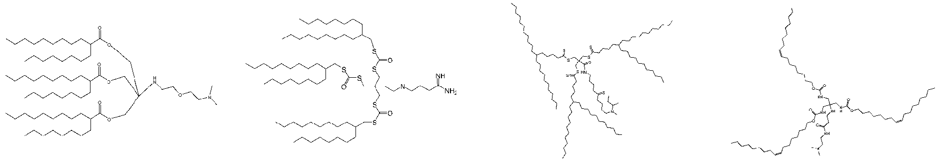

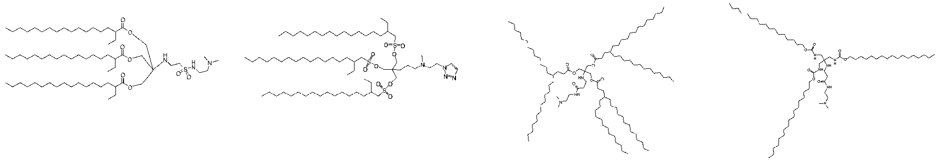

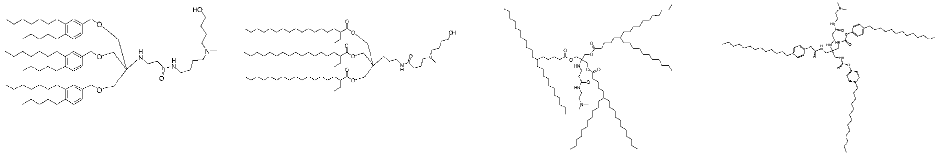

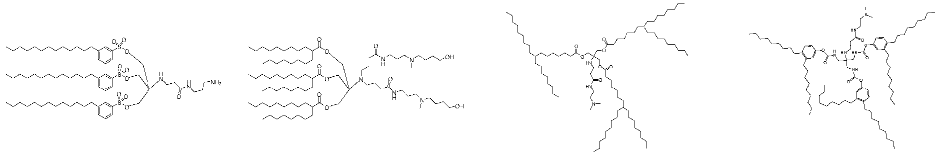

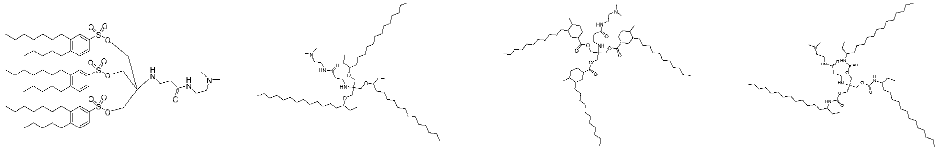

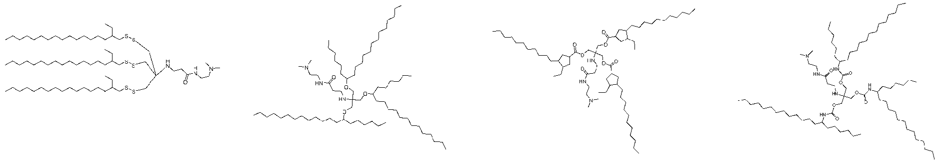

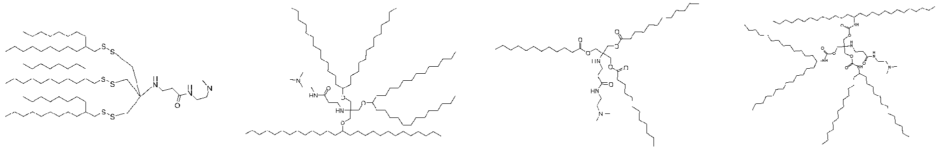

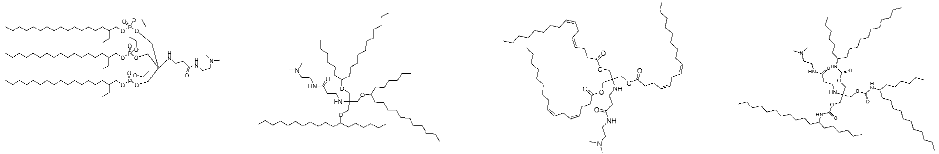

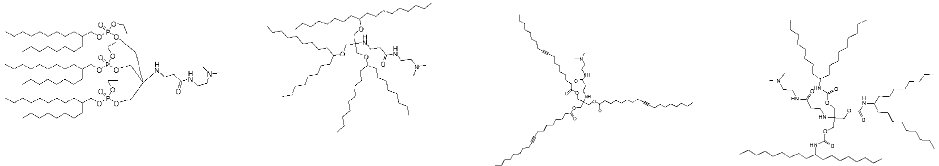

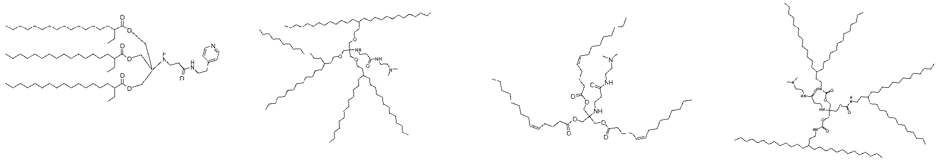

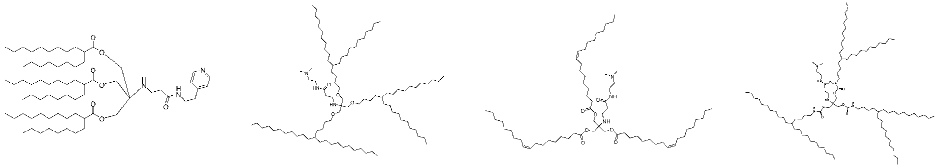

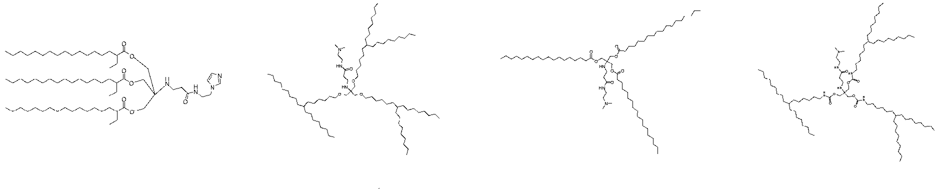

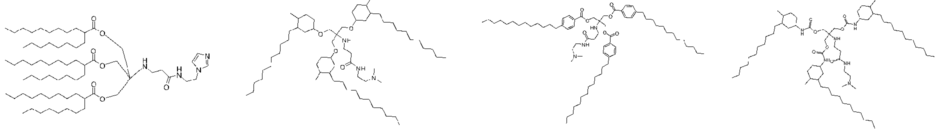

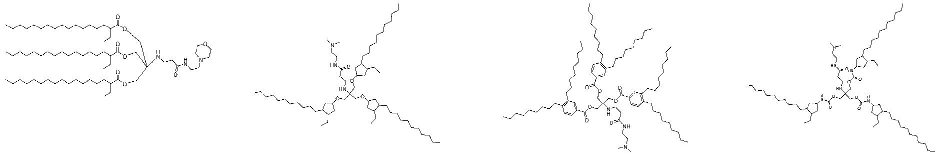

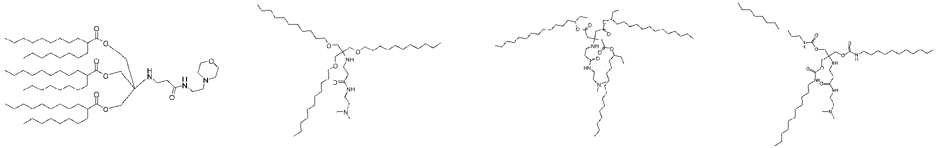

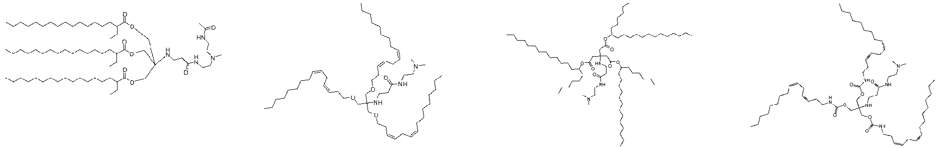

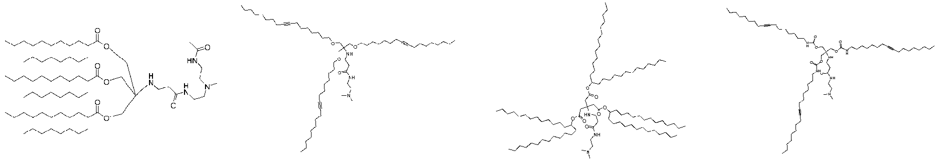

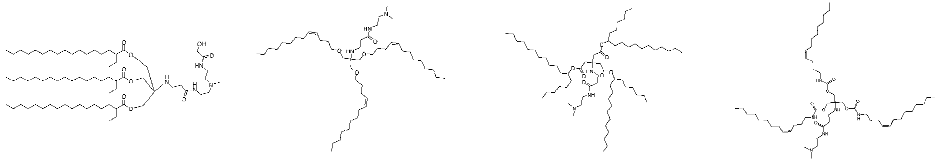

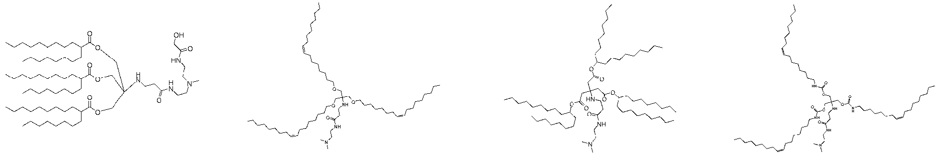

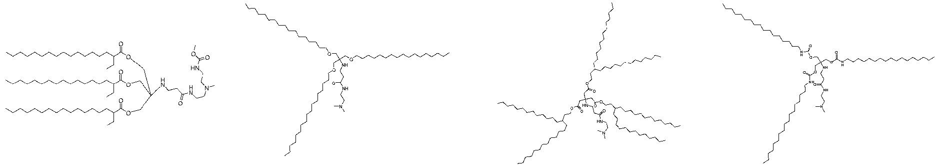

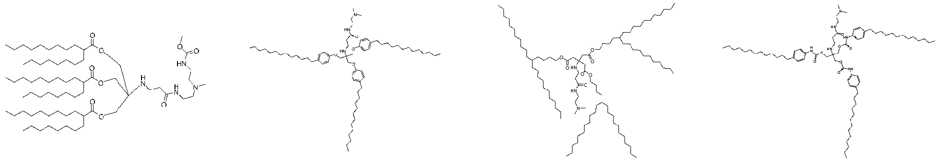

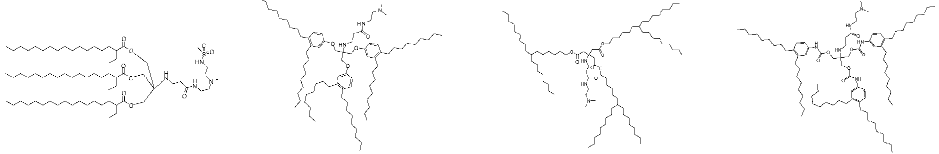

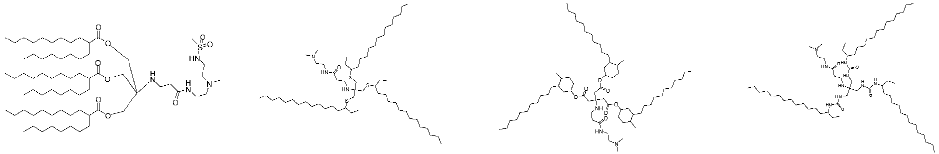

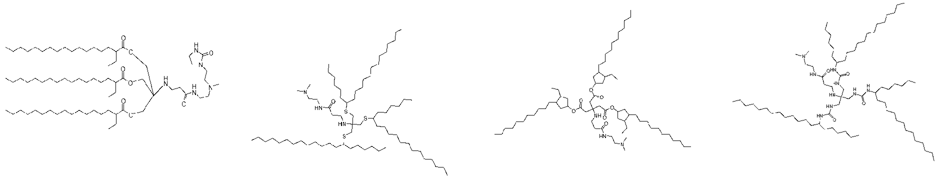

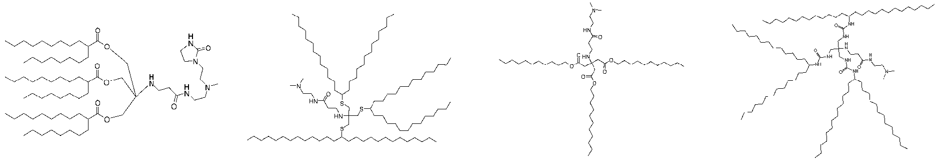

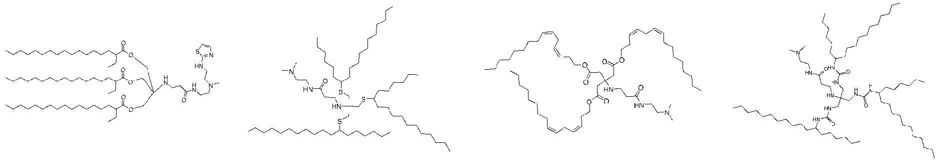

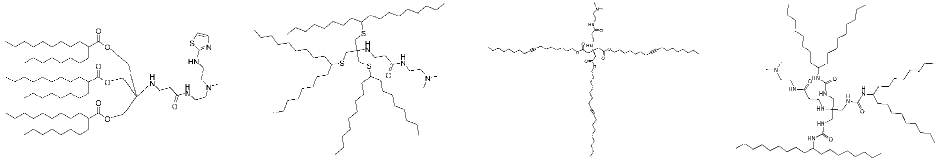

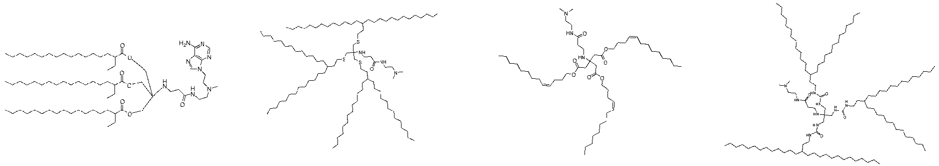

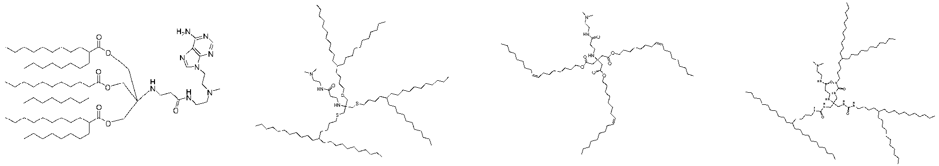

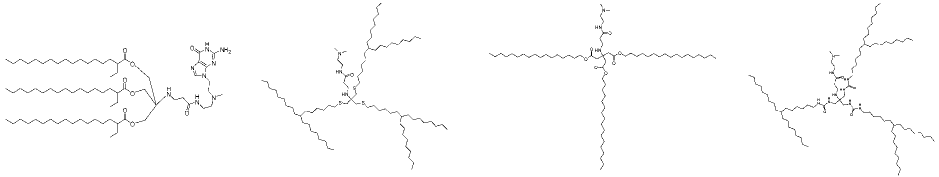

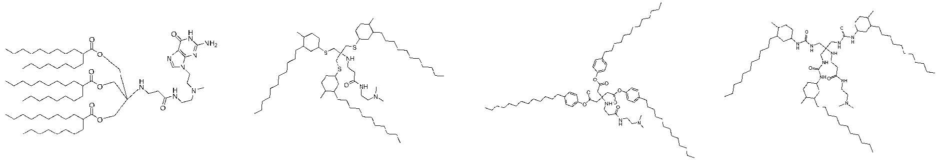

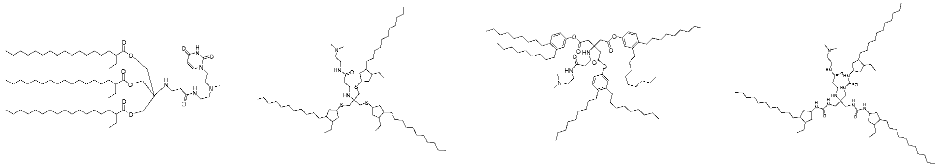

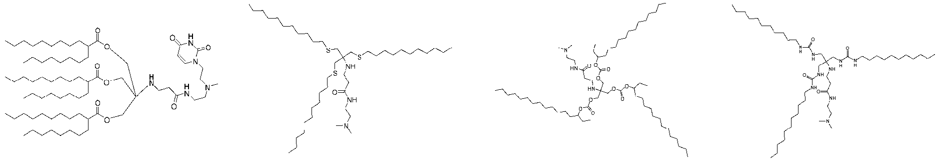

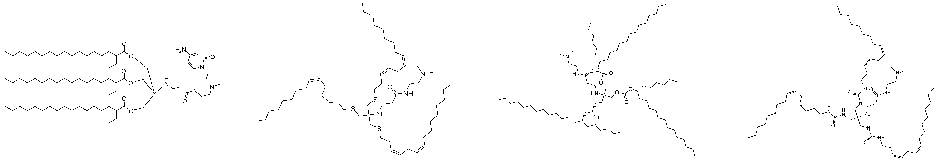

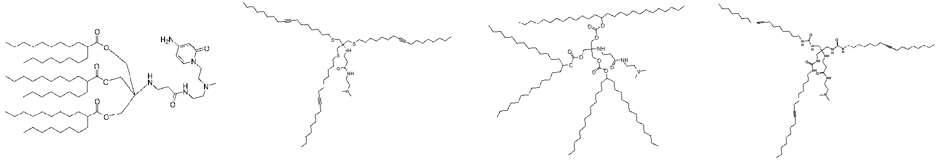

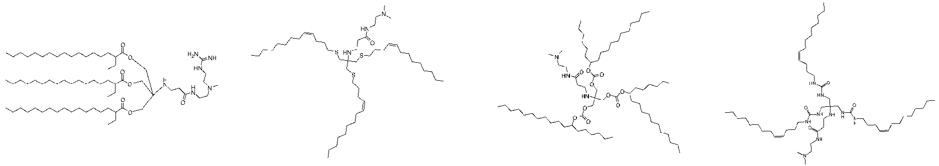

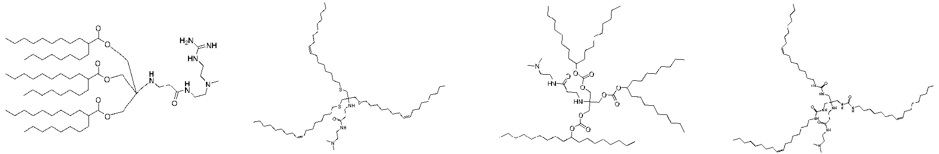

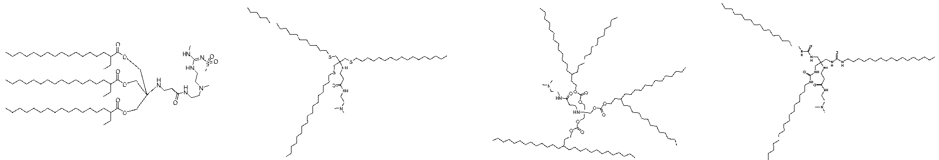

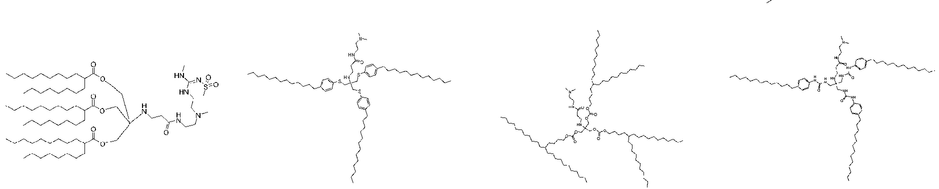

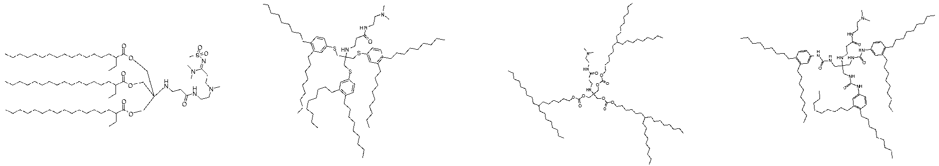

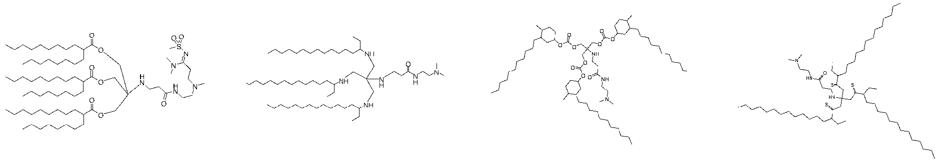

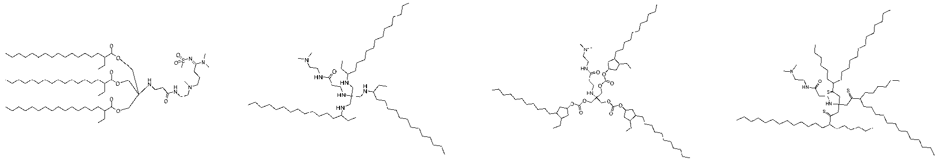

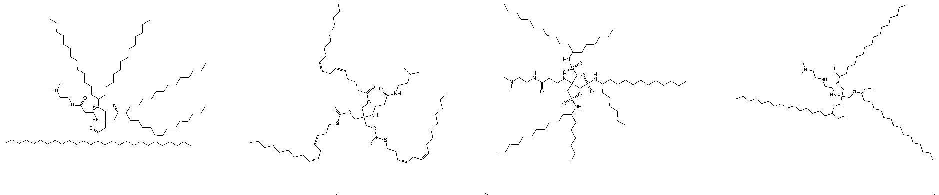

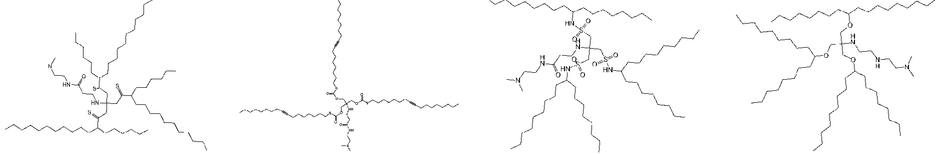

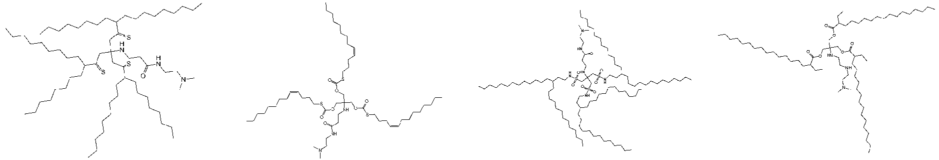

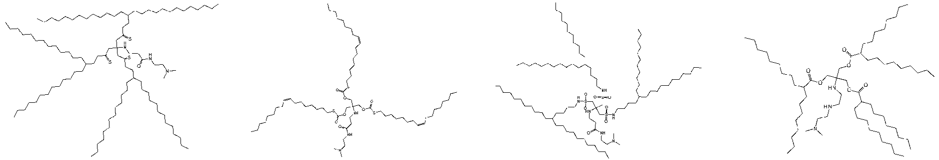

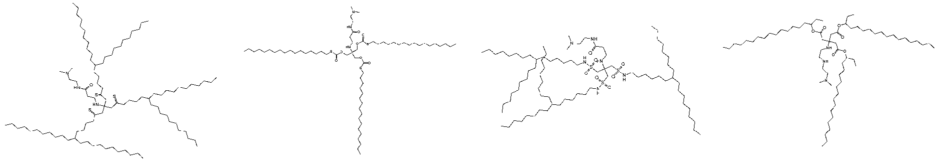

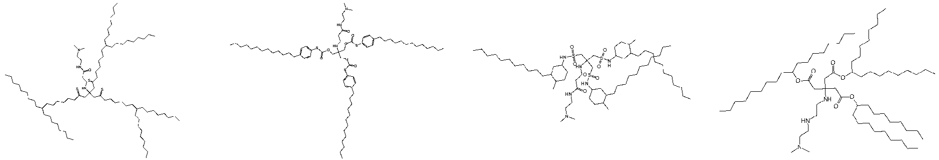

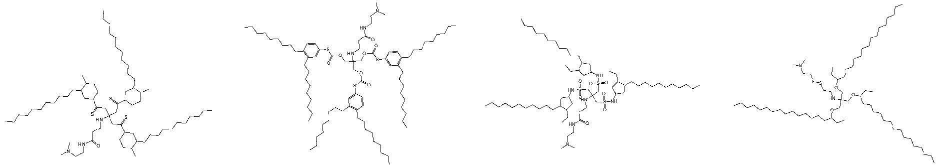

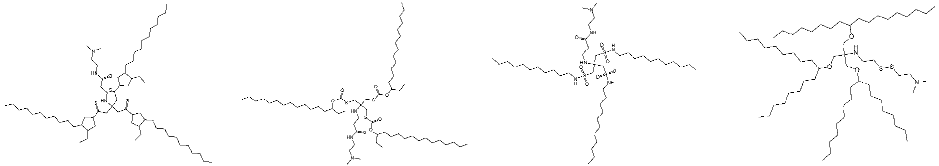

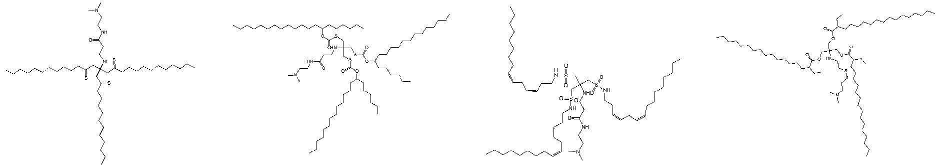

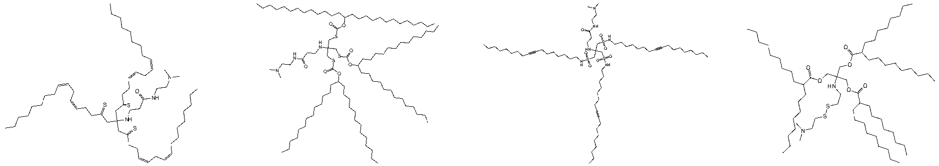

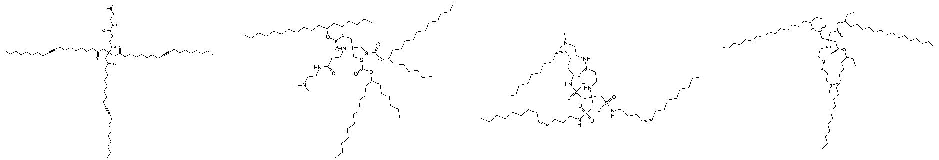

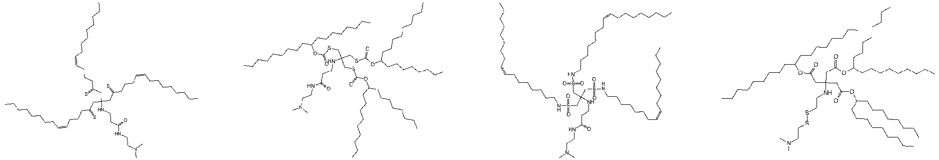

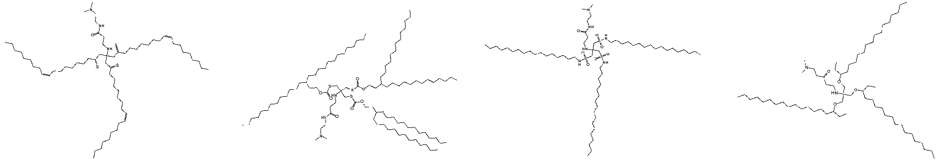

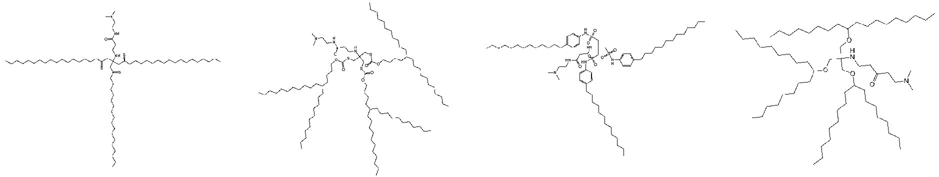

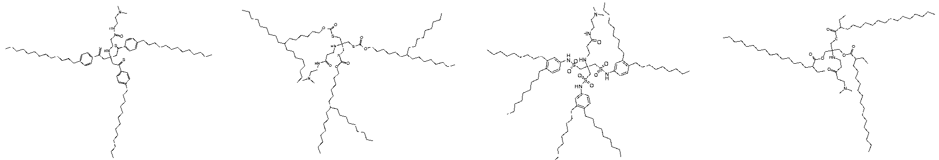

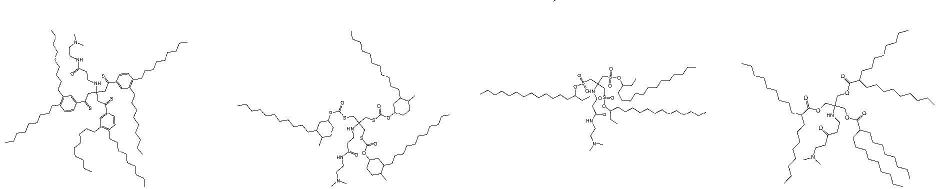

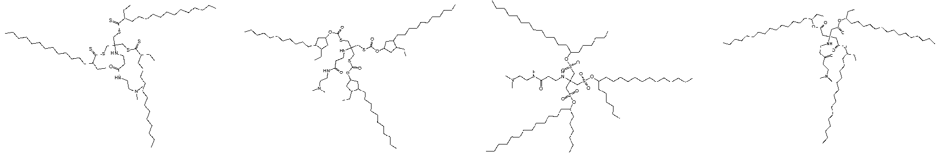

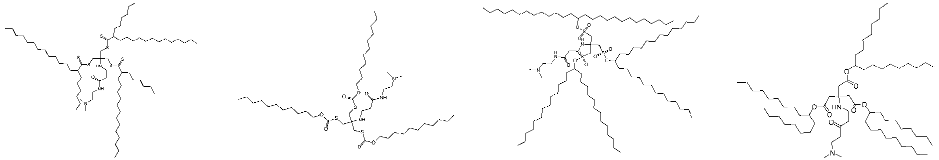

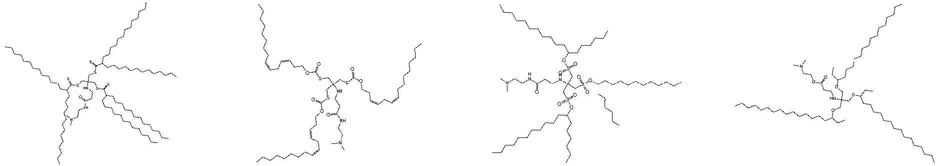

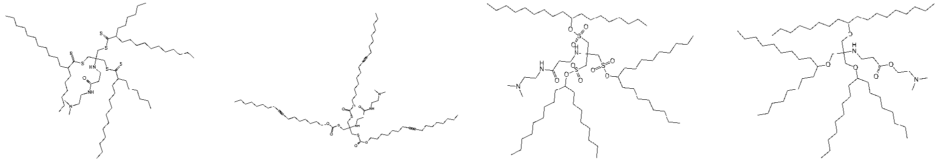

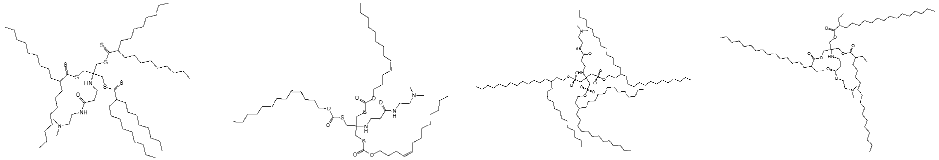

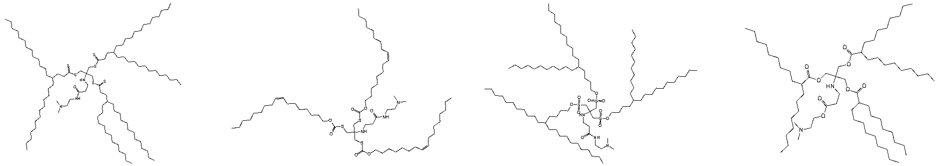

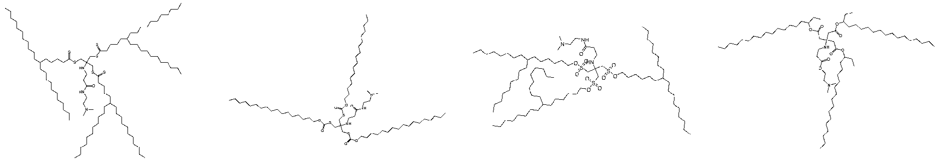

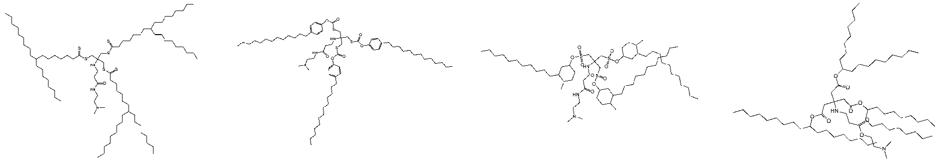

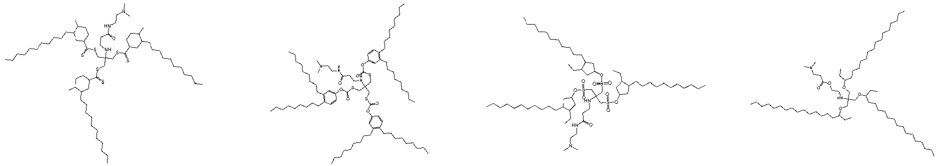

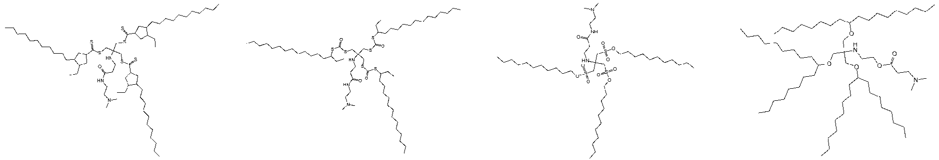

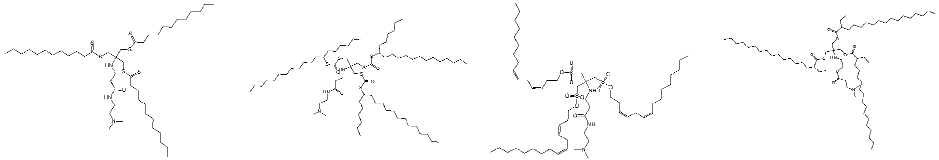

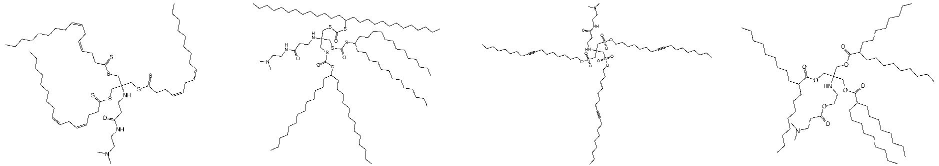

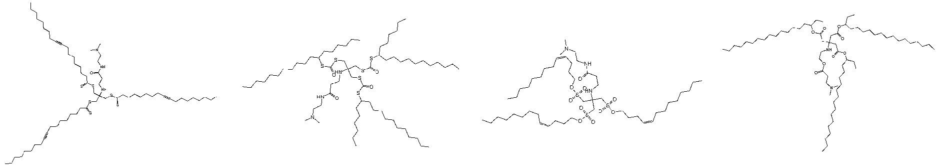

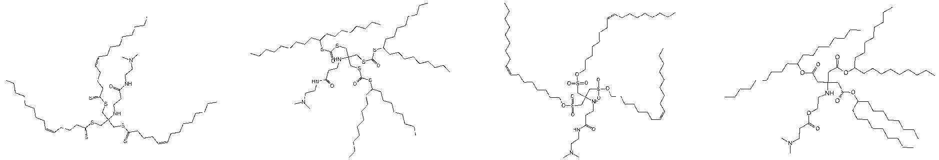

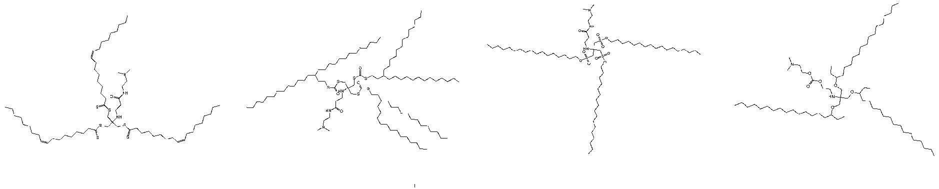

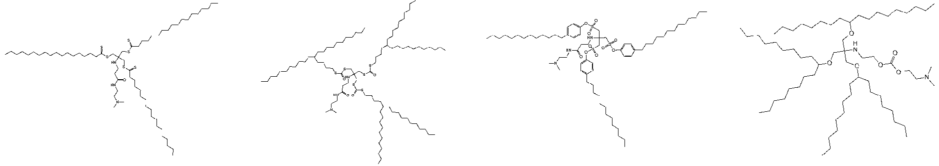

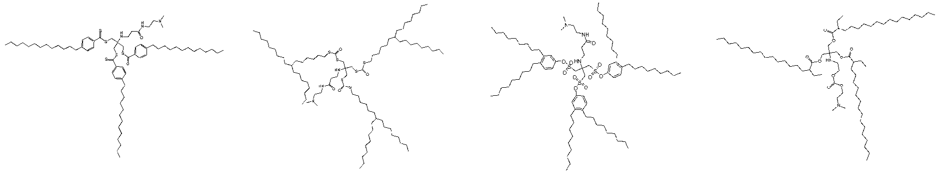

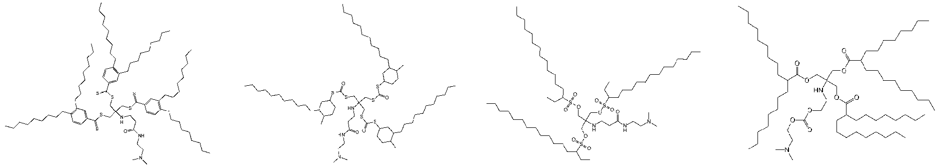

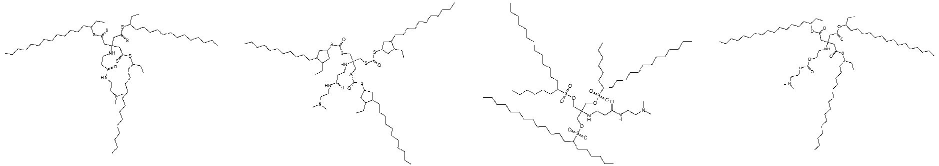

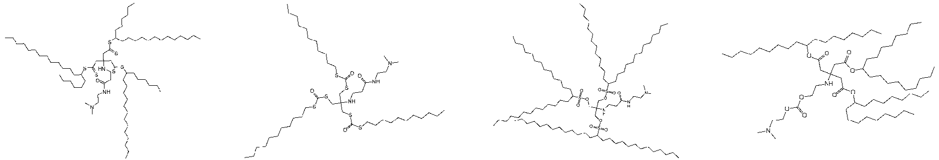

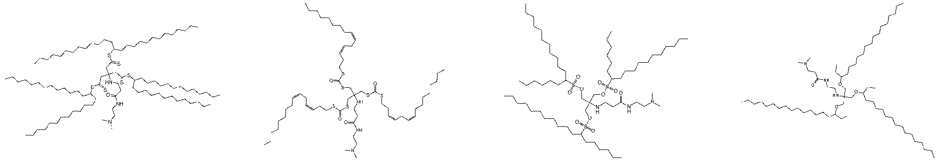

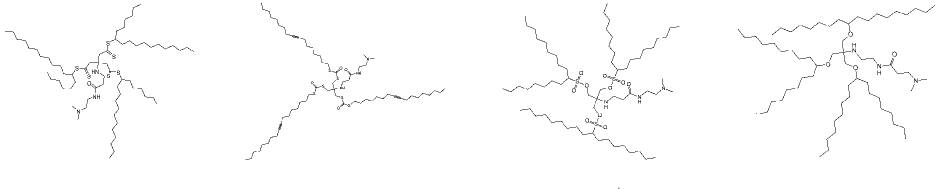

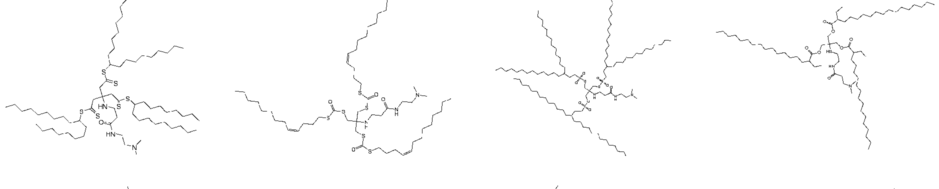

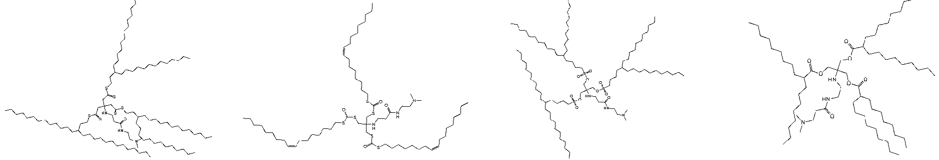

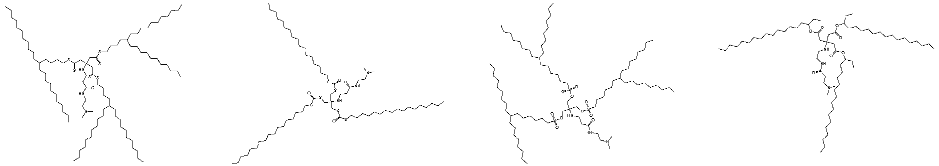

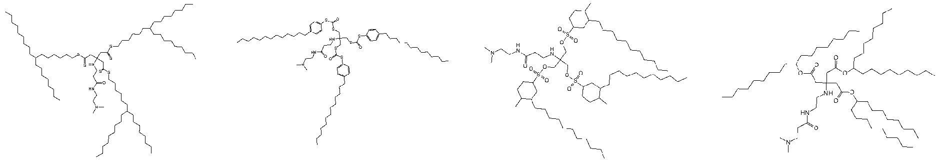

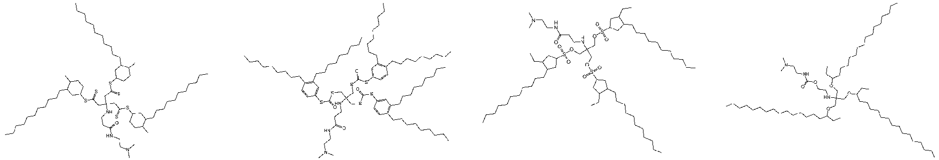

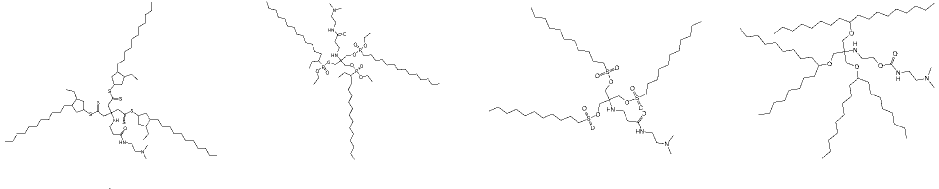

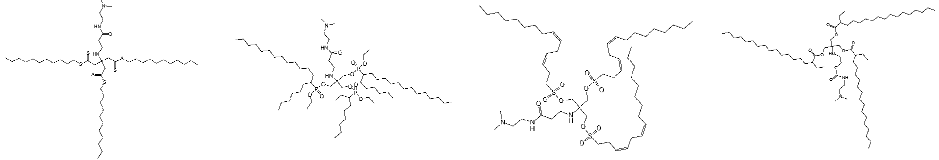

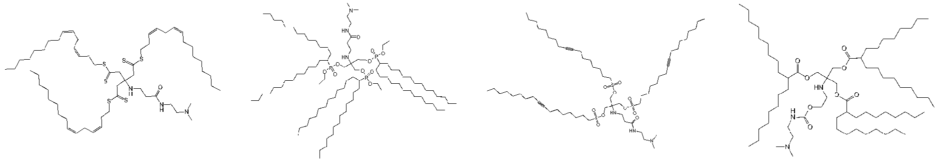

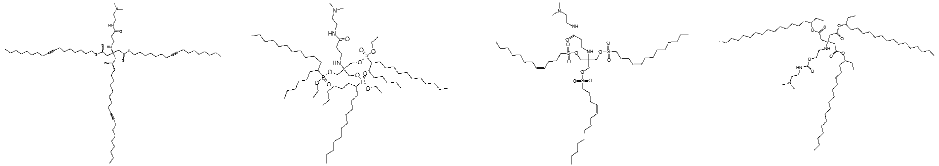

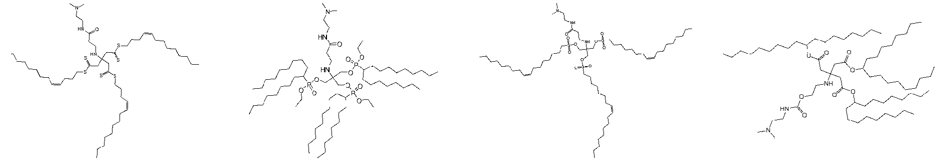

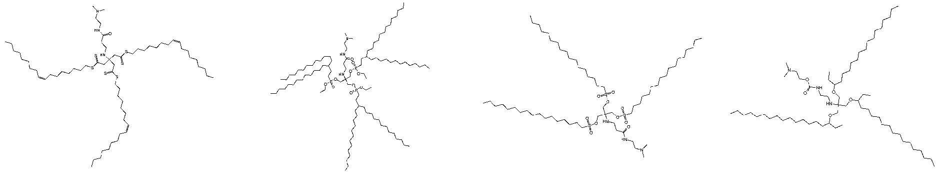

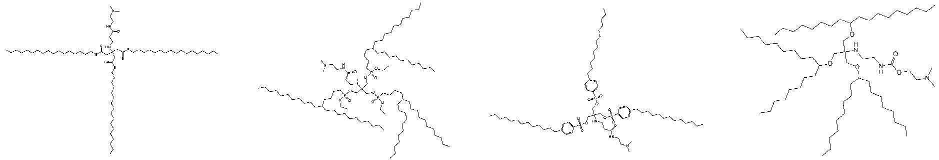

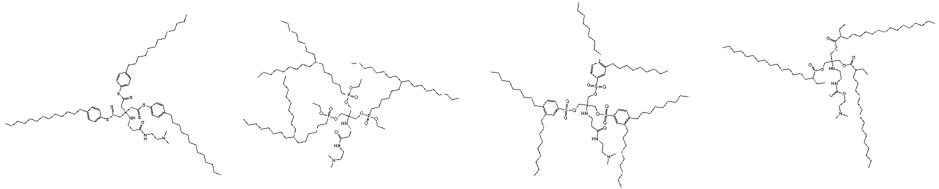

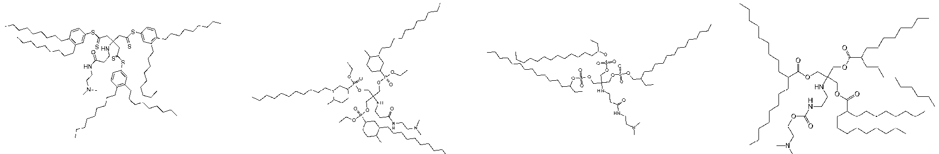

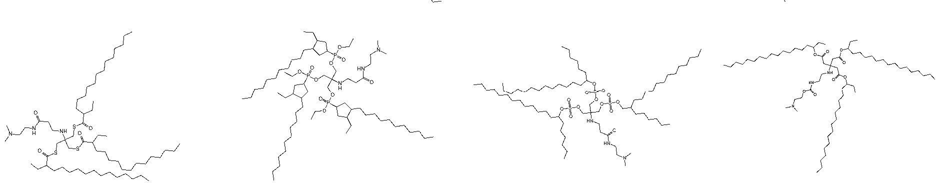

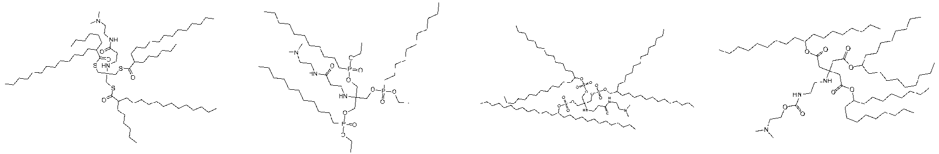

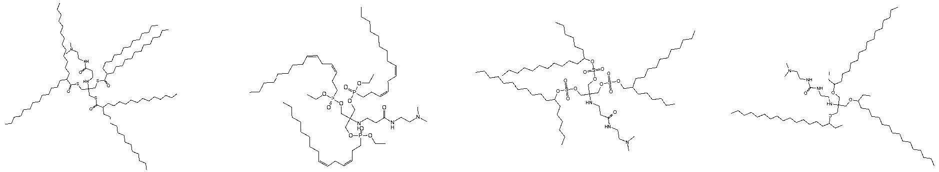

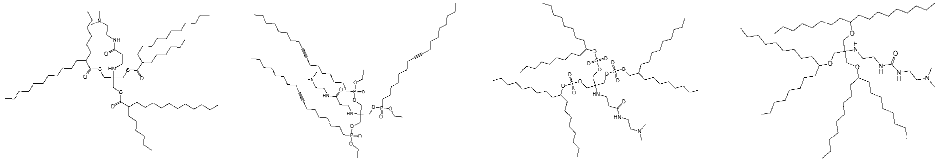

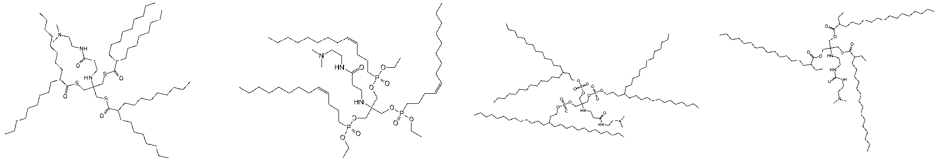

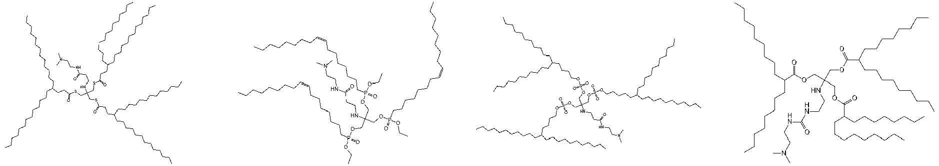

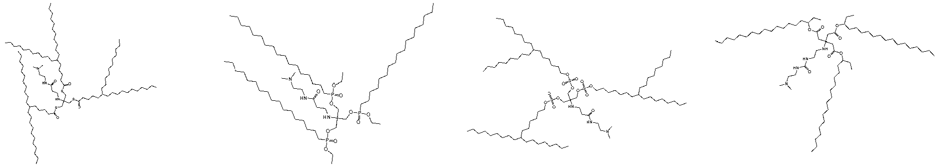

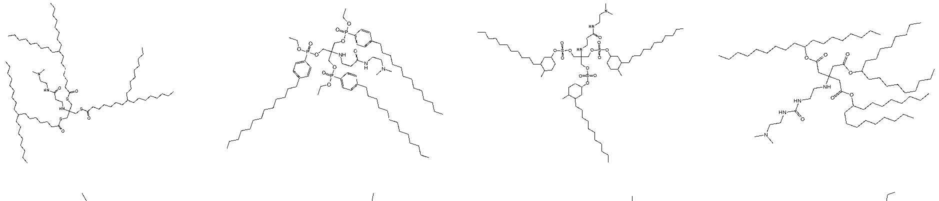

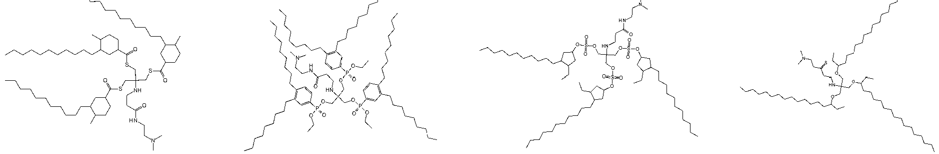

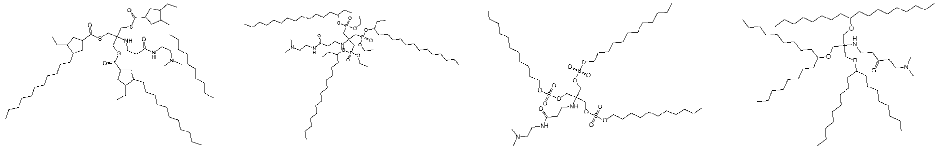

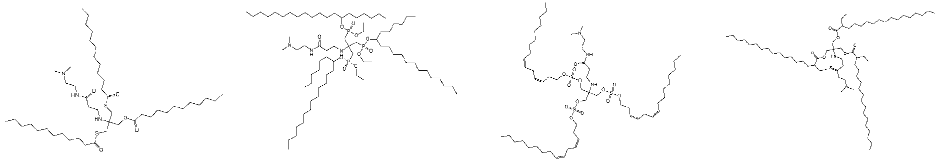

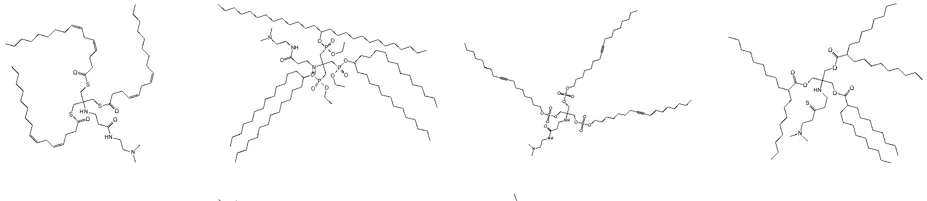

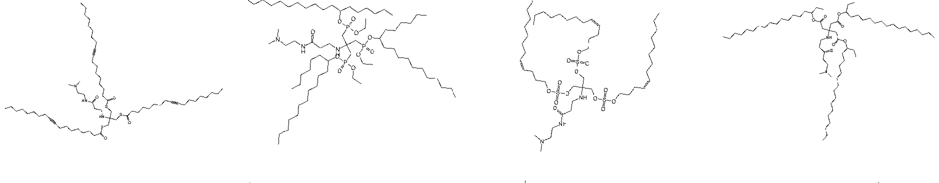

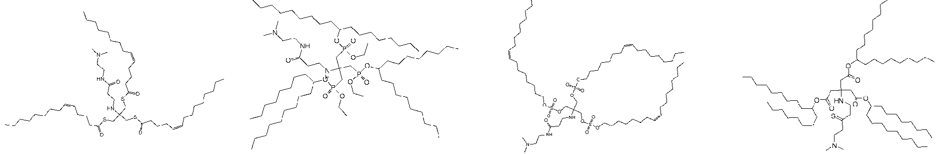

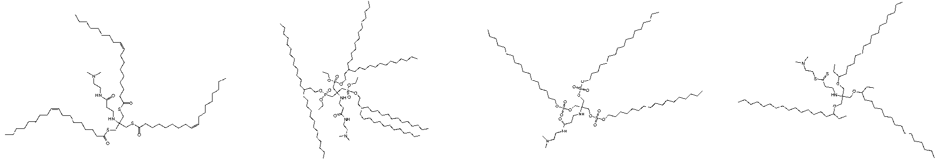

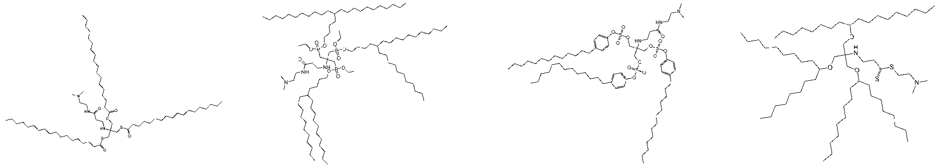

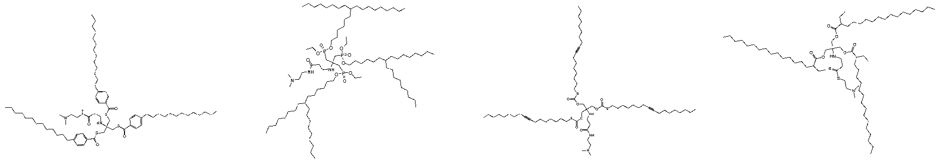

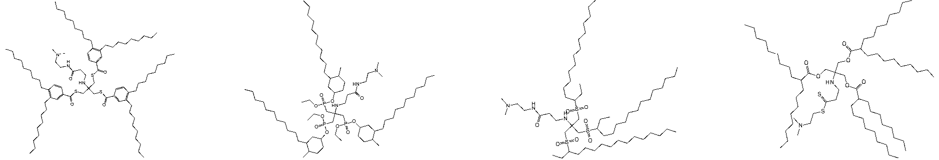

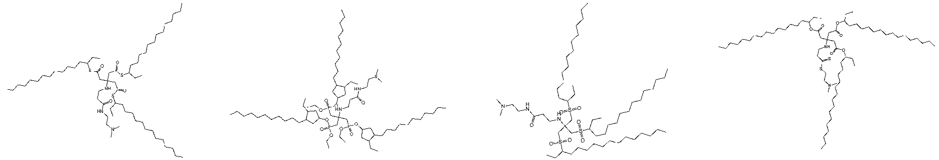

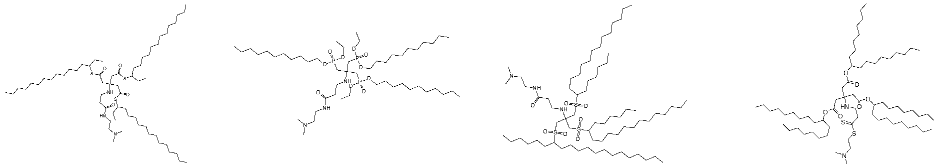

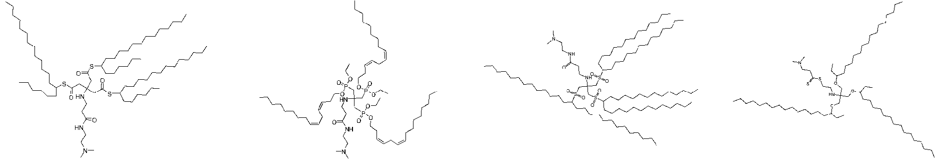

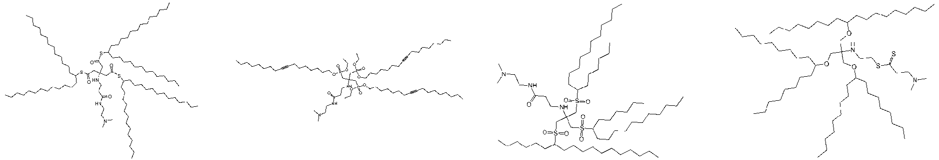

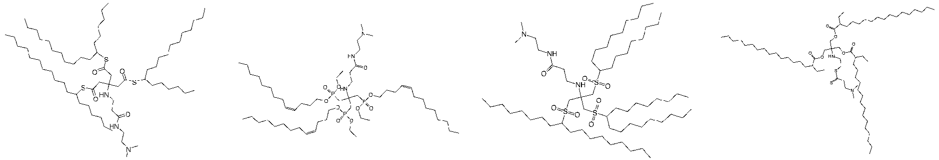

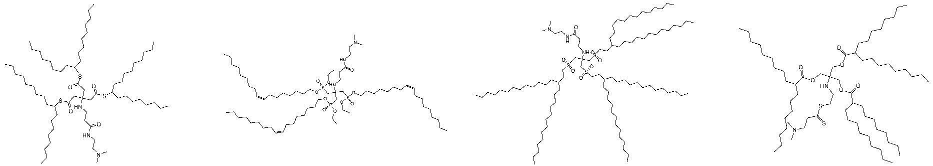

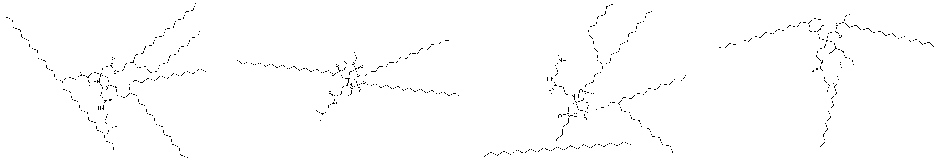

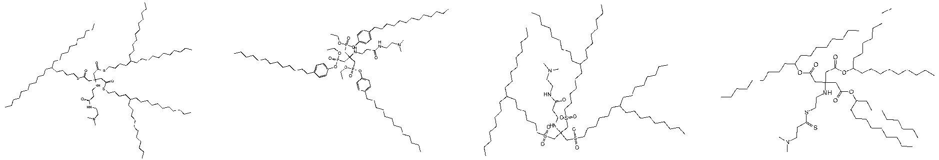

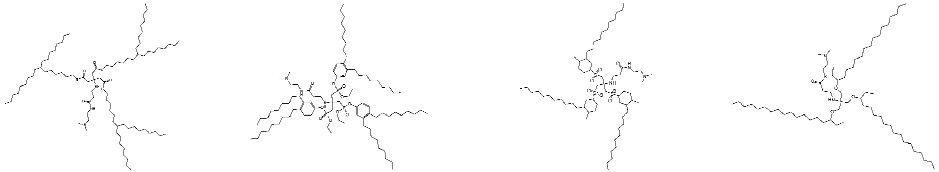

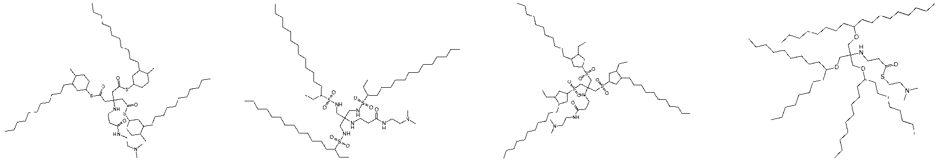

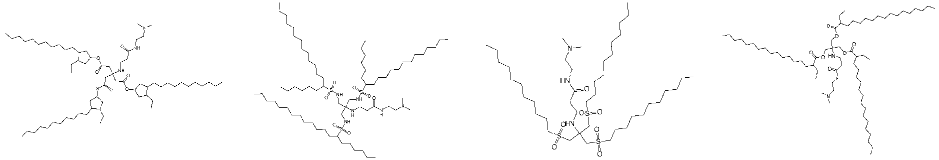

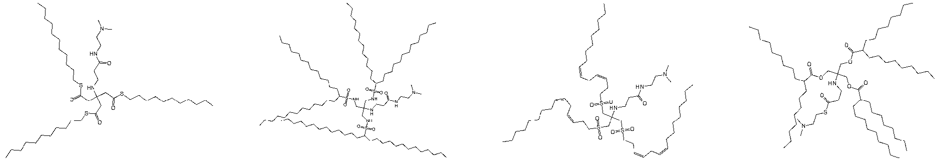

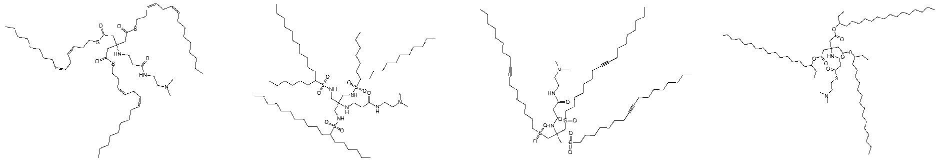

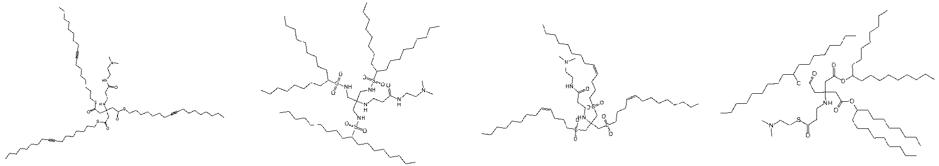

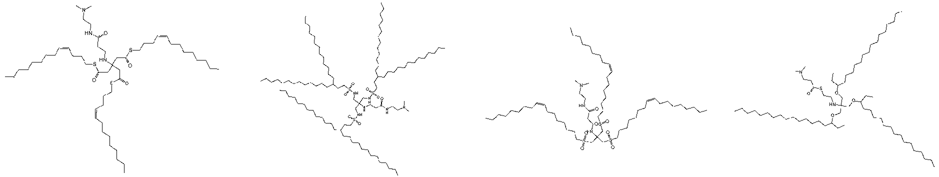

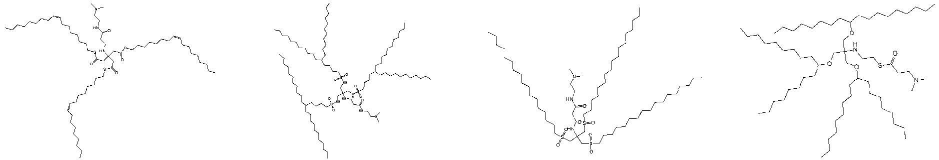

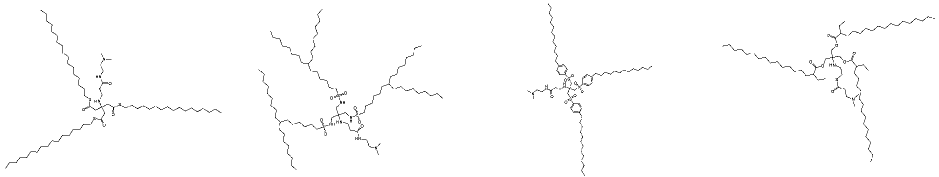

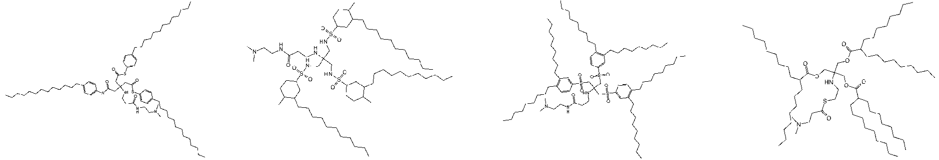

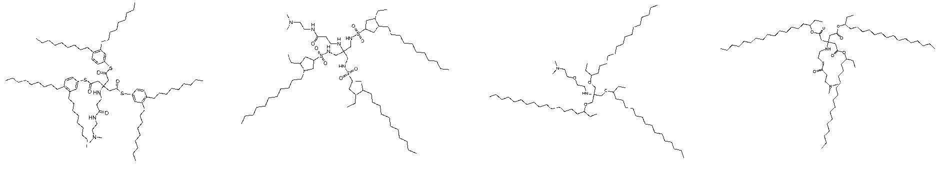

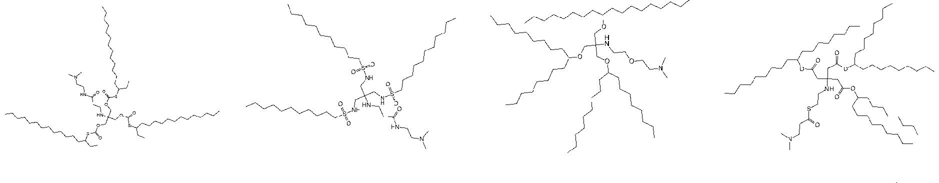

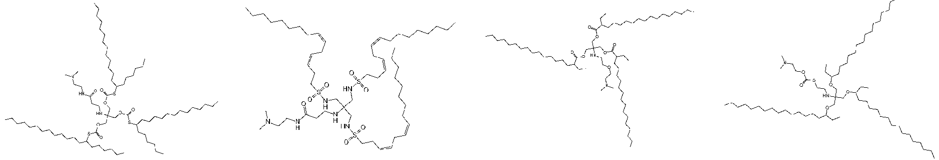

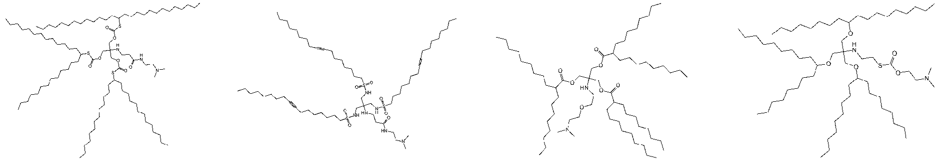

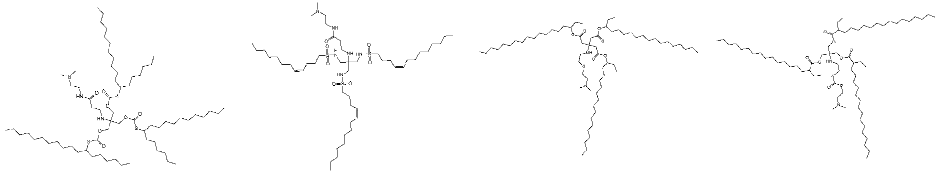

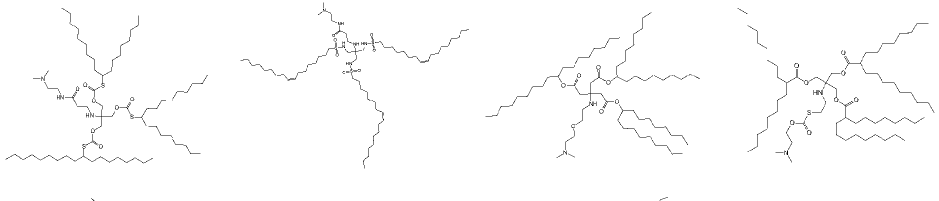

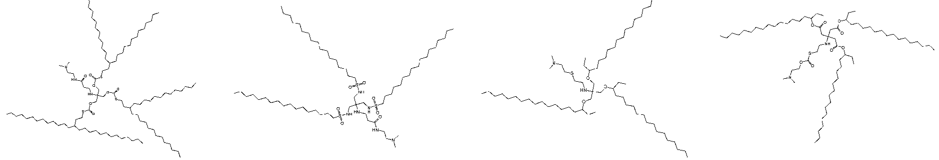

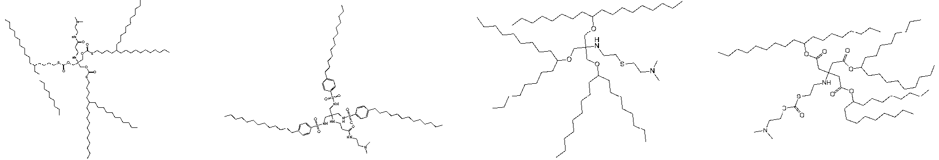

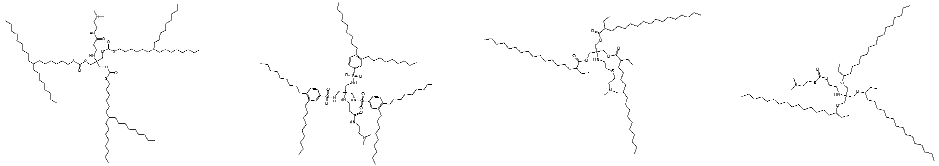

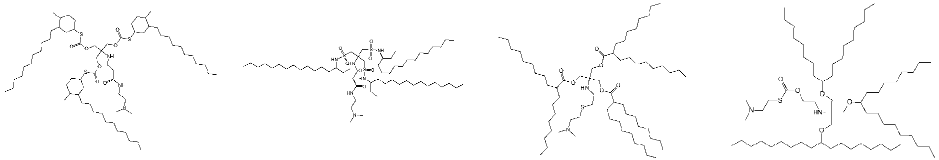

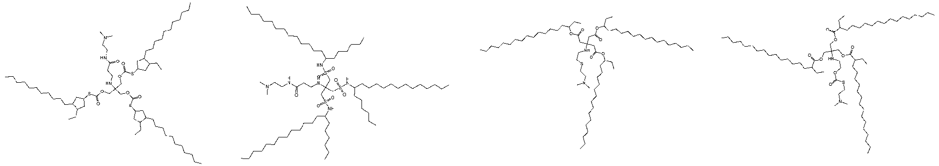

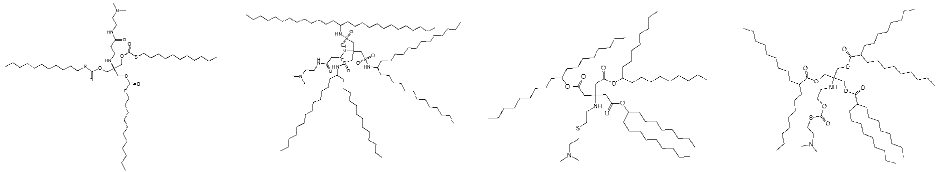

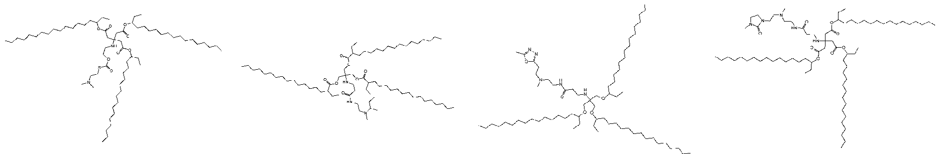

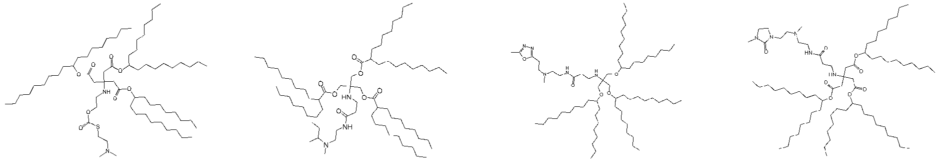

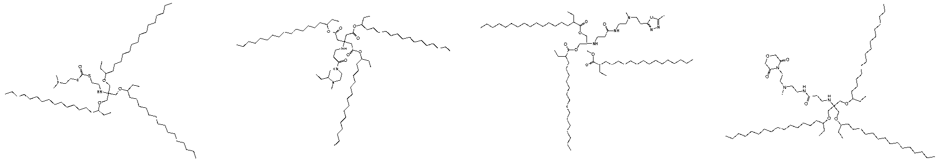

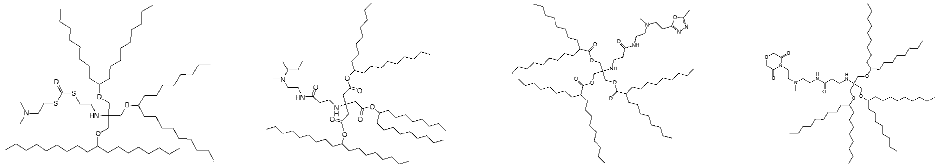

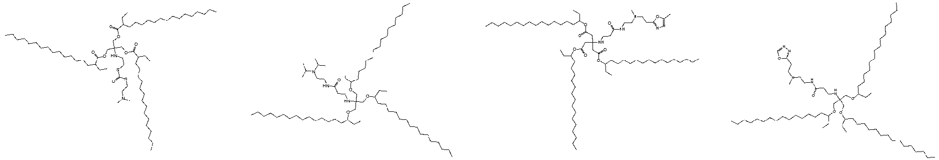

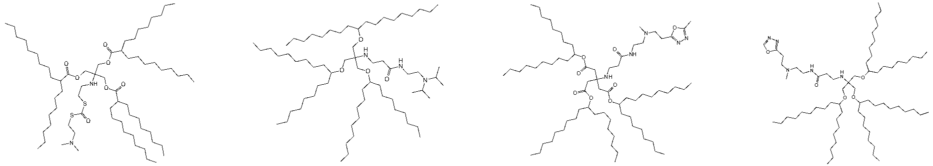

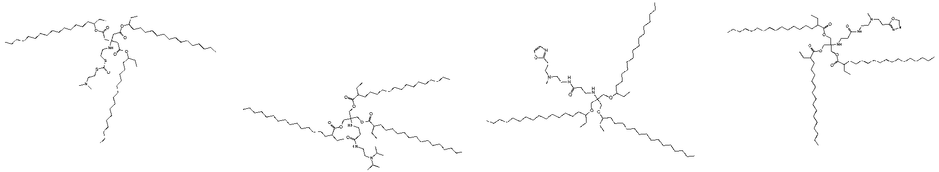

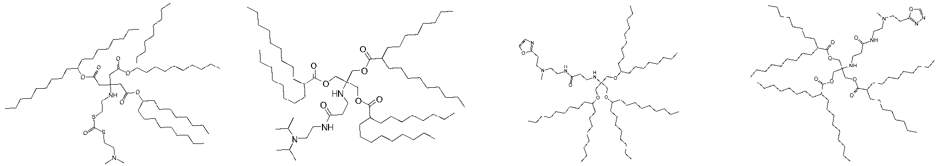

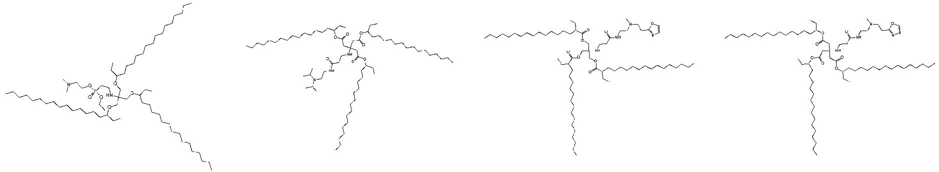

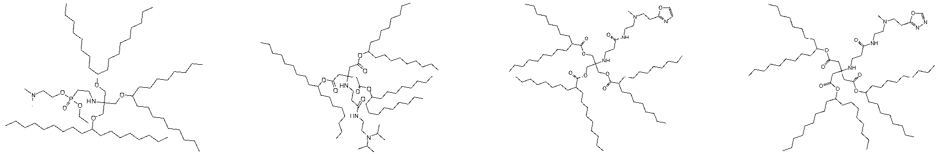

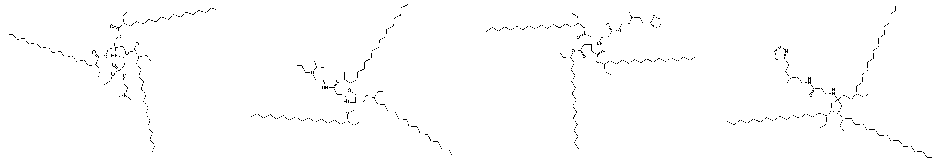

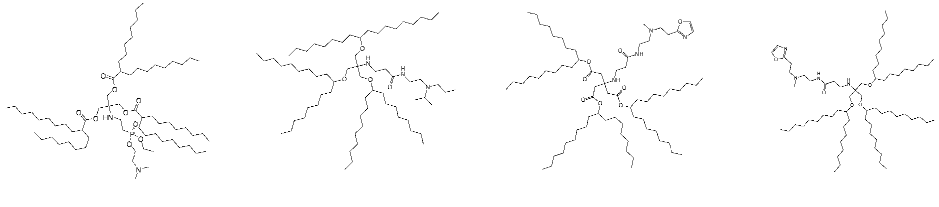

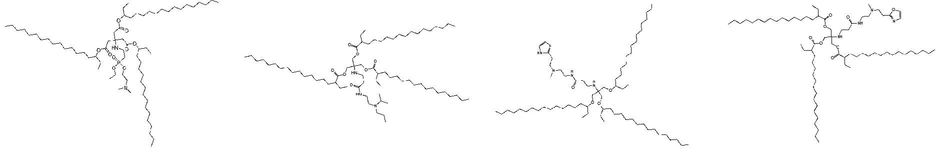

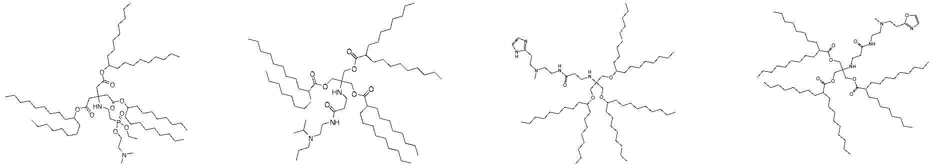

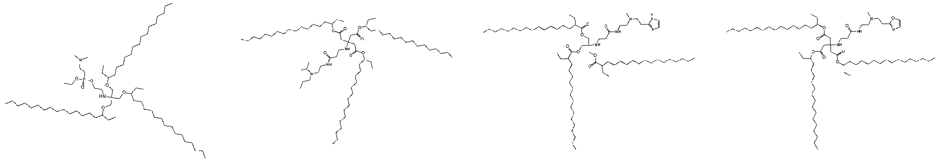

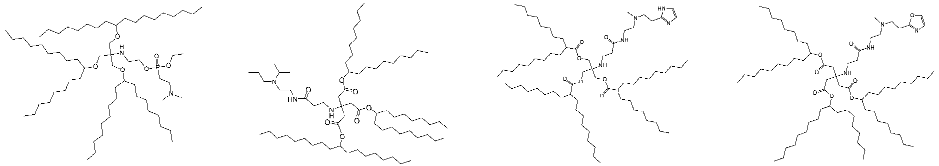

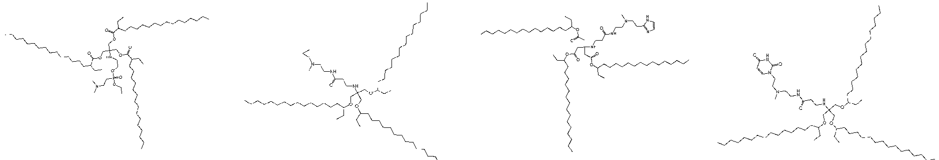

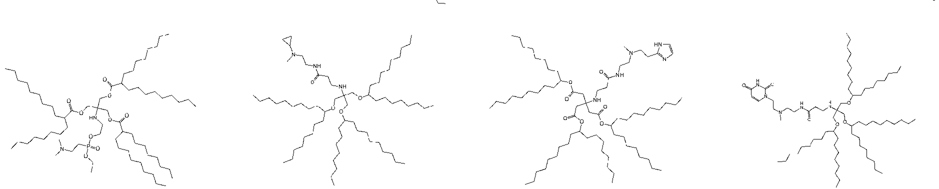

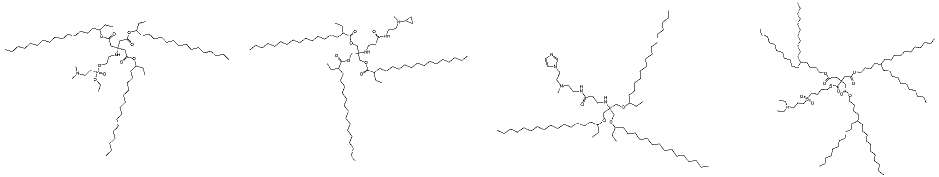

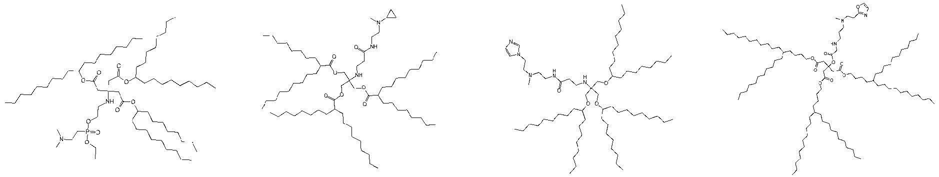

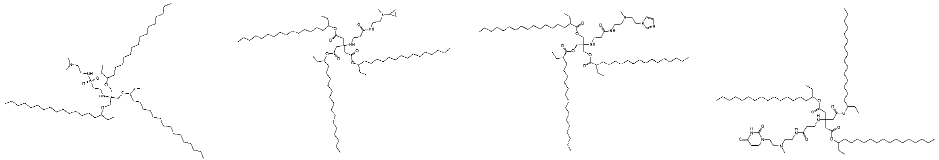

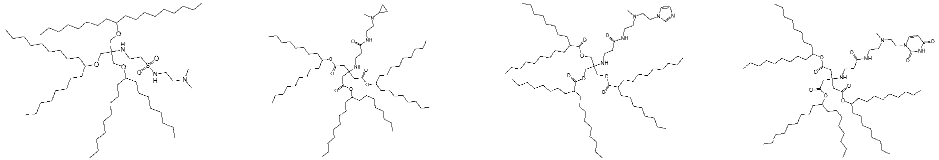

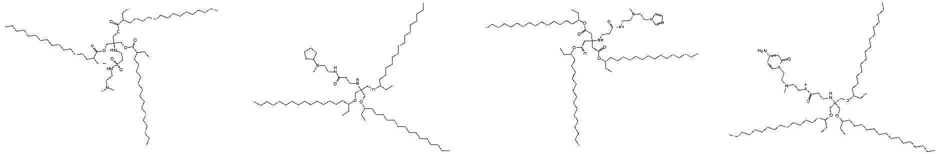

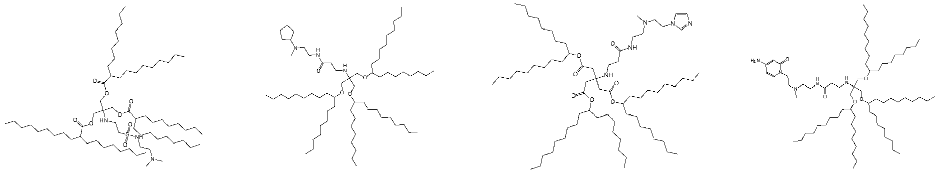

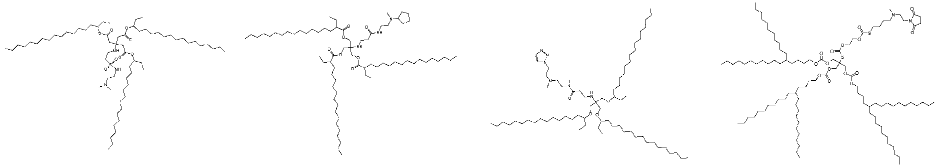

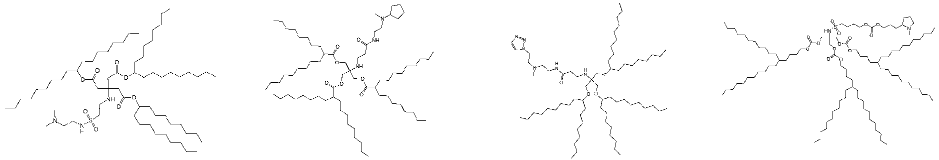

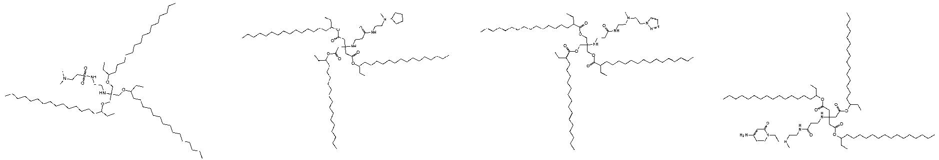

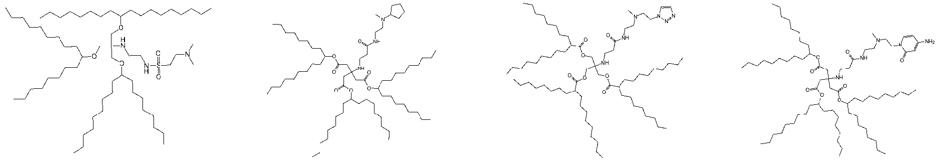

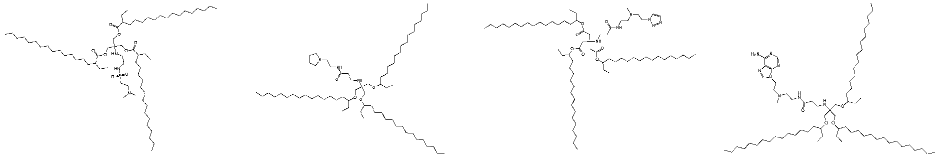

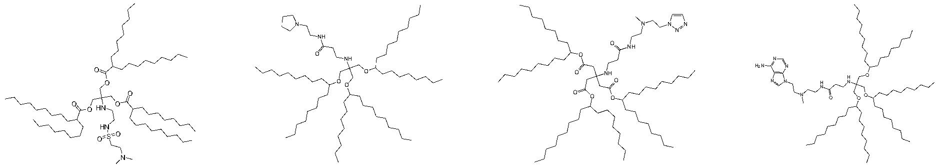

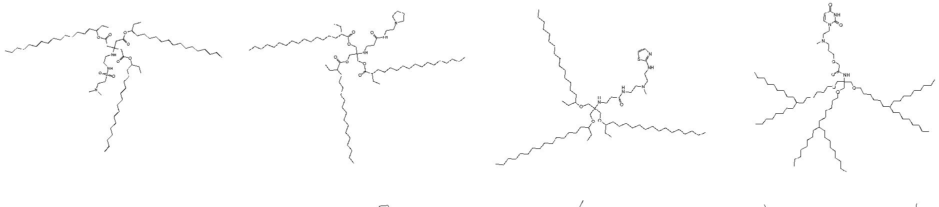

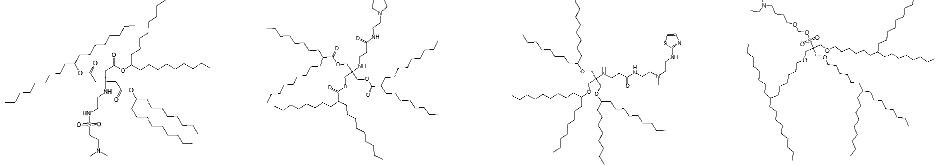

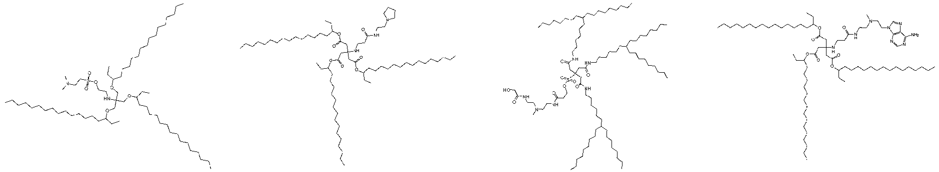

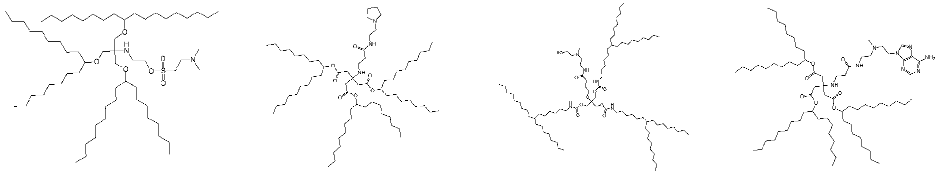

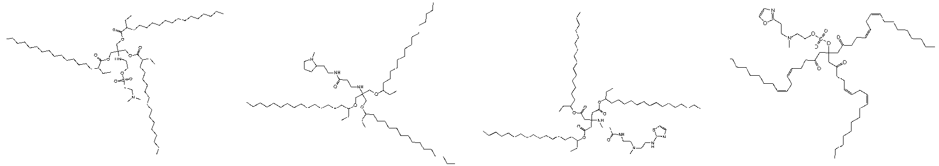

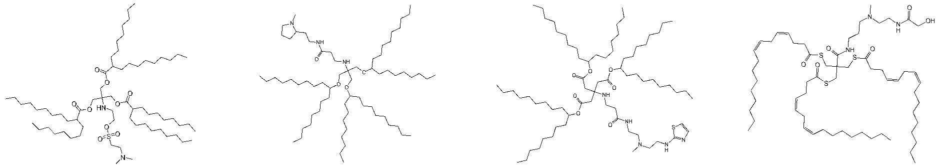

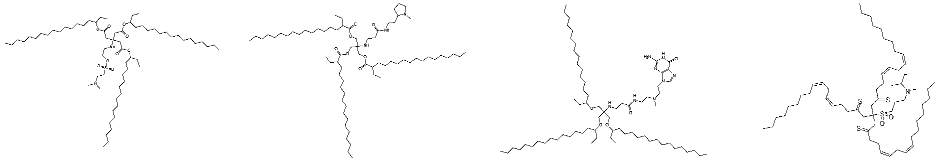

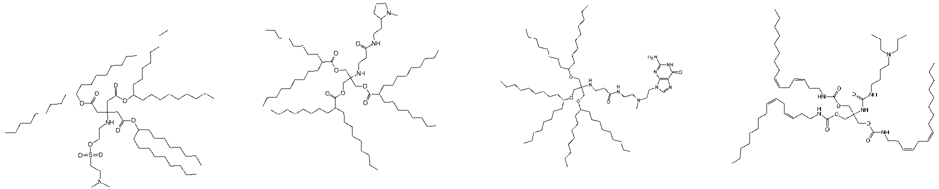

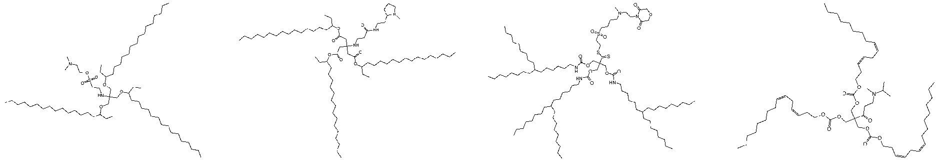

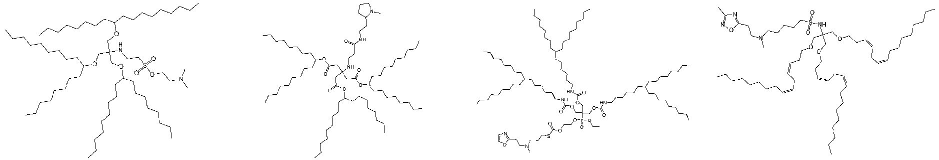

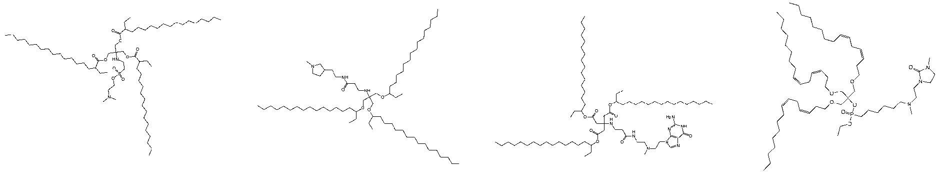

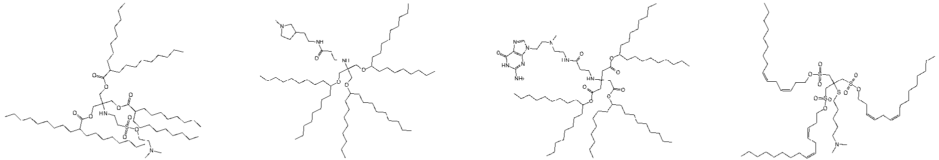

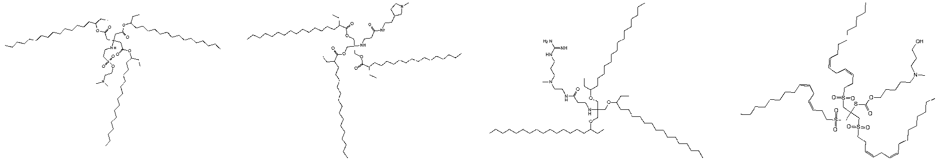

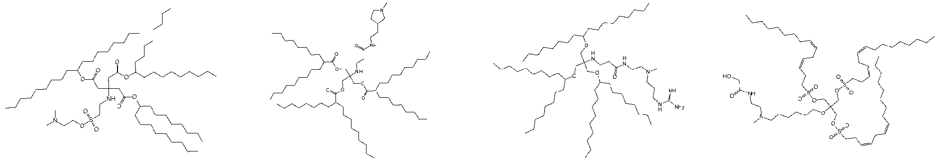

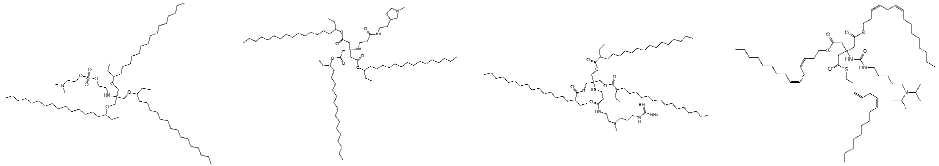

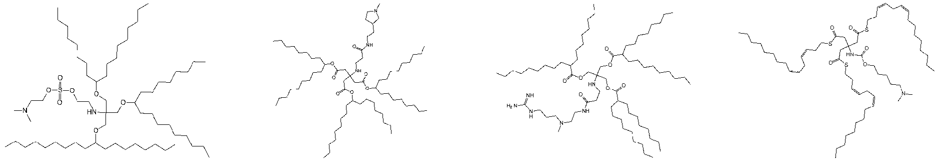

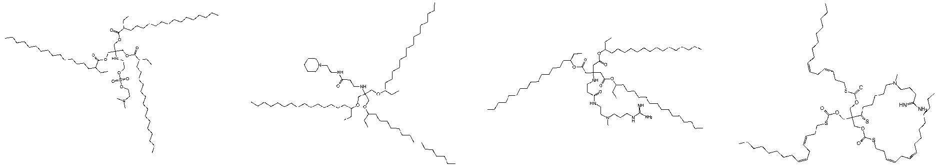

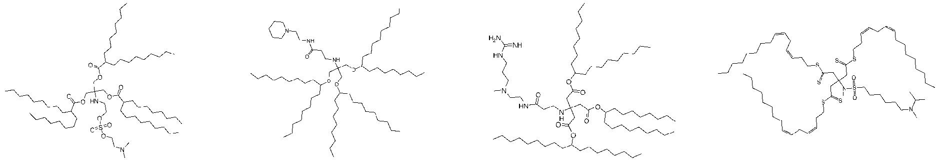

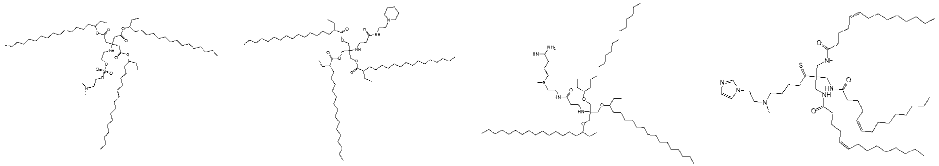

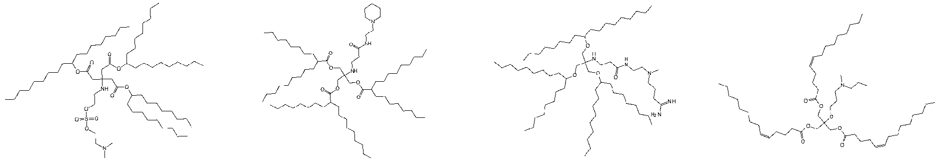

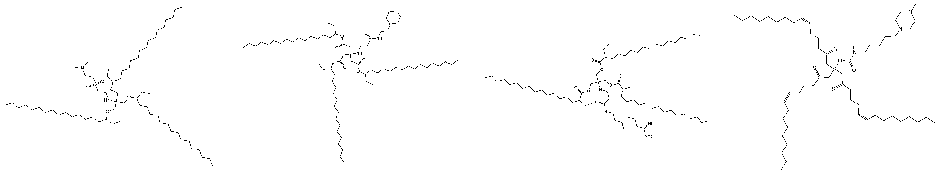

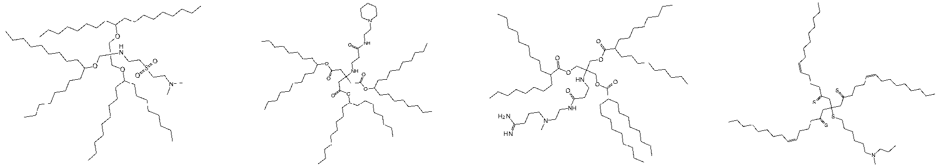

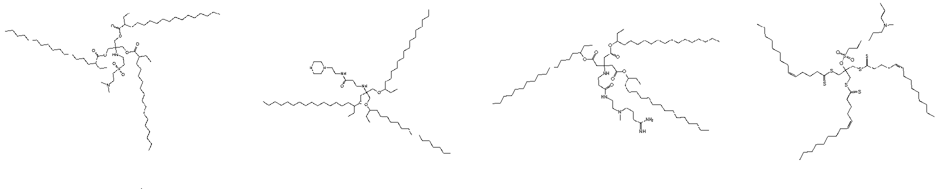

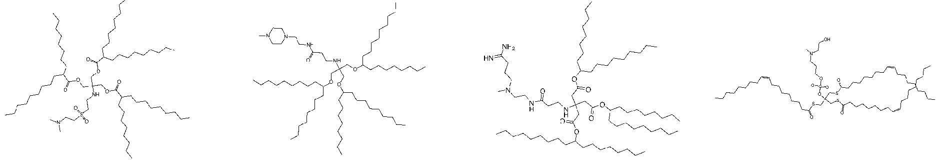

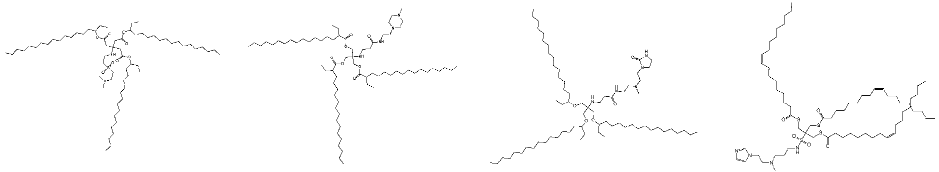

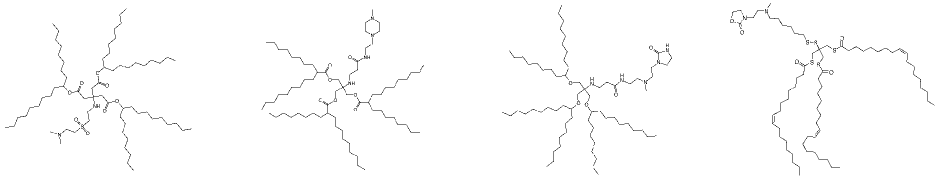

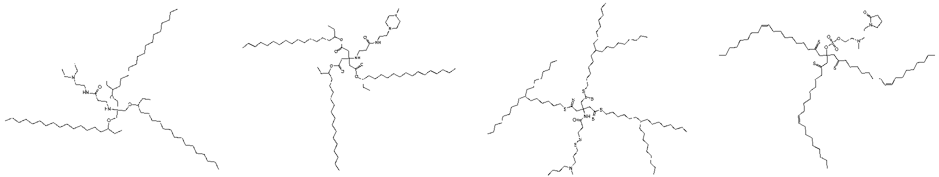

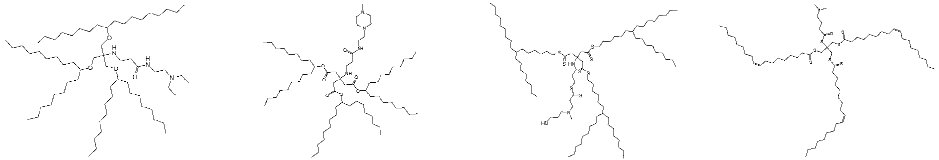

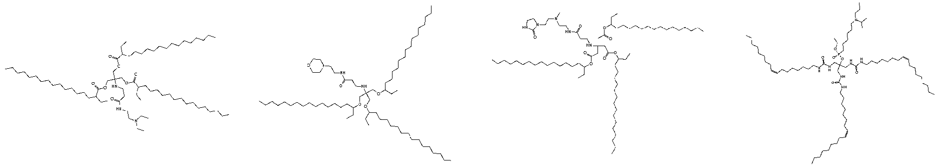

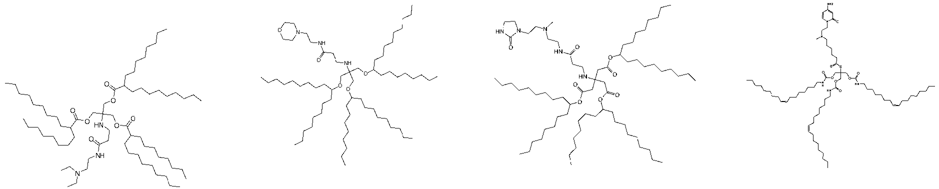

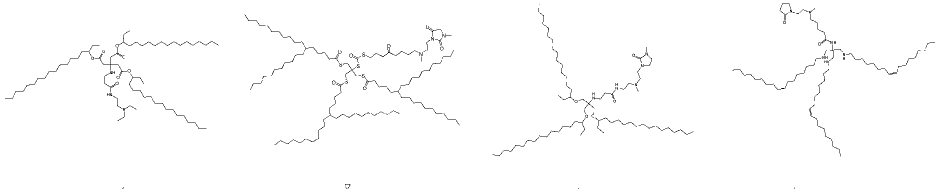

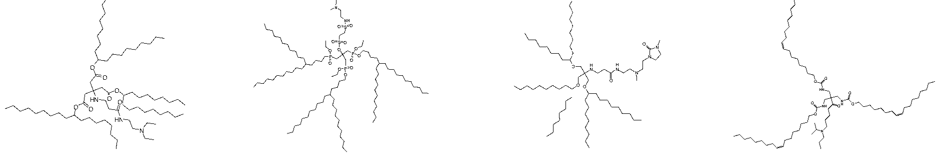

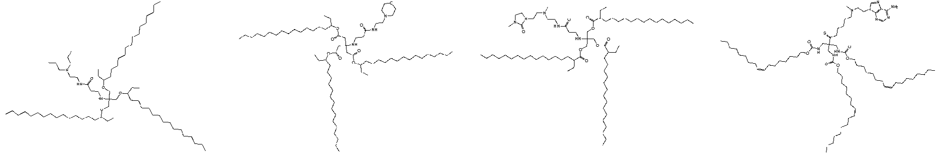

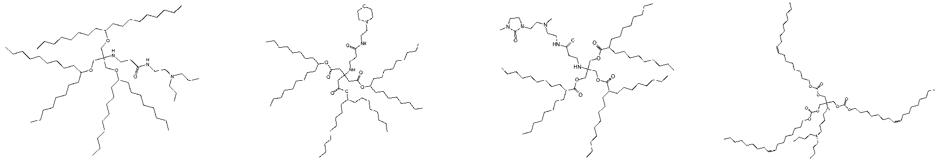

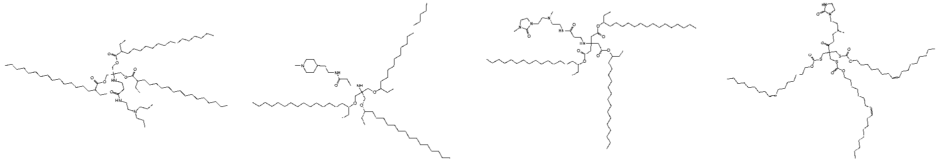

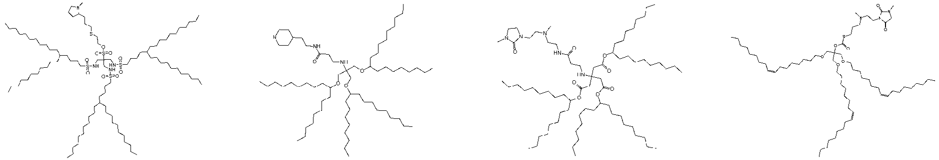

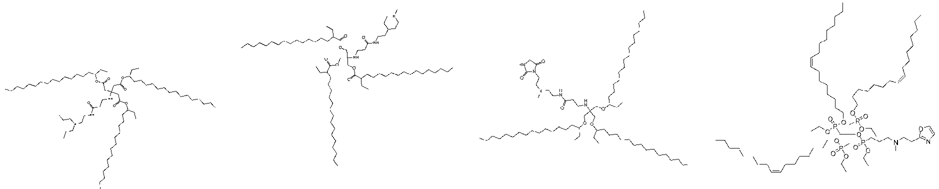

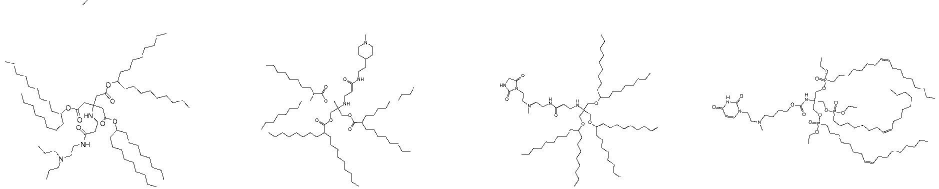

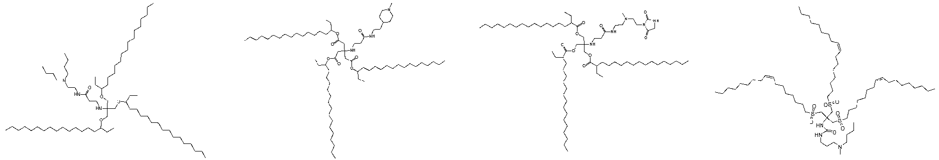

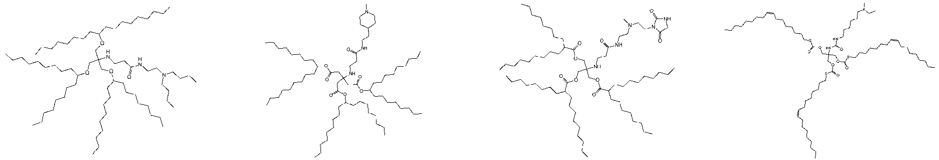

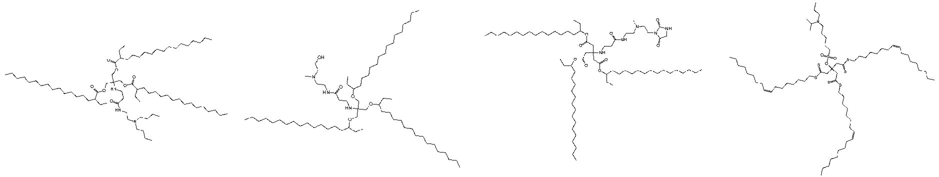

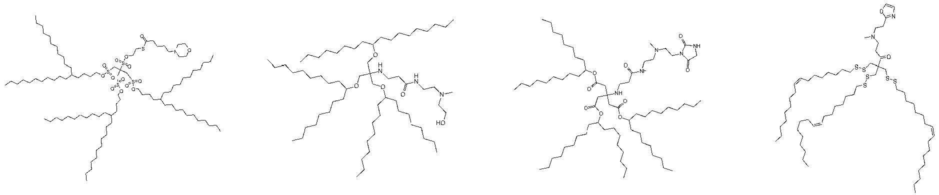

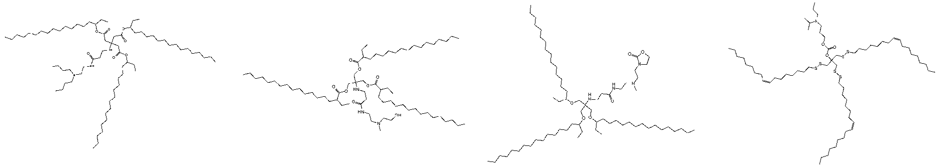

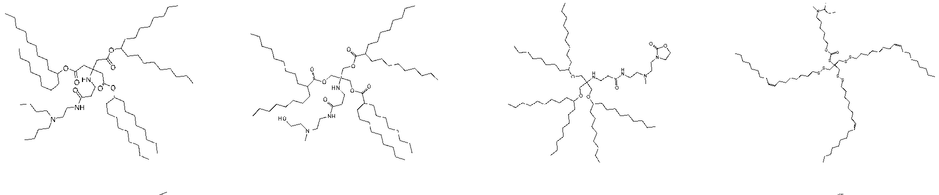

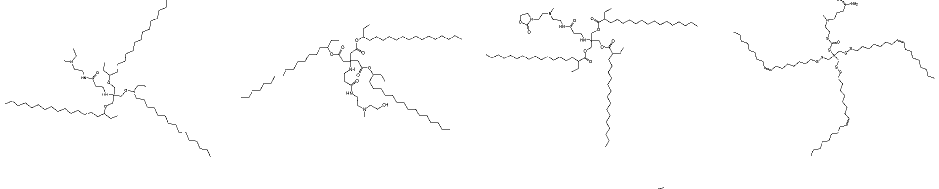

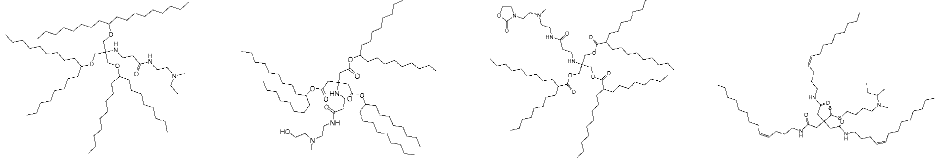

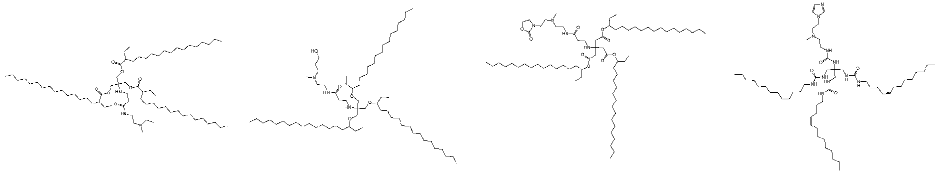

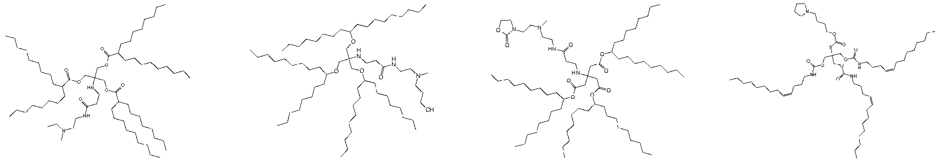

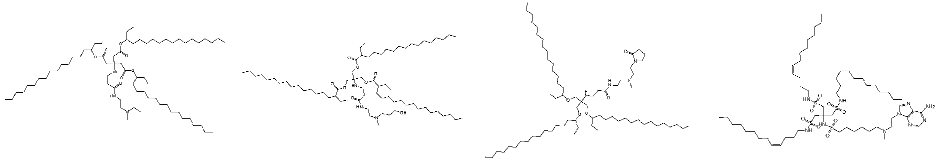

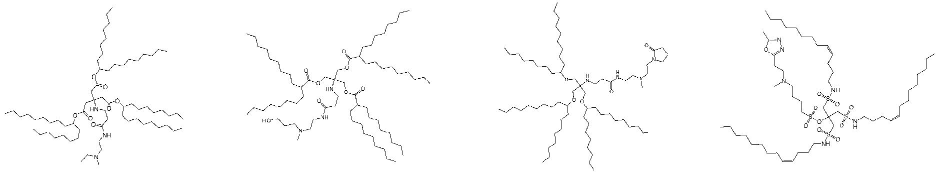

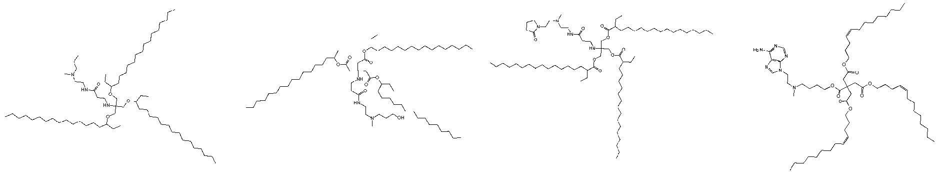

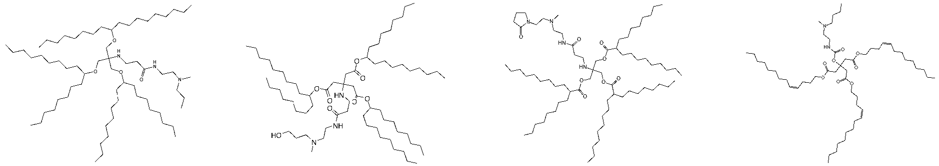

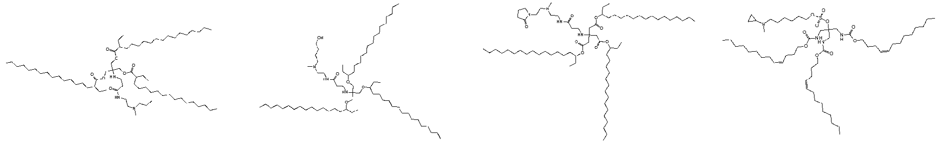

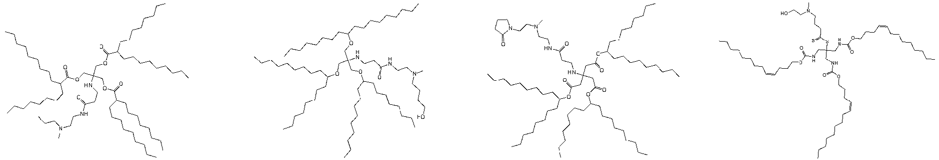

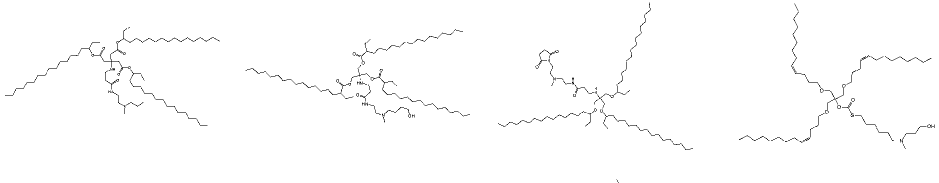

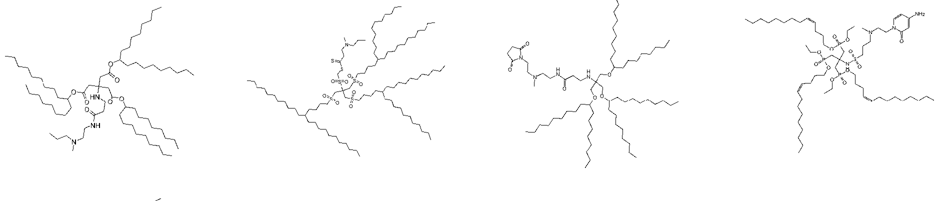

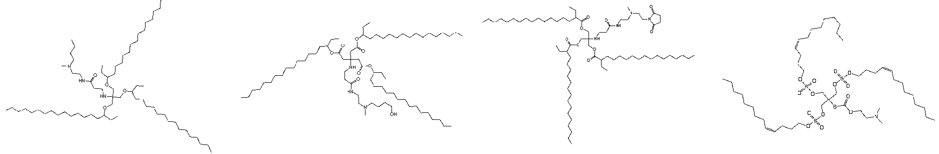

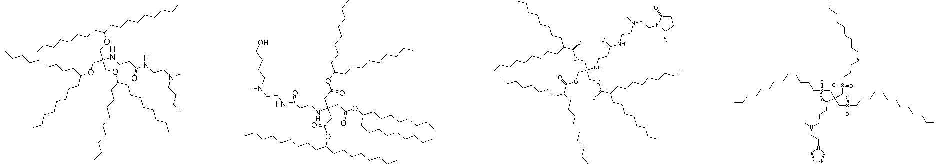

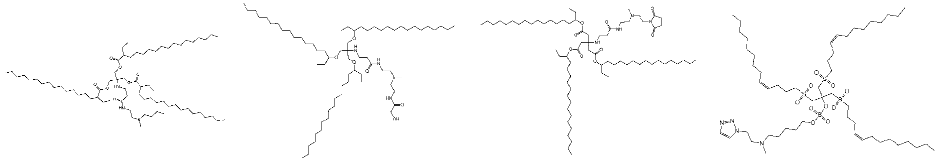

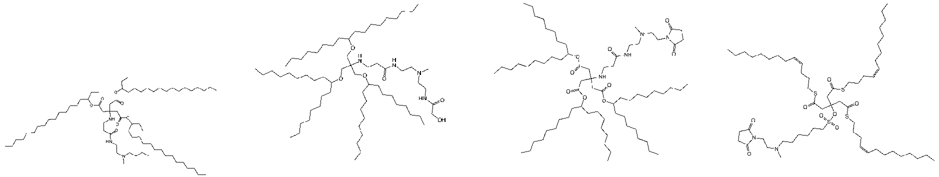

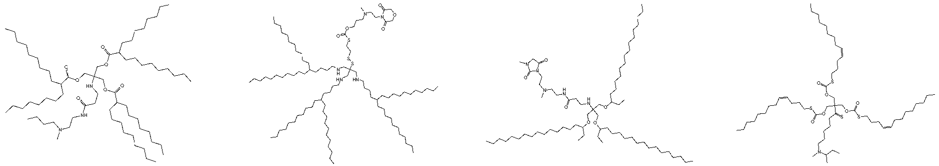

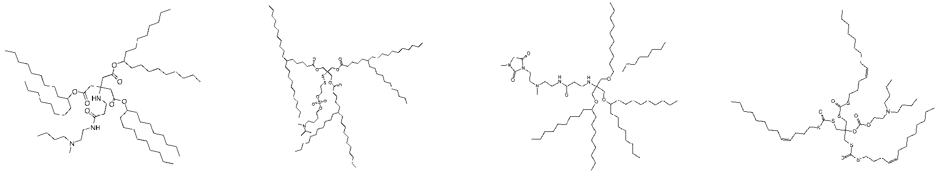

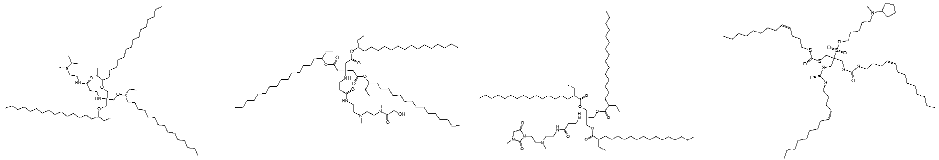

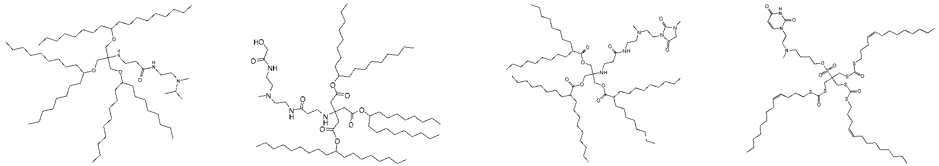

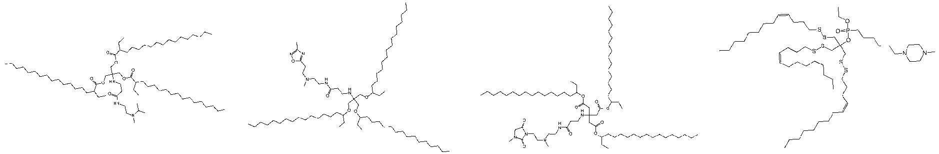

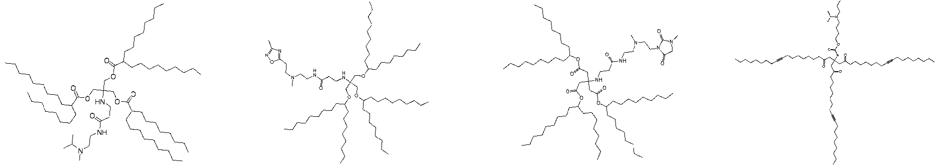

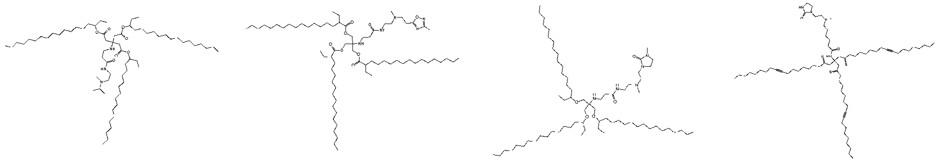

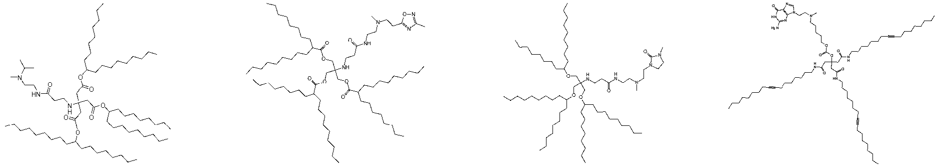

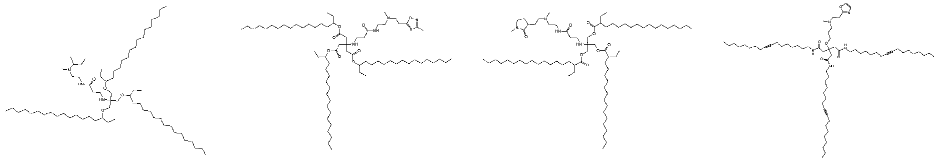

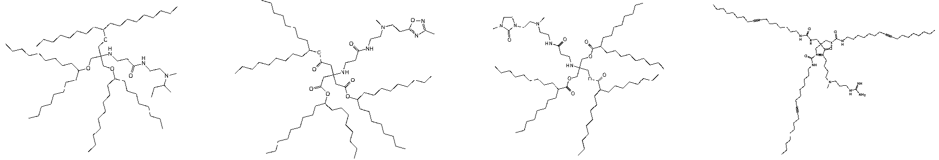

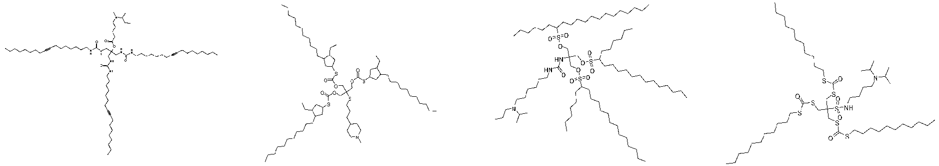

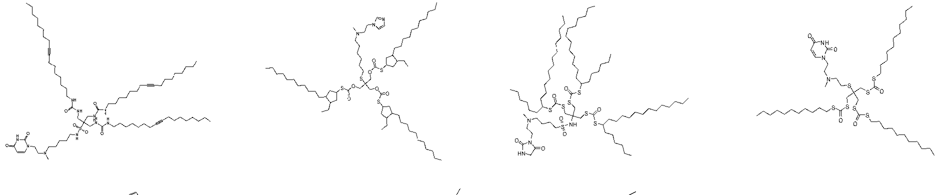

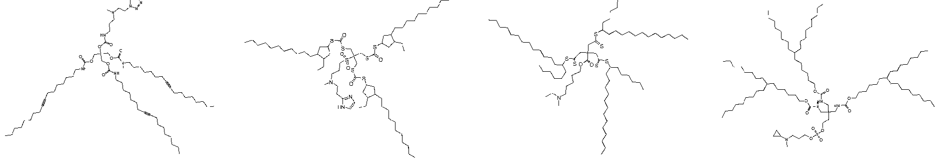

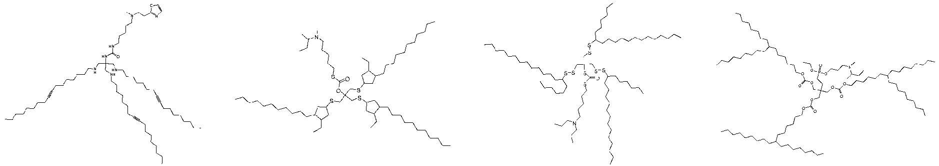

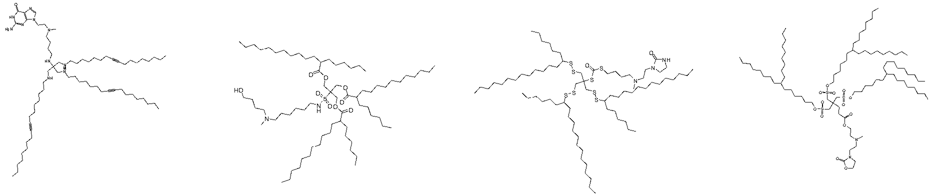

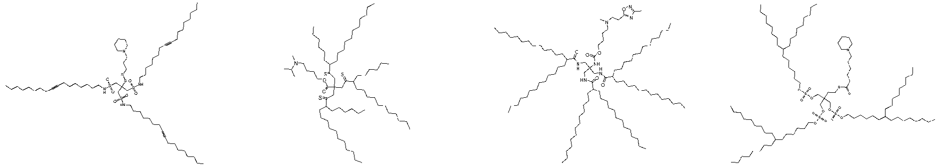

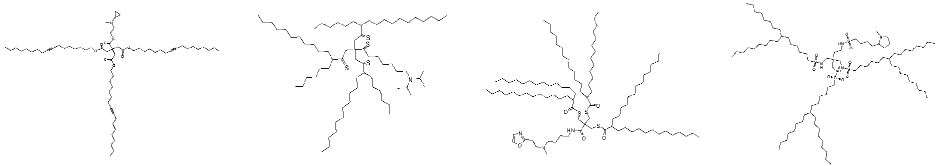

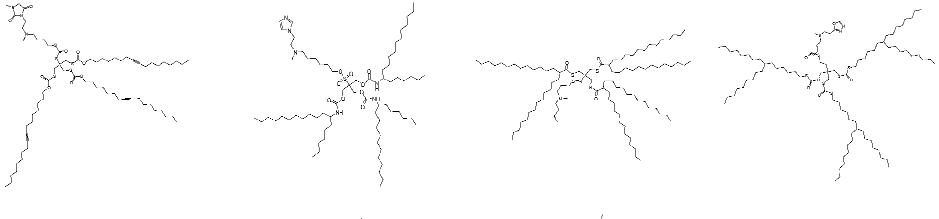

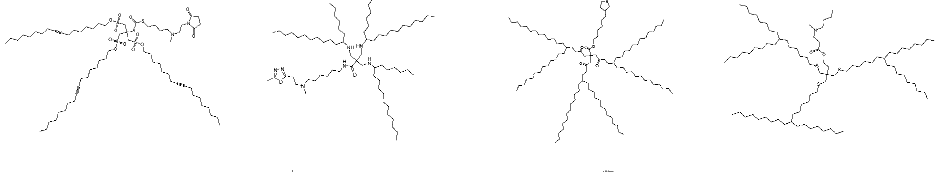

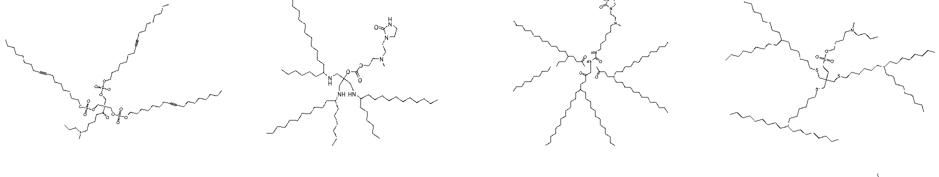

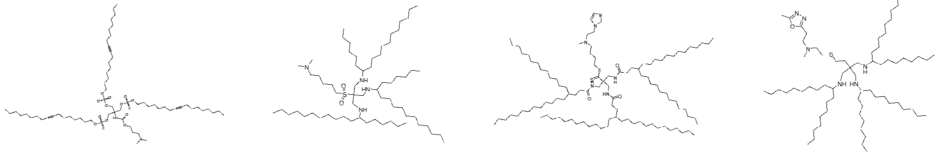

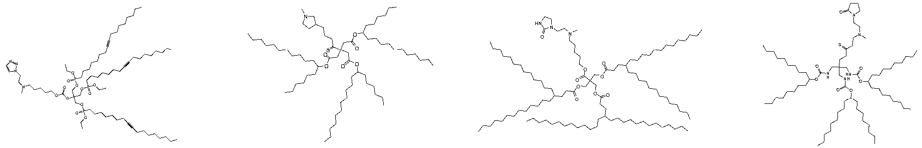

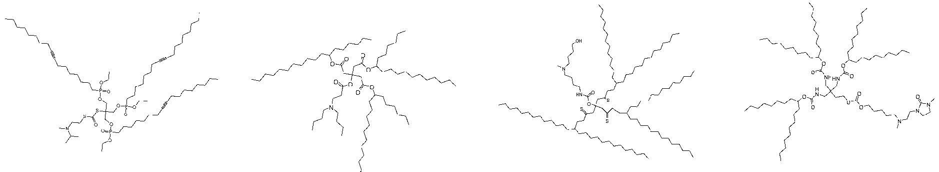

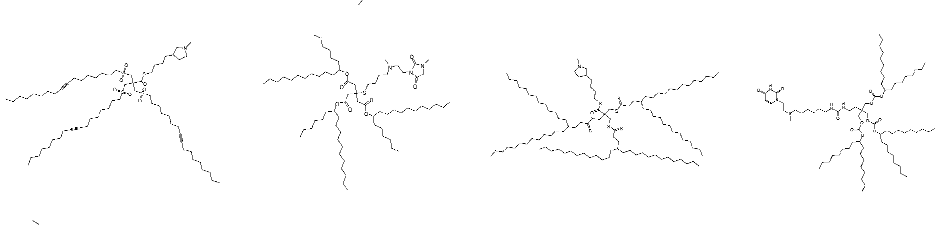

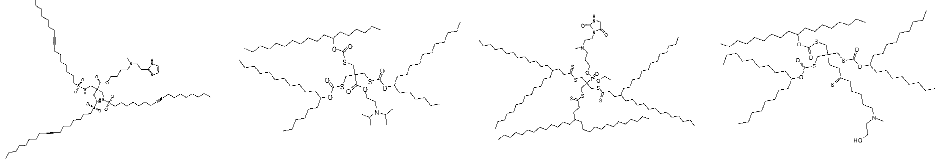

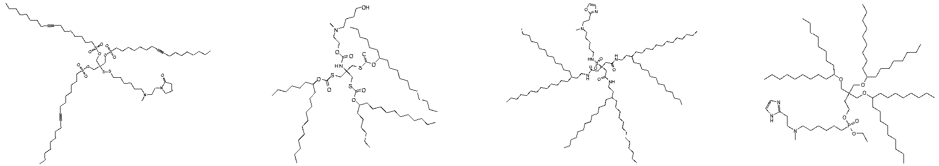

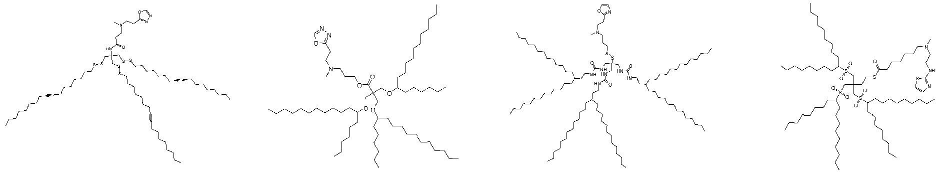

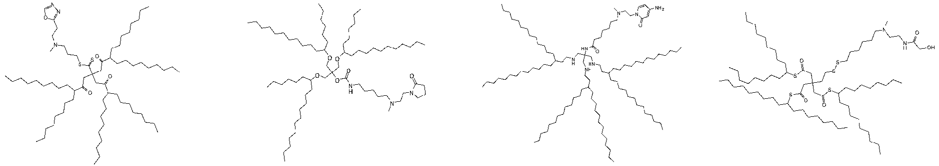

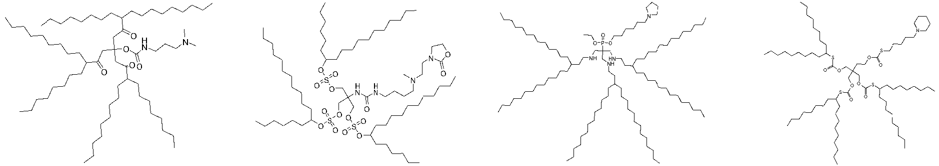

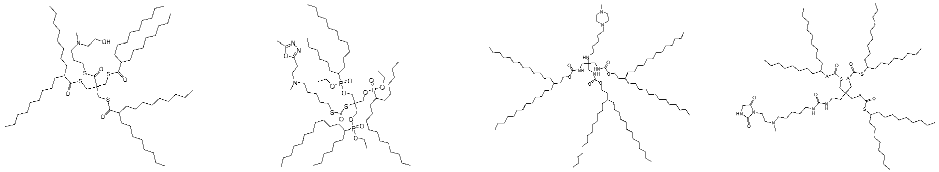

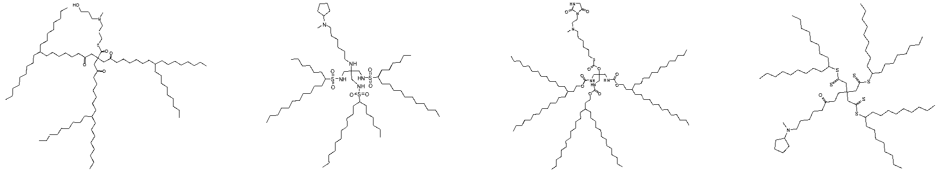

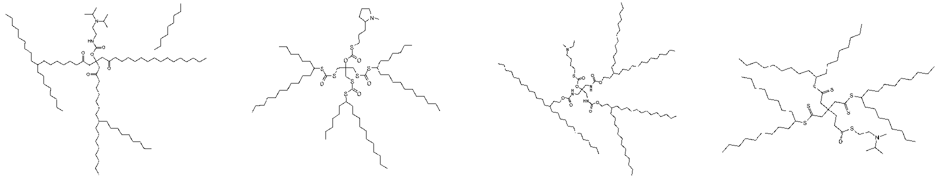

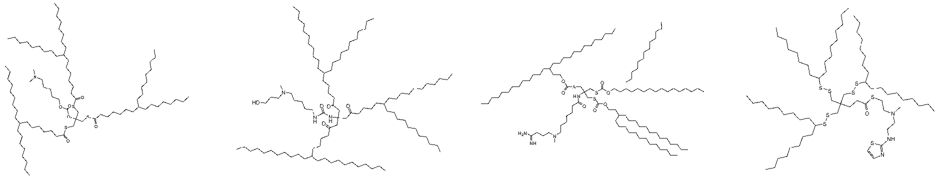

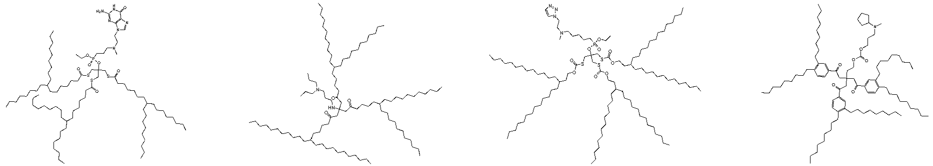

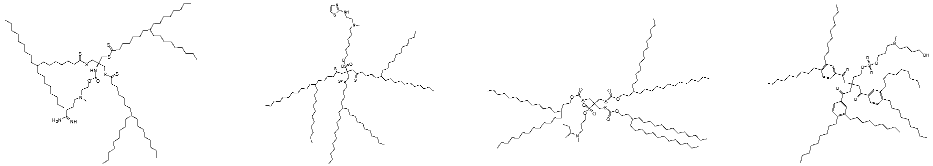

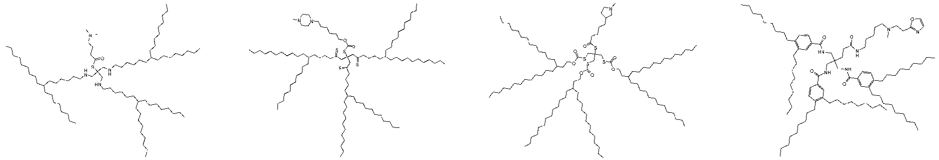

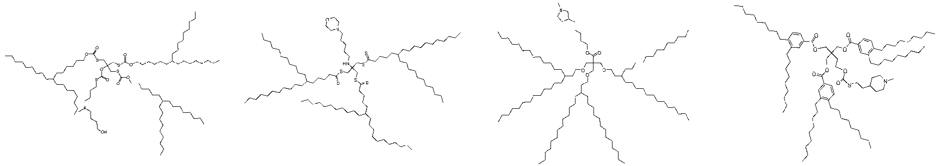

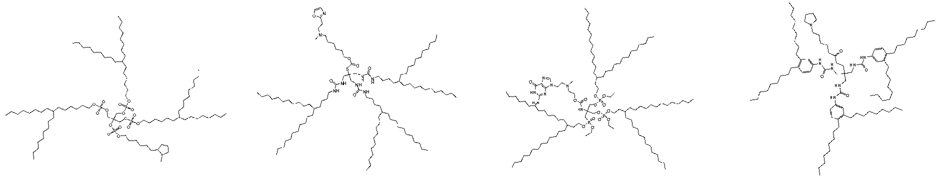

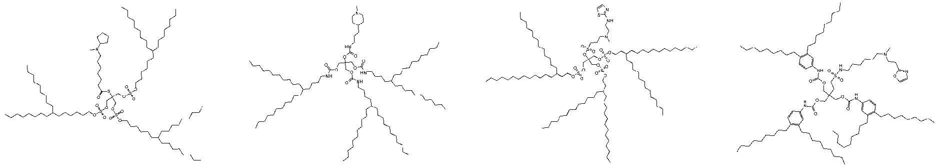

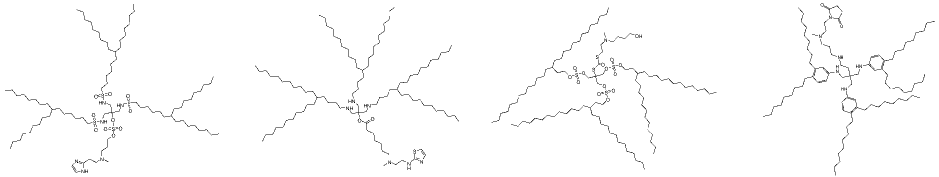

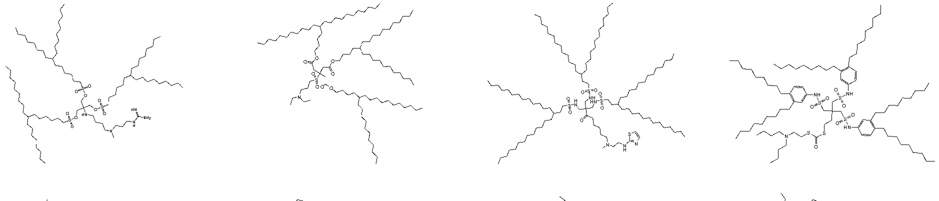

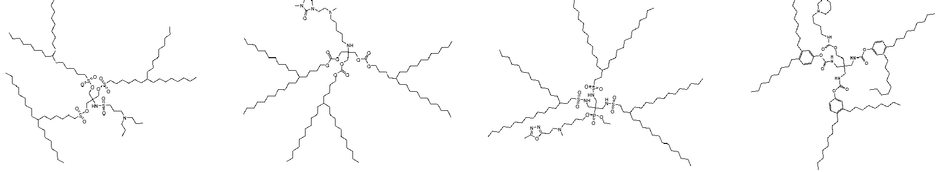

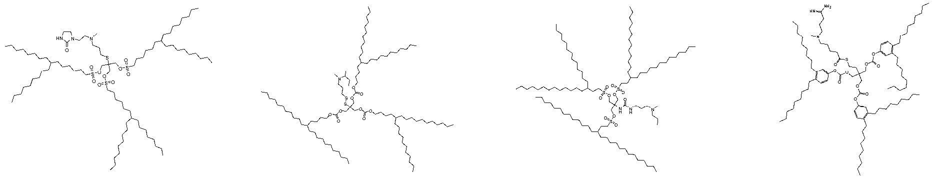

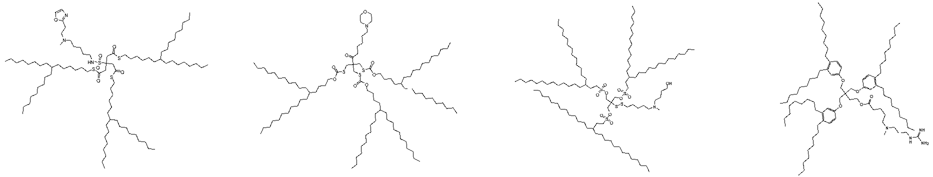

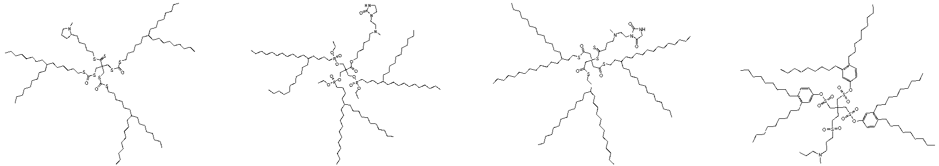

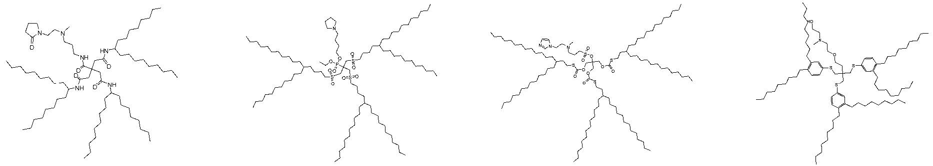

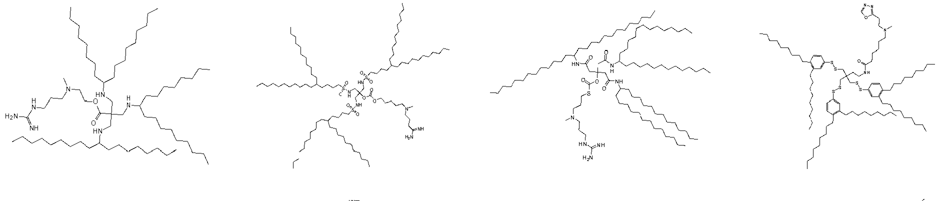

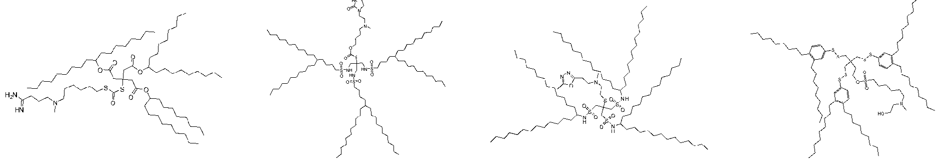

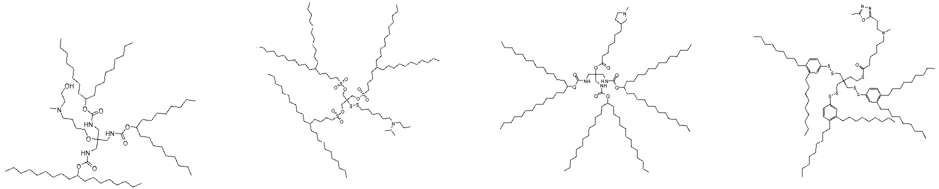

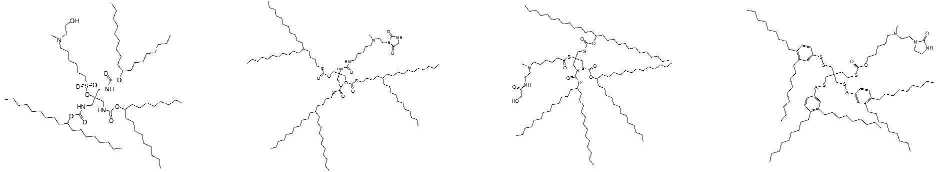

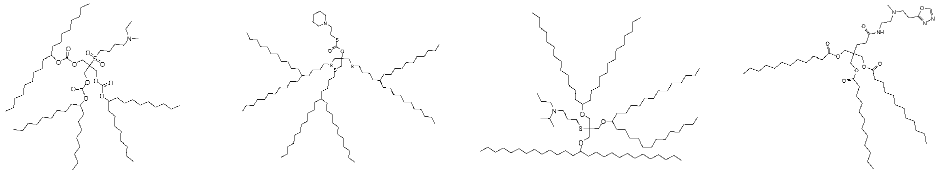

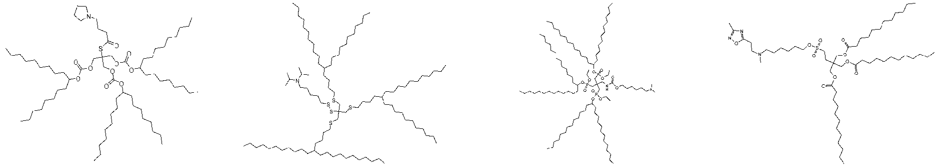

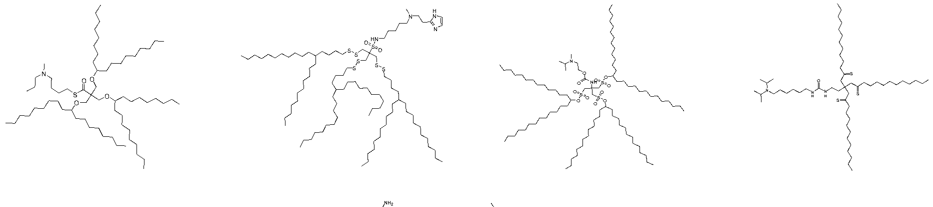

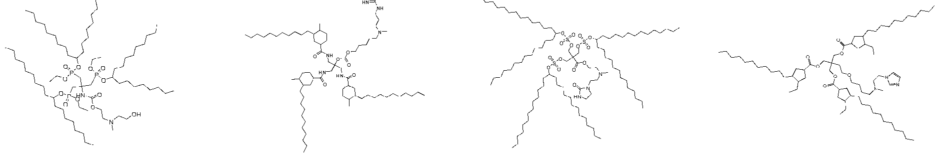

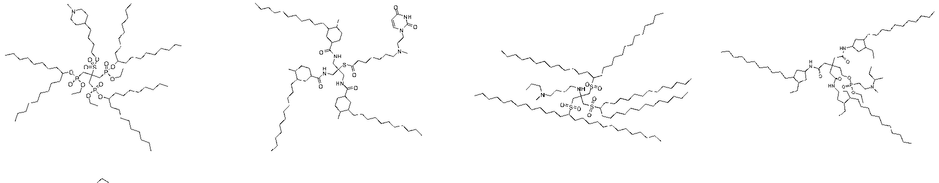

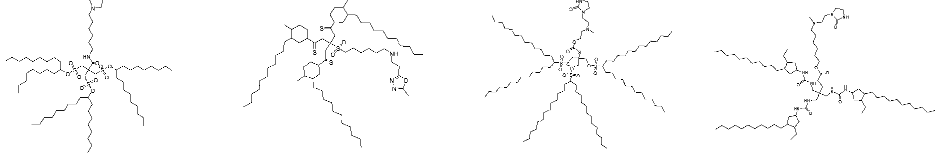

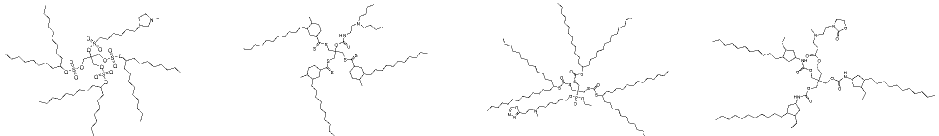

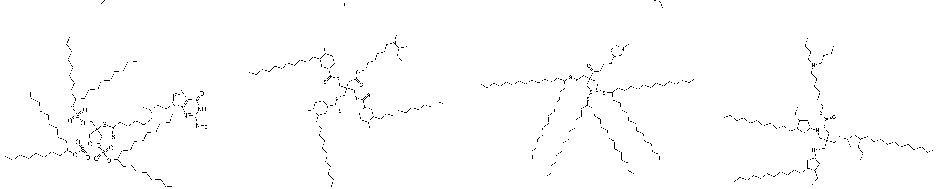

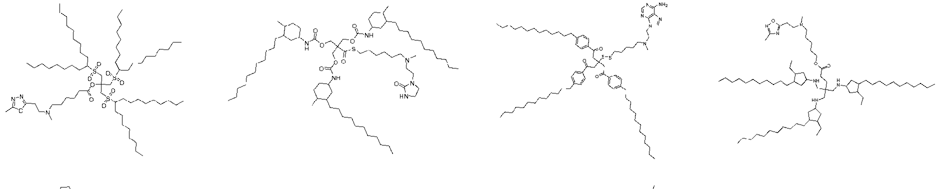

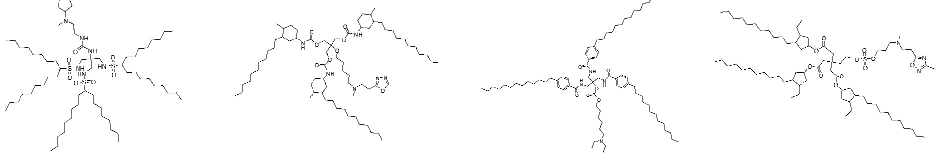

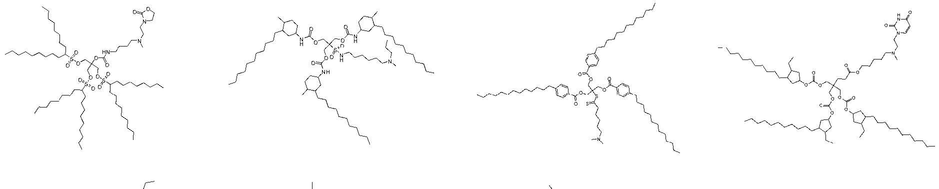

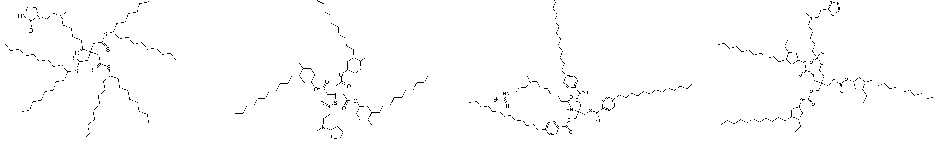

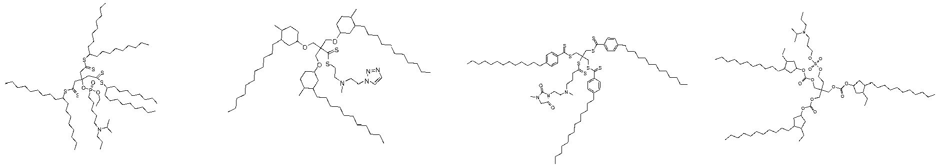

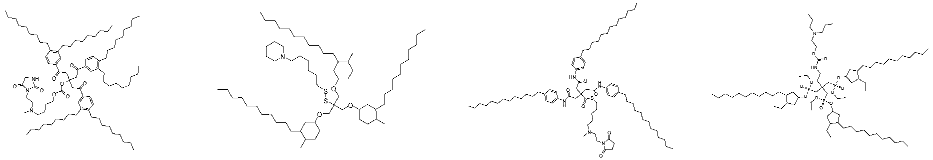

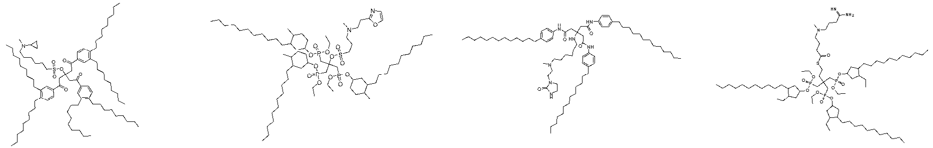

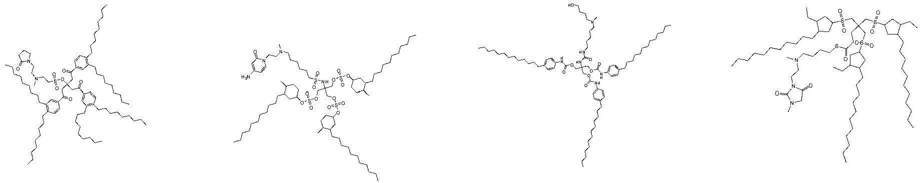

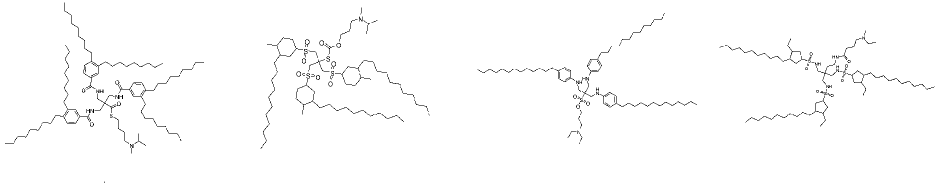

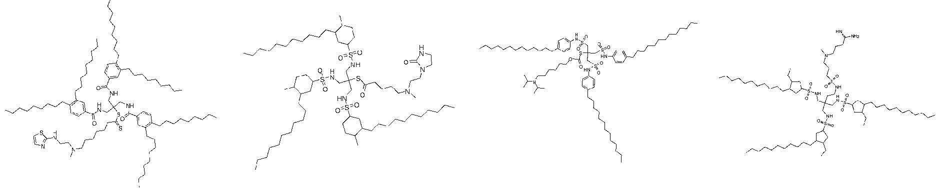

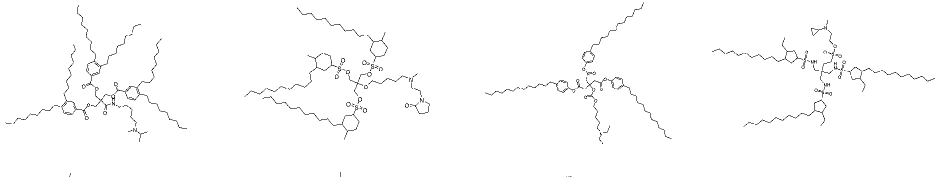

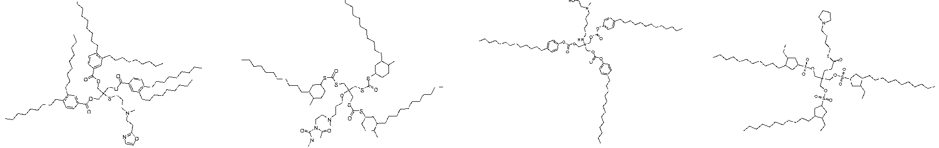

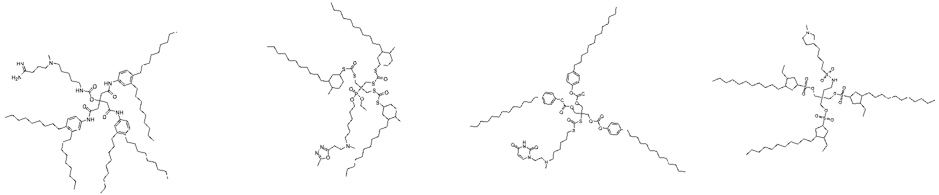

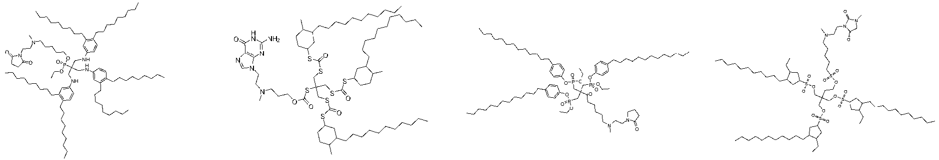

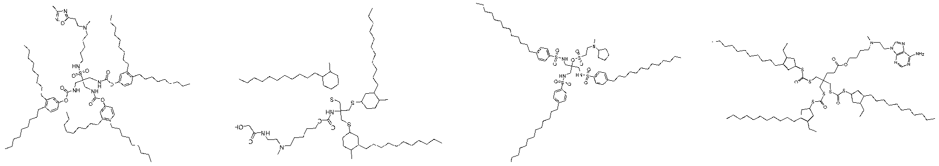

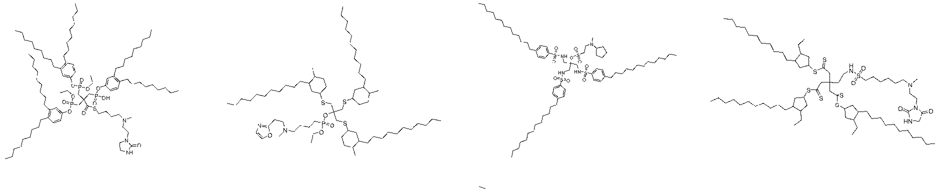

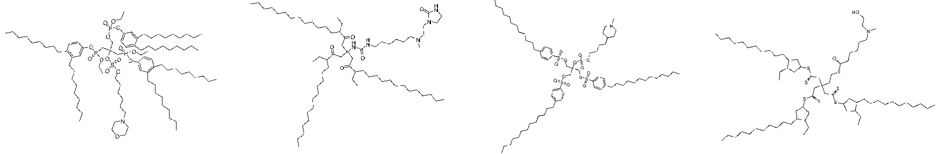

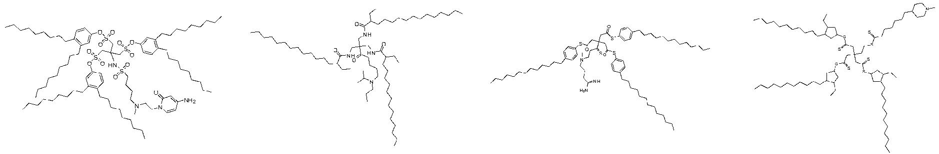

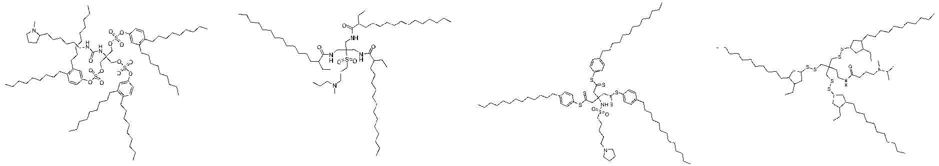

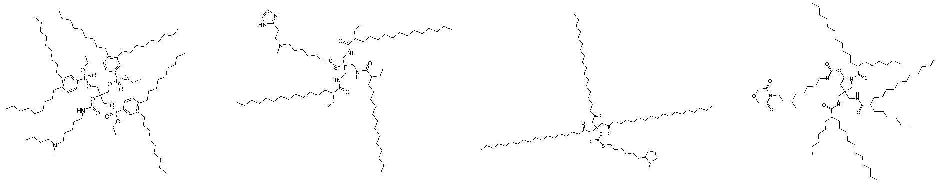

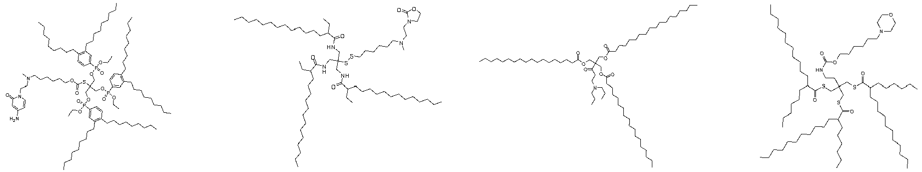

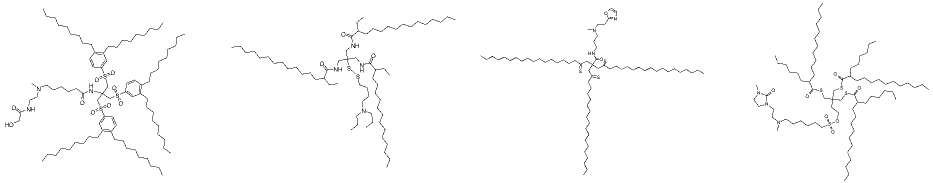

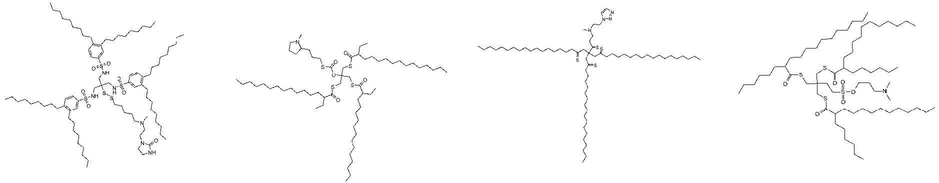

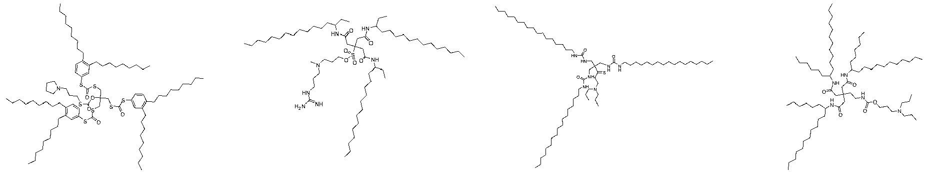

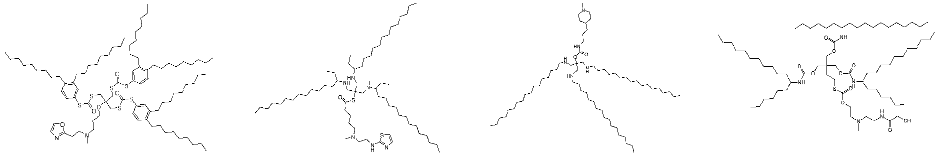

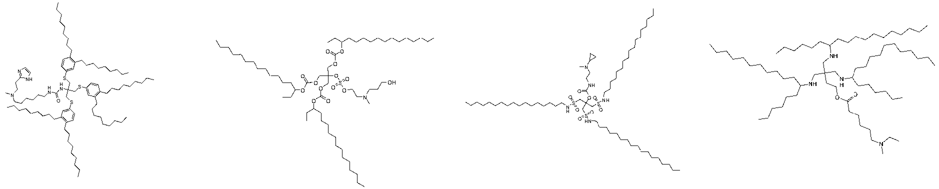

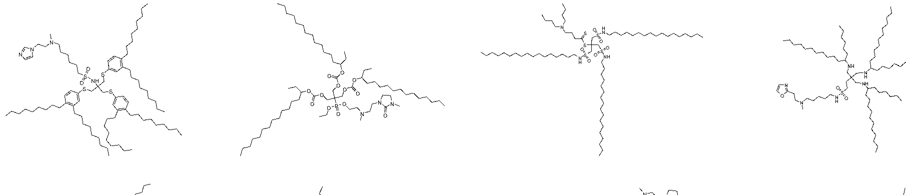

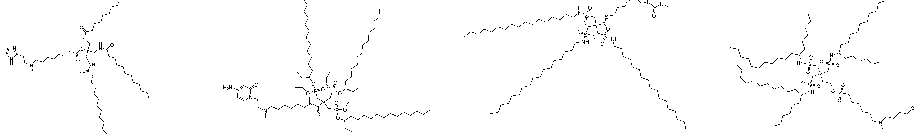

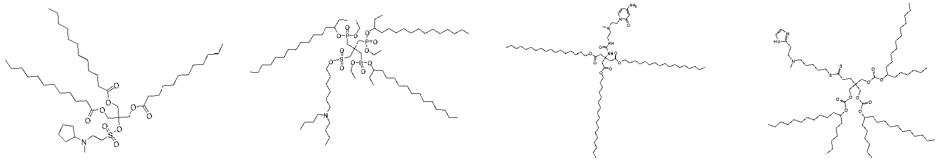

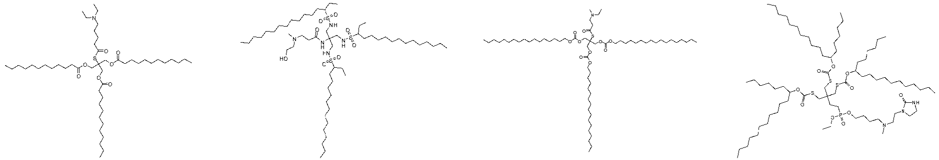

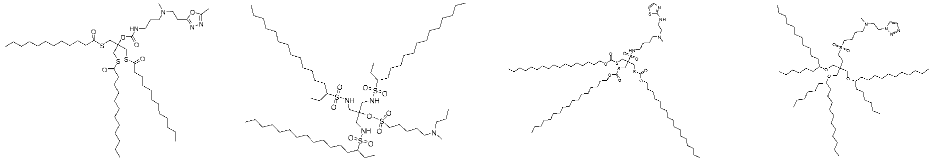

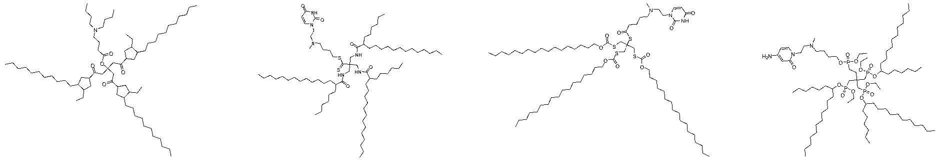

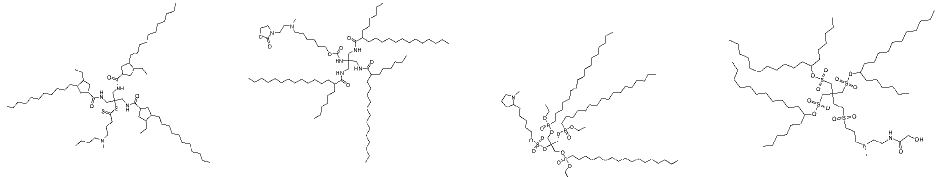

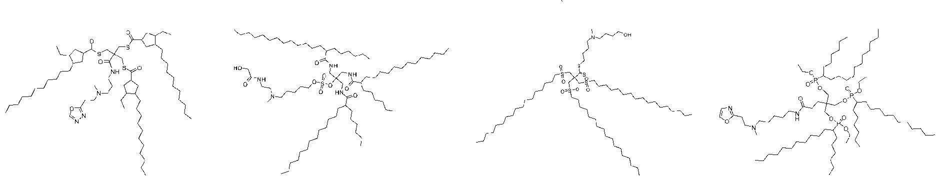

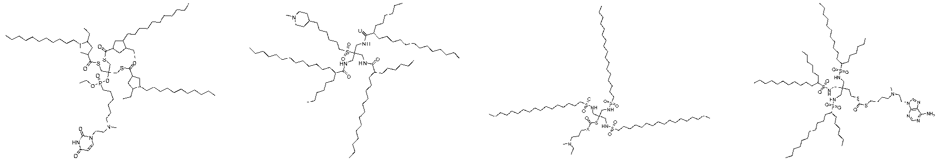

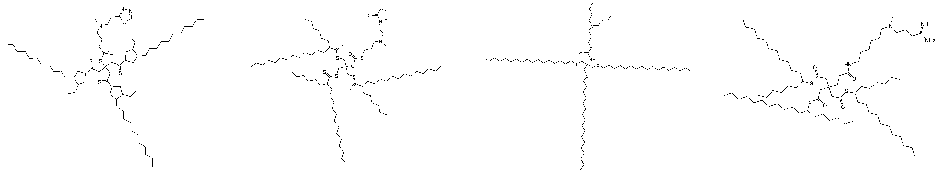

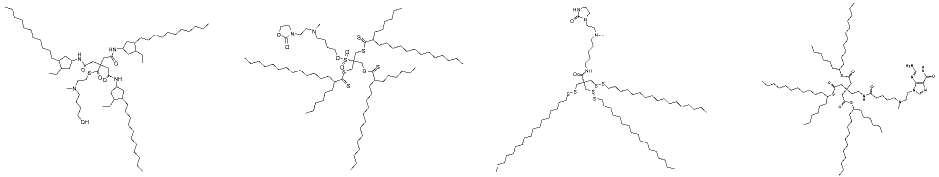

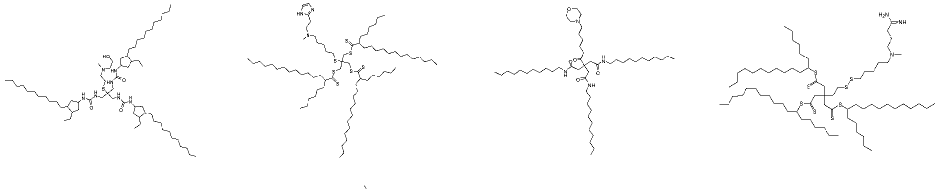

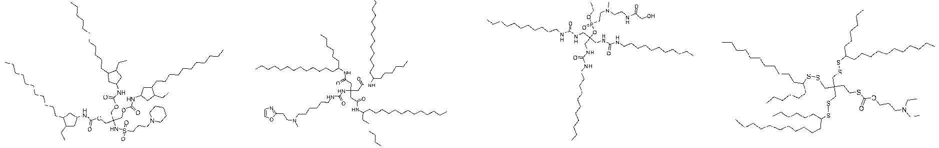

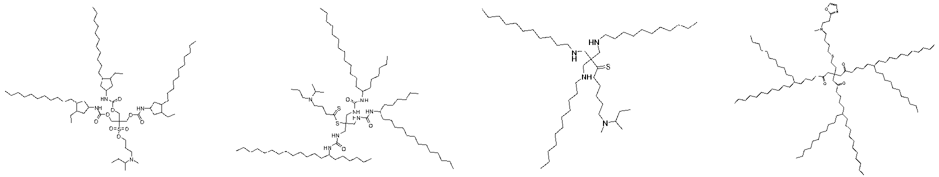

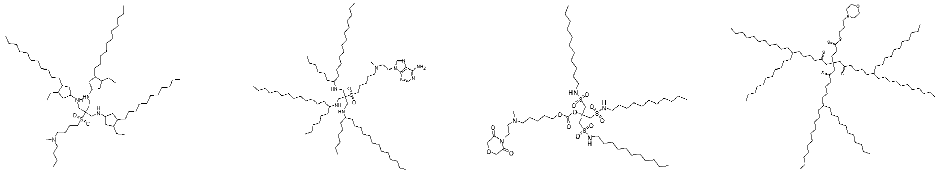

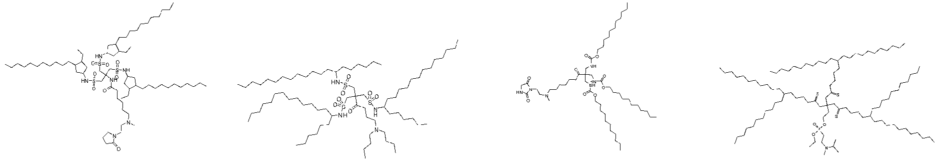

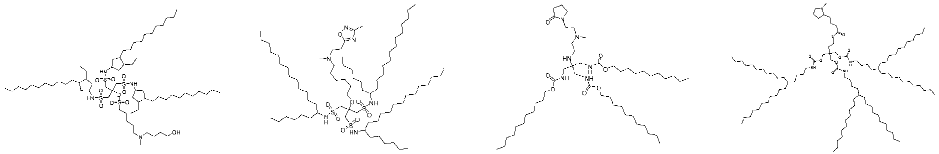

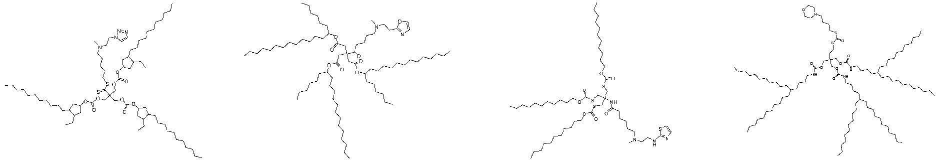

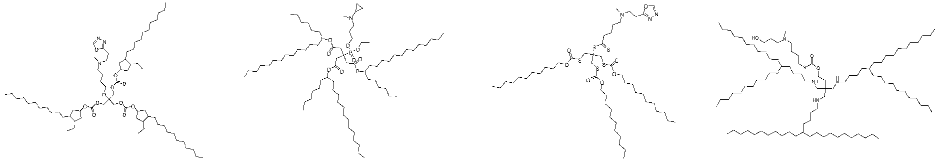

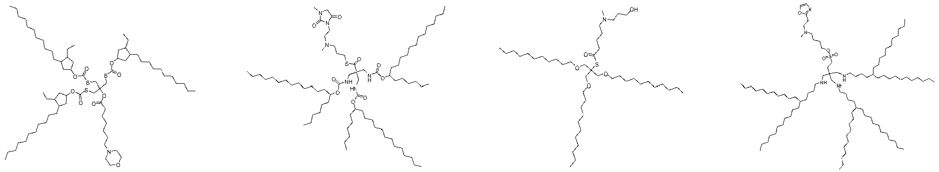

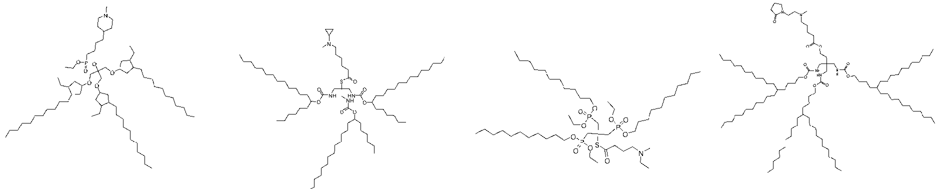

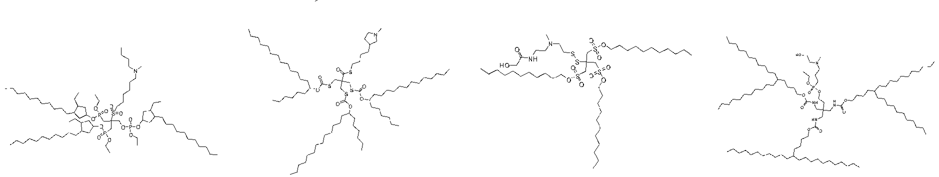

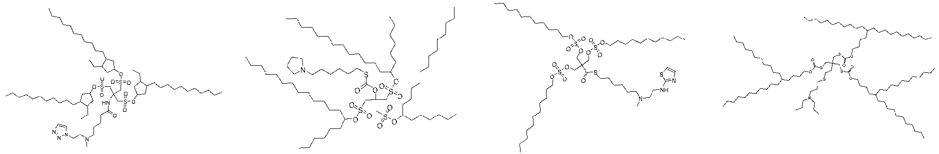

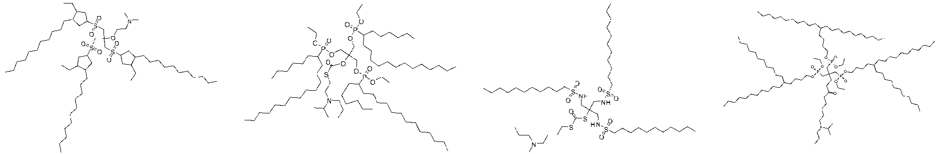

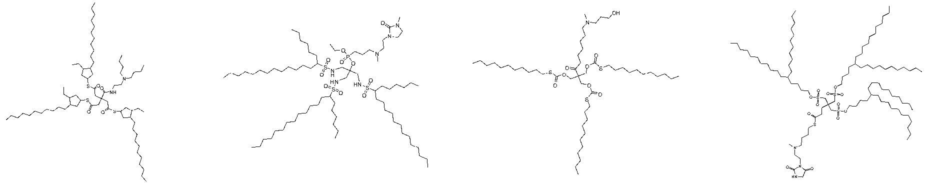

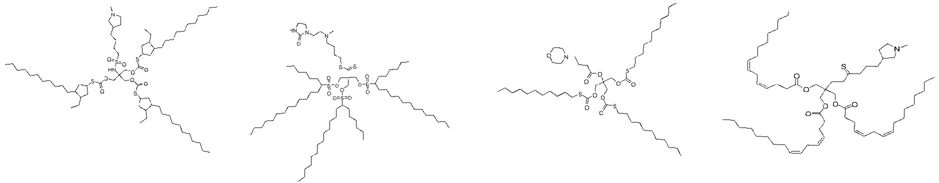

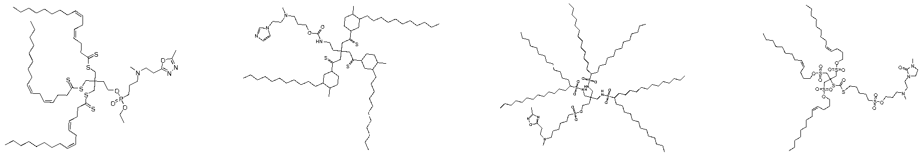

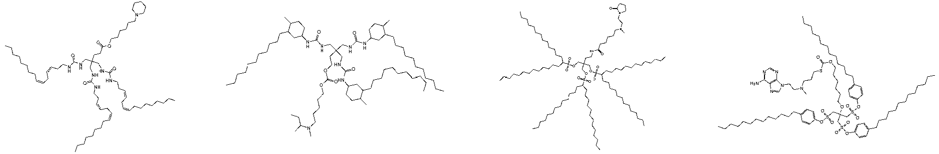

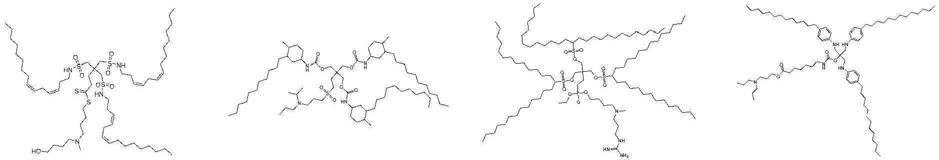

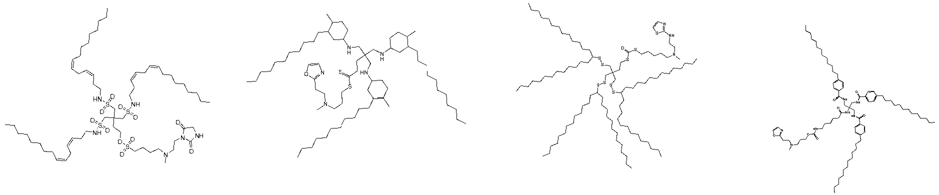

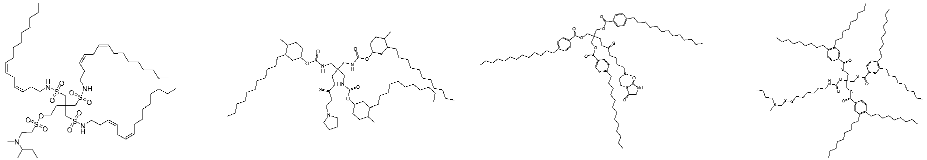

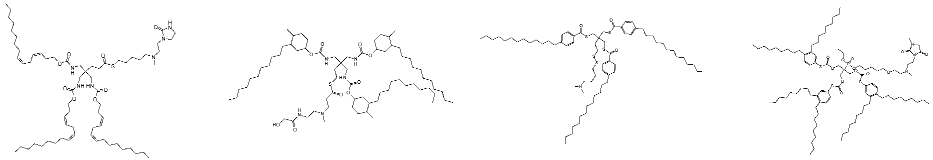

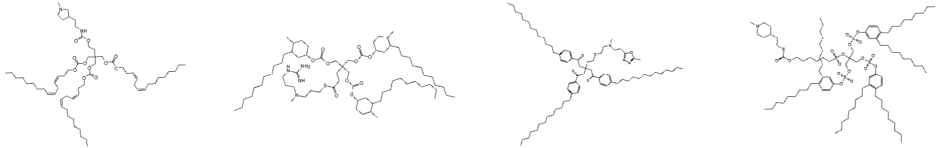

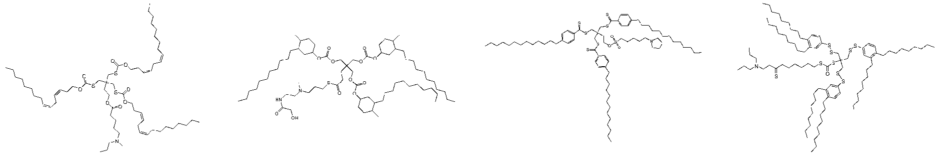

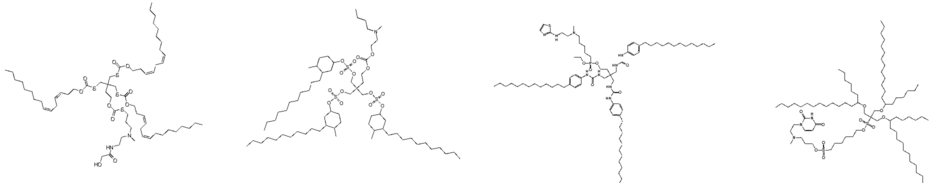

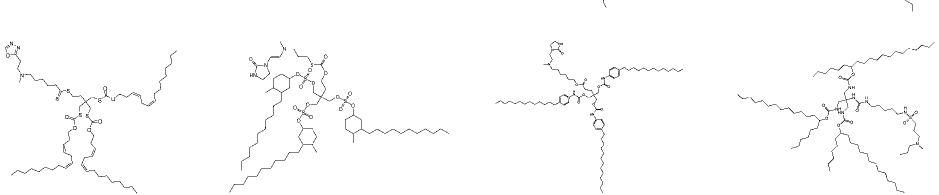

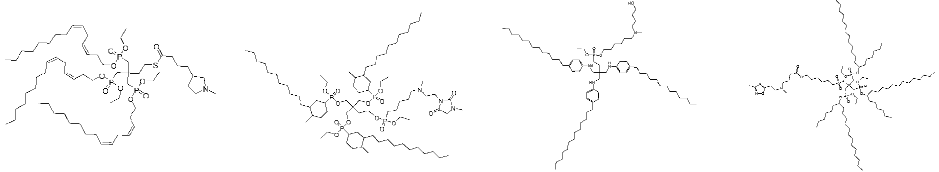

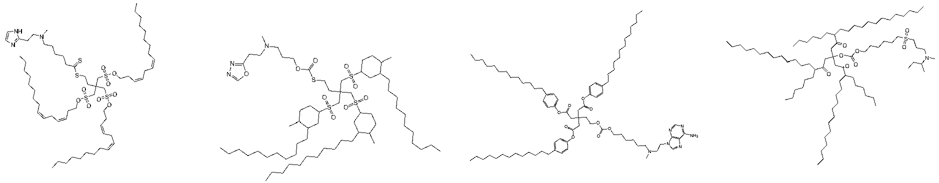

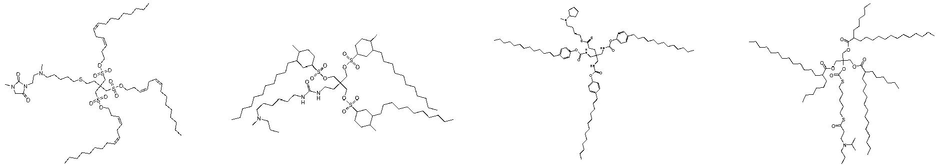

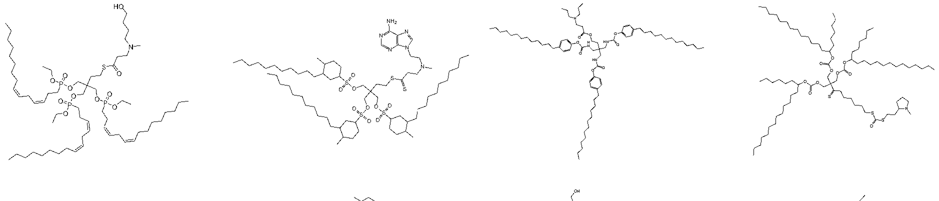

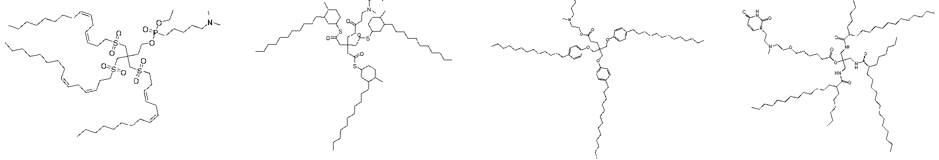

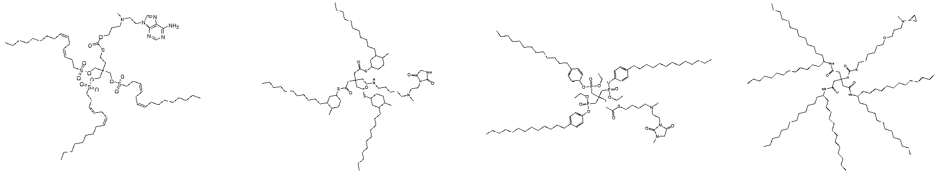

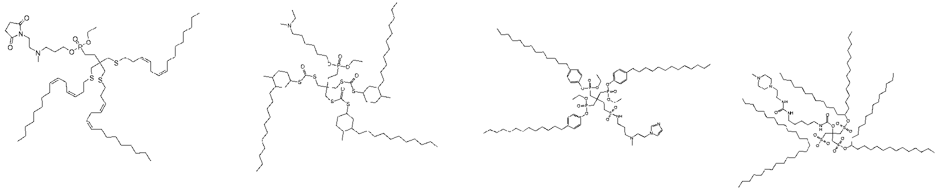

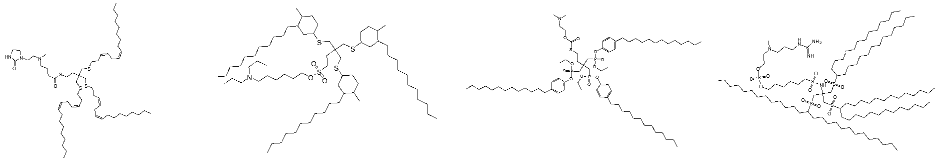

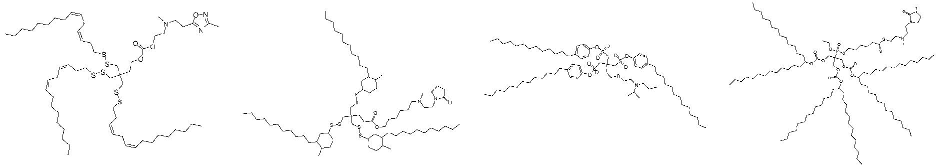

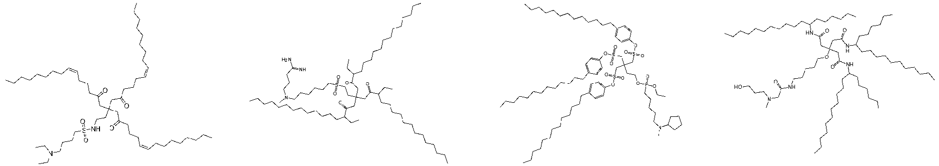

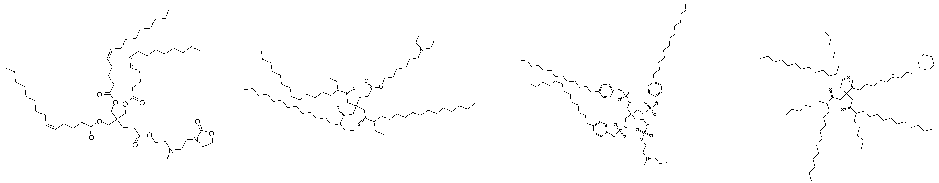

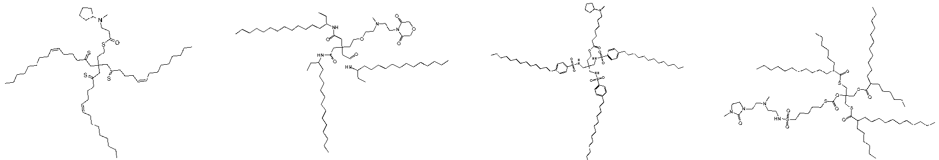

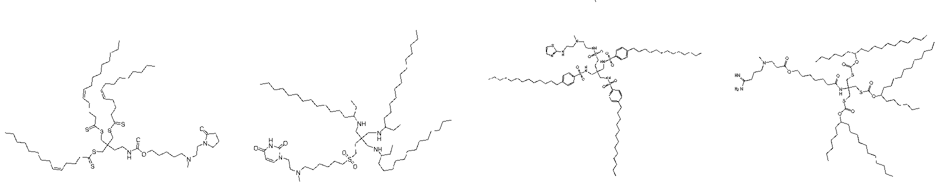

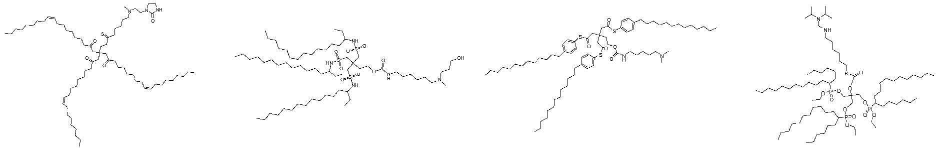

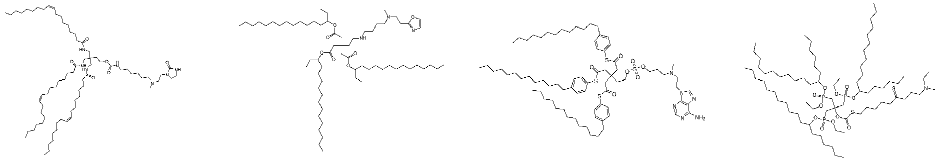

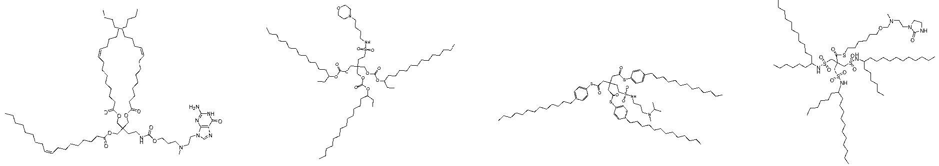

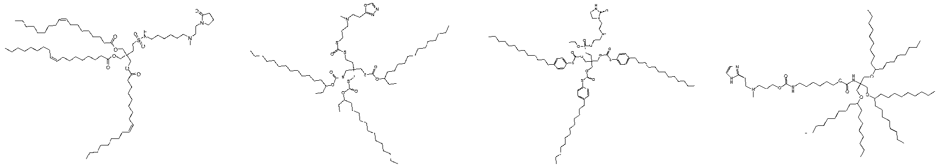

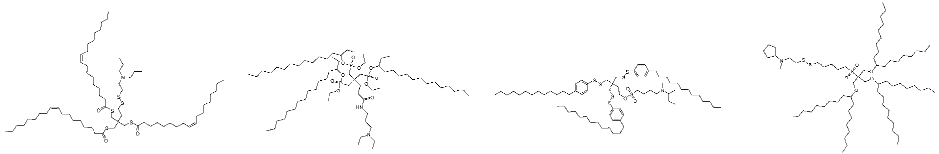

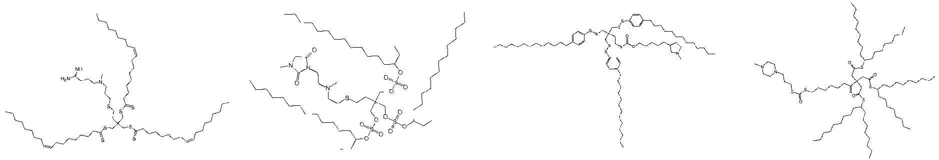

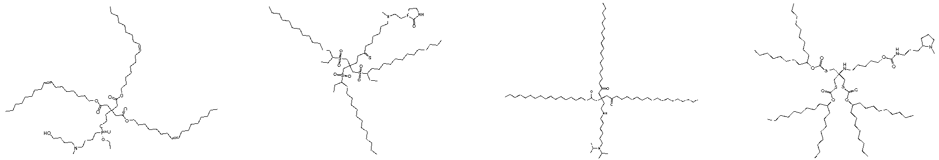

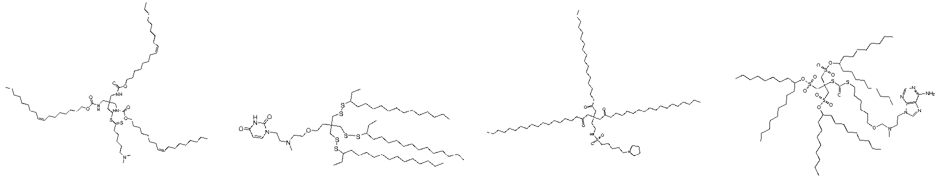

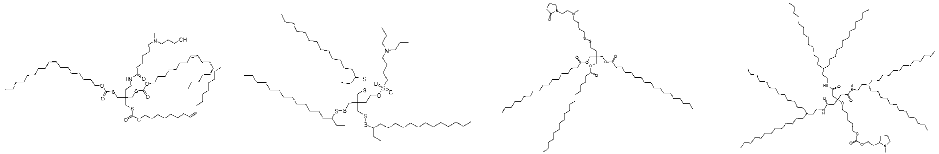

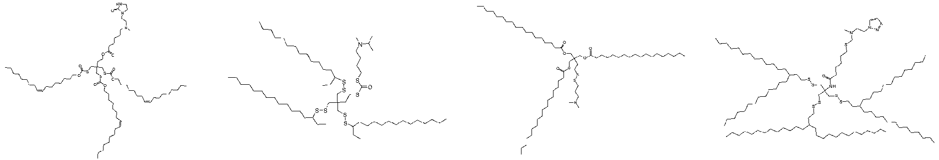

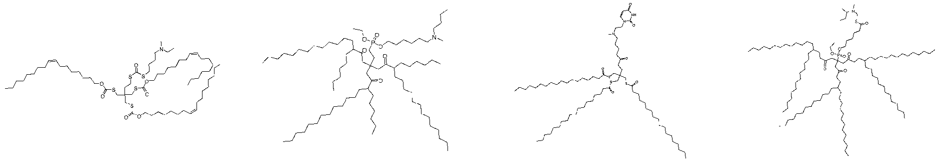

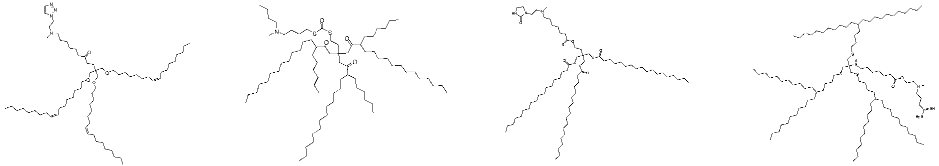

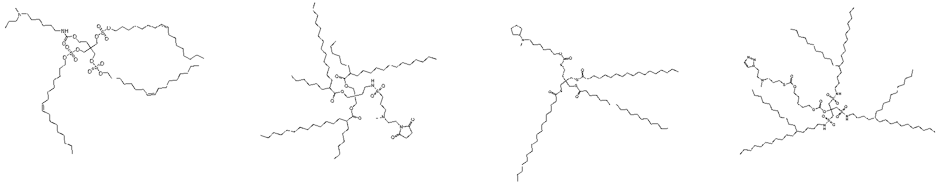

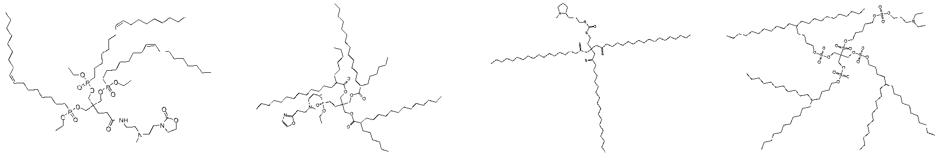

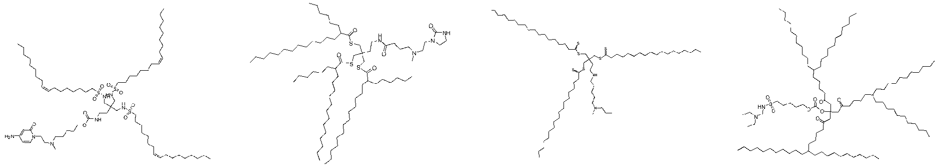

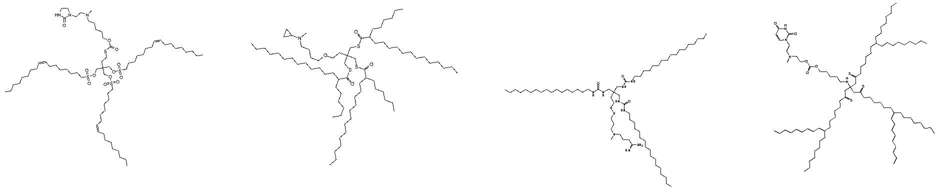

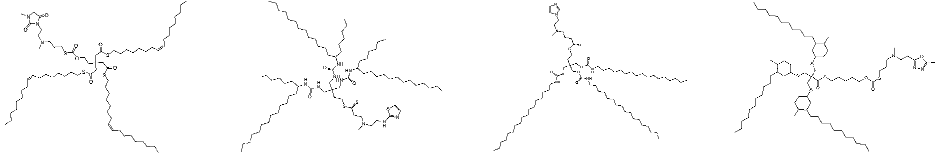

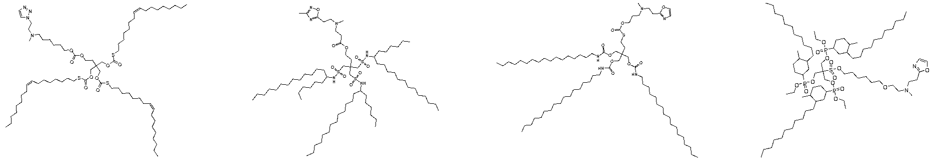

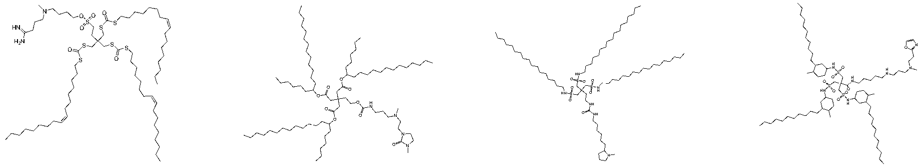

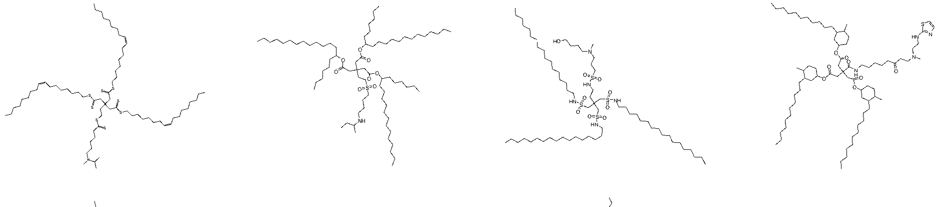

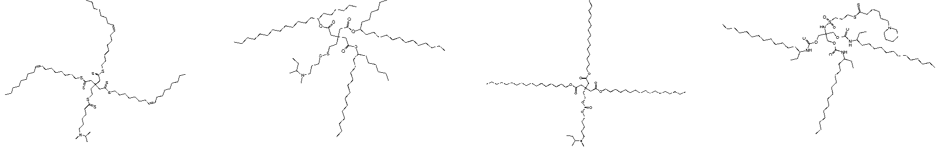

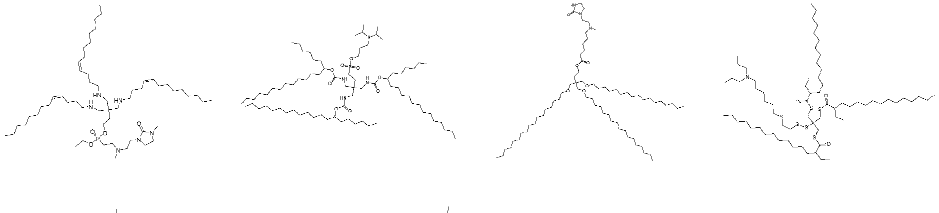

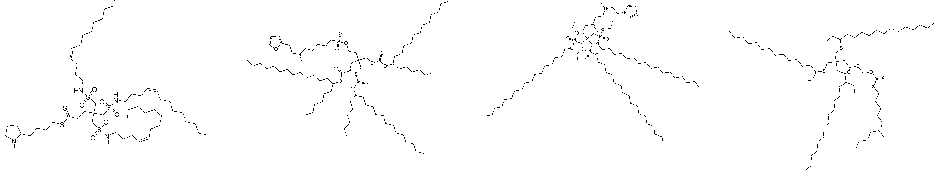

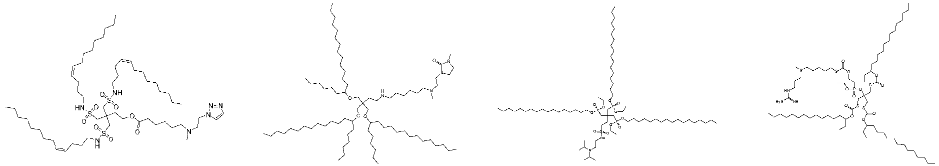

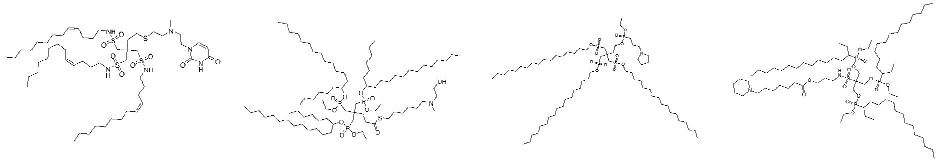

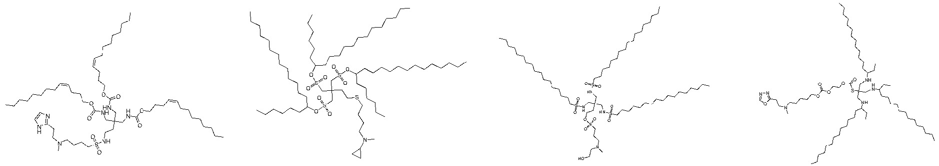

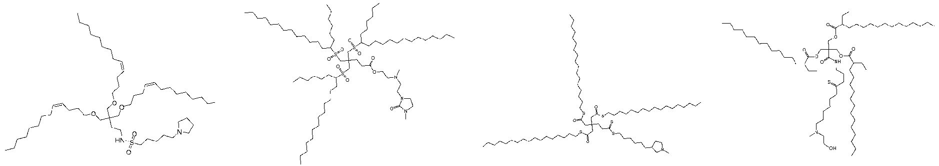

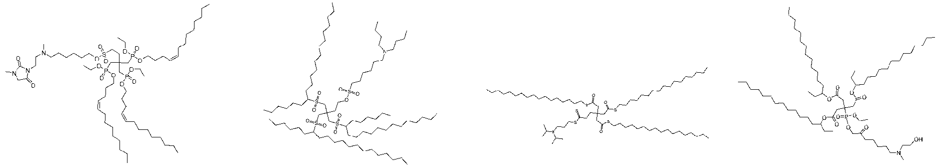

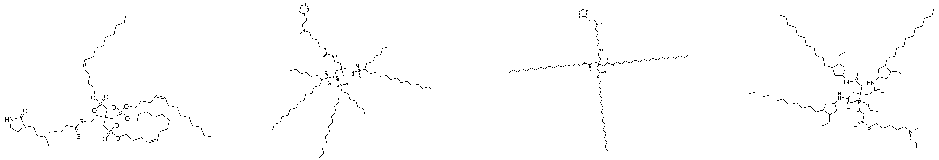

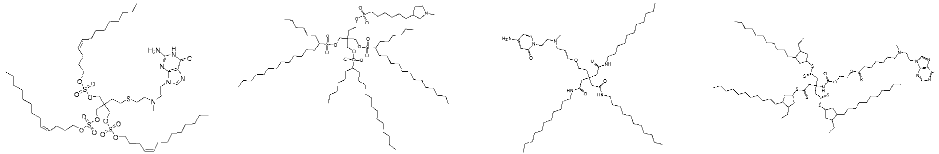

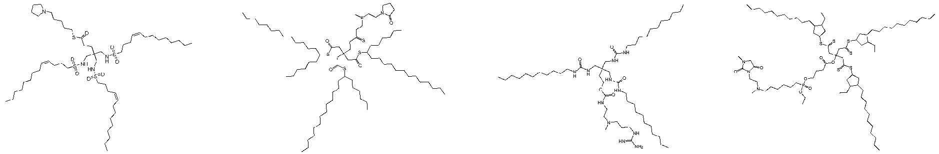

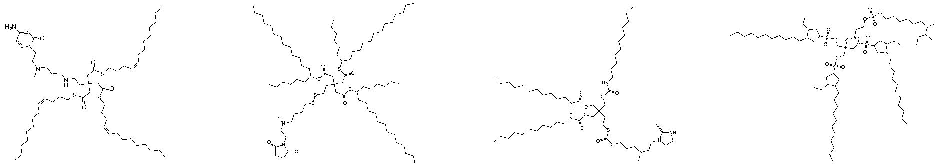

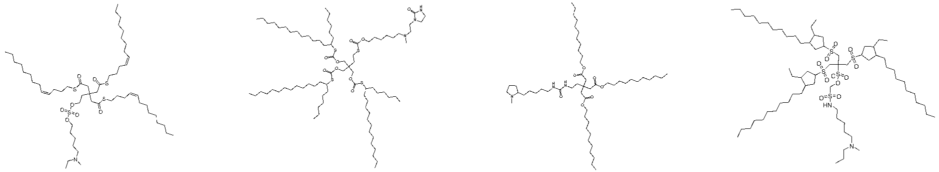

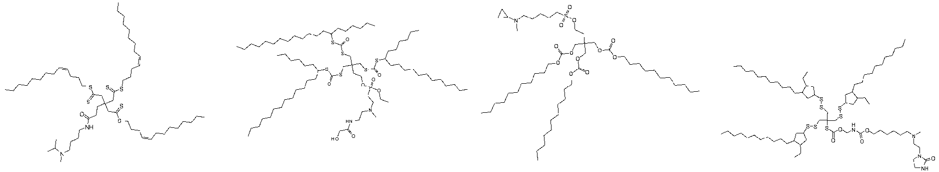

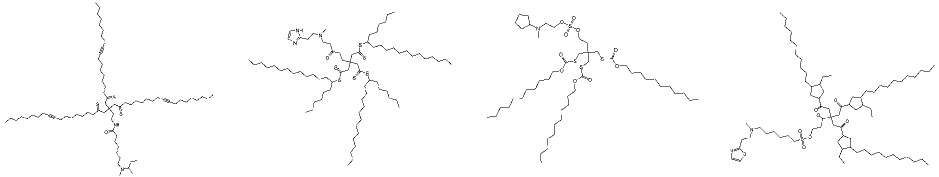

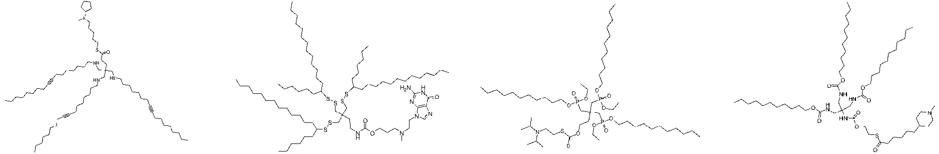

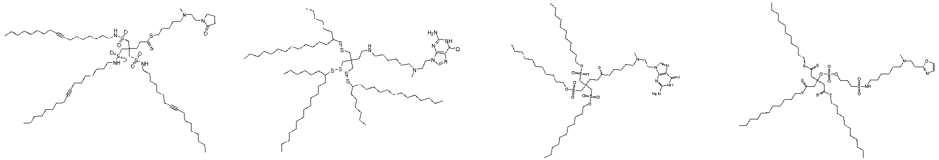

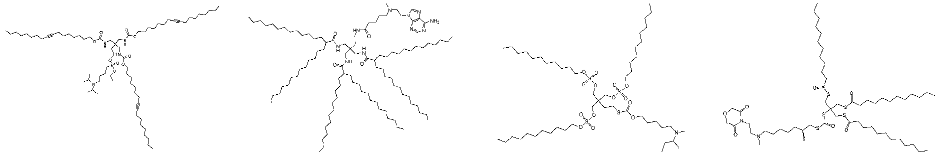

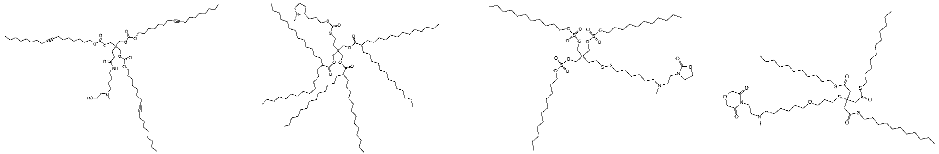

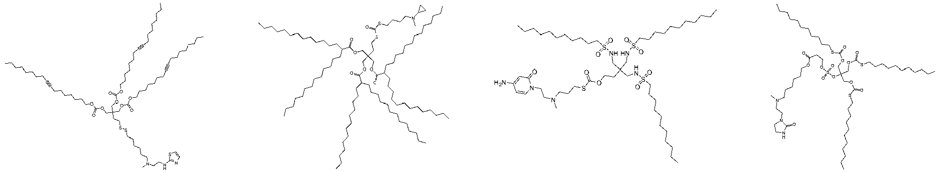

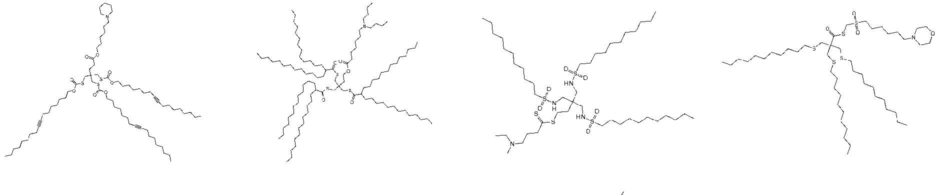

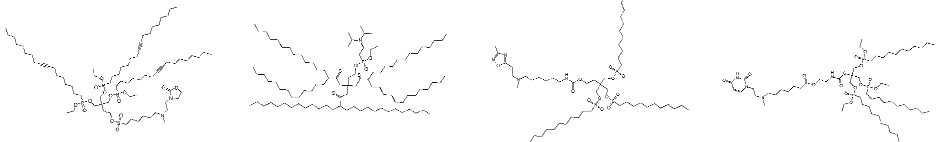

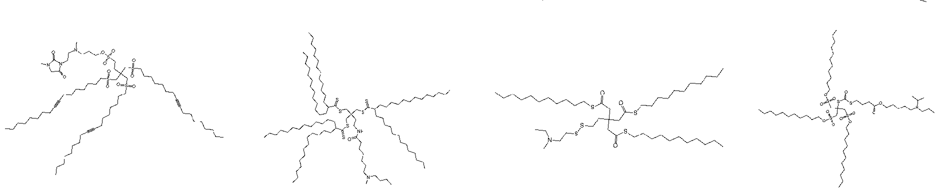

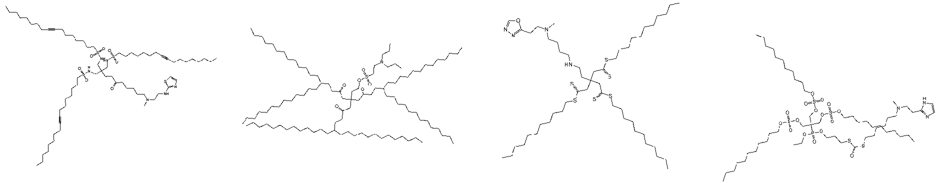

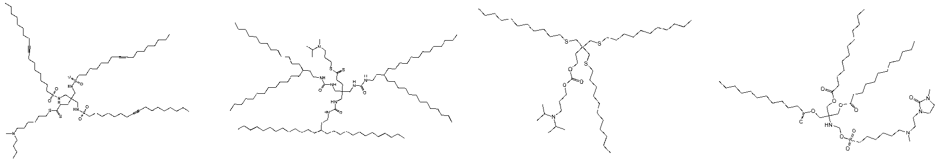

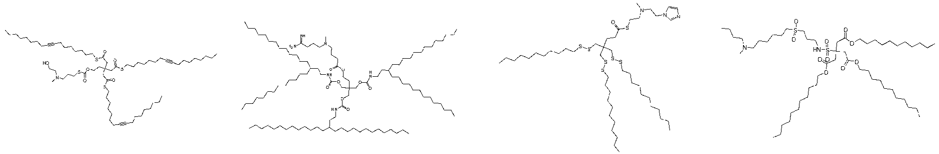

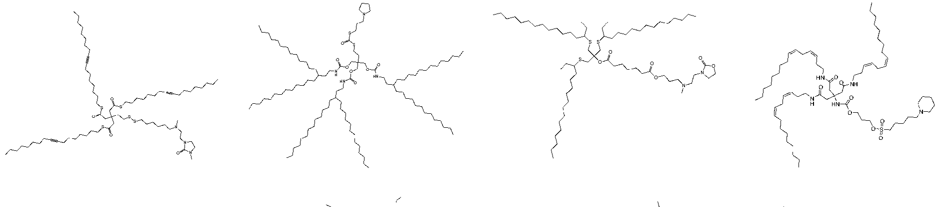

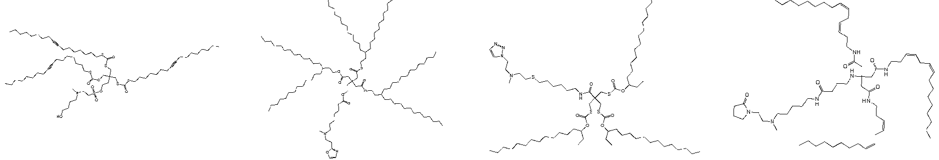

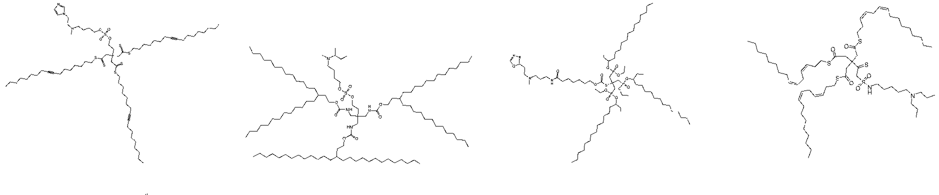

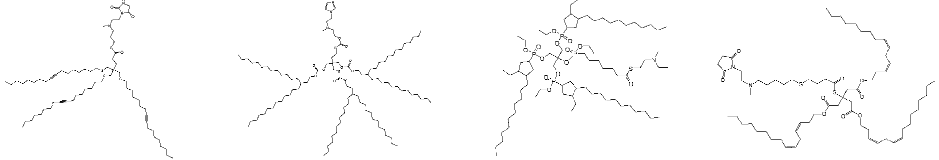

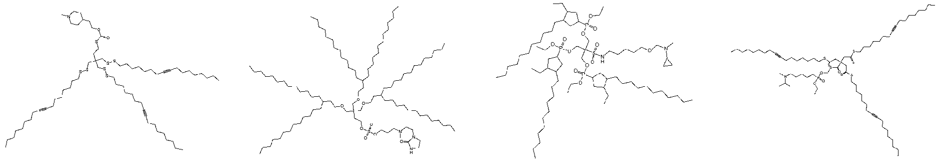

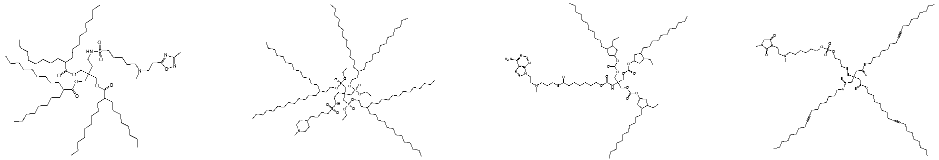

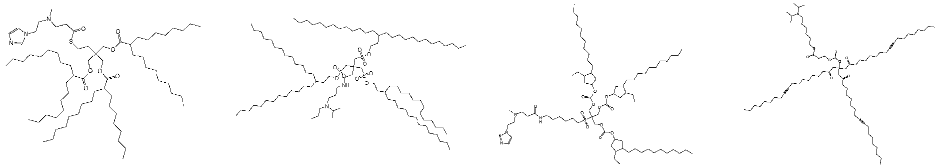

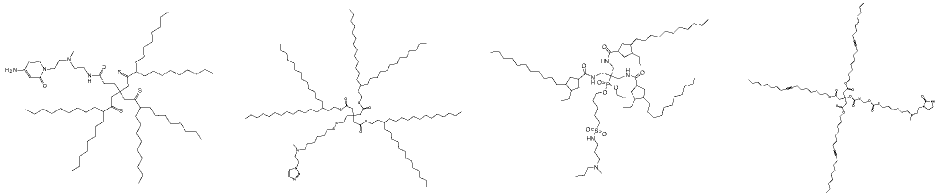

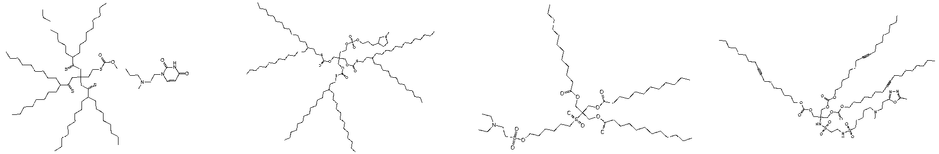

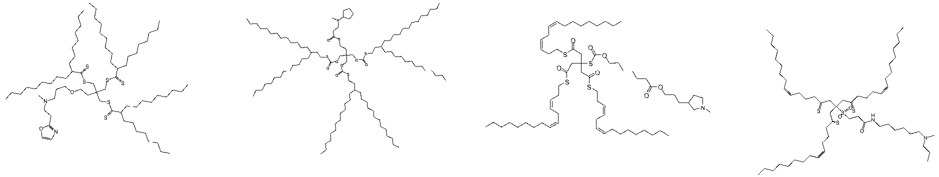

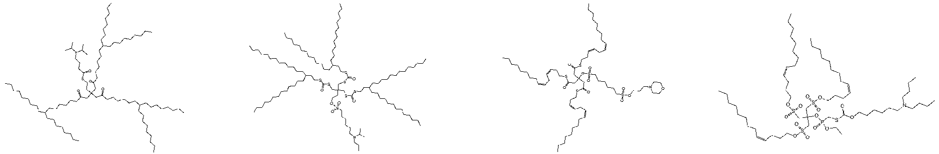

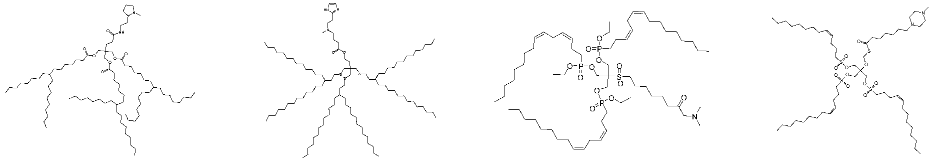

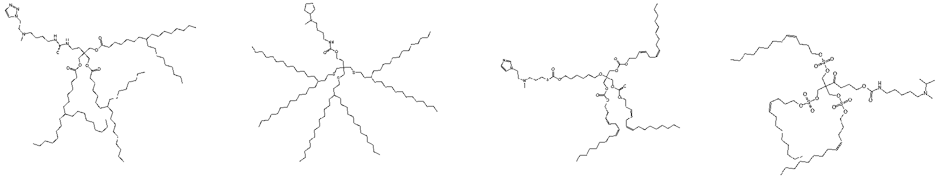

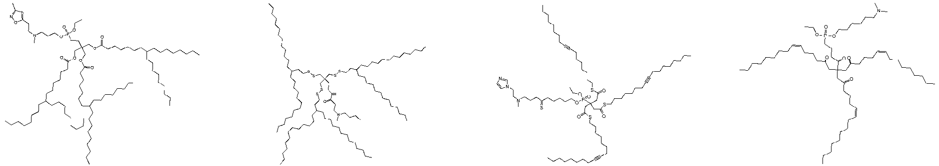

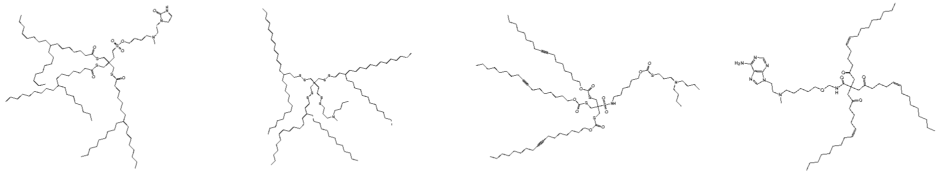

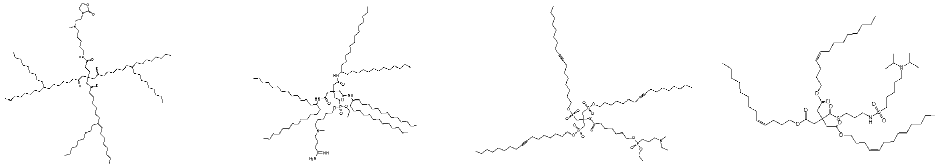

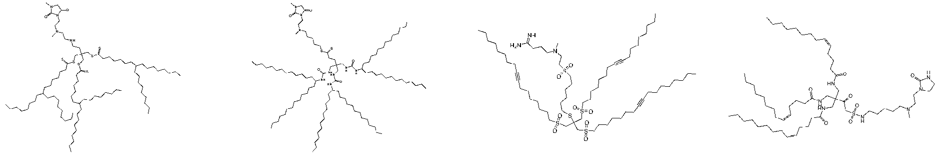

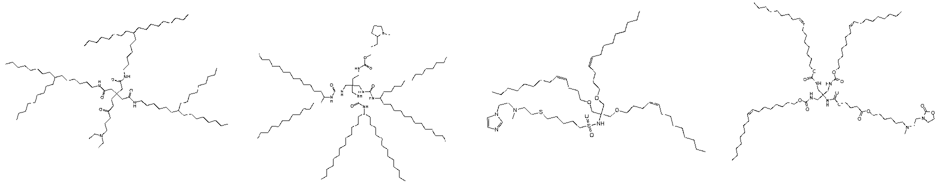

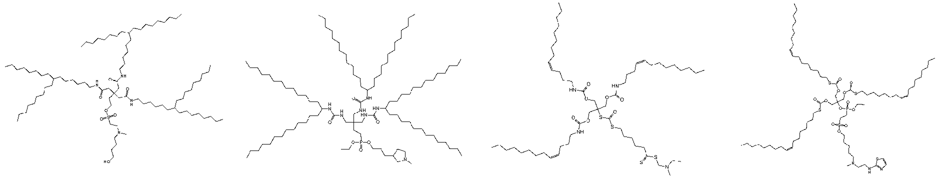

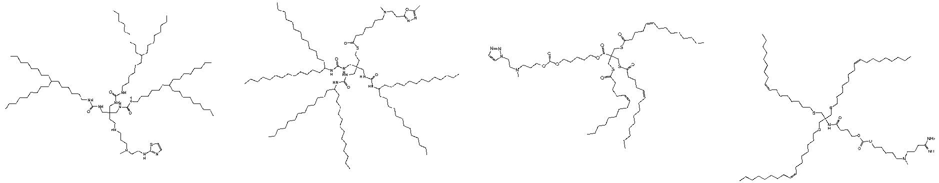

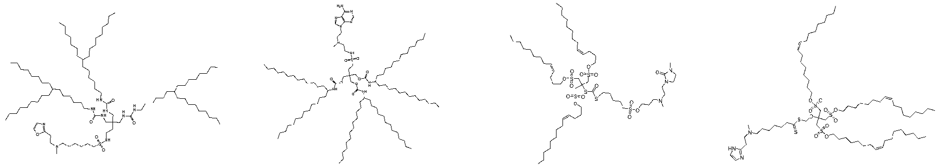

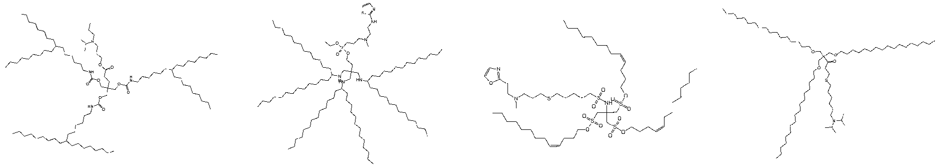

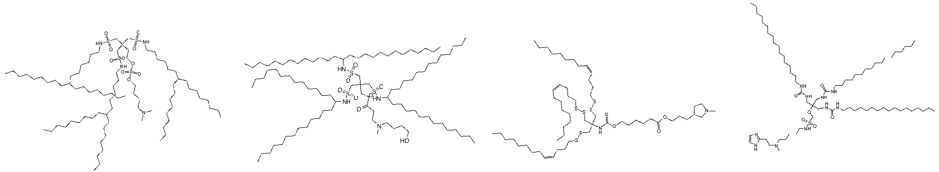

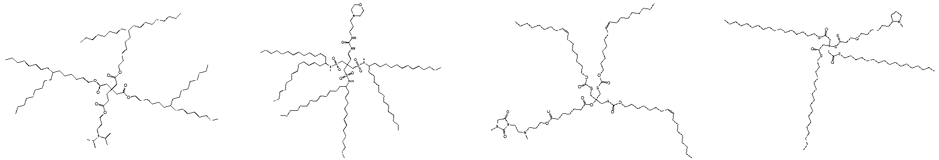

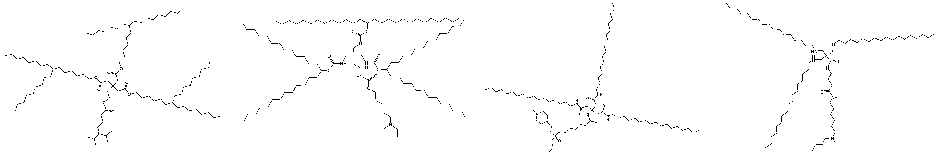

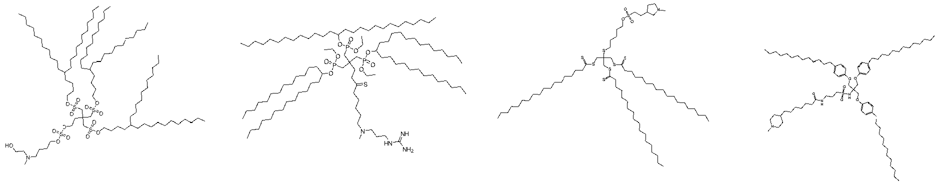

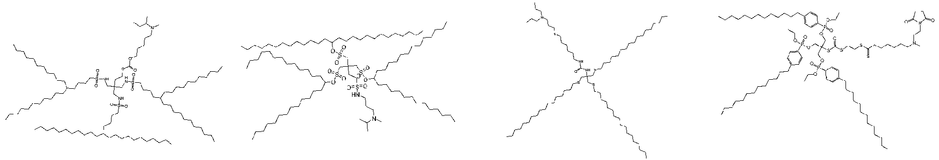

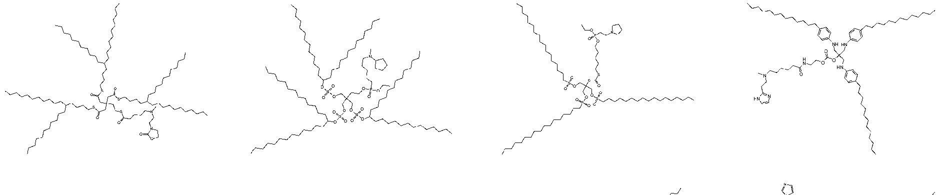

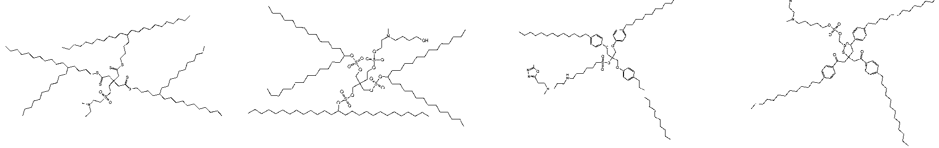

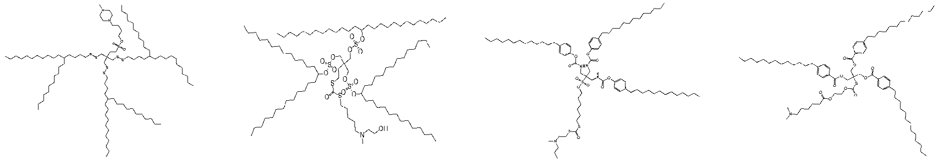

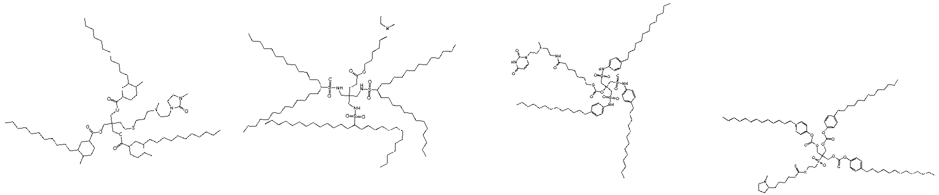

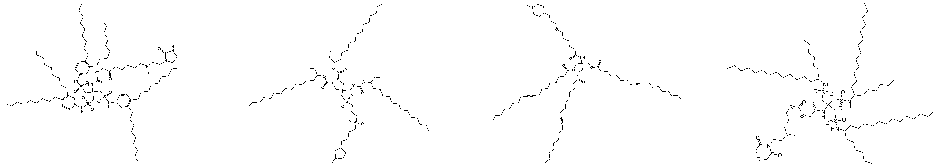

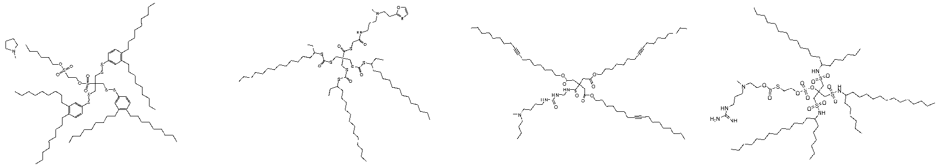

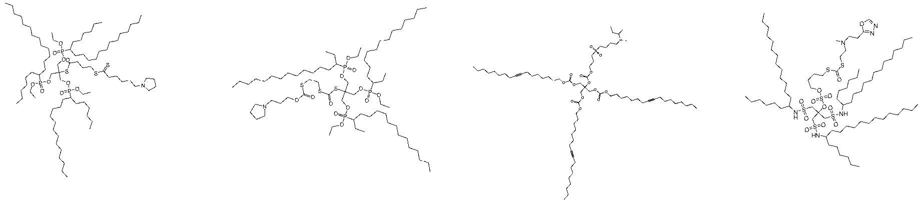

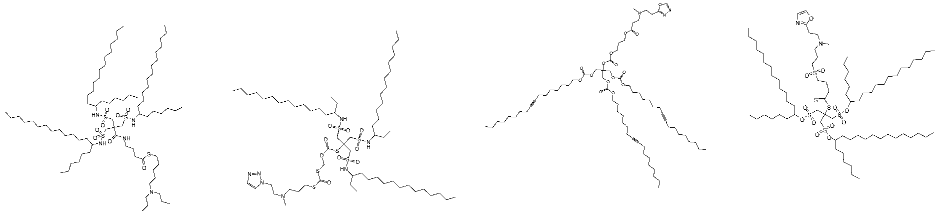

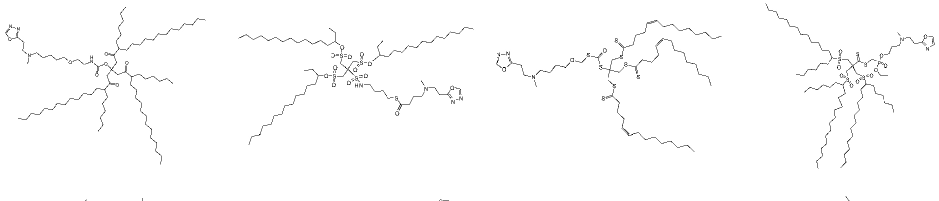

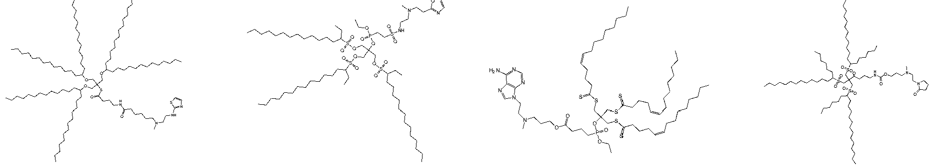

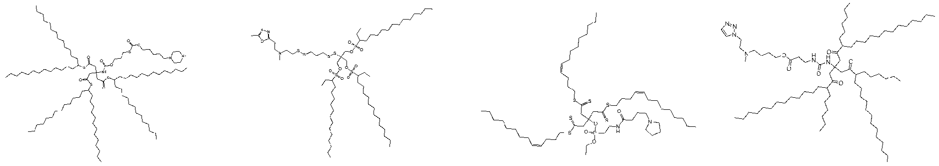

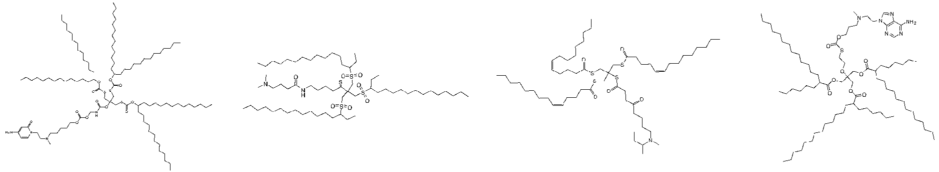

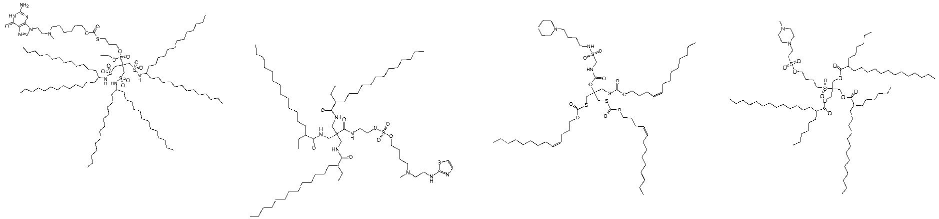

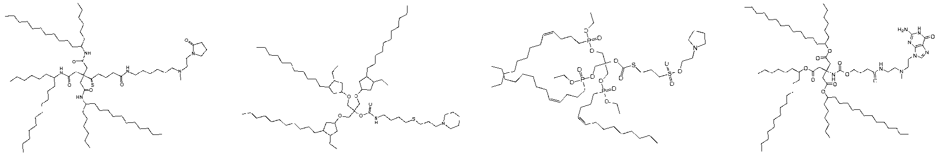

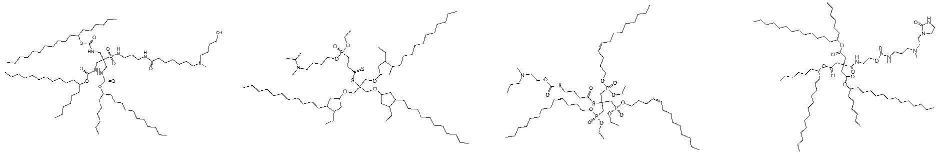

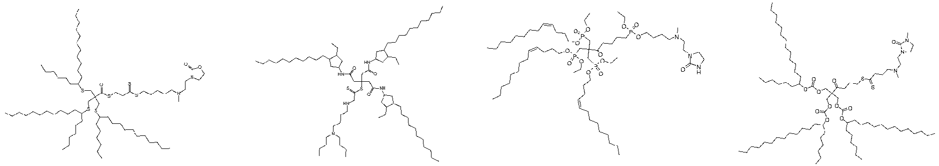

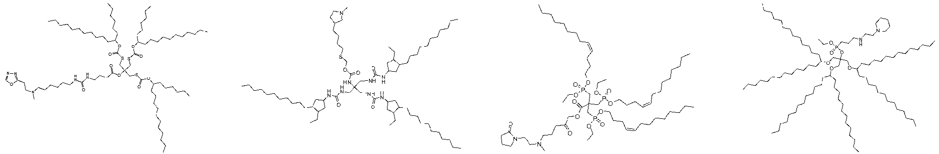

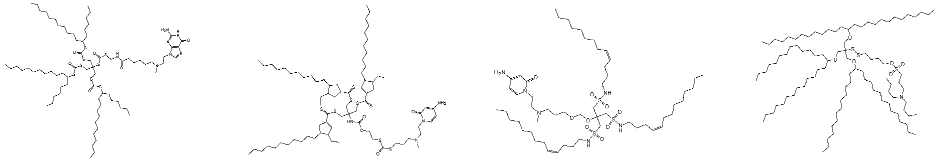

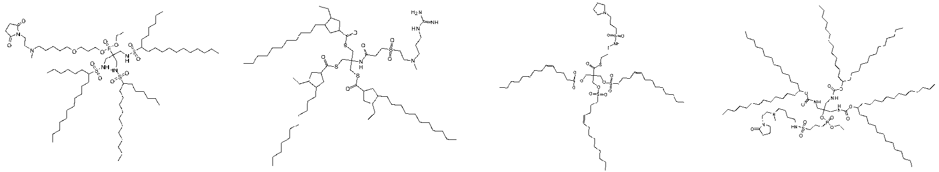

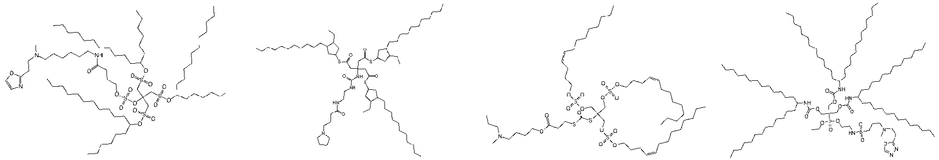

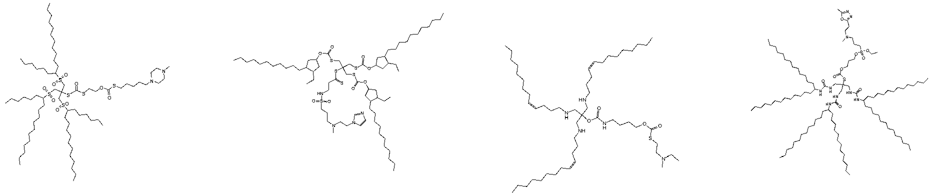

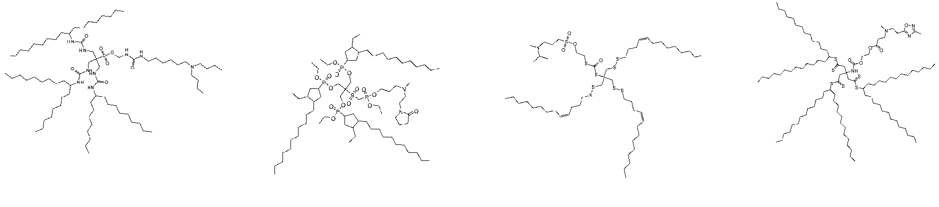

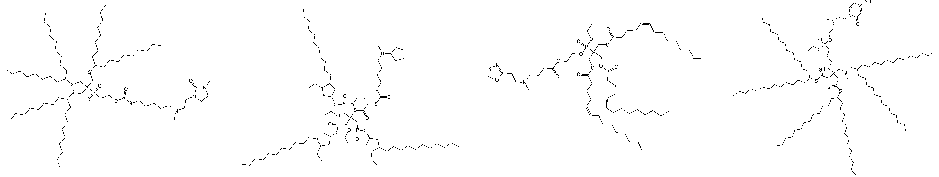

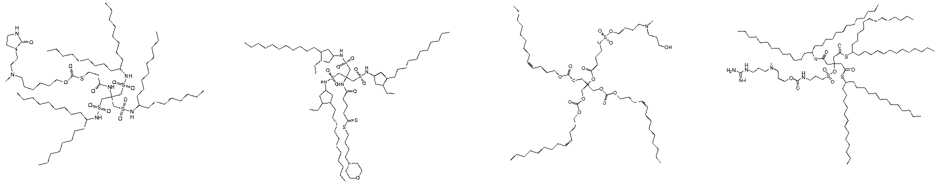

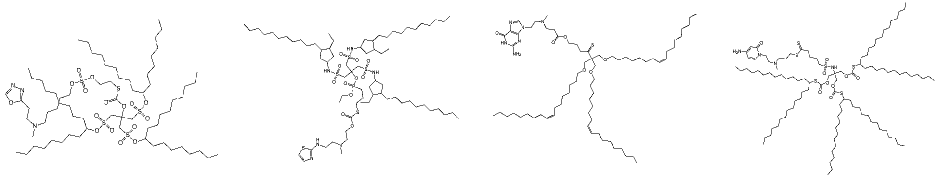

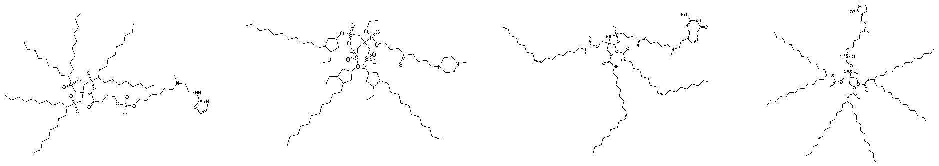

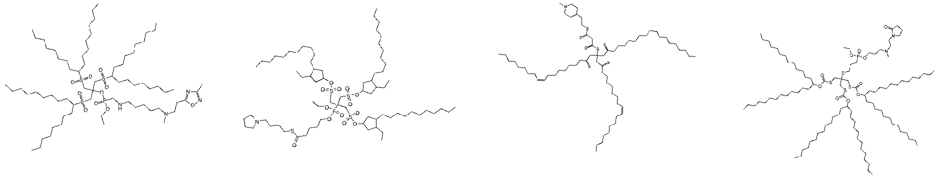

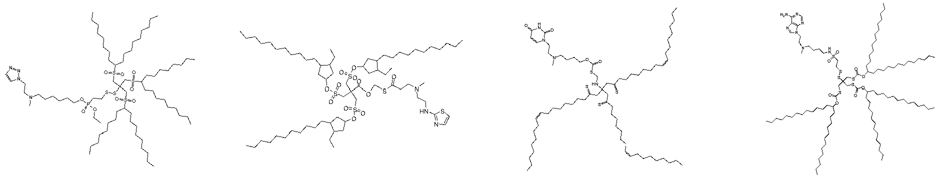

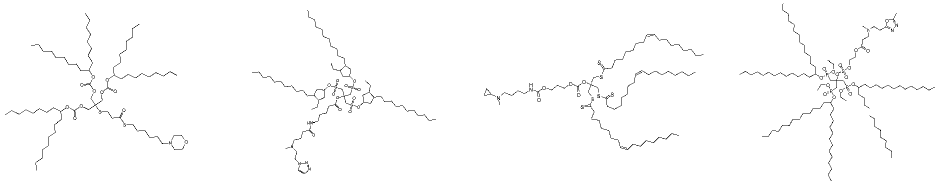

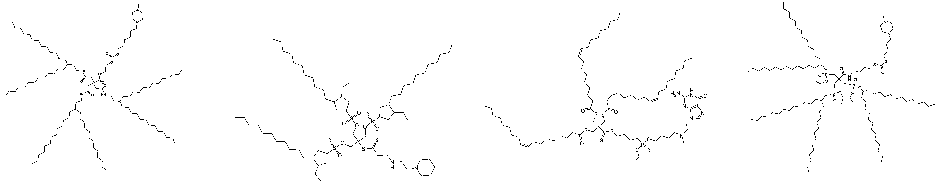

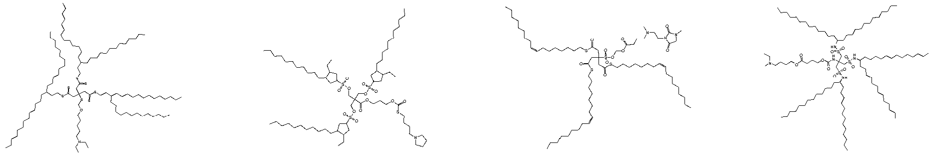

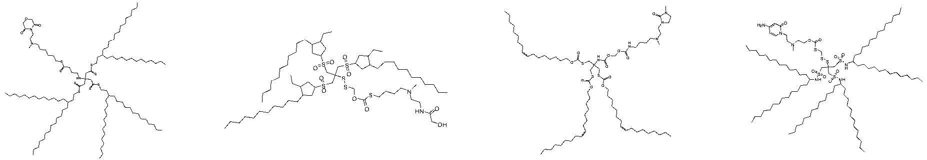

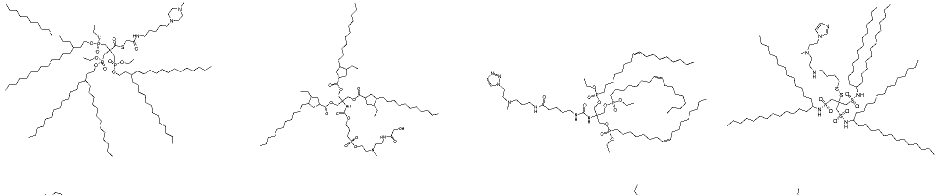

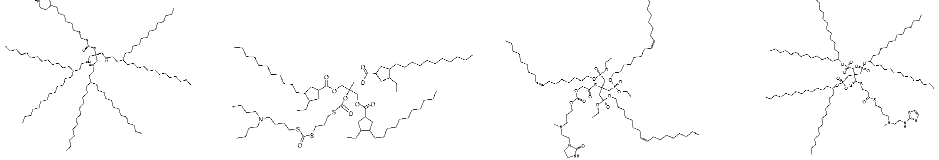

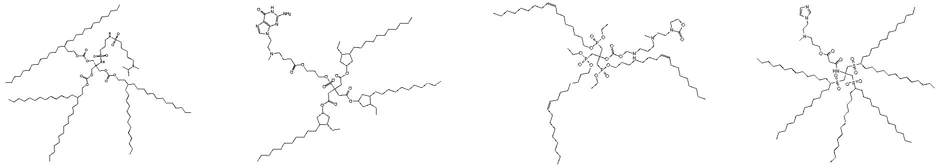

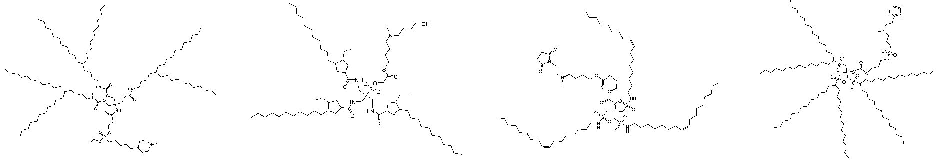

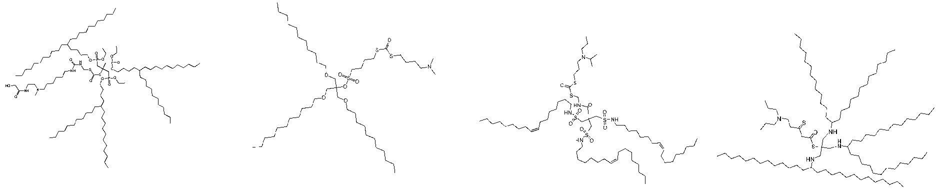

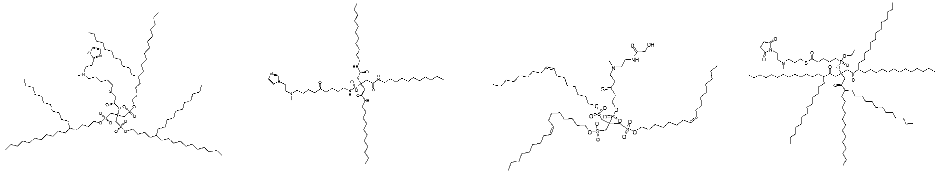

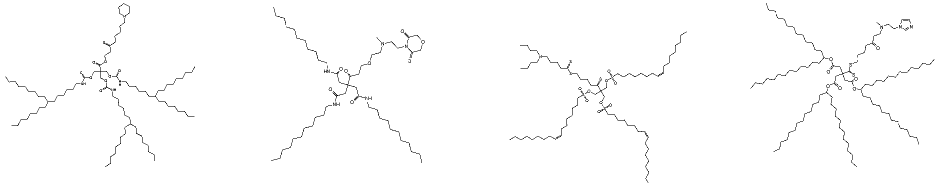

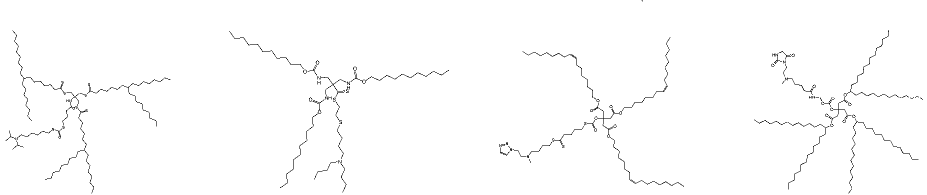

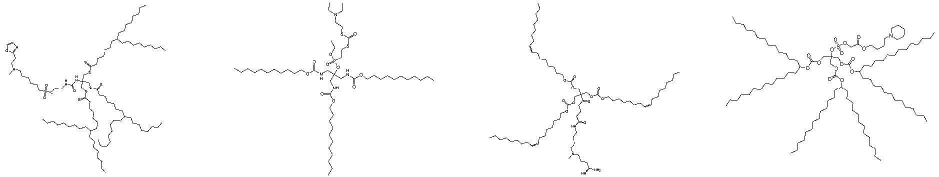

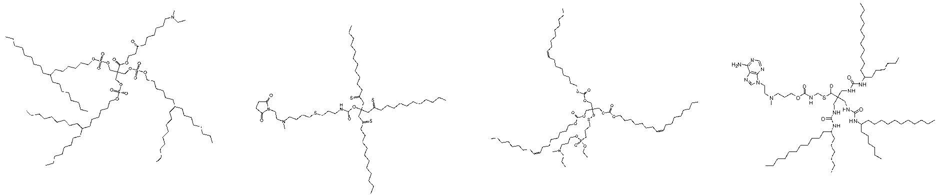

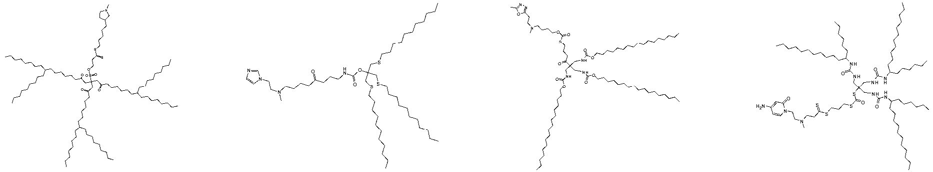

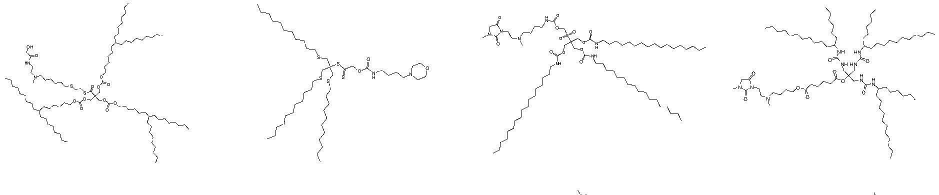

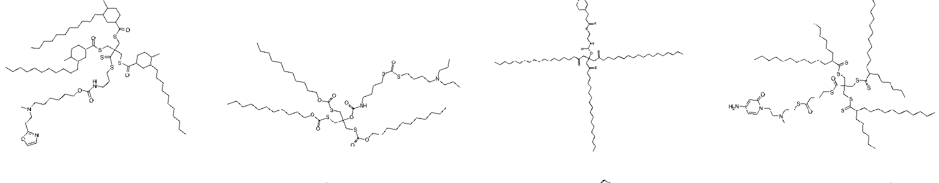

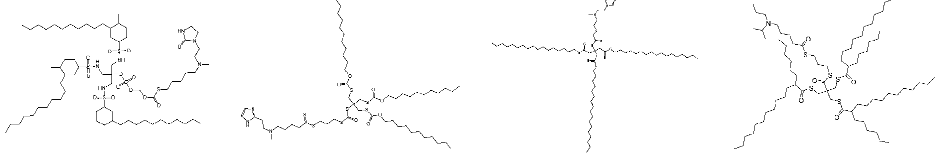

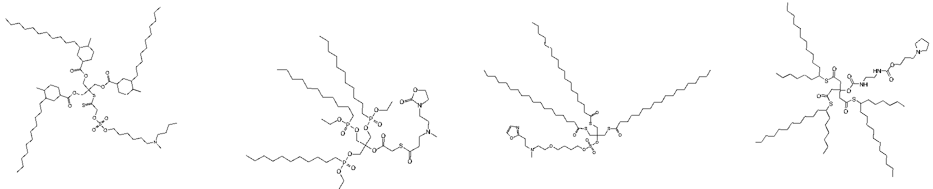

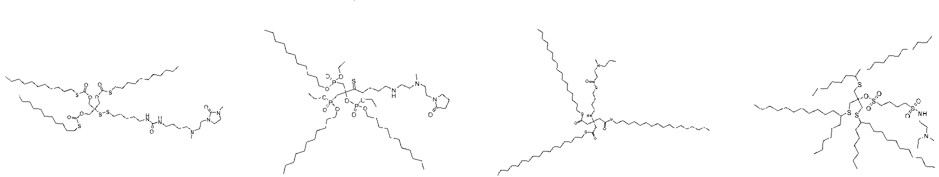

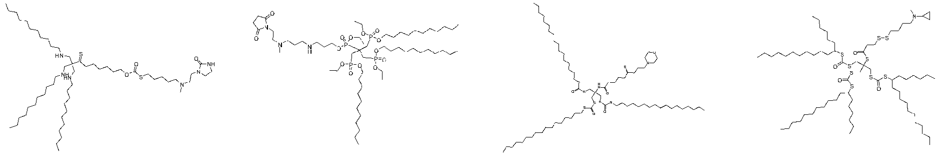

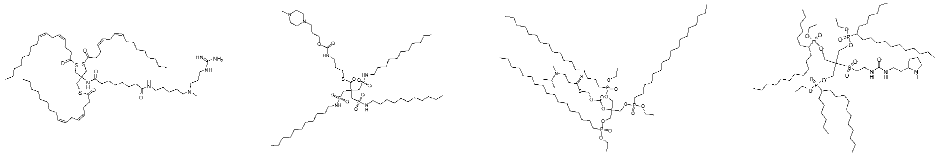

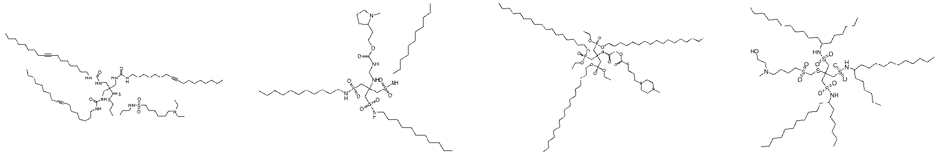

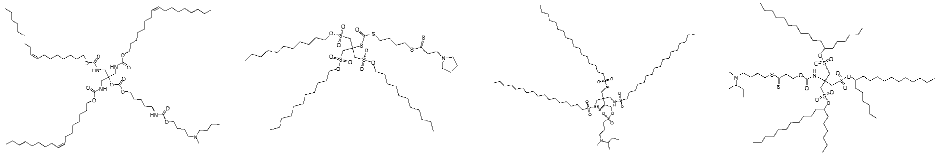

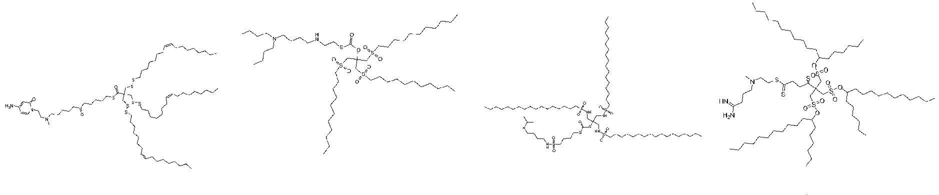

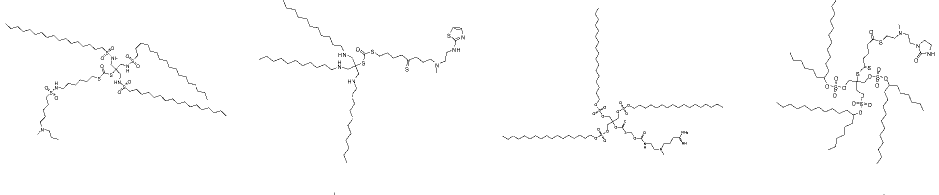

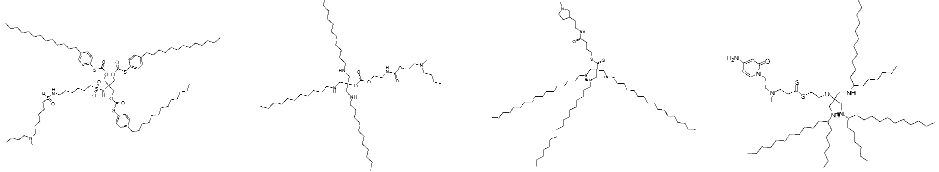

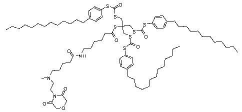

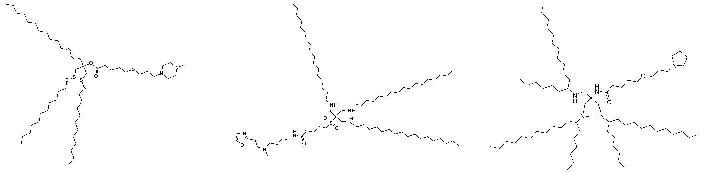

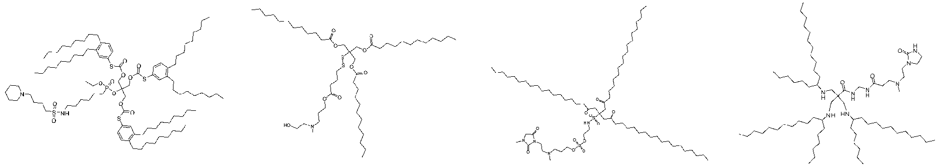

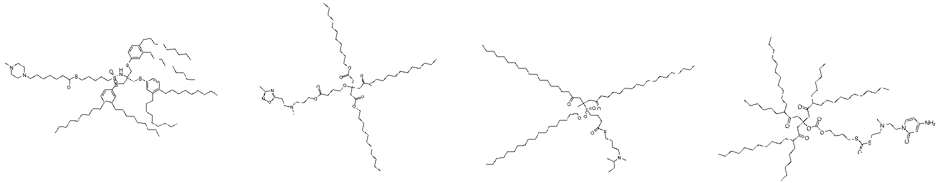

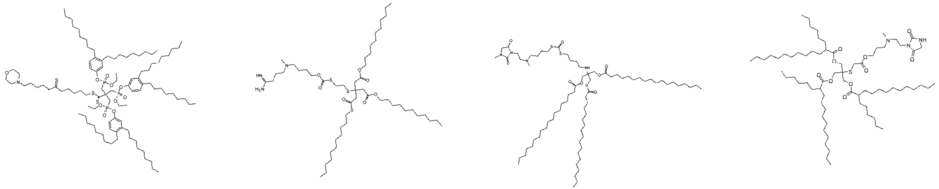

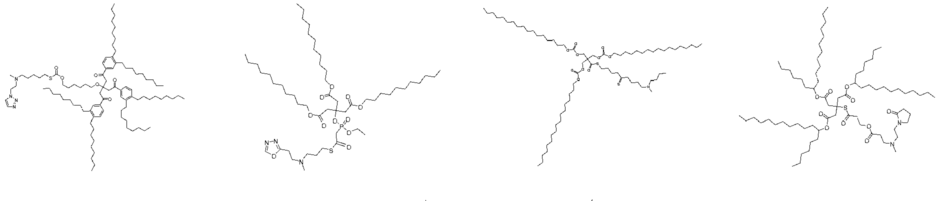

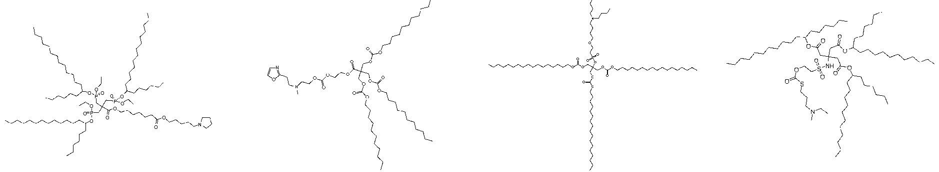

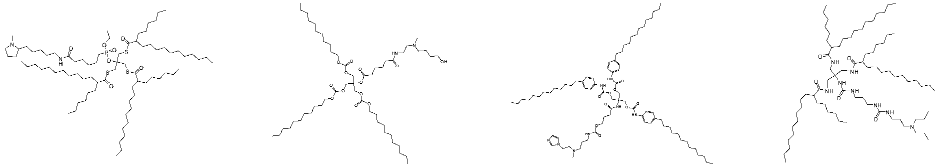

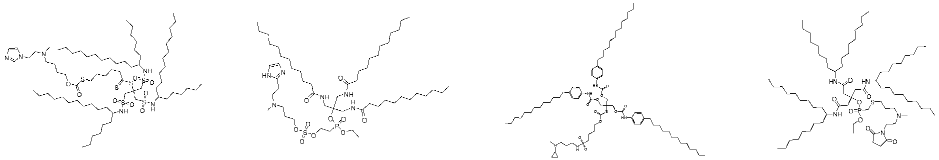

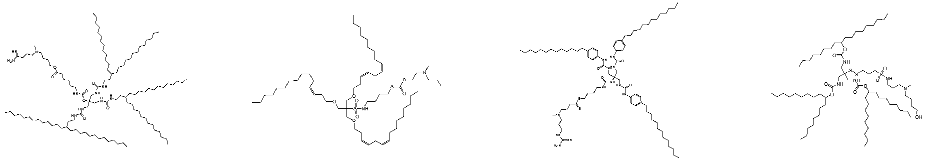

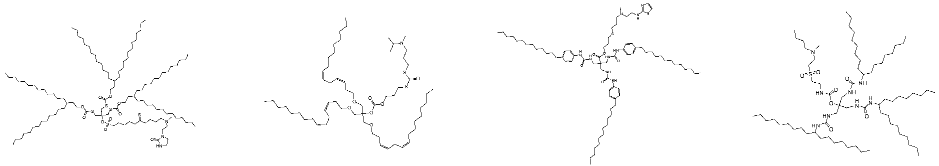

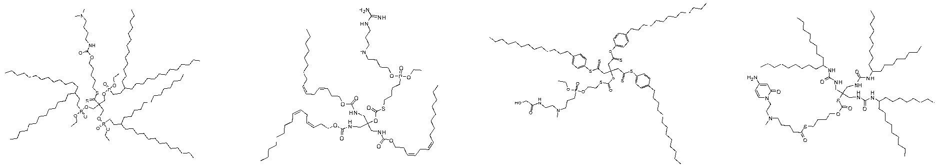

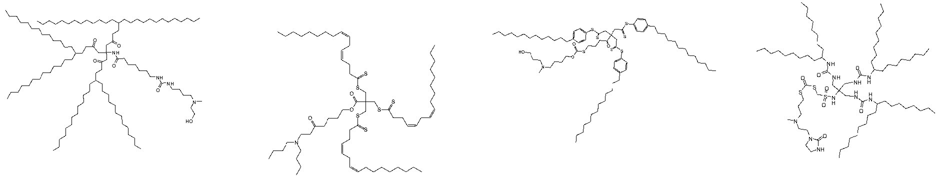

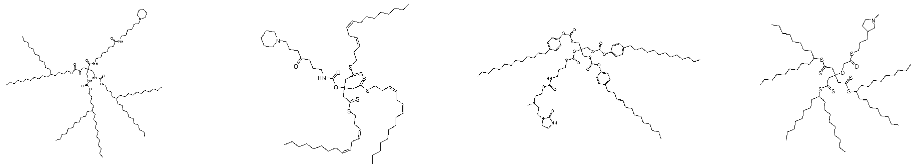

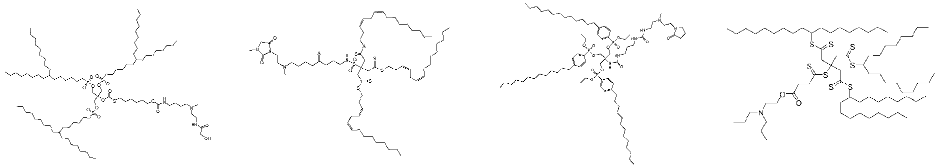

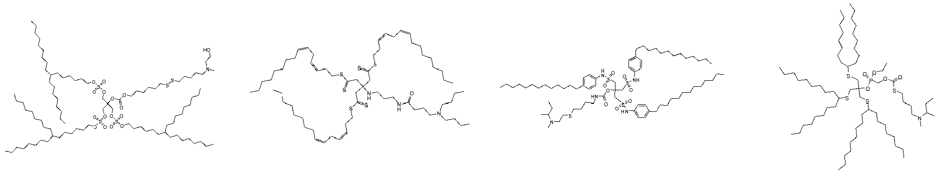

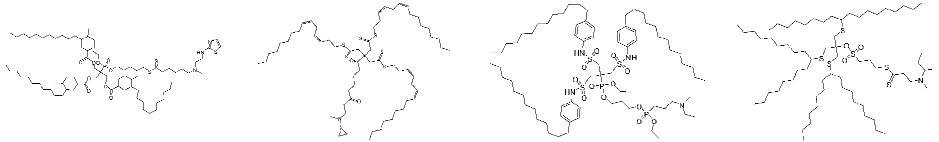

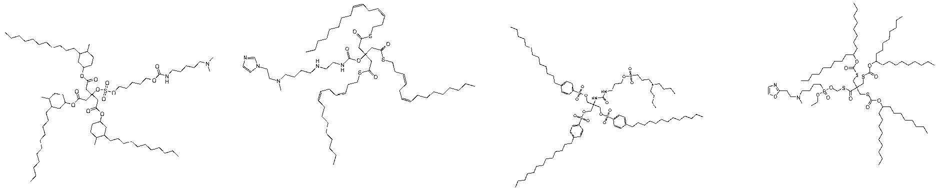

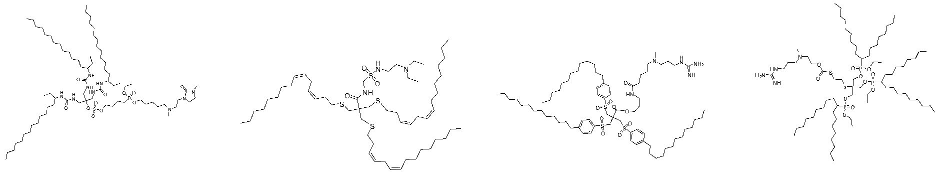

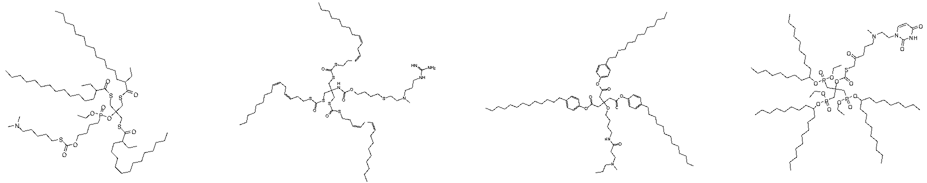

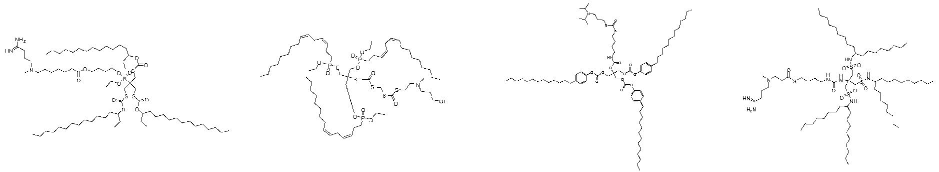

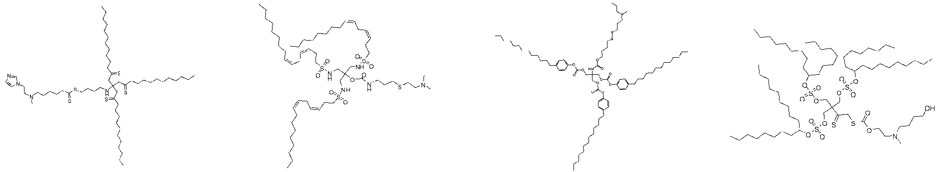

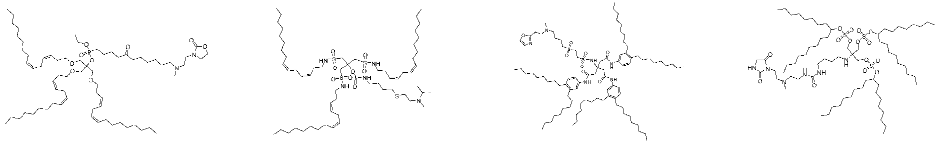

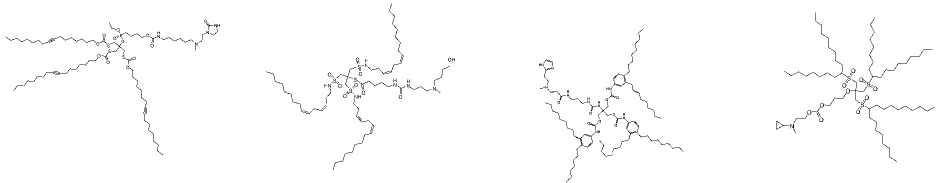

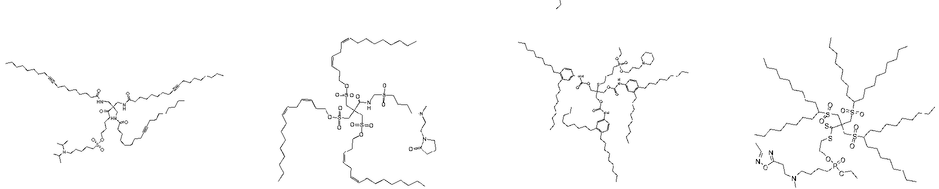

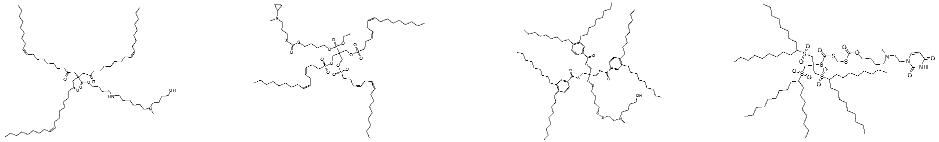

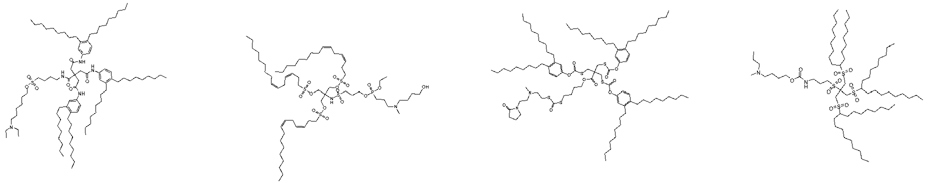

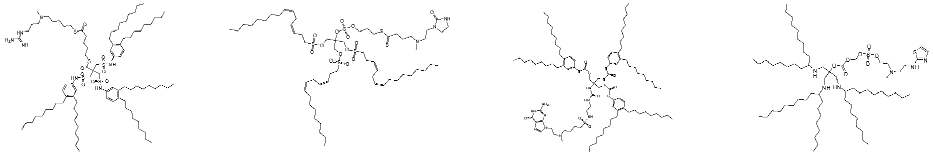

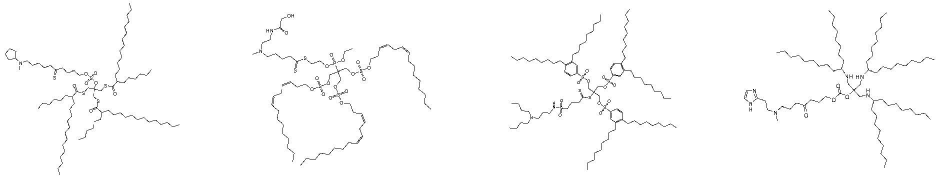

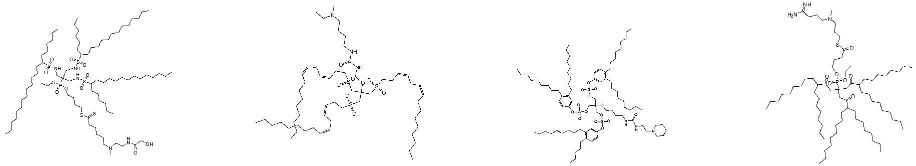

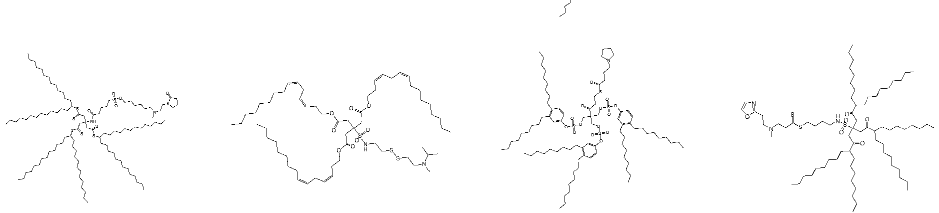

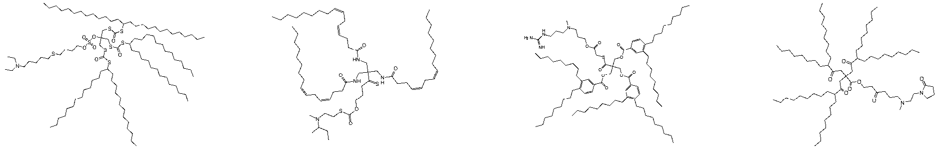

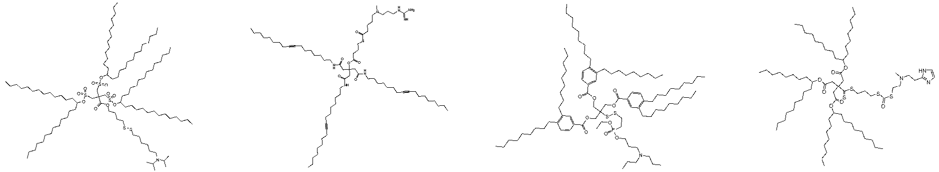

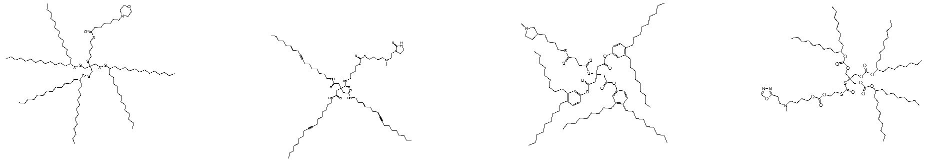

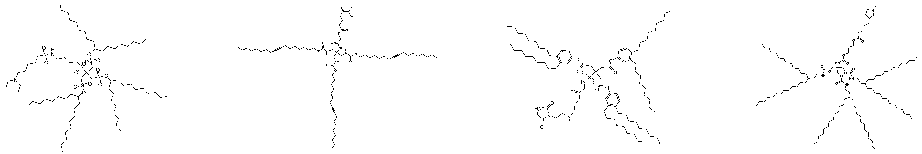

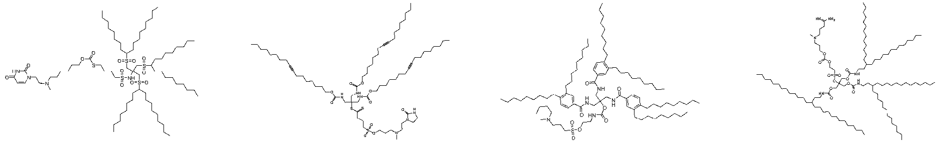

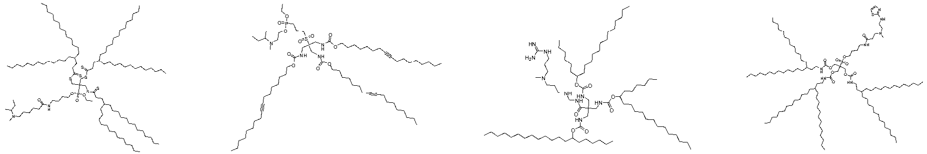

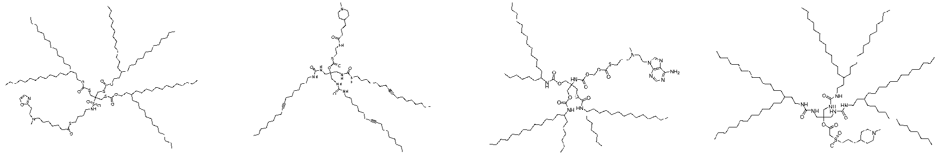

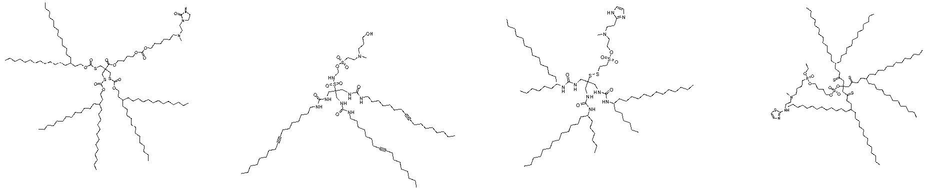

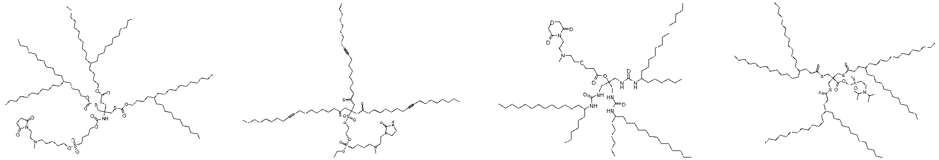

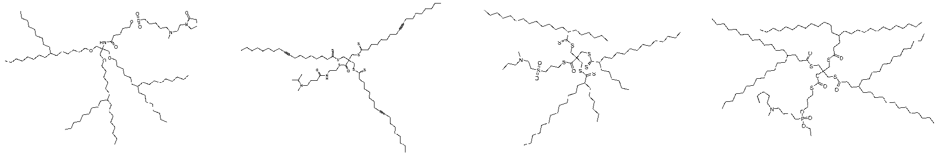

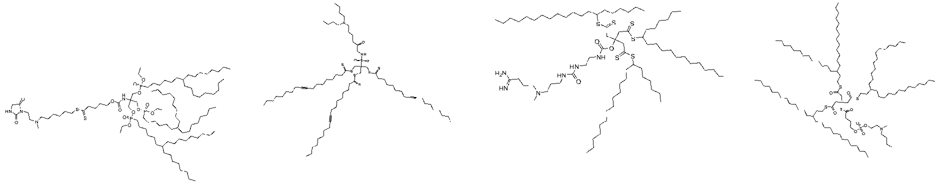

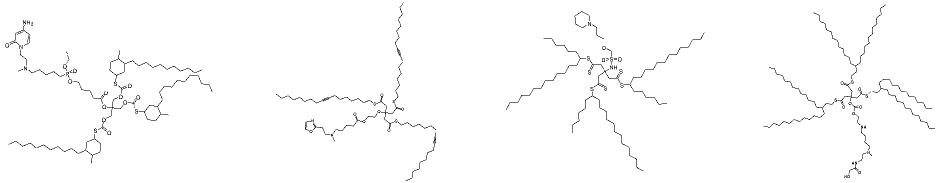

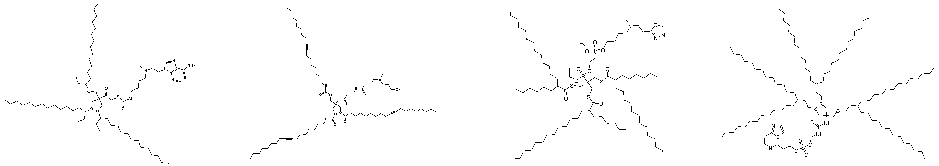

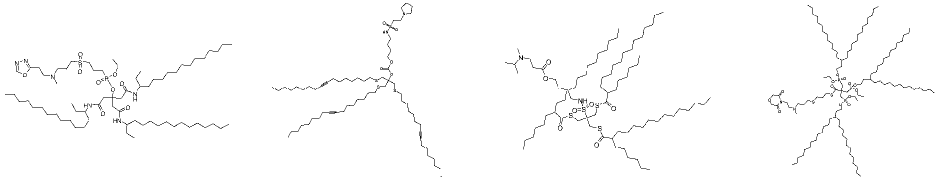

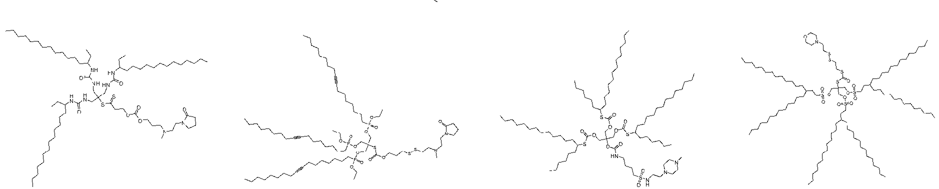

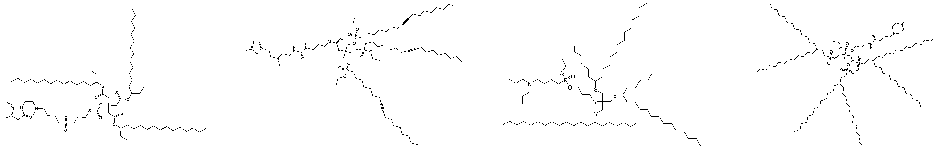

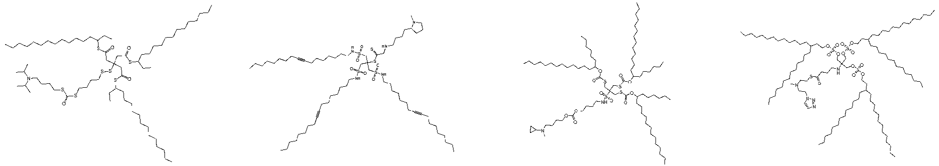

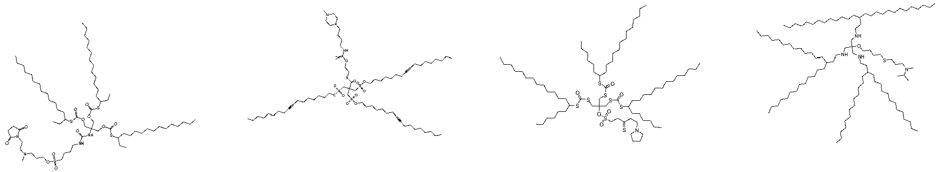

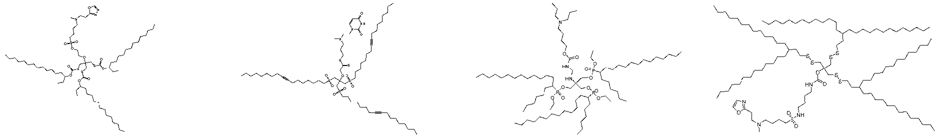

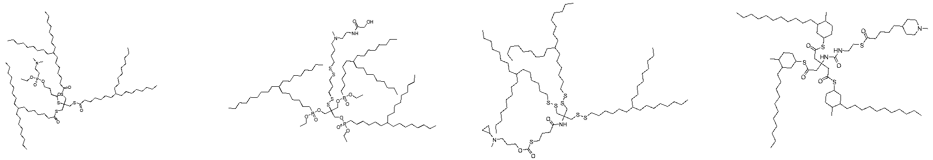

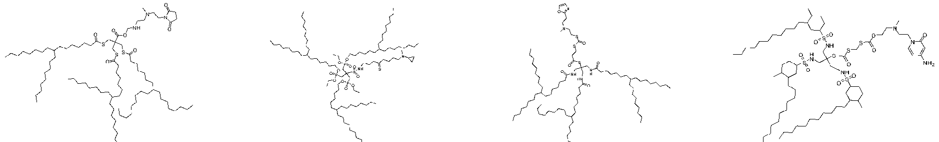

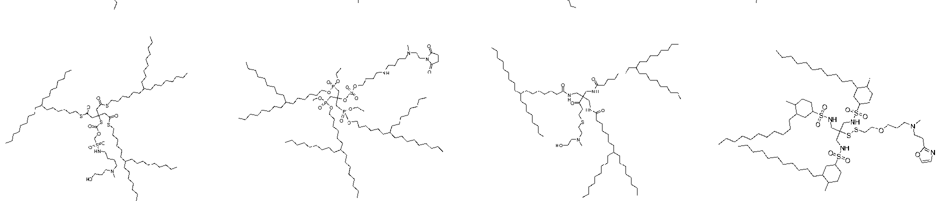

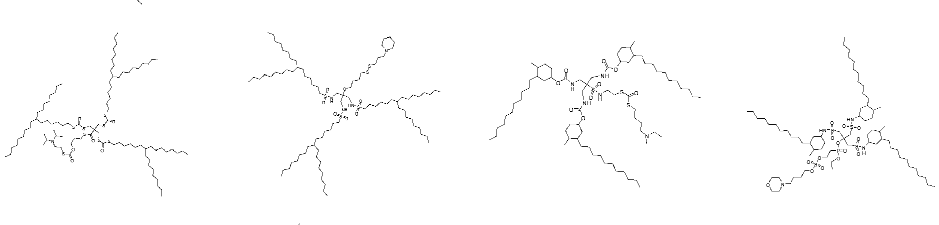

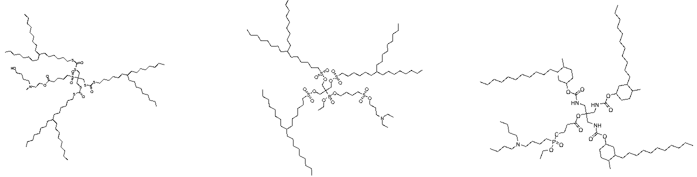

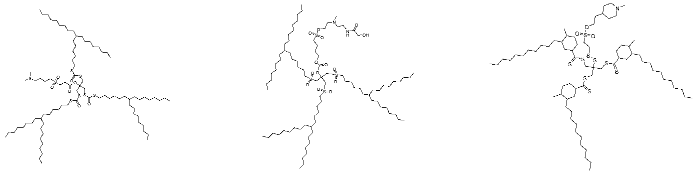

25. The pulmonary delivery system comprising ionizable lipids according to any one of claims 1 to 23, wherein the second ionizable lipid is a compound of Formula (2), or a salt, stereoisomer or tautomer thereof: 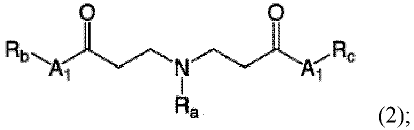
wherein A₁ is NH or O;
Rₐ is selected from C₆-C₂₄ alkyl, C₆-C₂₄ alkenyl, C₆-C₂₄ cycloalkyl, C₆-C₂₄ alcohol and C₆-C₂₄ short-chain polyoxyethylene; the C₆-C₂₄ alkyl, C₆-C₂₄ alkenyl, C₆-C₂₄ cycloalkyl, C₆-C₂₄ alcohol and C₆-C₂₄ short-chain polyoxyethylene are linear or branched;R_{b} and R_{c} are each independently selected from C₁-C₁₂ alkyl, C₂-C₁₂ alkenyl, C₁-C₁₂ alkynyl, C₃-C₁₂ cycloalkyl, C₆-C₁₂ aryl, C₁-C₁₂ alkyl alcohol, C₁-C₁₂ heterocyclyl and alkylamine;
the alkylamine has the structure: 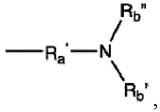 wherein Rₐ' is C₁-C₁₂ alkyl; R_{b}' and R_{b}" are each independently selected from H, C₁-C₆ alkylamine, 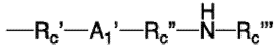 or 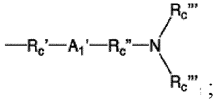 R_{c}" is selected from unsubstituted or amino-substituted C₁-C₆ alkyl; R_{c}‴ is H, 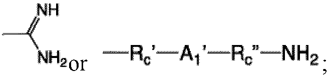
provided that when A₁' is -CO-NH-, -NH-CO- or -CO-O-, R_{c}' is C₁-C₆ alkyl; and when A₁' is -CO-, R_{c}' is absent.

26. The pulmonary delivery system comprising ionizable lipids according to claim 25, wherein the compound of Formula (2) is selected from at least one of the following compounds:

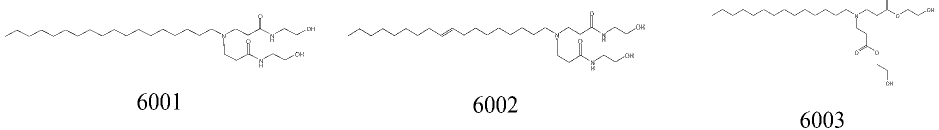

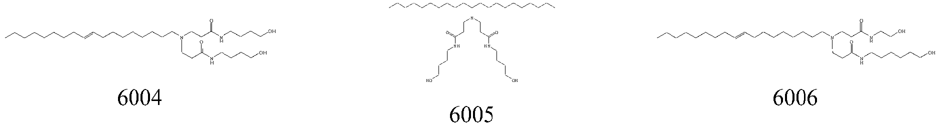

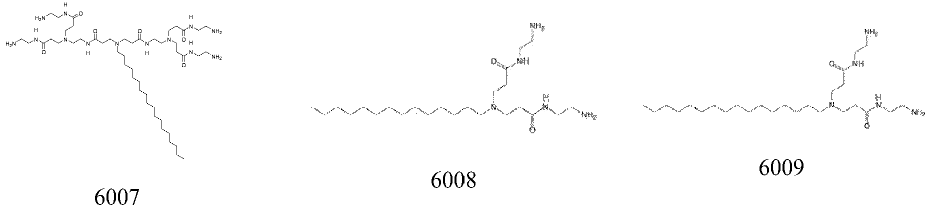

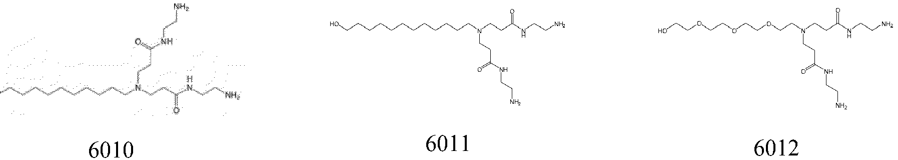

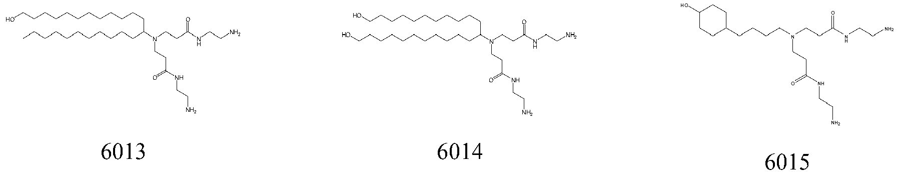

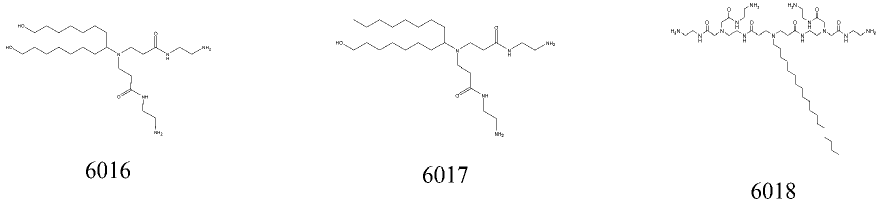

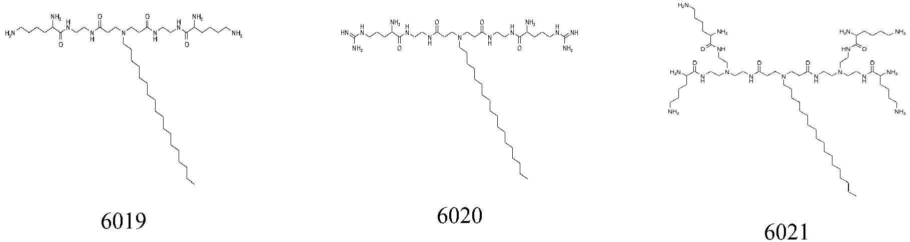

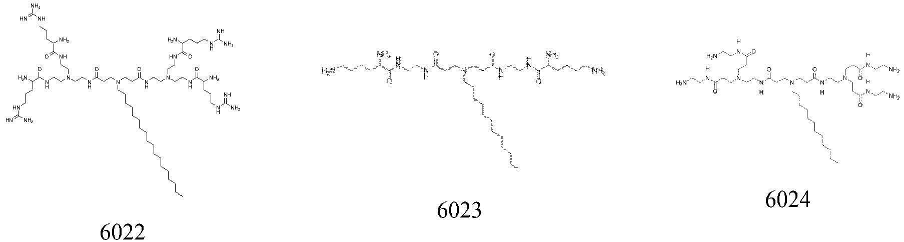

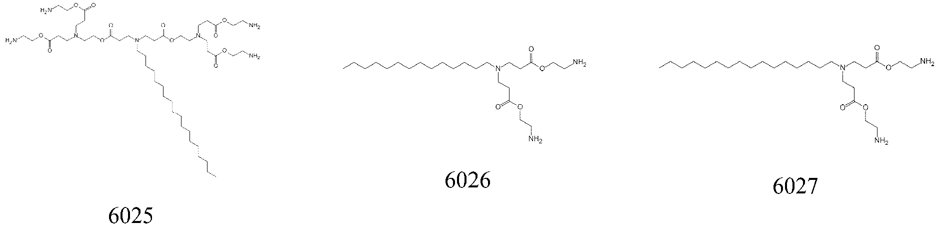

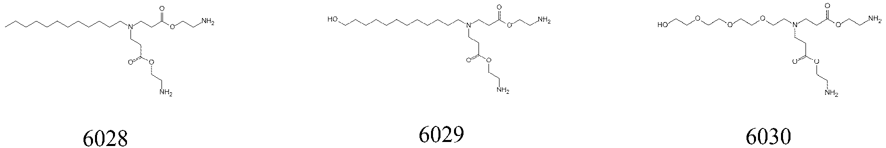

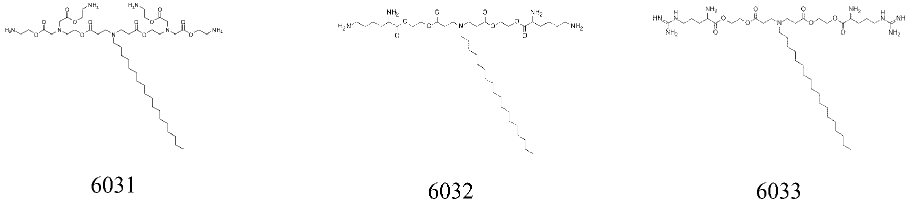

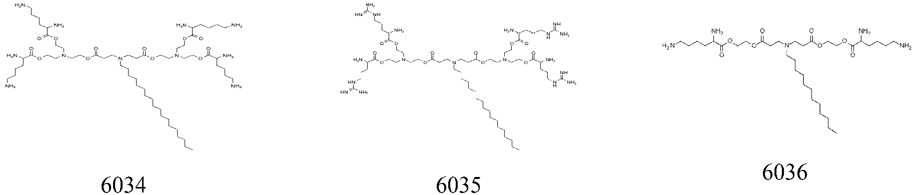

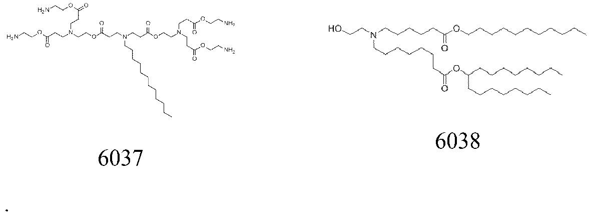

27. The pulmonary delivery system comprising ionizable lipids according to any one of claims 1 to 23, wherein the third ionizable lipid is selected from at least one of the following compounds:

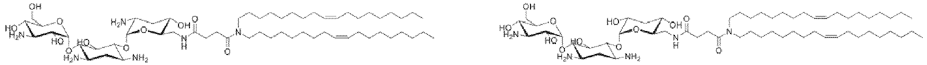

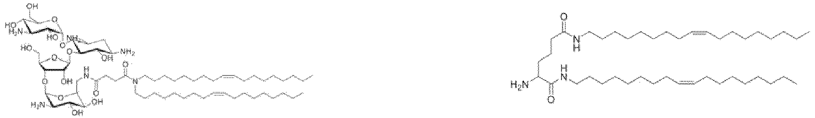

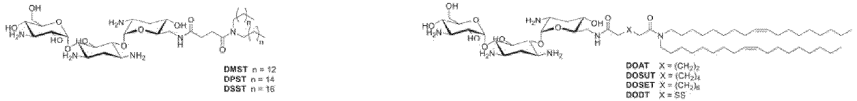

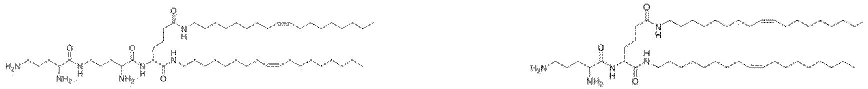

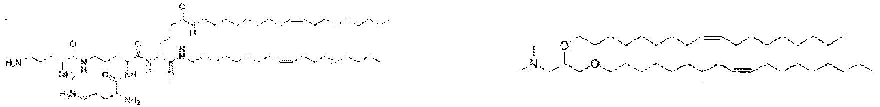

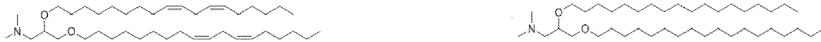

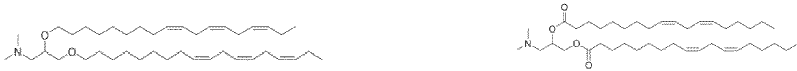

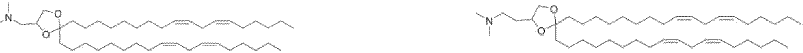

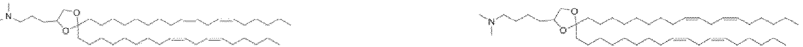

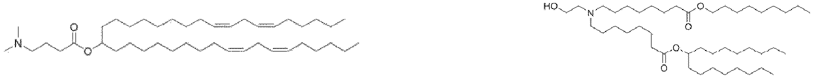

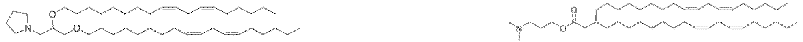

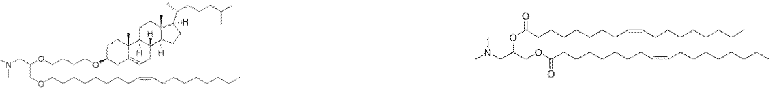

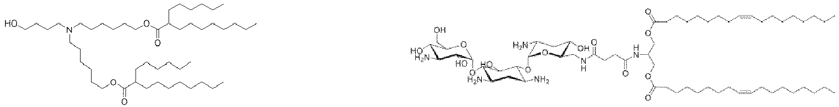

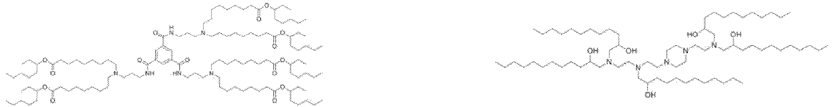

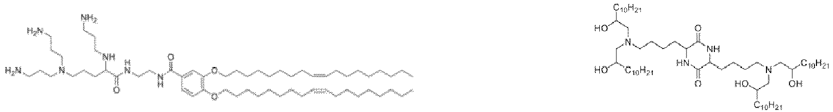

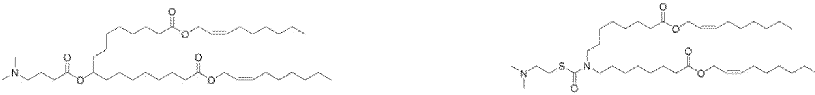

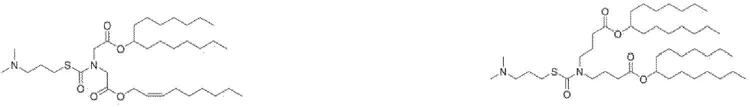

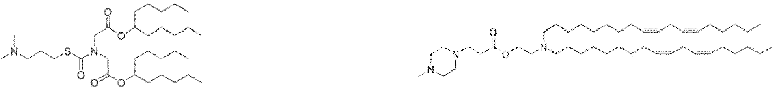

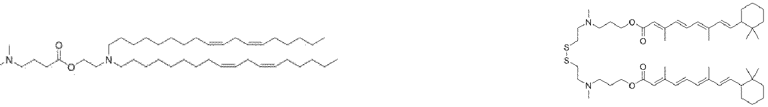

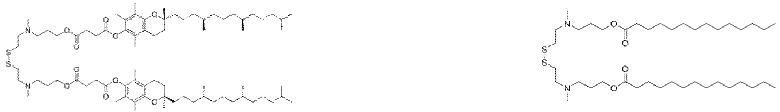

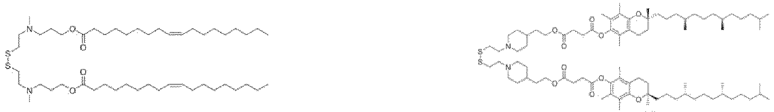

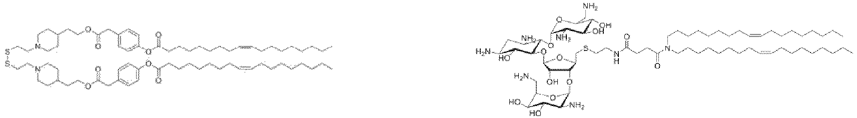

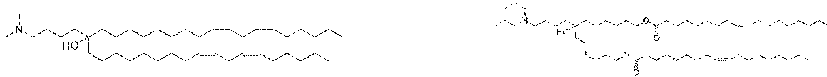

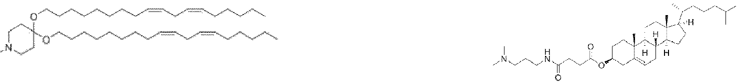

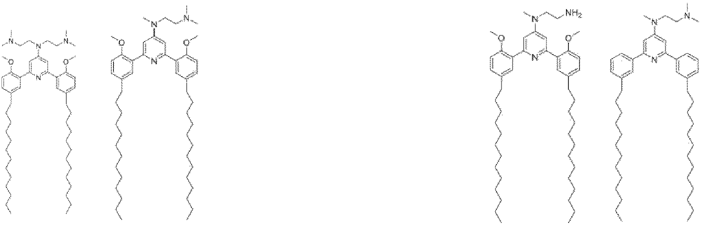

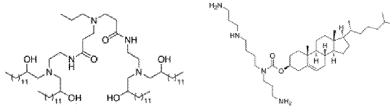

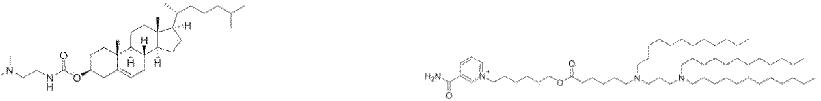

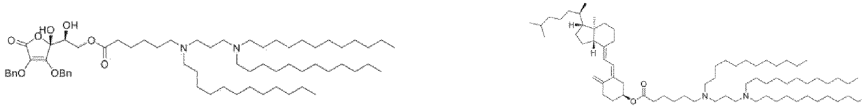

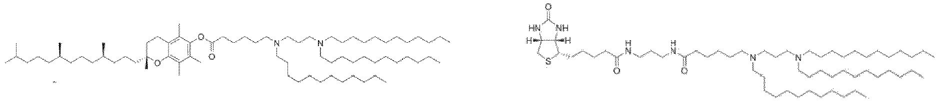

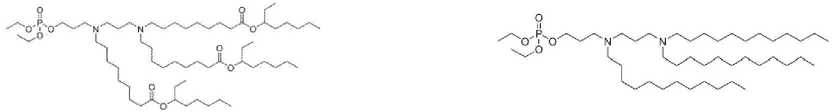

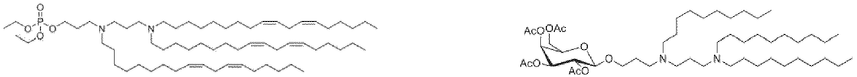

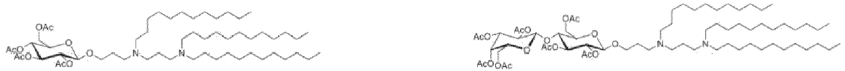

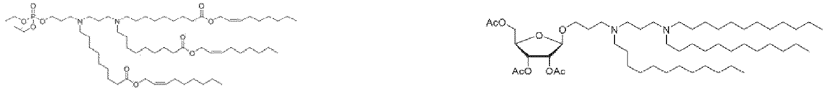

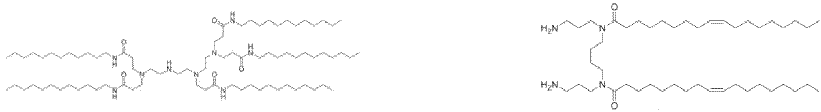

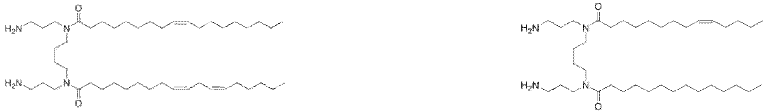

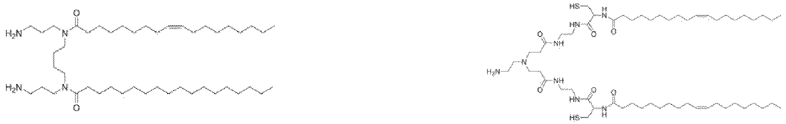

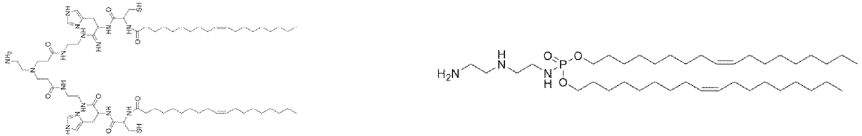

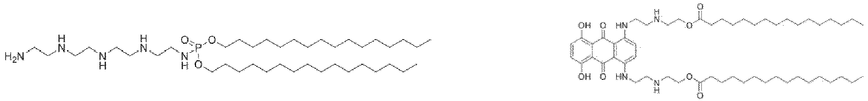

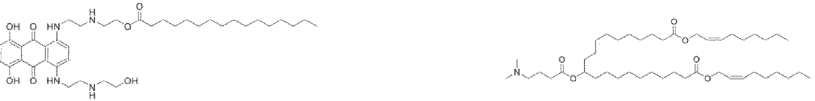

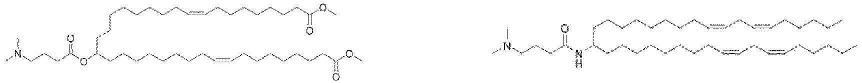

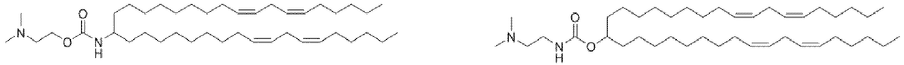

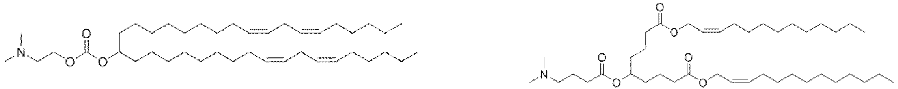

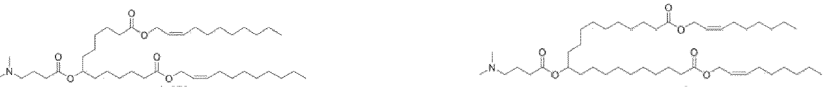

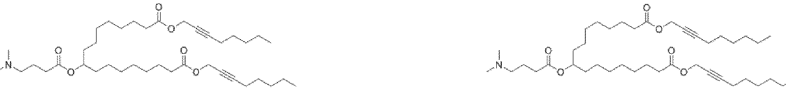

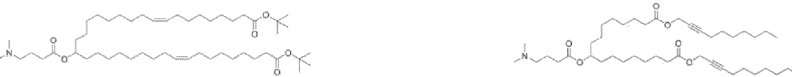

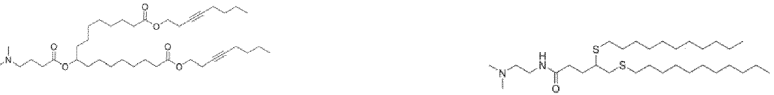

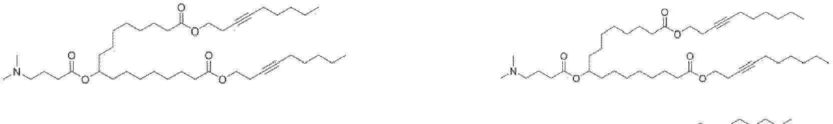

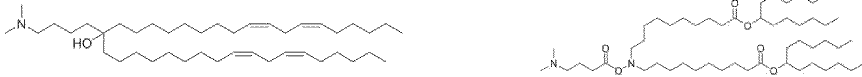

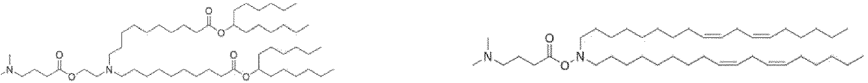

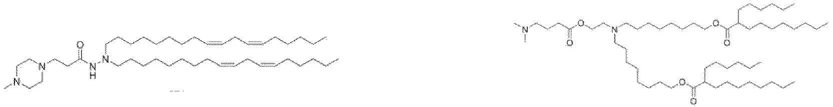

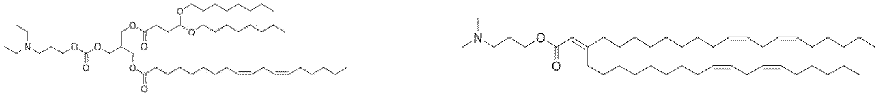

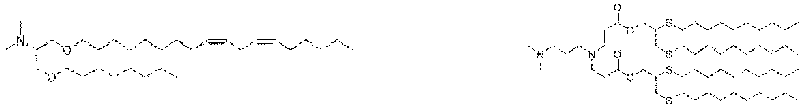

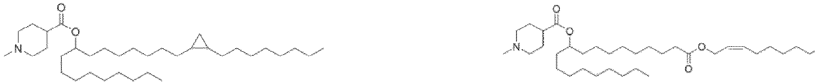

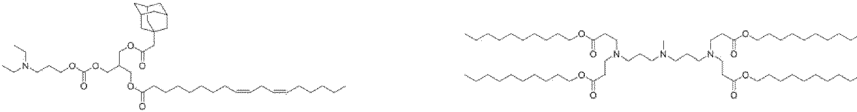

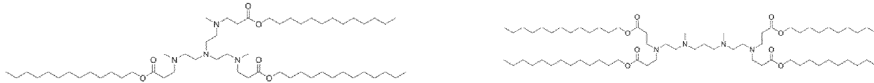

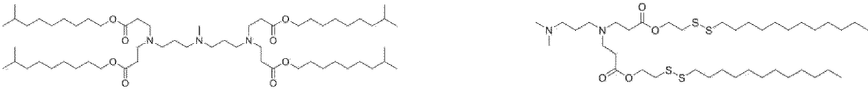

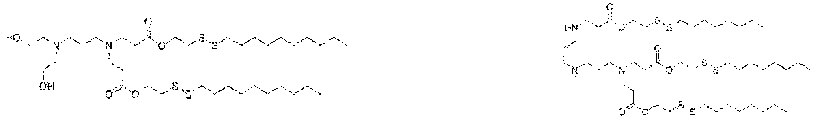

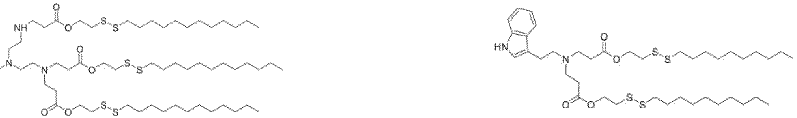

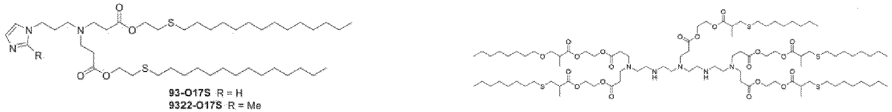

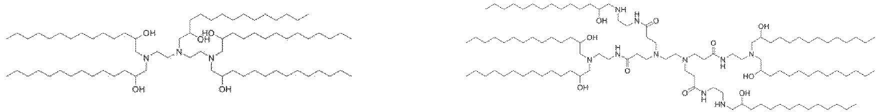

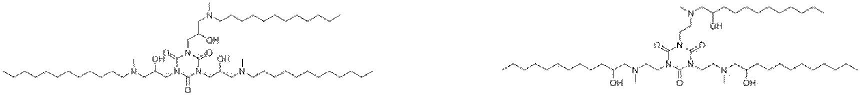

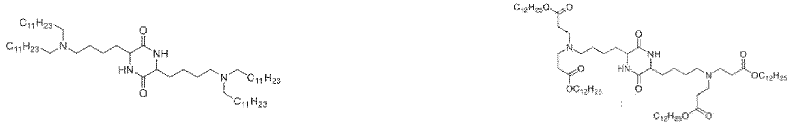

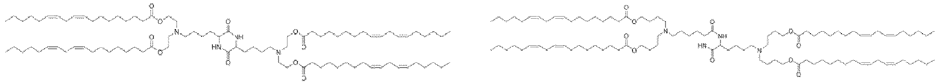

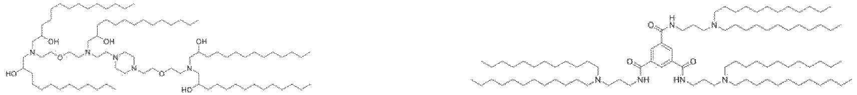

28. The pulmonary delivery system comprising ionizable lipids according to any one of claims 1 to 23, wherein the helper lipids are a mixture of phospholipid and steroid or a derivative thereof.

29. The pulmonary delivery system comprising ionizable lipids according to claim 28, wherein the phospholipid comprises at least one selected from 1,2-dilinoleoyl-sn-glycero-3-phosphocholine, 1,2-dimyristoyl-sn-glycero-3-phosphocholine, 1,2-dioleoyl-sn-glycero-3-phosphocholine, 1,2-dipalmitoyl-sn-glycero-3-phosphocholine, 1,2-distearoyl-sn-glycero-3-phosphocholine, 1,2-diundecanoyl-sn-glycero-3-phosphocholine, 1-palmitoyl-2-oleoyl-sn-glycero-3-phosphocholine, 1,2-di-O-octadecenyl-sn-glycero-3-phosphocholine, 1-oleoyl-2-cholesterylhemisuccinoyl-sn-glycero-3-phosphocholine, 1-hexadecyl-sn-glycero-3-phosphocholine, 1,2-dilinolenoyl-sn-glycero-3-phosphocholine, 1,2-diarachidonoyl-sn-glycero-3-phosphocholine, 1,2-didocosahexaenoyl-sn-glycero-3-phosphocholine, 1,2-dioleoyl-sn-glycero-3-phosphoethanolamine, 1,2-diphytanoyl-sn-glycero-3-phosphoethanolamine, 1,2-distearoyl-sn-glycero-3-phosphoethanolamine, 1,2-dilinoleoyl-sn-glycero-3-phosphoethanolamine, 1,2-dilinolenoyl-sn-glycero-3-phosphoethanolamine, 1,2-diarachidonoyl-sn-glycero-3-phosphoethanolamine, 1,2-didocosahexaenoyl-sn-glycero-3-phosphoethanolamine, sodium 1,2-dioleoyl-sn-glycero-3-phospho-rac-(1-glycerol), dipalmitoylphosphatidylglycerol, palmitoyloleoylphosphatidylethanolamine, distearoylphosphatidylethanolamine, dipalmitoylphosphatidylethanolamine, dimyristoylphosphatidylethanolamine, 1-stearoyl-2-oleoyl-phosphatidylethanolamine, 1-stearoyl-2-oleoyl-phosphatidylcholine, sphingomyelin, phosphatidylcholine, phosphatidylethanolamine, phosphatidylserine, phosphatidylinositol, phosphatidic acid, palmitoyloleoylphosphatidylcholine, lysophosphatidylcholine and lysophosphatidylethanolamine.

30. The pulmonary delivery system comprising ionizable lipids according to claim 28, wherein the steroid or derivative thereof comprises at least one selected from cholesterol, cholesterol stearate, coprosterol, sitosterol, ergosterol, campesterol, stigmasterol, brassicasterol, fucosterol, tomatidine, ursolic acid and α-tocopherol.

31. The pulmonary delivery system comprising ionizable lipids according to any one of claims 1 to 23, wherein the pulmonary delivery system comprising ionizable lipids further comprises an alcohol compound.

32. The pulmonary delivery system comprising ionizable lipids according to claim 31, wherein the alcohol compound comprises at least one selected from C₆-C₁₈ alkanol and polyethylene glycol; the molar ratio of the alcohol compound to the ionizable lipids is 0-2:1.

33. The pulmonary delivery system comprising ionizable lipids according to claim 1, wherein the amount of the ionizable lipid is 5-80 mol% of the total lipids in the formulation; and the amount of the helper lipid is 20-95 mol% of the total lipids in the formulation.

34. The pulmonary delivery system comprising ionizable lipids according to claim 1, wherein in the helper lipids,the molar ratio ofphospholipid and steroid or a derivative thereof is(5-60):(10-80).

35. The pulmonary delivery system comprising ionizable lipids according to claim 1, wherein the ionizable lipid is a first ionizable lipid, a second ionizable lipid, a third ionizable lipid, a mixture of the first ionizable lipid and the second ionizable lipid, or a mixture of the second ionizable lipid and the third ionizable lipid; in the mixture of the first ionizable lipid and the second ionizable lipid, the molar ratio of the first ionizable lipid to the second ionizable lipid is (5-90):(1-30); in the mixture of the second ionizable lipid and the third ionizable lipid, the molar ratio of the third ionizable lipid to the second ionizable lipid is (5-90):(1-30).

36. The pulmonary delivery system comprising ionizable lipids according to claim 1, wherein the pulmonary delivery system comprising ionizable lipids has a particle size of 20-400 nm, a polydispersity index (PDI) of less than 0.5, and a surfacepotential of -20 mV to 60 mV.

37. The pulmonary delivery system comprising ionizable lipids according to any one of claim 1 to 23, further comprises other excipients.

38. The pulmonary delivery system comprising ionizable lipids according to claim 37, wherein the pulmonary delivery system comprising ionizable lipids is in a liquid form or solid form.

39. A method for preparing the pulmonary delivery system comprising ionizable lipids according to any one of claims 1 to 38, comprising the step of mixing a bioactive substance, an ionizable lipid and a helper lipid in a solvent.

40. Use of the pulmonary delivery system comprising ionizable lipids according to any one of claims 1 to 38, in the manufacture of a medicament for delivering a bioactive substance by intranasal, intrabronchial or pulmonary administration.

41. The use according to claim 40, wherein the medicament is administered to a mammal or cells; the cells are at least one selected from respiratory epithelial cell populations, nasal cell populations, alveolar epithelial cell populations, lung cell populations, bronchial epithelial cell populations and human bronchial epithelial cell populations.

42. The use according to claim 40, wherein the medicament is administered by a nebulizer or an inhaler.
